# EUROPEAN PATENT APPLICATION

(11) **EP 3 971 176 A1**
(43) Date of publication of application: **23.03.2022**
(21) Application number: 20809358.3
(22) Date of filing: 15.05.2020
(51) Int. Cl.: C07D 401/04, C07D 307/52, C07D 235/06, C07D 207/16, A61K 31/4025, A61P 35/00

(54) **AROMATIC AMINE AR AND BET TARGETING PROTEIN DEGRADATION CHIMERA COMPOUND AND USE**

(30) Priority: 17.05.2019 CN 201910415060
(71) Applicant: Hinova Pharmaceuticals Inc., Chengdu, Sichuan 610041 (CN)
(72) Inventor: DU, Wu, Chengdu, Sichuan 610041 (CN); LV, Haibin, Chengdu, Sichuan 610041 (CN); LI, Haibo, Chengdu, Sichuan 610041 (CN); QIN, Dekun, Chengdu, Sichuan 610041 (CN); AI, Chaowu, Chengdu, Sichuan 610041 (CN); LI, Yu, Chengdu, Sichuan 610041 (CN); DUAN, Jingyi, Chengdu, Sichuan 610041 (CN); TU, Zhilin, Chengdu, Sichuan 610041 (CN); ZHANG, Chengzhi, Chengdu, Sichuan 610041 (CN); CHEN, Yuanwei, Chengdu, Sichuan 610041 (CN); LI, Xinghai, Chengdu, Sichuan 610041 (CN)
(74) Representative: Altmann Stößel Dick Patentanwälte PartG mbB
(86) International application number: PCT/CN2020/090480
(87) International publication number: WO 2020/233512

(57) **Abstract**

An aromatic amine androgen receptor (AR) and BET targeting protein degradation chimera compound and its use. Specifically provided is a compound represented by formula I. Experimental results show that the compound can target and degrade both AR and BRD4, and down-regulate the expression of AR and BRD4 proteins; the compound can inhibit the proliferation of a variety of prostate cancer cells; the compound can inhibit the proliferation of a prostate cancer cell line LNCaP/AR, which overexpresses the AR, and can achieve a good inhibition effect on a prostate cancer cell line 22RV1, which is resistant to a marketed prostate cancer drug (enzalutamide); the compound also shows good metabolic stability, and has a good application prospect in the preparation of an AR and/or BET protein degradation targeting chimera, and a drug for the treatment of related diseases regulated by the AR and BET.

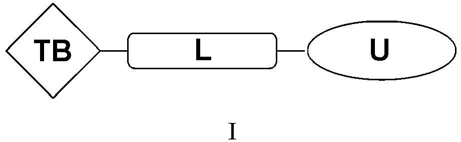

## Description

### Techinical field

The present invention relates to the field of medicinal synthesis, and in particular to an aromatic amine chimeric compound targeting the protein degradation of AR and BET as well as its use.

### Background technology

As the growing and aging of global population, the incidence of prostate cancer continues to increase. At present, the main treatment is androgen-deprivation therapy. Androgen receptor (AR) belongs to the nuclear receptor family and is a type of ligand-dependent transcription factor. The abnormal regulation of AR signaling pathway plays an important role in the occurrence and development of prostate cancer. Studies have shown that castration-resistant prostate cancer (CRPC) still depends on the role of AR. The androgen receptor contains 918 amino acids, and has a similar structure and function to other nuclear receptors. It consists of three important domains, namely the DNA binding domain (DBD), the ligand binding domain (LBD), and N-terminal domain (NTD), in which DBD and LBD are connected by a hinge region. The LBD present at C-terminal of AR is the site where AR binds to the ligand, which determines the specificity of the binding of the ligand to AR, and the ligand binds to the LBD to activate AR. At present, two transcriptional activation domains have been identified in AR, namely activation function 1 (AF1) in the NTD domain and the highly conserved hydrophobic pocket activation function 2 (AF2) in the LBD domain. Before 2010, docetaxel-based chemotherapy was the only treatment that could prolong the survival of patients with metastatic CRPC. Since 2011, three inhibitors of AR signaling pathway have been successively approved by FDA, namely abiraterone acetate and enzalutamide approved in 2011 and 2012 respectively for the treatment of metastatic castration-resistant prostate cancer (CRPC), as well as apalutamide just approved in 2018 for the treatment of non-metastatic CRPC.

Although abiraterone and enzalutamide, the second generation inhibitors of AR signaling pathway, have achieved some success in clinical treatment, drug resistance has emerged in clinic. F876L mutation in ligand-binding region is a missense mutation that causes resistance to enzalutamide and changes it from antagonist to agonist. In addition, AR splicing mutants, especially AR-v7 mutation lacking ligand-binding region, are an important reason to mediate the resistance to the second-generation drug. Therefore, there is an urgent need for novel inhibitors of AR signaling pathway to treat CRPC.

Bromodomain and extra-terminal domain (BET) is an epigenetic regulator that regulates the expression of genes by recognizing acetylated histones in DNA through BD1 and BD2 domains. BET protein family consists of BRD2, BRD3, BRD4 and BRDT. Except that BRDT only exists in testis, the other three protein subtypes are widely expressed in various tissues and cells. Studies have shown that the direct binding of BRD2\3\4 to AR can regulate the expression of its downstream genes, and this interaction between AR and BD1 can be blocked by BET inhibitors, so as to block AR-mediated gene transcription and inhibit the growth of CRPC tumors; it was also found that this interaction still had a good inhibitory effect on AR-v7 positive and androgen-independent 22Rv1 tumor model. In recent years, several BRD protein inhibitors have entered clinical trials for the treatment of CRPC, including OTX-105, ZEN003694 and GS-5829, among which GS-5829 can also be used for lymphoma.

The studies have also indicated that in advanced prostate cancer, the up-regulation of AR enhanced the chromatin opening mediated by bromodomain, and the cells with AR overexpression were more sensitive to BET inhibitors. In addition, studies have demonstrated that CRPC cells resistant to enzalutamide are still sensitive to BET inhibitors (such as JQ1). Therefore, compared with anti-androgen drugs alone, the combination of the inhibitors against AR and BET can better inhibit the growth of prostate cancer.

Traditional small molecule inhibitors inhibit the function of target proteins by binding to target proteins, but the long-term use of small molecule drugs will inevitably result in drug resistance. Moreover, in order to achieve the desired effect, small molecule compounds need to maintain a certain concentration in cells, and high concentrations of small molecules will have adverse reactions due to off-target. Therefore, finding small molecular compounds that can overcome these defects is of great significance in the research and development of new drugs.

In recent years, proteolytic targeting chimera (PROTACs) have attracted extensive attention as small molecules that can induce the degradation of target protein. As a bifunctional molecule, PROTACs include a small molecule compound that can bind to the protein of interest (POI), a linker group introduced at its appropriate position, and a small molecule compound that can bind to E3 ubiquitinase. As a small molecule probe, PROTACs can combine with POI and E3 ubiquitinase at the same time, so as to promote the ubiquitination of POI, which can be recognized and degraded by proteasome.

In the past 10 years, many tumor-related POI (including AR, ER, BRD4, ERRa, RIPK2, etc.) have been proved to be regulated and degraded based on PROTACs. And the latest research confirmed the catalytic properties of PROTACs, indicating that the chimeric molecular concentration lower than the concentration required for a single inhibitor can achieve the same therapeutic effect. Therefore, the new tumor treatment strategy of protein degradation induced by PROTACs can regulate the level of POI by the intracellular ubiquitin-proteasome degradation system, so as to overcome the defects of traditional small molecule inhibitors. Therefore, the preparation of compounds that can degrade AR and BET at the same time is of great significance in the preparation of PROTACs that has a dual targeting degradation on AR and BET and in the preparation of drugs for the treatment of malignant tumors (especially prostate cancer).

### Content of the invention

The object of the present invention is to provide a PROTAC capable of causing a targeting degradation on AR and/or BET.

The present invention provides a compound of formula I, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a combination thereof, or a metabolite thereof, or a metabolic precursor thereof, or an isotopic compound thereof: 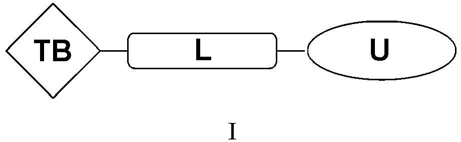 wherein, TB is an androgen receptor (AR) and/or a BET target recognition/binding part, L is a linker part, and U is a ubiquitin protease recognition/binding part; and the three parts are connected by chemical bonds; the structure of said TB is represented by formula (I-A): 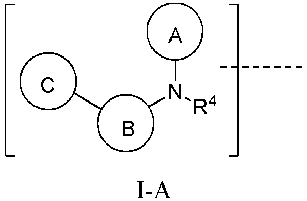 wherein, each of rings A, B and C is independently selected from the group consisting of none, substituted or unsubstituted unsaturated heterocycles, substituted or unsubstituted unsaturated carbocycles, and substituted or unsubstituted fused rings, and rings A, B and C are not none at the same time. Preferably, each of rings A, B and C is independently selected from the group consisting of none, a substituted or unsubstituted monocyclic aromatic ring, a substituted or unsubstituted monocyclic heteroaromatic ring, and a substituted or unsubstituted fused ring; more preferably, each of rings A, B and C is independently selected from the group consisting of none, a substituted or unsubstituted 3-8 membered monocyclic aromatic ring, a substituted or unsubstituted 3-8 membered monocyclic heteroaromatic ring, a substituted or unsubstituted heteroaromatic-ring-fused heteroaromatic ring, a substituted or unsubstituted benzene-fused aromatic ring, a substituted or unsubstituted benzene-fused heteroaromatic ring, a substituted or unsubstituted benzene-fused saturated carbocycle, and a substituted or unsubstituted benzene-fused saturated heterocyclic ring.

Each of the substituents in above rings A, B and C is independently selected from the group consisting of deuterium, halogen, -CN, hydroxyl, nitro, amino, 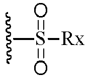 , -Q₀₋OH, -Q₃-C(O)R⁷, -Q₄-CO(O)R⁸, -Q₅-(O)COR⁹, -Q₆-NHC(O)R¹⁰, -Q₇-C(O)NHR¹¹, -Q₈-CN alkenyl substituted with one or more R¹², alkynyl substituted with one or more R¹³, alkyl substituted with one or more R¹, alkoxy substituted with one or more R², aryl or heteroaryl substituted with one or more R³, cycloalkyl substituted with one or more R⁵, and heterocyclic group substituted with one or more R⁶; each of R_{X}, R¹, R², R³, R⁵, R⁶, k⁷, R⁸, R⁹, R¹⁰, R¹¹, R¹², and R¹³ is independently selected from the group consisting of H, deuterium, halogen, -CN, hydroxyl, nitro, amino, alkyl or deuterated compounds thereof or halogenated compounds thereof, and -Qo-OH, wherein each of Q₀, Q₃, Q₄, Q₅, Q₆, Q₇, and Q₈ is independently selected from the group consisting of none, C₁-C₈ alkyl, and C₃-C₆ cycloalkyl;
R⁴ is selected from the group consisting of none, hydrogen, deuterium, halogen, -CN, hydroxyl, nitro, amino, 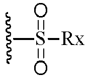 , -Qo-OH, -Q₃-C(O)R⁷, -Q₄-CO(O)R⁸, -Q₅-(O)COR⁹, -Q₆-NHC(O)R¹⁰, -Q₇-C(O)NHR¹¹, alkenyl substituted with one or more R¹², alkynyl substituted with one or more R¹³, alkyl substituted with one or more R¹, alkoxy substituted with one or more R², aryl or heteroaryl substituted with one or more R³, cycloalkyl substituted with one or more R⁵, and heterocyclic group substituted with one or more R⁶; each of R_{X}, R¹, R², R³, R⁵, R⁶, R⁷, R⁸, R⁹, R¹⁰, R¹¹, R¹², and R¹³ is independently selected from the group consisting of H, deuterium, halogen, -CN, hydroxyl, nitro, amino, alkyl or deuterated compounds thereof or halogenated compounds thereof, and -Qo-OH, wherein each of Q₀, Q₃, Q₄, Q₅, Q₆, and Q₇ is independently selected from 0-8 methylene groups;
or, any two groups of substituents in rings A, B, C and R⁴, together with the substituted atoms to which they are linked, are connected to form a ring; 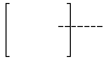 represents that formula I-A is the remaining group of the molecule 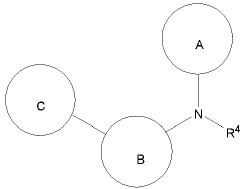 after removing any hydrogen.

BET is the abbreviation of bromodomain and extra-terminal domain, i.e. the bromodomain and extra-terminal domain.

Further, said TB has the structure of formula III: 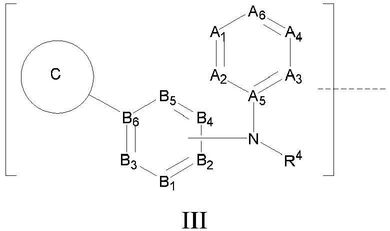 B₁ is CR^{b1} or N; B₂ is CR^{b2} or N; B₃ is CR^{b3} or N; B₄ is CR^{b4} or N; B₅ is CR^{b5} or N; B₆ is C; A₁ is CR^{a1} or N; A₂ is CR^{a2} or N; A₃ is CR^{a3} or N; A₄ is CR^{a4} or N; A₅ is C; A₆ is CR^{a6} or N; each of R^{b1}, R^{b2}, R^{b3}, R^{b4}, R^{b5}, R^{a1}, R^{a2}, R^{a3}, R^{a4}, and R^{a6} is independently selected from the group consisting of hydrogen, deuterium, -CN, amino, nitro, halogen, -Qo-OH, 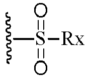 -Q₇-C(O)NHR¹¹, C₁-C₅ alkyl or deuterated compounds thereof or halogenated compounds thereof or cyanated compounds thereof, C₁-C₅ alkoxy or deuterated compounds thereof or halogenated compounds thereof or cyanated compounds thereof, a substituted or unsubstituted 3-6 membered cycloalkyl, a substituted or unsubstituted 4-6 membered unsaturated heterocyclic group, and a substituted or unsubstituted 5-6 membered heteroaryl group, or two adjacent substituents in the ring, together with the substituted atoms to which they are linked, form substituted or unsubstituted 3-6 membered heterocycles; wherein, each of the substituents in said 3-6 membered cycloalkyl, said 4-6 membered unsaturated heterocyclic group, and said 5-6 membered heteroaryl group is independently selected from the group consisting of -CN, amino, nitro, halogen, C₁-C₃ alkyl or deuterated compounds thereof or halogenated compounds thereof, and -Q₁-OH; each of Q₀ and Q₁ is independently selected from 0-5 methylene groups; each of Rₓ and R¹¹ is independently selected from the group consisting of H, deuterium, and C₁-C₃ alkyl;
ring C and R⁴ are as described above.

Further, said TB has the structure of formula VI-A: 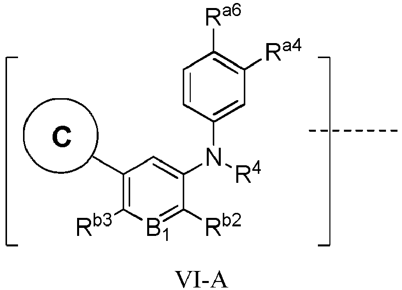 wherein, each of R^{a4} and R^{a6} is independently selected from the group consisting of H, deuterium, halogen, CN, C₁-C₅ alkyl or deuterated compounds thereof or halogenated compounds thereof or cyanated compounds thereof, C₁-C₅ alkoxy or deuterated compounds thereof or halogenated compounds thereof or cyanated compounds thereof, amino, amido, a substituted and unsubstituted 3-6 membered saturated cycloalkyl, and a substituted or unsubstituted 4-6 membered unsaturated heterocyclic group; wherein each of the substituents in said saturated cycloalkyl and said unsaturated heterocyclic group is independently selected from the group consisting of halogen, CN, a deuterated or non-deuterated C₁~C₂ alkyl, and -Q₁-OH; Q₁ is selected from 0-2 methylene groups;
R⁴ is selected from the group consisting of none, H, and a deuterated or non-deuterated C₁-C₂ alkyl;
B₁ is selected from the group consisting of CR^{b1} and N; R^{b1} is selected from the group consisting of H, deuterium, and halogen; preferably, B₁ is CH;
R^{b3} is selected from the group consisting of H, deuterium, and halogen;
R^{b2} is selected from the group consisting of deuterated or non-deuterated methyl and ethyl; ring C is as described above.

Further, ring C is selected from a 5-membered monocyclic heteroaromatic ring substituted with 0-4 substituents; the heteroatom in said 5-membered monocyclic heteroaromatic ring is selected from one or more of O, S and N; each of the substituents is independently selected from the group consisting of deuterium, halogen, C₁-C₆ alkyl and C₃-C₆ cycloalkyl.

Further, ring C is one of the following structures:

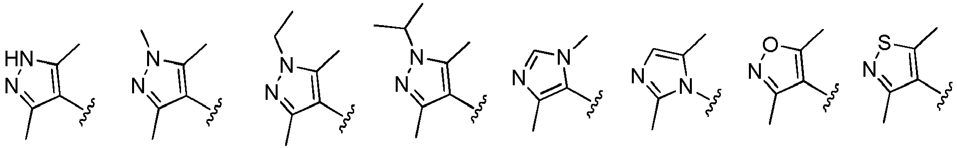

Further, ring C is none, and said TB has the structure of formula VI-B: 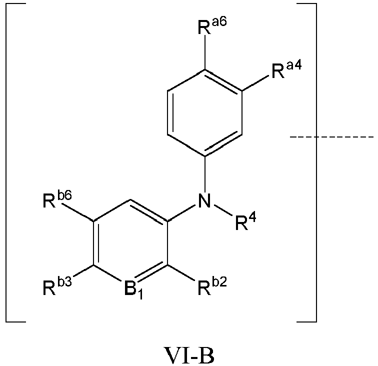 wherein, each of R^{a4} and R^{a6} is independently selected from the group consisting of H, deuterium, halogen, CN, C₁-C₅ alkyl or deuterated compounds thereof or halogenated compounds thereof or cyanated compounds thereof, C₁-C₅ alkoxy or deuterated compounds thereof or halogenated compounds thereof or cyanated compounds thereof, amino, amido, a substituted and unsubstituted 3-6 membered saturated cycloalkyl, and a substituted or unsubstituted 4-6 membered unsaturated heterocyclic group; wherein each of the substituents in said saturated cycloalkyl and said unsaturated heterocyclic group is independently selected from the group consisting of halogen, CN, a deuterated or non-deuterated C₁-C₂ alkyl, and -Q₁-OH; Q₁ is selected from 0-2 methylene groups;
R⁴ is selected from the group consisting of none, H, and a deuterated or non-deuterated C₁-C₂ alkyl;
B₁ is selected from the group consisting of CR^{b1} and N; R^{b1} is selected from the group consisting of H, deuterium, and halogen; preferably, B₁ is CH;
R^{b2} is selected from the group consisting of deuterated or non-deuterated methyl and ethyl;
R^{b3} and R^{b6}, together with the substituted atoms to which they are linked, form a substituted or unsubstituted 5-membered unsaturated heterocycle; wherein each of the substituents in the 5-membered unsaturated heterocycle is independently selected from the group consisting of -CN, amino, nitro, halogen, C₁-C₂ alkyl or deuterated compounds thereof or halogenated compounds thereof, and -Q₁-OH; Q1 is selected from 0-2 methylene groups.

Further, the structure of TB is selected from one of the following structures:

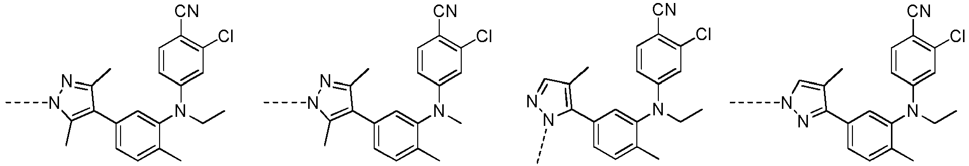

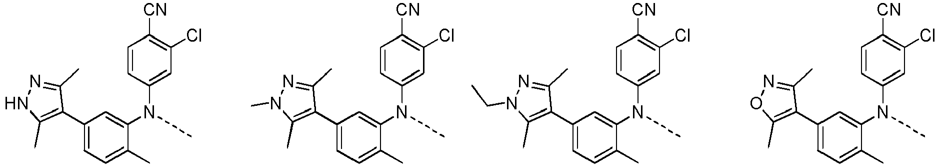

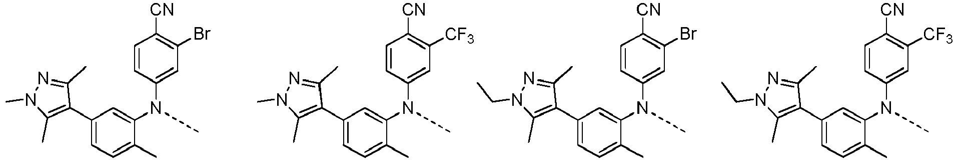

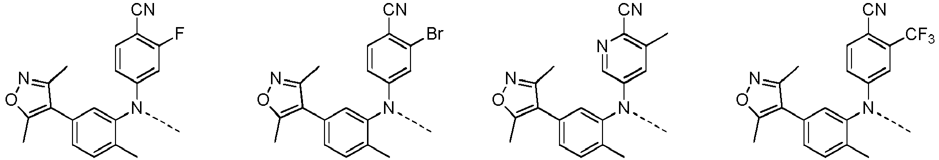

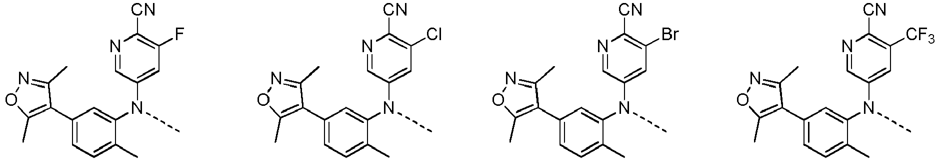

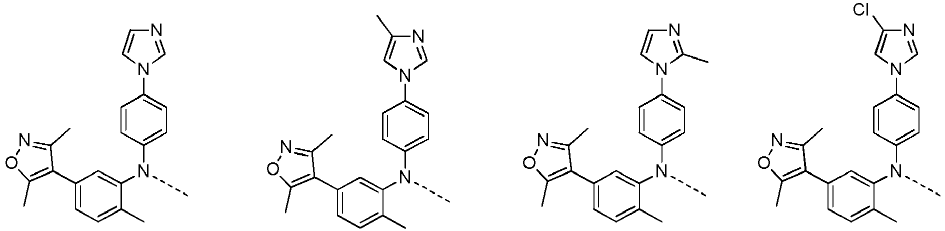

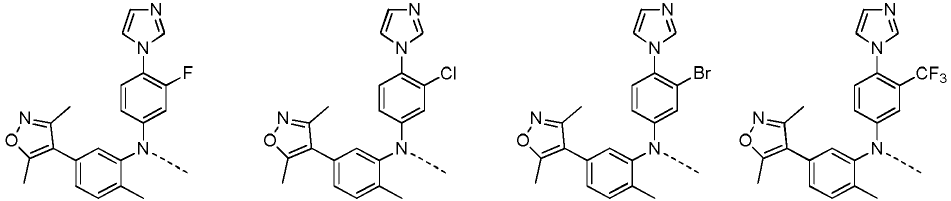

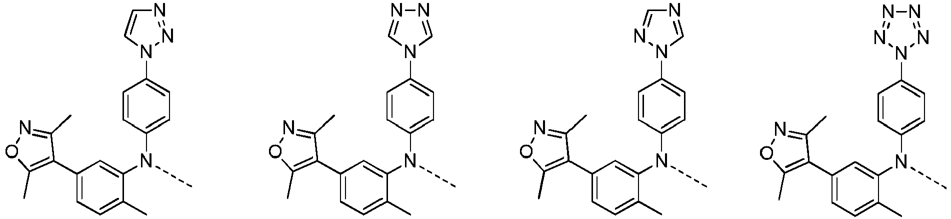

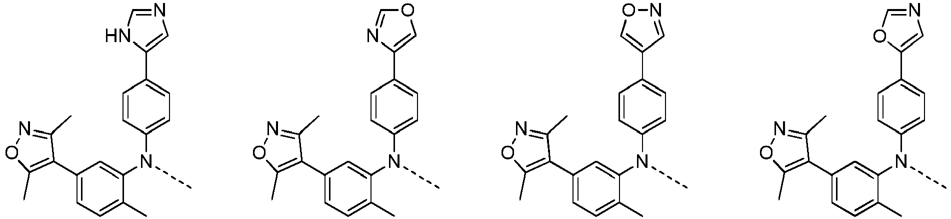

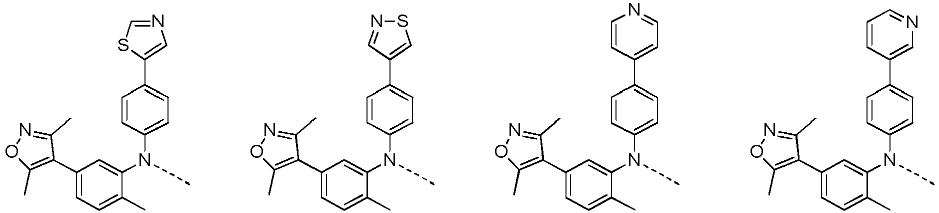

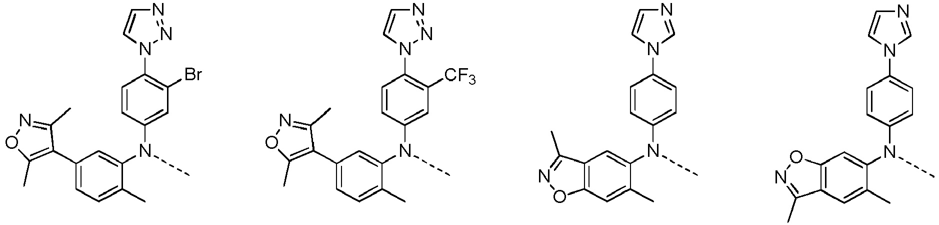

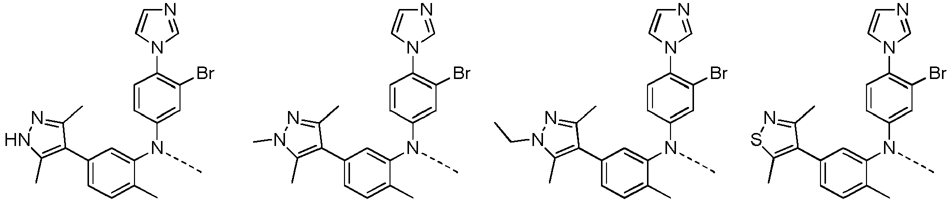

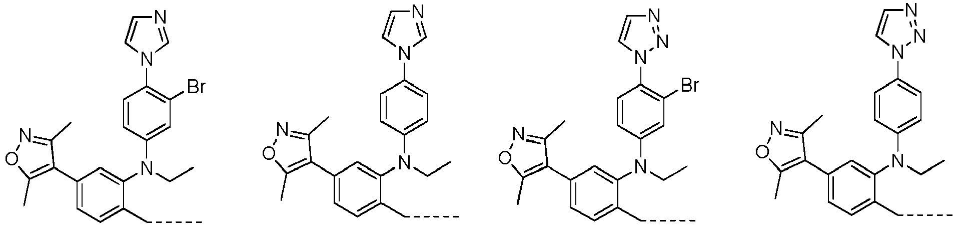

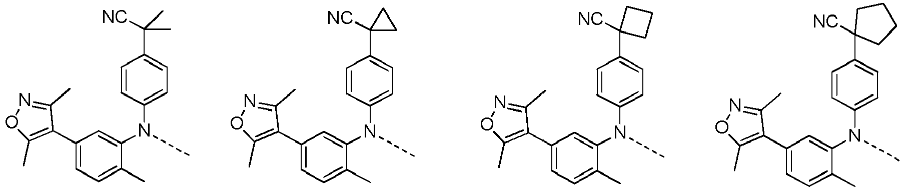

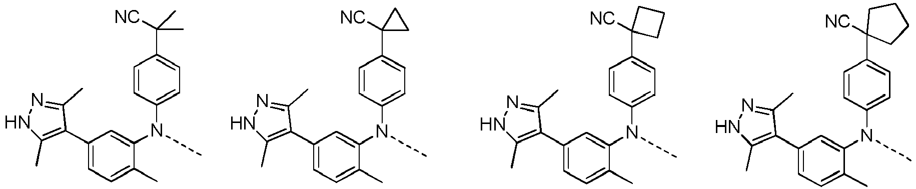

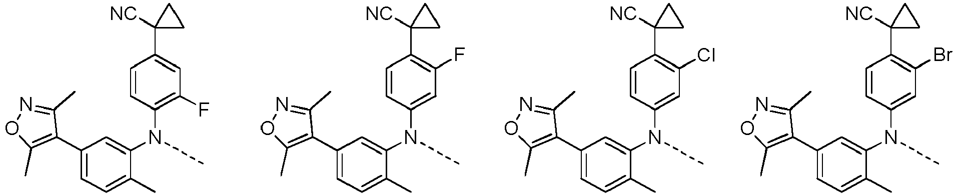

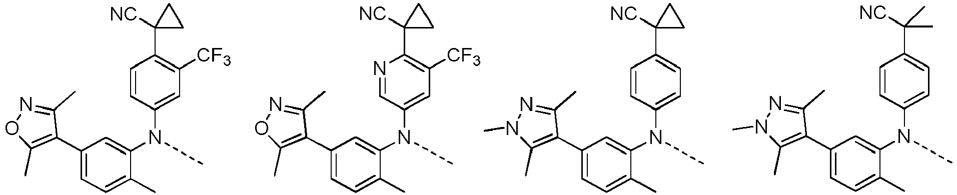

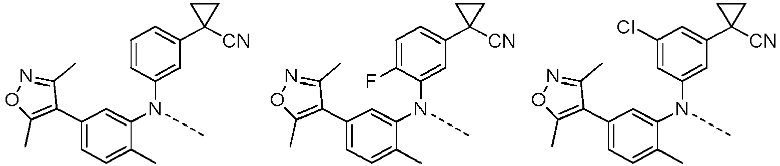

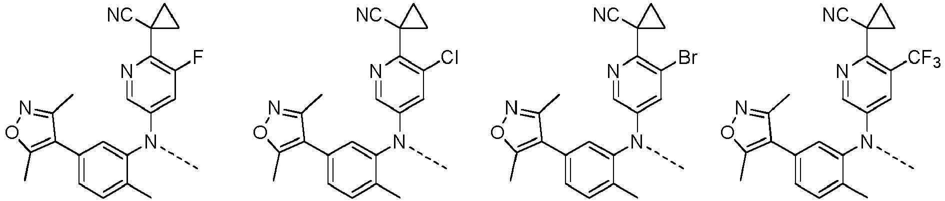

Further, said U has the structure of formula II-A: 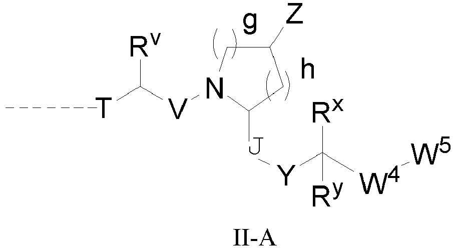 wherein, each of T and Y is respectively selected from the group consisting of none, O, S, NR^{T1}, and CR^{T2}R^{T3};
each of V and J is respectively selected from the group consisting of none, C=O, -SO-, -SO₂-, and CR^{s1}R^{s2};
each of R^{s1}, R^{s2}, R^{T1}, R^{T2}, and R^{T3} is respectively selected from the group consisting of H, deuterium, C₁₋₆ alkyl or a halogenated compound thereof or a deuterated compound thereof, and 3-8 membered cycloalkyl containing 0-2 heteroatoms, or R^{T2} and R^{T3} are linked to form a 3-8 membered ring containing 0-2 heteroatoms;
R^{v} is selected from the group consisting of H, deuterium, C₁₋₆ alkyl or a halogenated compound thereof or a deuterated compound thereof, and a cycloalkyl containing 0-3 heteroatoms or a halogenated compound thereof;
each of g and h is independently an integer of from 0 to 3, and g and h are not 0 at the same time;
Z is selected from the group consisting of H, deuterium, hydroxy, amino, C₁₋₆ alkyl, C₃₋₆ cycloalkyl, halogenated C₁₋₆ alkyl, -OR^{Z1}, -NR^{Z1}R^{Z2}, -COR^{Z3}, -CO₂R^{Z3}, -OCOR^{Z3}, -NHCOR^{Z3}, -CONHR^{Z3}, and -SO₂R^{Z3}; each of R^{Z1} and R^{Z2} is selected from the group consisting of H, deuterium, C₁₋₆ alkyl or a halogenated compound thereof or a deuterated compound thereof, and a 3-8 membered cycloalkyl with 0-2 heteroatoms; R^{Z3} is selected from the group consisting of substituted or unsubstituted C₁₋₆ alkyl, substituted or unsubstituted C₃₋₆ cycloalkyl, substituted or unsubstituted C₃₋₆ heterocyclic group, substituted or unsubstituted aryl, and substituted or unsubstituted heteroaryl; the substituent of R^{Z3} is selected from the group consisting of halogen and C₁₋₃ alkyl;
each of R^{x} and R^{y} is respectively selected from the group consisting of H, deuterium, C₁₋₆ alkyl, halogenated C₁₋₆ alkyl, C₁₋₆ alkyl substituted with the substituent containing a heteroatom, -L_{y}-OH, and a cycloalkyl with 0-3 heteroatoms or a halogenated compound thereof, or R^{x} and R^{y} are linked to form a 3-8 membered ring containing 0-2 heteroatoms; wherein, L_{y} is selected from the group consisting of 0-5 methylene groups;
each of W⁴ and W⁵ is respectively selected from the group consisting of aryl and heteroaryl substituted with 0-3 substituents; each of said substituents is independently selected from the group consisting of H, deuterium, halogen, hydroxy, amino, thiol, sulfonyl, sulfoxide, nitro, cyano, CF₃, heterocyclic group, C₁₋₆ alkyl, C₃₋₆ cycloalkyl, C₁₋₆ alkoxy, C₁₋₆ alkylamino, C₂₋₆ alkenyl, and C₂₋₆ alkynyl;
or,
said U has the structure of formula II-B: 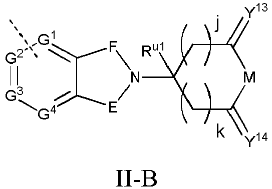
wherein, M is selected from the group consisting of O, S, and NR^{m}; wherein R^{m} is selected from the group consisting of H, deuterium, C₁₋₆ alkyl, C₃₋₆ cycloalkyl, C₃₋₆ heterocyclic group, and 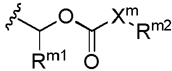
said R^{m1} is selected from the group consisting of H, deuterium, C₁₋₆ alkyl, and C₃₋₆ cycloalkyl; X^{m} is selected from the group consisting of none, O, S, and NR^{m3};
each of R^{m2} and R^{m3} is respectively selected from the group consisting of H, deuterium, C₁₋₆ alkyl, C₃₋₆ cycloalkyl, C₃₋₆ heterocyclic group, 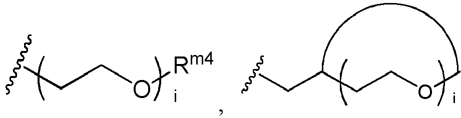 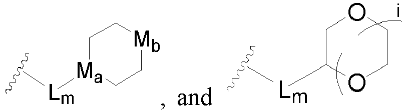 ; said i is an integer of from 0 to 12; R^{m4} is selected from the group consisting of H, deuterium, and C₁₋₆ alkyl; Lₘ is selected from the group consisting of 0-5 methylenes group; Mₐ is selected from the group consisting of N and CH; M_{b} is selected from the group consisting of O, S, CH₂, and NH;
each of E and F is respectively selected from the group consisting of CO, CS, NR^{e1}, O, S, SO₂, CH₂, CD₂, CR^{e2}R^{e3}, 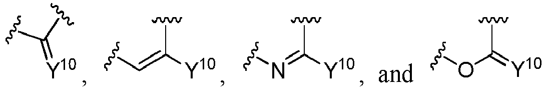 ; each of R^{e1}, R^{e2}, and R^{e3} is respectively selected from the group consisting of C₁₋₆ alkyl, C₁₋₆ alkoxy, H, deuterium, halogen, hydroxy, and amino;
each of Y¹⁰, Y¹³, and Y¹⁴ is respectively selected from the group consisting of O, S, and C₁₋₃ alkeylene;
each of j and k is respectively an integer of from 0 to 3, and j and k are not 0 at the same time;
each of G¹, G², G³, and G⁴ is respectively selected from the group consisting of O, S, N, CR^{g1}, CR^{g2}, CR^{g3}, and CR^{g4}; wherein each of R^{g1}, R^{g2}, R^{g3}, and R^{g4} is respectively selected from the group consisting of H, deuterium, halogen, hydroxy, amino, thiol, sulfonyl, sulfoxide, nitro, cyano, CF₃, heterocyclic group, C₁₋₆ alkyl, C₃₋₆ cycloalkyl, C₁₋₆ alkoxy, C₁₋₆ alkylamino, C₂₋₆ alkenyl, and C₂₋₆ alkynyl;
R^{u1} is selected from the group consisting of H, deuterium, and C₁₋₆ alkyl;
or,
said U has the structure of II-C:

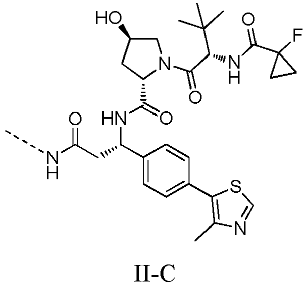

Further, said formula II-A has the structure of VIII-A: 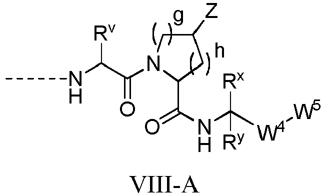 wherein, R^{v}, Z, g, h, R^{x}, R^{y}, W⁴, and W⁵ are as described above;
or, in said formula II-B, 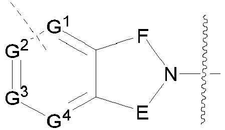 is selected from the group consisting of the structures of formulas (XI-B), (XI-C), (XI-D), (XI-E) and (XI-F): 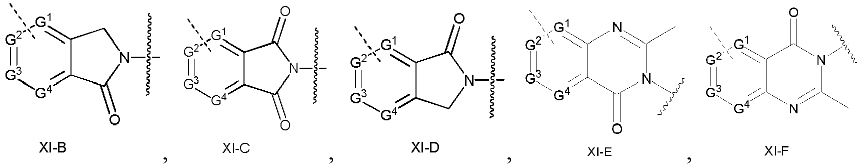 wherein, G¹, G², G³, and G⁴ are as described above.

Further, said formula VIII-A has the structure of IX-A: 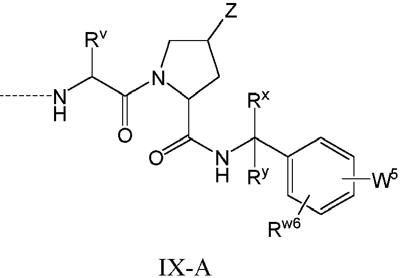 wherein, R^{w6} is selected from the group consisting of H, deuterium, halogen, hydroxy, amino, thiol, sulfonyl, sulfoxide, nitro, cyano, CF₃, heterocyclic group, C₁₋₆ alkyl, cycloalkyl, C₁₋₆alkoxy, C₁₋₆ alkylamino, C₂₋₆ alkenyl, and C₂₋₆ alkynyl;
W⁵ is selected from the group consisting of 5-6 membered aryl substituted with 0-3 substituents, and 5-6 membered heteroaryl; the heteroatom in said 5-6 membered heteroaryl is selected from one or more of O, S, and N; each of said substituents is respectively selected from the group consisting of halogen, hydroxy, amino, thiol, sulfonyl, sulfoxide, nitro, cyano, CF₃, heterocyclic group, C₁₋₆ alkyl, cycloalkyl, C₁₋₆alkoxy, C₁₋₆ alkylamino, C₂₋₆ alkenyl, and C₂₋₆ alkynyl;
R^{v}, Z, R^{x}, and R^{y} are as described above.

Further, said W⁵ is selected from the following structures:

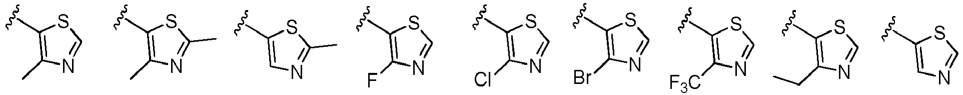

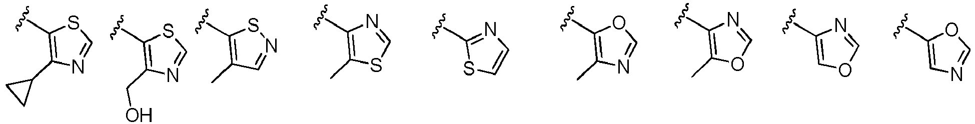

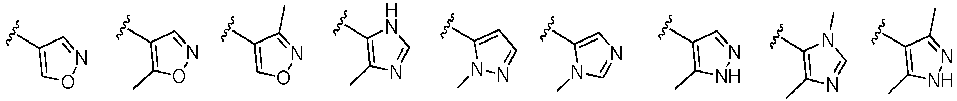

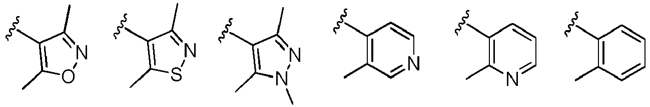

Further, said U is selected from the following structures:

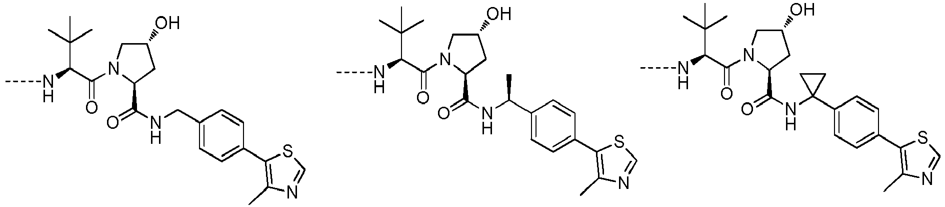

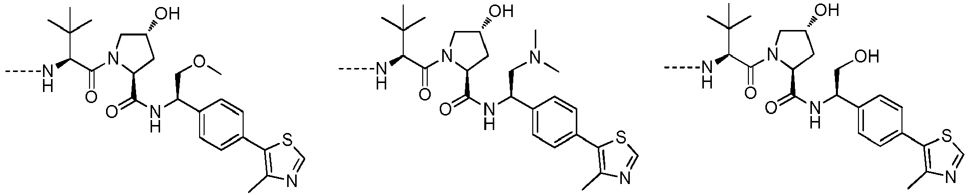

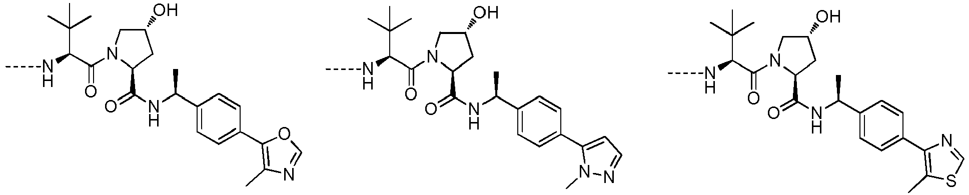

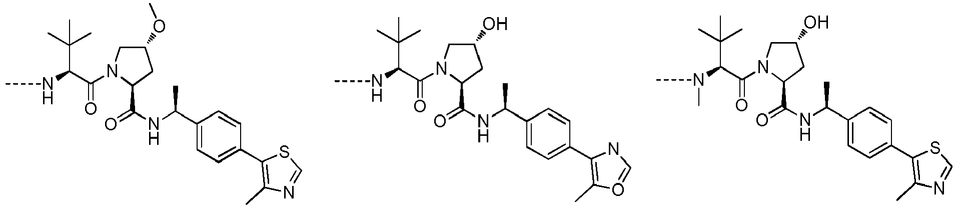

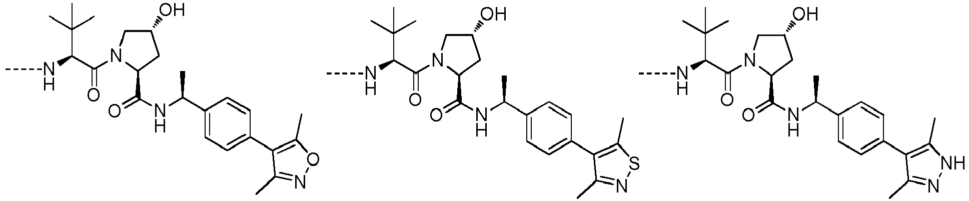

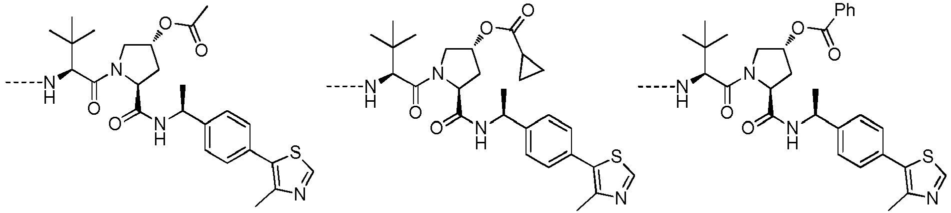

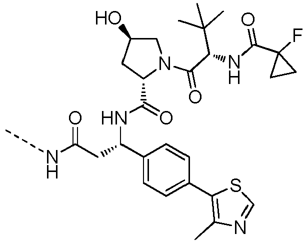

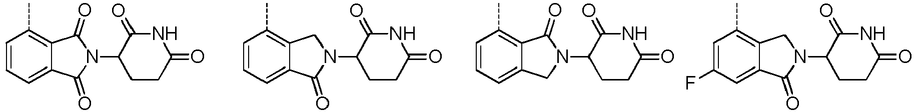

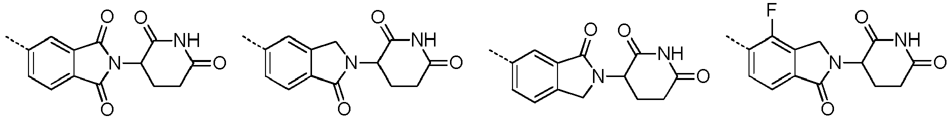

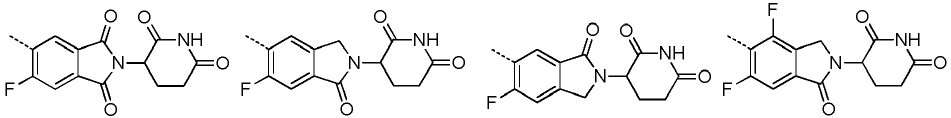

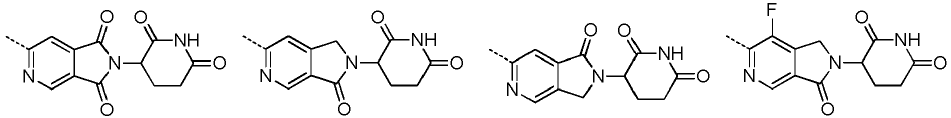

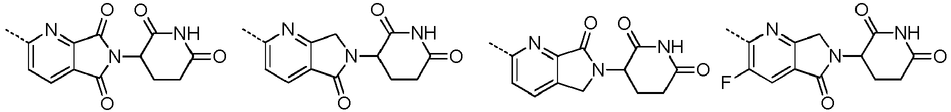

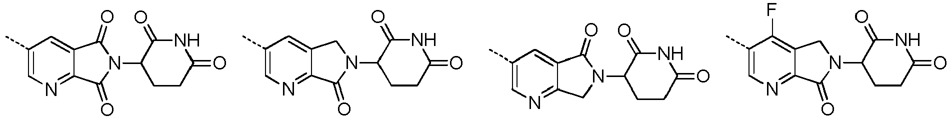

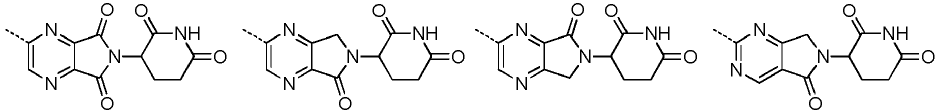

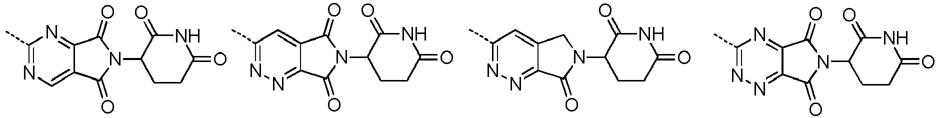

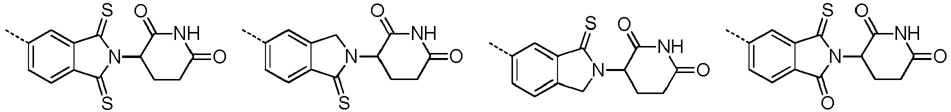

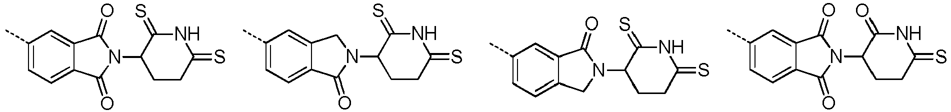

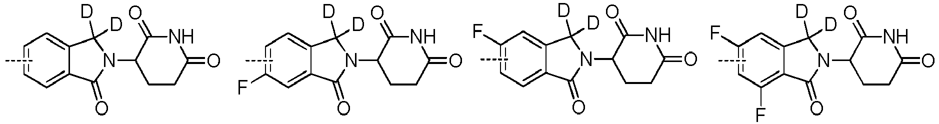

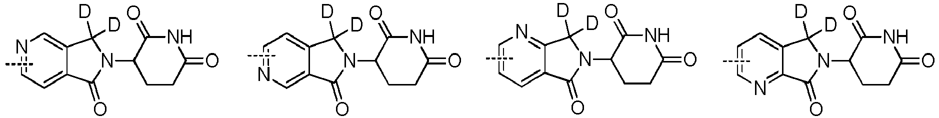

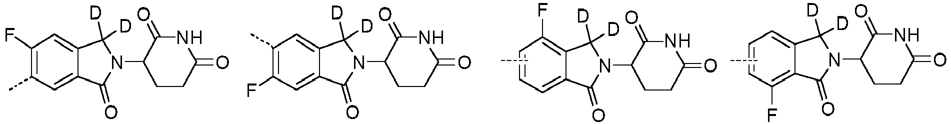

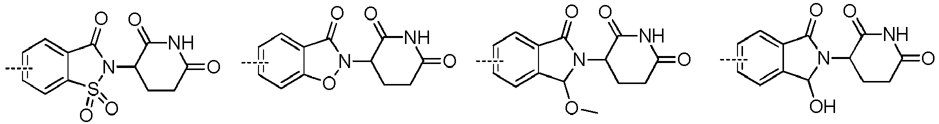

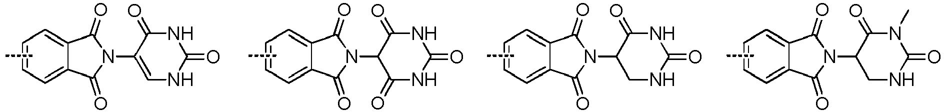

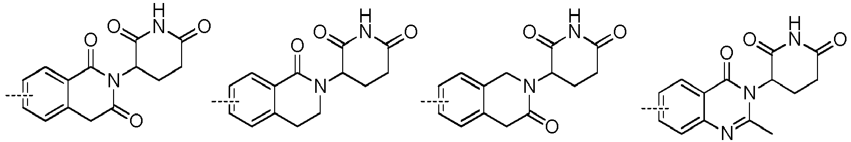

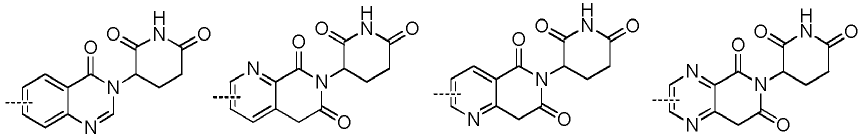

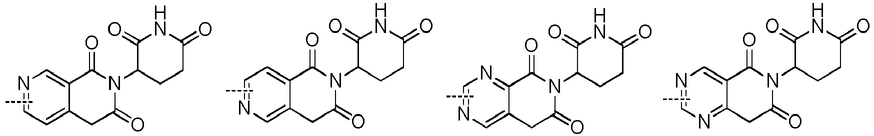

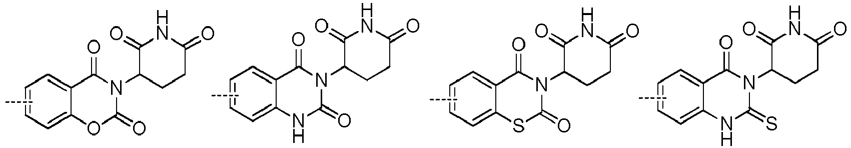

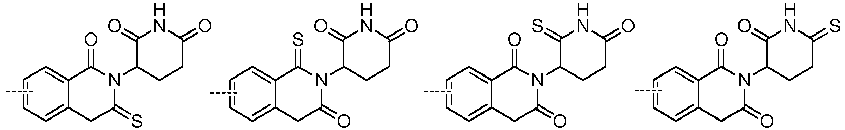

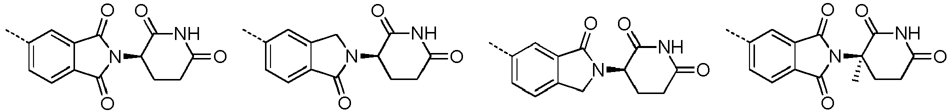

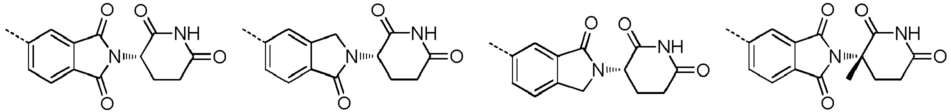

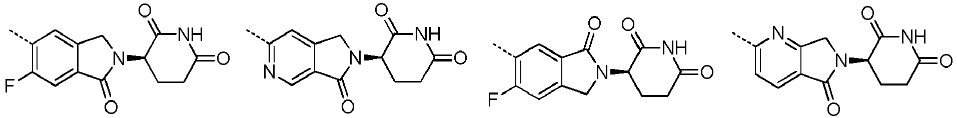

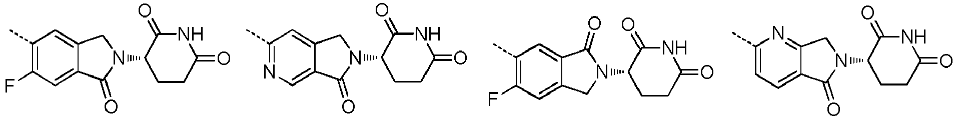

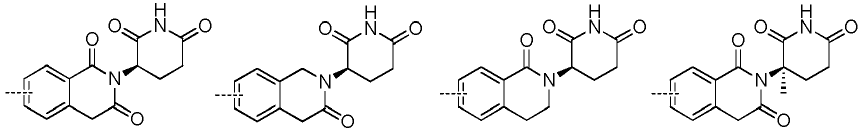

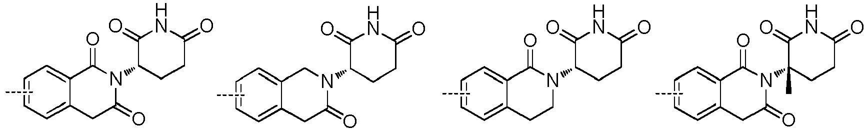

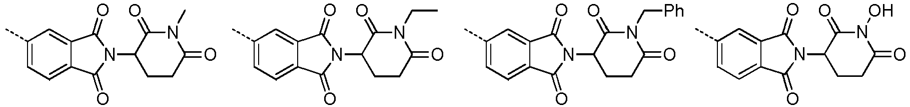

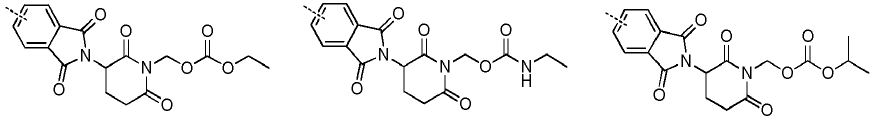

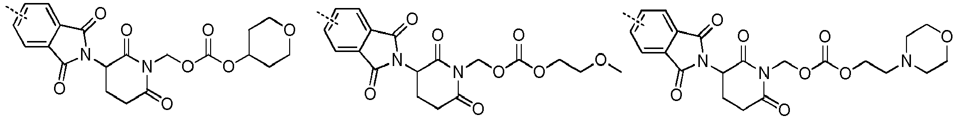

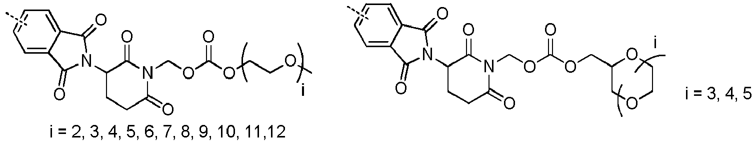

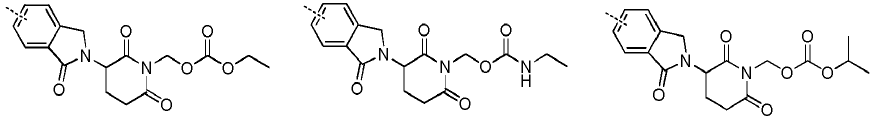

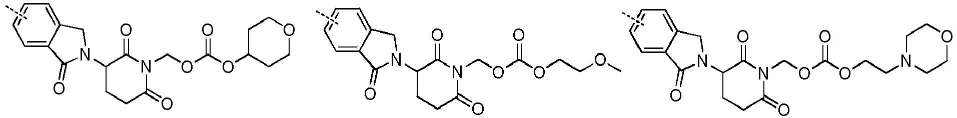

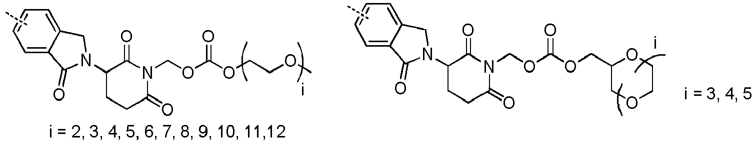

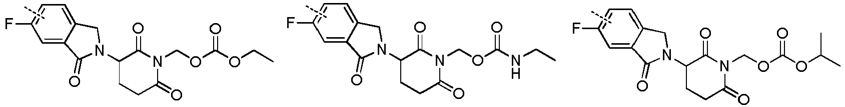

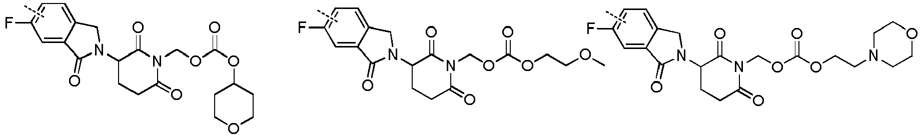

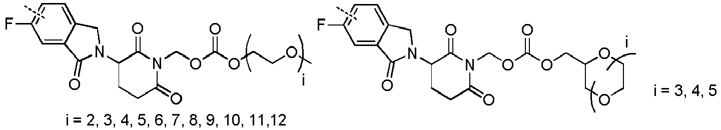

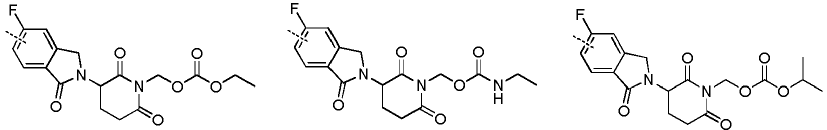

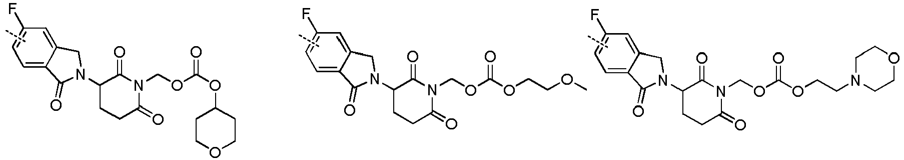

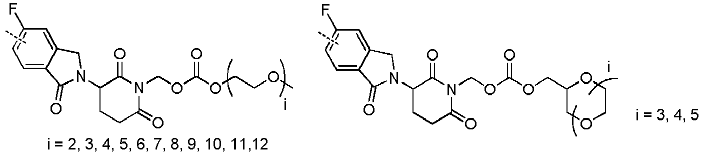

Further, said L has the structure of formula XII: 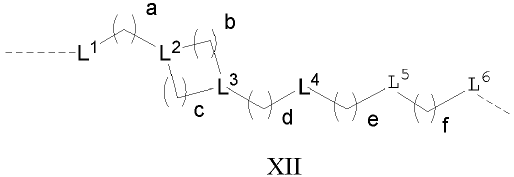 wherein, each of L¹, L², L³, L⁴, L⁵, and L⁶ is respectively selected from the group consisting of none, a bone, O, S, NR^{L1}, CR^{L2}R^{L3}, C=O, C=S, SO, SO₂, a substituted or unsubstituted alkenyl, a substituted or unsubstituted alkynyl, a substituted or unsubstituted monocycloalkyl, a substituted or unsubstituted monoheterocyclic group, a substituted or unsubstituted aryl, a substituted or unsubstituted heteroaryl, a substituted or unsubstituted bridged cycloalkyl, a substituted or unsubstituted bridged-heterocyclic group, a substituted or unsubstituted spirocycloalkyl, a substituted or unsubstituted spiroheterocyclic group, a substituted or unsubstituted fused cycloalkyl, and a substituted or unsubstituted fused heterocyclic group;
the above substituent is selected from the group consisting of C₁₋₆ alkyl, -L-OH, and halogen; L is selected from 0-6 methylene groups;
each of R^{L1}, R^{L2}, and R^{L3} is respectively selected from the group consisting of H, deuterium, C₁₋₆ alkyl or a halogenated compound thereof or a deuterated compound thereof, and a 3-8 membered cycloalkyl with 0-2 heteroatoms, or R^{L2} and R^{L3} are linked to form a 3-8 membered ring containing 0-2 heteroatoms;
each of a, b, c, d, e, and f is respectively an integer of from 0 to 5.

Further, said L has the structure of formula XII-A: 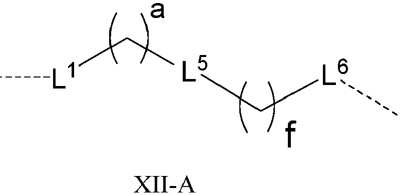 wherein, L₁, L₅, L₆, a, and f are as described above;
or, said L has the structure of formula XII-B: 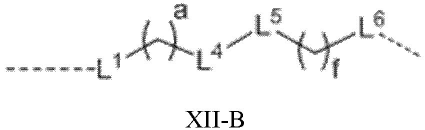 wherein, L₁, L₄, L₅, L₆, a, and f are as described above;
or, said L has the structure of formula XII-C: 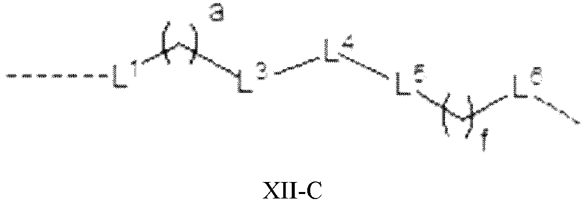 wherein, L₁, L₃, L₄, L₅, L₆, a, and f are as described above;
or, said L has the structure of formula XII-D: 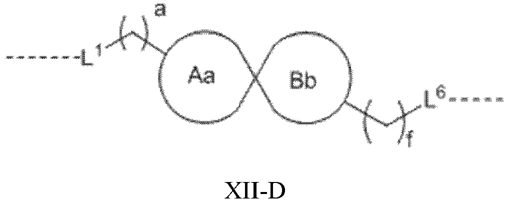 wherein, L₁, L₆, a, and f are as described above; rings Aa and Bb share one carbon atom, and each of rings Aa and Bb is independently selected from the group consisting of 3-6 membered saturated monocycloalkyl and 3-6 membered saturated monocyclic heterocyclyl;
or, said L has the structure of formula XII-E: 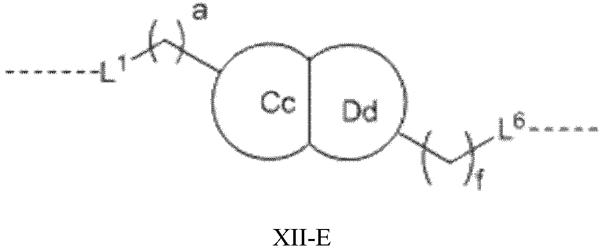 wherein, L₁, L₆, a, and f are as described above; rings Cc and Dd share two carbon atoms, and each of rings Cc and Dd is independently selected from the group consisting of 3-6 membered saturated monocycloalkyl or 3-6 membered saturated monocyclic heterocyclyl.

Further, said L is selected from the following structures: 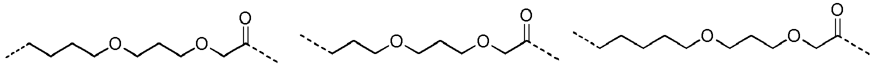 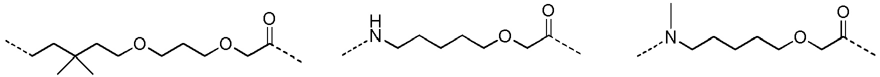 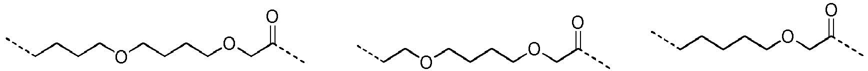 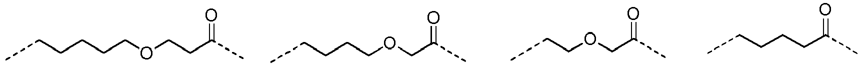 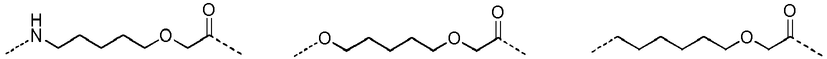 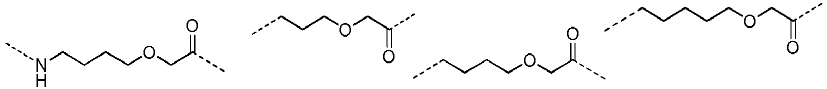 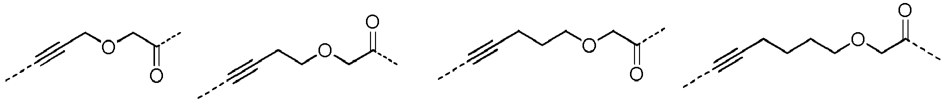 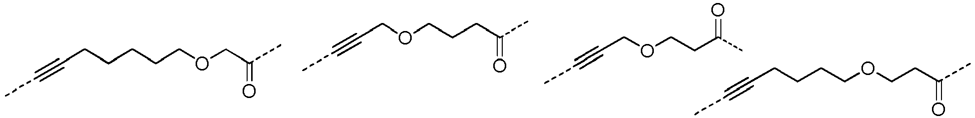 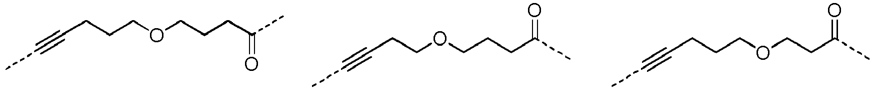 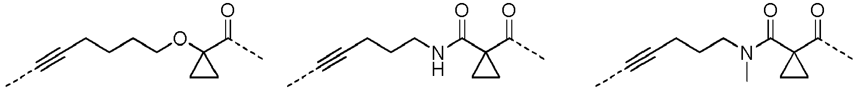 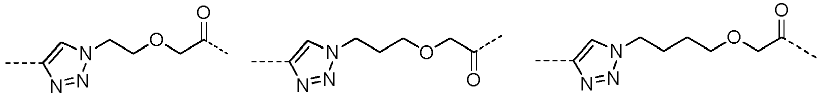 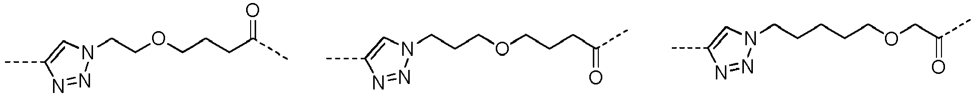 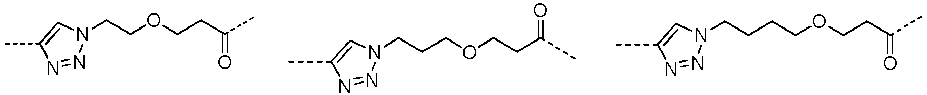 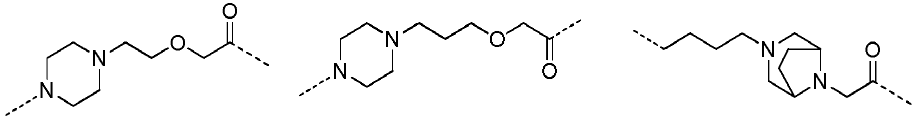 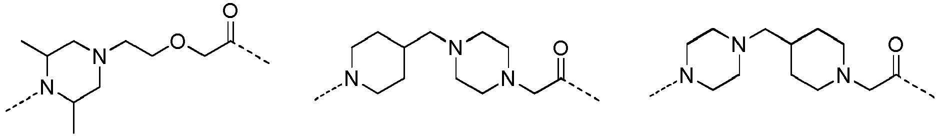 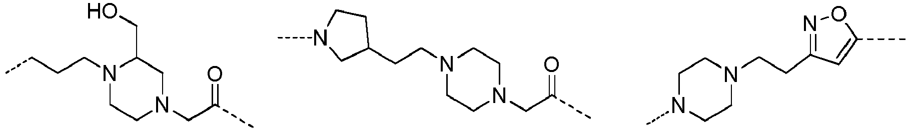 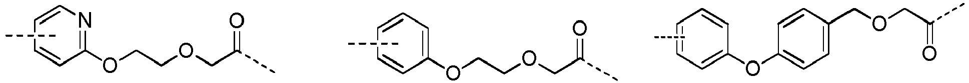 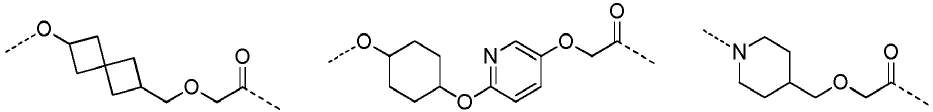 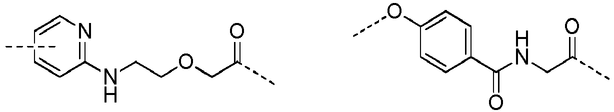 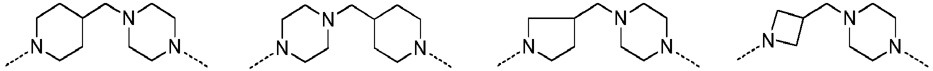 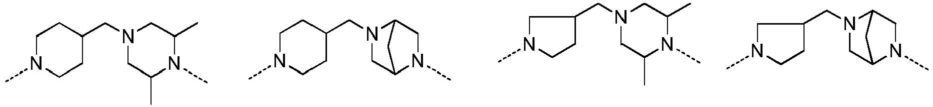 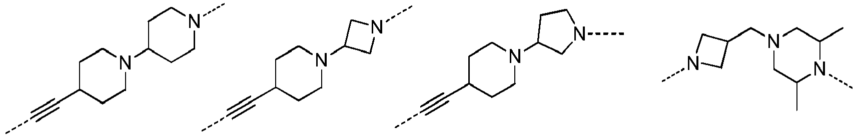 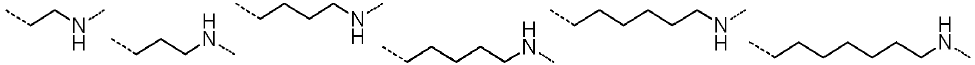 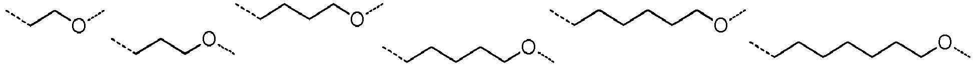 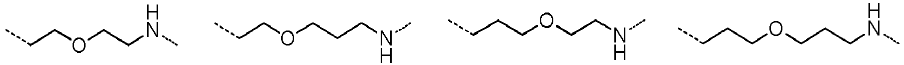 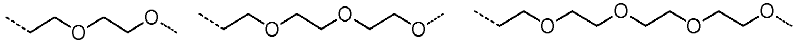 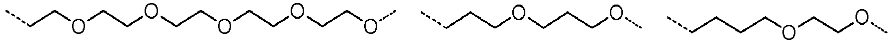 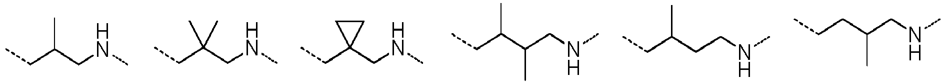 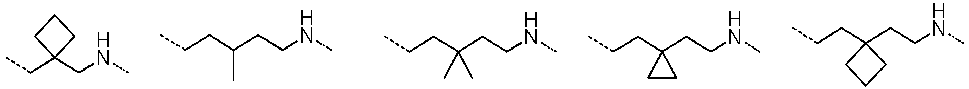 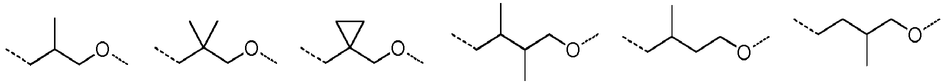 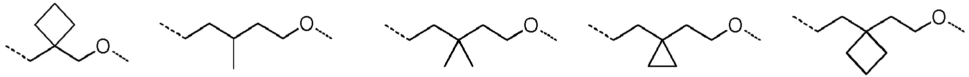 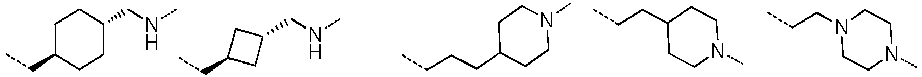 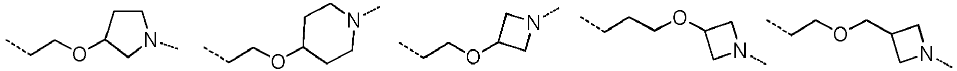 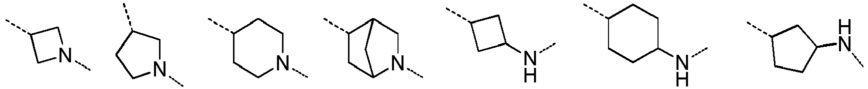 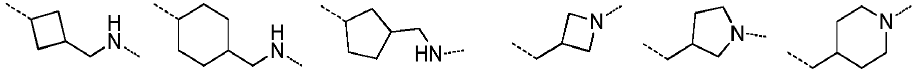 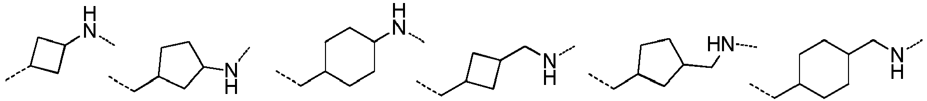 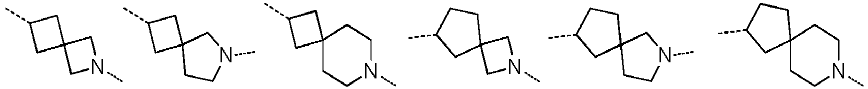 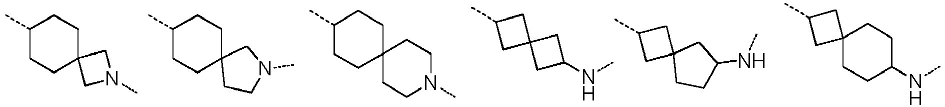 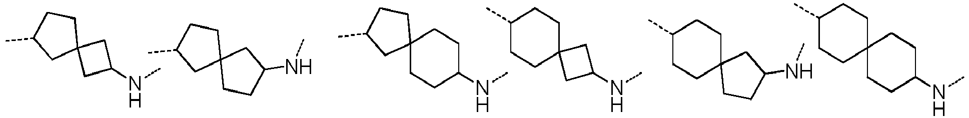 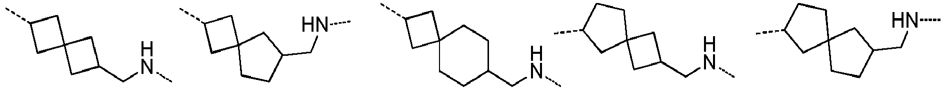 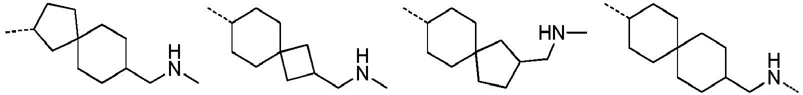 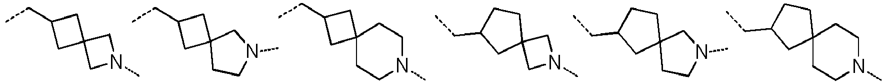 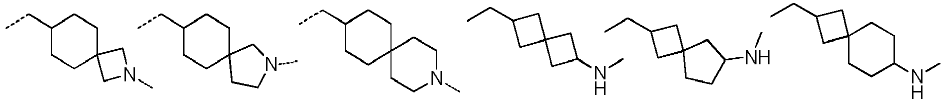 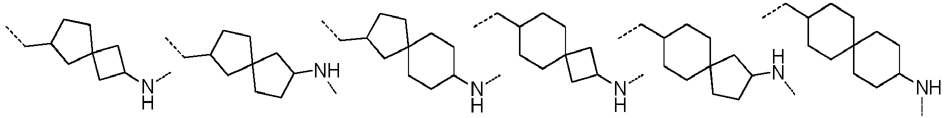 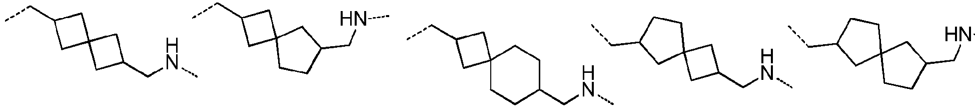 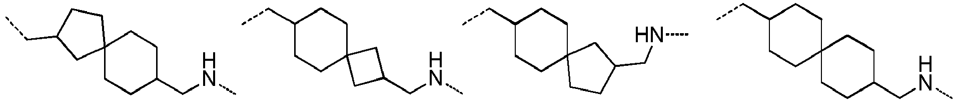 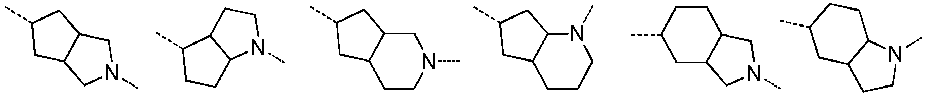 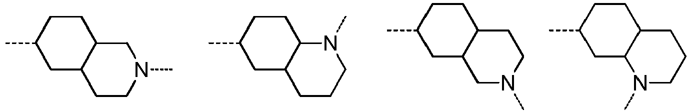 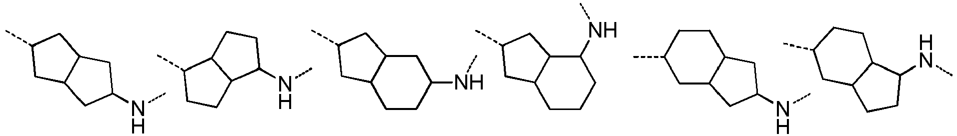 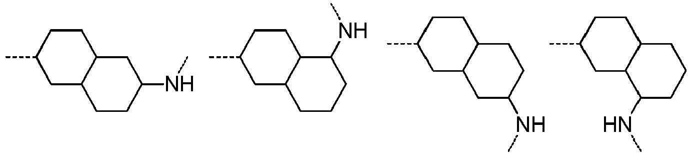 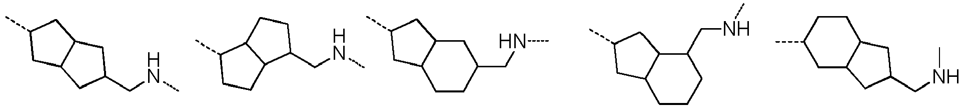 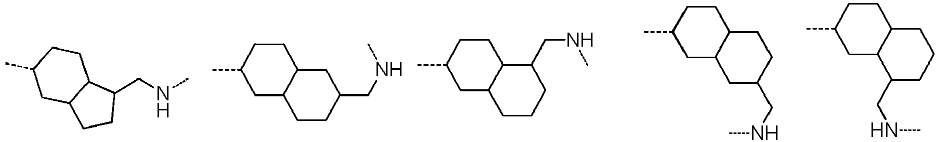 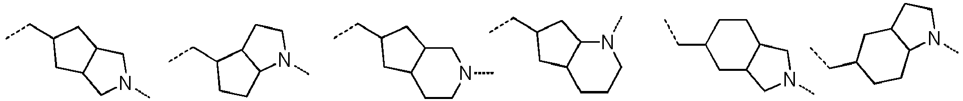 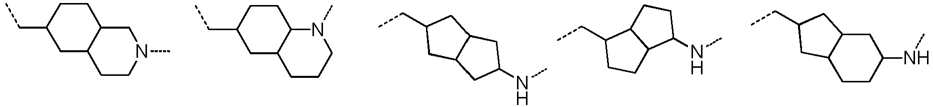 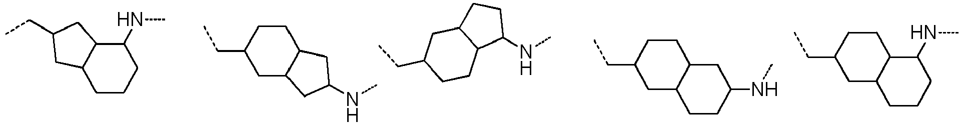 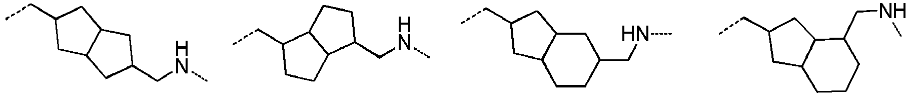 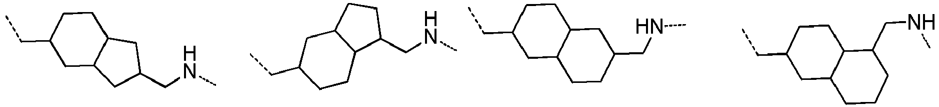 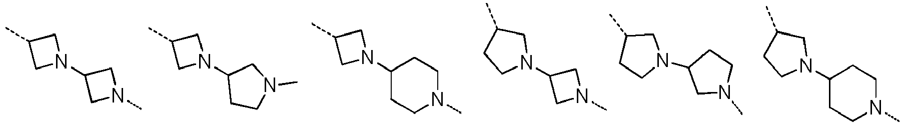 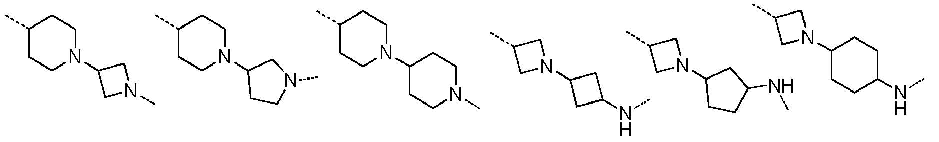 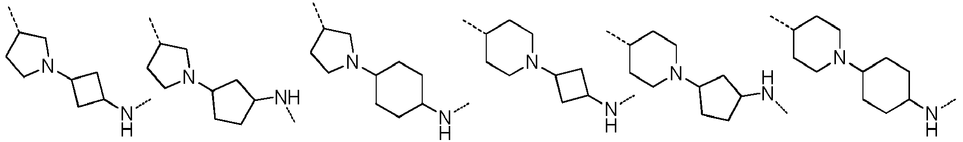 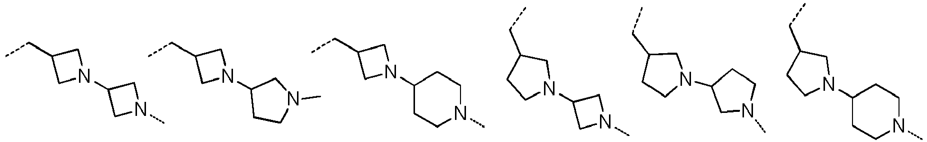 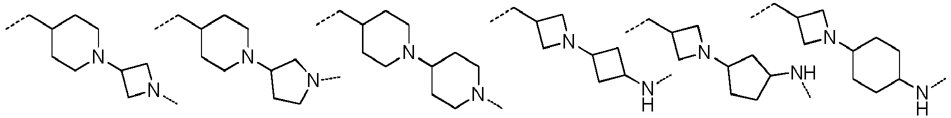 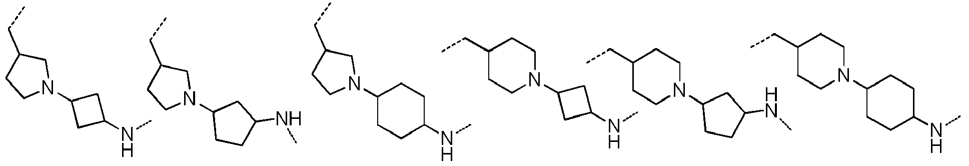 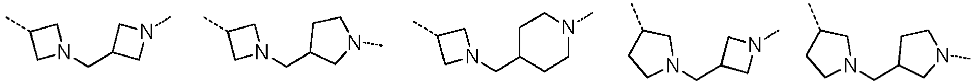 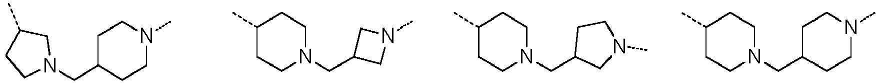 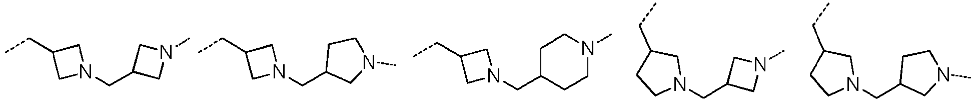 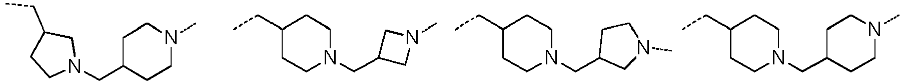 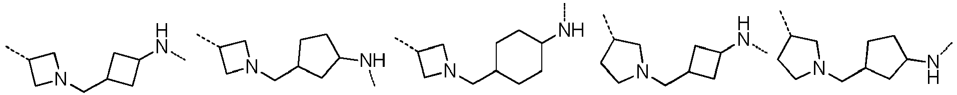 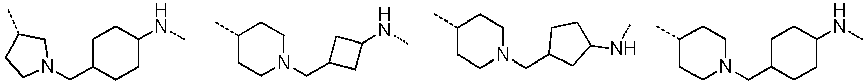 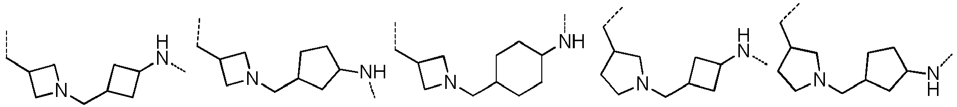 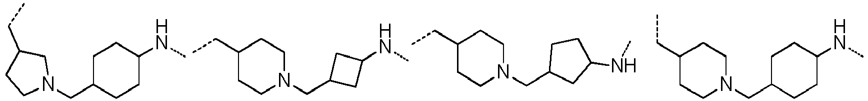 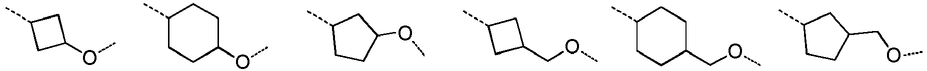 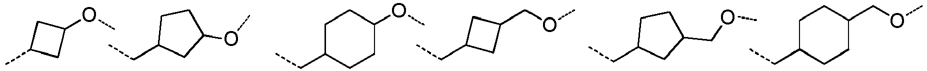 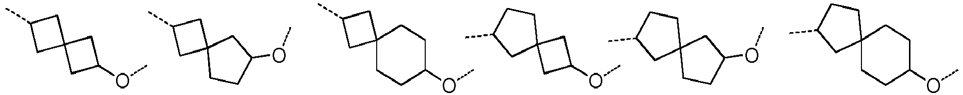 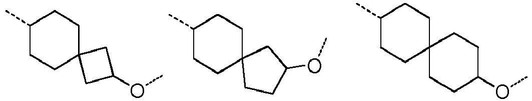 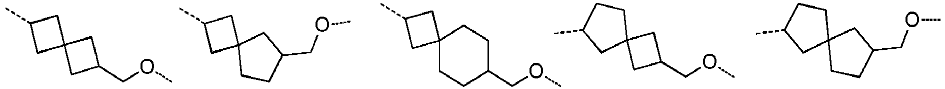 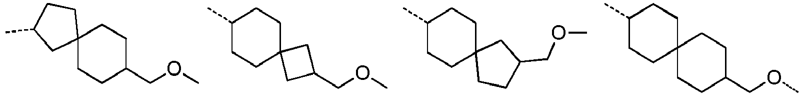 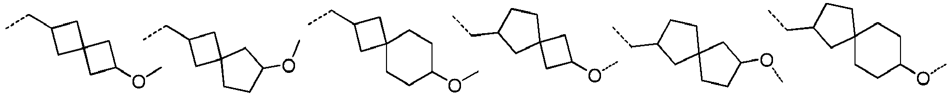 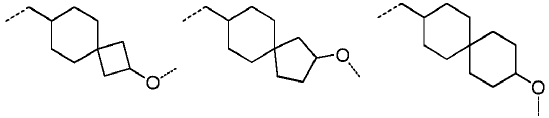 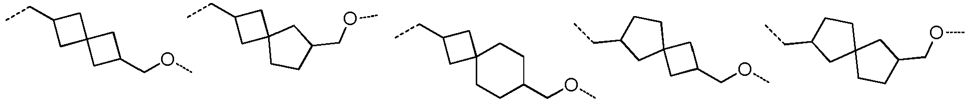 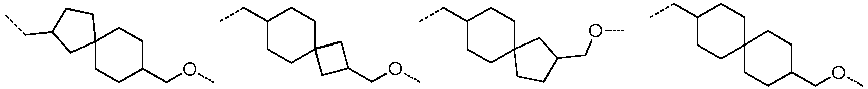 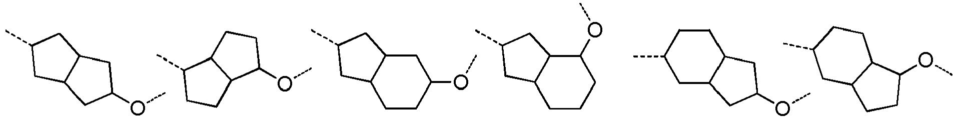 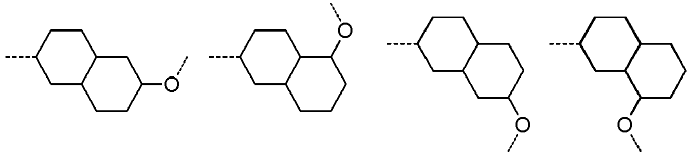 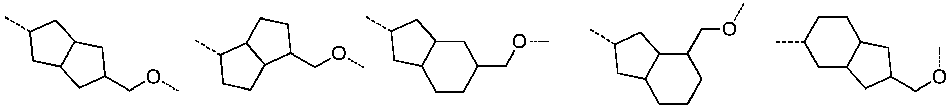 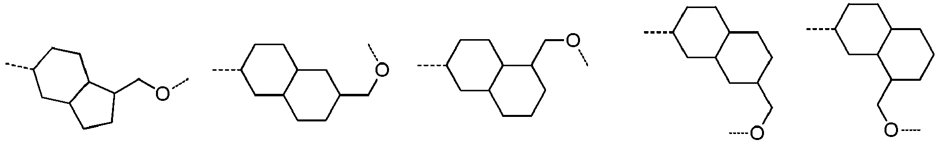 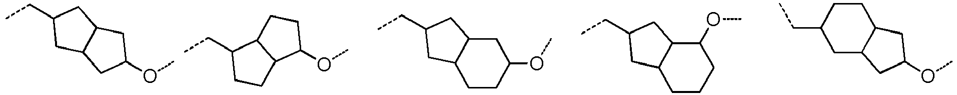 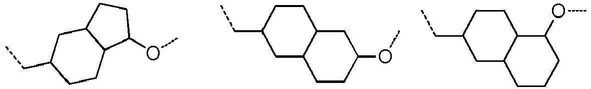 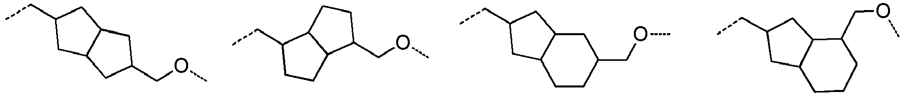 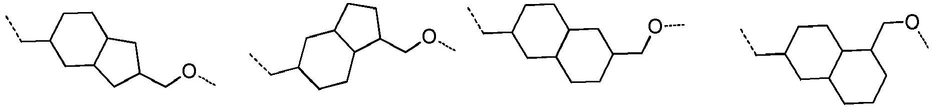 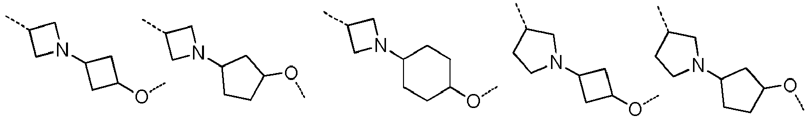 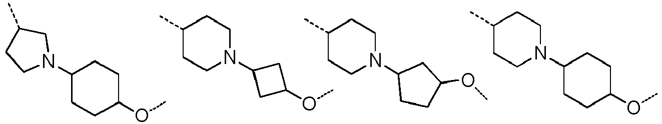 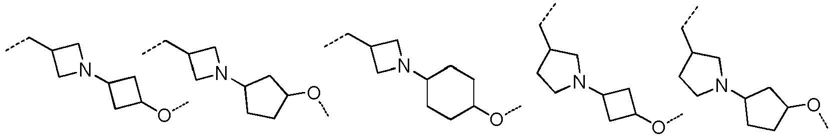 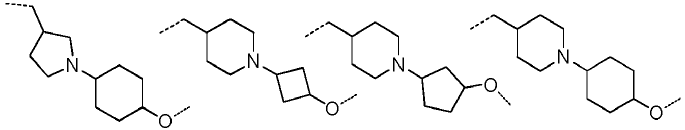 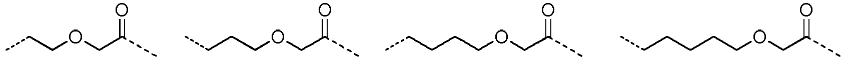 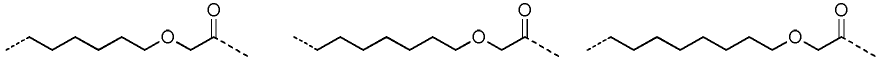 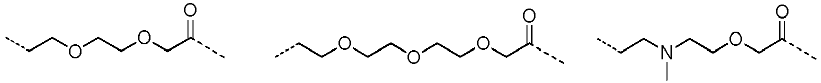 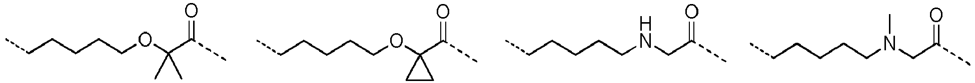 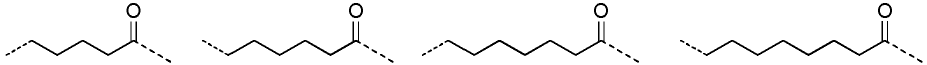 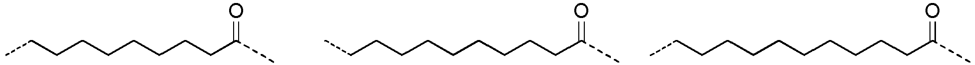 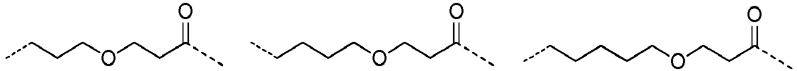 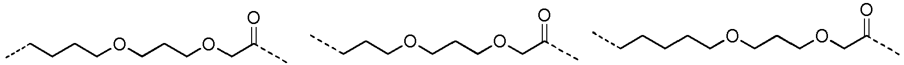 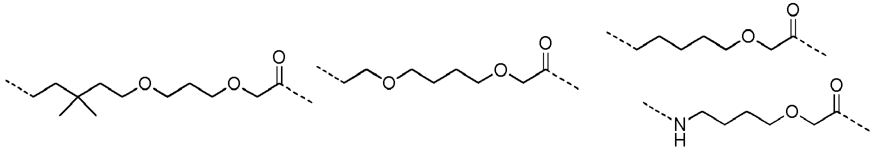 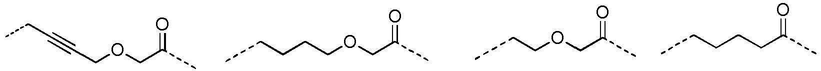 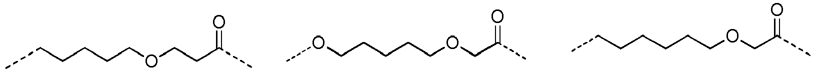 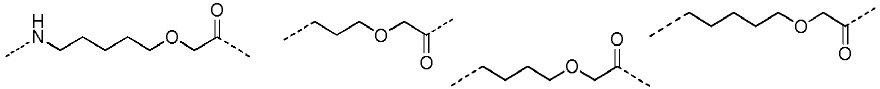 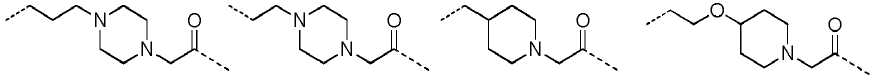 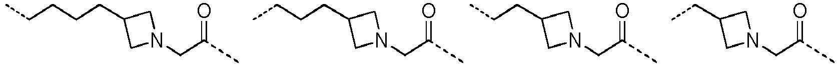 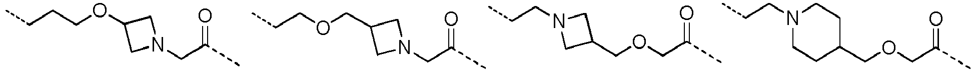 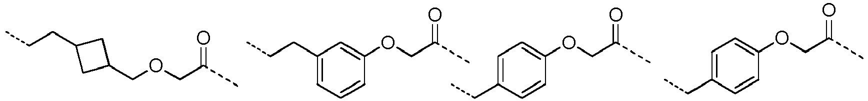 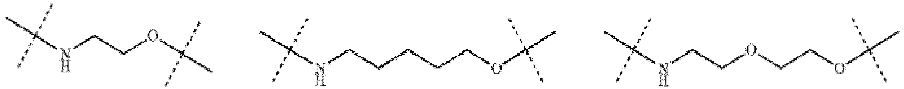 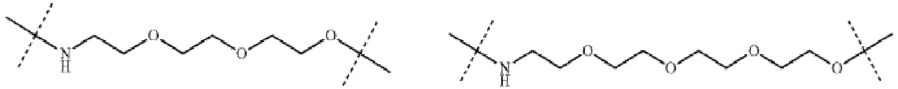 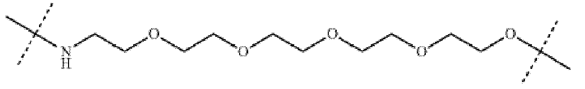 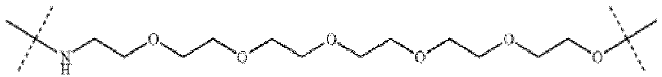 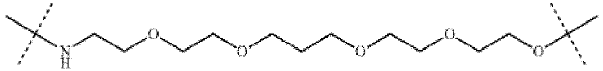 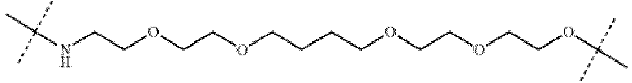 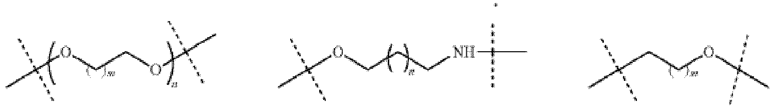 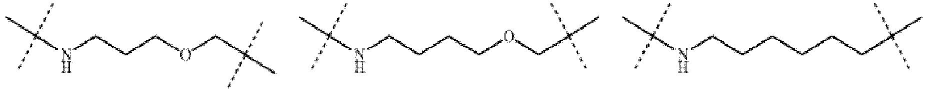 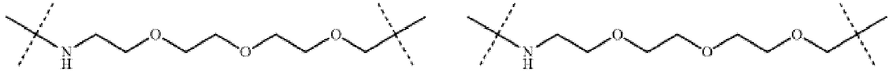 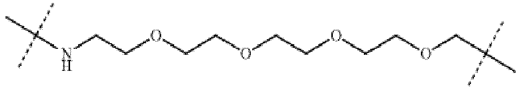 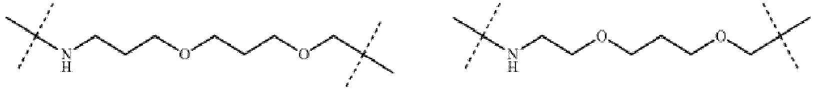 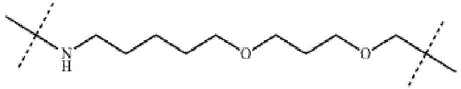 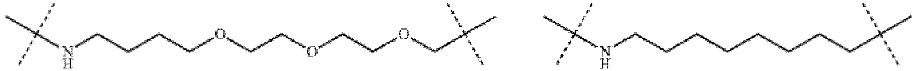 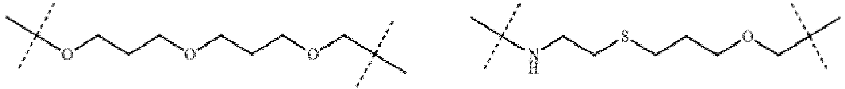 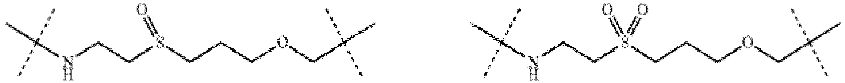 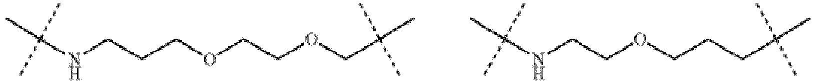 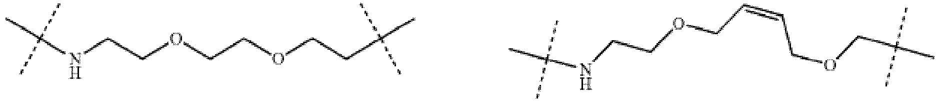 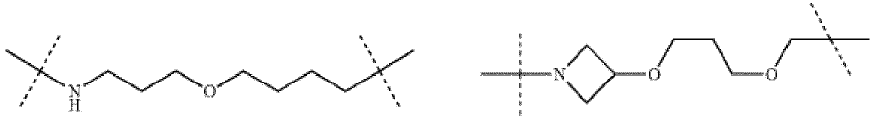 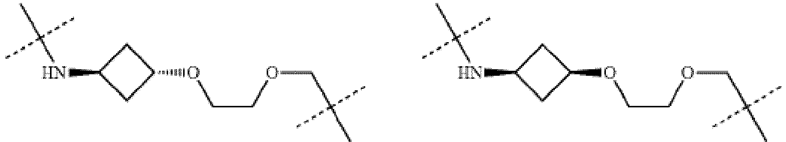 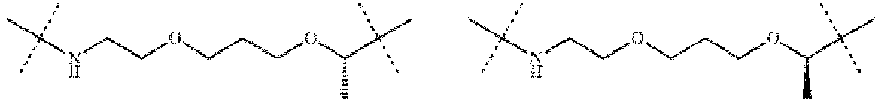 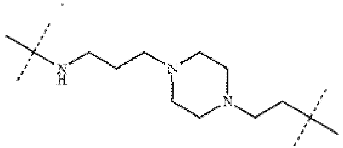 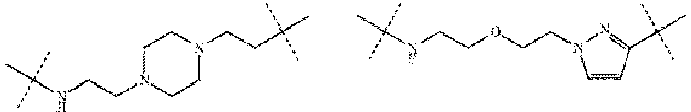 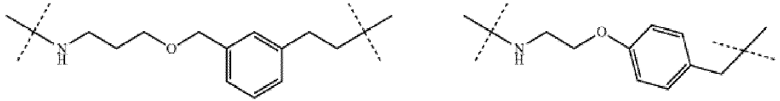 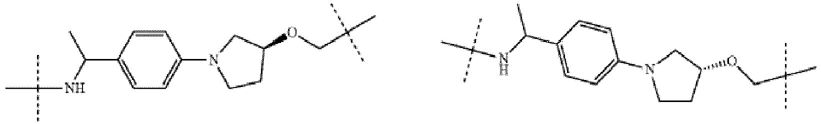 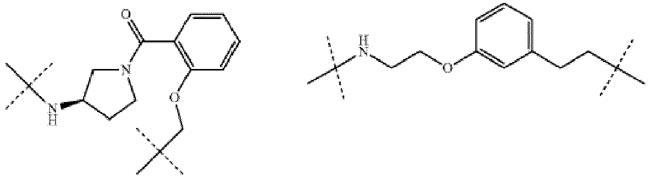 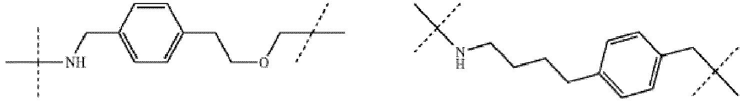 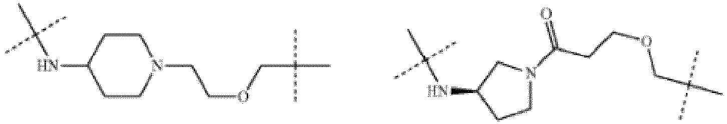 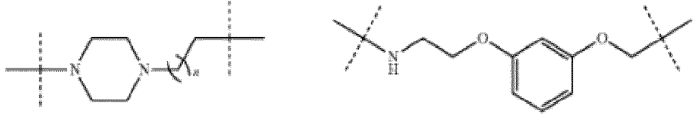 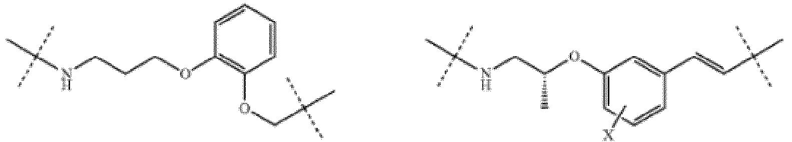 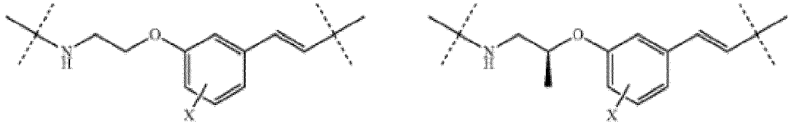 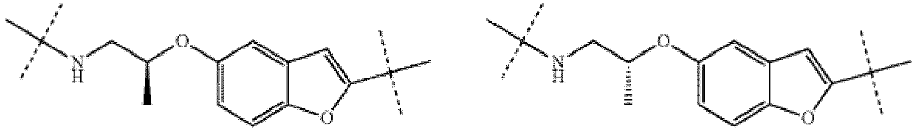 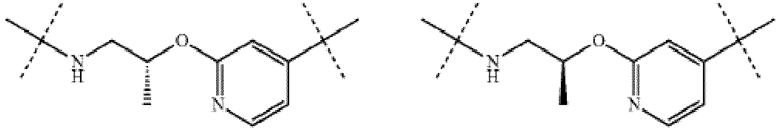 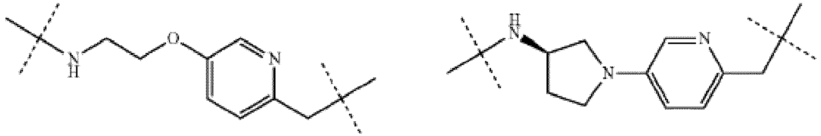 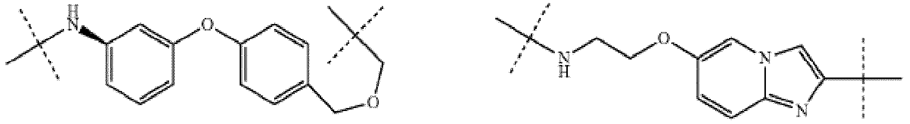 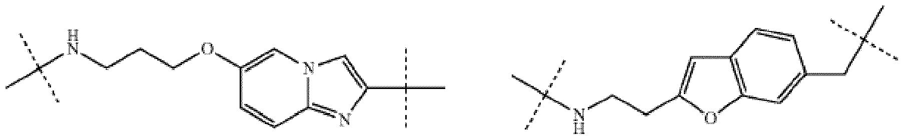 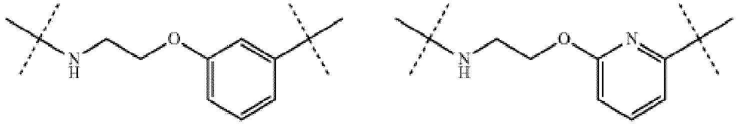 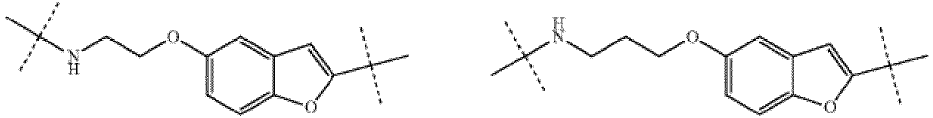 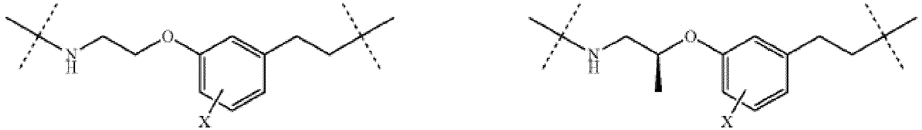 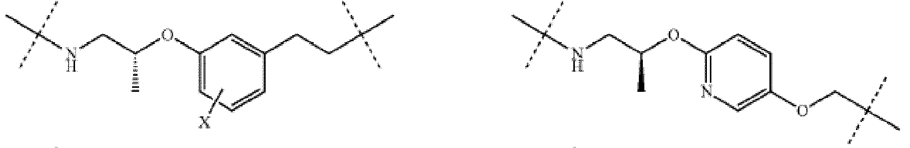 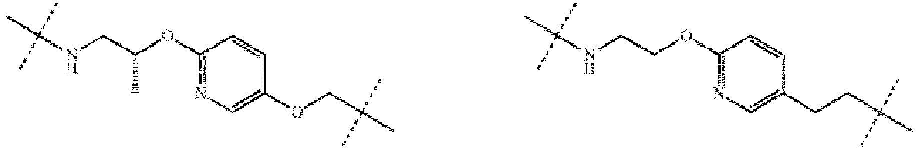 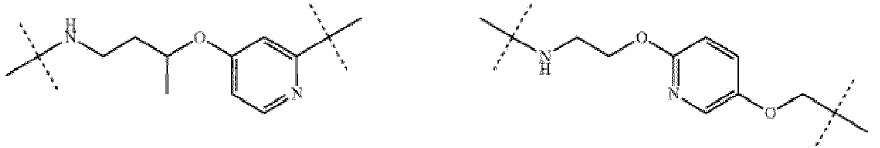 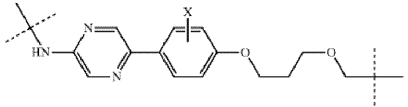 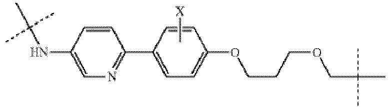 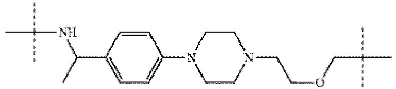 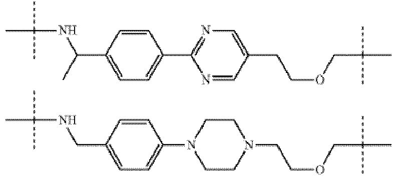 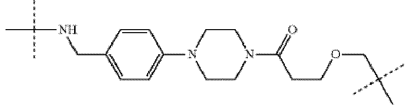 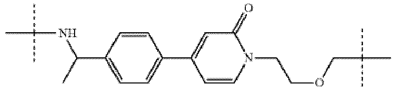 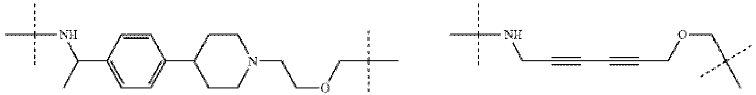 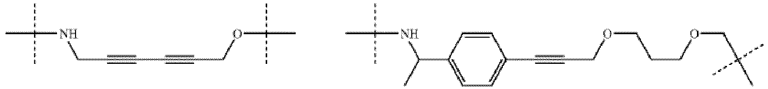 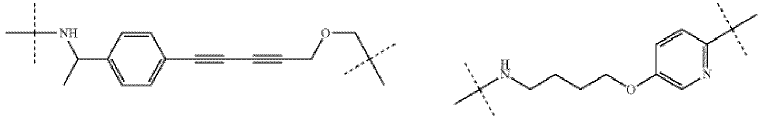 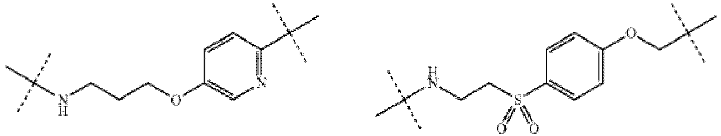 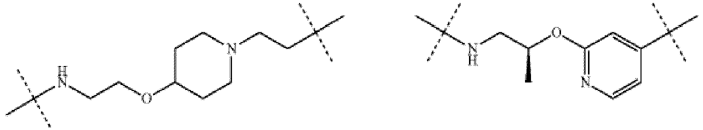 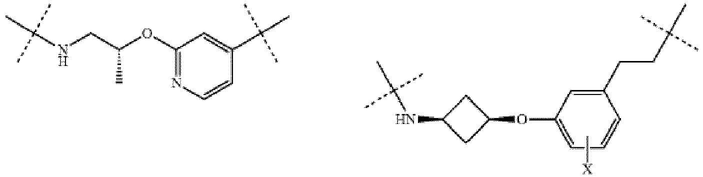 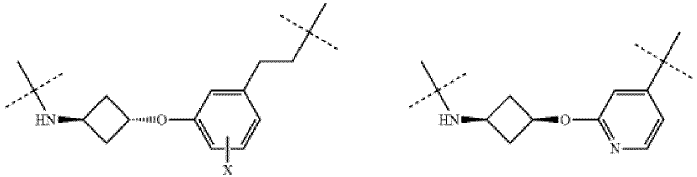 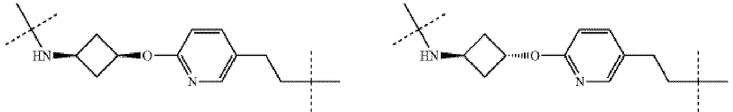 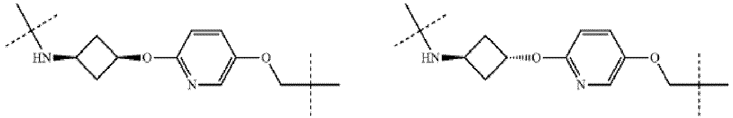 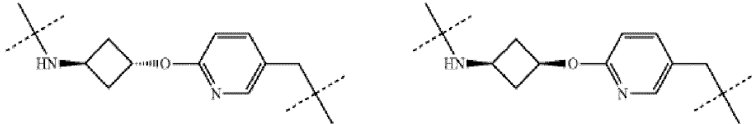 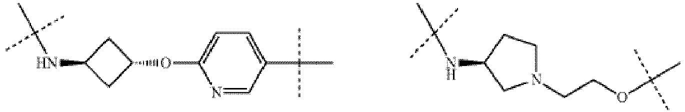 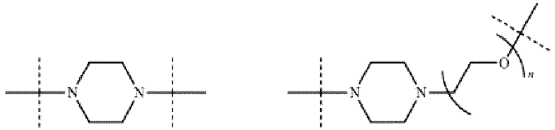 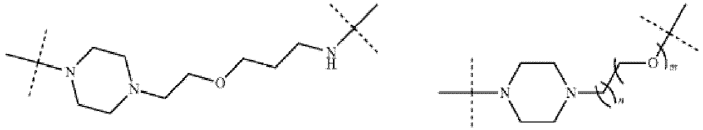 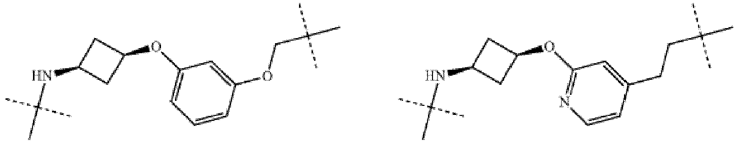 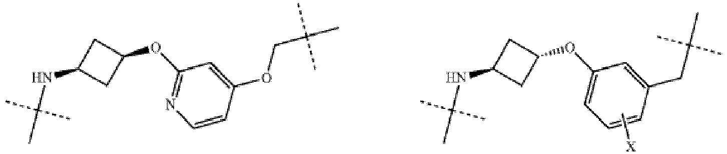 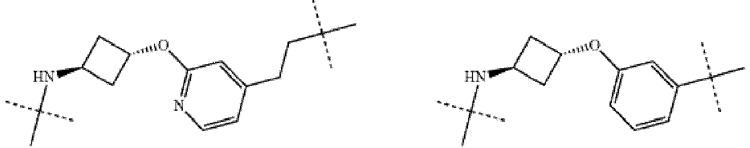 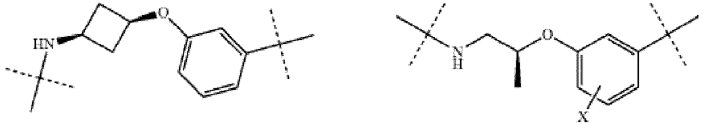 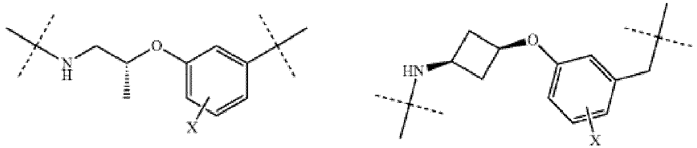 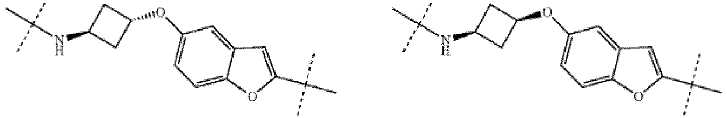 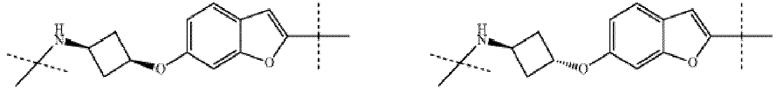 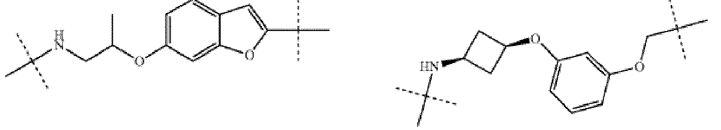 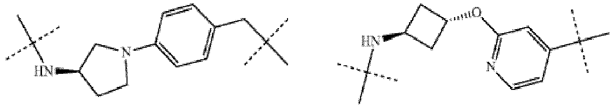 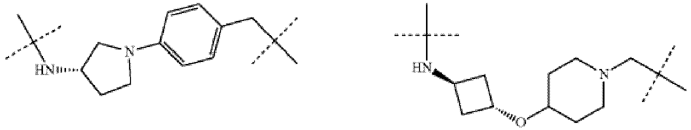 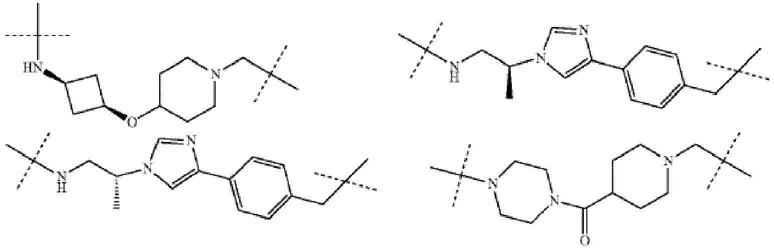 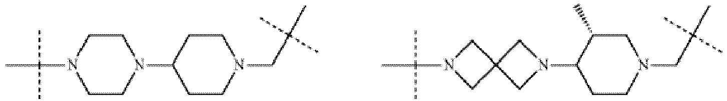 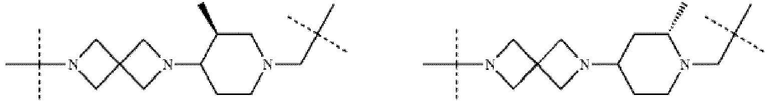 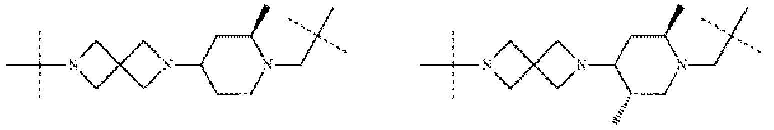 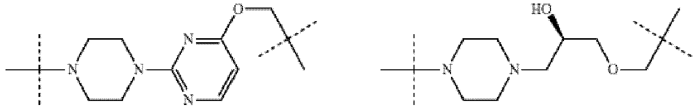 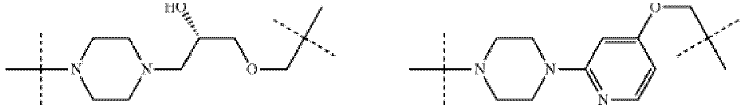 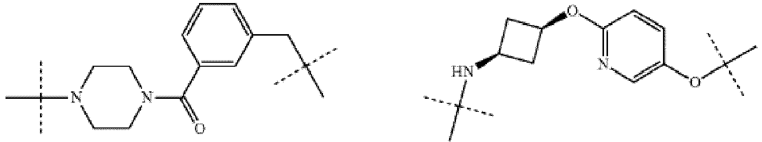 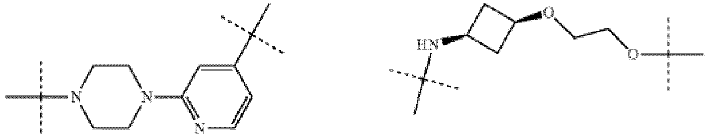 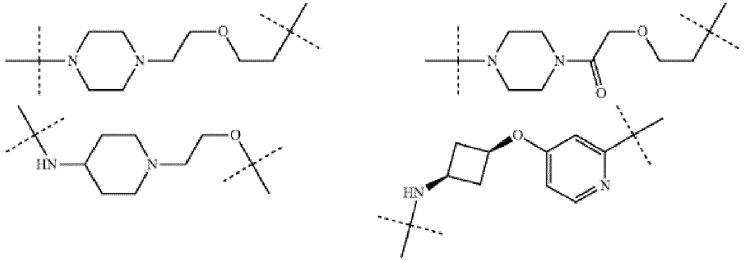 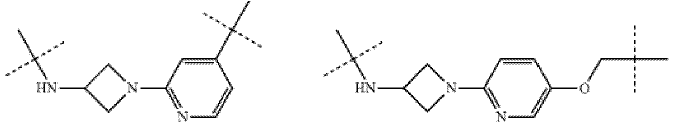 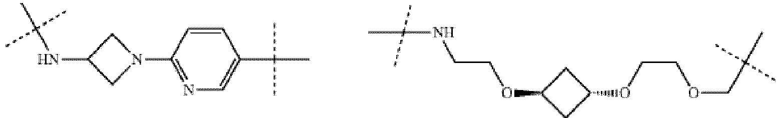 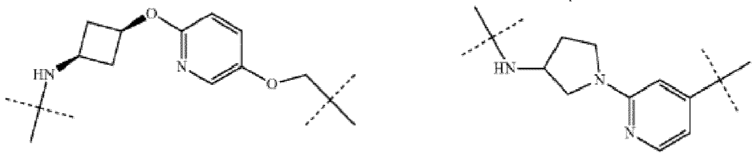 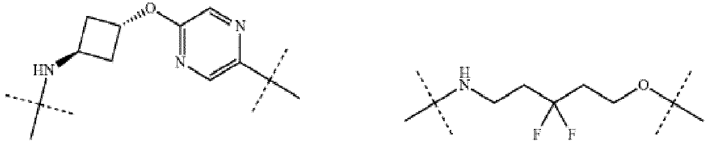 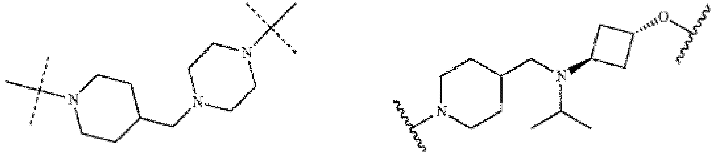 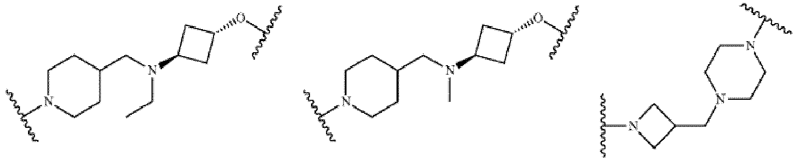 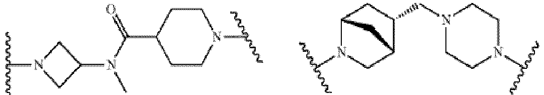 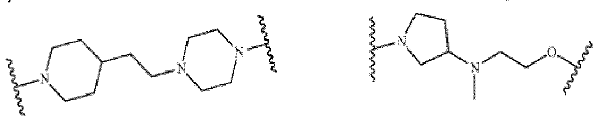 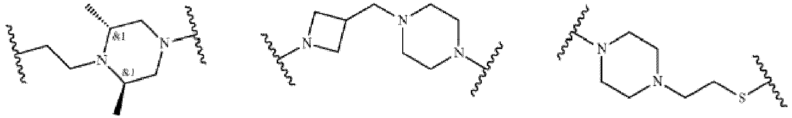 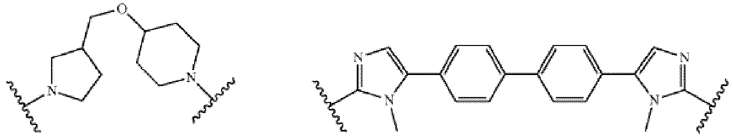 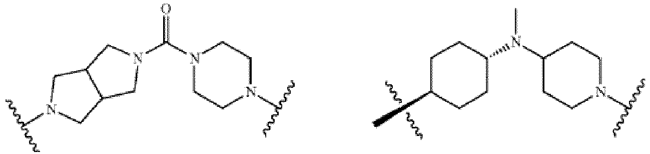 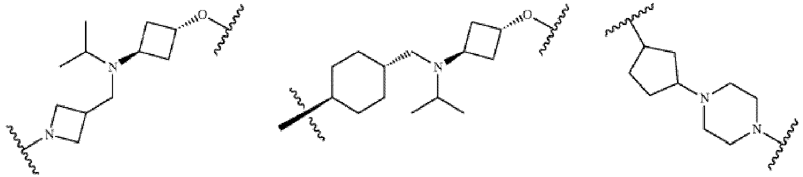 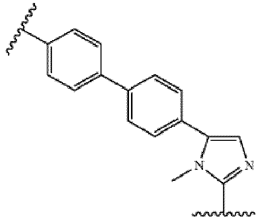 wherein, X is selected from the group consisting of H, deuterium and halogen; each of m and n is an integer of from 0 to 5.

Further, the structure of said compound is selected from the group consisting of:

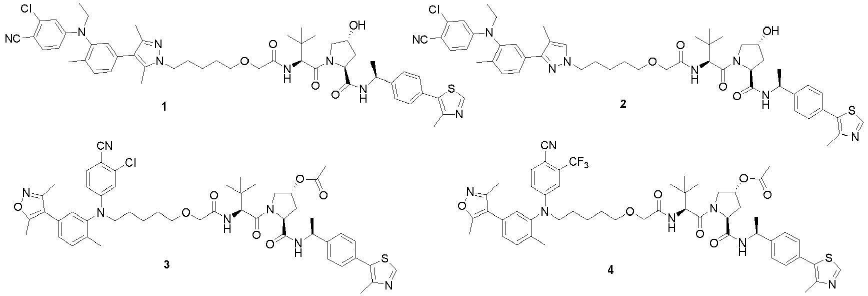

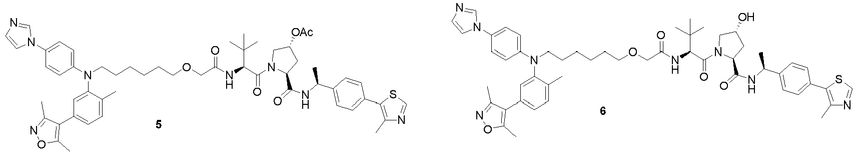

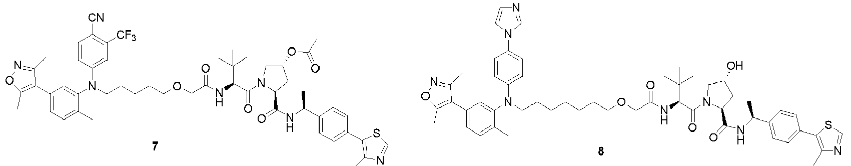

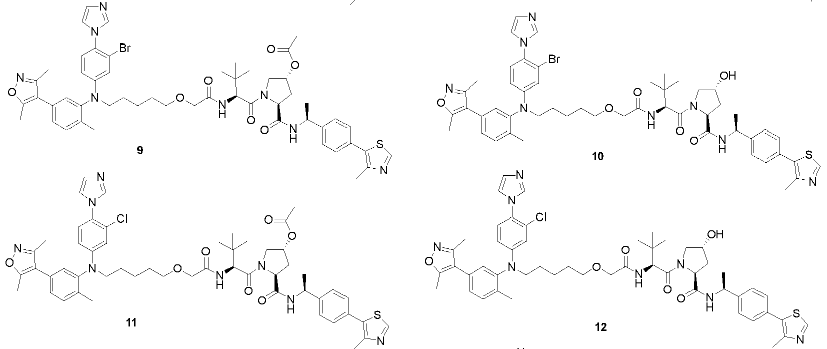

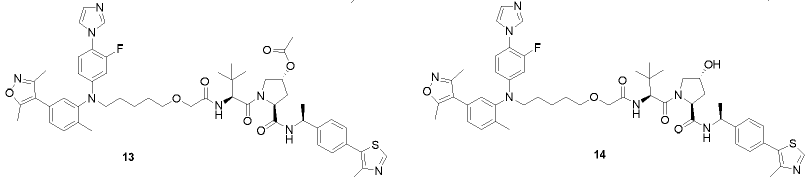

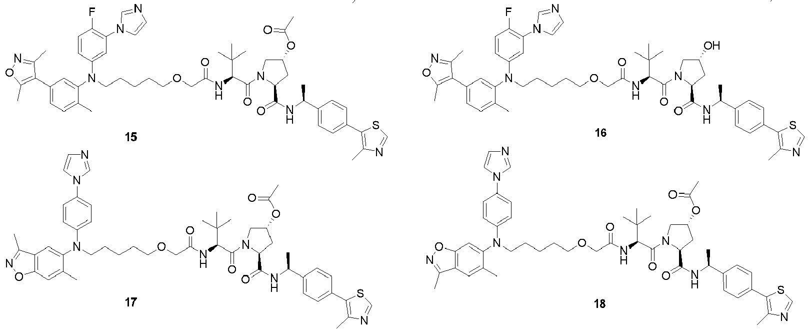

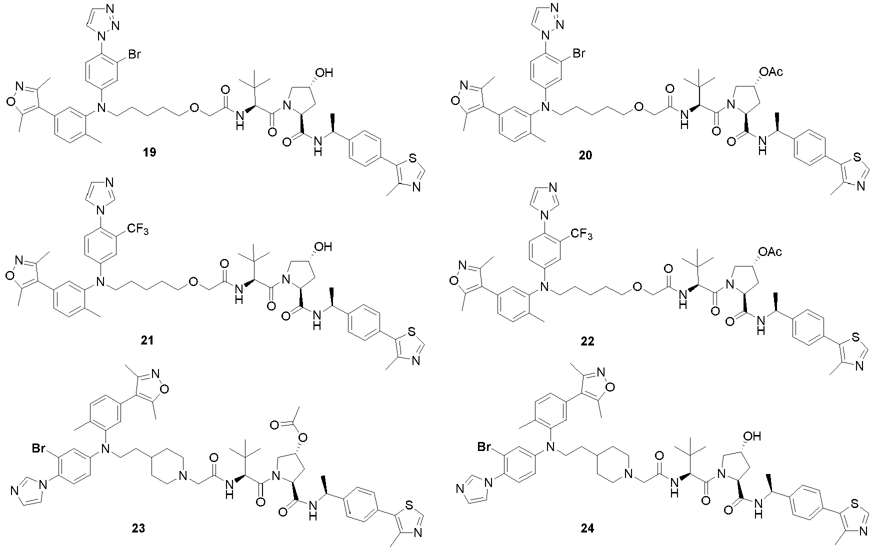

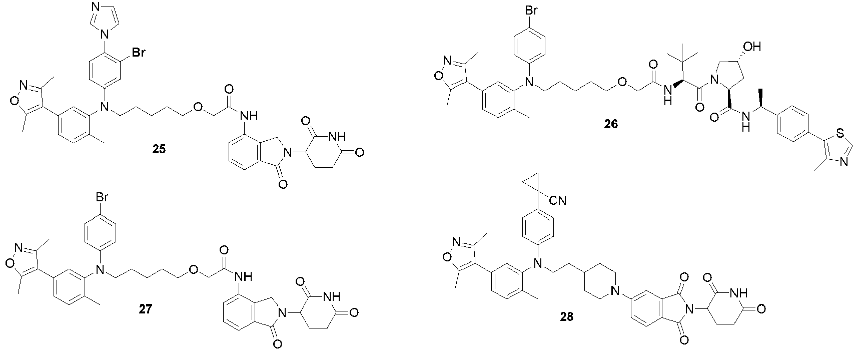

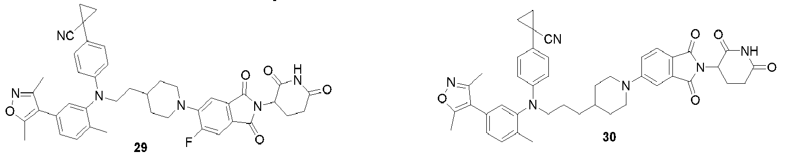

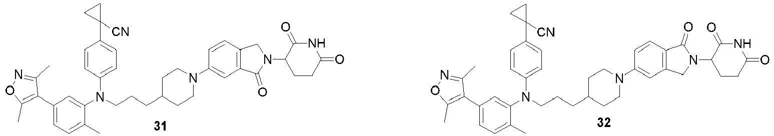

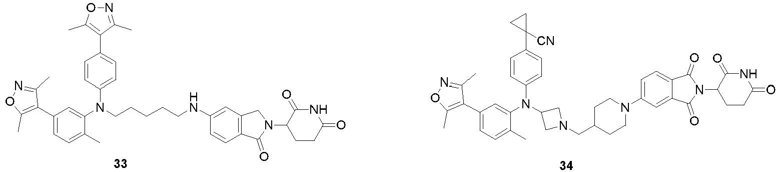

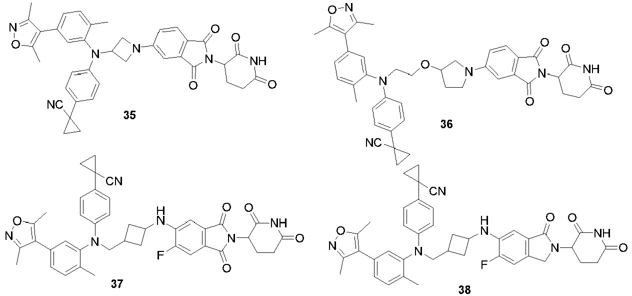

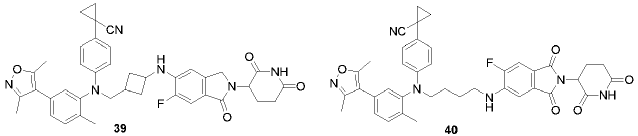

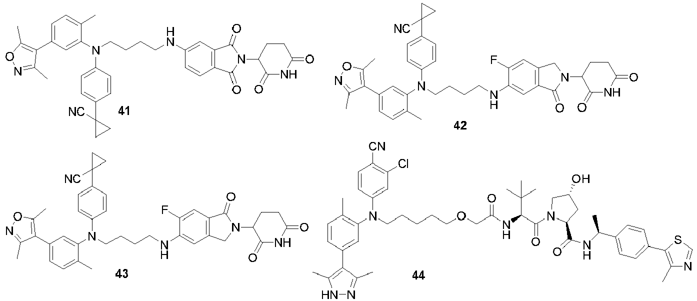

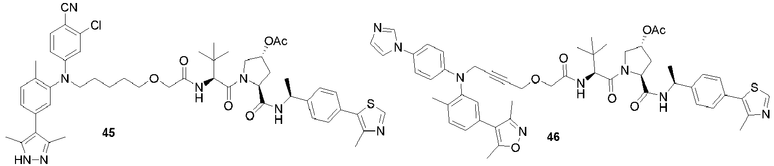

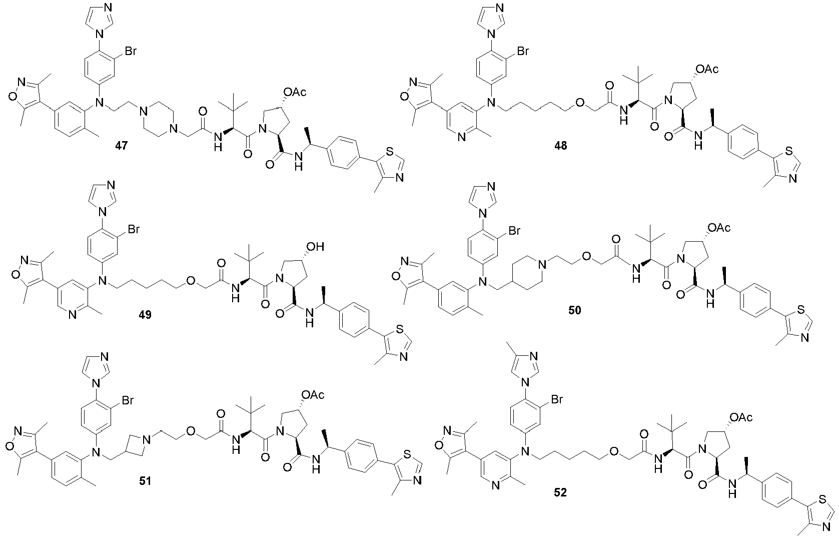

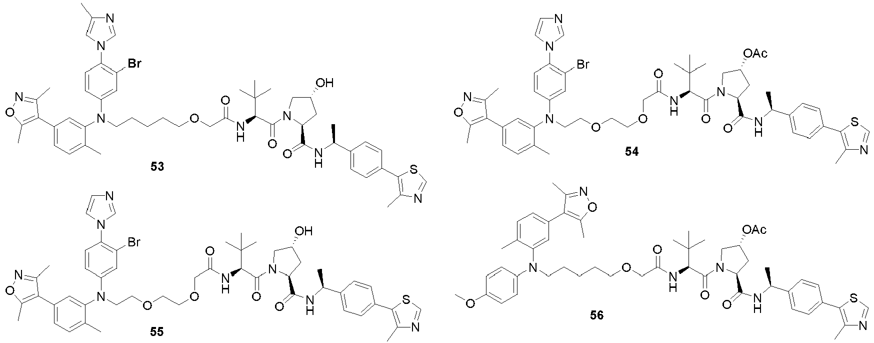

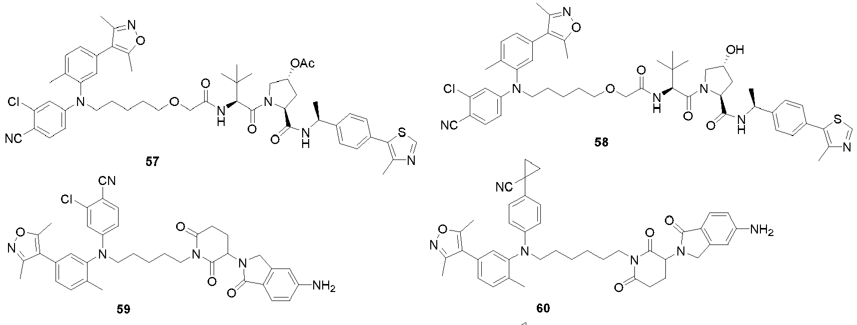

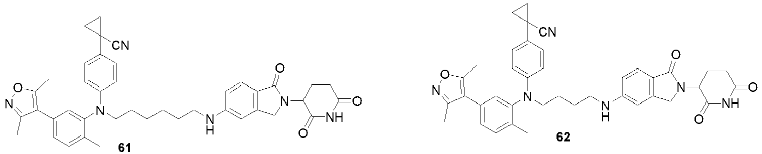

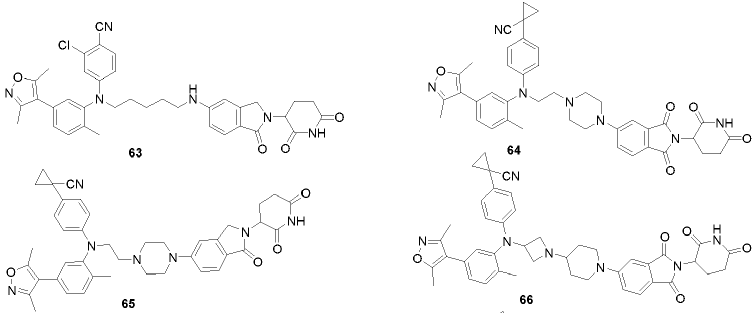

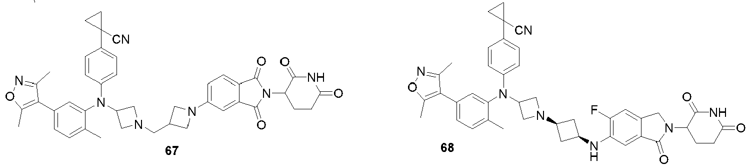

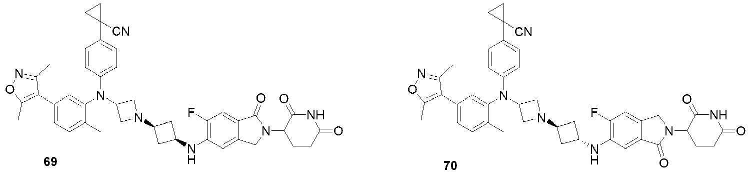

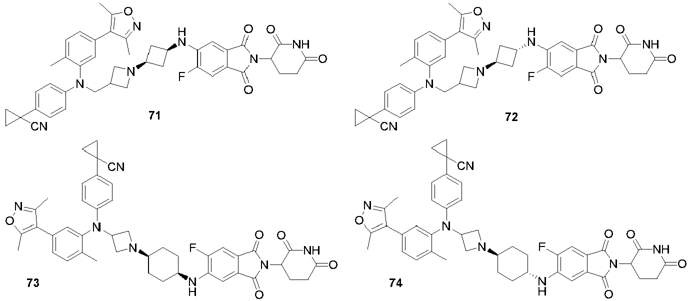

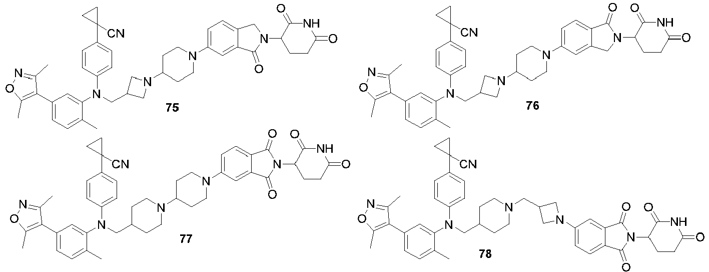

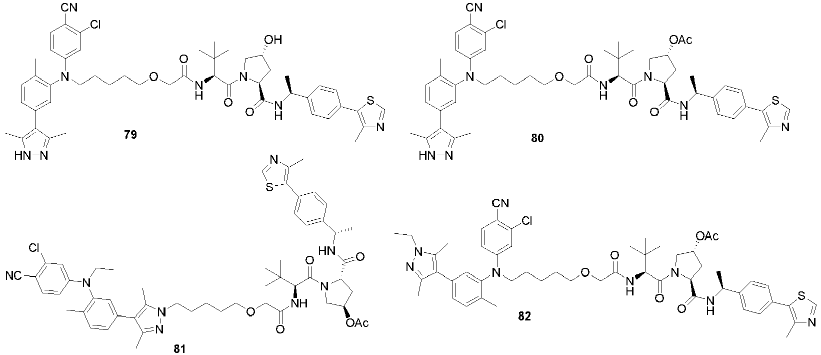

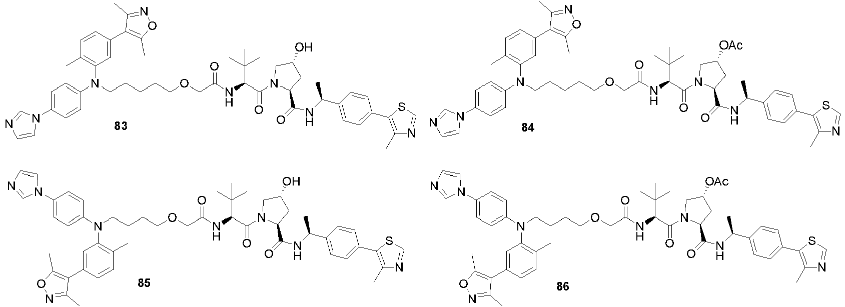

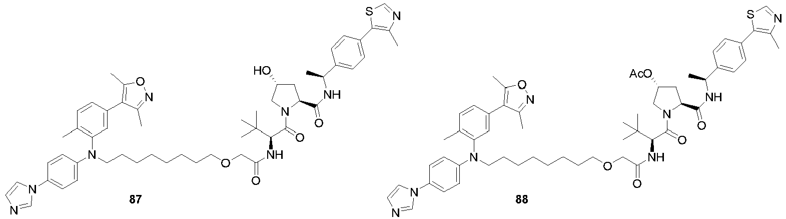

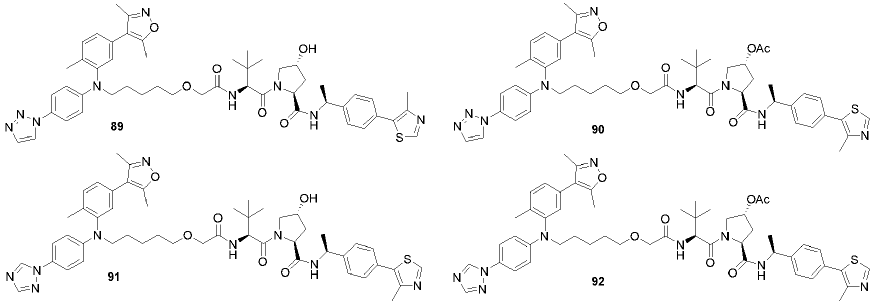

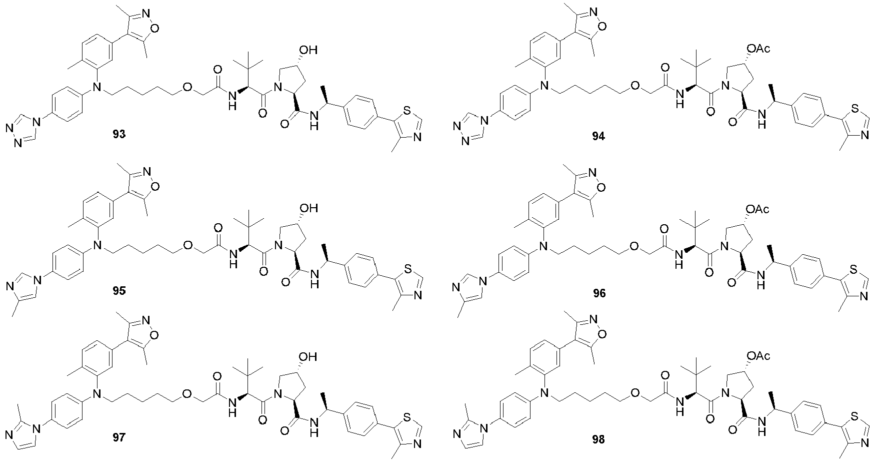

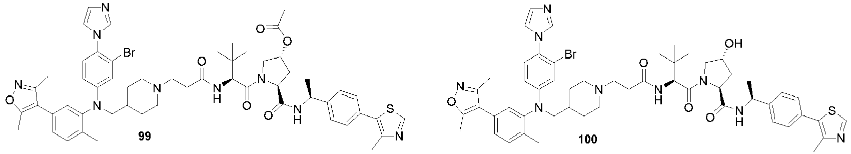

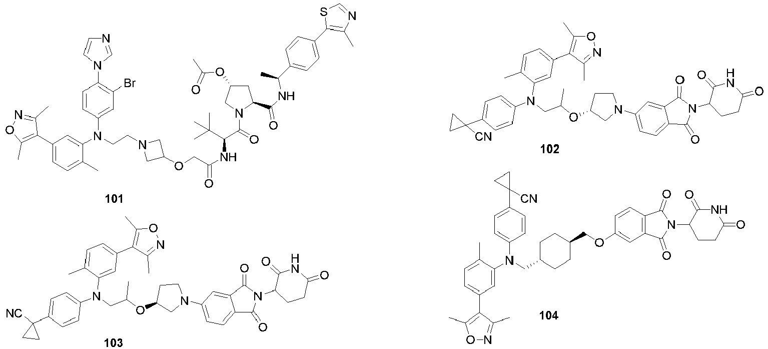

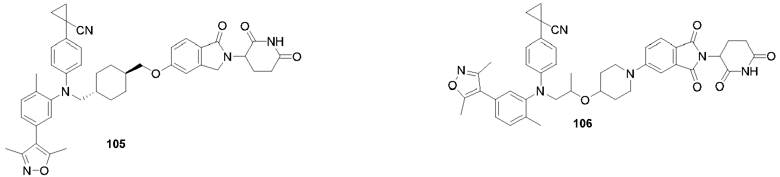

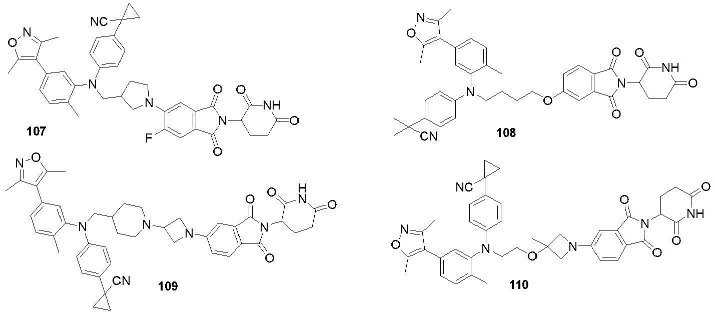

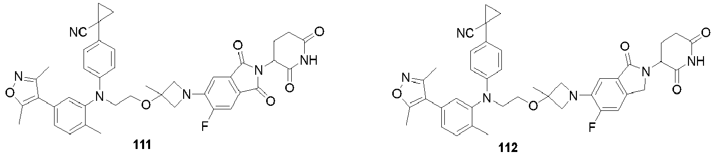

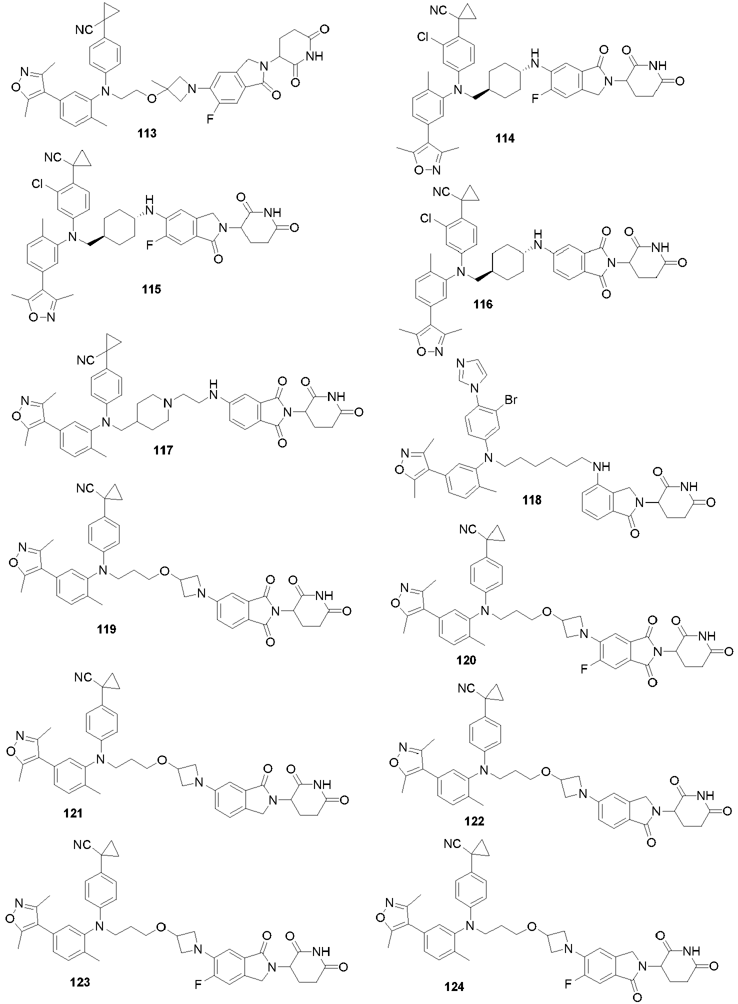

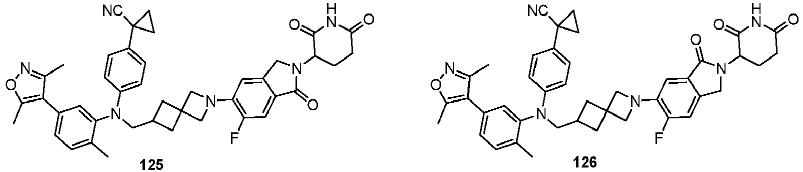

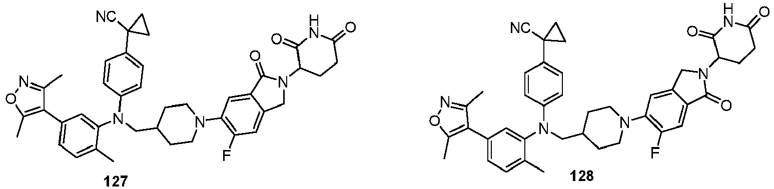

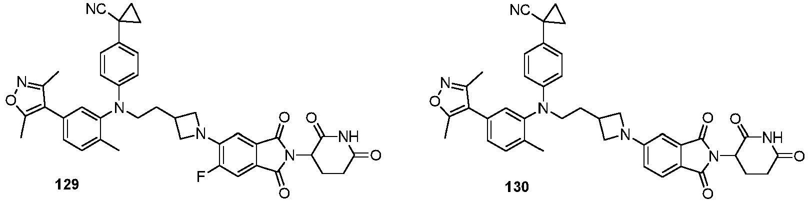

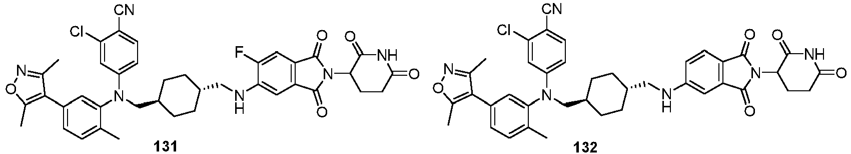

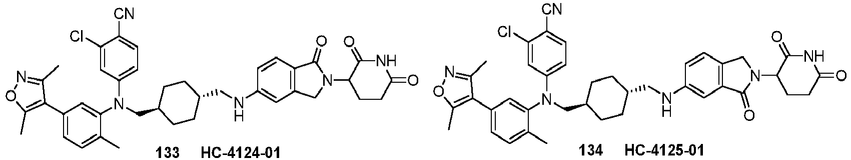

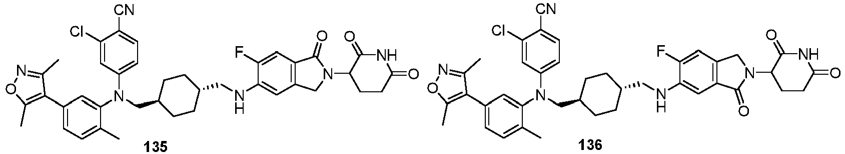

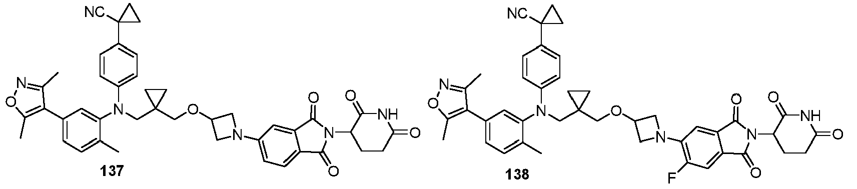

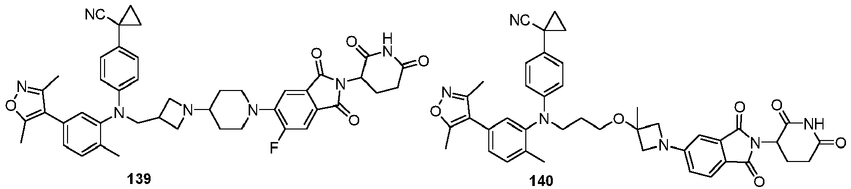

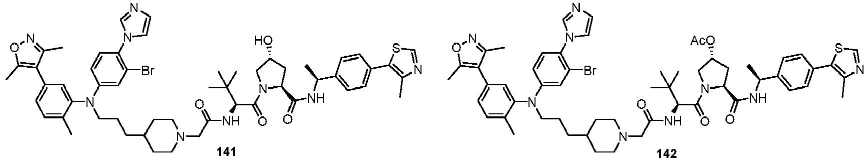

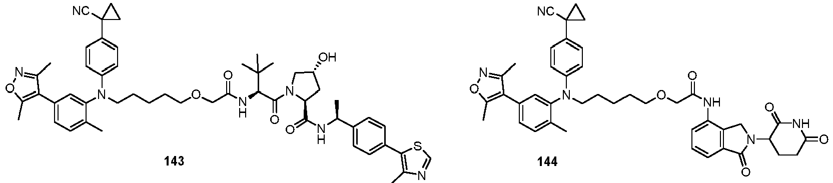

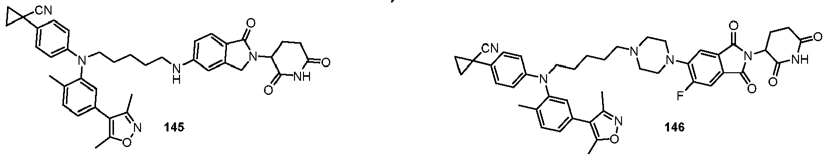

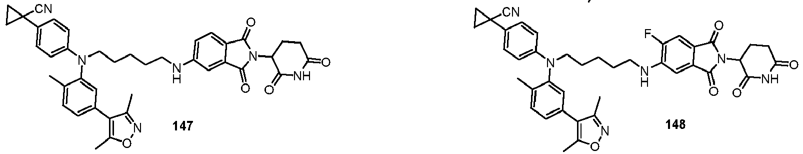

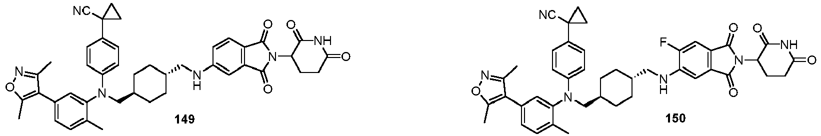

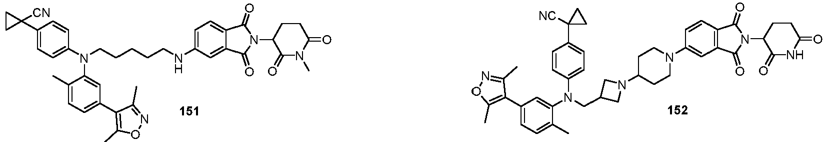

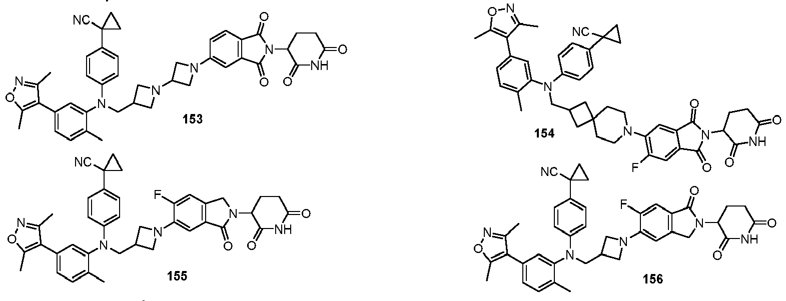

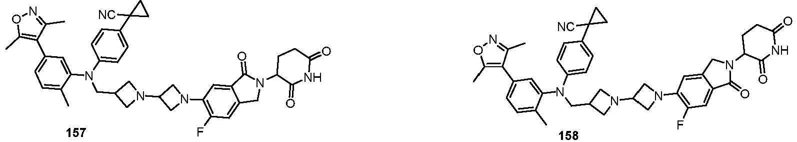

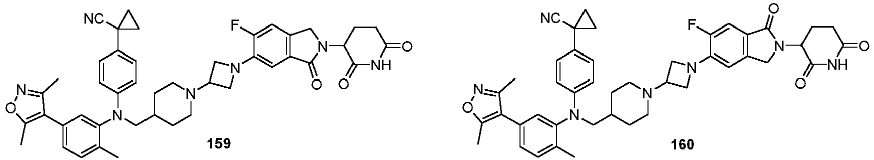

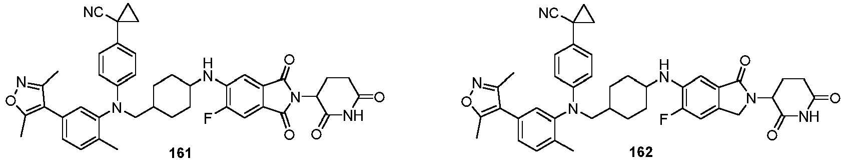

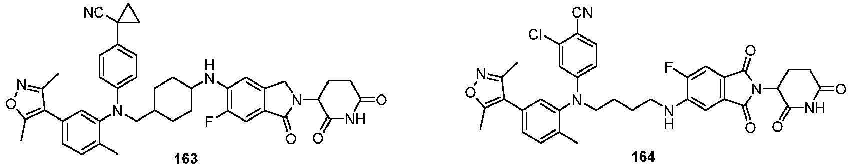

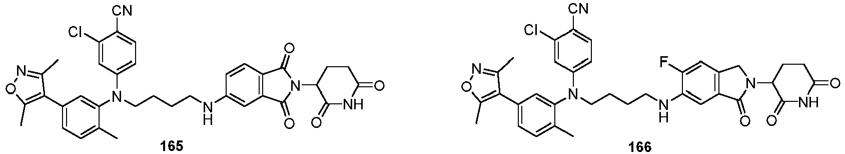

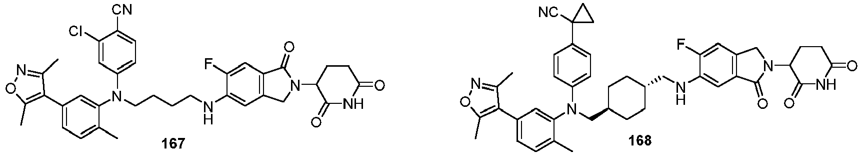

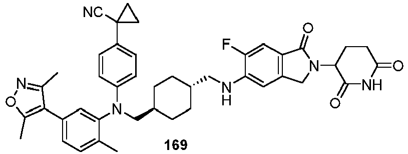

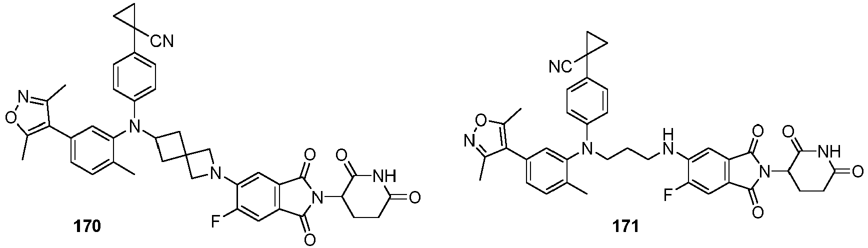

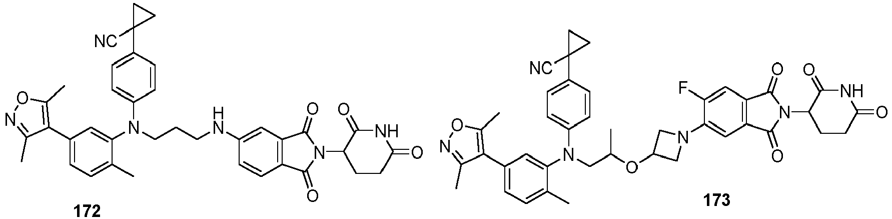

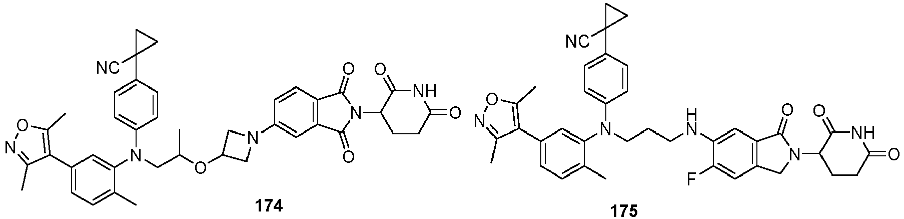

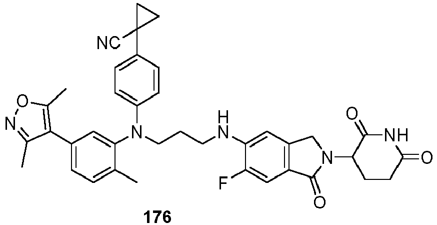

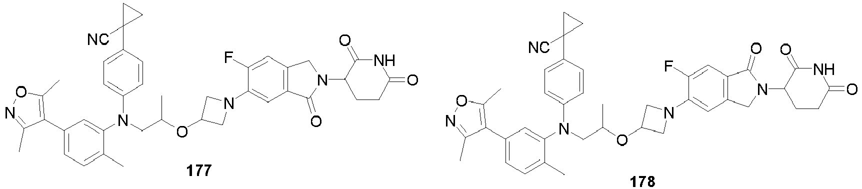

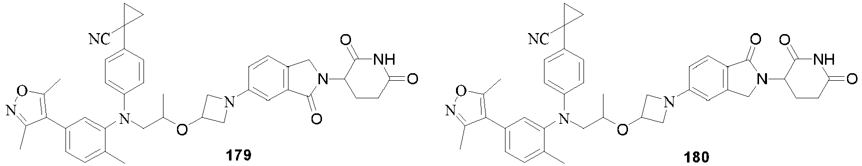

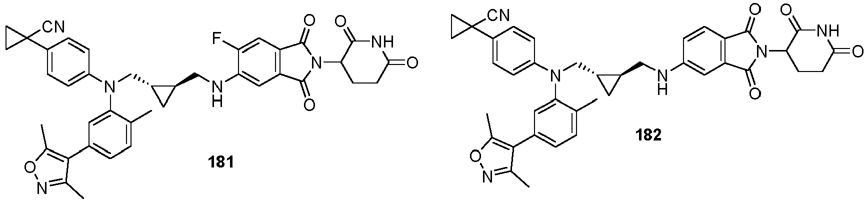

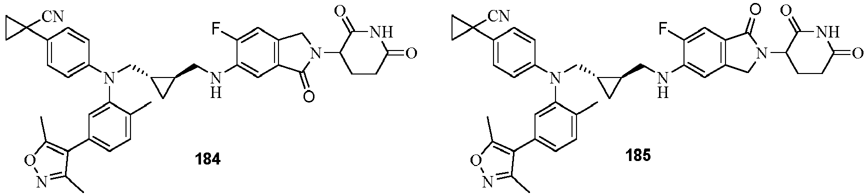

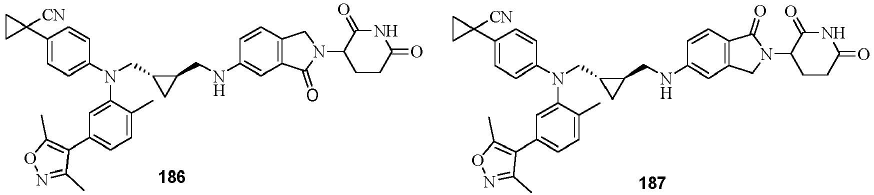

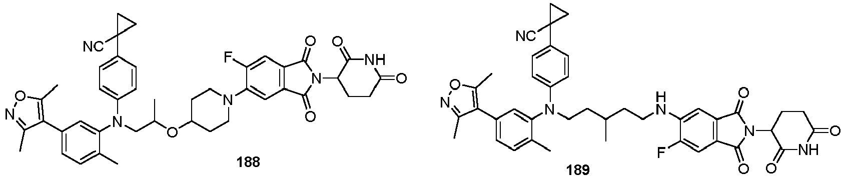

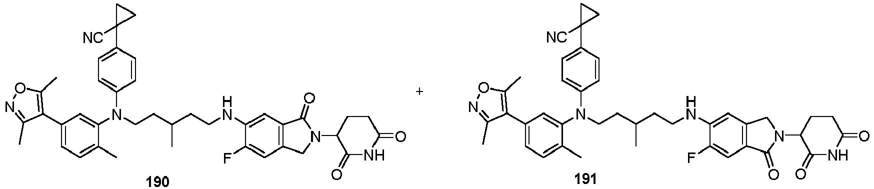

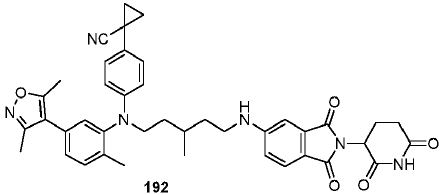

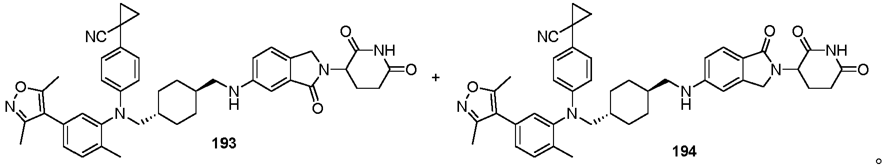

The present invention further provides the use of a compound mentioned above, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a combination thereof, or a metabolite thereof, or a metabolic precursor thereof, or an isotopic compound thereof in the preparation of chimeras targeting the protein degradation of androgen receptors and/or BET.

Further, the proteolytic targeting chimera can specifically recognize/bind AR and/or BET. Further, the proteolytic targeting chimera can degrade AR and/or BET.

Further, the proteolytic targeting chimera is a drug for the treatment of the diseases related to AR and/or BET.

Further, said disease is selected from the group consisting of prostate cancer, breast cancer and Kennedy's disease.

Experimental results show that the compound provided in the present invention can target and degrade both AR and BRD4, and down-regulate the expression of AR and BRD4 proteins; the compound can inhibit the proliferation of a variety of prostate cancer cells; the compound can inhibit the proliferation of a prostate cancer cell line LNCaP/AR, which overexpresses the AR, and can achieve a good inhibition effect on a prostate cancer cell line 22RV1, which is resistant to a marketed prostate cancer drug (enzalutamide); the compound also shows good metabolic stability, and has a good application prospect in the preparation of an AR and/or BET proteolytic targeting chimera, and a drug for the treatment of related diseases regulated by the AR and BET.

For the definition of the term used in the present invention: unless otherwise specified, the initial definition provided for the group or the term herein is applicable to those in the whole specification; for terms not specifically defined herein, according to the disclosure content and the context, the term should have the meaning commonly given by those skilled in the field.

In the present invention, 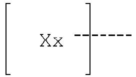 represents the group obtained by removing any hydrogen from Xx molecule in the brackets, for example, the formula I-A represents the remained group after any hydrogen was removed from the molecule

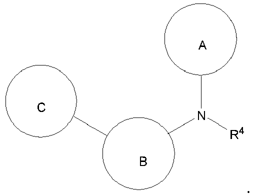

In the present invention, "recognition/combination" means recognition and combination.

In the present invention, "substitution" means that one, two or more hydrogens in a molecule are substituted by other different atoms or molecules, including one, two or more substitutions on the same or different atoms in the molecule.

In the present invention, the minimum and the maximum for the content of carbon atoms in hydrocarbon groups are represented by prefixes, for example, C₁-C₆ alkyl or C₁₋₆ alkyl means C₁, C₂, C₃, C₄, C₅, and C₆ alkyl, that is, any straight or branched alkyl containing 1-6 carbon atoms, such as methyl, ethyl, propyl, isopropyl, butyl, isobutyl, t-butyl, sec-butyl, pentyl, hexyl, and the same. Similarly, C₁-C₆ alkoxy means C₁, C₂, C₃, C₄, C₅, and C₆ alkoxy.

In the present invention, "solvate thereof" means a solvate formed by the compound of the present invention and a solvent, wherein the solvent includes (but is not limited to) water, ethanol, methanol, isopropanol, propanediol, tetrahydrofuran, and dichloromethane.

In the present invention, "pharmaceutically acceptable" means a certain carrier, vehicle, diluent, excipient, and/or formed salt is usually chemically or physically compatible with other ingredients constituting a certain pharmaceutical dosage form, as well as physiologically compatible with the recipient.

In the present invention, "salt" means acid and/or basic salt that is formed by the reaction of a compound or its stereoisomer with an inorganic and/or organic acid and/or base, and also includes zwitterionic salts (inner salts), and further includes quaternary ammonium salts, such as alkylammonium salt. These salts can be directly obtained during the final isolation and purification of a compound. The salts can also be obtained by mixing the compound or its stereoisomers with a certain amount of acid or base appropriately (for example, in equivalent). These salts may form a precipitate in the solution, and be collected by filtration, or recovered after evaporation of the solvent, or obtained by freeze-drying after reaction in an aqueous medium. The salt in the present invention may be compounds' hydrochloride, sulfate, citrate, benzenesulfonate, hydrobromide, hydrofluoride, phosphate, acetate, propionate, succinate, oxalate, malate, succinate, fumarate, maleate, tartrate or trifluoroacetate of compounds.

In the present invention, "aromatic ring" denote all-carbon monocyclic or fused polycyclic rings with conjugated π electron system, such as benzene and naphthene. Said aromatic ring can be fused to other cyclic groups (including saturated and unsaturated rings), but can not contain hetero atoms such as nitrogen, oxygen, or sulfur. At the same time, the point connecting with the parent must be on the carbon in the ring having the conjugated *π* electron system. "Monocyclic aromatic ring" means an all-carbon monocyclic ring with a conjugated π-electron system. Similarly, "aryl" means an all-carbon monocyclic or fused polycyclic group with a conjugated π-electronsystem, such as phenyl and naphthyl.

"Heteroaromatic ring" means a monocyclic or fused polycyclic ring having a conjugated π-electron system and containing one or more heteroatoms, which contains at least one ring heteroatom selected from N, O or S, while the rest of the ring atoms are C. Additionally, the ring has a fully conjugated π-electron system, such as furan, pyrrole, quinoline, thiophene, pyridine, pyrazole, N-alkylpyrrole, pyrimidine, pyrazine, imidazole, tetrazole, thienopyridyl and the like. The heteroaromatic ring may be fused on an aromatic ring, a heterocyclic ring or an alkane ring. "Monocyclic heteroaromatic ring" means a monocyclic ring with a conjugated π-electron system and containing one or more heteroatoms. Similarly, "heteroaryl" means a monocyclic or fused polycyclic group with a conjugated π-electron system and containing one or more heteroatoms.

Halogen is fluorine, chlorine, bromine, or iodine.

"Alkyl" is a hydrocarbon group formed by losing one hydrogen in an alkane molecule, such as methyl -CH₃, -CH₃CH₂, etc.

"Alkynyl" denotes an aliphatic hydrocarbon group with at least one C≡C triple bond. Said alkynyl can have a straight or branched chain. When the alkynyl has a limit on carbon numbers before it, for example, "C₂₋₆ alkynyl" denotes a straight or branched alkynyl having 2-6 carbons. "Alkenyl" denotes an aliphatic hydrocarbon group with at least one C=C double bond. Said alkenyl can have a straight or branched chain. When the alkenyl have a limit on carbon numbers before it, for example, "C₂₋₆ alkenyl" denotes a straight or branched alkenyl with 2-6 carbons.

"Cycloalkyl" denotes a saturated or unsaturated cyclic hydrocarbon substituents; cyclic hydrocarbon can have one or more rings. For example, "3-8 membered cycloalkyl" denotes a cycloalkyl having 3-8 carbons.

"Saturated cycloalkyl" denotes a saturated cycloalkyl; "unsaturated cycloalkyl" denotes an unsaturated cycloalkyl

"Monocyclic cycloalkyl" means that the cycloalkyl is monocyclic.

"Bridged cycloalkyl" denotes a polycyclic cycloalkyl group in which two rings share two non-adjacent carbon atoms.

"Spirocycloalkyl" refers to a polycyclic cycloalkyl in which two rings share one carbon atom. "Fused cycloalkyl" refers to a polycyclic cycloalkyl group in which two rings share two adjacent carbon atoms.

"Heterocyclic group" denotes a saturated or unsaturated cyclic hydrocarbon substituent; the cyclic hydrocarbon may be monocyclic or polycyclic, and carry at least one one ring heteroatom (including but not limited to O, S or N). For example, "3-8 membered heterocyclic group" denotes a heterocyclic group having 3-8 carbons.

"Saturated heterocyclic group" denotes a saturated heterocyclic group; "unsaturated heterocyclic group" denotes an unsaturated heterocyclic group.

"Monocyclic heterocyclic group" means that the heterocyclic group is monocyclic.

"Bridged heterocyclic group" means a polycyclic heterocyclic group in which two rings share two non-adjacent carbon atoms or heteroatoms.

"Spiroheterocyclic group" means a polycyclic heterocyclic group in which two rings share one carbon atom or heteroatom.

"Fused heterocyclic group" means a polycyclic heterocyclic group in which two rings share two adjacent carbon atoms or heteroatoms.

"Fused ring" means two rings that share two adjacent carbon atoms.

"Bridged ring" means two rings that share two non-adjacent carbon atoms.

"Spiro ring" means two rings that share a carbon atom.

In the present invention, "any two groups of substituents in rings A, B and C, and R⁴ are connected, together with the substituted atoms to which they are linked, to form a ring" means that any two groups are selected from the substituents in rings A, B and C as well as R⁴, and these two groups are linked with their respective connected atoms substituted to form another ring. Similarly, "R^{b3} and R^{b6}, together with the substituted atom to which they are linked, are connected to form a substituted or unsubstituted five-membered unsaturated heterocyclic ring" means that the two groups R^{b3} and R^{b6} are connected with their respective linked atoms substituted, to form a substituted or unsubstituted five-membered unsaturated heterocyclic ring. In the present invention, the isotope-substituted form of a compound means a compound obtained after any one or more atoms in the compound are substituted with an isotope.

Isotope means the different nuclides of the same element, which have the same number of protons and different neutrons. For example, there are three isotopes for hydrogen, i.e. H protium, D deuterium (also called heavy hydrogen), T tritium (also called super heavy hydrogen). Unless otherwise specified, hydrogen in the present invention is H. Carbon has several isotopes, including ¹²C, ¹³C and ¹⁴C. Unless otherwise specified, C is ¹²C in the present invention.

Obviously, based on the above content of the present invention, according to the common technical knowledge and the conventional means in the field, without department from above basic technical spirits, other various modifications, alternations, or changes can further be made.

By the following specific examples of said embodiments, the above content of the present invention is further illustrated. But it should not be construed that the scope of the above subject matter of the present invention is limited to the following examples. The techniques realized based on the above content of the present invention are all within the scope of the present invention.

### Examples

The starting materials and equipments used in the examples of the present invention are all known products and can be obtained by purchasing commercially available products. Intermediates SM-E-1, SM-E-2, SM-E-3, SM-E-4 were synthesized by the method in literature (PNAS 2016, 113, 7124; ACS Chemical Biology, 2018, 13, 553; US Pat. Appl. Publ., 20180099940).

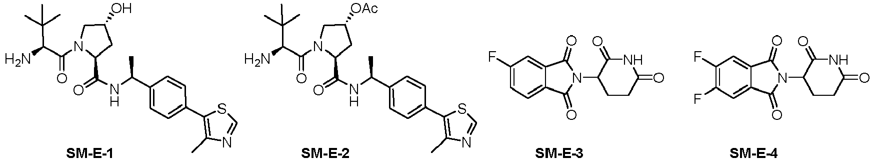

### Example Synthesis of compounds 1-194 according to the present invention:

### 1: (2S,4R)-1-((S)-2-(2-((5-(4-(3-((3-chloro-4-cyanophenyl)ethyl)amino)-4-methylphenyl)-3,5-d imethyl-1H-pyrazol-1-yl)pentyl)oxy)acetamido)-3,3-dimethylbutyryl)-4-hydroxy-N-((S)-1-(4-(4-methylthiazol-5-yl)phenyl)ethyl)pyrrolidinyl-2-formamide

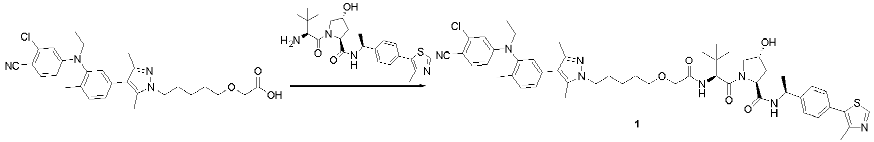

2-((5-(4-(3-((3-Chloro-4-cyanophenyl)(ethyl)amino)-4-methylphenyl)-3,5-dimethyl-1H-pyrazol -1-yl)pentyl)oxy)acetic acid (50 mg, 0.1 mmol) was dissolved in 5 mL of DMF, to which were successively added N,N-diisopropylethylamine (39 mg, 0.3 mmol), HATU (42 mg, 0.11 mmol), and (2*S*,4*R*)-1-((*S*)-2-amino-3,3-dimethylbutyryl)-4-hydroxy-*N*-((*S*)-1-(4-(4-methylthiazol-5-yl) phenyl)ethyl)-pyrrolidinyl-2-formamide (53 mg, 0.11 mmol). The mixture was allowed to react 2 h at room temperature, and then washed with 10 ml of water, followed by extraction with 10 mL of ethyl acetate. The organic layer was concentrated under reduced pressure, and the residue was separated and purified by thin layer chromatography (TLC), to provide 12 mg of white solid, which was the target compound (2*S*,4*R*)-1-((*S*)-2-(2-((5-(4-(3-((3-chloro-4-cyanophenyl)ethyl)amino)-4-methylphenyl)-3,5-dimethyl-1*H*-pyrazol-1-yl)pentyl)oxy)acetamido)-3,3-dimethylbutyryl)-4-hydroxy-*N-*((*S*)-1-(4-(4- methylthiazol-5-yl)phenyl)ethyl)pyrrolidinyl-2-formamide, with a yield of 25.5%. LC/MS (ESI⁺) calcd for C₅₁H₆₃ClN₈O₅S ([M+H]⁺) *m*/*z:* 935.4; found 935.6.

¹H NMR (400 MHz, CDCl₃): *δ* 8.67 (d, *J* = 2.3 Hz, 1H), 7.55 (d, *J* = 8.6 Hz, 1H), 7.47 - 7.33 (m, 7H), 7.20 (d, *J* = 10.7 Hz, 2H), 7.08 (d, *J* = 2.1 Hz, 1H), 7.02 - 6.96 (m, 1H), 6.93 (s, 1H), 5.12 - 5.03 (m, 1H), 4.76 (s, 1H), 4.54 (s, 2H), 4.25 (d, *J* = 5.3 Hz, 1H), 4.16 - 4.08 (m, 3H), 3.93 (d, *J=* 18.4 Hz, 3H), 3.61 (d, *J* = 11.3 Hz, 1H), 3.53 (d, *J=* 6.2 Hz, 2H), 3.01 (s, 1H), 2.80 (s, 1H), 2.53 (d, *J* = 4.6 Hz, 4H), 2.17 (d, *J* = 5.8 Hz, 4H), 2.06 - 1.99 (m, 6H), 1.47 (dd, *J* = 16.2, 9.4 Hz, 7H), 1.32 - 1.17 (m, 3H), 1.06 (d, *J* = 3.1 Hz, 9H).

### 2: (2S,4R)-1-((S)-2-(2-((5-(3-((3-chloro-4-cyanophenyl)(ethyl)amino)-4-methylphenyl)-4-methyl-1H-pyrazol-1-yl)pentyl)oxy)acetamido)-3,3-dimethylbutyryl)-4-hydroxy-N-((S)-1 -(4-(4-methylthiazol-5-yl)phenyl)ethyl)pyrrolidine-2-formamide

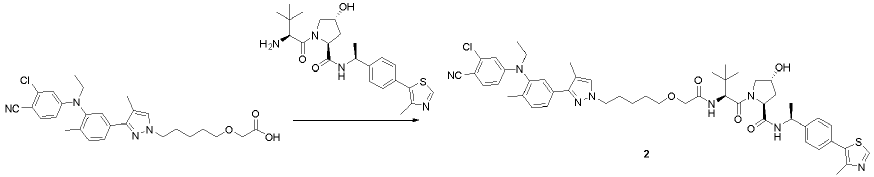

By referring to the method in above example, the target compound was synthesized according to the above route. LC/MS (ESI⁺) calcd for C₅₀H₆₁ClN₈O₅S ([M+H]⁺) *m*/*z:* 920.42; found 921.3.

### 3: (3R,5S)-1-((S)-2-(2-((5-((3-chloro-4-cyanophenyl)(5-(3,5-Dimethylisoxazol-4-yl)-2-methyl phenyl)amino)n-pentyl)oxy)acetylamino)-3,3-dimethylbutyryl)-5-(((S)-1-(4-(4-methylthiop hen-5-yl)phenyl)ethyl)formamido)pyrrolidinyl-3-acetate

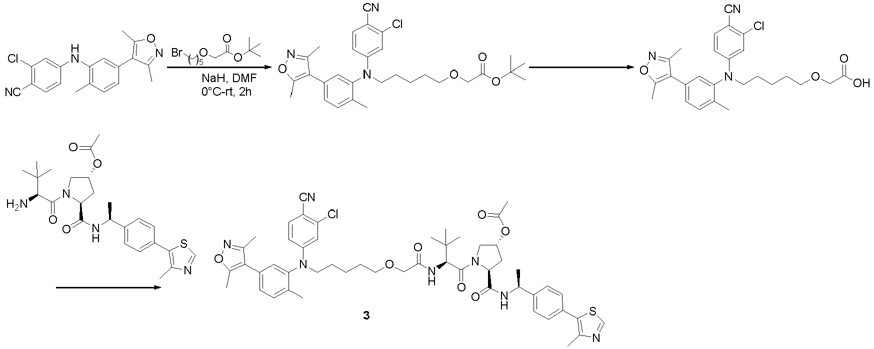

NaH (100 mg, 2.5 mmol) was added to 5 ml of tetrahydrofuran, to which was added 2-chloro-4-((5-(3,5-Dimethylisoxazol-4-yl)-2-methylphenyl)amino)benzonitrile (467 mg, 1.0 mmol), and the mixture was stirred and reacted 10 min, followed by addition of t-butyl 2-((5-((methanesulfonyl)oxy)n-pentyl)oxy)acetate (281 mg, 1.0 mmol). The resultant solution was allowed to react overnight at room temperature, and then washed with 10 ml of water, followed by extraction with 10 mL of ethyl acetate. The organic layer was concentrated under reduced pressure, and the residue was separated and purified by TLC, to provide 40 mg of compound t-butyl 2-((5-((3-chloro-4-cyanophenyl)(5-(dimethylisoxazol-4-yl)-2-methylphenyl) amino)n-pentyl)oxy)acetate, with a yield of 6%.

t-Butyl 2-((5-((3-chloro-4-cyanophenyl)(5-(dimethylisoxazol-4-yl)-2-methylphenyl)amino) n-pentyl)oxy)acetate (40 mg, 0.06 mmol) was dissolved in 3 mL of dichloromethane, to which was added 3 mL of trifluoroacetic acid, and the mixture was stirred at room temperature for 1 h. The reaction solution was concentrated to dryness under reduced pressure. The residue was washed with the saturated aqueous solution of sodium bicarbonate, and extracted with ethyl acetate. The organic layer was washed with saturated brine, dried over anhydrous sodium sulfate, and concentrated under reduced pressure to provide 20 mg of 2-((5-((3-chloro-4-cyanophenyl)(5-(3,5-Dimethylisoxazol-4-yl)-2-methylphenyl)amino)n-penty l)oxy)acetic acid, with a yield of 66%.

2-((5-((3-Chloro-4-cyanophenyl)(5-(3,5-Dimethylisoxazol-4-yl)-2-methylphenyl)amino)n-pent yl)oxy)acetic acid (50 mg, 0.1 mmol) was dissolved in 5 mL of DMF, to which were successively added *N,N*-diisopropylethylamine (39 mg, 0.3 mmol), HATU (42 mg, 0.11 mmol), and (3*R*,5*S*)-1-((*S*)-2-amino-3,3-dimethylbutyryl)-5-(((*S*)-1-(4-(4-methylthiazol-5-yl)phenyl) ethyl)aminomethyl)pyrrolidinyl-3-acetate (53 mg, 0.11 mmol). The mixture was allowed to react 2 h at room temperature, and then washed with 10 ml of water, followed by extraction with 10 mL of ethyl acetate. The organic layer was concentrated under reduced pressure, and the residue was separated and purified by TLC, to provide 12 mg of white solid, which is the target compound (3*R*,5*S*)-1-((*S*)-2-(2-((5-((3-chloro-4-cyanophenyl)(5-(3,5-dimethylisoxazol-4-yl)-2-methylphenyl)amino)n-pentyl)oxy)acetylamino)-3,3-dimethylbutyryl)-5-(((*S*)-1-(4-(4-m ethylthiophen-5-yl)phenyl)ethyl)formamido)pyrrolidinyl-3-acetate, with a yield of 25.5%.

LC/MS (ESI⁺) calcd for C₅₁H₆₀ClN₇O₇S ([M+H]⁺) *m*/*z:* 950.4; found 950.2.

¹H NMR (400 MHz, CDCl₃) *δ* 8.75 (s, 1H), 7.54 (d, 1H), 7.46 (d, *J=* 8.0 Hz, 1H), 7.38 (dd, *J* = 19.2, 8.2 Hz, 4H), 7.25 - 7.15 (m, 2H), 7.09 (d, *J=* 9.1 Hz, 1H), 6.99 (d, *J=* 1.5 Hz, 1H), 6.75 (s, 1H), 6.59 (s, 1H), 5.35 (s, 1H), 5.15 - 5.00 (m, 1H), 4.75 - 4.65 (m, 1H), 4.58 (d, *J=* 9.3 Hz, 1H), 4.05 (d, *J* = 11.6 Hz, 1H), 3.93 (s, 2H), 3.84 (dd, *J=* 11.5, 4.9 Hz, 1H), 3.52 (t, *J=* 6.5 Hz, 2H), 2.55 (s, 3H), 2.41 (s, 3H), 2.27 (s, 3H), 2.14 (s, 3H), 2.08 (d, *J=* 7.3 Hz, 2H), 2.04 (s, 3H), 1.48 (d, *J=* 6.9 Hz, 5H), 1.27 (d, *J=* 12.5 Hz, 6H), 1.04 (s, 9H).

### 4: (3R,5S)-1-((S)-2-(2-((5-((4-cyano-3-(trifluoromethyl)phenyl)(5-(3,5-dimethylisoxazol-4-yl)-2-methylphenyl)amino)n-pentyl)oxy)acetylamino)-3,3-dimethylbutyryl)-5-(((S)-1-(4-( 4-methylthiophen-5-yl)phenyl)ethyl)formamido)pyrrolidinyl-3-acetate

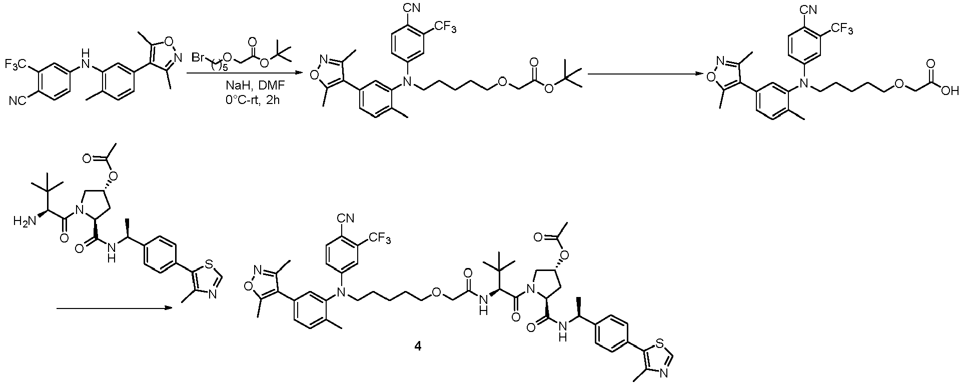

NaH (100 mg, 2.5 mmol) was added to 5 ml of tetrahydrofuran, to which was added 4-((5-(3,5-Dimethylisoxazol-4-yl)-2-methylphenyl)amino)-2-trifluoromethyl)benzonitrile (467 mg, 1.0 mmol), and the mixture was stirred and reacted 10 min, followed by addition of t-butyl 2-((5-((methanesulfonyl)oxy)n-pentyl)oxy)acetate (281 mg, 1.0 mmol). The resultant solution was allowed to react overnight at room temperature, and then washed with 10 ml of water, followed by extraction with 10 mL of ethyl acetate. The organic layer was concentrated under reduced pressure, and the residue was separated and purified by TLC, to provide 40 mg of compound t-butyl 2-((5-((4-cyano-3-(trifluoromethyl)phenyl)(5-(3,5-Dimethylisoxazol-4-yl)-2-methylphenyl)amino)n-pentyl)oxy)acetate, with a yield of 6%.

t-Butyl 2-((5-((4-cyano-3-(trifluoromethyl)phenyl)(5-(3,5-Dimethylisoxazol-4-yl)-2-methyl phenyl)amino)n-pentyl)oxy)acetate (40 mg, 0.06 mmol) was dissolved in 3 mL of dichloromethane, to which was added 3 mL of trifluoroacetic acid, and the mixture was stirred at room temperature for 1 h. The reaction solution was concentrated to dryness under reduced pressure. The residue was washed with the saturated aqueous solution of sodium bicarbonate, and extracted with ethyl acetate. The organic layer was washed with saturated brine, dried over anhydrous sodium sulfate, and concentrated under reduced pressure to provide 20 mg of 2-((5-((4-cyano-3-(trifluoromethyl)phenyl)(5-(dimethylisoxazol-4-yl)-2-methylphenyl)amino)n -pentyl)oxy)acetic acid, with a yield of 66%.

2-((5-((4-Cyano-3-(trifluoromethyl)phenyl)(5-(dimethylisoxazol-4-yl)-2-methylphenyl)amino)n -pentyl)oxy)acetic acid (50 mg, 0.1 mmol) was dissolved in 5 mL of DMF, to which were successively added *N,N*-diisopropylethylamine (39 mg, 0.3 mmol), HATU (42 mg, 0.11 mmol), and (3*R*,5*S*)-1-((*S*)-2-amino-3,3-dimethylbutyryl)-5-(((*S*)-1-(4-(4-methylthiazol-5-yl)phenyl) ethyl)aminomethyl)pyrrolidinyl-3-acetate (53 mg, 0.11 mmol). The mixture was allowed to react 2 h at room temperature, and then washed with 10 ml of water, followed by extraction with 10 mL of ethyl acetate. The organic layer was concentrated under reduced pressure, and the residue was separated and purified by TLC, to provide 12 mg of white solid, which is the target compound (3*R*,5*S*)-1-((*S*)-2-(2-((5-((4-cyano-3-(trifluoromethyl)phenyl)(5-(3,5-dimethyl isoxazol-4-yl)-2-methylphenyl)amino)n-pentyl)oxy)acetylamino)-3,3-dimethylbutyryl)-5-(((*S*)-1-(4-(4-methylthiophen-5-yl)phenyl)ethyl)formamido)pyrrolidinyl-3-acetate, with a yield of 25.5%.

LC/MS (ESI⁺) calcd for C₅₂H₆₀F₃N₇O₇S ([M+H]⁺) *m*/*z:* 984.4; found 984.3.

¹H NMR (400 MHz, CDCl₃) *δ* 8.75 (s, 1H), 7.54 (d, 1H), 7.46 (d, *J=* 8.0 Hz, 1H), 7.38 (dd, *J* = 19.2, 8.2 Hz, 4H), 7.25 - 7.15 (m, 2H), 7.09 (d, *J=* 9.1 Hz, 1H), 6.99 (d, *J=* 1.5 Hz, 1H), 6.75 (s, 1H), 6.59 (s, 1H), 5.35 (s, 1H), 5.15 - 5.00 (m, 1H), 4.75 - 4.65 (m, 1H), 4.58 (d, *J=* 9.3 Hz, 1H), 4.05 (d, *J* = 11.6 Hz, 1H), 3.93 (s, 2H), 3.84 (dd, *J=* 11.5, 4.9 Hz, 1H), 3.52 (t, *J=* 6.5 Hz, 2H), 2.55 (s, 3H), 2.41 (s, 3H), 2.27 (s, 3H), 2.14 (s, 3H), 2.08 (d, *J=* 7.3 Hz, 2H), 2.04 (s, 3H), 1.48 (d, *J=* 6.9 Hz, 5H), 1.27 (d, *J=* 12.5 Hz, 6H), 1.04 (s, 9H).

### 5: (3R,5S)-1-((S)-2-(2-((6-((4-(1H-imidazol-1-yl)phenyl)(5-(3,5-Dimethylisoxazol-4-yl)-2-methylphenyl)amino)n-hexyl)oxy)acetylamino)-3,3-dimethylbutyryl)-5-(((S)-1-(4-(4-met hylthiazol-5-yl)phenyl)ethyl)formylamino)pyrrolidinyl-3-acetate

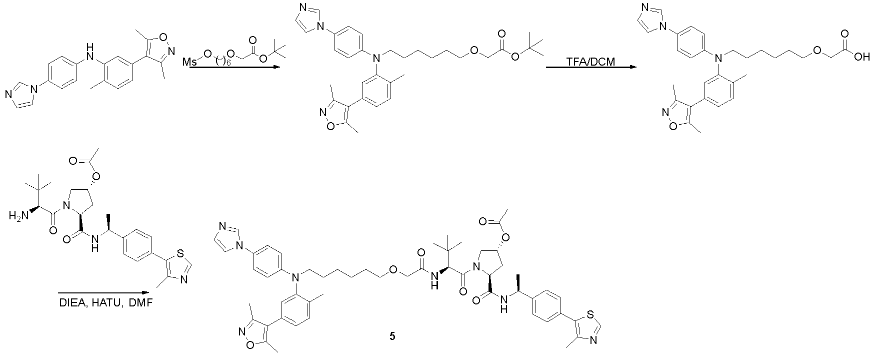

60% sodium hydride (50 mg, 1.25 mmol) was dissolved in 3 mL of dimethylsulfoxide, to which was added *N*-(4-(1*H*-imidazol-1-yl)phenyl)-5-(3,5-Dimethylisoxazol-4-yl)-2- methylaniline (344 mg, 1.0 mmol), and the mixture was stirred for 10 min at room temperature, followed by addition of t-butyl 2-((6-(methanesulfonyl)oxy)n-hexyl)oxy)acetate (561 mg, 2.0 mmol). The resultant solution was allowed to react overnight at 60 °C, and then washed with 10 ml of water, followed by extraction with 10 mL of ethyl acetate. The organic layer was concentrated under reduced pressure, and the residue was separated and purified by TLC, to provide 90 mg of compound t-butyl 2-((6-((4-(1*H*-imidazol-1-yl)phenyl)(5-(3,5-Dimethylisoxazol-4-yl)-2-methylphenyl)amino)n-hexyl)oxy)acetate, with a yield of 26%. The compound t-butyl 2-((6-((4-(1*H*-imidazol-1-yl)phenyl)(5-(3,5-Dimethylisoxazol-4-yl)-2-methylphenyl)amino)n-hexyl)oxy)acetate (90 mg, 0.26 mmol), obtained in previous step, was dissolved in 3 mL of dichloromethane, to which was added 2 mL of trifluoroacetic acid, and the mixture was allowed to react at room temperature for 1 h. The reaction solution was concentrated to dryness under reduced pressure, to provide 2-((6-((4-(1*H-*imidazol-1-yl)phenyl) (5-(3,5-Dimethylisoxazol-4-yl)-2-methylphenyl)amino)n-hexyl)oxy)acetic acid.

The obtained compound was divided into two equal parts. The first part was added in 5 mL DMF, to which were successively added diisopropylethylamine (55 mg, 0.43 mmol), HATU (35 mg, 0.09 mmol), and (3*R*,5*S*)-1-((*S*)-2-amino-3,3-dimethylbutyryl)-5-(((*S*)-1-(4-(4-methylthiazol-5-yl)phenyl)ethyl)aminomethyl)pyrrolidinyl-3-acetate (46 mg, 0.09 mmol). The mixture was allowed to react 2 h at room temperature, and then washed with 10 ml of water, followed by extraction with 10 mL of ethyl acetate. The organic layer was concentrated under reduced pressure, and the residue was separated and purified by TLC, to provide 20 mg of compound (3*R*,5*S*)-1-((S)-2-(2-((6-((4-(1*H-*imidazol-1-yl)phenyl)(5-(3,5-dimethylisoxazol-4-yl)-2-methylphenyl)amino)n-hexyl)oxy)acetylamino)-3,3-dimethylbutyryl)-5-(((*S*)-1-(4-(4-m ethylthiazol-5-yl)phenyl)ethyl)formylamino)pyrrolidinyl-3-acetate, with a yield of 23%.

LC/MS (ESI+) calcd for C₅₄H₆₆N₈O₇S ([M+H]⁺) *m*/*z:* 970.48; found 971.4.

### 6: (2S,4R)-1-((S)-2-(2-((6-((4-(1H-imidazol-1-yl)phenyl)(5-(3,5-Dimethylisoxazol-4-yl)-2-methylphenyl)amino)n-hexyl)oxy)acetylamino)-3,3-dimethylbutyryl)-4-hydroxy-N-((S)-1-( 4-(4-methylthiazol-5-yl)phenyl)ethyl)pyrrolidinyl-2-formamide

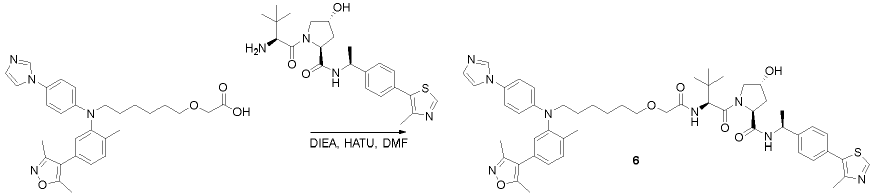

The other part 2-((6-((4-(1*H*-imidazol-1-yl)phenyl)(5-(3,5-Dimethylisoxazol-4-yl)-2-methylphenyl)amino)n-hexyl)oxy)acetic acid was added in 5 mL of DMF, to which were successively added diisopropylethylamine (55 mg, 0.43 mmol), HATU (35 mg, 0.09 mmol), and (2*S*,4*R*)-1-((*S*)-2-amino-3,3-dimethylbutyryl)-4-hydroxy-N-((*S*)-1-(4-(4-methylthiazol-5-yl)phenyl)ethyl)-pyrrolidinyl-2-formamide (41 mg, 0.09 mmol). The mixture was allowed to react 2 h at room temperature, and then washed with 10 ml of water, followed by extraction with 10 mL of ethyl acetate. The organic layer was concentrated under reduced pressure, and the residue was separated and purified by TLC, to provide 21 mg of compound (2*S*,4*R*)-1-((*S*)-2-(2-((6-((4-(1*H*-imidazol-1-yl)phenyl)(5-(3,5-Dimethylisoxazol-4-yl)-2-methyl phenyl)amino)n-hexyl)oxy)acetylamino)-3,3-dimethylbutyryl)-4-hydroxy-*N*-((*S*)-1-(4-(4-methy lthiazol-5-yl)phenyl)ethyl)pyrrolidinyl-2-formamide.

LC/MS (ESI+) calcd for C₅₂H₆₄N₈O₆S ([M+H]⁺) *m*/*z:* 929.5; found 929.5_{∘}

### 7: (3R,5S)-1-((S)-2-(2-((7-((4-(1H-imidazol-1-yl)phenyl)(5-(3,5-Dimethylisoxazol-4-yl)-2-methylphenyl)aminoheptyl)oxy)acetylamino)-3,3-dimethylbutyryl)-5-(((S)-1-(4-(4-methy lthiazol-5-yl)phenyl)ethyl)formylamino)pyrrolidinyl-3-acetate

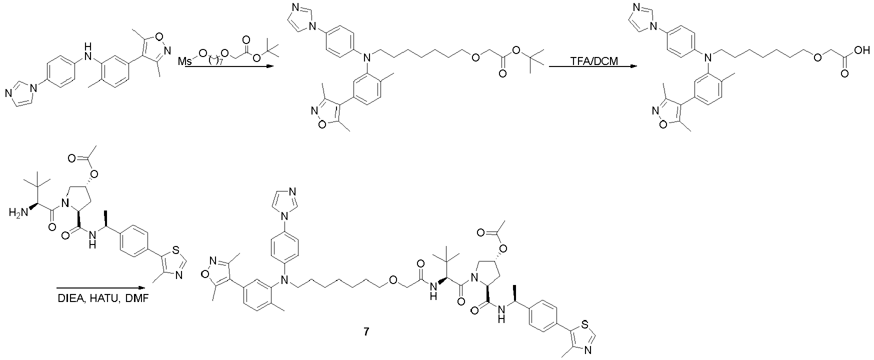

60% sodium hydride (50 mg, 1.25 mmol) was dissolved in 3 mL of dimethylsulfoxide, to which was added N-(4-(1H-imidazol-1-yl)phenyl)-5-(3,5-Dimethylisoxazol-4-yl)- 2-methylaniline (344 mg, 1.0 mmol), and the mixture was stirred for 10 min at room temperature, followed by addition of t-butyl 2-((7-(methanesulfonyl)oxyn-heptyl)oxy)acetate (561 mg, 2.0 mmol). The resultant solution was allowed to react overnight at 60 °C, and then washed with 10 ml of water, followed by extraction with 10 mL of ethyl acetate. The organic layer was concentrated under reduced pressure, and the residue was separated and purified by TLC, to provide 90 mg of compound t-butyl 2-((7-((4-(1*H*-imidazol-1-yl)phenyl) (5-(3,5-Dimethylisoxazol-4-yl)-2-methylphenyl)aminoheptyl)oxy)acetate, with a yield of 26%. The compound t-butyl 2-((7-((4-(1*H*-imidazol-1-yl)phenyl)(5-(3,5-Dimethylisoxazol-4-yl)-2-methylphenyl)aminoheptyl)oxy)acetate (90 mg, 0.26 mmol), obtained in previous step, was dissolved in 3 mL of dichloromethane, to which was added 2 mL of trifluoroacetic acid, and the mixture was allowed to react at room temperature for 1 h. The reaction solution was concentrated to dryness under reduced pressure, to provide 2-((7-((4-(1*H*-imidazol-1-yl)phenyl)(5-(3,5-Dimethylisoxazol-4-yl)-2-methylphenyl)aminohept yl)oxy)acetic acid.

The obtained compound was divided into two equal parts. The first part was added in 5 mL DMF, to which were successively added diisopropylethylamine (55 mg, 0.43 mmol), HATU (35 mg, 0.09 mmol), and (3*R*,5*S*)-1-((*S*)-2-amino-3,3-dimethylbutyryl)-5-(((*S*)-1-(4-(4-methyl thiazol-5-yl)phenyl)ethyl)aminomethyl)pyrrolidinyl-3-acetate (46 mg, 0.09 mmol). The mixture was allowed to react 2 h at room temperature, and then washed with 10 ml of water, followed by extraction with 10 mL of ethyl acetate. The organic layer was concentrated under reduced pressure, and the residue was separated and purified by TLC, to provide 20 mg of compound (3R,5S)-1-((*S*)-2-(2-((7-((4-(1*H*-imidazol-1-yl)phenyl)(5 -(3,5 -Dimethylisoxazol-4-yl)-2-methyl phenyl)aminoheptyl)oxy)acetylamino)-3,3-dimethylbutyryl)-5-(((S)-1-(4-(4-methylthiazol-5-yl) phenyl)ethyl)formylamino)pyrrolidinyl-3-acetate, with a yield of 23%. LC/MS (ESI⁺) calcd for C₅₅H₆₈N₈O₇S ([M+H]⁺) *m*/*z:* 984.49; found 985.3.

### 8: (2S,4R)-1-((S)-2-(2-((7-((4-(1H-imidazol-1-yl)phenyl)(5-(3,5-Dimethylisoxazol-4-yl)-2-methylphenyl)aminoheptyl)oxy)acetylamino)-3,3-dimethylbutyryl)-4-hydroxy-N-((S)-1-( 4-(4-methylthiazol-5-yl)phenyl)ethyl)pyrrolidinyl-2-formamide

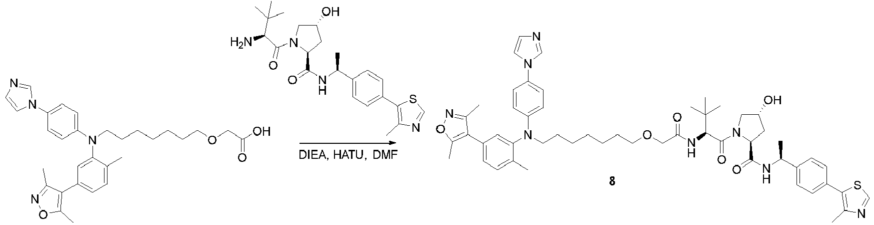

The other part 2-((7-((4-(1*H*-imidazol-1-yl)phenyl)(5-(3,5-Dimethylisoxazol-4-yl)-2-methylphenyl)aminoheptyl)oxy)acetic acid was added in 5 mL of DMF, to which were successively added diisopropylethylamine (55 mg, 0.43 mmol), HATU (35 mg, 0.09 mmol), and (2*S*,4*R*)-1-((*S*)-2-amino-3,3-dimethylbutyryl)-4-hydroxy-*N*-((*S*)-1-(4-(4-methylthiazol-5-yl) phenyl)ethyl)-pyrrolidinyl-2-formamide (41 mg, 0.09 mmol). The mixture was allowed to react 2 h at room temperature, and then washed with 10 ml of water, followed by extraction with 10 mL of ethyl acetate. The organic layer was concentrated under reduced pressure, and the residue was separated and purified by TLC, to provide 21 mg of compound (2*S*,4*R*)-1-((*S*)-2-(2-((7-((4-(1*H*-imidazol-1-yl)phenyl)(5-(3,5-Dimethylisoxazol-4-yl)-2-methyl phenyl)aminoheptyl)oxy)acetylamino)-3,3-dimethylbutyryl)-4-hydroxy-*N*-((*S*)-1-(4-(4-methylt hiazol-5-yl)phenyl)ethyl)pyrrolidinyl-2-formamide. LC/MS (ESI⁺) calcd for C₅₄H₆₆N₈O₇S ([M+H]⁺) *m*/*z:* 943.5; found 943.5.

### 9: (3R,5S)-1-((S)-2-(2-((5-((3-bromo-4-(1H-imidazol-1-yl)phenyl)(5-(3,5-dimethylisoxazol-4-yl)-2-methylphenyl)amino)n-pentyl)oxy)acetylamino)-3,3-dimethylbutyryl)-5-(((S)-1-(4-( 4-methylthiazol-5-yl)phenyl)ethyl)formylamino)pyrrolidinyl-3-acetate

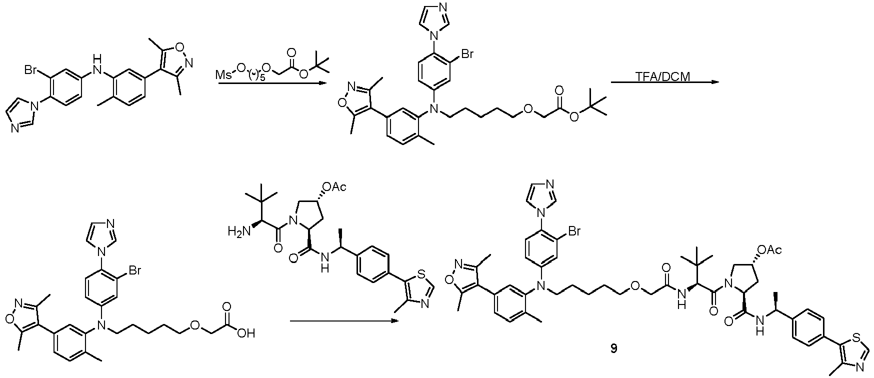

60% sodium hydride (50 mg, 1.25 mmol) was dissolved in 3 mL of dimethylsulfoxide, to which was added *N*-(3-bromo-4-(1*H*-imidazol-1-yl)phenyl)-5-(3,5-Dimethylisoxazol-4-yl)-2-methylaniline (344 mg, 1.0 mmol), and the mixture was stirred for 10 min at room temperature, followed by addition of t-butyl 2-((5-((methanesulfonyl)oxy)n-pentyl)oxy)acetate (561 mg, 2.0 mmol). The resultant solution was allowed to react overnight at 60 °C, and then washed with 10 ml of water, followed by extraction with 10 mL of ethyl acetate. The organic layer was concentrated under reduced pressure, and the residue was separated and purified by TLC, to provide 90 mg of compound t-butyl 2-((5-((3-bromo-4-(1*H*-imidazol-1-yl)phenyl) (5-(3,5-Dimethylisoxazol-4-yl)-2-methylphenyl)amino)n-pentyl)oxy)acetate, with a yield of 26%.

The compound t-butyl 2-((5-((3-bromo-4-(1*H*-imidazol-1-yl)phenyl)(5-(3,5-dimethylisoxazol-4-yl)-2-methylphenyl)amino)n-pentyl)oxy)acetate (90 mg, 0.26 mmol), obtained in previous step, was dissolved in 3 mL of dichloromethane, to which was added 2 mL of trifluoroacetic acid, and the mixture was allowed to react at room temperature for 1 h. The reaction solution was concentrated to dryness under reduced pressure, to provide 2-((5-((3-bromo-4-(1*H*-imidazol-1-yl)phenyl)(5-(3,5-Dimethylisoxazol-4-yl)-2-methylphenyl)a mino)n-pentyl)oxy)acetic acid.

2-((5-((3-Bromo-4-(1*H*-imidazol-1-yl)phenyl)(5-(3,5-Dimethylisoxazol-4-yl)-2-methylphenyl)a mino)n-pentyl)oxy)acetic acid was divided into two equal parts. The first part was added in 5 mL DMF, to which were successively added diisopropylethylamine (55 mg, 0.43 mmol), HATU (35 mg, 0.09 mmol), and (3*R*,5*S*)-1-((*S*)-2-amino-3,3-dimethylbutyryl)-5-(((*S*)-1-(4-(4-methylthiazol-5-yl)phenyl)ethyl)aminomethyl)pyrrolidinyl-3-acetate (46 mg, 0.09 mmol). The mixture was allowed to react 2 h at room temperature, and then washed with 10 ml of water, followed by extraction with 10 mL of ethyl acetate. The organic layer was concentrated under reduced pressure, and the residue was separated and purified by TLC, to provide 20 mg of compound (3*R*,5*S*)-1-((*S*)-2-(2-((5-((3-bromo-4-(1*H*-imidazol-1-yl)phenyl)(5-(3,5-dimethylisoxazol-4-yl)-2-methylphenyl)amino)n-pentyl)oxy)acetamido)-3,3-dimethylbutyryl)-5 -(((*S*)-1-(4-(4-methylthiazol-5-yl)phenyl)ethyl)formylamino)pyrrolidinyl-3-acetate, with a yield of 23%. LC/MS (ESI⁺) calcd for C₅₃H₆₃BrN₈O₇S ([M+H]⁺) *m*/*z:* 1035.4; found 1035.4. ¹H NMR (400 MHz, CDCl₃) *δ* 8.68 (s, 1H), 8.23 (s, 1H), 7.52 (d, *J=* 5.3 Hz, 1H), 7.45 (d, *J=* 7.9 Hz, 1H), 7.42 - 7.29 (m, 5H), 7.21 (dd, *J* = 7.8, 1.7 Hz, 1H), 7.12 (d, *J* = 7.0 Hz, 2H), 7.04 - 6.98 (m, 2H), 6.76 (d, *J* = 2.4 Hz, 1H), 6.50 (d, *J* = 6.3 Hz, 1H), 5.34 (s, 1H), 5.14 - 5.01 (m, 1H), 4.80 - 4.71 (m, 1H), 4.54 (d, *J* = 9.3 Hz, 1H), 4.07 (d, *J* = 11.9 Hz, 1H), 3.94 (q, *J* = 15.3 Hz, 2H), 3.83 (dd, *J* = 11.6, 4.8 Hz, 1H), 3.54 (dd, *J* = 6.0, 4.3 Hz, 2H), 2.80 (s, 3H), 2.66 - 2.55 (m, 1H), 2.53 (s, 3H), 2.43 (s, 3H), 2.29 (s, 3H), 2.19 (s, 3H), 2.06 (s, 3H), 179-1.63(m, 5H), 1.48 (dd, *J=* 18.4, 6.8 Hz, 4H), 1.02 (d, *J=* 8.4 Hz, 9H).

### 10: (2S,4R)-1-((S)-2-(2-((5-((3-bromo-4-(1H-imidazol-1-yl)phenyl)(5-(3,5-dimethylisoxazol-4-yl)-2-methylphenyl)amino)n-pentyl)oxy)acetylamino)-3,3-dimethylbutyryl)-4-hydroxy-N -((S)-1-(4-(4-methylthiazol-5-yl)phenyl)ethyl)pyrrolidinyl-2-formamide

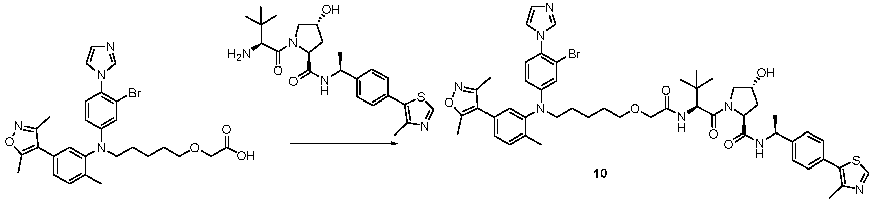

The other part 2-((5-((3-bromo-4-(1*H*-imidazol-1-yl)phenyl)(5-(3,5-Dimethylisoxazol-4-yl)-2-methylphenyl)amino)n-pentyl)oxy)acetic acid was added in 5 mL of DMF, to which were successively added diisopropylethylamine (55 mg, 0.43 mmol), HATU (35 mg, 0.09 mmol), and (2*S*,4*R*)-1-((*S*)-2-amino-3,3-dimethylbutyryl)-4-hydroxy-*N*-((*S*)-1-(4-(4-methylthiazol-5-yl) phenyl)ethyl)-pyrrolidinyl-2-formamide (41 mg, 0.09 mmol). The mixture was allowed to react 2 h at room temperature, and then washed with 10 ml of water, followed by extraction with 10 mL of ethyl acetate. The organic layer was concentrated under reduced pressure, and the residue was separated and purified by TLC, to provide 21 mg of compound (2*S*,4*R*)-1-((S)-2-(2-((5-((3-bromo-4-(1*H*-imidazol-1-yl)phenyl)(5-(3,5-Dimethylisoxazol-4-yl)-2-methylphenyl)amino)n-pentyl)oxy)acetylamino)-3,3-dimethylbutyryl)-4-hydroxy-*N*-((*S*)-1-(4 -(4-methylthiazol-5-yl)phenyl)ethyl)pyrrolidinyl-2-formamide. LC/MS (ESI⁺) calcd for C₅₁H₆₁BrN₈O₆S ([M+H]⁺) *m*/*z:* 993.4; found 993.4. ¹H NMR (400 MHz, CDCl₃) *δ* 8.68 (s, 1H), 7.45 (d, *J* = 7.8 Hz, 2H), 7.38 (m, 6H), 7.21 (d, *J* = 7.7 Hz, 4H), 7.12 (m, 1H), 7.02 (s, 1H), 6.74 (s, 1H), 6.54 (s, 1H), 5.08 (s, 1H), 4.74 (s, 1H), 4.53 (s, 1H), 4.12 (s, 1H), 3.92 (s, 2H), 3.59 (d, *J=* 36.6 Hz, 6H), 2.81 (s, 1H), 2.52 (s, 3H), 2.43 (s, 3H), 2.29 (s, 3H), 2.19 (s, 3H), 1.46 (d, *J=* 6.5 Hz, 8H), 1.25 (s, 2H), 1.04 (s, 9H).

### 11: (3R,5S)-1-((S)-2-(2-((5-((3-chloro-4-(1H-imidazol-1-yl)phenyl)(5-(3,5-dimethylisoxazol-4-yl)-2-methylphenyl)amino)n-pentyl)oxy)acetylamino)-3,3-dimethylbutyryl)-5-(((S)-1-(4-( 4-methylthiazol-5-yl)phenyl)ethyl)formylamino)pyrrolidinyl-3-acetate

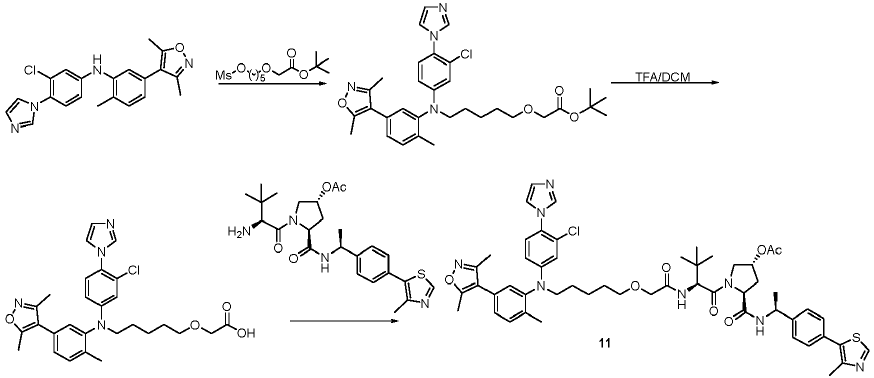

60% sodium hydride (50 mg, 1.25 mmol) was dissolved in 3 mL of dimethylsulfoxide, to which was added *N*-(3-chloro-4-(1*H*-imidazol-1-yl)phenyl)-5-(3,5-Dimethylisoxazol-4-yl)-2-methylaniline (344 mg, 1.0 mmol), and the mixture was stirred for 10 min at room temperature, followed by addition of t-butyl 2-((5-((methanesulfonyl)oxy)n-pentyl)oxy)acetate (561 mg, 2.0 mmol). The resultant solution was allowed to react overnight at 60 °C, and then washed with 10 ml of water, followed by extraction with 10 mL of ethyl acetate. The organic layer was concentrated under reduced pressure, and the residue was separated and purified by TLC, to provide 90 mg of compound t-butyl 2-((5-((3-chloro-4-(1*H*-imidazol-1-yl)phenyl)

(5-(3,5-Dimethylisoxazol-4-yl)-2-methylphenyl)amino)n-pentyl)oxy)acetate, with a yield of 26%.

The compound t-butyl 2-((5-((3-chloro-4-(1*H*-imidazol-1-yl)phenyl)(5-(3,5-dimethylisoxazol-4-yl)-2-methylphenyl)amino)n-pentyl)oxy)acetate (90 mg, 0.26 mmol), obtained in previous step, was dissolved in 3 mL of dichloromethane, to which was added 2 mL of trifluoroacetic acid, and the mixture was allowed to react at room temperature for 1 h. The reaction solution was concentrated to dryness under reduced pressure, to provide 2-((5-((3-chloro-4-(1*H-*imidazol-1-yl)phenyl)(5-(3,5-Dimethylisoxazol-4-yl)-2-methylphenyl)amino)n-pentyl)oxy)acet ic acid.

2-((5-((3-Chloro-4-(1*H*-imidazol-1-yl)phenyl)(5-(3,5-Dimethylisoxazol-4-yl)-2-methylphenyl)a mino)n-pentyl)oxy)acetic acid was divided into two equal parts. The first part was added in 5 mL DMF, to which were successively added diisopropylethylamine (55 mg, 0.43 mmol), HATU (35 mg, 0.09 mmol), and (3*R*,5*S*)-1-((*S*)-2-amino-3,3-dimethylbutyryl)-5-(((*S*)-1-(4-(4-methylthiazol-5-yl)phenyl)ethyl)aminomethyl)pyrrolidinyl-3-acetate (46 mg, 0.09 mmol). The mixture was allowed to react 2 h at room temperature, and then washed with 10 ml of water, followed by extraction with 10 mL of ethyl acetate. The organic layer was concentrated under reduced pressure, and the residue was separated and purified by TLC, to provide 20 mg of compound (3*R*,5*S*)-1-((*S*)-2-(2-((5-((3-chloro-4-(1*H*-imidazol-1-yl)phenyl)(5-(3,5-Dimethylisoxazol-4-yl)-2-methylphenyl)amino)n-pentyl)oxy)acetamido)-3,3-dimethylbuty ryl)-5-(((*S*)-1-(4-(4-methylthiazol-5-yl)phenyl)ethyl)formylamino)pyrrolidinyl-3-acetate, with a yield of 23%. LC/MS (ESI⁺) calcd for C₅₃H₆₃ClN₈O₇S ([M+H]⁺) *m*/*z:* 991.4; found 991.4.

¹H NMR (400 MHz, CDCl₃) *δ* 8.68 (s, 1H), 8.11 (s, 1H), 7.52 (d, *J* = 5.3 Hz, 1H), 7.45 (d, *J*= 7.9 Hz, 1H), 7.42 - 7.35 (m, 5H), 7.29 (dd, *J* = 7.8, 1.7 Hz, 1H), 7.12 (d, *J* = 7.0 Hz, 2H), 7.05 - 7.00 (m, 2H), 6.59 (d, *J=* 2.4 Hz, 1H), 6.43 (d, *J* = 6.3 Hz, 1H), 5.35 (s, 1H), 5.10 - 5.05 (m, 1H), 4.77 - 4.72 (m, 1H), 4.59-4.54 (m, 1H), 4.06 (d, *J* = 11.9 Hz, 1H), 3.94 (q, *J* = 15.3 Hz, 2H), 3.83 (dd, *J=* 11.6, 4.8 Hz, 1H), 3.53 (dd, *J=* 6.0, 4.3 Hz, 2H), 2.80 (s, 3H), 2.66 - 2.55 (m, 1H), 2.53 (s, 3H), 2.42 (s, 3H), 2.28 (s, 3H), 2.16(s, 3H), 2.04 (s, 3H), 179-1.63(m, 5H), 1.60-1.45 (dd, *J=* 18.4, 6.8 Hz, 4H), 1.04 (d, *J=* 8.4 Hz, 9H).

### 12: (2S,4R)-1-((S)-2-(2-((5-((3-chloro-4-(1H--imidazol-1-yl)phenyl)(5-(3,5-dimethylisoxazol-4-yl)-2-methylphenyl)amino)n-pentyl)oxy)acetylamino)-3,3-dimethylbut yryl)-4-hydroxy-N-((S)-1-(4-(4-methylthiazol-5-yl)phenyl)ethyl) pyrrolidinyl-2-formamide

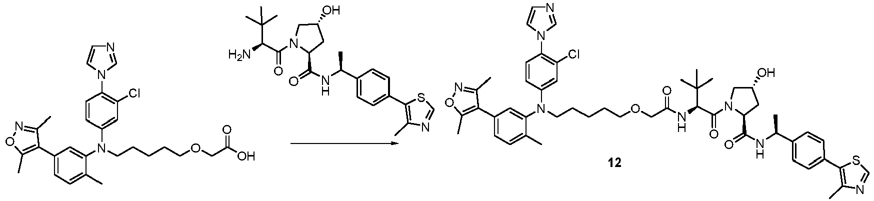

The other part 2-((5-((3-chloro-4-(1*H*-imidazol-1-yl)phenyl)(5-(3,5-Dimethylisoxazol-4-yl)-2-methylphenyl)amino)n-pentyl)oxy)acetic acid was added in 5 mL of DMF, to which were successively added diisopropylethylamine (55 mg, 0.43 mmol), HATU (35 mg, 0.09 mmol), and (2*S*,4*R*)-1-((*S*)-2-amino-3,3-dimethylbutyryl)-4-hydroxy-*N*-((*S*)-1-(4-(4-methylthiazol-5-yl) phenyl)ethyl)-pyrrolidinyl-2-formamide (41 mg, 0.09 mmol). The mixture was allowed to react 2 h at room temperature, and then washed with 10 ml of water, followed by extraction with 10 mL of ethyl acetate. The organic layer was concentrated under reduced pressure, and the residue was separated and purified by TLC, to provide 21 mg of compound (2*S*,4*R*)-1-((*S*)-2-(2-((5-((3-chloro-4-(1*H*-imidazol-1-yl)phenyl)(5-(3,5-Dimethylisoxazol-4-yl)-2-methylphenyl)amino)n-pentyl)oxy)acetylamino)-3,3-dimethylbutyryl)-4-hydroxy-*N*-((*S*)-1-(4 -(4-methylthiazol-5-yl)phenyl)ethyl) pyrrolidinyl-2-formamide. LC/MS (ESI⁺) calcd for C₅₁H₆₁ClN₈O₆S ([M+H]⁺) *m*/*z:* 949.4; found 949.4.

¹H NMR (400 MHz, CDCl₃) *δ* 8.68 (s, 1H), 7.52(d, *J* = 7.8 Hz, 2H), 7.40-7.32(m, 6H), 7.19 (d, *J* = 7.7 Hz, 4H), 7.14-7.11 (m, 1H), 7.02 (s, 1H), 6.58 (s, 1H), 6.45 (s, 1H), 5.08 (s, 1H), 4.74 (s, 1H), 4.54 (s, 1H), 4.14 (s, 1H), 3.93 (s, 2H), 3.53 (d, *J=* 36.6 Hz, 6H), 2.81 (s, 1H), 2.53 (s, 3H), 2.43 (s, 3H), 2.29 (s, 3H), 2.19 (s, 3H), 1.46 (d, *J=* 6.5 Hz, 8H), 1.25 (s, 2H), 1.05 (s, 9H).

### 13: (3R,5S)-1-((S)-2-(2-((5-((3-fluoro-4-(1H-imidazol-1-yl)phenyl)(5-(3,5-dimethylisoxazol-4-yl)-2-methylphenyl)amino)n-pentyl)oxy)acetylamino)-3,3-dimethylbutyryl)-5-(((S)-1-(4-( 4-methylthiazol-5-yl)phenyl)ethyl)formylamino)pyrrolidinyl-3-acetate

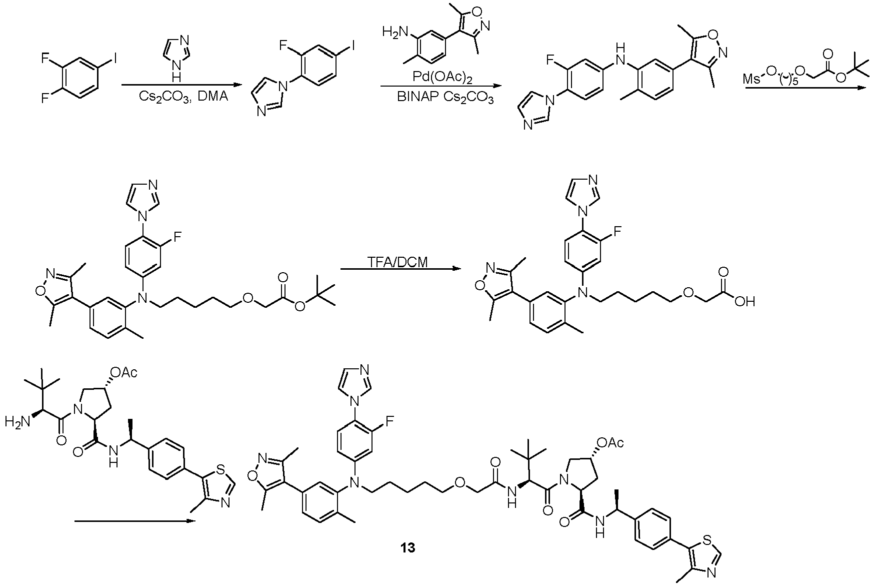

60% sodium hydride (50 mg, 1.25 mmol) was dissolved in 3 mL of dimethylsulfoxide, to which was added *N*-(3-fluoro-4-(1*H*-imidazol-1-yl)phenyl)-5-(3,5-Dimethylisoxazol-4-yl)-2-methylaniline (344 mg, 1.0 mmol), and the mixture was stirred for 10 min at room temperature, followed by addition of t-butyl 2-((5-((methanesulfonyl)oxy)n-pentyl)oxy)acetate (561 mg, 2.0 mmol). The resultant solution was allowed to react overnight at 60 °C, and then washed with 10 ml of water, followed by extraction with 10 mL of ethyl acetate. The organic layer was concentrated under reduced pressure, and the residue was separated and purified by TLC, to provide 90 mg of compound t-butyl 2-((5-((3-fluoro-4-(1H-imidazol-1-yl) phenyl)(5-(3,5-Dimethylisoxazol-4-yl)-2-methylphenyl)amino)n-pentyl)oxy)acetate, with a yield of 26%.

The compound t-butyl 2-((5-((3-fluoro-4-(1*H*-imidazol-1-yl)phenyl)(5-(3,5-dimethylisoxazol-4-yl)-2-methylphenyl)amino)n-pentyl)oxy)acetate (90 mg, 0.26 mmol), obtained in previous step, was dissolved in 3 mL of dichloromethane, to which was added 2 mL of trifluoroacetic acid, and the mixture was allowed to react at room temperature for 1 h. The reaction solution was concentrated to dryness under reduced pressure, to provide 2-((5-((3-fluoro-4-(1*H*-imidazol-1-yl)phenyl)(5-(3,5-Dimethylisoxazol-4-yl)-2-methylphenyl)amino)n-pentyl)oxy )acetic acid.

2-((5-((3-Fluoro-4-(1*H*-imidazol-1-yl)phenyl)(5-(3,5-Dimethylisoxazol-4-yl)-2-methylphenyl)a mino)n-pentyl)oxy)acetic acid was divided into two equal parts. The first part was added in 5 mL of DMF, to which were successively added diisopropylethylamine (55 mg, 0.43 mmol), HATU (35 mg, 0.09 mmol), and (3*R*,5*S*)-1-((*S*)-2-amino-3,3-dimethylbutyryl)-5-(((*S*)-1-(4-(4-methylthiazol-5-yl)phenyl)ethyl)aminomethyl)pyrrolidinyl-3-acetate (46 mg, 0.09 mmol). The mixture was allowed to react 2 h at room temperature, and then washed with 10 ml of water, followed by extraction with 10 mL of ethyl acetate. The organic layer was concentrated under reduced pressure, and the residue was separated and purified by TLC, to provide 20 mg of compound (3*R*,5*S*)-1-((*S*)-2-(2-((5-((3-fluoro-4-(1*H*-imidazol-1-yl)phenyl) (5-(3,5-Dimethylisoxazol-4-yl)-2-methylphenyl)amino)n-pentyl)oxy)acetylamino)-3,3-dimethyl butyryl)-5-(((*S*)-1-(4-(4-methylthiazol-5-yl)phenyl)ethyl)formylamino)pyrrolidinyl-3-acetate, with a yield of 23%.

LC/MS (ESI⁺) calcd for C₅₃H₆₃FN₈O₇S ([M+H]⁺) *m*/*z:* 991.4; found 975.4.

¹H NMR (400 MHz, CDCl₃) *δ* 8.68 (s, 1H), 7.96 (s, 1H), 7.52 (d, *J* = 5.3 Hz, 1H), 7.40 (d, *J* = 7.9 Hz, 1H), 7.38 - 7.30 (m, 6H), 7.16-7.13 (m, 2H), 7.03 - 7.00 (m, 2H),6.32 (d, *J=* 2.4 Hz, 1H),5.76 (d, *J* = 6.3 Hz, 1H), 5.36 (s, 1H), 5.10 - 5.06 (m, 1H), 4.71-4.65 (m, 1H), 4.61-4.56 (m, 1H), 4.06 (d, *J* = 11.9 Hz, 1H), 3.94 (q, *J* = 15.3 Hz, 2H), 3.63 (m, 1H), 3.53 (dd, *J* = 6.0, 4.3 Hz, 2H), 2.80 (s, 3H), 2.66 - 2.55 (m, 1H), 2.53 (s, 3H), 2.42 (s, 3H), 2.28 (s, 3H), 2.16(s, 3H), 2.04 (s, 3H), 179-1.63(m, 5H), 1.60-1.45 (dd, *J* = 18.4, 6.8 Hz, 4H), 1.04 (d, *J=* 8.4 Hz, 9H).

### 14: (2S,4R)-1-((S)-2-(2-((5-((3-fluoro-4-(1H-imidazol-1-yl)phenyl)(5-(3,5-dimethylisoxazol-4-yl)-2-methylphenyl)amino)n-pentyl)oxy)acetylamino)-3,3-dimethylbutyryl)-4-hydroxy-N -((S)-1-(4-(4-methylthiazol-5-yl)phenyl)ethyl)pyrrolidinyl-2-formamide

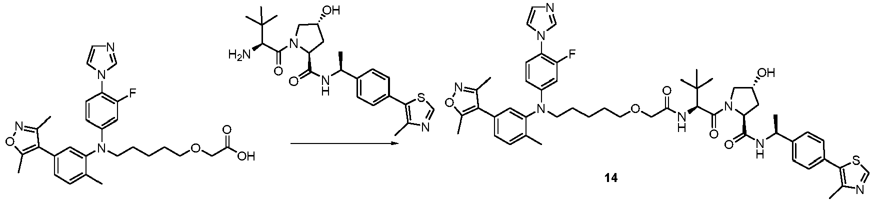

The other part 2-((5-((3-fluoro-4-(1*H*-imidazol-1-yl)phenyl)(5-(3,5-Dimethylisoxazol-4-yl)-2-methylphenyl)amino)n-pentyl)oxy)acetic acid was added in 5 mL of DMF, to which were successively added diisopropylethylamine (55 mg, 0.43 mmol), HATU (35 mg, 0.09 mmol), and (2*S*,4*R*)-1-((*S*)-2-amino-3,3-dimethylbutyryl)-4-hydroxy-*N*-((*S*)-1-(4-(4-methylthiazol-5-yl) phenyl)ethyl)-pyrrolidinyl-2-formamide (41 mg, 0.09 mmol). The mixture was allowed to react 2 h at room temperature, and then washed with 10 ml of water, followed by extraction with 10 mL of ethyl acetate. The organic layer was concentrated under reduced pressure, and the residue was separated and purified by TLC, to provide 21 mg of compound (2*S*,4*R*)-1-((*S*)-2-(2-((5-((3-fluoro-4-(1*H*-imidazol-1-yl)phenyl)(5-(3,5-Dimethylisoxazol-4-yl)-2-methylphenyl)amino)n-pentyl)oxy)acetylamino)-3,3-dimethylbutyryl)-4-hydroxy-*N*-((*S*)-1-(4 -(4-methylthiazol-5-yl)phenyl)ethyl)pyrrolidinyl-2-formamide. LC/MS (ESI⁺) calcd for C₅₁H₆₁FN₈O₆S ([M+H]⁺) *m*/*z:* 933.4; found 933.3.

### 15: (3R,5S)-1-((S)-2-(2-((5-((5-(3,5-Dimethylisoxazol-4-yl)-2-methylphenyl)(4-fluoro-3-(1H-imidazol-1-yl)phenyl)amino)phenyl)oxy)acetamido)-3,3-dimethylbutyryl)-5-(((S)-1-(4-(4-methylthiazol-5-yl)phenyl)ethyl)formylamino)pyrrolidinyl-3-acetate

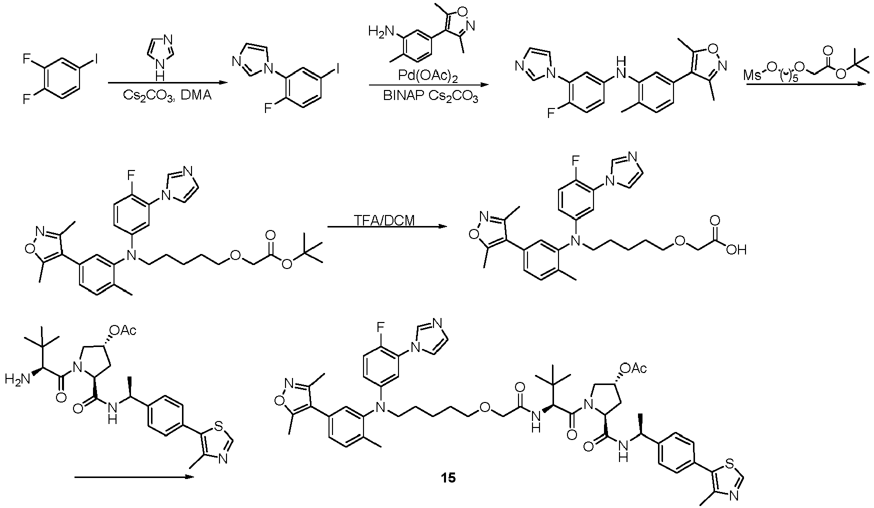

60% sodium hydride (50 mg, 1.25 mmol) was dissolved in 3 mL of dimethylsulfoxide, to which was added 5-(3,5 -Dimethylisoxazol-4-yl)-*N-*(4-fluoro-3-(1*H-*imidazol-1-yl)phenyl)-2-methylaniline (344 mg, 1.0 mmol), and the mixture was stirred for 10 min at room temperature, followed by addition of t-butyl 2-((5-((methanesulfonyl)oxy)n-pentyl)oxy)acetate (561 mg, 2.0 mmol). The resultant solution was allowed to react overnight at 60 °C, and then washed with 10 ml of water, followed by extraction with 10 mL of ethyl acetate. The organic layer was concentrated under reduced pressure, and the residue was separated and purified by TLC, to provide 90 mg of compound t-butyl 2-((5-((5-(3,5-Dimethylisoxazol-4-yl)-2-methyl phenyl)(4-fluoro-3-(1*H*-imidazol-1-yl)phenyl)amino)phenyl)oxy)acetate, with a yield of 26%. The compound t-butyl 2-((5-((5-(3,5-Dimethylisoxazol-4-yl)-2-methylphenyl)(4-fluoro-3-(1*H*-imidazol-1-yl)phenyl)amino)phenyl)oxy)acetate (90 mg, 0.26 mmol), obtained in previous step, was dissolved in 3 mL of dichloromethane, to which was added 2 mL of trifluoroacetic acid, and the mixture was allowed to react at room temperature for 1 h. The reaction solution was concentrated to dryness under reduced pressure, to provide 2-((5-((5-(3,5-Dimethylisoxazol-4-yl)-2-methylphenyl)(4-fluoro-3-(1H-imidazol-1-yl)phenyl)a mino)phenyl)oxy)acetic acid.

The obtained compound was divided into two equal parts. The first part was added in 5 mL of DMF, to which were successively added diisopropylethylamine (55 mg, 0.43 mmol), HATU (35 mg, 0.09 mmol), and (3*R*,5*S*)-1-((*S*)-2-amino-3,3-dimethylbutyryl)-5-(((*S*)-1-(4-(4-methylthiazol-5-yl)phenyl)ethyl)aminomethyl)pyrrolidinyl-3-acetate (46 mg, 0.09 mmol). The mixture was allowed to react 2 h at room temperature, and then washed with 10 ml of water, followed by extraction with 10 mL of ethyl acetate. The organic layer was concentrated under reduced pressure, and the residue was separated and purified by TLC, to provide 20 mg of compound

(3*R*,5*S*)-1-((*S*)-2-(2-((5-((5-(3,5-Dimethylisoxazol-4-yl)-2-methylphenyl)(4-fluoro-3-(1*H*-imidazol-1-yl)phenyl)amino)phenyl)oxy)acetamido)-3,3-dimethylbutyryl)-5-(((*S*)-1-(4-(4-methylthiazol-5-yl)phenyl)ethyl)formylamino)pyrrolidinyl-3-acetate, with a yield of 23%. LC/MS (ESI⁺) calcd for C₅₃H₆₃FN₈O₇S ([M+H]⁺) *m*/*z:* 991.4; found 975.3. ¹H NMR (400 MHz, CDCl₃) *δ* 8.68 (s, 1H), 7.96 (s, 1H), 7.52 (d, *J* = 5.3 Hz, 1H), 7.40 (d, *J* = 7.9 Hz, 1H), 7.38 - 7.30 (m, 6H), 7.16-7.13 (m, 2H), 7.03 - 7.00 (m, 2H),6.32 (d, *J* = 2.4 Hz, 1H),5.76 (d, *J* = 6.3 Hz, 1H), 5.36 (s, 1H), 5.10 - 5.06 (m, 1H), 4.71-4.65 (m, 1H), 4.61-4.56 (m, 1H), 4.06 (d, *J* = 11.9 Hz, 1H), 3.94 (q, *J=* 15.3 Hz, 2H), 3.63 (m, 1H), 3.53 (dd, *J=* 6.0, 4.3 Hz, 2H), 2.80 (s, 3H), 2.66 - 2.55 (m, 1H), 2.53 (s, 3H), 2.42 (s, 3H), 2.28 (s, 3H), 2.16(s, 3H), 2.04 (s, 3H), 179-1.63(m, 5H), 1.60-1.45 (dd, *J* = 18.4, 6.8 Hz, 4H), 1.04 (d, *J=* 8.4 Hz, 9H).

### 16: (2S,4R)-1-((S)-2-(2-((5-((5-(3,5-Dimethylisoxazol-4-yl)-2-methylphenyl)(4-fluoro-3-(1H-imidazol-1-yl)phenyl)amino)phenyl)oxy)acetylamino)-3,3-dimethylbutyryl)-4-hydrox y-N-((S)-1-(4-(4-methylthiazol-5-yl)phenyl)ethyl)pyrrolidinyl-2-formamide

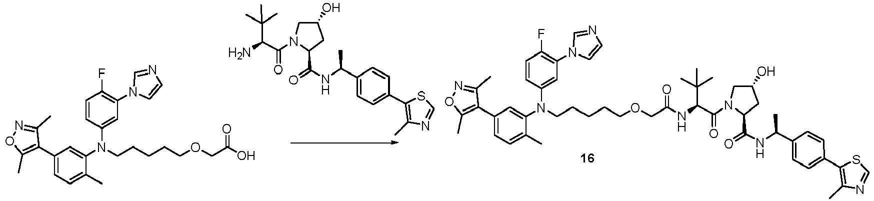

2-((5-((5-(3,5-Dimethylisoxazol-4-yl)-2-methylphenyl)(4-fluoro-3-(1H-imidazol-1-yl)phenyl)a mino)phenyl)oxy)acetic acid was dissolved in 5 mL of DMF, to which were successively added diisopropylethylamine (55 mg, 0.43 mmol), HATU (35 mg, 0.09 mmol), and (2*S*,4*R*)-1-((*S*)-2-amino-3,3-dimethylbutyryl)-4-hydroxy-*N*-((*S*)-1-(4-(4-methylthiazol-5-yl)phe nyl)ethyl)-pyrrolidinyl-2-formamide (41 mg, 0.09 mmol). The mixture was allowed to react 2 h at room temperature, and then washed with 10 ml of water, followed by extraction with 10 mL of ethyl acetate. The organic layer was concentrated under reduced pressure, and the residue was separated and purified by TLC, to provide 21 mg of compound (2*S*,4*R*)-1-((*S*)-2-(2-((5-((5-(3,5-Dimethylisoxazol-4-yl)-2-methylphenyl)(4-fluoro-3-(1*H*-imida zol-1-yl)phenyl)amino)phenyl)oxy)acetylamino)-3,3-dimethylbutyryl)-4-hydroxy-*N*-((*S*)-1-(4-( 4-methylthiazol-5-yl)phenyl)ethyl)pyrrolidinyl-2-formamide. LC/MS (ESI⁺) calcd for C₅₁H₆₁FN₈O₆S ([M+H]⁺) *m*/*z:* 933.4; found 933.3.

### 17: (3R,5S)-1-((S)-2-(2-((5-((4-(1H-imidazol-1-yl)phenyl)(3,6-dimethylbenzo[d]isoxazol-5-yl)amino)pentyl)oxy)acetamido)-3,3-dimethylbutyryl)-5-((S)-1-(4-(4-methylthiazol-5-yl) phenyl)ethyl)carbamoyl)pyrrolidine-3-yl acetate

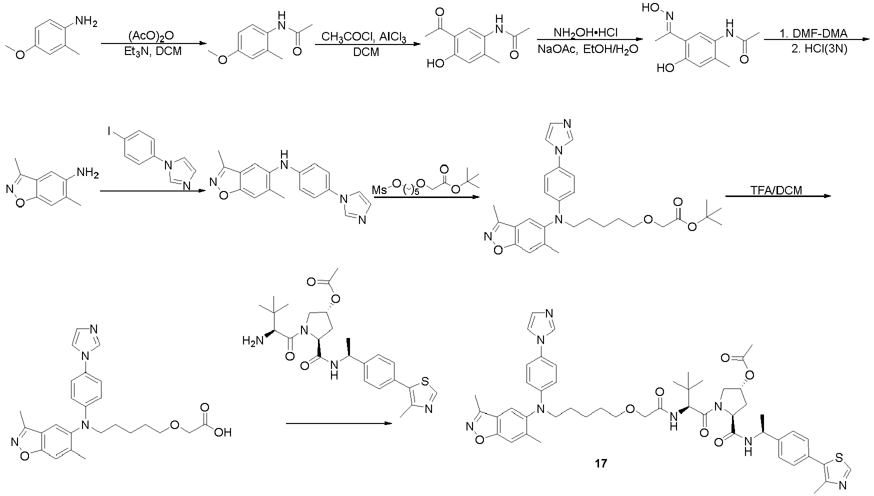

By referring to the method in the above example, the target compound was synthesized according to the above route. LC/MS (ESI⁺) calcd for C₅₀H₆₀N₈O₇S ([M+H]⁺) *m*/*z:* 916.43; found 917.4.

### 18: (3R,5S)-1-((S)-2-(2-((5-((4-(1H-imidazol-1-yl)phenyl)(3,5-dimethylbenzo[d]isoxazol-6-yl)amino)pentyl)oxy)acetamido)-3,3-dimethylbutyryl)-5-((S)-1-(4-(4-methylthiazol-5-yl) phenyl)ethyl)carbamoyl)pyrrolidine-3-yl acetate(18)

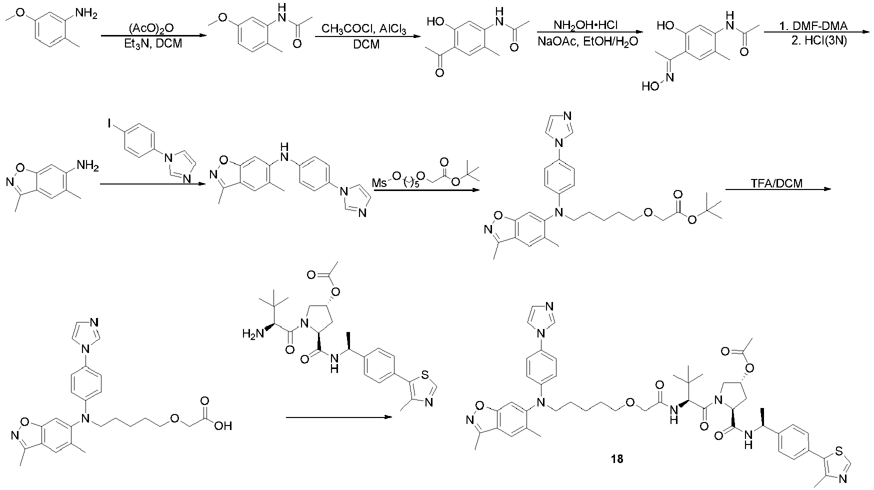

By referring to the method in the above example, the target compound was synthesized according to the above route. LC/MS (ESI⁺) calcd for C₅₀H₆₀N₈O₇S ([M+H]⁺) *m*/*z:* 916.43; found 917.3. ¹H NMR (400 MHz, CDCl₃) *δ* 8.79 (d, *J* = 5.8 Hz, 2H), 7.60 (s, 1H), 7.55 - 7.49 (m, 2H), 7.38 (dd, *J* = 9.8, 5.3 Hz, 7H), 7.13 (d, *J* = 8.7 Hz, 1H), 6.59 (d, *J* = 8.4 Hz, 2H), 6.08 (d, *J* = 8.9 Hz, 1H), 5.37 - 5.33 (m, 2H), 5.10 - 5.05 (m, 2H), 4.75 - 4.68 (m, 2H), 4.58 (d, *J* = 9.1 Hz, 2H), 3.95 (s, 2H), 3.56 - 3.51 (m, 3H), 2.60 (s, 3H), 2.05 (s, 3H), 2.04 (s, 3H), 2.02 (s, 3H), 1.48 (d, *J=* 6.8 Hz, 6H), 1.06 (s, 5H), 1.03 (s, 9H).

### 19: (2S,4R)-1-((S)-2-(2-((5-((3-bromo-4-(1H-1,2,3-triazol-1-yl)phenyl)(5-(3,5-dimethyl isoxazol-4-yl)-2-methylphenyl)amino)pentyl)oxy)acetamido)-3,3-dimethylbutyryl)-4-hydro xy-N-((S)-1-(4-methylthiazol-5-yl)phenyl)ethyl)pyrrolidine-2-formamide

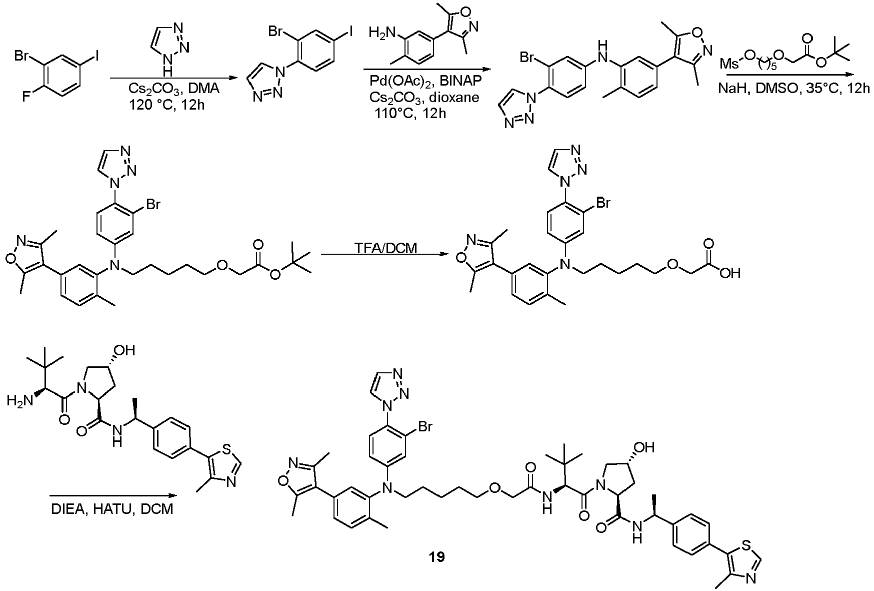

By referring to the method in the above example, the target compound was synthesized according to the above route. LC/MS (ESI⁺) calcd for C₅₀H₆₀BrN₉O₆S ([M+H]⁺) *m*/*z:* 993.36; found 994.3. ¹H NMR (400 MHz, CDCl₃) *δ* 8.82 - 8.67 (m, 2H), 7.83 (s, 2H), 7.44 (d, *J* = 8.0 Hz, 2H), 7.39 (t, *J* = 5.6 Hz, 5H), 7.20 (s, 1H), 7.18 (d, *J* = 8.4 Hz, 2H), 7.04 (s, 1H), 6.76 (d, *J* = 2.7 Hz, 1H), 6.51 (dd, *J* = 8.8, 2.7 Hz, 1H), 5.30 (s, 2H), 5.14 - 5.02 (m, 2H), 4.74 (dd, *J* = 16.1, 8.0 Hz, 2H), 4.54 (d, *J* = 8.6 Hz, 3H), 4.13 (d, *J* = 10.4 Hz, 2H), 3.76 - 3.68 (m, 2H), 3.65 - 3.57 (m, 3H), 3.18 (dt, *J* = 11.9, 7.5 Hz, 2H), 2.54 (s, 2H), 2.54 (s, 3H), 2.43 (s, 3H), 2.29 (s, 3H), 2.19 (s, 3H), 1.07 (s, 4H), 1.06 (s, 9H).

### 20: (3R,5S)-1-((S)-2-(2-((5-((3-bromo-4-(1H-1,2,3-triazol-1-yl)phenyl)(5-(3,5-dimethyl isoxazol-4-yl)-2-methylphenyl)amino)pentyl)oxy)acetamido)-3,3-dimethylbutyryl)-5-((S)-1 -(4-methylthiazol-5-yl)phenyl)ethyl)carbamoyl)pyrrolidine-3-yl acetate

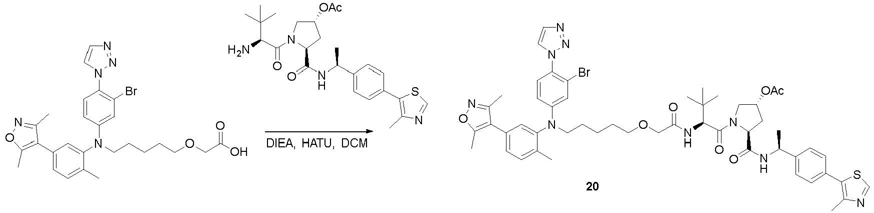

By referring to the method in the above example, the target compound was synthesized according to the above route. LC/MS (ESI⁺) calcd for C₅₂H₆₂BrN₉O₇S ([M+H]⁺) *m*/*z:* 1035.37; found 519.7. ¹H NMR (400 MHz, CDCl₃) *δ* 8.86 (s, 1H), 8.82 (t, *J* = 4.7 Hz, 1H), 7.84 (s, 2H), 7.44 (d, 7= 8.1 Hz, 1H), 7.39 (t, *J* = 5.2 Hz, 4H), 7.29 (d, *J* = 2.7 Hz, 1H), 7.20 (dd, *J* = 7.8, 1.7 Hz, 1H), 7.13 (d, *J* = 9.2 Hz, 1H), 7.04 (d, *J* = 1.6 Hz, 1H), 6.76 (d, *J* = 2.6 Hz, 1H), 6.51 (dd, *J* = 8.9, 2.7 Hz, 1H), 5.13 - 5.01 (m, 2H), 4.72 (dd, *J* = 14.5, 7.9 Hz, 2H), 4.58 (d, *J* = 9.3 Hz, 2H), 4.12 (q, *J* = 7.2 Hz, 2H), 3.83 (dd, *J* = 11.6, 4.9 Hz, 2H), 3.63 (s, 2H), 3.53 (dd, *J* = 8.3, 4.4 Hz, 4H), 3.01 (d, *J* = 2.8 Hz, 2H), 2.75 - 2.63 (m, 2H), 2.43 (s, 3H), 2.29 (s, 3H), 2.19 (s, 3H), 2.05 (s, 5H), 2.04 (s, 3H), 1.05 (s, 5H), 1.04 (s, 9H). LC/MS (ESI⁺) calcd for C₅₂H₆₂BrN₉O₇S ([M+H]⁺) m/z: 1035.37; found 519.7.

### 21: (2S,4R)-1-((S)-2-(2-((5-((4-(1H-imidazol-1-yl)-3-(trifluoromethyl)phenyl)(5-(3,5-dimethylisoxazol-4-yl)-2-methylphenyl)amino)pentyl)oxy)acetamido)-3,3-dimethylbutyryl )-4-hydroxy-N-((S)-1-(4-(4-methylthiazol-5-yl)phenyl)ethyl)pyrrolidine-2-carboxamide

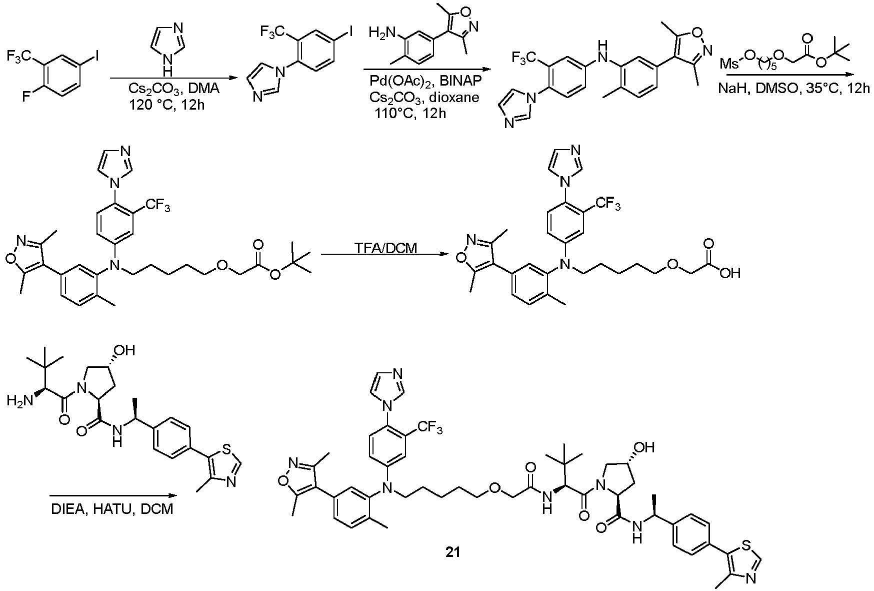

By referring to the method in the above example, the target compound was synthesized according to the above route. LC/MS (ESI⁺) calcd for C₅₂H₅₁F₃N₈O₆S ([M+H]⁺) *m*/*z:* 982.44; found 984.3. ¹H NMR (400 MHz, CDCl₃) *δ* 8.68 (s, 1H), 7.45 - 7.33 (m, 9H), 7.08 (s, 1H), 7.03 (d, *J* = 1.6 Hz, 1H), 6.77 (s, 1H), 6.67 (d, *J* = 8.8 Hz, 1H), 5.10 - 5.04 (m, 1H), 4.72 (t, *J* = 8.1 Hz, 1H), 4.53 (d, *J* = 8.4 Hz, 3H), 4.12 (d, *J* = 7.4 Hz, 2H), 3.93 (d, *J* = 2.7 Hz, 2H), 3.73 (d, *J* = 8.3 Hz, 2H), 3.64 (s, 2H), 3.22 - 3.13 (m, 2H), 2.53 (s, 2H), 2.52 (s, 3H), 2.42 (s, 3H), 2.27 (s, 3H), 2.19 (s, 3H), 1.77 (s, 3H), 1.71 - 1.62 (m, 5H), 1.45 (d, *J* = 3.2 Hz, 6H), 1.25 (s, 3H), 1.05 (s, 9H).

### 22: (3R,5S)-1-((S)-2-(2-((5-((4-(1H-imidazol-1-yl)-3-(trifluoromethyl)phenyl)(5-(3,5-dimethylisoxazol-4-yl)2-methylphenyl)amino)pentyl)oxy)acetamido)-3,3-dimethylbutyryl) -5-((S)-1-(4-methylthiazol-5-yl)phenyl)ethyl)carbamoyl)pyrrolidine-3-yl acetate

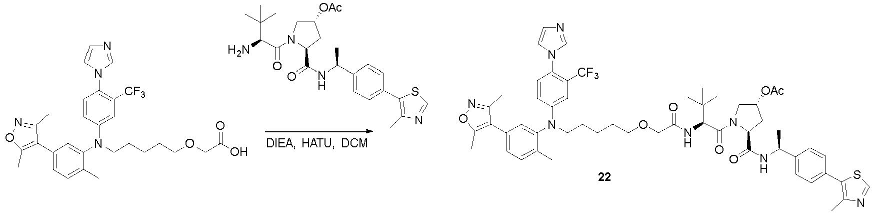

By referring to the method in the above example, the target compound was synthesized according to the above route. LC/MS (ESI⁺) calcd for C₅₄H₆₃F₃N₈O₇S ([M+H]⁺) *m*/*z:* 1024.45; found 513.3. ¹H NMR (400 MHz, CDCl₃) *δ* 8.73 (s, 1H), 7.52 (s, 1H), 7.48 (d, *J* = 8.1 Hz, 1H), 7.39 (d, *J* = 8.4 Hz, 7H), 7.24 - 7.19 (m, 1H), 7.01 (d, *J* = 8.3 Hz, 1H), 6.75 (s, 1H), 5.35 (s, 1H), 5.30 (s, 1H), 5.08 (s, 1H), 4.73 (s, 1H), 4.59 (d, *J=* 8.9 Hz, 1H), 4.06 (d, *J=* 12.1 Hz, 1H), 3.94 (s, 1H), 3.83 (s, 1H), 3.76 - 3.67 (m, 1H), 3.53 (s, 2H), 2.56 (s, 2H), 2.54 (s, 3H), 2.42 (s, 3H), 2.28 (s, 3H), 2.19 (s, 3H), 2.10 (s, 1H), 2.06 - 2.01 (m, 6H), 1.52 - 1.43 (m, 10H), 1.25 (s, 3H), 1.04 (s, 3H), 1.03 (s, 9H).

### 23: (3R,5S)-1-((S)-2-(2-(4-(2-((3-Bromo-4-(1H-imidazol-1-yl)phenyl)(5-(3,5-dimethyl isoxazol-4-yl)-2-methylphenyl)amino)ethyl)piperidin-1-yl)acetamido)-3,3-dimethylbutanol )-5-((S)-1-(4-methylthiazol-5-yl)phenyl)ethyl)carbamoyl)pyrrolidine-3-yl acetate

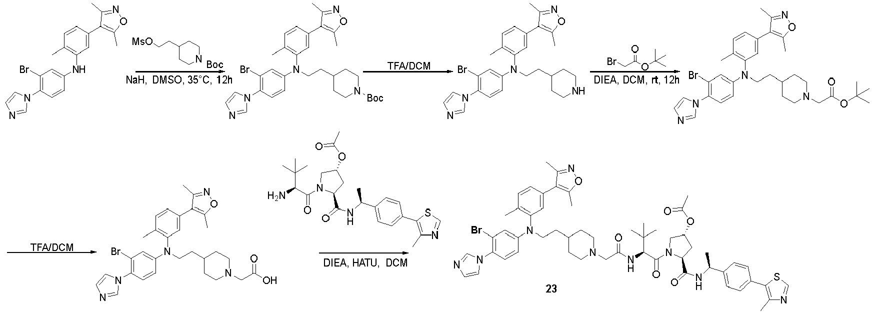

By referring to the method in the above example, the target compound was synthesized according to the above route. LC/MS (ESI⁺) calcd for C₅₆H₆₆BrN₉O₆S ([M+H]⁺) *m*/*z:* 1059.40; found 1060.4. ¹H NMR (400 MHz, CDCl₃) *δ* 8.60 (d, *J=* 6.7 Hz, 1H), 7.78 (s, 1H), 7.59 (s, 1H), 7.38 - 7.26 (m, 7H), 7.14 - 7.09 (m, 2H), 7.02 (d, *J* = 8.8 Hz, 1H), 6.99 (s, 1H), 6.94 (d, *J* = 1.7 Hz, 1H), 6.68 (d, *J* = 2.7 Hz, 1H), 6.36 (dd, *J* = 8.8, 2.6 Hz, 1H), 5.27 (s, 1H), 5.06 - 4.95 (m, 1H), 4.73 - 4.61 (m, 1H), 4.41 (d, *J* = 8.9 Hz, 1H), 4.02 (d, *J* = 11.5 Hz, 1H), 3.76 (dd, *J* = 11.6, 4.9 Hz, 1H), 3.57 (s, 2H), 2.99 (s, 2H), 2.87 (s, 2H), 2.70 - 2.56 (m, 2H), 2.46 (d, *J* = 2.4 Hz, 4H), 2.36 (s, 4H), 2.22 (s, 4H), 2.12 (s, 4H), 1.98 (d, *J=* 2.5 Hz, 6H), 1.77 - 1.56 (m, 8H), 1.41 (d, *J=* 7.1 Hz, 5H), 1.32 (s, 4H), 1.18 (s, 4H), 1.00 (s, 2H), 0.98 (s, 9H).

### 24: (2S,4R)-1-((S)-2-(2-(4-((3-Bromo-4-(1H-imidazol-1-yl)phenyl)(5-(3,5-dimethylisoxazol-4-yl)-2-methylphenyl)amino)ethyl)piperidin-1-yl)acetamido)-3,3-dimethylbutyryl)-4-hydr oxy-N-((S)-1-(4-(4-methylthiazol-5-yl)phenyl)ethyl)pyrrolidine-2-formamide

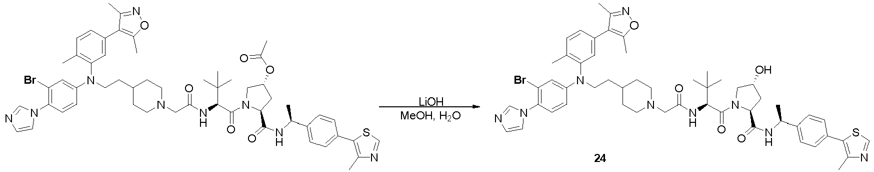

By referring to the method in the above example, the target compound was synthesized according to the above route. LC/MS (ESI⁺) calcd for C₅₃H₆₄BrN₉O₅S ([M+H]⁺) *m*/*z:* 1017.39; found 509.6.

### 25: 2-((5-((3-Bromo-4-(1H-imidazol-1-yl)phenyl)(5-(3,5-Dimethylisoxazol-4-yl)-2-methyl phenyl)amino)pentyl)oxy)-N-(2-(2,6-dioxopiperidin-3-yl)-1-oxoisoindol-4-yl)acetamide

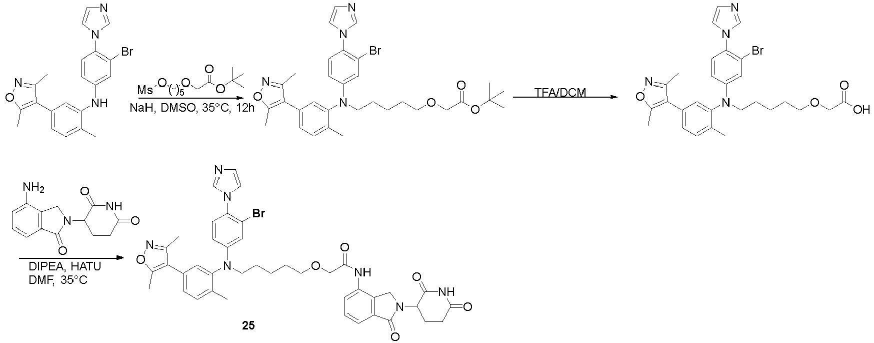

By referring to the method in the above example, the target compound was synthesized according to the above route. LC/MS (ESI⁺) calcd for C₄₁H₄₂BrN₇O₆ ([M+H]⁺) *m*/*z:* 807.24; found 808.2. ¹H NMR (400 MHz, CDCl₃) *δ* 8.31 (s, 1H), 8.23 (s, 1H), 7.84 (s, 1H), 7.66 (d, *J* = 7.2 Hz, 1H), 7.59 (d, *J* = 8.1 Hz, 1H), 7.46 - 7.33 (m, 3H), 7.17 (s, 2H), 7.12 (dd, *J* = 7.8, 1.7 Hz, 2H), 7.07 (d, *J* = 8.9 Hz, 1H), 7.03 (s, 1H), 6.96 (d, *J* = 1.6 Hz, 1H), 6.64 (d, *J* = 2.5 Hz, 1H), 6.44 (dd, *J* = 9.0, 2.5 Hz, 1H), 5.13 (dd, *J* = 13.2, 5.0 Hz, 1H), 4.37 (s, 3H), 4.03 (s, 3H), 3.56 (t, *J=* 6.5 Hz, 6H), 3.42 (s, 3H), 2.35 (s, 5H), 2.21 (s, 5H), 2.12 (s, 7H), 1.68 (dd, *J=* 14.4, 7.2 Hz, 8H), 1.48 - 1.33 (m, 4H).

### 26: (2S,4R)-1-((S)-2-(2-((5-((4-Bromophenyl)(5-(3,5-Dimethylisoxazol-4-yl)-2-methyl phenyl)amino)pentyl)oxy)acetamido)-3,3-dimethylbutyryl)-4-hydroxy-N-((S)-1-(4-(4-meth ylthiazol-5-yl)phenyl)ethyl)pyrrolidine-2-formamide

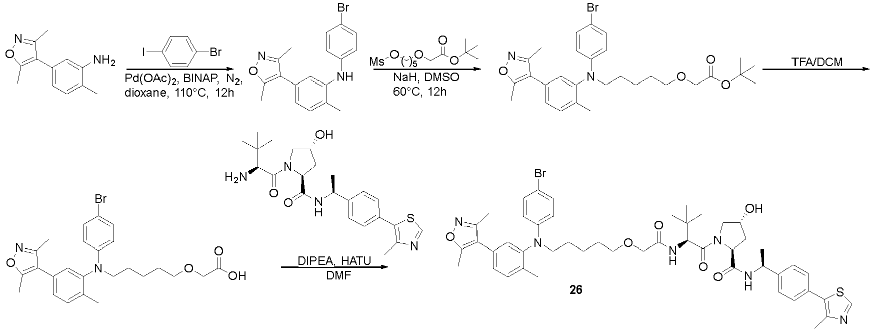

By referring to the method in the above example, the target compound was synthesized according to the above route. LC/MS (ESI⁺) calcd for C₄₈H₅₉BrN₆O₆S ([M+H]⁺) *m*/*z:* 926.34; found 927.2. ¹H NMR (400 MHz, CDCl₃) *δ* 8.89 (s, 1H), 7.33 (s, 3H), 7.31 (d, *J=* 4.0 Hz, 3H), 7.28 (d, *J* = 3.1 Hz, 1H), 7.15 (dd, *J* = 8.7, 3.5 Hz, 3H), 7.04 (d, *J* = 7.7 Hz, 1H), 6.94 (s, 1H), 6.30 (d, *J* = 9.0 Hz, 2H), 5.09 - 4.92 (m, 1H), 4.67 (s, 1H), 4.46 (d, *J* = 6.4 Hz, 2H), 4.14 - 4.01 (m, 4H), 3.98 (d, *J=* 6.7 Hz, 2H), 3.60 - 3.38 (m, 8H), 2.48 (s, 4H), 2.33 (s, 3H), 2.20 (s, 4H), 2.05 (s, 4H), 1.39 (d, *J=* 6.6 Hz, 5H), 1.19 (d, *J=* 3.6 Hz, 6H), 0.99 (d, *J=* 4.3 Hz, 10H).

### 27: 2-((5-((4-Bromophenyl)(5-(3,5-Dimethylisoxazol-4-yl)-2-methylphenyl)amino)pentyl) oxy)-N-(2-(2,6-dioxopiperidin-3-yl)-1-oxoisoindolin-4-yl)acetamide

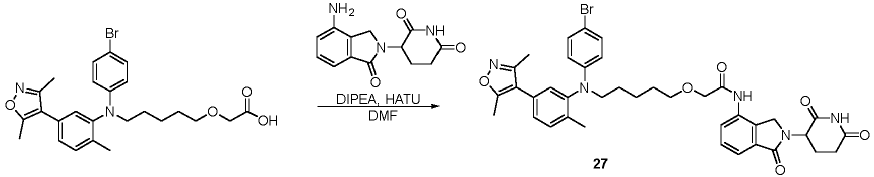

By referring to the method in the above example, the target compound was synthesized according to the above route. LC/MS (ESI⁺) calcd for C₃₈H₄₀BrN₅O₆ ([M+H]⁺) *m*/*z:* 741.22; found 742.2. ¹H NMR (400 MHz, CDCl₃) *δ* 8.67 (s, 0H), 7.70 (t, *J* = 7.9 Hz, 1H), 7.29 (d, *J* = 7.5 Hz, 1H), 7.15 (dd, *J* = 8.9, 4.5 Hz, 2H), 7.04 (d, *J=* 5.9 Hz, 1H), 6.93 (d, *J* = 3.6 Hz, 1H), 6.30 (d, *J* = 8.9 Hz, 1H), 4.37 (s, 1H), 4.15 - 3.94 (m, 7H), 3.59 - 3.36 (m, 6H), 2.33 (s, 3H), 2.20 (s, 3H), 2.05 (s, 3H), 1.39 (d, *J* = 11.9 Hz, 7H), 1.18 (s, 8H).

### 28: 1-(4-((5-(3,5-Dimethylisoxazol-4-yl)-2-methylphenyl)(2-(1-(2-(2,6-dioxopiperidin-3-yl)-1,3-dioxoisoindolin-5-yl)piperidin-4-yl)ethyl)amino)phenyl)cyclopropane-1-carbonitrile

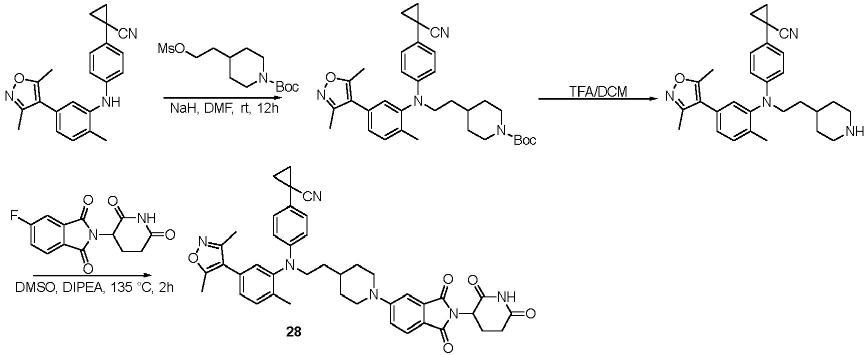

By referring to the method in the above example, the target compound was synthesized according to the above route. LC/MS (ESI⁺) calcd for C₄₂H₄₂N₆O₅ ([M+H]⁺) *m*/*z:* 710.32; found 711.3. ¹H NMR (400 MHz, DMSO-*d*₆) *δ* 11.09 (s, 1H), 7.64 (d, *J* = 8.5 Hz, 1H), 7.45 (d, *J=* 7.9 Hz, 1H), 7.35 - 7.18 (m, 3H), 7.17 - 7.08 (m, 3H), 6.45 (d, *J=* 8.8 Hz, 2H), 5.06 (dd, *J* = 12.9, 5.4 Hz, 1H), 3.65 (s, 2H), 2.92 (t, *J* = 12.3 Hz, 3H), 2.39 (s, 3H), 2.22 (s, 3H), 2.07 (s, 3H), 1.76 (d, *J* = 11.5 Hz, 2H), 1.59 (dd, *J=* 7.2, 4.6 Hz, 4H), 1.31 (q, *J* = 4.8 Hz, 2H).

### 29: 1-(4-((5-(3,5-Dimethylisoxazol-4-yl)-2-methylphenyl)(2-(1-(2-(2,6-dioxopiperidin-3-yl)-6-fluoro-1,3-dioxoisoindolin-5-yl)piperidin-4-yl)ethyl)amino)phenyl)cyclopropane-1-carbo nitrile

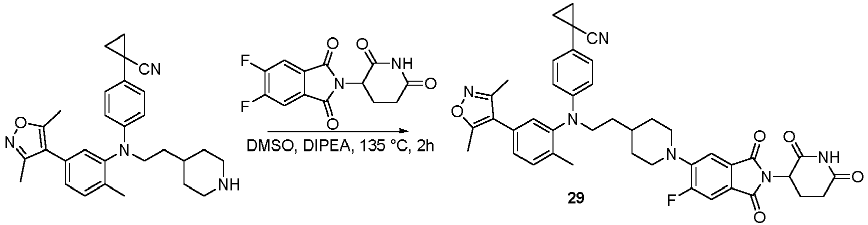

By referring to the method in the above example, the target compound was synthesized according to the above route. LC/MS (ESI⁺) calcd for C₄₂H₄₁FN₆O₅ ([M+H]⁺) *m*/*z:* 728.31; found 729.3. ¹H NMR (400 MHz, DMSO-*d*₆) *δ* 11.11 (s, 1H), 7.70 (d, *J* = 11.5 Hz, 1H), 7.48 - 7.40 (m, 2H), 7.27 (d, *J* = 7.8 Hz, 1H), 7.18 - 7.09 (m, 3H), 6.47 (d, *J* = 8.8 Hz, 2H), 5.10 (dd, *J* = 12.6, 5.3 Hz, 1H), 3.67 (s, 2H), 3.58 (d, *J* = 11.3 Hz, 2H), 2.84 (t, *J* = 12.3 Hz, 3H), 2.40 (s, 3H), 2.23 (s, 3H), 2.08 (s, 3H), 1.80 (d, *J=* 12.2 Hz, 2H), 1.66 - 1.49 (m, 5H), 1.31 (d, *J* = 2.4 Hz, 4H).

### 30: 1-(4-((5-(3,5-Dimethylisoxazol-4-yl)-2-methylphenyl)(3-(1-(2-(2,6-dioxopiperidin-3-yl) -1,3-dioxoisoindolin-5-yl)piperidin-4-yl)propyl)amino)phenyl)cyclopropane-1-nitrile

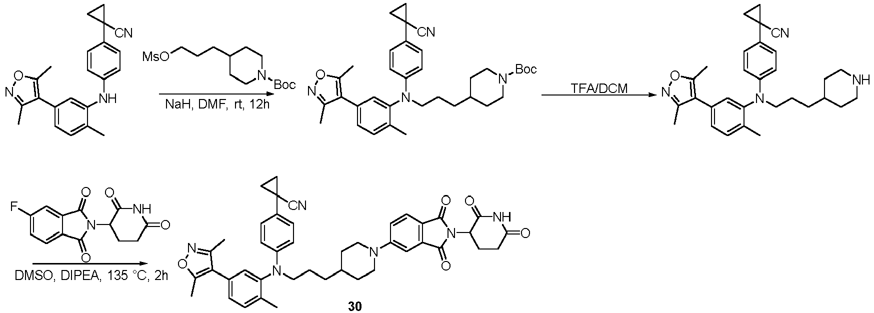

By referring to the method in the above example, the target compound was synthesized according to the above route. LC/MS (ESI⁺) calcd for C₄₃H₄₄N₆O₅ ([M+H]⁺) *m*/*z:* 724.34; found 725.3. ¹H NMR (400 MHz, CDCl₃) *δ* 8.01 (s, 1H), 7.74 (d, *J* = 8.5 Hz, 1H), 7.41 (d, *J* = 8.1 Hz, 2H), 7.15 (dd, *J=* 12.1, 5.3 Hz, 3H), 7.03 (d, *J=* 1.7 Hz, 1H), 6.47 (d, *J=* 8.8 Hz, 2H), 5.05 - 4.91 (m, 2H), 4.19 - 4.05 (m, 2H), 3.93 (d, *J* = 13.1 Hz, 2H), 3.66 - 3.54 (m, 2H), 3.01 (t, *J* = 11.7 Hz, 3H), 2.96 - 2.69 (m, 5H), 2.43 (s, 3H), 2.29 (s, 3H), 2.16 (s, 3H), 2.07 (s, 2H), 1.84 (d, *J=* 12.3 Hz, 3H), 1.79 - 1.70 (m, 4H), 1.63 (dd, *J=* 7.4, 4.8 Hz, 4H), 1.45 (d, *J=* 10.7 Hz, 3H), 1.40 - 1.34 (m, 2H), 1.29 (dd, *J=* 13.8, 6.7 Hz, 4H).

### 31: 1-(4-((5-(3,5-Dimethylisoxazol-4-yl)-2-methylphenyl)(3-(1-(2-(2,6-dioxopiperidin-3-yl)-3-oxoisoindolin-5-yl)piperidin-4-yl)propyl)amino)phenyl)cyclopropane-1-nitrile

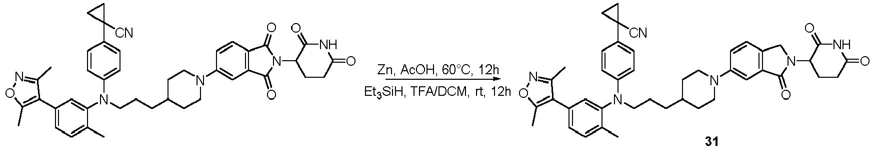

By referring to the method in the above example, the target compound was synthesized according to the above route. LC/MS (ESI⁺) calcd for C₄₃H₄₆N₆O₄ ([M+H]⁺) *m*/*z:* 710.36; found 711.3.

### 32: 1-(4-((5-(3,5-Dimethylisoxazol-4-yl)-2-methylphenyl)(3-(1-(2-(2,6-dioxopiperidin-3-yl)-1-oxoisoindolin-5-yl)piperidin-4-yl)propyl)amino)phenyl)cyclopropane-1-nitrile

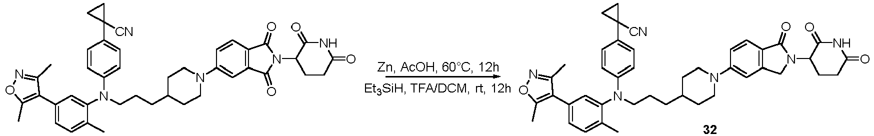

By referring to the method in the above example, the target compound was synthesized according to the above route. LC/MS (ESI⁺) calcd for C₄₃H₄₆N₆O₄ ([M+H]⁺) *m*/*z:* 710.36; found 711.3.

### 33: 3-(5-((5-((5-(3,5-Dimethylisoxazol-4-yl)-2-methylphenyl) (4-(3,5-dimethylisoxazol-4-yl) phenyl)amino)pentyl)amino)-1-oxoisoindolin-2-yl)piperidine-2,6-dione

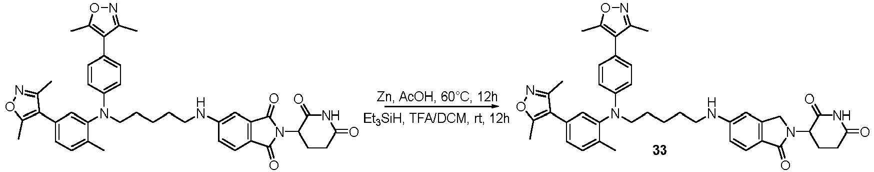

By referring to the method in the above example, the target compound was synthesized according to the above route. LC/MS (ESI⁺) calcd for C₄₁H₄₄N₆O₅ ([M+H]⁺) *m*/*z:* 700.34; found 701.3. ¹H NMR (400 MHz, CDCl₃) *δ* 9.39 - 9.28 (m, 1H), 8.28 (s, 1H), 7.98 (s, 1H), 7.67 (d, *J=* 8.4 Hz, 1H), 7.42 (d, *J=* 7.8 Hz, 1H), 7.15 (d, *J=* 7.9 Hz, 1H), 7.08 (s, 1H), 6.76 (s, 1H), 6.66 (d, *J* = 7.9 Hz, 1H), 6.58 (d, *J* = 8.5 Hz, 2H), 6.45 (d, *J* = 8.6 Hz, 1H), 5.22 (d, *J* = 7.7 Hz, 2H), 4.30 - 4.21 (m, 2H), 3.66 (s, 2H), 3.58 (s, 1H), 3.47 (s, 3H), 3.27 (s, 2H), 3.25 - 3.16 (m, 2H), 3.04 (s, 1H), 2.48 - 2.37 (m, 5H), 2.33 - 2.25 (m, 5H), 2.21 (s, 2H), 1.92 (s, 2H), 1.81 (s, 2H), 1.73 (s, 2H), 1.28 (s, 6H).

### 34: 1-(4-((5-(3,5-Dimethylisoxazol-4-yl)-2-methylphenyl) (1-((1-(2-(2,6-dioxopiperidin-3-yl)-1,3-dioxoisoindolin-5-yl)piperidin-4-yl)methyl)azetidin-3-yl)amino)phenyl)cyclopropan e-1-nitrile

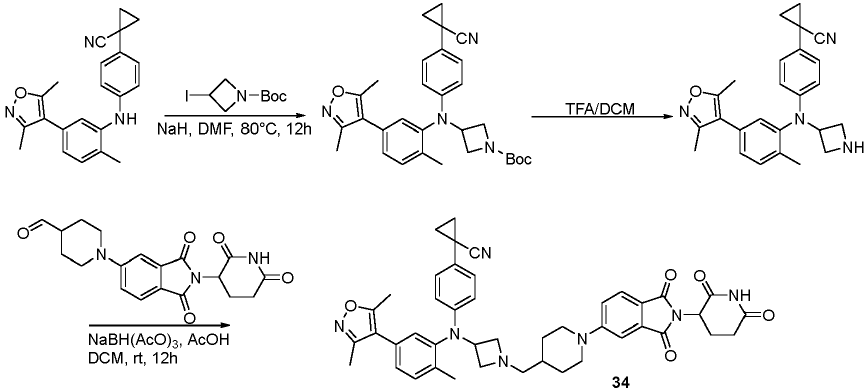

By referring to the method in the above example, the target compound was synthesized according to the above route. LC/MS (ESI⁺) calcd for C₄₄H₄₅N₇O₅ ( [M+H]⁺ ) *m*/*z*: 751.35; found 752.3. ¹H NMR (400 MHz, CDCl₃) *δ* 8.01 (s, 1H), 7.87 (d, *J* = 7.5 Hz, 1H), 7.69 (d, *J* = 8.5 Hz, 1H), 7.44 (dd, *J* = 15.4, 8.0 Hz, 2H), 7.15 (d, *J* = 8.6Hz, 2H), 7.03 (d*, J* = 10.1 Hz, 1H), 6.95 (s, 1H), 6.40 (d, *J* = 8.7 Hz, 1H), 5.00 - 4.91 (m, 1H), 4.86 (s, 1H), 4.24 (s, 2H), 3.95 (d, *J* = 11.9 Hz, 2H), 3.17 (s, 2H), 2.95 (dd, *J=* 26.9, 14.6 Hz, 3H), 2.81 (dd, *J* = 32.6, 13.9 Hz, 2H), 2.62 (s, 2H), 2.42 (s, 3H), 2.28 (s, 3H), 2.23 (s, 3H), 2.15 (s, 1H), 1.90 (d, *J =* 14.2 Hz, 3H), 1.65 (s, 2H), 1.36 (s, 2H), 0.90 (s, 2H).

### 35: 1-(4-((5-(3,5-Dimethylisoxazol-4-yl)-2-methylphenyl)(1-(2-(2,6-dioxopiperidin-3-yl)-1,3-dioxoisoindolin-5-yl)azetidin-3-yl)amino)phenyl)cyclopropane-1-nitrile

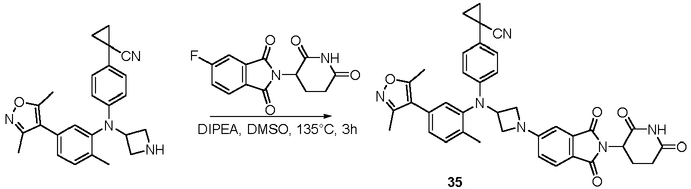

By referring to the method in the above example, the target compound was synthesized according to the above route. LC/MS (ESI⁺) calcd for C₃₈H₃₄N₆O₅ ( [M+H]⁺ ) *m*/*z*: 654.26; found 655.3.

### 36: 1-(4-((5-(3,5-Dimethylisoxazol-4-yl)-2-methylphenyl)(2-((1-(2-(2,6-dioxopiperidin-3-yl)-1,3-dioxoisoindolin-5-yl)pyrrolidin-3-yl)oxy)ethyl)amino)phenyl)cyclopropane-1-nitrile

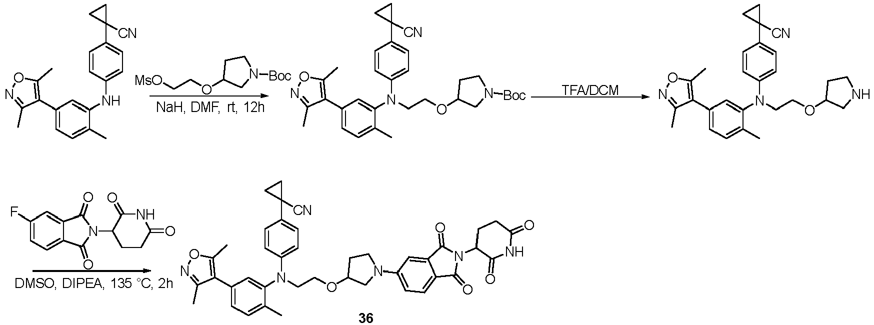

By referring to the method in the above example, the target compound was synthesized according to the above route. LC/MS (ESI⁺) calcd for C₄₁H₄₀N₆O₆ ( [M+H]⁺ ) *m*/*z*: 712.30; found 713.3.

### 37: 1-(4-((5-(3,5-Dimethylisoxazol-4-yl)-2-methylphenyl)(3-((2-(2,6-dioxopiperidin-3-yl)-6-fluoro-1,3-dioxoisoindolin-5-yl)amino)cyclobutyl)methyl)amino)phenyl)cyclopropane-1-nitrile

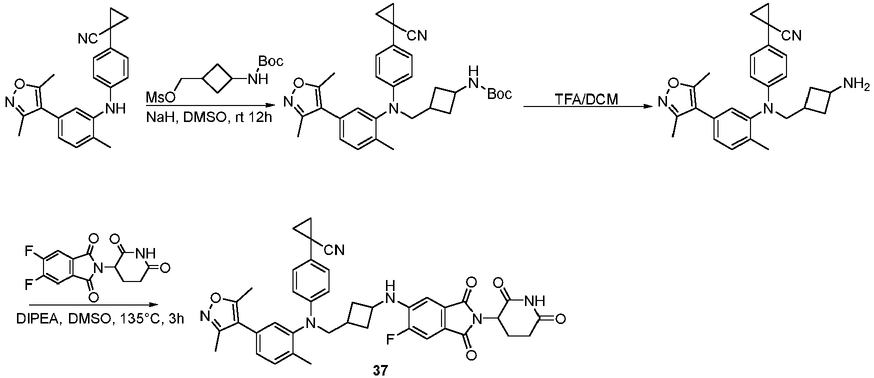

By referring to the method in the above example, the target compound was synthesized according to the above route. LC/MS (ESI⁺) calcd for C₄₀H₃₇FN₆O₅ ( [M+H]⁺ ) *m*/*z*: 700.28; found 701.2. ¹H NMR (400 MHz, CDCl₃) *δ* 8.07 (s, 1H), 7.42 (t, *J* = 9.3 Hz, 2H), 7.15 (t, *J* = 8.1 Hz, 3H), 7.04 (s, 1H), 6.86 (d, *J* = 7.0 Hz, 1H), 6.50 (d, *J* = 8.8 Hz, 2H), 4.93 (dd, *J* = 12.2, 5.1 Hz, 1H), 4.83 (s, 1H), 4.14 (dt, *J* = 12.5, 6.2 Hz, 2H), 3.82 (d, *J* = 7.4 Hz, 2H), 2.96-2.86 (m, 2H), 2.79 (dd, *J* = 17.9, 8.3 Hz, 2H), 2.43 (s, 3H), 2.29 (s, 3H), 2.14 (s, 3H), 2.07 (s, 2H), 1.63 (dd, *J* = 7.3, 4.8 Hz, 4H), 1.30 (d, *J* = 3.4 Hz, 2H).

### 38: 1-(4-((5-(3,5-Dimethylisoxazol-4-yl)-2-methylphenyl)(3-((2-(2,6-dioxopiperidin-3-yl)-6-fluoro-3-oxoisoindolin-5-yl)amino)cyclobutyl)methyl)phenyl)cyclopropane-1-carbonate

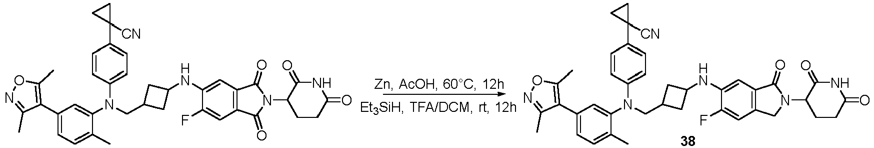

By referring to the method in the above example, the target compound was synthesized according to the above route. LC/MS (ESI⁺) calcd for C₄₀H₃₉FN₆O₄ ( [M+H]⁺) *m*/*z*: 686.30; found 687.3.

### 39: 1-(4-((5-(3,5-Dimethylisoxazol-4-yl)-2-methylphenyl)(3-((2-(2,6-dioxopiperidin-3-yl)-6-fluoro-1-oxoisoindolin-5-yl)amino)cyclobutyl)methyl)amino)phenyl)cyclopropane-1-nitrile

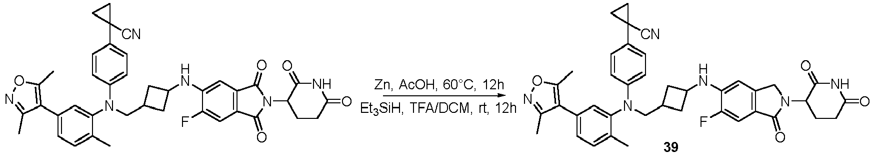

By referring to the method in the above example, the target compound was synthesized according to the above route. LC/MS (ESI+) calcd for C₄₀H₃₉FN₆O₄ ( [M+H]⁺) m/z: 686.30; found 687.3_{∘}

### 40: 1-(4-((5-(3,5-Dimethylisoxazol-4-yl)-2-methylphenyl)(4-((2-(2,6-dioxopiperidin-3-yl)-6-fluoro-1,3-dioxoisoindolin-5-yl)amino)butyl)amino)phenyl)cyclopropane-1-nitrile

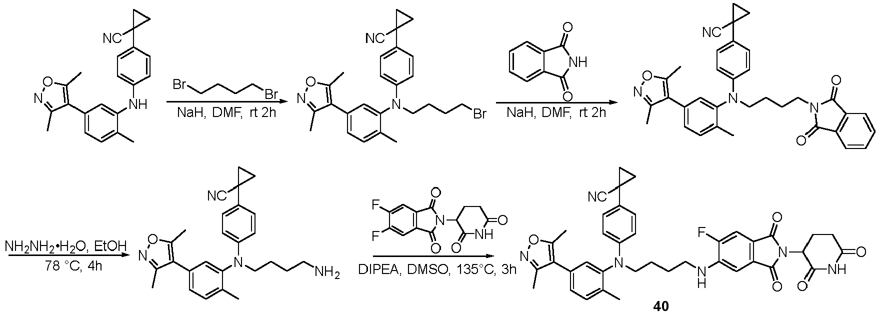

By referring to the method in the above example, the target compound was synthesized according to the above route. LC/MS (ESI⁺) calcd for C₃₉H₃₇FN₆O₅ ( [M+H]⁺ ) *m*/*z*: 688.28; found 689.2.

### 41: 1-(4-((5-(3,5-Dimethylisoxazol-4-yl)-2-methylphenyl)(4-((2-(2,6-dioxopiperidin-3-yl)-1,3-dioxoisoindolin-5-yl)amino)butyl)amino)phenyl)cyclopropane-1-nitrile

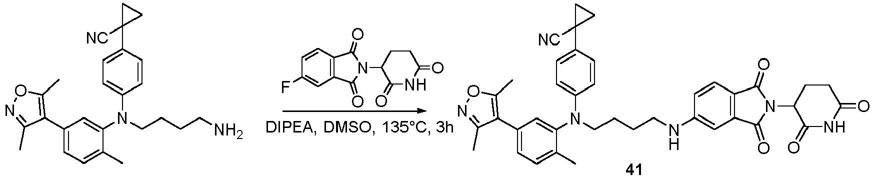

By referring to the method in the above example, the target compound was synthesized according to the above route. LC/MS (ESI⁺) calcd for C₃₉H₃₈N₆O₅ ( [M+H]⁺ ) *m*/*z*: 670.29; found 671.3. ¹H NMR (400 MHz, CDCl₃) *δ* 7.97 (s, 1H), 7.63 (d, *J* = 8.2 Hz, 1H), 7.41 (d, *J* = 7.9 Hz, 1H), 7.14 (t, *J* = 8.6 Hz, 2H), 7.03 (s, 1H), 6.96 (s, 1H), 6.74 (d, *J* = 8.6 Hz, 1H), 6.48 (d, *J* = 8.9 Hz, 2H), 5.00 - 4.91 (m, 1H), 3.66 (d, *J* = 7.5 Hz, 2H), 3.29 (t, *J* = 6.8 Hz, 2H), 2.96 - 2.69 (m, 4H), 2.40 (d, *J* = 10.8 Hz, 3H), 2.26 (d, *J* = 14.3 Hz, 3H), 2.14 (d, *J* = 13.1 Hz, 3H), 1.78 (d, *J* = 27.4 Hz, 5H), 0.90 (s, 3H).

### 42: 1-(4-((5-(3,5-Dimethylisoxazol-4-yl)-2-methylphenyl)(4-((2-(2,6-dioxopiperidin-3-yl)-6-fluoro-3-oxoisoindolin-5-yl)amino)butyl)amino)phenyl)cyclopropane-1-nitrile

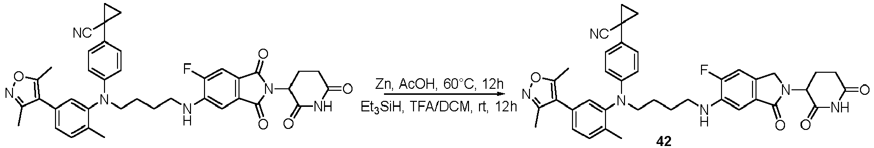

By referring to the method in the above example, the target compound was synthesized according to the above route. LC/MS (ESI⁺) calcd for C₃₉H₃₉FN₆O₄ ( [M+H]⁺) *m*/*z*: 674.30; found 675.3. ¹H NMR (400 MHz, CDCl₃) *δ* 8.01 (s, 1H), 7.98 (s, 1H), 7.74 - 7.68 (m, 1H), 7.40 (d, *J* = 7.9 Hz, 1H), 7.31 (s, 1H), 7.12 (d, *J* = 8.7 Hz, 3H), 7.02 (s, 1H), 6.48 (d, *J* = 8.8 Hz, 2H), 5.25 - 5.16 (m, 2H), 4.36 (s, 2H), 3.67 - 3.61 (m, 2H), 3.27 (s, 2H), 2.96 - 2.89 (m, 2H), 2.41 (s, 3H), 2.27 (s, 3H), 2.15 (s, 3H), 2.06 (d, *J* = 3.9 Hz, 1H), 1.92 (s, 1H), 1.76 (d, *J* = 6.4 Hz, 5H), 1.62 (d, *J* = 2.5 Hz, 3H), 1.30 (d, *J* = 2.5 Hz, 3H).

### 43: 1-(4-((5-(3,5-Dimethylisoxazol-4-yl)-2-methylphenyl)(4-((2-(2,6-dioxopiperidin-3-yl)-6-fluoro-1-oxoisoindolin-5-yl)amino)butyl)amino)phenyl)cyclopropane-1-nitrile(43)

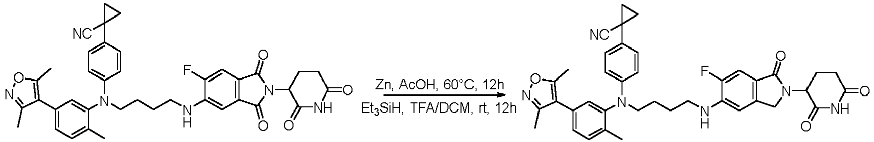

By referring to the method in the above example, the target compound was synthesized according to the above route. LC/MS (ESI⁺) calcd for C₃₉H₃₉FN₆O₄ ( [M+H]⁺) *m*/*z*: 674.30; found 675.3. ¹H NMR (400 MHz, CDCl₃) *δ* 8.19 (s, 1H), 7.44 (d, *J* = 10.4 Hz, 1H), 7.40 (d, *J* = 7.8 Hz, 1H), 7.13 (t, *J* = 9.4 Hz, 3H), 7.03 (s, 1H), 6.62 (d, *J* = 7.0 Hz, 1H), 6.48 (d, *J* = 8.6 Hz, 2H), 5.19 (d, *J* = 8.2 Hz, 2H), 4.37 (d, *J* = 15.4 Hz, 1H), 4.23 (d, *J* = 15.7 Hz, 1H), 3.71 - 3.62 (m, 2H), 3.26 (d, *J* = 6.5 Hz, 2H), 2.99 - 2.75 (m, 3H), 2.41 (s, 3H), 2.28 (s, 3H), 2.15 (s, 3H), 1.83 (d, *J* = 6.2 Hz, 2H), 1.77 (d, *J* = 6.6 Hz, 2H), 1.62 (d, *J* = 2.2 Hz, 2H), 1.32 - 1.25 (m, 3H).

### 44: (2S,4R)-1-((S)-2-(2-((5-((3-chloro-4-cyanophenyl)(5-(3,5-dimethyl-1H-pyrazol-4-yl)-2-methylphenyl)amino)pentyl)oxy)acetylamino)-3,3-dimethylbutyryl)-4-hydroxy-N-((S)-1-(4-(4-methylthiazol-5-yl)phenyl)ethyl)pyrrolidinyl-2-formamide (44)

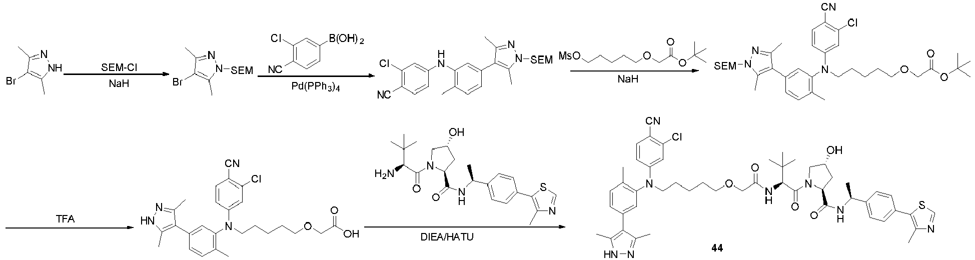

Sodium hydride (4.8 g, 120 mmol) was added to 150 mL of tetrahydrofuran, to which was added 3,5-dimethyl-4-bromopyrazole (10.5 g, 60 mmol) in an ice bath, and the solution was stirred for 30 min, followed by addition of SEM-Cl (10.0 g, 60 mmol). The reaction solution was slowly warmed to room temperature, and stir overnight. The reaction solution was poured to 500 mL of ice water, and extracted with 300 mL of ethyl acetate. The aqueous layer was extracted again with 100 mL of ethyl acetate. The organic layers were combined, washed with saturated brine, dried over anhydrous sodium sulfate, concentrated, and purified by column chromatography, to provide the product 4-bromo-3,5-dimethyl-1-((2-(trimethylsilyl)ethoxy) methyl)-1H-pyrazole (17 g), with a yield of 93%, LC/MS (ESI⁺) calcd for C₁₁H₂₁BrN₂OSi ([M+H]⁺) *m*/*z* 305.1.

### 4-Bromo-3,5-dimethyl-1-((2-(trimethylsilyl)ethoxy)methyl)-1H-pyrazole (7.29 g, 23.9 mmol) and (3-chloro-4-cyanophenyl)boric acid (3.97 g , 26.3 mmol) were dissolved in 180 mL mixed solution of toluene/water (1:1, v/v), to which were successively added potassium carbonate (8.3 g, 60 mmol) and tetrakis(triphenylphosphine)palladium (1.39 g, 1.2 mmol), and then under argon protection, the mixture was allowed to react overnight at 95 °C. The reaction solution was extracted with 100 mL of ethyl acetate, and the water layer was further extracted with 80 mL of ethyl acetate once. The organic layers were combined, washed with saturated brine, dried over anhydrous sodium sulfate, concentrated, and purified by column chromatography, to provide 2-chloro-4-((5-(3,5-dimethyl-1-((2-(trimethylsilyl)ethoxy)methyl)-1H-pyrazol-4-yl)-2-methylp henyl)amino)benzonitrile (5.05 g), with a yield of 63%. LC/MS (ESI⁺) calcd for C₂₅H₃₁ClN₄OSi ([M+H]⁺) m/z 467.2.

NaH (40 mg, 1.0 mmol) was added to 5 mL of tetrahydrofuran, to which was added 2-chloro-4-((5-(3,5-trimethylsilyl)ethoxy)methyl)-1H-pyrazol-4-yl)-2-methylphenyl)amino)ben zonitrile (233 mg, 1.0 mmol), and the mixture was stirred and reacted 30 min at room temperature, to which was added 2-t-butyl (4-((methanesulfonyl)oxy)butoxy)acetate (140 mg, 0.5 mmol). The mixture was allowed to react overnight at room temperature, and then washed with 10 ml of water, followed by extraction with 10 mL of ethyl acetate. The organic layer was concentrated under reduced pressure, and the residue was separated and purified by TLC, to provide t-butyl 2-((5-((3-chloro-4-cyanophenyl)(5-(3,5-dimethyl-1-((2-(trimethylsilyl)ethoxy) methyl)-1H-pyrazol-4-yl)-2-methylphenyl)amino)pentyl)oxy)acetate (260 mg), with a yield of 78%. LC/MS (ESI⁺) calcd for C₃₆H₅₁ClN₄O₄Si ([M+H]⁺) *m*/*z* 667.4.

2-((5-((3-Chloro-4-cyanophenyl)(5-(3,5-dimethyl-1-((2-(trimethylsilyl)ethoxy)methyl)-1H-pyra zol-4-yl)-2-methylphenyl)amino)pentyl)oxy)acetate (260 mg, 0.39 mmol) was dissolved in 3 mL of dichloromethane, to which was added 3 mL of trifluoroacetic acid, and the mixture was allowed to react at room temperature for 1 h. The reaction solution was concentrated to dryness under reduced pressure. The residue was washed with the saturated aqueous solution of sodium bicarbonate, and extracted with ethyl acetate. The organic layer was washed with saturated brine, dried over anhydrous sodium sulfate, and concentrated under reduced pressure, to provide crude 2-((5-((3-chloro-4-cyanophenyl)(5-(3,5-dimethyl-1-((2-(trimethylsilyl)ethoxy) methyl)-1H-pyrazol-4-yl)-2-methylphenyl)amino)pentyl)oxy)acetic acid (200 mg).

2-((5-((3-Chloro-4-cyanophenyl)(5-(3,5-dimethyl-1-((2-(trimethylsilyl)ethoxy)methyl)-1H-pyra zol-4-yl)-2-methylphenyl)amino)pentyl)oxy)acetic acid (100 mg, 0.75 mmol) was dissolved in 5 mL of DMF, to which were successively added *N*,*N-*diisopropylethylamine (66 mg, 0.51 mmol), HATU (70 mg, 0.18 mmol), and (2*S*,4*R*)-1-((*S*)-2-amino-3,3-dimethylbutyryl)-4-hydroxy-*N*-((*S*)-1-(4-(4-methylthiazol-5-yl)phenyl)ethyl)-pyrrolidinyl-2-formamide trifluoroacetate (103 mg, 0.18 mmol). The mixture was allowed to react 2 h at room temperature, and then washed with 10 ml of water, followed by extraction with 10 mL of ethyl acetate. The organic layer was concentrated under reduced pressure, and the residue was separated and purified by TLC, to provide 20 mg of product (2*S*,4*R*)-1-((*S*)-2-(2-((5-((3-chloro-4-cyanophenyl)(5-(3,5-dimethyl-1H-pyrazol-4-yl)-2-methylp henyl)amino)pentyl)oxy)acetylamino)-3,3-dimethylbutyryl)-4-hydroxy-N-((*S*)-1-(4-(4-methylth iazol-5-yl)phenyl)ethyl)pyrrolidinyl-2-formamide as a white solid.

### 45: (3R,5S)-1-((S)-2-(2-((5-((3-chloro-4-cyanophenyl)(5-(3,5-dimethyl-1H-pyrazol-4-yl)-2-methylphenyl)amino)pentyl)oxy)acetylamino)-3,3-dimethylbutyryl)-5-(((S)-1-(4-(4-methyl thiazol-5-yl)phenyl)ethyl)pyrrolidinyl-3-acetate (45)

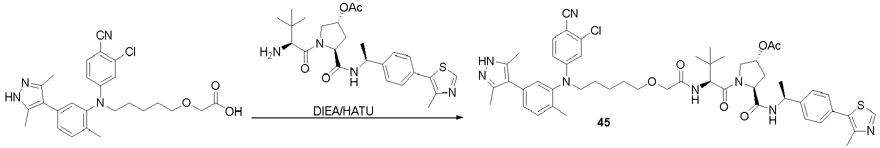

2-((5-((3-Chloro-4-cyanophenyl)(5-(3,5-dimethyl-1-((2-(trimethylsilyl)ethoxy)methyl)-1H-pyra zol-4-yl)-2-methylphenyl)amino)pentyl)oxy)acetic acid (100 mg, 0.75 mmol) was dissolved in 5 mL of DMF, to which were successively added *N*,*N-*diisopropylethylamine (66 mg, 0.51 mmol), HATU (70 mg, 0.18 mmol), and (3*R*,5*S*)-1-((*S*)-2-amino-3,3-dimethylbutyryl)-5-(((*S*)-1-(4-(4-methylthiazol-5-yl)phenyl)ethyl)aminomethyl)pyrrolidinyl-3-acetate trifluoroacetate (108 mg, 0.18 mmol). The mixture was allowed to react 2 h at room temperature, and then washed with 10 ml of water, followed by extraction with 10 mL of ethyl acetate. The organic layer was concentrated under reduced pressure, and the residue was separated and purified by TLC, to provide 25 mg of white solid product. LC/MS (ESI⁺) calcd for C₅₁H₆₁ClN₈O₆S ([M+H]⁺) *m*/*z* 949.5. ¹H NMR (400 MHz, DMSO-*d₆*) *δ* 12.44 - 12.20 (m, 1H), 8.98 (s, 1H), 8.48 - 8.41 (m, 1H), 7.62 - 7.56 (m, 1H), 7.43 (s, 3H), 7.40 - 7.34 (m, 2H), 7.34 - 7.30 (m, 1H), 7.29 - 7.24 (m, 1H), 7.08 - 7.03 (m, 1H), 6.66 - 6.56 (m, 1H), 6.49 - 6.40 (m, 1H), 5.23 - 5.16 (m, 1H), 4.92 - 4.85 (m, 1H), 4.49 - 4.43 (m, 1H), 4.43 - 4.38 (m, 1H), 3.89 (s, 2H), 3.87 - 3.83 (m, 1H), 3.78 - 3.72 (m, 1H), 3.53 - 3.41 (m, 3H), 2.69 (s, 1H), 2.45 (s, 3H), 2.29 - 2.23 (m, 1H), 2.19 (s, 6H), 2.06 (s, 3H), 1.98 (s, 3H), 1.72 - 1.51 (m, 5H), 1.46 - 1.38 (m, 2H), 1.35 (d, *J* = 7.0 Hz, 3H), 0.91 (d, *J* = 6.4 Hz, 9H).

### 46: (3R,5S)-1-((S)-2-(2-((4-((4-(1H-imidazol-1-yl)phenyl)(5-(3,5-dimethylisoxazol-4-yl)-2-methylphenyl)amino)but-2-yn-1-yl)oxy)acetamido)-3,3-dimethylbutanol)-5-((S)-1-(4-meth ylthiazol-5-yl)phenyl)ethyl)carbamoyl)pyrrolidine-3-yl acetate (46)

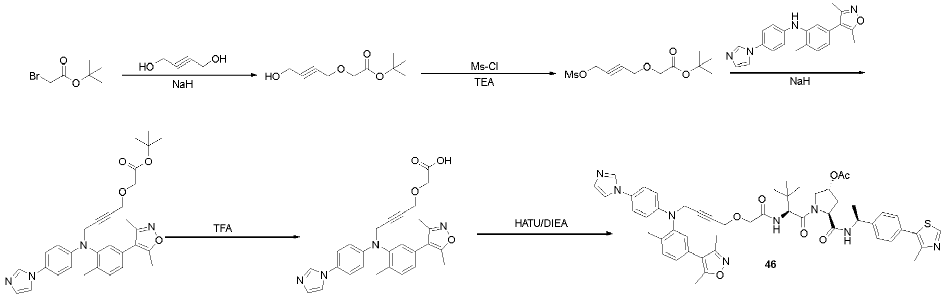

Isobut-2-yne-1,4-diol (12.9 g, 150 mmol) was dissolved in 150 mL of tetrahydrofuran, to which was slowly added sodium hydride (4.0 g, 100 mmol) in an ice-water bath, and the mixture was stirred for 15 min. Then, t-butyl bromoacetate (19.5 g, 100 mmol) was added dropwise, and after addition, the reaction solution was slowly warmed to room temperature, and allowed to react overnight. 300 mL of water was added into the reaction system to quench the reaction. The reaction solution was extracted with 300 mL of ethyl acetate. The organic layer was further washed with water and saturated brine in sequence, dried over anhydrous sodium sulfate, filtered, concentrated to dryness under reduced pressure, and separated and purified by column chromatography, to provide the product t-butyl 2-((4-hydroxybut-2-yn-1-yl)oxy)acetate (7.9 g), with a yield of 39.5%. LC/MS (ESI⁺) calcd for C₁₀H₁₆O₄ ([M+H]⁺) *m*/*z* 201.1.

t-Butyl 2-((4-hydroxybut-2-yn-1-yl)oxy)acetate (3.9 g, 19.5 mmol) was dissolved in 40 mL of dichloromethane, to which was added triethylamine (4.04 g, 40 mmol), and then methanesulfonyl chloride (2.5 g, 22 mmol) was added dropwise in an ice-water bath. After addition, the temperature was maintained, and the mixture was allowed to react for 3 h. The reaction solution was wahed twice with 30 mL of water, washed once with saturated brine, dried over anhydrous magnesium sulfate, and filtered. The filtrate was concentrated to dry under reduced pressure, and the residue was separated and purified by column chromatography, to provide the product t-butyl 2-((4-((methanesulfonyl)oxy)but-2-yn-1-yl)oxy)acetate (1.45 g), with a yield of 26.7%. LC/MS (ESI⁺) calcd for C₁₁H₁₈O₆S ([M+H]⁺) *m*/*z* 279.1.

*N*-(4-(1H-imidazol-1-yl)phenyl)-5-(3,5-dimethylisoxazol-4-yl)-2-methylaniline (172 mg, 0.5 mmol) was dissolved in 5 mL of DMSO, to which was added NaH (40 mg, 1.0 mmol), and the mixture was stirred for 10 min at room temperature, followed by addition of t-butyl 2-((4-((methanesulfonyl)oxy)but-2-yn-1-yl)oxy)acetate (417 mg, 1.5 mmol). The reaction solution was allowed to react overnight at 65 °C and then cooled to room temperature. 10 mL of water was added, and then the resultant solution was extracted with 10 mL of ethyl acetate. The organic layer was respectively washed with water and saturated brine, dried over anhydrous sodium sulfate, concentrated to dryness under reduced pressure, and separated and purified by column chromatography, to provide the product t-butyl 2-((4-((4-(1H-imidazol-1-yl)phenyl) (5-(3,5-dimethylisoxazol-4-yl)-2--methylphenyl)amino)but-2-yn-1-yl)oxy)acetate (20 mg), with a yield of 8%. LC/MS (ESI⁺) calcd for C₃₁H₃₄N₄O₄ ([M+H]⁺) *m*/*z* 527.3.

t-Butyl 2-((4-((4-(1H-imidazol-1-yl)phenyl)(5-(3,5-dimethylisoxazol-4-yl)-2-methylphenyl) amino)but-2-yn-1-yl)oxy)acetate (20 mg, 0.038 mg) was dissolved in 2 mL of dichloromethane, to which was added 1 mL of trifluoroacetic acid, and the mixture was stirred for 30 min at room temperature, and then concentrated to dry under reduced pressure. To the residue, was added 5 mL of water, and then the resultant solution was extracted with 5 mL of dichloromethane, followed by separation and purification by TLC, to provide 8 mg of product, which was dissolved in 5 mL of dichloromethane. DIEA (0.1 mL) and HATU (8 mg, 0.02 mmol) were added, and the resultant solution was stirred for 10 min at room temperature, to which was added (3*R*,5*S*)-1-((*S*)-2-amino-3,3-dimethylbutanol)-5-((*S*)-1-(4-(4-methylthiazol-5-yl)phenyl) ethyl)carbamoyl)pyrrolidine-3-yl acetate (8 mg, 0.017 mmol). The resultant solution was allowed to react 2 h at room temperature, washed with water, extracted with 5 mL of dichloromethane, separated and purified by TLC, to provide the product (3*R*,5,*S*)-1-((*S*)-2-(2-((4-((4-(1H-imidazol-1-yl)

phenyl)(5-(3,5-dimethylisoxazol-4-yl)-2-methylphenyl)amino)but-2-yn-1-yl)oxy)acetamido)-3, 3-dimethylbutanol)-5-((*S*)-1-(4-methylthiazol-5-yl)phenyl)ethyl)carbamoyl)pyrrolidine-3-yl acetate (6 mg), with a yield of 17%. LC/MS (ESI⁺) calcd for C₅₂H₅₈N₈O₇S ([M+H]⁺) *m*/*z* 939.4ₒ ¹H NMR (400 MHz, CDCl₃) *δ* 8.68 (s, 1H), 8.10 (s, 1H), 7.58 (d, *J* = 6.8 Hz, 1H), 7.39 (dt, *J* = 11.9, 7.2 Hz, 8H), 7.25 (s, 1H), 7.17 (d, *J* = 4.9 Hz, 1H), 7.03 (d, *J* = 9.0 Hz, 1H), 6.72 (d, *J* = 8.9 Hz, 2H), 6.14 (d, *J* = 8.5 Hz, 1H), 5.35 (s, 2H), 5.09 (s, 1H), 4.72 (d, *J* = 7.5 Hz, 1H), 4.61 - 4.57 (m, 1H), 4.43 (s, 1H), 4.25 (s, 2H), 4.04 (s, 1H), 3.97 (s, 2H), 3.84 (dd, *J* = 9.8, 5.6 Hz, 1H), 2.77 - 2.70 (m, 1H), 2.61 (dd, *J* = 13.6, 6.8 Hz, 1H), 2.53 (d, *J* = 1.1 Hz, 3H), 2.42 (s, 3H), 2.28 (s, 3H), 2.23 (d, *J* = 7.6 Hz, 3H), 2.02 (d, *J* = 2.6 Hz, 3H), 1.49 - 1.46 (m, 3H), 1.03 (s, 9H).

### 47: (3R,5S)-1-((S)-2-(2-(4-(2-((3-bromo-4-(1H-imidazol-1-yl)phenyl)(5-(3,5-dimethyl isoxazol-4-yl)-2-methylphenyl)amino)ethyl)piperazin-1-yl)acetamido)-3,3-dimethylbutanol )-5-((S)-1-(4-methylthiazol-5-yl)phenyl)ethyl)carbamoyl)pyrrolidine-3-ylacetate (47)

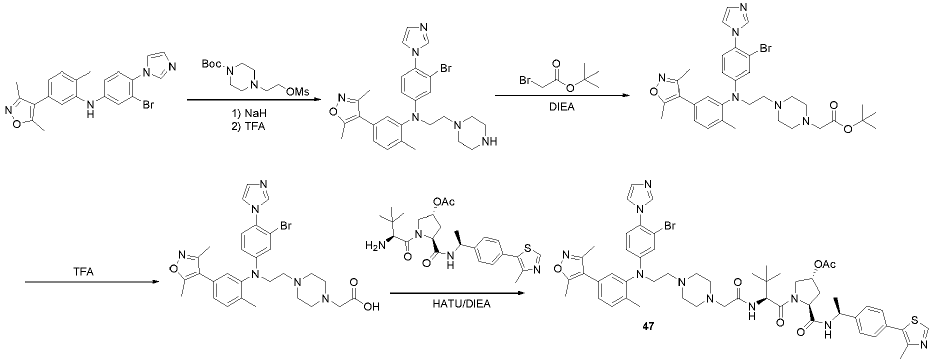

*N*-(3-bromo-4-(1H-imidazol-1-yl)phenyl)-5-(3,5-dimethylisoxazol-4-yl)-2-methylaniline (106 mg, 0.25 mmol) was dissolved in 3 mL of DMSO, to which was added NaH (20 mg, 0.5 mmol), and the mixture was stirred for 15 min at room temperature, followed by addition of t-butyl 4-(2-((methanesulfonyl)oxy)ethyl)piperazine-1-carboxylate (154 mg, 0.5 mmol). The reaction solution was allowed to react 1 h at room temperature, and then 10 mL of water was added, followed by extraction with 10 mL of ethyl acetate. The organic layer was respectively washed with water and saturated brine, dried over anhydrous sodium sulfate, concentrated to dryness under reduced pressure, and separated and purified by column chromatography, to provide the product, which was dissolved in 3 mL of dichloromethane, to which was added 2 mL of trifluoroacetic acid. The mixture was stirred for 2 h at room temperature, and then concentrated to dry under reduced pressure. To the residue, was added 3 mL of water, and then the resultant solution was extracted with 10 mL of ethyl acetate. The organic layer was respectively washed with water and saturated brine, dried over anhydrous sodium sulfate, and concentrated to dryness under reduced pressure, to provide the product N-(3-bromo-4-(1H-imidazol-1-yl) phenyl)-5-(3,5-dimethylisoxazol-4-yl)-2-methyl-N-(2-(piperazin-1-yl)ethyl)aniline (30 mg), with a yield of 22.4%. LC/MS (ESI⁺) calcd for C₂₇H₃₁BrN₆O ([M+H]⁺) *m*/*z* 535.2. *N*-(3-Bromo-4-(1H-imidazol-1-yl)phenyl)-5-(3,5-dimethylisoxazol-4-yl)-2-methyl-*N*-(2-(pipera zin-1-yl)ethyl)aniline (30 mg, 0.056 mmol) was dissolved in 3 mL of dichloromethane, to which were successively added DIEA (29 mg, 0.22 mmol) and t-butyl bromoacetate (12 mg, 0.06 mmol), and the mixture was stirred and reacted overnight, followed by addition of 10 mL dichloromethane. The solution was washed twice with 5 mL of water and once with saturated brine, dried over anhydrous magnesium sulfate, and filtered. The filtrate was concentrated under reduced pressure to dryness, and then separated and purified by column chromatography to provide the product t-butyl 2-(4-(2-((3-bromo-4-(1H-imidazol-1-yl)phenyl)(5-(3,5-dimethyl isoxazol-4-yl)-2-methylphenyl)amino)ethyl)piperazin-1-yl)acetate (15 mg), with a yield of 41%, LC/MS (ESI⁺) calcd for C₃₃H₄₁BrN₆O₃ ([M+H]⁺) *m*/*z* 649.2.

t-Butyl 2-(4-(2-((3-bromo-4-(1H-imidazol-1-yl)phenyl)(5-(3,5-dimethylisoxazol-4-yl)-2-methylphenyl)amino)ethyl)piperazin-1-yl)acetate (15 mg, 0.023 mmol) was dissolved in 3 mL of dichloromethane, to which was added 3 mL of trifluoroacetic acid. The reaction solution was stirred for 30 min at room temperature, and concentrated to dryness under reduced pressure, to obtain the product, which was dissolved in 5 mL of dichloromethane. Then, DIEA (0.1 mL) and HATU (12 mg, 0.03 mmol) were added, and the mixture was stirred at room temperature for 10 min, to which was added (3*R*,5*S*)-1-((*S*)-2-amino-3,3-dimethylbutanol)-5-((*S*)-1-(4-(4-methylthiazol-5-yl)phenyl)ethyl)carbamoyl)pyrrolidine-3-yl acetate (15 mg, 0.033 mmol). The reaction solution was allowed to react at room temperature for 2 h, washed with water, extracted with 5 mL of dichloromethane, and then separated and purified by TLC, to provide the product (3*R*,5*S*)-1-((*S*)-2-(2-(4-(2-((3-bromo-4-(1H-imidazol-1-yl)phenyl)(5-(3,5-dimethylisoxazol-4-yl)-2-methylphenyl)amino)ethyl)piperazin-1-yl)acetamido)-3,3-dimethylbu tanol)-5-((*S*)-1-(4-methylthiazol-5-yl)phenyl)ethyl)carbamoyl)pyrrolidine-3-yl acetate (12 mg), with a yield of 49.1%. LC/MS (ESI⁺) calcd for C₅₄H₆₅BrN₁₀O₆S ([M+H]⁺) *m*/*z* 1061.4. ¹H NMR (400 MHz, CDCl₃) *δ* 8.69 (s, 1H), 7.67 (s, 1H), 7.64 (d, *J* = 6.3 Hz, 1H), 7.40 (dt, *J* = 22.6, 8.0 Hz, 5H), 7.21 (dd, *J* = 12.6, 2.4 Hz, 3H), 7.11 (d, *J* = 9.1 Hz, 1H), 7.05 (s, 2H), 6.85 (s, 1H), 6.54 (d, *J* = 8.7 Hz, 1H), 5.35 (s, 1H), 5.12 - 5.06 (m, 1H), 4.73 - 4.66 (m, 1H), 4.49 (d, *J* = 9.0 Hz, 1H), 4.11 - 4.05 (m, 1H), 4.02 (s, 1H), 3.88 (s, 1H), 3.86 - 3.77 (m, 2H), 3.05 (s, 2H), 2.80 (s, 2H), 2.68 (dd, *J* = 10.3, 9.5 Hz, 6H), 2.53 (s, 3H), 2.43 (s, 3H), 2.29 (s, 3H), 2.22 (s, 1H), 2.19 (s, 3H), 2.08 (s, 1H), 2.05 (s, 3H), 2.02 (d, *J* = 3.0 Hz, 1H), 1.46 (d, *J* = 5.9 Hz, 3H), 1.04 (d, *J* = 6.5 Hz, 9H).

### 48: (3R,5S)-1-((S)-2-(2-((5-((3-bromo-4-(1H-imidazol-1-yl)phenyl)(5-(3,5-dimethylisoxazol-4-yl)-2-methylpyridin-3-yl)amino)pentyl)oxy)acetamido)-3,3-dimethylbutanol)-5-((S)-1-(4-methylthiazol-5-yl)phenyl)ethyl)carbamoyl)pyrrolidine-3-yl acetate (48)

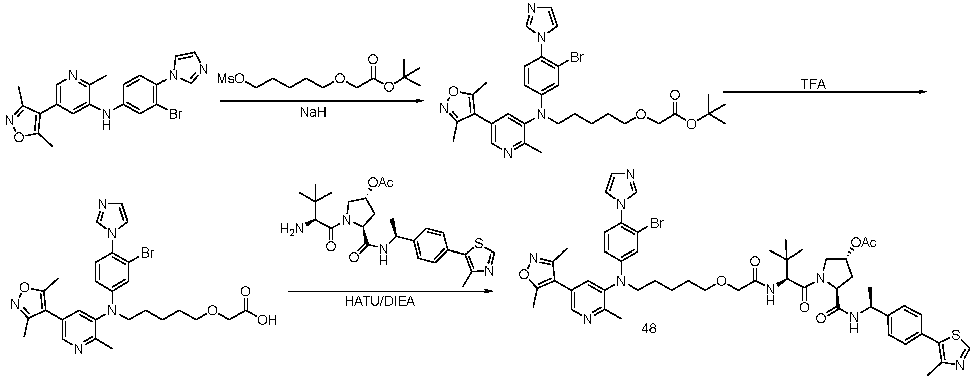

*N*-(3-bromo-4-(1H-imidazol-1-yl)phenyl)-5-(3,5-dimethylisoxazol-4-yl)-2-methylpyridin-3-ami ne (220 mg, 0.5 mmol) was dissolved in 6 mL of DMSO, and the mixture was heated to dissolve and make the solution become clear, to which was added NaH (40 mg, 1.0 mmol). The mixture was stirred for 15 min, to which was added t-butyl 2-((5-((methanesulfonyl)oxy)pentyl)oxy)acetate (421 mg, 3.0 mmol). The resultant mixture was allowed to react for 4 h at 60 °C, and then cooled to room temperature. 10 mL of water was added, and then the resultant solution was extracted with 10 mL of ethyl acetate. The organic layer was respectively washed with water and saturated brine, dried over anhydrous sodium sulfate, concentrated to dryness under reduced pressure, and then separated and purified by TLC, to provide the product t-butyl 2-((5-((3-bromo-4-(1H-imidazol-1-yl)phenyl)(5-(3,5-dimethylisoxazol-4-yl)-2-methylpyridin-3-yl)amino)pentyl)oxy)acetate (94 mg), with a yield of 30.1%. LC/MS (ESI⁺) calcd for C₃₁H₃₈BrN₅O4 ([M+H]⁺) *m*/*z* 624.2.

t-Butyl 2-((5-((3-Bromo-4-(1H-imidazol-1-yl)phenyl)(5-(3,5-dimethylisoxazol-4-yl)-2-methyl pyridin-3-yl)amino)pentyl)oxy)acetate (80 mg, 0.13 mmol) was dissolved in 3 mL of dichloromethane, to which was added 3 mL of trifluoroacetic acid. The reaction solution was stirred for 30 min at room temperature, and concentrated to dryness under reduced pressure, to obtain the product 2-((5-((3-bromo-4-(1H-imidazol-1-yl)phenyl)(5-(3,5-dimethylisoxazol-4-yl)-2-methylpyridin-3-yl)amino)pentyl)oxy)acetic acid (72 mg).

2-((5-((3-Bromo-4-(1H-imidazol-1-yl)phenyl)(5-(3,5-dimethylisoxazol-4-yl)-2-methylpyridin-3 -yl)amino)pentyl)oxy)acetic acid (54 mg, 0.1 mmol) was dissolved in 20 mL of dichloromethane, to which were added DIEA (40 mg, 0.33 mmol) and HATU (43 mg, 0.11 mmol) in two batches, and the mixture was stirred for 10 min, followed by addition of (3*R*,5*S*)-1-((*S*)-2-amino-3,3-dimethylbutanol)-5-((*S*)-1-(4-(4-methylthiazol-5-yl)phenyl)ethyl)ca rbamoyl)pyrrolidine-3-yl acetate (50 mg, 0.1 mmol). The resultant solution was allowed to react overnight at room temperature, washed with water, extracted with 5 mL of dichloromethane, and then separated and purified by TLC, to provide the product (3*R*,5*S*)-1-((*S*)-2-(2-((5-((3-bromo-4-(1H-imidazol-1-yl)phenyl)(5-(3,5-dimethylisoxazol-4-yl)-2-methylpyridin-3-yl)a mino)pentyl)oxy)acetamido)-3,3-dimethylbutanol)-5-((*S*)-1-(4-methylthiazol-5-yl)phenyl)ethyl) carbamoyl)pyrrolidine-3-yl acetate (32 mg), with a yield of 30.9%. LC/MS (ESI⁺) calcd for C₅₂H₆₂BrN₉O₇S ([M+H]⁺) *m*/*z* 1036.4. ¹H NMR(400 MHz, CDCl₃) *δ* 8.61 (s, 1H), 8.41 (d, *J* = 1.6 Hz, 1H), 7.81 (s, 1H), 7.37 - 7.25 (m, 6H), 7.17 (s, 1H), 7.07 (t, *J* = 7.8 Hz, 1H), 7.01 (d, *J* = 7.9 Hz, 2H), 6.71 (d, *J* = 2.2 Hz, 1H), 6.42 (dd, *J* = 8.7, 2.4 Hz, 1H), 5.28 (s, 1H), 5.00 (d, *J* = 7.0 Hz, 1H), 4.68 - 4.62 (m, 1H), 4.50 (d, *J* = 9.2 Hz, 1H), 3.99 (d, *J* = 9.0 Hz, 1H), 3.88 (s, 2H), 3.77 (dd, *J* = 11.7, 4.7 Hz, 1H), 3.61 - 3.52 (m, 2H), 3.47 (t, *J* = 6.4 Hz, 2H), 2.46 (s, 3H), 2.40 (s, 3H), 2.37 (d, *J* = 5.0 Hz, 3H), 2.24 (s, 3H), 1.97 (s, 3H), 1.70 - 1.60 (m, 4H), 1.40 (d, *J* = 6.8 Hz, 4H), 0.96 (s, 9H).

### 49: (2S,4R)-1-((S)-2-(2-((5-((3-bromo-4-(1H-imidazol-1-yl)phenyl)(5-(3,5-dimethylisoxazol-4-yl)-2-methylpyridin-3-yl)amino)pentyl)oxy)acetamido)-3,3-dimethylbutyryl)-4-hydroxy-N-((S)-1-(4-(4-methylthiazol-5-yl)phenyl)ethyl)pyrrolidine-2-formamide(49)

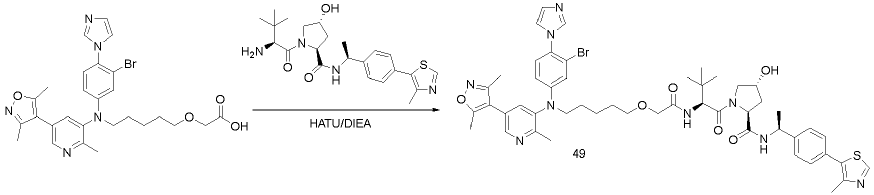

2-((5-((3-Bromo-4-(1H-imidazol-1-yl)phenyl)(5-(3,5-dimethylisoxazol-4-yl)-2-methylpyridin-3 -yl)amino)pentyl)oxy)acetic acid (18 mg, 0.033 mmol) was dissolved in 20 mL of dichloromethane, to which were added DIEA (13 mg, 0.1 mmol) and HATU (15 mg, 0.04 mmol) in two batches, and the mixture was stirred for 10 min, followed by addition of (2*S*,4*R*)-1-((*S*)-2-amino-3,3-dimethylbutanol)-4-hydroxy-*N*-((*S*)-1-(4-methylthiazol-5-yl)phenyl )ethyl)pyrrolidine-2-formamide (15 mg, 0.033 mmol). The resultant solution was allowed to react overnight at room temperature, washed with water, extracted with 5 mL of dichloromethane, and then separated and purified by TLC, to provide the product (2*S*,4*R*)-1-((*S*)-2-(2-((5-((3-bromo-4-(1H-imidazol-1-yl)phenyl)(5-(3,5-dimethylisoxazol-4-yl)-2-methylpyridin-3-yl)amino)pentyl)oxy)acetamido)-3,3-dimethylbutyryl)-4-hydroxy-*N*-((*S*)-1-( 4-(4-methylthiazol-5-yl)phenyl)ethyl)pyrrolidine-2-formamide (25 mg), with a yield of 76.1%. LC/MS (ESI⁺) calcd for C₅₀H₆₀BrN₉O₆S ([M+H]⁺) *m*/*z* 994.4. ¹H NMR (400 MHz, CDCl₃) *δ* 8.61 (s, 1H), 8.40 (s, 1H), 7.91 (d, *J* = 18.4 Hz, 1H), 7.39 - 7.24 (m, 7H), 7.16 - 7.09 (m, 2H), 7.07 (s, 1H), 6.68 (s, 1H), 6.47 (d, *J* = 6.9 Hz, 1H), 5.04 - 4.98 (m, 1H), 4.64 (t, *J* = 7.6 Hz, 1H), 4.49 (d, *J* = 8.7 Hz, 2H), 4.10-4.01 (m, 2H), 3.99 (s, 1H), 3.86 (d, *J* = 2.6 Hz, 2H), 3.56 (d, *J* = 11.0 Hz, 2H), 3.47 (s, 2H), 2.74 (s, 3H), 2.45 (s, 3H), 2.40 (s, 3H), 2.37 (s, 3H), 2.24 (s, 3H), 1.60 (d, *J* = 6.4 Hz, 2H), 1.40 (d, *J* = 6.6 Hz, 6H), 0.98 (s, 9H).

### 50: (3R,5S)-1-((S)-2-(2-(4-((3-bromo-4-(1H-imidazol-1-yl)phenyl)(5-(3,5-dimethylisoxazol-4-yl)-2-methylphenyl)amino)methyl)piperidin-1-yl)ethoxy)acetamido)-3,3-dimethylbutyry l)-5-((S)-1-(4-methylthiazol-5-yl)phenyl)ethyl)carbamoyl)pyrrolidine-3-yl acetate (50)

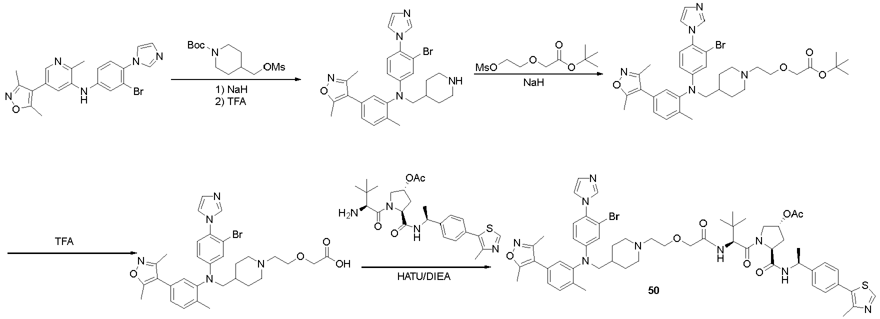

LC/MS (ESI⁺) calcd for C₅₆H₆₈BrN₉O₇S ([M+H]⁺) *m*/*z* 1090.5. ¹H NMR (400 MHz, CDCl₃) *δ* 8.68 (s, 1H), 7.58 (s, 1H), 7.46 - 7.32 (m, 5H), 7.30 (s, 1H), 7.21 - 7.11 (m, 3H), 7.05 (t, *J* = 9.7 Hz, 3H), 6.77 (d, *J* = 2.5 Hz, 1H), 6.45 (dd, *J=* 8.7, 2.5 Hz, 1H), 5.35 (s, 1H), 5.07 (d, *J* = 7.2 Hz, 1H), 4.70 (s, 1H), 4.57 (d, *J* = 9.2 Hz, 1H), 4.07 - 4.02 (m, 1H), 4.02 - 3.93 (m, 2H), 3.84 (dd, *J* = 11.6, 4.7 Hz, 1H), 3.70 (d, *J* = 5.6 Hz, 2H), 3.53 (s, 1H), 3.12 (d, *J* = 7.6 Hz, 2H), 2.71 (d, *J* = 13.5 Hz, 2H), 2.68 - 2.61 (m, 1H), 2.53 (s, 3H), 2.43 (s, 3H), 2.29 (s, 3H), 2.19 (d, *J* = 9.6 Hz, 4H), 2.14 (s, 3H), 2.05 (d, *J* = 5.1 Hz, 3H), 1.84 (d, *J* = 10.7 Hz, 2H), 1.52 (d, *J* = 3.2 Hz, 1H), 1.47 (d, *J* = 7.0 Hz, 3H), 1.44 (d, *J* = 6.7 Hz, 2H), 1.03 (s, 9H).

### 51: (3R,5S)-1-((S)-2-(2-(3-((3-bromo-4-(1H-imidazol-1-yl)phenyl) (5-(3,5-dimethylisoxazol-4-yl)-2-methylphenyl)amino)methyl)azetidine-1-yl)ethoxy)acetam ido)-3,3-dimethylbutanol)-5-((S)-1-(4-methylthiazol-5-yl)phenyl)ethyl)carbamoyl)pyrrolid ine-3-yl acetate (51)

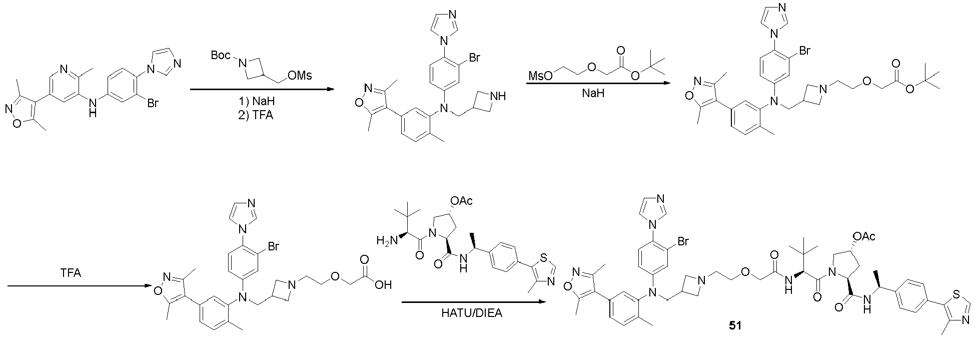

LC/MS (ESI⁺) calcd for C₅₄H₆₄BrN₉O₇S ([M+H]⁺) *m*/*z* 1062.4. ¹H NMR (400 MHz, CDCl₃) *δ* 8.68 (s, 2H), 7.86 (s, 1H), 7.40 (dt, *J* = 14.4, 7.7 Hz, 7H), 7.20 (dd, *J* = 18.8, 7.1 Hz, 3H), 7.07 (d, *J* = 0.8 Hz, 1H), 6.96 (d, *J* = 1.0 Hz, 1H), 6.84 (d, *J* = 2.1 Hz, 1H), 5.35 (s, 1H), 5.11 - 5.05 (m, 1H), 4.75 - 4.68 (m, 1H), 4.58 (d, *J* = 8.9 Hz, 1H), 4.09 (d, *J* = 14.8 Hz, 2H), 4.00 (d, *J* = 14.5 Hz, 2H), 3.94 - 3.82 (m, 3H), 3.31 (s, 1H), 2.51 (d, *J* = 4.4 Hz, 4H), 2.43 (s, 3H), 2.28 (s, 3H), 2.25 - 2.19 (m, 2H), 2.16 (s, 4H), 2.08 (s, 3H), 2.01 (d, *J* = 6.3 Hz, 4H), 1.52 - 1.42 (m, 5H), 1.03 (s, 9H).

### 52: (3R,5S)-1-((S)-2-(2-((5-((3-bromo-4-(4-methyl-1H-imidazol-1-yl)phenyl)(5-(3,5-dimethylisoxazol-4-yl)-2-methylphenyl)amino)pentyl)oxy)acetamido)-3,3-dimethylbutyryl )-5-((S)-1-(4-methylthiazol-5-yl)phenyl)ethyl)carbamoyl)pyrrolidine-3-yl acetate (52)

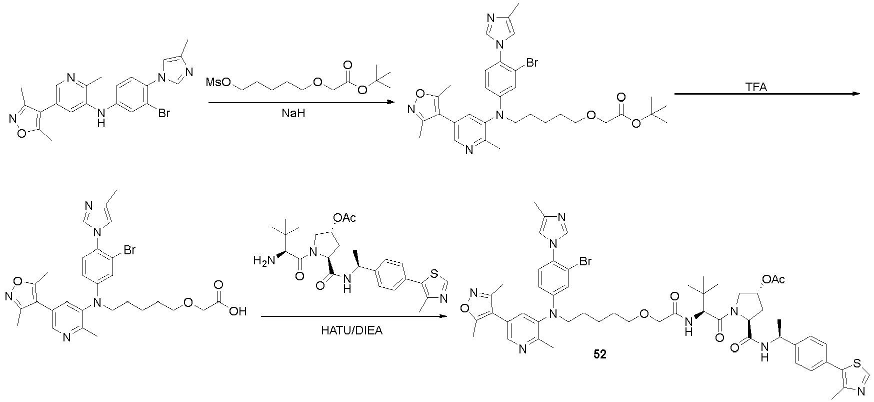

LC/MS (ESI⁺) calcd for C₅₃H₆₄BrN₉O₇S ([M+H]⁺) m/e 1049.4_{∘}

### 53: (2S,4R)-1-((S)-2-(2-((5-((3-bromo-4-(4-methyl-1H-imidazol-1-yl)phenyl)(5-(3,5-dimethylisoxazol-4-yl)-2-methylphenyl)amino)pentyl)oxy)acetamido)-3,3-dimethylbutyryl )-4-hydroxy-N-((S)-1-(4-methylthiazol-5-yl)phenyl)ethyl)pyrrolidine-2-carboxamide (53)

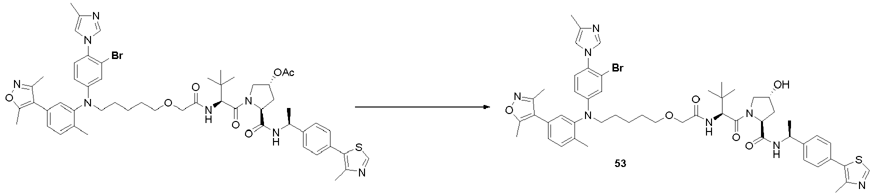

(3*R*,5*S*)-1-((*S*)-2-(2-((5-((3-bromo-4-(4-methyl-1H-imidazol-1-yl)phenyl)(5-(3,5-dimethylisoxa zol-4-yl)-2-methylphenyl)amino)pentyl)oxy)acetamido)-3,3-dimethylbutyryl)-5-((*S*)-1-(4-meth ylthiazol-5-yl)phenyl)ethyl)carbamoyl)pyrrolidine-3-yl acetate (13 mg, 0.012 mmol) was dissolved in 2 mL of methanol, to which was added 0.5 mL of LiOH aqueous solution, and the mixture was stirred at room temperature for 30 min, followed by addition of 10 mL ethyl acetate. The resultant solution was successively washed with water and saturated brine, dried over anhydrous sodium sulfate, and concentrated under reduced pressure, to provide the product (2*S*,4*R*)-1-((*S*)-2-(2-((5-((3-bromo-4-(4-methyl-1H-imidazol-1-yl)phenyl)(5-(3,5-dimethylisoxa zol-4-yl)-2-methylphenyl)amino)pentyl)oxy)acetamido)-3,3-dimethylbutyryl)-4-hydroxy-*N*-((*S*) -1-(4-methylthiazol-5-yl)phenyl)ethyl)pyrrolidine-2-carboxamide (8 mg), with a yield of 66%. LC/MS (ESI⁺) calcd for C₅₁H₆₂BrN₉O₆S ([M+H]⁺) *m*/*z* 1007.3. ¹H NMR (400 MHz, CDCl₃) *δ* 8.67 (s, 1H), 7.71 (s, 1H), 7.39 (ddd, *J* = 17.9, 14.9, 8.0 Hz, 6H), 7.18 (d, *J* = 8.2 Hz, 2H), 7.07 (d*, J* = 8.8 Hz, 1H), 7.03 (s, 1H), 6.80 - 6.70 (m, 2H), 6.45 (dd, *J* = 8.8, 2.6 Hz, 1H), 5.11 - 5.04 (m, 1H), 4.72 (t, *J* = 7.9 Hz, 1H), 4.57 (d, *J* = 8.9 Hz, 1H), 4.52 (s, 1H), 4.10 (d, *J* = 11.4 Hz, 1H), 3.92 (s, 2H), 3.68 - 3.57 (m, 3H), 3.53 (t, *J* = 6.4 Hz, 2H), 2.53 (d, *J* = 3.0 Hz, 3H), 2.42 (s, 3H), 2.32 (s, 3H), 2.28 (s, 3H), 2.18 (s, 3H), 2.08 (d, *J* = 8.0 Hz, 4H), 1.80 - 1.71 (m, 2H), 1.71 - 1.63 (m, 2H), 1.49 (d, *J* = 7.1 Hz, 1H), 1.47 (d, *J=* 6.9 Hz, 3H), 1.04 (s, 9H).

### 54: (3R,5S)-1-((S)-2-(2-(2-((3-bromo-4-(1H-imidazol-1-yl)phenyl)(5-(3,5-dimethylisoxazol-4-yl)-2-methylphenyl)amino)ethoxy)acetamido)-3,3-dimethylbutyryl)-5-(S)-1-(4-methylth iazol-5-yl)phenyl)ethyl)carbamoyl)pyrrolidine-3-yl acetate (54)

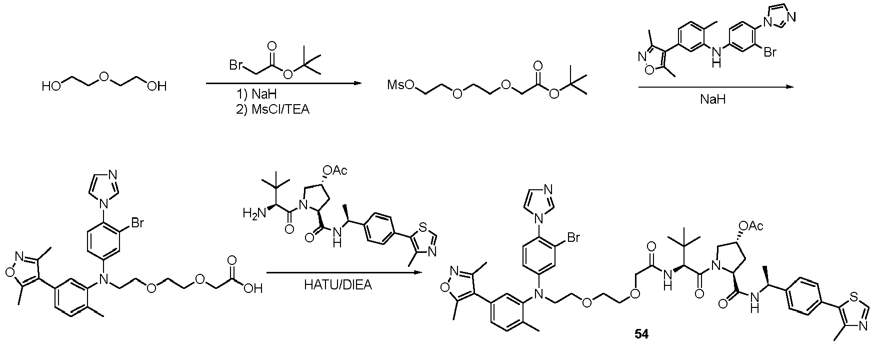

2,2'-Oxybis(ethan-1-ol) (5.3 g, 50 mmol) was dissolved in 50 mL of tetrahydrofuran, to which was added sodium hydride (1.0 g, 25 mmol) in an ice-water bath, and the mixture was stirred for 15 min. Then, t-butyl bromoacetate (4.9 g, 25 mmol) was added dropwise, and after addition, the temperature was maintained, and the reaction solution was allowed to react 1 h. 80 mL of water was added to quench the reaction. The reaction solution was extracted with 100 mL of ethyl acetate. The organic layer was washed with saturated brine, dried over anhydrous sodium sulfate, filtered, and concentrated to dryness under reduced pressure, to provide the product t-butyl 2-(2-(2-hydroxyethoxy)ethoxy)acetate (2.7 g), with a yield of 24.5%. LC/MS (ESI⁺) calcd for C₁₀H₂₀O₅ ([M+H]⁺) *m*/*z* 221.1. t-Butyl 2-(2-(2-hydroxyethoxy)ethoxy)acetate (1.34 g, 12 mmol) was dissolved in 15 mL of dichloromethane, to which was added triethylamine (1.44 g, 14.4 mmol), and then MsCl was added in an ice-water bath. After addition, the temperature was maintained, and the mixture was allowed to react for 1 h. The reaction solution was wahed with 10 mL of water. The organic layer was respectively washed with water and saturated brine, dried over anhydrous magnesium sulfate, and concentrated to dry under reduced pressure. The residue was separated and purified by column chromatography, to provide the product t-butyl 2-(2-(2-(2-(((methylsulfonyl)oxy)ethoxy)ethoxy)acetate (650 mg), with a yield of 79.1%. LC/MS (ESI⁺) calcd for C₁₁H₂₂O₇S ([M+H]⁺) *m*/*z* 299.1.

*N*-(3-Bromo-4-(1H-imidazol-1-yl)phenyl)-5-(3,5 -dimethylisoxazol-4-yl)-2-methylaniline (85 mg, 0.2 mmol) was dissolved in 5 mL of DMSO, and the mixture was heated to 50 °C to dissolve and make the solution become clear, to which was then added NaH (16 mg, 0.4 mmol). The mixture was stirred for 10 min at room temperature, and then t-butyl 2-(2-(2-(2-(((methylsulfonyl)oxy)ethoxy)ethoxy)acetate (184 mg, 0.6 mmol) was added. The temperature was maintained, and the resultant solution was allowed to react 2 h. Heating was stopped, and 10 mL of water was added to quench the reaction. The reaction solution was extracted with 15 mL of ethyl acetate. The organic layer was respectively washed with water and saturated brine, concentrated to dryness under reduced pressure, and separated by TLC, to provide the product 2-(2-(2-((3-bromo-4-(1H-imidazol-1-yl)phenyl)(5-(3,5-dimethylisoxazol-4-yl)-2-methylphenyl)amino)ethoxy)ethoxy)acetic acid (50 mg), with a yield of 43.9%. LC/MS (ESI⁺) calcd for C₂₇H₂₉BrN₄O₅ ([M+H]⁺) *m*/*z* 569.2.

2-(2-(2-((3-Bromo-4-(1H-imidazol-1-yl)phenyl)(5-(3,5-dimethylisoxazol-4-yl)-2-methylphenyl )amino)ethoxy)ethoxy)acetic acid (50 mg, 0.088 mmol) was dissolved in 5 mL of dichloromethane, to which were added DIEA (34 mg, 0.26 mmol) and (3*R*,5*S*)-1-((*S*)-2-amino-3,3-dimethylbutanol)-5-((*S*)-1-(4-(4-methylthiazol-5-yl)phenyl)ethyl)ca rbamoyl)pyrrolidine-3-yl acetate (43 mg, 0.088 mmol). The mixture was allowed to react at room temperature for 2 h. The reaction solution was wahed with water, and then extracted with 5 mL of dichloromethane, followed by purification by TLC, to provide the product (3*R,*5*S*)-1-((*S*)-2-(2-(2-((3-bromo-4-(1*H*-imidazol-1-yl)phenyl)(5-(3,5-dimethylisoxazol-4-yl)-2 -methylphenyl)amino)ethoxy)acetamido)-3,3-dimethylbutyryl)-5-((*S*)-1-(4-methylthiazol-5-yl)p henyl)ethyl)carbamoyl)pyrrolidine-3-yl acetate (10 mg), with a yield of 10.9%. LC/MS (ESI⁺) calcd for C₅₂H₆₁BrN₈O₈S ([M+H]⁺) *m*/*z* 1036.4.

### 55: (2S,4R)-1-((S)-2-(2-(2-((3-bromo-4-(1H-imidazol-1-yl)phenyl)(5-(3,5-dimethylisoxazol-4-yl)-2-methylphenyl)amino)ethoxy)ethoxy)acetamido)-3,3-dimethylbutyryl)-4-hydroxy-N -((S)-1-(4-methylthiazol-5-yl)phenyl)ethyl)pyrrolidine-2-formamide (55)

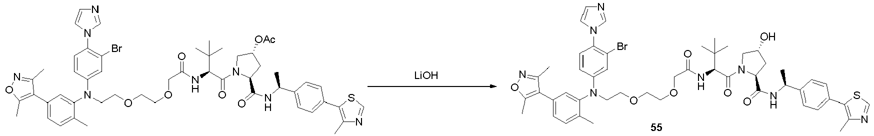

By referring to the method in the above example, the target compound was synthesized according to the above route. LC/MS (ESI⁺) calcd for C₅₀H₅₉BrN₈O₇S ([M+H]⁺) *m*/*z* 996.2.

### 56: (3R,5S)-1-((S)-2-(2-((5-((5-(3,5-dimethylisoxazol-4-yl)-2-methylphenyl)(4-methoxyphenyl)amino)pentyl)oxy)acetamido)-3,3-dimethylbutyryl)-5-((S)-1-(4-(4-methylt hiazol-5-yl)phenyl)ethyl)carbamoyl)pyrrolidine-3-yl acetate (56)

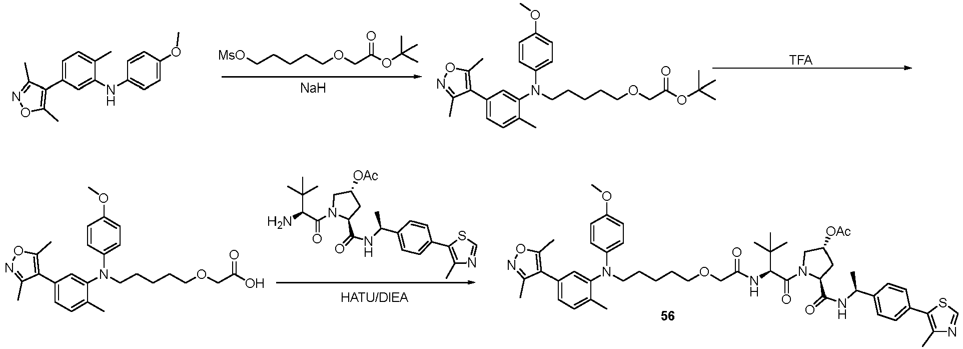

By referring to the method in above example, the target compound was synthesized according to the above route. LC/MS (ESI⁺) calcd for C₅₁H₆₄N₆O₈S ([M+H]⁺) *m*/*z* 921.3. ¹H NMR (400 MHz, CDCl₃) *δ* 9.33 (s, 1H), 7.43 (dd, J = 20.7, 8.3 Hz, 5H), 7.31 (d, J = 7.4 Hz, 1H), 7.13 (d, J = 9.1 Hz, 1H), 7.04 (d, J = 8.0 Hz, 2H), 6.77 (d, J = 9.0 Hz, 2H), 6.55 (d, J = 9.0 Hz, 2H), 5.34 (s, 1H), 5.09 - 5.04 (m, 1H), 4.76 - 4.71 (m, 1H), 4.57 (d, J = 9.2 Hz, 1H), 4.06 (d, J = 12.2 Hz, 1H), 3.93 (d, J = 3.4 Hz, 2H), 3.82 (dd, J = 11.7, 4.8 Hz, 1H), 3.74 (s, 3H), 3.59 (dd, J = 8.6, 6.4 Hz, 2H), 3.50 (t, J = 6.5 Hz, 2H), 2.75 (s, 3H), 2.74 - 2.66 (m, 2H), 2.40 (s, 3H), 2.27 (d, J = 2.4 Hz, 3H), 2.12 (s, 3H), 2.04 (s, 3H), 1.67 (dd, J = 17.5, 10.4 Hz, 4H), 1.47 (d, J = 6.9 Hz, 3H), 1.42 (d, J = 8.8 Hz, 2H), 1.04 (s, 9H).

### 57: (3R,5S)-1-((S)-2-(2-((5-((3-chloro-4-cyanophenyl)(5-(3,5-dimethylisoxazol-4-yl)-2-methylphenyl)amino)pentyl)oxy)acetamido)-3,3-dimethylbutyryl)-5-((S)-1-(4-(4-methylthi azol-5-yl)phenyl)ethyl)carbamoyl)pyrrolidine-3-yl acetate (57)

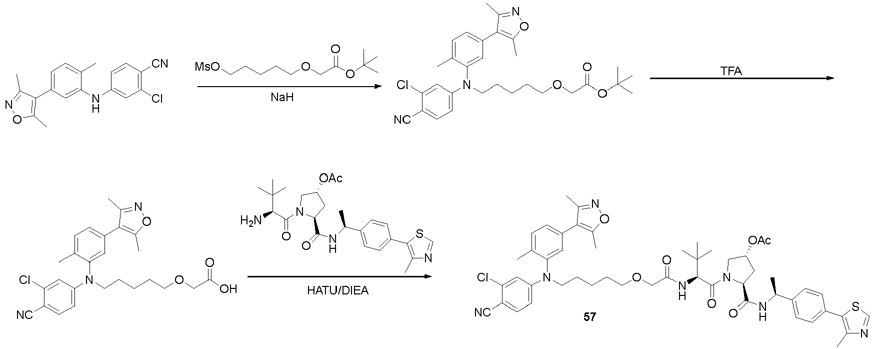

By referring to the method in the above example, the target compound was synthesized according to the above route. LC/MS (ESI⁺) calcd for C₅₁H₆₀CLN₇O₇S ([M+H]⁺) *m*/*z* 950.3. ¹H NMR (400 MHz, CDCl₃) *δ* 8.78 (s, 1H), 7.40 (dt, *J* = 18.1, 8.1 Hz, 6H), 7.22 (dd, *J* = 7.8, 1.7 Hz, 2H), 7.11 (d, *J* = 9.2 Hz, 1H), 6.99 (d, *J* = 1.5 Hz, 1H), 6.50 (s, 1H), 6.35 (d, *J* = 8.3 Hz, 1H), 5.36 (s, 1H), 5.07 (dd, *J* = 14.2, 7.0 Hz, 1H), 4.73 - 4.67 (m, 1H), 4.58 (d, *J* = 9.3 Hz, 1H), 4.05 (d, *J* = 11.7 Hz, 1H), 3.94 (s, 2H), 3.84 (dd, *J* = 11.6, 4.9 Hz, 1H), 3.59 (d, *J* = 5.4 Hz, 1H), 3.52 (t, *J* = 6.4 Hz, 2H), 2.74 - 2.65 (m, 1H), 2.56 (s, 3H), 2.42 (s, 3H), 2.28 (s, 3H), 2.14 (s, 4H), 2.05 (d, *J* = 2.4 Hz, 3H), 1.73 (d, *J* = 8.8 Hz, 2H), 1.71 - 1.66 (m, 2H), 1.48 (d, *J* = 6.9 Hz, 3H), 1.44 (s, 2H), 1.26 (s, 1H), 1.04 (s, 9H).

### 58: (2S,4R)-1-((S)-2-(2-((5-((3-chloro-4-cyanophenyl)(5-(3,5-dimethylisoxazol-4-yl)-2-methylphenyl)amino)pentyl)oxy)acetamido)-3,3-dimethylbutyryl)-4-hydroxy-N-((S)-1-(4-methylthiazol-5-yl)phenyl)ethyl)pyrrolidine-2-formamide (58)

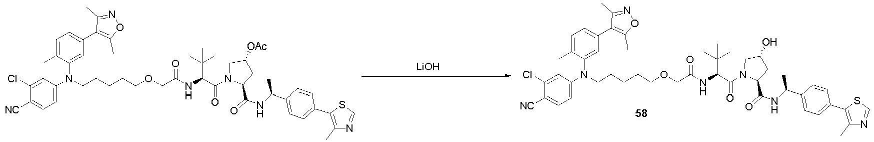

By referring to the method in the above example, the target compound was synthesized according to the above route. LC/MS (ESI⁺) calcd for C₄₉H₅₈ClN₇O₆S ([M+H]⁺) *m*/*z* 908.3. 1H NMR (400 MHz, CDCl₃) *δ* 8.80 (s, 1H), 7.44 (d, *J* = 7.9 Hz, 1H), 7.39 (dt, *J* = 14.3, 7.2 Hz, 4H), 7.33 (d, *J* = 7.7 Hz, 1H), 7.21 (dd, *J* = 7.9, 1.7 Hz, 1H), 7.17 (d, *J* = 8.7 Hz, 1H), 6.99 (d, *J* = 1.6 Hz, 1H), 6.61 (s, 1H), 6.49 (s, 1H), 6.36 (d, *J* = 9.0 Hz, 1H), 5.11 - 5.02 (m, 1H), 4.72 (t, *J* = 7.9 Hz, 1H), 4.55 (d, *J* = 8.8 Hz, 2H), 4.12 (d, *J* = 11.9 Hz, 1H), 3.93 (d, *J* = 3.7 Hz, 2H), 3.72 (dd, *J* = 8.6, 4.5 Hz, 2H), 3.62 (dd, *J* = 11.4, 3.5 Hz, 1H), 3.52 (t, *J* = 6.4 Hz, 2H), 3.20 - 3.11 (m, 2H), 2.54 (s, 3H), 2.42 (s, 3H), 2.28 (s, 3H), 2.14 (s, 3H), 1.47 (dd, *J* = 6.7, 2.4 Hz, 6H), 1.43 (s, 3H), 1.05 (s, 9H).

### 59: 4-((5-(3-(5-amino-1-oxoisoindolin-2-yl)-2,6-dioxopiperidin-1-yl)pentyl)(5-(3,5-dimethylisoxazol-4-yl)-2-methylphenyl)amino)-2-chlorobenzonitrile (59)

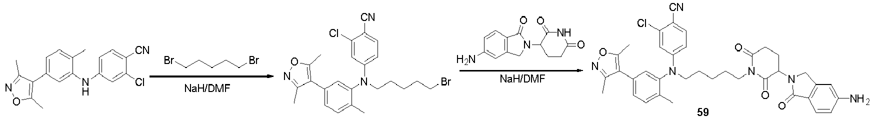

2-Chloro-4-((5-(3,5-dimethylisoxazol-4-yl)-2-methylphenyl)amino)benzonitrile (160 mg, 0.5 mmol) was dissolved in 5 mL of DMF, to which was added NaH (40 mg, 1.0 mmol), and the mixture was stirred 10 min, followed by addition of 1,5-dibromopentane (230 mg, 1.0 mmol). The mixture was allowed to react overnight at room temperature, and then 10 mL of water was added to quench the reaction. The reaction solution was extracted with 15 mL of ethyl acetate.

The organic layer was washed with saturated brine, dried over anhydrous sodium sulfate, concentrated to dryness under reduced pressure, and separated and purified by TLC, to provide the product 4-((5-bromopentyl)(5-(3,5-dimethylisoxazol-4-yl)-2-methylphenyl)amino)-2-chlorobenzonitrile (130 mg), with a yield of 53.4%, LC/MS (ESI⁺) calcd for C₂₄H₂₅BrClN₃O ([M+H]⁺) *m*/*z* 486.1.

3-(5-Amino-1-oxoisoindol-2-yl)piperidine-2,6-dione (42 mg, 0.16 mmol) was dissolved in 3 mL of DMF, and the mixture was stirred for 5 min at room temperature, to which was added 4-((5-bromopentyl)(5-(3,5-dimethylisoxazol-4-yl)-2-methylphenyl)amino)-2-chlorobenzonitrile (78 mg, 0.16 mmol). The mixture was allowed to react 1 h at room temperature, and then 10 mL of water was added to quench the reaction. The reaction solution was extracted with 10 mL of ethyl acetate. The organic layer was washed with saturated brine, dried over anhydrous sodium sulfate, concentrated to dryness under reduced pressure, and separated and purified by TLC, to provide the product 4-((5-(3-(5-amino-1-oxoisoindol-2-yl)-2,6-dioxopiperidin-1-yl)pentyl)(5-(3,5-dimethylisoxazol-4-yl)-2-methylphenyl)amino)-2-chlorobenzonitrile (20 mg), with a yield of 15.8%. LC/MS (ESI⁺) calcd for C₃₇H₃₇ClN₆O₄ ([M+H]⁺) *m*/*z* 665.2. ¹H NMR (400 MHz, CDCl₃) *δ* 7.66 (d, *J* = 7.9 Hz, 1H), 7.42 (d, *J* = 7.9 Hz, 1H), 7.29 (d, *J* = 8.8 Hz, 1H), 7.19 (d, *J* = 7.9 Hz, 1H), 6.96 (s, 1H), 6.89 - 6.72 (m, 2H), 6.45 (s, 1H), 6.38 (d, *J* = 6.9 Hz, 1H), 5.07 (d, *J* = 10.8 Hz, 1H), 4.29 (d, *J* = 13.4 Hz, 2H), 3.76 (d, *J* = 6.7 Hz, 2H), 3.57 (d, *J* = 46.0 Hz, 2H), 2.96 (d, *J* = 17.5 Hz, 1H), 2.86 (d, *J* = 25.5 Hz, 1H), 2.41 (s, 3H), 2.27 (s, 3H), 2.18 (s, 2H), 2.12 (s, 3H), 1.70 (s, 2H), 1.62 - 1.49 (m, 2H), 1.39 - 1.27 (m, 2H).

### 60: 1-(4-((5-(3-(5-amino-1-oxoisoindolin-2-yl)-2,6-dioxopiperidin-1-yl)pentyl)(5-(3,5-dimethylisoxazol-4-yl)-2-methylphenyl)amino)phenyl)cyclopropane-1-nitrile (60)

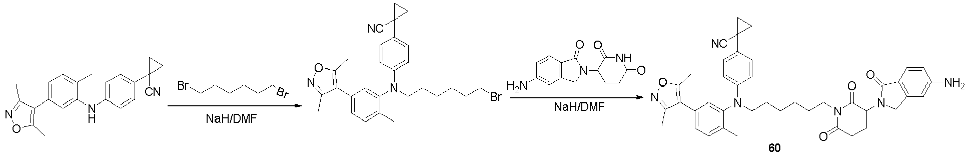

By referring to the method in the above example, the target compound was synthesized according to the above route. LC/MS (ESI⁺) calcd for C₄₁H₄₄N₆O₄ ([M+H]⁺) *m*/*z* 685.3. ¹H NMR (400 MHz, DMSO-*d₆*) *δ* 7.44 (d, *J* = 7.9 Hz, 1H), 7.34 (d, *J* = 8.3 Hz, 1H), 7.25 (d, *J* = 7.9 Hz, 1H), 7.17 - 7.05 (m, 3H), 6.61 (d, *J* = 8.0 Hz, 2H), 6.44 (d, *J* = 8.8 Hz, 2H), 5.83 (s, 2H), 5.05 (dd, *J* = 13.2, 5.0 Hz, 1H), 4.24 (d, *J* = 16.4 Hz, 1H), 4.07 (d, *J* = 16.6 Hz, 1H), 3.56 (dd, *J* = 13.4, 7.0 Hz, 4H), 3.32 (s, 2H), 3.02 - 2.89 (m, 1H), 2.70 (d, *J* = 20.9 Hz, 1H), 2.39 (s, 3H), 2.33 - 2.25 (m, 1H), 2.21 (s, 3H), 2.05 (s, 3H), 1.96 (s, 1H), 1.60 - 1.57 (m, 2H), 1.42 - 1.35 (m, 2H), 1.32 - 1.29 (m, 2H), 1.23 (s, 4H).

### 61: 1-(4-((5-(3,5-dimethylisoxazol-4-yl)-2-methylphenyl)(6-((2-(2,6-dioxopiperidin-3-yl)-1-oxoisoindolin-5-yl)amino)hexyl)amino)phenyl)cyclopropane-1-nitrile (61)

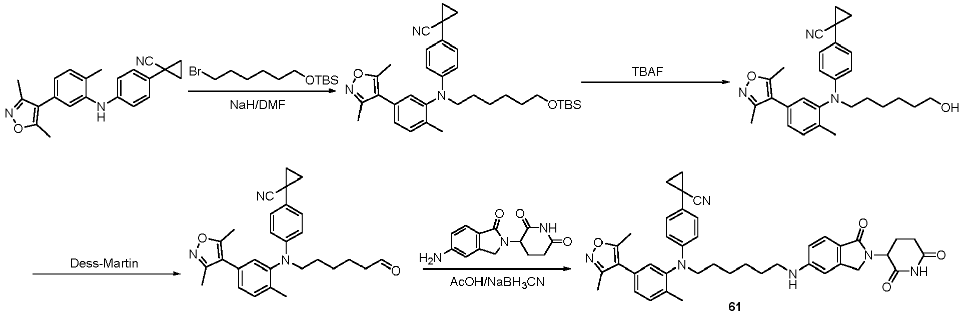

1-(4-((5-(3,5-Dimethylisoxazol-4-yl)-2-methylphenyl)amino)phenyl)cyclopropane-1-carbonitril e (343 mg, 1.0 mmol) was dissolved in 10 mL of DMF, to which was added NaH (80 mg, 2.0 mmol), and the mixture was stirred for 5 min, followed by addition of (6-bromohexyl)oxy(t-butyl)dimethylsilane (362 mg, 1.2 mmol). The mixture was allowed to react 3 h at room temperature, and then 10 mL of water was added to quench the reaction. The reaction solution was extracted with 15 mL of ethyl acetate. The organic layer was washed with saturated brine, dried over anhydrous sodium sulfate, concentrated to dryness under reduced pressure, and separated and purified by TLC, to provide the product 1-(4-((6-((t-butyldimethylsilyl)oxy)hexyl)(5-(3,5-dimethylisoxazol-4-yl)-2-methylphenyl)amin o)phenyl)cyclopropane-1-carbonitrile (220 mg), with a yield of 39.4%. LC/MS (ESI⁺) calcd for C₃₄H₄₇N₃O₂Si ([M+H]⁺) *m*/*z* 558.3.

1-(4-((6-((t-Butyldimethylsilyl)oxy)hexyl)(5-(3,5-dimethylisoxazol-4-yl)-2-methylphenyl)amin o)phenyl)cyclopropane-1-carbonitrile (220 mg, 0.39 mmol) was dissolved in 10 mL of tetrahydrofuran, to which was added tetrabutylammonium fluoride (203 mg, 0.78 mmol). The mixture was allowed to react at 40 °C for 2 h, concentrated under reduced pressure to dryness, and then separated and purified by TLC, to provide 1-(4-((5-(3,5-dimethylisoxazol-4-yl)-2-methylphenyl)(6-hydroxyhexyl)amino)phenyl)cyclopro pane-1-nitrile (150 mg), with a yield of 85.8%. LC/MS (ESI⁺) calcd for C₂₈H₃₃N₃O₂ ([M+H]⁺) *m*/*z* 444.3.

1-(4-((5-(3,5-Dimethylisoxazol-4-yl)-2-methylphenyl)(6-hydroxyhexyl)amino)phenyl)cyclopro pane-1-nitrile (62 mg, 0.14 mmol) was dissolved in 10 mL of dichloromethane, to which was added Dess-Martin periodinane (89 mg, 0.21 mmol). The resultant solution was allowed to react at room temperature overnight, and filtered. The filtrate was concentrated under reduced pressure to dryness. The residue was separated and purified by TLC, to provide 1-(4-((5-(3,5-dimethylisoxazol-4-yl)-2-methylphenyl)(6-oxohexyl)amino)phenyl)cyclopropane-1-nitrile (58 mg), with a yield of 93.8%. LC/MS (ESI⁺) caled for C₂₈H₃₁N₃O₂ ([M+H]⁺) *m*/*z* 442.2.

1-(4-((5-(3,5-Dimethylisoxazol-4-yl)-2-methylphenyl)(6-oxohexyl)amino)phenyl)cyclopropane -1-nitrile (55 mg, 0.12 mmol) and 3-(5-amino-1-oxoisoindolin-2-yl)piperidine-2,6-dione (32 mg, 0.12 mmol) were dissolved in 5 mL of dichloromethane, to which was added one drop of acetic acid. The mixture was allowed to react at 35 °C for 1 h, and then cooled to room temperature, to which was added sodium cyanoborohydride (60 mg, 1.0 mmol). The resultant solution was allowed to react at room temperature overnight, washed with water, and concentrated under reduced pressure to dry. The residue was separated and purified by TLC, to provide the product 1-(4-((5-(3,5-dimethylisoxazol-4-yl)-2-methylphenyl)(6-((2-(2,6-dioxopiperidin-3-yl)-1-oxoisoindolin-5-yl)amino)hexyl)amino)phenyl)cyclopropane-1-nitrile (10 mg), with a yield of 12.2%. LC/MS (ESI⁺) calcd for C₄₁H₄₄N₆O₄ ([M+H]⁺) *m*/*z* 685.3. ¹H NMR (400 MHz, CDCl₃) *δ* 8.05 (s, 1H), 7.64 (d, *J* = 8.3 Hz, 1H), 7.37 (d, *J* = 7.9 Hz, 1H), 7.11 (dd, *J* = 13.3, 5.2 Hz, 3H), 7.00 (d, *J* = 1.4 Hz, 1H), 6.61 (d, *J* = 8.4 Hz, 1H), 6.54 (s, 1H), 6.45 (d, *J* = 8.8 Hz, 2H), 5.19 (dd, *J=* 13.3, 5.1 Hz, 1H), 4.37 (d, *J* = 15.7 Hz, 1H), 4.22 (d, *J* = 15.7 Hz, 1H), 3.62 - 3.53 (m, 2H), 3.16 (t, *J* = 7.0 Hz, 2H), 2.95 - 2.77 (m, 2H), 2.40 (s, 3H), 2.32 (dd, *J* = 13.0, 5.2 Hz, 1H), 2.26 (s, 3H), 2.22 - 2.17 (m, 1H), 2.13 (s, 3H), 1.69 - 1.59 (m, 10H), 1.42 (s, 2H), 1.27 (dd, *J* = 7.5, 5.0 Hz, 2H).

### 62: 1-(4-((5-(3,5-dimethylisoxazol-4-yl)-2-methylphenyl)(4-((2-(2,6-dioxopiperidin-3-yl)-1-oxoisoindolin-5-yl)amino)butyl)amino)phenyl)cyclopropane-1-nitrile (62)

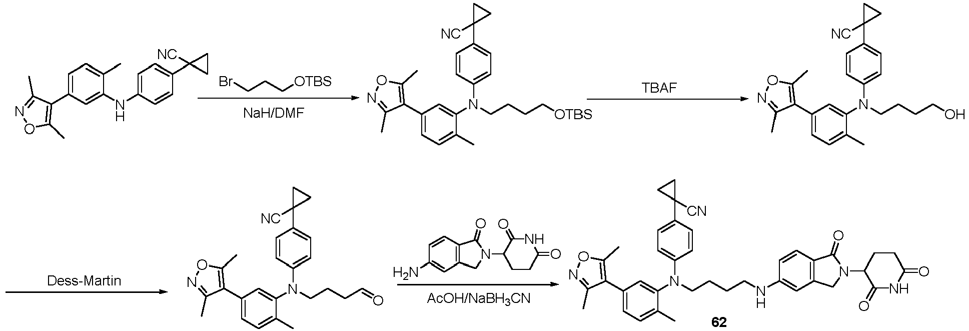

By referring to the method in the above example, the target compound was synthesized according to the above route. LC/MS (ESI⁺) calcd for C₃₉H₄₀N₆O₄ ([M+H]⁺) *m*/*z* 657.3. ¹H NMR (400 MHz, CDCl₃) *δ* 8.02 (s, 1H), 7.69 (d, *J* = 7.7 Hz, 1H), 7.37 (d, *J* = 7.9 Hz, 1H), 7.15 - 7.11 (m, 1H), 7.10 (d, *J* = 8.7 Hz, 2H), 6.99 (s, 1H), 6.81 (s, 2H), 6.45 (d, *J* = 8.6 Hz, 2H), 5.18 (dd, *J* = 12.1, 4.0 Hz, 1H), 4.30 (dd, *J* = 53.5, 16.0 Hz, 2H), 3.61 (s, 2H), 3.23 (s, 2H), 2.88 (t, *J* = 20.6 Hz, 2H), 2.44 - 2.30 (m, 4H), 2.28 - 2.19 (m, 4H), 2.11 (s, 3H), 1.76 (s, 4H), 1.60 (dd, *J* = 7.2, 4.9 Hz, 2H), 1.29 - 1.24 (m, 2H).

### 63: 2-chloro-4-((5-(3,5-dimethylisoxazol-4-yl)-2-methylphenyl)(5-((2-(2,6-dioxopiperidin-3-yl)-1-oxoisoindolin-5-yl)amino)pentyl)amino)benzonitrile (63)

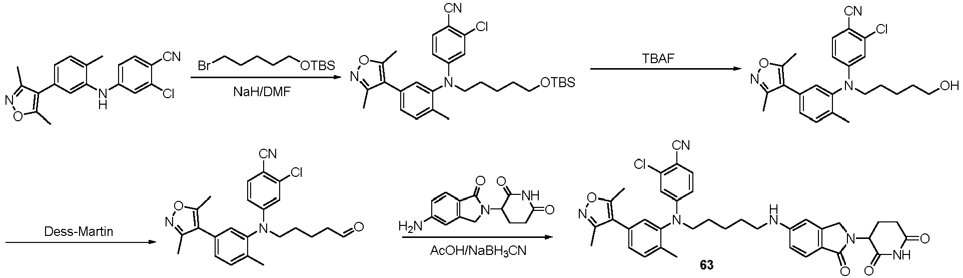

By referring to the method in the above example, the target compound was synthesized according to the above route. LC/MS (ESI⁺) calcd for C₃₇H₃₇ClN₆O₄ ([M+H]⁺) *m*/*z* 665.2.

### 64: 1-(4-((5-(3,5-Dimethylisoxazol-4-yl)-2-methylphenyl)(2-(4-(2-(2,6-dioxopiperidin-3-yl)-1,3-dioxoisoindolin-5-yl)piperazin-1-yl)ethyl)amino)phenyl)cyclopropane-1-nitrile (64)

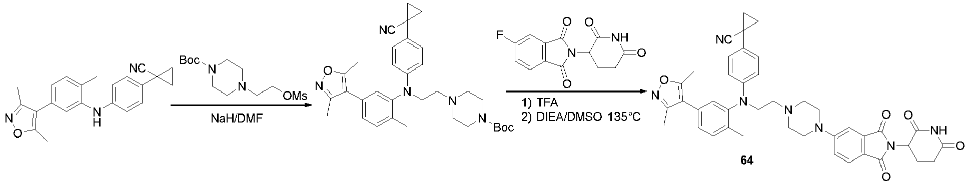

1-(4-((5-(3,5-Dimethylisoxazol-4-yl)-2-methylphenyl)amino)phenyl)cyclopropane-1-carbonitril e (170 mg, 0.5 mmol) was dissolved in 5 mL of DMF, to which was added NaH (40 mg, 1.0 mmol), and the mixture was stirred for 5 min, followed by addition of t-butyl 4-(2-((methylsulfonyl)oxy)ethyl)piperazine-1-carboxylic acid (231 mg, 0.75 mmol). The mixture was allowed to react 4 h at 60 °C, and then 10 mL of water was added to quench the reaction. The reaction solution was extracted with 15 mL of ethyl acetate. The organic layer was washed with saturated brine, dried over anhydrous sodium sulfate, concentrated to dryness under reduced pressure, and separated and purified by TLC, to provide the product t-butyl 4-(2-(((4-(1-cyanocyclopropyl)phenyl)(5-(3,5-dimethylisoxazol-4-yl)-2-methylphenyl)amino)et hyl)piperazine-1-carboxylat (52 mg), with a yield of 19%. LC/MS (ESI⁺) calcd for C₃₃H₄₁N₅O₃ ([M+H]⁺) *m*/*z* 555.3.

t-Butyl 4-(2-(((4-(1-cyanocyclopropyl)phenyl)(5-(3,5-dimethylisoxazol-4-yl)-2-methylphenyl) amino)ethyl)piperazine-1-carboxylate (50 mg, 0.09 mmol) was dissolved in 5 mL of dichloromethane, to which was added 3 mL of TFA. The solution was stirred at room temperature for 2 h, and then concentrate to dryness under reduced pressure, to which was added 10 mL of ethyl acetate. The resultant solution was washed twice with the saturated aqueous solution of sodium bicarbonate, washed once with saturated brine, dried over anhydrous sodium sulfate, and concentrated to dryness under reduced pressure, to obtain 20 mg of product. The obtained product was dissolved in 3 mL of DMSO, to which were successively added 2-(2,6-dioxopiperidin-3-yl)-5-fluoroisoindoline-1,3-dione (12 mg, 0.043 mmol) and DIEA (0.5 mL). The solution was allowed to react 3 h at 135 °C, and then cooled to room temperature, to which was added 10 mL of ethyl acetate. The resultant solution was washed twice with 8 mL of water, washed once with saturated brine, and then concentrated to dryness under reduced pressure. The residue was separated and purified by TLC, to provide the product 1-(4-((5-(3,5-dimethylisoxazol-4-yl)-2-methylphenyl)(2-(4-(2-(2,6-dioxopiperidin-3-yl)-1,3-dioxoisoindolin-5-yl)piperazin-1-yl)ethyl)amino)phenyl)cyclopropane-1-nitrile (7 mg), with a yield of 23%. LC/MS (ESI⁺) calcd for C₄₁H₄₁N₇O₅ ([M+H]⁺) *m*/*z* 712.3. ¹H NMR (400 MHz, CDCl₃) *δ* 8.03 (s, 1H), 7.74 (d, *J* = 8.0 Hz, 1H), 7.41 (d, *J* = 7.5 Hz, 1H), 7.30 (s, 1H), 7.17 (d, *J* = 7.4 Hz, 3H), 7.09 (d, *J* = 9.0 Hz, 1H), 6.99 (d, *J* = 7.5 Hz, 1H), 6.66 (s, 2H), 4.94 (dd, *J* = 12.4, 5.6 Hz, 1H), 4.33 (s, 2H), 3.90 (s, 4H), 3.70 (s, 2H), 2.99 - 2.67 (m, 6H), 2.41 (s, 3H), 2.29 - 2.23 (m, 3H), 2.16 (s, 3H), 2.14 - 2.10 (m, 1H), 2.02 (d, *J* = 12.9 Hz, 1H), 1.29 (d, *J* = 2.5 Hz, 4H).

### 65: 1-(4-((5-(3,5-dimethylisoxazol-4-yl)-2-methylphenyl)(2-(4-(2-(2,6-dioxopiperidin-3-yl)-1-oxoisoindolin-5-yl)piperazin-1-yl)ethyl)amino)phenyl)cyclopropane-1-nitrile (65)

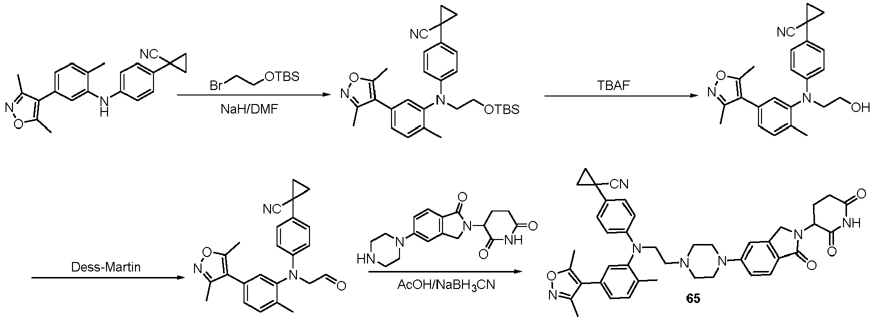

By referring to the method in the above example, the target compound was synthesized according to the above route. LC/MS (ESI⁺) calcd for C₄₁H₄₃N₇O₄ ([M+H]⁺) *m*/*z* 698.3. ¹H NMR (400 MHz, CDCl₃) *δ* 7.73 (d, *J* = 8.5 Hz, 1H), 7.38 (d, *J* = 7.7 Hz, 1H), 7.12 (d, *J* = 8.6 Hz, 3H), 7.05 (s, 1H), 6.97 (d, *J* = 9.3 Hz, 1H), 6.85 (s, 1H), 6.51 (d, *J* = 8.6 Hz, 2H), 5.20 (dd, *J* = 13.2, 4.9 Hz, 1H), 4.41 (d, *J* = 15.8 Hz, 1H), 4.26 (d, *J* = 15.7 Hz, 1H), 3.85 - 3.79 (m, 2H), 3.29 (s, 4H), 3.00 (d, *J* = 6.6 Hz, 2H), 2.77 - 2.70 (m, 2H), 2.65 (s, 4H), 2.40 (s, 3H), 2.32 (d, *J* = 7.8 Hz, 2H), 2.27 (s, 3H), 2.15 (s, 3H), 1.27 (d, *J* = 9.3 Hz, 4H).

### 66: 1-(4-((5-(3,5-dimethylisoxazol-4-yl)-2-methylphenyl)(1-(1-(2-(2,6-dioxopiperidin-3-yl)-1,3-dioxoisoindolin-5-yl)piperidin-4-ylazetidin-3-yl)amino)phenyl)cyclopropane-1-nitrile (66)

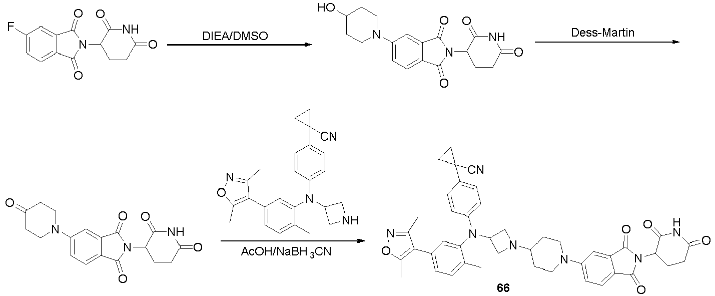

2-(2,6-Dioxopiperidin-3-yl)-5-fluoroisoindole-1,3-dione (414 mg, 1.5 mmol) and piperidin-4-ol (202 mg, 2.0 mmol) were dissolved in 5 mL of DMSO, to which was added DIEA (0.5 mL). The solution was allowed to react overnight at 130 °C, and then cooled to room temperature, to which was added 10 mL of ethyl acetate. The resultant solution was washed twice with 8 mL of water, washed once with saturated brine, and then concentrated to dryness under reduced pressure. The residue was separated and purified by TLC, to provide the product 2-(2,6-dioxopiperidin-3-yl)-5-(4-hydroxypiperidin-1-yl)isoindoline-1,3-dione (180 mg), with a yield of 33.6%. LC/MS (ESI⁺) calcd for C₁₈H₁₉N₃O₅ ([M+H]⁺) *m*/*z* 358.1.

2-(2,6-Dioxopiperidin-3-yl)-5-(4-hydroxypiperidin-1-yl)isoindoline-1,3-dione (180 mg, 0.50 mmol) was dissolved in 10 mL of dichloromethane, to which was added Dess-Martin periodinane (424 mg, 1.0 mmol), and the mixture was allowed to react at room temperature. The reaction solution was filtered. The filtrate was concentrated to dry, and the residue was separated and purified by TLC, to provide 2-(2,6-dioxopiperidin-3-yl)-5-(4-oxopiperidin-1-yl) isoindoline-1,3-dione (150 mg), with a yield of 84.4%. LC/MS (ESI⁺) calcd for C₁₈H₁₇N₃O₅ ([M+H]⁺) *m*/*z* 356.1.

2-(2,6-Dioxopiperidin-3-yl)-5-(4-oxopiperidin-1-yl)isoindoline-1,3-dione (28 mg, 0.079 mmol) and 1-(4-(azetidin-3-yl(5-(3,5-dimethylisoxazol-4-yl)-2-methylphenyl)amino)phenyl) cyclopropane-1-nitrile (35 mg, 0.088 mmol) were dissolved in 5 mL of dichloromethane, to which was added one drop of acetic acid. The mixture was stirred for 1 h at room temperature, and then sodium cyanoborohydride (63 mg, 1.0 mmol) was added. The resultant solution was allowed to react at room temperature overnight, and then washed with 5 mL of water. The organic layer was concentrated under reduced pressure to dry. The residue was separated and purified by TLC, to provide the product 1-(4-((5-(3,5-dimethylisoxazol-4-yl)-2-methylphenyl) (1-(1-(2-(2,6-dioxopiperidin-3-yl)-1,3-dioxoisoindolin-5-yl)piperidin-4-ylazetidin-3-yl)amino)p henyl)cyclopropane-1-carbonitrile as yellow soild (11 mg), with a yield of 18.9%. LC/MS (ESI⁺) calcd for C₄₃H₄₃N₇O₅ ([M+H]⁺) *m*/*z* 738.3. ¹H NMR (400 MHz, CDCl₃) *δ* 8.14 (s, 1H), 7.66 (d, *J* = 8.5 Hz, 1H), 7.42 (d, *J* = 7.9 Hz, 1H), 7.25 (d, *J* = 1.5 Hz, 1H), 7.18 (d, *J =* 7.4 Hz, 1H), 7.12 (d, *J* = 8.7 Hz, 2H), 7.02 (d, *J* = 8.5 Hz, 1H), 6.91 (s, 1H), 6.37 (d, *J* = 8.6 Hz, 2H), 4.93 (dd, *J* = 12.2, 5.2 Hz, 1H), 4.58 - 4.48 (m, 1H), 3.84 - 3.66 (m, 4H), 3.06 (t, *J* = 10.2 Hz, 2H), 2.87 (s, 1H), 2.82 - 2.74 (m, 4H), 2.40 (s, 3H), 2.27 (s, 3H), 2.20 (s, 4H), 1.75 (d, *J* = 10.5 Hz, 2H), 1.61 (dd, *J* = 7.2, 4.8 Hz, 2H), 1.44 - 1.37 (m, 2H), 1.26 (t, *J* = 5.7 Hz, 4H).

### 67: 1-(4-((5-(3,5-dimethylisoxazol-4-yl)-2-methylphenyl)(1-((1-(2-(2,6-dioxopiperidin-3-yl)-1,3-dioxoisoindolin-5-yl)azetidin-3-yl)methyl)azetidine-3-yl)amino)phenyl)cyclopropane-1 -carbonitrile (67)

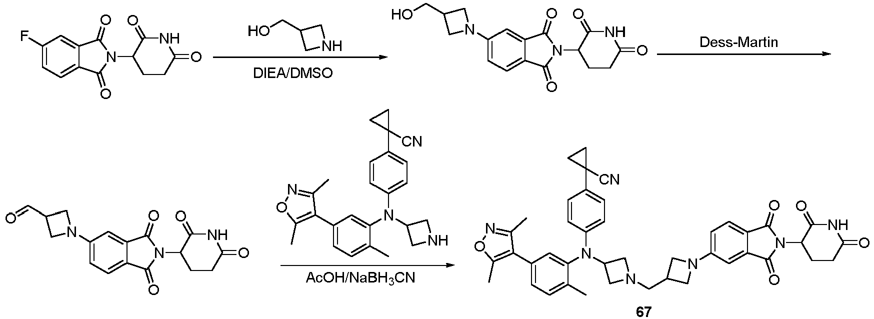

By referring to the method in the above example, the target compound was synthesized according to the above route. LC/MS (ESI⁺) calcd for C₄₂H₄₁N₇O₅ ([M+H]⁺) *m*/*z* 724.3. ¹H NMR (400 MHz, CDCl₃) *δ* 8.60 (s, 1H), 7.63 (d, *J* = 8.3 Hz, 1H), 7.43 (d, *J* = 8.0 Hz, 1H), 7.23 - 7.17 (m, 1H), 7.13 (d, *J* = 8.7 Hz, 2H), 6.91 (s, 1H), 6.78 (d, *J* = 1.7 Hz, 1H), 6.48 (dd, *J* = 8.3, 1.9 Hz, 1H), 6.37 (d, *J* = 8.8 Hz, 2H), 4.92 (dd, *J* = 12.1, 5.3 Hz, 1H), 4.72-4.66 (m, 1H), 4.10 (t, *J* = 7.2 Hz, 2H), 4.02 (d, *J* = 5.2 Hz, 1H), 3.70 (d, *J* = 5.8 Hz, 2H), 3.03 (s, 2H), 2.86 (t, *J* = 15.2 Hz, 4H), 2.77 (dd, *J* = 19.5, 9.5 Hz, 2H), 2.40 (s, 3H), 2.26 (s, 3H), 2.20 (s, 3H), 2.15 - 2.08 (m, 2H), 1.62 (dd, *J* = 7.3, 4.8 Hz, 2H), 1.30 - 1.27 (m, 2H).

### 68: 1-(4-((5-(3,5-dimethylisoxazol-4-yl)-2-methylphenyl)(1-((1s,3s)-3-((2-(2,6-dioxopiperidin-3-yl)-6-fluoro-3-oxoisoindolin-5-yl)amino)cyclobutyl)azetidin-3-yl)amino)p henyl)cyclopropane-1-nitrile (68)

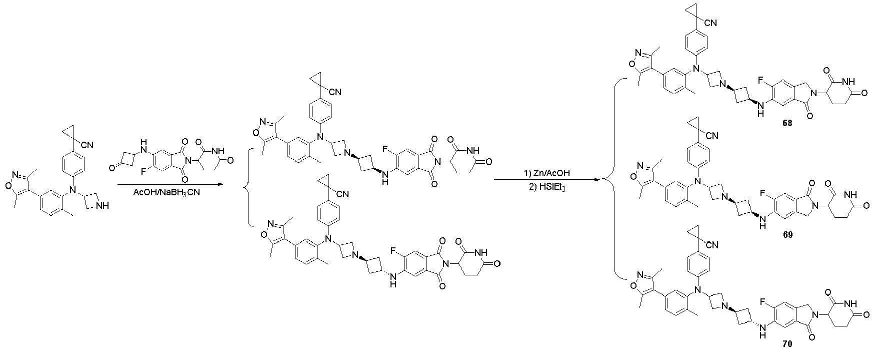

By referring to the method in the above example, the target compound was synthesized according to the above route. LC/MS (ESI⁺) calcd for C₄₂H₄₂FN₇O₄ ([M+H]⁺) *m*/*z* 728.3.

### 69: 1-(4-((5-(3,5-dimethylisoxazol-4-yl)-2-methylphenyl)(1-((1s,3s)-3-((2-(2,6-dioxopiperidin-3-yl)-6-fluoro-1-oxoisoindolin-5-yl)amino)cyclobutyl)azetidin-3-yl)amino)p henyl)cyclopropane-1-nitrile (69)

By referring to the method in the above example, the target compound was synthesized according to the above route. LC/MS (ESI⁺) calcd for C₄₂H₄₂FN₇O₄ ([M+H]⁺) *m*/*z* 728.3.

### 70: 1-(4-((5-(3,5-dimethylisoxazol-4-yl)-2-methylphenyl)(1-(((1r,3r)-3-((2-(2,6-dioxopiperidin-3-yl)-6-fluoro-3-oxoisoindolin-5-yl)amino)cyclobutyl)azetidin-3-yl)amino)p henyl)cyclopropane-1-nitrile (70)

By referring to the method in the above example, the target compound was synthesized according to the above route. LC/MS (ESI⁺) calcd for C₄₂H₄₂FN₇O₄ ([M+H]⁺) *m*/*z* 728.3.

### 71: 1-(4-((5-(3,5-dimethylisoxazol-4-yl)-2-methylphenyl)((1-((1s,3s)-3-((2-(2,6-dioxopiperidin-3-yl)-6-fluoro-1,3-dioxoisoindolin-5-yl)amino)cyclobutyl)azetidin-3-yl)met hyl)amino)phenyl)cyclopropane-1-nitrile (71)

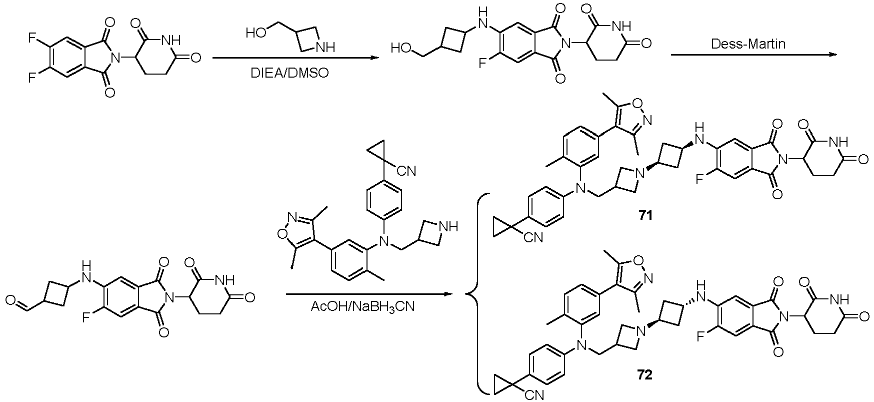

By referring to the method in the above example, the target compound was synthesized according to the above route. LC/MS (ESI⁺) calcd for C₄₃H₄₂FN₇O₅ ([M+H]⁺) *m*/*z* 756.3. ¹H NMR (400 MHz, CDCl₃) *δ* 8.21 - 8.05 (m, 1H), 7.41 (t, *J* = 8.9 Hz, 2H), 7.15 (t, *J* = 7.9 Hz, 3H), 7.01 (d, *J* = 7.2 Hz, 1H), 6.94 (d, *J* = 7.1 Hz, 1H), 6.50 (t, *J* = 7.6 Hz, 2H), 4.93 (dd, *J* = 12.3, 5.3 Hz, 1H), 4.83 - 4.76 (m, 1H), 4.16 (dd, *J* = 14.3, 7.4 Hz, 1H), 3.88 (d, *J* = 6.7 Hz, 2H), 3.37 (t, *J* = 7.1 Hz, 1H), 3.18 (s, 1H), 2.98 - 2.72 (m, 5H), 2.43 (s, 3H), 2.36 (d, *J* = 10.1 Hz, 1H), 2.28 (d, *J* = 9.3 Hz, 3H), 2.13 (d, *J* = 5.3 Hz, 3H), 2.02 - 1.95 (m, 2H), 1.63 (dd, *J* = 7.2, 4.5 Hz, 2H), 1.29 (dt, *J* = 7.0, 4.6 Hz, 4H).

### 72: 1-(4-((5-(3,5-dimethylisoxazol-4-yl)-2-methylphenyl][(1-((1r,3r)-3-((2-(2,6-dioxopiperidin-3-yl)-6-fluoro-1,3-dioxoisoindolin-5-yl)amino)cyclobutyl)azetidin-3-yl)met hyl)amino)phenyl)cyclopropane-1-nitrile (72)

By referring to the method in the above example, the target compound was synthesized according to the above route. LC/MS (ESI⁺) calcd for C₄₃H₄₂FN₇O₅ ([M+H]⁺) *m*/*z* 756.3. ¹H NMR (400 MHz, CDCl₃) *δ* 8.15 (s, 1H), 7.44 - 7.38 (m, 2H), 7.19 - 7.09 (m, 3H), 6.98 (d, *J* = 8.9 Hz, 2H), 6.50 (d, *J* = 8.7 Hz, 2H), 5.09 (s, 1H), 4.95 - 4.88 (m, 1H), 3.88 (d, *J* = 6.9 Hz, 2H), 3.79 (d, *J* = 7.0 Hz, 1H), 3.40 (t, *J* = 6.8 Hz, 2H), 3.04 (d, *J* = 6.4 Hz, 2H), 2.79 (ddd, *J* = 44.4, 33.7, 13.4 Hz, 6H), 2.42 (d, *J* = 5.3 Hz, 3H), 2.31 - 2.26 (m, 3H), 2.11 (s, 3H), 2.08 (s, 1H), 1.63 (dd, *J* = 7.2, 4.7 Hz, 2H), 1.32 - 1.25 (m, 4H)_{∘}

### 73: 1-(4-((5-(3,5-dimethylisoxazol-4-yl)-2-methylphenyl)(1-((1s,4s)-4-((2-(2,6-dioxopiperidin-3-yl)-6-fluoro-1,3-dioxoisoindolin-5-yl)amino)cyclohexyl)azetidin-3-yl)ami no)phenyl)cyclopropane-1-nitrile (73)

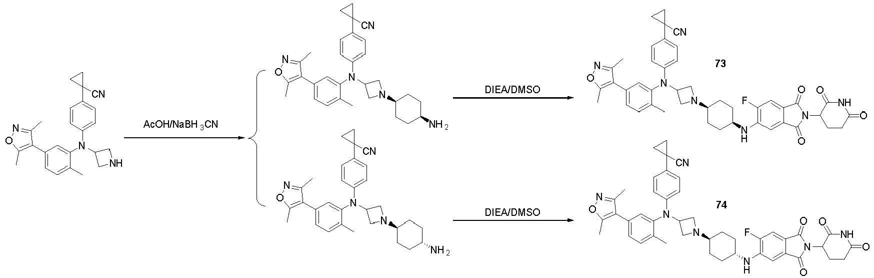

By referring to the method in the above example, the target compound was synthesized according to the above route. LC/MS (ESI⁺) calcd for C₄₄H₄₄FN₇O₅ ([M+H]⁺) *m*/*z* 770.3. ¹H NMR (400 MHz, CDCl₃) δ 8.28 (s, 1H), 7.40 (dd, *J* = 14.3, 8.8 Hz, 2H), 7.19 (d, *J* = 7.8 Hz, 1H), 7.12 (d, *J* = 8.8 Hz, 2H), 7.04 (d, *J* = 7.0 Hz, 1H), 6.91 (d, *J* = 1.5 Hz, 1H), 6.38 (d, *J* = 8.8 Hz, 2H), 4.91 (dd, *J* = 12.2, 5.3 Hz, 1H), 4.81 (s, 1H), 4.61 (s, 1H), 3.86 (s, 2H), 3.54 (s, 1H), 2.92 - 2.65 (m, 7H), 2.40 (s, 3H), 2.27 (s, 3H), 2.20 (s, 3H), 2.15 - 2.10 (m, 2H), 1.79 (s, 2H), 1.72 (s, 2H), 1.63 - 1.60 (m, 2H), 1.28 (dd, *J* = 7.6, 5.1 Hz, 4H).

### 74: 1-(4-((5-(3,5-dimethylisoxazol-4-yl)-2-methylphenyl)(1-(((1r,4r)-4-((2-(2,6-dioxopiperidin-3-yl)-6-fluoro-1,3-dioxoisoindolin-5-yl)amino)cyclohexyl)azetidin-3-yl)ami no)phenyl)cyclopropane-1-nitrile (74)

By referring to the method in the above example, the target compound was synthesized according to the above route. LC/MS (ESI⁺) calcd for C₄₄H₄₄FN₇O₅ ([M+H]⁺) *m*/*z* 770.3.

### 75: 1-(4-((5-(3,5-dimethylisoxazol-4-yl)-2-methylphenyl)((1-(1-(2-(2,6-dioxopiperidin-3-yl)-3-oxoisoindolin-5-yl)piperidin-4-yl)azetidin-3-yl)methyl)amino)phenyl)cyclopropane-1-carbonitrile (75)

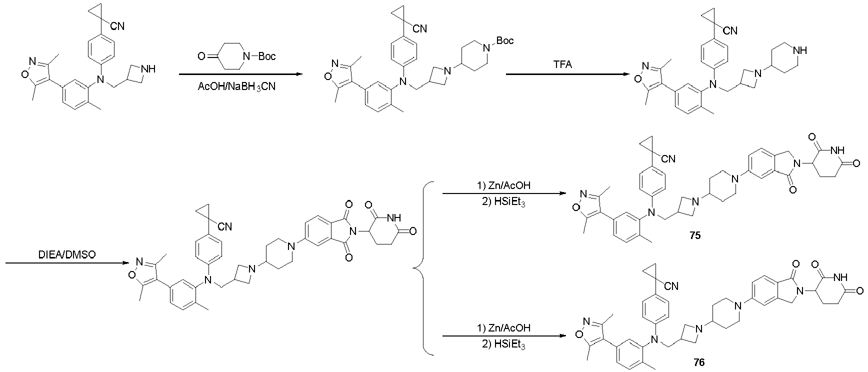

By referring to the method in the above example, the target compound was synthesized according to the above route. LC/MS (ESI⁺) calcd for C₄₄H₄₇N₇O₄ ([M+H]⁺) *m*/*z* 738.4. ¹H NMR (400 MHz, CDCl₃) *δ* 8.16 (s, 1H), 7.70 (d, *J* = 8.6 Hz, 1H), 7.37 (d, *J* = 7.8 Hz, 1H), 7.12 (dd, *J* = 13.3, 6.3 Hz, 3H), 6.97 (d, *J* = 1.3 Hz, 1H), 6.95 (dd, *J* = 9.0, 1.2 Hz, 1H), 6.86 (s, 1H), 6.51 (d, *J* = 8.3 Hz, 2H), 5.24 - 5.12 (m, 1H), 4.57 (s, 4H), 4.38 (d, *J* = 15.8 Hz, 1H), 4.25 (dd, *J* = 15.4, 8.2 Hz, 1H), 3.90 (d, *J* = 5.2 Hz, 2H), 3.75 (d, *J* = 12.6 Hz, 2H), 3.67 (s, 1H), 3.51 (s, 1H), 2.95 - 2.83 (m, 4H), 2.40 (s, 3H), 2.37 (dd, *J* = 7.8, 4.0 Hz, 2H), 2.34 - 2.29 (m, 2H), 2.27 (s, 3H), 2.10 (s, 3H), 1.81 (s, 2H), 1.61 (dd, *J* = 7.3, 4.8 Hz, 2H), 1.28 (t, *J* = 3.7 Hz, 2H).

### 76: 1-(4-((5-(3,5-dimethylisoxazol-4-yl)-2-methylphenyl)((1-(1-(2-(2,6-dioxopiperidin-3-yl)-1-oxoisoindolin-5-yl)piperidinyl-4-yl)azetidin-3-yl)methyl)amino)phenyl)cyclopropane-1-carbonitrile (76)

By referring to the method in the above example, the target compound was synthesized according to the above route. LC/MS (ESI⁺) calcd for C₄₄H₄₇N₇O₄ ([M+H]⁺) *m*/*z* 738.4.

### 77: 1-(4-((5-(3,5-dimethylisoxazol-4-yl)-2-methylphenyl)((1'-(2-(2,6-dioxopiperidin-3-yl)-1,3-dioxoisoindolin-5-yl)-[1,4'-bipiperidine]-4-yl)methyl)amino)phenyl)cyclopropane-1-nit rile (77)

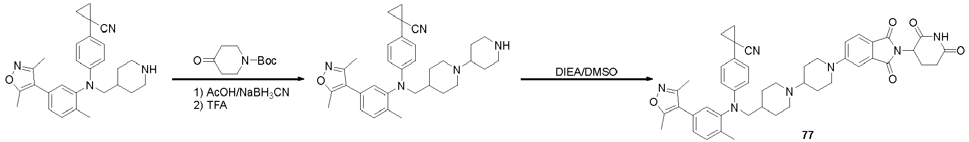

By referring to the method in the above example, the target compound was synthesized according to the above route. LC/MS (ESI⁺) calcd for C₄₆H₄₉N₇O₅ ([M+H]⁺) *m*/*z* 780.4. ¹H NMR (400 MHz, CDCl₃) *δ* 8.10 (s, 1H), 7.69 (d, *J* = 8.5 Hz, 1H), 7.37 (d, *J* = 7.9 Hz, 1H), 7.29 (s, 1H), 7.12 (t, *J* = 7.5 Hz, 3H), 7.06 (dd, *J* = 14.3, 8.0 Hz, 2H), 6.49 (d, *J* = 8.8 Hz, 2H), 4.95 (dd, *J* = 12.2, 5.2 Hz, 1H), 4.02 (d, *J* = 13.1 Hz, 2H), 3.53 (d, *J* = 6.3 Hz, 2H), 3.11 - 2.70 (m, 7H), 2.43 (s, 3H), 2.29 (s, 3H), 2.24 (d, *J* = 6.5 Hz, 1H), 2.18 - 2.12 (m, 1H), 2.09 (s, 3H), 2.00 (d, *J* = 14.9 Hz, 2H), 1.89 (d, *J* = 13.1 Hz, 5H), 1.67 (d, *J* = 12.8 Hz, 2H), 1.64 - 1.60 (m, 2H), 1.31 - 1.26 (m, 4H).

### 78: 1-(4-((5-(3,5-dimethylisoxazol-4-yl)-2-methylphenyl)((1-((1-(2-(2,6-dioxopiperidin-3-yl)-1,3-dioxoisoindolin-5-yl)azetidin-3-yl)methyl)piperidin-4-yl)methyl)amino)phenyl)cycl opropane-1-carbonitrile (78)

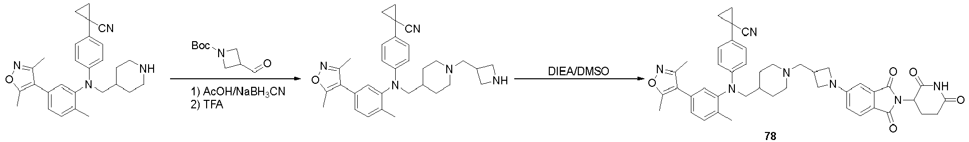

By referring to the method in the above example, the target compound was synthesized according to the above route. LC/MS (ESI⁺) calcd for C₄₅H₄₇N₇O₅ ([M+H]⁺) *m*/*z* 766.4. ¹H NMR (400 MHz, CDCl₃) *δ* 8.34 (s, 1H), 7.63 (d, *J* = 8.3 Hz, 1H), 7.36 (d, *J* = 7.9 Hz, 1H), 7.11 (t, *J* = 7.9 Hz, 3H), 7.05 (d, *J* = 1.4 Hz, 1H), 6.79 (d, *J* = 1.9 Hz, 1H), 6.53 - 6.41 (m, 3H), 4.92 (dd, *J* = 12.2, 5.3 Hz, 1H), 4.15 (d, *J* = 2.3 Hz, 1H), 3.67 (d, *J* = 8.1 Hz, 2H), 3.51 (d, *J* = 6.1 Hz, 2H), 3.13 - 2.73 (m, 6H), 2.69 (d, *J* = 7.0 Hz, 2H), 2.42 (s, 3H), 2.28 (s, 3H), 2.25 - 2.17 (m, 1H), 2.15 - 2.09 (m, 1H), 2.09 (d, *J* = 5.3 Hz, 3H), 2.00 (d, *J* = 10.5 Hz, 2H), 1.82 (d, *J* = 11.4 Hz, 2H), 1.61 (dd, *J* = 7.4, 4.8 Hz, 2H), 1.35 (d, *J* = 16.2 Hz, 2H), 1.28 (t, *J* = 3.7 Hz, 3H).

### 79: (2S,4R)-1-((S)-2-(2-((5-((3-chloro-4-cyanophenyl)(5-(3,5-dimethyl-1H-pyrazol-4-yl)-2-methylphenyl)amino)pentyl)oxy)acetylamino)-3,3-dimethylbutyryl)-4-hydroxy-N-((S)-1-(4-(4-methylthiazol-5-yl)phenyl)ethyl)pyrrolidinyl-2-formamide (79)

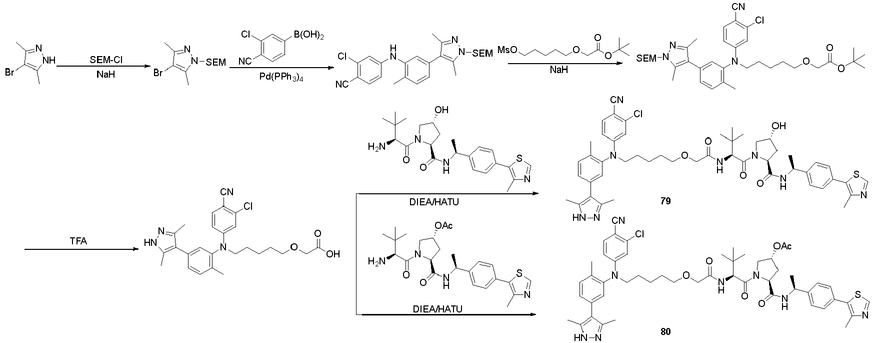

NaH (4.8 g, 120 mmol) was added to 150 mL of tetrahydrofuran, to which was added 3,5-dimethyl-4-bromopyrazole (10.5 g, 60 mmol) in an ice bath, and the mixture was stirred for 30 min, followed by addition of SEM-Cl (10.0 g, 60 mmol). The reaction solution was slowly warmed to room temperature, and stirred overnight. The reaction solution was poured to 500 mL of ice water, and extracted with 300 mL of ethyl acetate. The aqueous layer was further extracted with 100 mL of ethyl acetate. The organic layers were combined, washed with saturated brine, dried over anhydrous sodium sulfate, concentrated, and purified by column chromatography, to provide the product 4-bromo-3,5-dimethyl-1-((2-(trimethylsilyl)ethoxy) methyl)-1H-pyrazole (17 g), with a yield of 93%. LC/MS (ESI⁺) calcd for C₁₁H₂₁BrN₂OSi ([M+H]⁺) *m*/*z* 305.1.

4-Bromo-3,5-dimethyl-1-((2-(trimethylsilyl)ethoxy)methyl)-1H-pyrazole (7.29 g, 23.9 mmol) and (3-chloro-4-cyanophenyl)boric acid (3.97 g, 26.3 mmol) were dissolved in a mixed solution of toluene/water (1:1, 180 mL), to which were added potassium carbonate (8.3 g, 60 mmol) and add tetrakis(triphenylphosphine)palladium (1.39 g, 1.2 mmol). Under argon protection, the solution was allowed to react at 95 °C overnight, and then extracted with 100 mL of ethyl acetate. The aqueous layer was futher extracted once with 80 mL of ethyl acetate. The organic layers were combined, washed with saturated brine, dried over anhydrous sodium sulfate, concentrated, and purified by column chromatography, to provide 2-chloro-4-((5-(3,5-dimethyl-1-((2-(trimethylsilyl)ethoxy)methyl)-1H-pyrazol-4-yl)-2-methylphenyl)amino)benzonitrile (5.05 g), with a yield of 63%. LC/MS (ESI⁺) calcd for C₂₅H₃₁ClN₄OSi ([M+H]⁺) *m*/*z* 467.2. NaH (40 mg, 1.0 mmol) was added in 5 mL of tetrahydrofuran, to which was added 2-chloro-4-((5-(3,5-trimethylsilyl)ethoxy)methyl)-1H-pyrazol-4-yl)-2-methylphenyl)amino)ben zonitrile (233 mg, 1.0 mmol), and the mixture was stirred and reacted 30 min at room temperature, to which was added 2-t-butyl (4-((methanesulfonyl)oxy)butoxy)acetate (140 mg, 0.5 mmol). The mixture was allowed to react overnight at room temperature, and then washed with 10 ml of water, followed by extraction with 10 mL of ethyl acetate. The organic layer was concentrated under reduced pressure, and the residue was separated and purified by TLC, to provide t-butyl 2-((5-((3-chloro-4-cyanophenyl)(5-(3,5-dimethyl-1-((2-(trimethylsilyl)ethoxy) methyl)-1H-pyrazol-4-yl)-2-methylphenyl)amino)pentyl)oxy)acetate (260 mg), with a yield of 78%. LC/MS (ESI⁺) calcd for C₃₆H₅₁ClN₄O₄Si ([M+H]⁺) *m*/*z* 667.4.

2-((5-((3-Chloro-4-cyanophenyl)(5-(3,5-dimethyl-1-((2-(trimethylsilyl)ethoxy)methyl)-1H-pyra zol-4-yl)-2-methylphenyl)amino)pentyl)oxy)acetate (260 mg, 0.39 mmol) was dissolved in 3 mL of dichloromethane, to which was added 3 mL of trifluoroacetic acid, and the mixture was allowed to react at room temperature for 1 h. The reaction solution was concentrated to dryness under reduced pressure. The residue was washed with the saturated aqueous solution of sodium bicarbonate, and extracted with ethyl acetate. The organic layer was washed with saturated brine, dried over anhydrous sodium sulfate, and concentrated under reduced pressure, to provide crude 2-((5-((3-chloro-4-cyanophenyl)(5-(3,5-dimethyl-1-((2-(trimethylsilyl)ethoxy) methyl)-1H-pyrazol-4-yl)-2-methylphenyl)amino)pentyl)oxy)acetic acid (200 mg).

2-((5-((3-Chloro-4-cyanophenyl)(5-(3,5-dimethyl-1-((2-(trimethylsilyl)ethoxy)methyl)-1H-pyra zol-4-yl)-2-methylphenyl)amino)pentyl)oxy)acetic acid (100 mg, 0.75 mmol) was dissolved in 5 mL of DMF, to which were successively added *N*,*N*-diisopropylethylamine (66 mg, 0.51 mmol), HATU (70 mg, 0.18 mmol), and (2*S*,4*R*)-1-((*S*)-2-amino-3,3-dimethylbutyryl)-4-hydroxy-*N*-((*S*)-1-(4-(4-methylthiazol-5-yl)phenyl)ethyl)-pyrrolidinyl-2-formamide trifluoroacetate (103 mg, 0.18 mmol). The solution was allowed to react 2 h at room temperature, and then washed with 10 mL of water, followed by extraction with 10 mL of ethyl acetate. The organic layer was concentrated under reduced pressure, and the residue was separated and purified by TLC, to provide 20 mg of white solid product (2*S*,4*R*)-1-((*S*)-2-(2-((5-((3-chloro-4-cyanophenyl)(5-(3,5-dimethyl-1H-pyrazol-4-yl)-2-methylp henyl)amino)pentyl)oxy)acetylamino)-3,3-dimethylbutyryl)-4-hydroxy-N-((*S*)-1-(4-(4-methylth iazol-5-yl)phenyl)ethyl)pyrrolidinyl-2-formamide.

LC/MS (ESI⁺) calcd for C₄₉H₅₉ClN₈O₅S ([M+H]⁺) *m*/*z* 907.5. ¹H NMR (400 MHz, DMSO-*d₆*) *δ* 12.30 (s, 1H), 8.98 (s, 1H), 8.43 (d, *J* = 7.8 Hz, 1H), 7.59 (d, *J* = 9.0 Hz, 1H), 7.44 (dd, *J* = 8.0, 5.0 Hz, 3H), 7.36 (d, *J* = 8.3 Hz, 2H), 7.32 - 7.25 (m, 2H), 7.06 (s, 1H), 6.61 (s, 1H), 6.47 (s, 1H), 5.14 (d, *J* = 3.4 Hz, 1H), 4.93 - 4.85 (m, 1H), 4.52 (d, *J* = 9.6 Hz, 1H), 4.43 (t, *J* = 8.4 Hz, 1H), 4.28 (s, 1H), 3.89 (s, 2H), 3.83 (d, *J* = 8.8 Hz, 1H), 3.56 (t, *J =* 9.7 Hz, 2H), 3.44 (d, *J =* 6.3 Hz, 2H), 2.45 (s, 3H), 2.19 (s, 6H), 2.06 (s, 3H), 2.03 (s, 1H), 1.77 (d, *J=* 8.5 Hz, 1H), 1.59 (dd, *J =* 19.4, 12.7 Hz, 4H), 1.43 (dd, *J* = 20.6, 7.7 Hz, 2H), 1.34 (d, *J* = 7.0 Hz, 3H), 0.90 (s, 9H).

### 80: (3R,5S)-1-((S)-2-(2-((5-((3-chloro-4-cyanophenyl)(5-(3,5-dimethyl-1H-pyrazol-4-yl)-2-methylphenyl)amino)pentyl)oxy)acetylamino)-3,3-dimethylbutyryl)-5-(((S)-1-(4-(4-meth ylthiazol-5-yl)phenyl)ethyl)pyrrolidinyl-3-acetate (80)

2-((5-((3-Chloro-4-cyanophenyl)(5-(3,5-dimethyl-1-((2-(trimethylsilyl)ethoxy)methyl)-1H-pyra zol-4-yl)-2-methylphenyl)amino)pentyl)oxy)acetic acid (100 mg, 0.75 mmol) was dissolved in 5 mL of DMF, to which were successively added *N*,*N-*diisopropylethylamine (66 mg, 0.51 mmol), HATU (70 mg, 0.18 mmol), and (3*R*,5*S*)-1-((*S*)-2-amino-3,3-dimethylbutyryl)-5-(((*S*)-1-(4-(4-methylthiazol-5-yl)phenyl)ethyl)aminomethyl)pyrrolidinyl-3-acetate trifluoroacetate (108 mg, 0.18 mmol). The solution was allowed to react 2 h at room temperature, and then washed with 10 mL of water, followed by extraction with 10 mL of ethyl acetate. The organic layer was concentrated under reduced pressure, and the residue was separated and purified by TLC, to provide 25 mg of white solid product (3*R*,5*S*)-1-((*S*)-2-(2-((5-((3-chloro-4-cyanophenyl)(5-(3,5-dimethyl-1H-pyrazol-4-yl)-2-methylp henyl)amino)pentyl)oxy)acetylamino)-3,3-dimethylbutyryl)-5-(((*S*)-1-(4-(4-methylthiazol-5-yl) phenyl)ethyl)pyrrolidinyl-3-acetate.

LC/MS (ESI⁺) calcd for C₅₁H₆₁ClN₈O₆S ([M+H]⁺) *m*/*z* 949.5.

¹H NMR (400 MHz, DMSO-*d₆*) *δ* 12.44 - 12.20 (m, 1H), 8.98 (s, 1H), 8.48 - 8.41 (m, 1H), 7.62 - 7.56 (m, 1H), 7.43 (s, 3H), 7.40 - 7.34 (m, 2H), 7.34 - 7.30 (m, 1H), 7.29 - 7.24 (m, 1H), 7.08 - 7.03 (m, 1H), 6.66 - 6.56 (m, 1H), 6.49 - 6.40 (m, 1H), 5.23 - 5.16 (m, 1H), 4.92 - 4.85 (m, 1H), 4.49 - 4.43 (m, 1H), 4.43 - 4.38 (m, 1H), 3.89 (s, 2H), 3.87 - 3.83 (m, 1H), 3.78 - 3.72 (m, 1H), 3.53 - 3.41 (m, 3H), 2.69 (s, 1H), 2.45 (s, 3H), 2.29 - 2.23 (m, 1H), 2.19 (s, 6H), 2.06 (s, 3H), 1.98 (s, 3H), 1.72 - 1.51 (m, 5H), 1.46 - 1.38 (m, 2H), 1.35 (d, *J* = 7.0 Hz, 3H), 0.91 (d, *J* = 6.4 Hz, 9H).

### 81: (3R,5S)-1-((S)-2-(2-((5-(4-(3-((3-chloro-4-cyanophenyl)(ethyl)amino)-4-methylphenyl)-3,5-dimethyl-1H-pyrazol-1-yl)pentyl)oxy)acetamido)-3,3-dimethylbutyryl)-5-((S)-1-(4-met hylthiazol-5-yl)phenyl)ethyl)carbamoyl)pyrrolidine-3-yl acetate (81)

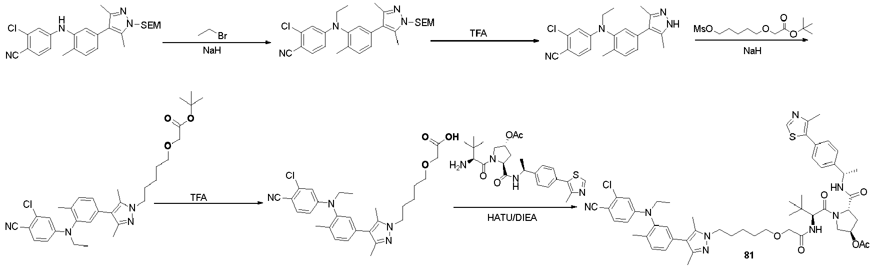

2-Chloro-4-((5-(3,5-dimethyl-1-((2-(trimethylsilyl)ethoxy)methyl)-1H-pyrazol-4-yl)-2-methylp henyl)amino)benzonitrile (467 mg, 1.0 mmol) was dissolved in 40 mL of tetrahydrofuran, to which was added NaH (240 mg, 6.0 mmol), and the mixture was stirred at room temperature for 10 min, followed by addition of bromoethanol (545 mg, 5.0 mmol). The resultant solution was stirred at room temperature for 3 h, and the reaction was quenched with 40 mL of ice water. The solution was extracted with 40 mL of ethyl acetate. The organic layer was washed with saturated brine, dried over anhydrous sodium sulfate, concentrated under reduced pressure to dryness, and separated and purified by TLC, to provide 2-chloro-4-((5-(3,5-dimethyl-1-((2-(trimethylsilyl)ethoxy)methyl)-1H-pyrazol-4-yl)-2-methylphenyl)(ethyl)amino)benzonitrile (250 mg), with a yield of 50%. LC/MS (ESI⁺) calcd for C₂₇H₃₅ClN₄OSi ([M+H]⁺) *m*/*z* 495.2. 2-Chloro-4-((5-(3,5-dimethyl-1-((2-(trimethylsilyl)ethoxy)methyl)-1H-pyrazol-4-yl)-2-methylp henyl)(ethyl)amino)benzonitrile (250 mg, 0.5 mmol) was dissolved in 5 mL of dichloromethane, to which was added 5 mL of trifluoroacetic acid, and the mixture was allowed to react at room temperature for 4 h. The reaction solution was concentrated to dryness under reduced pressure. The residue was washed with the saturated aqueous solution of sodium bicarbonate, and extracted with ethyl acetate. The organic layer was washed with saturated brine, dried over anhydrous sodium sulfate, and concentrated under reduced pressure, to provide crude 2-chloro-4-((5-(3,5-dimethyl-1H-pyrazol-4-yl)-2-methylphenyl)(ethyl)amino)benzonitrile (200 mg). LC/MS (ESI⁺) calcd for C₂₁H₂₁ClN₄ ([M+H]⁺) *m*/*z* 365.1.

2-Chloro-4-((5-(3,5-dimethyl-1H-pyrazol-4-yl)-2-methylphenyl)(ethyl)amino)benzonitrile (60 mg, 0.16 mmol) was dissolved in 5 mL of tetrahydrofuran, to which was added NaH (13 mg, 0.32 mmol) in an ice bath, and the mixture was stirred for 30 min, followed by addition of 2-t-butyl (4-((methanesulfonyl)oxy)butoxy)acetate (90 mg, 0.32 mmol). The resultant solution was allowed to react overnight at room temperature, and the reaction solution was washed with 10 mL of water. The solution was extracted with 10 mL of ethyl acetate. The organic layer was concentrated under reduced pressure, and separated and purified by TLC, to provide t-butyl 2-((5-(4-(3-((3-chloro-4-cyanophenyl)(ethyl)amino)-4-methylphenyl)-3,5-dimethyl-1H-pyrazol -1-yl)pentyl)oxy)acetate (60 mg). The product was dissolved in 5 mL of dichloromethane, to which was added 5 mL of trifluoroacetic acid, and the mixture was allowed to react at room temperature for 2 h. The reaction solution was concentrated to dryness under reduced pressure. The residue was washed with the saturated aqueous solution of sodium bicarbonate, and extracted with ethyl acetate. The organic layer was washed with saturated brine, dried over anhydrous sodium sulfate, and concentrated under reduced pressure, to provide 2-((5-(4-(3-((3-chloro-4-cyanophenyl)(ethyl)amino)-4-methylphenyl)-3,5-dimethyl-1H-pyrazol -1-yl)pentyl)oxy)acetic acid (50 mg), with a yield of 61.4%. LC/MS (ESI⁺) calcd for C₂₈H₃₃CIN₄O₃ ([M+H]⁺) *m*/*z* 509.2. 2-((5-(4-(3-((3-Chloro-4-cyanophenyl)(ethyl)amino)-4-methylphenyl)-3,5-dimethyl-1H-pyrazol -1-yl)pentyl)oxy)acetic acid (50 mg, 0.1 mmol) was dissolved in 5 mL of DMF, to which were successively added N,N-diisopropylethylamine (39 mg, 0.3 mmol), HATU (42 mg, 0.11 mmol), and (3*R*,5*S*)-1-((*S*)-2-amino-3,3-dimethylbutyryl)-5-(((*S*)-1-(4-(4-methylthiazol-5-yl)phenyl) ethyl)aminomethyl)pyrrolidinyl-3-acetate (53 mg, 0.11 mmol). The solution was allowed to react 2 h at room temperature, and then washed with 10 mL of water, followed by extraction with 10 mL of ethyl acetate. The organic layer was concentrated under reduced pressure, and the residue was separated and purified by TLC, to provide 12 mg of white solid product, which was the target compound (3*R*,5*S*)-1-((*S*)-2-(2-((5-(4-(3-((3-chloro-4-cyanophenyl) (ethyl)amino)-4-methylphenyl)-3,5-dimethyl-1H-pyrazol-1-yl)pentyl)oxy)acetamido)-3,3-dimet hylbutyryl)-5-((*S*)-1-(4-methylthiazol-5-yl)phenyl)ethyl)carbamoyl)pyrrolidine-3-yl acetate. LC/MS (ESI⁺) calcd for C₅₃H₆₅ClN₈O₆S ([M+H]⁺) *m*/*z* 977.3.

¹H NMR (400 MHz, DMSO-*d₆*) *δ* 8.98 (s, 1H), 8.51 - 8.43 (m, 1H), 7.61 - 7.56 (m, 1H), 7.43 (d, *J* = 8.6 Hz, 3H), 7.36 (d, *J* = 8.4 Hz, 3H), 7.27 - 7.22 (m, 1H), 7.02 (d, *J* = 1.7 Hz, 1H), 6.70 - 6.61 (m, 1H), 6.48 - 6.38 (m, 1H), 5.20 (s, 1H), 4.92 - 4.85 (m, 1H), 4.47 (t, *J* = 8.4 Hz, 1H), 4.42 (d, *J* = 9.1 Hz, 1H), 3.98 (t, *J* = 6.8 Hz, 2H), 3.91 (d, *J* = 2.9 Hz, 2H), 3.89 - 3.85 (m, 1H), 3.76 (dd, *J* = 11.7, 4.0 Hz, 1H), 3.47 (t, *J* = 6.4 Hz, 2H), 2.45 (s, 3H), 2.30 - 2.24 (m, 1H), 2.22 (d, *J* = 1.9 Hz, 3H), 2.13 (s, 3H), 2.08 (s, 3H), 1.99 (s, 3H), 1.96 (dd, *J* = 10.2, 3.7 Hz, 1H), 1.77 - 1.72 (m, 2H), 1.61 - 1.54 (m, 2H), 1.35 (d, *J* = 7.0 Hz, 5H), 1.16 (t, *J* = 7.1 Hz, 3H), 0.93 (s, 9H).

### 82: (3R,5S)-1-((S)-2-(2-((5-((3-chloro-4-cyanophenyl)(5-(1-ethyl-3,5-dimethyl-1H-pyrazol-4-yl)-2-methylphenyl)amino)pentyl)oxy)acetamido)-3,3-dimethylbutyryl)-5-((S)-1-(4-meth ylthiazol-5-yl)phenyl)ethyl)carbamoyl)pyrrolidine-3-yl acetate (82)

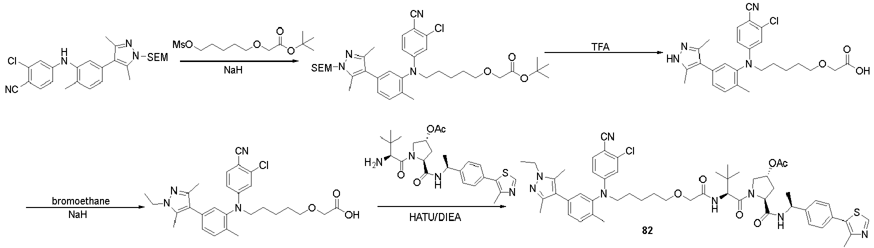

NaH (100 mg, 2.5 mmol) was added in 5 mL of tetrahydrofuran, to which was added 2-chloro-4-((5-(3,5-dimethyl-1-((2-(trimethylsilyl)ethoxy)methyl)-1H-pyrazol-4-yl)-2-methylp henyl)amino)benzonitrile (467 mg, 1.0 mmol), and the mixture was stirred and reacted 10 min at room temperature, to which was added 2-t-butyl (4-((methanesulfonyl)oxy)pentyloxy)acetate (281 mg, 1.0 mmol). The mixture was allowed to react overnight at room temperature, and then washed with 10 ml of water, followed by extraction with 10 mL of ethyl acetate. The organic layer was concentrated under reduced pressure, and the residue was separated and purified by TLC, to provide compound t-butyl 2-((5-((3-chloro-4-cyanophenyl)(5-(3,5-dimethyl-1-((2-(trimethylsilyl)ethoxy)methyl)-1H-pyrazol-4-yl)-2-methylphenyl)amino)pentyl)oxy)acetate (40 mg), with a yield of 6%. LC/MS (ESI⁺) calcd for C₃₆H₅₁ClN₄O₄Si ([M+H]⁺) *m*/*z* 667.4.

2-((5-((3-Chloro-4-cyanophenyl)(5-(3,5-dimethyl-1-((2-(trimethylsilyl)ethoxy)methyl)-1H-pyra zol-4-yl)-2-methylphenyl)amino)pentyl)oxy)acetate (40 mg, 0.06 mmol) was dissolved in 3 mL of dichloromethane, to which was added 3 mL of trifluoroacetic acid, and the mixture was stirred at room temperature for 1 h. The reaction solution was concentrated to dryness under reduced pressure. The residue was washed with the saturated aqueous solution of sodium bicarbonate, and extracted with ethyl acetate. The organic layer was washed with saturated brine, dried over anhydrous sodium sulfate, and concentrated under reduced pressure, to provide 20 mg of 2-((5-((3-chloro-4-cyanophenyl)(5-(3,5-dimethyl-1-((2-(trimethylsilyl) ethoxy)methyl)-1H-pyrazol-4-yl)-2-methylphenyl)amino)pentyl)oxy)acetic acid, with a yield of 66%. LC/MS (ESI⁺) calcd for C₂₆H₂₉ClN₄O₃ ([M+H]⁺) *m*/*z* 480.2.

2-((5-((3-Chloro-4-cyanophenyl)(5-(3,5-dimethyl-1-((2-(trimethylsilyl)ethoxy)methyl)-1H-pyra zol-4-yl)-2-methylphenyl)amino)pentyl)oxy)acetic acid (20 mg, 0.04 mmol) was dissolved in 3 mL of tetrahydrofuran, to which was added NaH (4 mg, 0.1 mmol). The mixture was stirred at room temperature for 10 min, to which was added bromoethane (7 mg, 0.06 mmol). The mixture was allowed to react 3 h at room temperature, and then washed with 10 ml of water, followed by extraction with 10 mL of ethyl acetate. The organic layer was concentrated under reduced pressure, and the residue was separated and purified by TLC, to provide 15 mg of compound 2-((5-((3-chloro-4-cyanophenyl)(5-(1-ethyl-3,5-dimethyl-1H-pyrazol-4-yl)-2-methylphenyl)amino)pentyl)oxy)acetic acid, with a yield of 74%. LC/MS (ESI⁺) calcd for C₂₈H₃₃ClN₄O₃ ([M+H]⁺) *m*/*z* 509.2.

2-((5-((3-Chloro-4-cyanophenyl)(5-(1-ethyl-3,5-dimethyl-1H-pyrazol-4-yl)-2-methylphenyl)am ino)pentyl)oxy)acetic acid (15 mg, 0.03 mmol) was dissolved in 3 mL of DMF, to which were added diisopropylethylamine (19.3 mg, 0.15 mmol), HATU (13 mg, 0.033 mmol), and (3*R*,5*S*)-1-((*S*)-2-amino-3,3-dimethylbutyryl)-5-(((*S*)-1-(4-(4-methylthiazol-5-yl)phenyl)ethyl)a minomethyl)pyrrolidinyl-3-acetate (15 mg, 0.03 mmol). The solution was allowed to react 3 h at room temperature, and then washed with 10 mL of water, followed by extraction with 10 mL of ethyl acetate. The organic layer was concentrated under reduced pressure, and the residue was separated and purified by TLC, to provide 15 mg of compound (3*R*,5*S*)-1-((*S*)-2-(2-((5-(3-chloro-4-cyanophenyl)(5-(1-ethyl-3,5-dimethyl-1H-pyrazol-4-yl)-2-methylphenyl)amino)pentyl)oxy)acetylamino)-3,3-trimethylbutyryl)-5-(((*S*)-1-(4-(4-methylthia zol-5-yl)phenyl)ethyl)aminoformyl)pyrrolidinyl-3-acetate. LC/MS (ESI⁺) calcd for C₅₃H₆₅ClN₈O₆S ([M+H]⁺) *m*/*z* 978.3. ¹H NMR (400 MHz, CDCl₃) *δ* 8.68 (s, 1H), 7.37 (dt, *J* = 13.4, 8.3 Hz, 6H), 7.24 - 7.16 (m, 2H), 7.11 (d, *J* = 9.3 Hz, 1H), 6.95 (s, 1H), 6.51 (s, 1H), 6.36 (d, *J* = 7.7 Hz, 1H), 5.36 (s, 1H), 5.12 - 5.03 (m, 1H), 4.74 - 4.67 (m, 1H), 4.58 (d, *J* = 9.2 Hz, 1H), 4.07 (dt, *J* = 10.9, 5.3 Hz, 3H), 3.93 (s, 2H), 3.83 (dd, *J* = 11.7, 4.6 Hz, 1H), 3.77 - 3.64 (m, 1H), 3.51 (t, *J* = 6.4 Hz, 3H), 2.53 (s, 3H), 2.25 (s, 6H), 2.10 (d, *J* = 14.4 Hz, 5H), 2.04 (s, 3H), 1.67 (d, *J* = 8.6 Hz, 6H), 1.48 (d, *J* = 6.9 Hz, 3H), 1.41 (d, *J* = 7.2 Hz, 3H), 1.04 (s, 9H).

### 83: (2S,4R)-1-((S)-2-(2-((5-((4-(1H-imidazol-1-yl)phenyl)(5-(3,5-dimethylisoxazol-4-yl)-2-methylphenyl)amino)pentyl)oxy)acetylamino)-3,3-dimethylbutyryl)-4-hydroxy-N-((S)-1-(4-(4-methylthiazol-5-yl)phenyl)ethyl)-pyrrolidinyl-2-formamide (83)

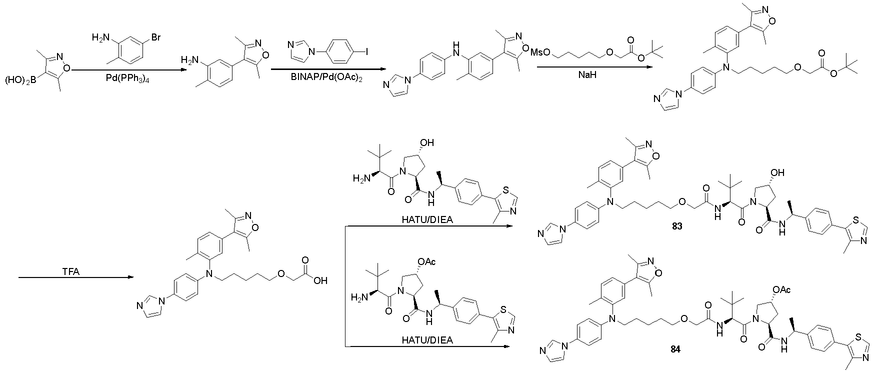

(3,5-dimethylisoxazol-4-yl)boric acid (16.9 g, 120 mmol) and 5-bromo-2-methylaniline (22.3 g, 120 mmol) were dissolved in 240 mL of 1,4-dioxane, to which were successively added 80 mL of water, potassium carbonate (41.5 g, 300 mmol), and tetrakis(triphenylphosphine)palladium (4.16 g, 3.6 mmol). Under argon protection, the mixture was allowed to react overnight at 85 °C. The water layer was removed, and the organic layer was concentrated to dry. The reaction solution was washed with 300 mL of water, and then extracted with 300 mL of ethyl acetate. The water layer was further extracted with ethyl acetate once. The organic layers were combined, washed with saturated brine, dried over anhydrous sodium sulfate, concentrated, and purified by column chromatography, to provide 13.5 g of compound 5-(3,5-dimethylisoxazol-4-yl)-2-methylaniline, with a yield of 56%. LC/MS (ESI⁺) calcd for C₁₂H₁₄N₂O ([M+H]⁺) *m*/*z* 203.1.

5-(3,5-Dimethylisoxazol-4-yl)-2-methylaniline (2.17 g, 8 mmol) was dissolved in 30 mL of 1,4-dioxane, to which were successively added 1-(4-iodobenzene)-1H-imidazole (1.62 g, 8 mmol), cesium carbonate (6.5 g, 20 mmol), BINAP (249 mg, 0.4 mmol), and palladium acetate (90 mg, 0.4 mmol). Under argon protection, the mixture was allowed to react overnight at 95 °C. The reaction solution was filtered. The filtrate was washed with 50 mL of water, extracted with 50 mL of ethyl acetate, washed with saturated brine, dried over anhydrous sodium sulfate, concentrated, and purified by column chromatography, to provide 1.0 g of compound N-(4-(1H-imidazol-1-yl)phenyl)-5-(3,5-dimethylisoxazol-4-yl)-2-methylaniline, with a yield of 36%. LC/MS (ESI⁺) calcd for C₂₁H₂₀N₄O ([M+H]⁺) *m*/*z* 344.2.

60% sodium hydride (50 mg, 1.25 mmol) was dissolved in 3 mL of dimethylsulfoxide, to which was added *N*-(4-(1H-imidazol-1-yl)phenyl)-5-(3,5-dimethylisoxazol-4-yl)-2-methylaniline (344 mg, 1.0 mmol). The mixture was stirred for 10 min at room temperature, and then 2-t-butyl (4-((methanesulfonyl)oxy)pentyloxy)acetate (561 mg, 2.0 mmol) was added. The mixture was allowed to react overnight at 60 °C, and then washed with 10 ml of water, followed by extraction with 10 mL of ethyl acetate. The organic layer was concentrated under reduced pressure, and the residue was separated and purified by TLC, to provide compound t-butyl ((5-((4-(1H-imidazol-1-yl)phenyl)(5-(3,5-dimethylisoxazol-4-yl)-2-methylanilino)amino)pentyl )oxy)acetate (90 mg), with a yield of 26%. LC/MS (ESI⁺) calcd for C₃₂H₄₀N₄O₄ ([M+H]⁺) *m*/*z* 545.3.

t-Butyl 2-((5-((4-(1H-imidazol-1-yl)phenyl)(5-(3,5-dimethylisoxazol-4-yl)-2-methylanilino) amino)pentyl)oxy)acetate (90 mg, 0.26 mmol) was dissolved in 3 mL of dichloromethane, to which was added 3 mL of trifluoroacetic acid, and the mixture was stirred at room temperature for 1 h. The reaction solution was concentrated to dryness under reduced pressure, to provide 2-((5-((4-(1H-imidazol-1-yl)phenyl)(5-(3,5-dimethylisoxazol-4-yl)-2-methylanilino)amino)pent yl)oxy)acetic acid, which was divided into two equal parts. The first part was added into 5 mL of DMF, to which were successively added diisopropylethylamine (55 mg, 0.43 mmol), HATU (35 mg, 0.09 mmol), and (2*S*,4*R*)-1-((*S*)-2-amino-3,3-dimethylbutyryl)-4-hydroxy-*N*-((*S*)-1-(4-(4-methylthiazol-5-yl)phenyl)ethyl)-pyrrolidinyl-2-formamide (41 mg, 0.09 mmol). The mixture was allowed to react 2 h at room temperature, and then washed with 10 ml of water, followed by extraction with 10 mL of ethyl acetate. The organic layer was concentrated under reduced pressure, and the residue was separated and purified by TLC, to provide 21 mg of compound (2*S*,4*R*)-1-((*S*)-2-(2-((5-((4-(1H-imidazol-1-yl)phenyl)(5-(3,5-dimethylisoxazol-4-yl)-2-methylphenyl)amino)pentyl)oxy)acetylamino)-3,3-dimethylbutyryl)-4-hydroxy-*N*-((*S*)-1-(4-(4-methylthiazol-5-yl)phenyl)ethyl)-pyrrolidinyl-2-formamide, with a yield of 25%. LC/MS (ESI⁺) calcd for C₅₁H₆₂N₈O₆S ([M+H]⁺) *m*/*z* 915.4. ¹H NMR (400 MHz, CDCl₃) *δ* 8.67 (s, 1H), 8.51 (s, 1H), 7.58 (d, *J* = 8.7 Hz, 1H), 7.46 - 7.30 (m, 7H), 7.16 (dd, *J* = 7.2, 5.4 Hz, 3H), 7.04 (s, 1H), 6.59 (d, *J* = 5.2 Hz, 2H), 5.12 - 5.05 (m, 1H), 4.81 - 4.75 (m, 1H), 4.59 - 4.51 (m, 2H), 4.18 - 4.14 (m, 1H), 4.07 - 4.02 (m, 1H), 3.95 - 3.88 (m, 2H), 3.63 (dd, *J* = 8.2, 2.0 Hz, 2H), 3.53 (dd, *J* = 5.6, 2.6 Hz, 2H), 2.59 - 2.49 (m, 4H), 2.42 (s, 3H), 2.28 (s, 3H), 2.23 (d, *J* = 7.5 Hz, 1H), 2.17 (s, 3H), 1.78 (d, *J* = 6.2 Hz, 2H), 1.68 (d, *J* = 6.5 Hz, 2H), 1.49 (d, *J* = 7.5 Hz, 2H), 1.45 (d, *J* = 7.0 Hz, 3H), 1.06 (s, 9H).

### 84: (3R,5S)-1-((S)-2-(2-((5-((4-(1H-imidazol-1-yl)phenyl)(5-(3,5-dimethylisoxazol-4-yl)-2-methylphenyl)amino)pentyl)oxy)acetylamino)-3,3-dimethylbutyryl)-5-(((S)-1-(4-(4-meth ylthiazol-5-yl)phenyl)ethyl)aminoformyl)pyrrolidinyl-3-yl acetate (84)

The second part was added into 5 mL of DMF, to which were successively added diisopropylethylamine (55 mg, 0.43 mmol), HATU (35 mg, 0.09 mmol), and (3*R*,5*S*)-1-((*S*)-2-amino-3,3-dimethylbutyryl)-5-(((*S*)-1-(4-(4-methylthiazol-5-yl)phenyl)ethyl)a minomethyl)pyrrolidinyl-3-acetate (46 mg, 0.09 mmol). The mixture was allowed to react 2 h at room temperature, and then washed with 10 ml of water, followed by extraction with 10 mL of ethyl acetate. The organic layer was concentrated under reduced pressure, and the residue was separated and purified by TLC, to provide 20 mg of compound (3*R*,5*S*)-1-((*S*)-2-(2-((5-((4-(1H-imidazol-1-yl)phenyl)(5-(3,5-dimethylisoxazol-4-yl)-2-methylp henyl)amino)pentyl)oxy)acetylamino)-3,3-dimethylbutyryl)-5-(((S)-1-(4-(4-methylthiazol-5-yl) phenyl)ethyl)aminoformyl)pyrrolidinyl-3-yl acetate, with a yield of 23%.

LC/MS (ESI⁺) calcd for C₅₃H₆₄N₈O₇S ([M+H]⁺) *m*/*z* 957.4. ¹H NMR (400 MHz, CDCl₃) *δ* 8.67 (s, 1H), 7.88 (s, 1H), 7.40 (dd, *J* = 8.1, 3.7 Hz, 3H), 7.35 (d, *J* = 8.3 Hz, 2H), 7.31 (d, *J* = 7.7 Hz, 1H), 7.21 - 7.14 (m, 5H), 7.12 (d, *J* = 9.2 Hz, 1H), 7.05 (d, *J* = 1.7 Hz, 1H), 6.56 (d, *J* = 9.0 Hz, 2H), 5.35 (s, 1H), 5.11 - 5.05 (m, 1H), 4.74 - 4.69 (m, 1H), 4.58 (d, *J* = 9.2 Hz, 1H), 4.06 (d, *J* = 10.9 Hz, 1H), 3.94 (s, 2H), 3.84 (dd, *J* = 11.6, 4.9 Hz, 1H), 3.64 (d, *J* = 3.2 Hz, 2H), 3.53 (t, *J* = 6.5 Hz, 2H), 2.53 (d, *J* = 4.2 Hz, 3H), 2.42 (s, 3H), 2.28 (s, 3H), 2.17 (s, 3H), 2.10 (d, *J =* 12.4 Hz, 1H), 2.04 (s, 3H), 1.77 - 1.66 (m, 5H), 1.47 (t, *J* = 6.6 Hz, 5H), 1.04 (s, 9H).

### 85: (2S,4R)-1-((S)-2-(2-(4-((4-(1H-imidazol-1-yl)phenyl)(5-(3,5-dimethylisoxazol-4-yl)-2-methylphenyl)amino)butoxy)acetamido)-3,3-dimethylbutyryl)-4-hydroxy-N-((S)-1-(4-(4-methylthiazol-5-yl)phenyl)ethyl)pyrrolidine-2-carboxamide (85)

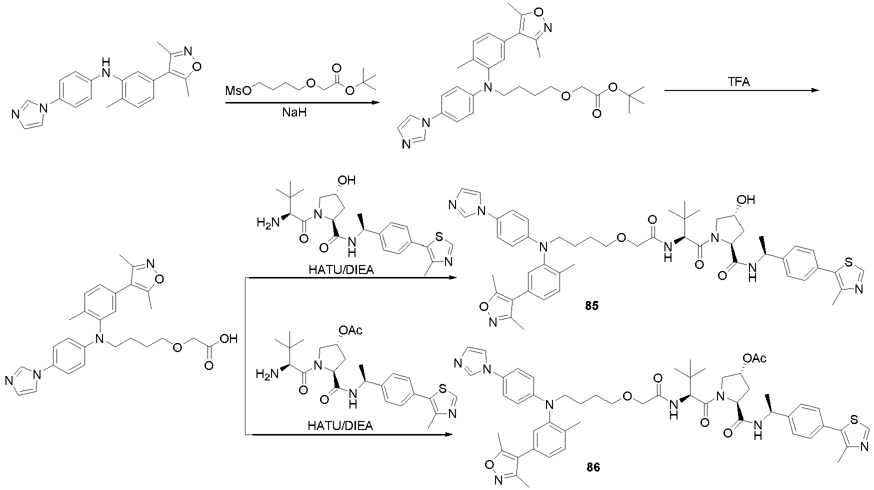

60% sodium hydride (40 mg, 1.0 mmol) was dissolved in 3 mL of dimethylsulfoxide, to which was added *N*-(4-(1H-imidazol-1-yl)phenyl)-5-(3,5-dimethylisoxazol-4-yl)-2-methylaniline (172 mg, 0.5 mmol). The mixture was stirred for 10 min at room temperature, and then 2-t-butyl (4-((methanesulfonyl)oxy)butoxy)acetate (665 mg, 2.5 mmol) was added. The mixture was allowed to react overnight at 60 °C, and then washed with 10 ml of water, followed by extraction with 10 mL of ethyl acetate. The organic layer was concentrated under reduced pressure, and the residue was separated and purified by TLC, to provide 30 mg of compound 2-t-butyl (4-((4-(1H-imidazol-1-yl)phenyl)(5-(3,5-dimethylisoxazol-4-yl)-2-methylphenyl) amino)butyl)acetate, with a yield of 11.3%. LC/MS (ESI⁺) calcd for C₃₁H₃₈N₄O₄ ([M+H]⁺) *m*/*z* 531.3.

2-t-Butyl (4-((4-(1H-imidazol-1-yl)phenyl)(5 -(3,5 -dimethylisoxazol-4-yl)-2-methylphenyl) amino)butyl)acetate (30 mg, 0.057 mmol) was dissolved in 3 mL of dichloromethane, to which was added 3 mL of trifluoroacetic acid, and the mixture was stirred at room temperature for 1 h. The reaction solution was concentrated to dryness under reduced pressure, to provide compound 2-(4-((4-(1H-imidazol-1-yl)phenyl)(5-(3,5-dimethylisoxazol-4-yl)-2-methylphenyl) amino)butoxy)acetic acid (27 mg). LC/MS (ESI⁺) calcd for C₂₇H₃₀N₄O₄ ([M+H]⁺) *m*/*z* 475.2. 2-(4-((4-(1H-Imidazol-1-yl)phenyl)(5-(3,5-dimethylisoxazol-4-yl)-2-methylphenyl)amino)butyl )acetic acid (4.5 mg, 0.01 mmol) was added into 1 mL of DMF, to which were added diisopropylethylamine (6.5 mg, 0.05 mmol), HATU (4.0 mg, 0.01 mmol), and (2*S*,4*R*)-1-((*S*)-2-amino-3,3-dimethylbutyryl)-4-hydroxy-*N*-((*S*)-1-(4-(4-methylthiazol-5-yl)phe nyl)ethyl)-pyrrolidinyl-2-formamide (5.0 mg, 0.01 mmol). The mixture was allowed to react 2 h at room temperature, and then washed with 10 ml of water, followed by extraction with 10 mL of ethyl acetate. The organic layer was concentrated under reduced pressure, and the residue was separated and purified by TLC, to provide (2*S*,4*R*)-1-((*S*)-2-(2-(4-((4-(1H-imidazol-1-yl)phenyl)(5-(3,5-dimethylisoxazol-4-yl)-2-methylphenyl)amino)butoxy)acetamido)-3,3-dimeth ylbutyryl)-4-hydroxy-*N*-((*S*)-1-(4-(4-methylthiazol-5-yl)phenyl)ethyl)pyrrolidine-2-carboxamid e (4 mg), with a yield of 44.4%. LC/MS (ESI⁺) calcd for C₅₀H₆₀N₈O₆S ([M+H]⁺) *m*/*z* 902.3. ¹H NMR (400 MHz, CDCl₃) *δ* 8.67 (s, 1H), 8.49 (s, 1H), 7.45 (s, 1H), 7.43 - 7.31 (m, 8H), 7.21 (s, 1H), 7.19 - 7.12 (m, 2H), 7.06 (d, *J* = 1.3 Hz, 1H), 6.60 (d, *J* = 8.9 Hz, 2H), 5.06 (d, *J* = 8.6 Hz, 1H), 4.70 (d, *J* = 7.9 Hz, 1H), 4.59 (d, *J* = 8.7 Hz, 1H), 4.53 (s, 1H), 4.21 (d, *J* = 5.5 Hz, 1H), 4.10 (d, *J* = 5.8 Hz, 1H), 3.96 (s, 2H), 3.69 (d, *J* = 6.8 Hz, 2H), 3.60 (s, 2H), 2.52 (d, *J* = 7.4 Hz, 4H), 2.42 (s, 3H), 2.28 (s, 3H), 2.24 - 2.19 (m, 1H), 2.17 (s, 3H), 1.87 (d, *J* = 7.1 Hz, 2H), 1.47 (dd, *J* = 14.1, 6.3 Hz, 5H), 1.03 (s, 9H).

### 86: (3R,5S)-1-((S)-2-(2-(4-((4-(1H-imidazol-1-yl)phenyl)(5-(3,5-dimethylisoxazol-4-yl)-2-methylphenyl)amino)butoxy)acetamido)-3,3-dimethylbutyryl)-5-((S)-1-(4-(4-methylthiaz ol-5-yl)phenyl)ethyl)carbamoyl)pyrrolidine-3-yl acetate (86)

2-(4-((4-(1H-imidazol-1-yl)phenyl)(5-(3,5-dimethylisoxazol-4-yl)-2-methylphenyl)amino)buto xy)acetic acid (22.5 mg, 0.046 mmol) was dissolved in 3 mL of DMF, to which were added diisopropylethylamine (31 mg, 0.24 mmol), HATU (20 mg, 0.05 mmol), and (3*R*,5*S*)-1-((*S*)-2-amino-3,3-dimethylbutyryl)-5-(((*S*)-1-(4-(4-methylthiazol-5-yl)phenyl)ethyl)a minomethyl)pyrrolidinyl-3-acetate (25 mg, 0.05 mmol). The mixture was allowed to react 3 h at room temperature, and then washed with 10 ml of water, followed by extraction with 10 mL of ethyl acetate. The organic layer was concentrated under reduced pressure, and the residue was separated and purified by TLC, to provide (3*R*,5*S*)-1-((*S*)-2-(2-(4-((4-(1H-imidazol-1-yl)phenyl) (5-(3,5-dimethylisoxazol-4-yl)-2-methylphenyl)amino)butoxy)acetamido)-3,3-dimethylbutyryl) -5-((*S*)-1-(4-(4-methylthiazol-5-yl)phenyl)ethyl)carbamoyl)pyrrolidine-3-yl acetate (12 mg), with a yield of 27.6%.

LC/MS (ESI⁺) calcd for C₅₂H₆₂N₈O₇S ([M+H]⁺) *m*/*z* 943.3. ¹H NMR (400 MHz, CDCl₃) *δ* 8.67 (s, 1H), 8.28 (s, 1H), 7.55 (d, *J* = 7.8 Hz, 1H), 7.46 - 7.32 (m, 7H), 7.21 (d, *J* = 8.7 Hz, 2H), 7.17 (dd, *J* = 7.8, 1.7 Hz, 1H), 7.09 - 7.03 (m, 2H), 6.59 (d, *J* = 8.8 Hz, 2H), 5.34 (s, 1H), 5.12 - 5.05 (m, 1H), 4.78 - 4.70 (m, 1H), 4.57 (d, *J* = 9.2 Hz, 1H), 4.07 (s, 2H), 3.97 (d, *J* = 4.7 Hz, 1H), 3.84 (dd, *J* = 11.5, 4.8 Hz, 1H), 3.72 - 3.66 (m, 2H), 3.58 (d, *J* = 6.2 Hz, 2H), 2.52 (s, 3H), 2.42 (s, 3H), 2.28 (s, 3H), 2.17 (s, 3H), 2.04 (s, 1H), 2.04 (s, 3H), 1.71 (dd, *J* = 14.1, 7.5 Hz, 5H), 1.45 (d, *J* = 6.9 Hz, 3H), 1.02 (s, 9H).

### 87: (2S,4R)-1-((S)-2-(2-((8-((4-(1H-imidazol-1-yl)phenyl)(5-(3,5-methylisoxazo-4-yl)-2-methylphenyl)amino)cctyl)oxy)acetylamino)-3,3-dimethylbutyryl)-4-hydroxy-N-((S)-1-(4-( 4-methylthiazol-5-yl)phenyl)ethyl)pyrrolidinyl-2-formamide (87)

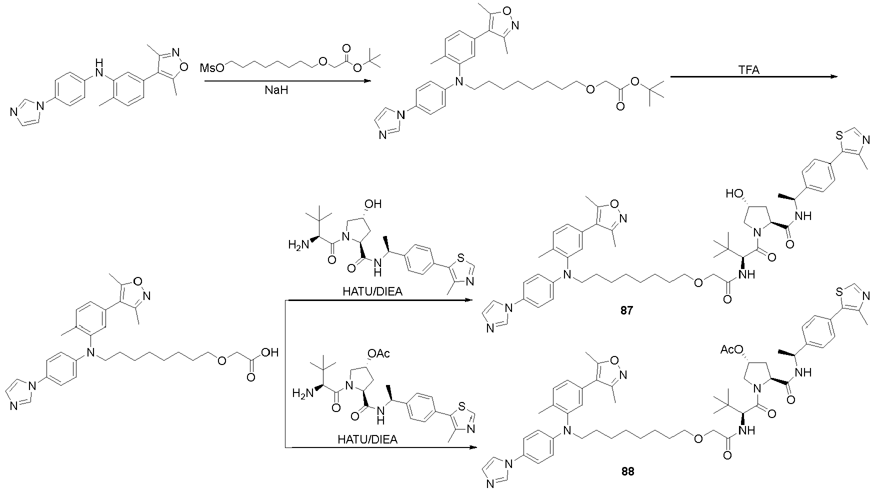

60% sodium hydride (40 mg, 1.0 mmol) was dissolved in 3 mL of dimethylsulfoxide, to which was added compound N-(4-(1H-imidazol-1-yl)phenyl)-5-(3,5-dimethylisoxazol-4-yl)-2-methylaniline (172 mg, 0.5 mmol). The mixture was stirred for 10 min at room temperature, and then 2-t-butyl ((8-((methylsulfonyl)oxy)cctyl)oxy)acetate (805 mg, 2.5 mmol) was added. The mixture was allowed to react overnight at 60 °C, and then washed with 10 ml of water, followed by extraction with 10 mL of ethyl acetate. The organic layer was concentrated under reduced pressure, and the residue was separated and purified by TLC, to provide 30 mg of compound 2-t-butyl ((8-((4-(1H-imidazol-1-yl)phenyl)-5-(3,5-dimethylisoxazol-4-yl)-2-methylanilino)amino)cctyl)oxy)acetate, with a yield of 10%. LC/MS (ESI⁺) calcd for C₃₅H₄₆N₄O₄ ([M+H]⁺) *m*/*z* 587.3.

2-t-Butyl ((8-((4-(1H-imidazol-1-yl)phenyl)-5-(3,5-dimethylisoxazol-4-yl)-2-methylanilino) amino)cctyl)oxy)acetate (30 mg, 0.09 mmol) was dissolved in 3 mL of dichloromethane, to which was added 3 mL of trifluoroacetic acid, and the mixture was allowed to react at room temperature for 1 h. The reaction solution was concentrated to dryness under reduced pressure, to provide compound 2-((8-((4-(1H-imidazol-1-yl)phenyl)-5-(3,5-dimethylisoxazol-4-yl)-2-methylanilino)amino)cctyl)oxy)acetic acid (24 mg), with a yield of 88.5%.

Compound 2-((8-((4-(1H-imidazol-1-yl)phenyl)-5-(3,5-dimethylisoxazol-4-yl)-2-methylanilino) amino)cctyl)oxy)acetic acid (4 mg, 0.008 mmol) was dissolved in 3 mL of DMF, to which were added *N*,*N*-diisopropylethylamine (4.9 mg, 0.038 mmol), HATU (3.1 mg, 0.008 mmol), and (2*S*,4*R*)-1-((*S*)-2-amino-3,3-dimethylbutyryl)-4-hydroxy-*N*-((*S*)-1-(4-(4-methylthiazol-5-yl)phe nyl)ethyl)-pyrrolidinyl-2-formamide (3.6 mg, 0.008 mmol). The mixture was allowed to react 3 h at room temperature, and then washed with 10 ml of water, followed by extraction with 10 mL of ethyl acetate. The organic layer was concentrated under reduced pressure, and the residue was separated and purified by TLC, to provide (2*S*,4*R*)-1-((*S*)-2-(2-((8-((4-(1H-imidazol-1-yl) phenyl)(5-(3,5-methylisoxazol-4-yl)-2-methylphenyl)amino)cctyl)oxy)acetylamino)-3,3-dimeth ylbutyryl)-4-hydroxy-*N*-((*S*)-1-(4-(4-methylthiazol-5-yl)phenyl)ethyl)pyrrolidinyl-2-formamide (5 mg), with a yield of 69.3%. LC/MS (ESI⁺) calcd for C₅₄H₆₈N₈O₆S ([M+H]⁺) *m*/*z* 957.4. ¹H NMR (400 MHz, CDCl₃) *δ* 8.67 (s, 1H), 7.95 (s, 1H), 7.45 - 7.33 (m, 7H), 7.21 - 7.13 (m, 5H), 7.04 (d, *J* = 1.7 Hz, 1H), 6.57 - 6.51 (m, 2H), 5.10 - 5.05 (m, 1H), 4.74 (d, *J* = 8.0 Hz, 1H), 4.55 (d, *J* = 8.9 Hz, 1H), 4.19 - 4.10 (m, 2H), 3.95 - 3.90 (m, 2H), 3.63 - 3.58 (m, 3H), 3.50 (td, *J* = 6.6, 1.7 Hz, 2H), 2.57 - 2.51 (m, 4H), 2.42 (s, 3H), 2.28 (s, 3H), 2.16 (s, 3H), 2.12 (d, *J* = 8.1 Hz, 1H), 1.61 (dd, *J* = 7.5, 5.4 Hz, 4H), 1.47 (d, *J* = 6.9 Hz, 5H), 1.35 (m, 6H), 1.06 (s, 9H).

### 88: (3R,5S)-1-((S)-2-(2-((8-((4-(1H-imidazol-1-yl)phenyl)(5-(3,5-dimethylisoxazol-4-yl)-2-methylphenyl)amino)cctyl)oxy)acetylamino)-3,3-dimethylbutyryl)-5-(((S)-1-(4-(4-methyl thiazol-5-yl)phenyl)ethyl)carbamoyl)pyrrolidine-3-yl acetate (88)

Compound 2-((8-((4-(1H-imidazol-1-yl)phenyl)-5-(3,5-dimethylisoxazol-4-yl)-2-methylanilino) amino)cctyl)oxy)acetic acid (20 mg, 0.038 mmol)was dissolved in 3 mL of DMF, to which were successively added N,N-diisopropylethylamine (24.4 mg, 0.188 mmol), HATU (15.9 mg, 0.042 mmol), and (3*R*,5*S*)-1-((*S*)-2-amino-3,3-dimethylbutyryl)-5-(((*S*)-1-(4-(4-methylthiazol-5-yl)phenyl)ethyl)aminomethyl)pyrrolidinyl-3-acetate (20.4 mg, 0.042 mmol). The mixture was allowed to react 3 h at room temperature, and then washed with 10 ml of water, followed by extraction with 10 mL of ethyl acetate. The organic layer was concentrated under reduced pressure, and the residue was separated and purified by TLC, to provide 15 mg of compound (3*R*,5*S*)-1-((*S*)-2-(2-((8-((4-(1H-imidazol-1-yl)phenyl)(5-(3,5-dimethylisoxazol-4-yl)-2-methylp henyl)amino)cctyl)oxy)acetylamino)-3,3-dimethylbutyryl)-5-(((*S*)-1-(4-(4-methylthiazol-5-yl)p henyl)ethyl)carbamoyl)pyrrolidine-3-yl acetate. LC/MS (ESI⁺) calcd for C₅₆H₇₀N₈O₇S ([M+H]⁺) *m*/*z* 999.4. ¹H NMR (400 MHz, CDCl₃) *δ* 8.67 (s, 1H), 8.39 (s, 1H), 7.49 (d, *J* = 7.9 Hz, 1H), 7.42 (d, *J* = 8.1 Hz, 2H), 7.39 - 7.34 (m, 5H), 7.20 (d, *J* = 9.0 Hz, 2H), 7.17 (dd, *J* = 7.9, 1.8 Hz, 1H), 7.14 (d, *J* = 9.2 Hz, 1H), 7.04 (d, *J* = 1.7 Hz, 1H), 6.56 (d, *J* = 9.0 Hz, 2H), 5.34 (s, 1H), 5.09 - 5.04 (m, 1H), 4.84 - 4.75 (m, 1H), 4.58 (d, *J* = 9.3 Hz, 1H), 4.07 (d, *J* = 11.0 Hz, 1H), 3.94 (s, 2H), 3.86 - 3.81 (m, 1H), 3.61 (d, *J* = 4.8 Hz, 2H), 3.53 - 3.47 (m, 2H), 2.52 (s, 3H), 2.42 (s, 3H), 2.28 (s, 3H), 2.17 (s, 3H), 2.04 (s, 3H), 1.72 (s, 3H), 1.62 (d, *J* = 7.0 Hz, 3H), 1.44 (d, *J* = 7.0 Hz, 3H), 1.36 (s, 8H), 1.04 (s, 9H).

### 89: (2S,4R)-1-((S)-2-(2-((5-((4-(1H-1,2,3-triazol-1-yl)phenyl)(5-(3,5-dimethylisoxazol-4-yl)-2-methylphenyl)amino)pentyl)oxy)acetylamino)-3,3-dimethylbutyryl)-4-hydroxy-N-((S)-1-(4-(4-methylthiazol-5-yl)phenyl)ethyl)-pyrrolidinyl-2-formamide (89)

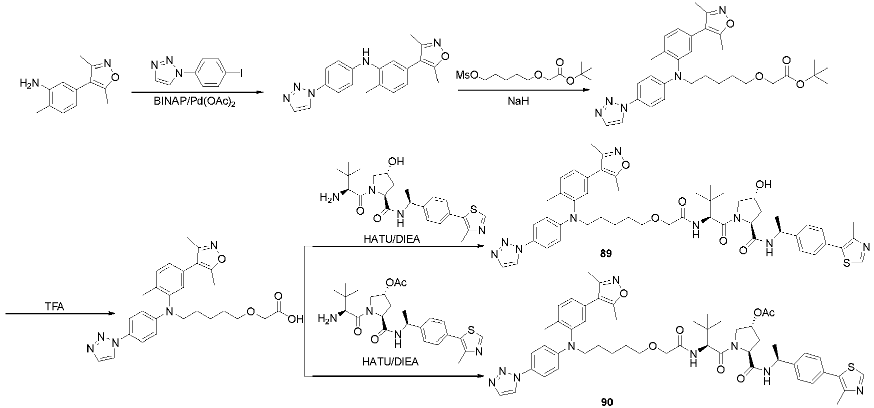

5-(3,5-Dimethylisoxazol-4-yl)-2-methylaniline (405 mg, 2.0 mmol) was dissolved in 10 mL of 1,4-dioxane, to which were successively added 1-(4-iodobenzene)-1H-1,2,3-triazole (542 mg, 2.0 mmol), cesium carbonate (1.6 g, 5 mmol), BINAP (62 mg, 0.1 mmol), and palladium acetate (23 mg, 0.1 mmol). Under argon protection, the mixture was allowed to react overnight at 95 °C. The reaction solution was filtered. The filtrate was washed with 20 mL of water, extracted with 20 mL of ethyl acetate, washed with saturated brine, dried over anhydrous sodium sulfate, concentrated, and purified by column chromatography, to provide 290 mg of compound *N*-(4-(1H-1,2,3-triazol-1-yl)phenyl)-5-(3,5-dimethylisoxazol-4-yl)-2-methylaniline, with a yield of 42%. LC/MS (ESI⁺) calcd for C₂₀H₁₉N₅O ([M+H]⁺) *m*/*z* 346.2.

60% sodium hydride (48 mg, 1.2 mmol) was dissolved in 3 mL of dimethylsulfoxide, to which was added compound *N*-(4-(1H-1,2,3-triazol-1-yl)phenyl)-5-(3,5-dimethylisoxazol-4-yl)-2-methylaniline (207 mg, 0.6 mmol). The mixture was stirred for 10 min at room temperature, and then 2-t-butyl (4-((methanesulfonyl)oxy)pentyloxy)acetate (841 mg, 3.0 mmol) was added. The mixture was allowed to react overnight at 60 °C, and then washed with 10 ml of water, followed by extraction with 10 mL of ethyl acetate. The organic layer was concentrated under reduced pressure, and the residue was separated and purified by TLC, to provide 120 mg of compound 2-t-butyl ((5-((4-(1H-1,2,3-triazol-1-yl)phenyl)(5-(3,5-dimethylisoxazol-4-yl)-2-methylanilino)amino)pentyl)oxy)acetate, with a yield of 36.6%. LC/MS (ESI⁺) calcd for C₃₁H₃₉N₅O₄ ([M+H]⁺) *m*/*z* 546.3.

t-butyl 2-((5 -((4-(1H-1,2,3 -triazol-1-yl)phenyl)(5-(3,5-dimethylisoxazol-4-yl)-2-methylanilino) amino)pentyl)oxy)acetate (120 mg, 0.22 mmol) was dissolved in 3 mL of dichloromethane, to which was added 3 mL of trifluoroacetic acid, and the mixture was allowed to react at room temperature for 2 h. The reaction solution was concentrated to dryness under reduced pressure. The residue was added with the saturated aqueous solution of sodium bicarbonate, and extracted with 10 mL of ethyl acetate. The organic layer was washed with saturated brine, dried over anhydrous sodium sulfate, and filtered. The filtrate was concentrated under reduced pressure, to provide 2-((5-((4-(1H-1,2,3-triazol-1-yl)phenyl)(5-(3,5-dimethylisoxazol-4-yl)-2-methylanilino)amino)pentyl)oxy)acetic acid (40 mg), with a yield of 37.1%.

2-((5-((4-(1H-1,2,3-Triazol-1-yl)phenyl)(5-(3,5-dimethylisoxazol-4-yl)-2-methylanilino)amino) pentyl)oxy)acetic acid (10 mg, 0.02 mmol) was dissolved in 1 mL of DMF, to which were successively added diisopropylethylamine (13 mg, 0.1 mmol), HATU (9 mg, 0.02 mmol), and (2*S*,4*R*)-1-((*S*)-2-amino-3,3-dimethylbutyryl)-4-hydroxy-*N*-((*S*)-1-(4-(4-methylthiazol-5-yl)phe nyl)ethyl)-pyrrolidinyl-2-formamide (9 mg, 0.02 mmol). The mixture was allowed to react 2 h at room temperature, and then washed with 10 ml of water, followed by extraction with 10 mL of ethyl acetate. The organic layer was concentrated under reduced pressure, and the residue was separated and purified by TLC, to provide 10 mg of compound (2*S*,4*R*)-1-((*S*)-2-(2-((5-((4-(1H-1,2,3-triazol-1-yl)phenyl)(5-(3,5-dimethylisoxazol-4-yl)-2-met hylphenyl)amino)pentyl)oxy)acetylamino)-3,3-dimethylbutyryl)-4-hydroxy-*N*-((*S*)-1-(4-(4-met hylthiazol-5-yl)phenyl)ethyl)-pyrrolidinyl-2-formamide. LC/MS (ESI⁺) calcd for C₅₀H₆₁N₉O₆S ([M+H]⁺) *m*/*z* 916.3.

### 90: (3R,5S)-1-((S)-2-(2-((5-((4-(1H-1,2,3-triazol-1-yl)phenyl)(5-(3,5-dimethylisoxazol-4-yl)-2-methylphenyl)amino)pentyl)oxy)acetylamino)-3,3-dimethylbutyryl)-5-(((S)-1-(4-(4-meth ylthiazol-5-yl)phenyl)ethyl)aminoformyl)pyrrolidinyl-3-yl acetate

2-((5-((4-(1H-1,2,3-Triazol-1-yl)phenyl)(5-(3,5-dimethylisoxazol-4-yl)-2-methylanilino)amino) pentyl)oxy)acetic acid (30 mg, 0.06 mmol) was dissolved in 4 mL of DMF, to which were successively added diisopropylethylamine (40 mg, 0.31 mmol), HATU (27 mg, 0.07 mmol), and (3*R*,5*S*)-1-((*S*)-2-amino-3,3-dimethylbutyryl)-5-(((*S*)-1-(4-(4-methylthiazol-5-yl)phenyl) ethyl)aminomethyl)pyrrolidinyl-3-acetate (30 mg, 0.06 mmol). The mixture was allowed to react 2 h at room temperature, and then washed with 10 ml of water, followed by extraction with 10 mL of ethyl acetate. The organic layer was concentrated under reduced pressure, and the residue was separated and purified by TLC, to provide 30 mg of compound (3*R*,5*S*)-1-((S)-2-(2-((5-((4-(1H-1,2,3-triazol-1-yl)phenyl)(5-(3,5-dimethylisoxazol-4-yl)-2-met hylphenyl)amino)pentyl)oxy)acetylamino)-3,3-dimethylbutyryl)-5-(((*S*)-1-(4-(4-methylthiazol-5-yl)phenyl)ethyl)aminoformyl)pyrrolidinyl-3-yl acetate. LC/MS (ESI⁺) calcd for C₅₂H₆₃N₉O₇S ([M+H]⁺) *m*/*z* 959.3. ¹H NMR (400 MHz, CDCl₃) *δ* 8.67 (s, 1H), 7.84 (d, *J* = 1.0 Hz, 1H), 7.80 (d, *J* = 0.9 Hz, 1H), 7.49 (d, *J* = 9.1 Hz, 2H), 7.38 (dt, *J* = 20.0, 7.5 Hz, 6H), 7.18 - 7.12 (m, 2H), 7.06 (d, *J* = 1.8 Hz, 1H), 6.59 (d, *J* = 9.1 Hz, 2H), 5.36 (s, 1H), 5.11 - 5.04 (m, 1H), 4.71 (dd, *J* = 8.2, 6.4 Hz, 1H), 4.58 (d, *J* = 9.3 Hz, 1H), 4.07 - 4.02 (m, 1H), 3.94 (d, *J* = 1.6 Hz, 2H), 3.84 (dd, *J* = 11.6, 4.8 Hz, 1H), 3.66 (d, *J* = 5.2 Hz, 2H), 3.53 (dd, *J* = 8.0, 5.0 Hz, 2H), 2.52 (s, 3H), 2.42 (s, 3H), 2.29 (s, 3H), 2.17 (s, 3H), 2.05 (d, *J* = 1.1 Hz, 1H), 2.03 (s, 3H), 1.81 - 1.75 (m, 2H), 1.68 (d, *J* = 7.7 Hz, 2H), 1.47 (dd, *J* = 11.2, 6.0 Hz, 6H), 1.04 (s, 9H).

### 91: (2S,4R)-1-((S)-2-(2-((5-((4-(1H-1,2,4-triazol-1-yl)phenyl)(5-(3,5-dimethylisoxazol-4-yl)-2-methylphenyl)amino)pentyl)oxy)acetylamino)-3,3-dimethylbutyryl)-4-hydroxy-N-((S)-1-(4-(4-methylthiazol-5-yl)phenyl)ethyl)-pyrrolidinyl-2-formamide (91)

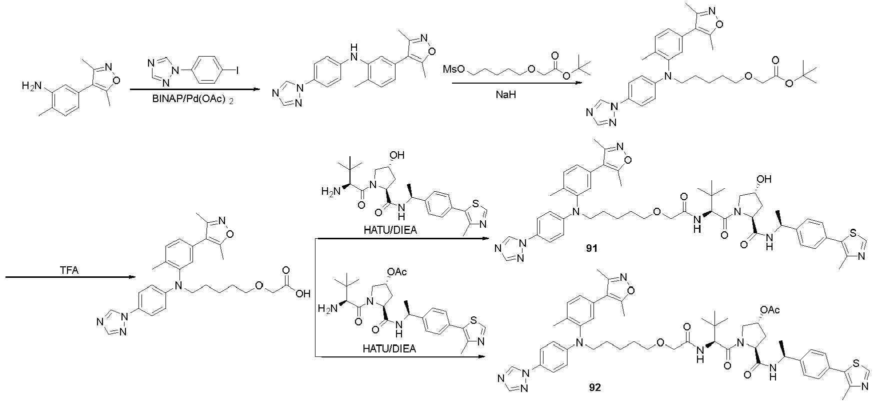

5-(3,5-Dimethylisoxazol-4-yl)-2-methylaniline (405 mg, 2.0 mmol) was dissolved in 10 mL of 1,4-dioxane, to which were successively added 1-(4-iodobenzene)-1H-1,2,3-triazole (542 mg, 2.0 mmol), cesium carbonate (1.6 g, 5 mmol), BINAP (62 mg, 0.1 mmol), and palladium acetate (23 mg, 0.1 mmol). Under argon protection, the mixture was allowed to react overnight at 95 °C. The reaction solution was filtered. The filtrate was washed with 20 mL of water, extracted with 20 mL of ethyl acetate, washed with saturated brine, dried over anhydrous sodium sulfate, concentrated, and purified by column chromatography, to provide 255 mg of compound N-(4-(1H-1,2,4-triazol-1-yl)phenyl)-5-(3,5-dimethylisoxazol-4-yl)-2-methylaniline, with a yield of 37%. LC/MS (ESI⁺) calcd for C₂₀H₁₉N₅O ([M+H]⁺) *m*/*z* 346.2.

60% sodium hydride (40 mg, 1.0 mmol) was dissolved in 3 mL of dimethylsulfoxide, to which was added *N*-(H-1,2,4-triazol-1-yl)phenyl)-5-(3,5-dimethylisoxazol-4-yl)-2-methylaniline (173 mg, 0.5 mmol). The mixture was stirred for 10 min at room temperature, and then 2-t-butyl (4-((methanesulfonyl)oxy)pentyloxy)acetate (720 mg, 2.5 mmol) was added. The mixture was allowed to react overnight at 60 °C, and then washed with 10 ml of water, followed by extraction with 10 mL of ethyl acetate. The organic layer was concentrated under reduced pressure, and the residue was separated and purified by TLC, to provide 110 mg of compound 2-t-butyl ((5 -((4-(1H-1,2,4-triazol-1-yl)phenyl)(5-(3,5-dimethylisoxazol-4-yl)-2-methylanilino) amino)pentyl)oxy)acetate, with a yield of 40%. LC/MS (ESI⁺) calcd for C₃₁H₃₉N₅O₄ ([M+H]⁺) *m*/*z* 546.3.

Compound t-butyl 2-((5-((4-(1H-1,2,4-triazol-1-yl)phenyl)(5-(3,5-dimethylisoxazol-4-yl)-2-methylanilino)amino)pentyl)oxy)acetate (110 mg, 0.2 mmol) was dissolved in 3 mL of dichloromethane, to which was added 3 mL of trifluoroacetic acid, and the mixture was allowed to react at room temperature for 1 h. The reaction solution was concentrated to dryness under reduced pressure, to provide 2-((5-((4-(1H-1,2,4-triazol-1-yl)phenyl)(5-(3,5-dimethylisoxazol-4-yl)-2-methylanilino)amino)pentyl)oxy)acetic acid (88 mg), with a yield of 90.0%.

2-((5-((4-(1H-1,2,4-triazol-1-yl)phenyl)(5-(3,5-dimethylisoxazol-4-yl)-2-methylanilino)amino) pentyl)oxy)acetic acid (22 mg, 0.04 mmol) was dissolved in 1 mL of DMF, to which were successively added diisopropylethylamine (29 mg, 0.22 mmol), HATU (19 mg, 0.05 mmol), and (2*S*,4*R*)-1-((*S*)-2-amino-3,3-dimethylbutyryl)-4-hydroxy-*N*-((*S*)-1-(4-(4-methylthiazol-5-yl)phenyl)ethyl)-pyrrolidinyl-2-formamide (20 mg, 0.04 mmol). The mixture was allowed to react 3 h at room temperature, and then washed with 10 ml of water, followed by extraction with 10 mL of ethyl acetate. The organic layer was concentrated under reduced pressure, and the residue was separated and purified by TLC, to provide 5 mg of compound (2*S*,4*R*)-1-((*S*)-2-(2-((5-((4-(1H-1,2,4-triazol-1-yl)phenyl)(5-(3,5-dimethylisoxazol-4-yl)-2-met hylphenyl)amino)pentyl)oxy)acetylamino)-3,3-dimethylbutyryl)-4-hydroxy-*N*-((*S*)-1-(4-(4-met hylthiazol-5-yl)phenyl)ethyl)-pyrrolidinyl-2-formamide. LC/MS (ESI⁺) calcd for C₅₀H₆₁N₉O₆S ([M+H]⁺) *m*/*z* 916.3. ¹H NMR (400 MHz, CDCl₃) *δ* 8.71 (s, 1H), 8.43 (s, 1H), 8.05 (s, 1H), 7.39 (dt, *J* = 16.3, 8.8 Hz, 8H), 7.19 (d, *J* = 8.6 Hz, 1H), 7.16 (dd, *J* = 7.9, 1.8 Hz, 1H), 7.05 (d, *J* = 1.7 Hz, 1H), 6.57 (d, *J* = 9.0 Hz, 2H), 5.39 - 5.33 (m, 1H), 5.11 - 5.05 (m, 1H), 4.76 - 4.71 (m, 1H), 4.58 - 4.51 (m, 2H), 4.16 - 4.12 (m, 1H), 3.93 (d, *J* = 4.1 Hz, 2H), 3.67 - 3.59 (m, 3H), 3.52 (s, 2H), 2.53 (s, 3H), 2.42 (s, 3H), 2.28 (s, 3H), 2.16 (s, 3H), 2.09 (s, 2H), 1.78 - 1.76 (m, 2H), 1.51 - 1.48 (m, 2H), 1.46 (d, *J* = 6.9 Hz, 3H), 1.31 - 1.28 (m, 2H), 1.06 (s, 9H).

### 92: (3R,5S)-1-((S)-2-(2-((5-((4-(1H-1,2,4-triazol-1-yl)phenyl)(5-(3,5-dimethylisoxazol-4-yl)-2-methylphenyl)amino)pentyl)oxy)acetylamino)-3,3-dimethylbutyryl)-5-(((S)-1-(4-(4-meth ylthiazol-5-yl)phenyl)ethyl)aminoformyl)pyrrolidinyl-3-yl acetate (92)

2-((5-((4-(1H-1,2,4-triazol-1-yl)phenyl)(5-(3,5-dimethylisoxazol-4-yl)-2-methylanilino)amino) pentyl)oxy)acetic acid (66 mg, 0.14 mmol) was dissolved in 4 mL of DMF, to which were successively added diisopropylethylamine (87 mg, 0.68 mmol), HATU (57 mg, 0.15 mmol), and (3*R*,5*S*)-1-((S)-2-amino-3,3-dimethylbutyryl)-5-(((*S*)-1-(4-(4-methylthiazol-5-yl)phenyl) ethyl)aminomethyl)pyrrolidinyl-3-acetate (66 mg, 0.14 mmol). The mixture was allowed to react 3 h at room temperature, and then washed with 10 ml of water, followed by extraction with 10 mL of ethyl acetate. The organic layer was concentrated under reduced pressure, and the residue was separated and purified by TLC, to provide 20 mg of compound (3*R*,5*S*)-1-((S)-2-(2-((5-((4-(1H-1,2,4-triazol-1-yl)phenyl)(5-(3,5-dimethylisoxazol-4-yl)-2-met hylphenyl)amino)pentyl)oxy)acetylamino)-3,3-dimethylbutyryl)-5-(((*S*)-1-(4-(4-methylthiazol-5-yl)phenyl)ethyl)aminoformyl)pyrrolidinyl-3-yl acetate, with a yield of 14.9%. LC/MS (ESI⁺) calcd for C₅₂H₆₃N₉O₇S ([M+H]⁺) *m*/*z* 959.3. ¹H NMR (400 MHz, CDCl₃) *δ* 8.68 (s, 1H), 8.40 (s, 1H), 8.04 (s, 1H), 7.45 - 7.37 (m, 6H), 7.34 (d, *J* = 8.2 Hz, 2H), 7.17 - 7.11 (m, 2H), 7.05 (d, *J* = 1.8 Hz, 1H), 6.57 (d, *J* = 9.1 Hz, 2H), 5.36 (s, 1H), 5.13 - 5.03 (m, 1H), 4.71 (dd, *J* = 8.2, 6.4 Hz, 1H), 4.58 (d, *J* = 9.3 Hz, 1H), 4.05 (d, *J* = 9.5 Hz, 1H), 3.94 (s, 2H), 3.85 (d, *J* = 4.8 Hz, 1H), 3.65 (d, *J* = 5.1 Hz, 2H), 3.53 (t, *J* = 6.5 Hz, 2H), 2.52 (s, 3H), 2.42 (s, 3H), 2.28 (s, 3H), 2.16 (s, 3H), 2.09 (s, 1H), 2.04 (s, 3H), 1.70 (dd, *J* = 14.8, 7.4 Hz, 5H), 1.46 (d, *J* = 7.0 Hz, 5H), 1.04 (s, 9H).

### 93: (2S,4R)-1-((S)-2-(2-((5-((4-(1H-1,3,4-triazol-1-yl)phenyl)(5-(3,5-dimethylisoxazol-4-yl)-2-methytphenyt)amino)pentyt)oxy)acetytamino)-3,3-dimethytbutyryt)-4-hydroxy-N-((S)-1-(4-(4-methylthiazol-5-yl)phenyl)ethyl)-pyrrolidinyl-2-formamide (93)

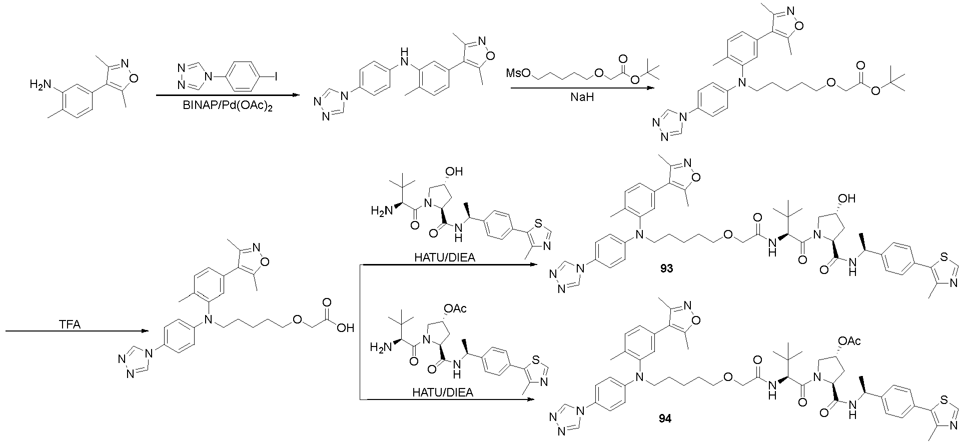

5-(3,5-Dimethylisoxazol-4-yl)-2-methylaniline (607 mg, 3.0 mmol) was dissolved in 10 mL of 1,4-dioxane, to which were successively added 1-(4-iodobenzene)-1H-1,3,4-triazole (813 mg, 3.0 mmol), cesium carbonate (2.4 g, 7.5 mmol), BINAP (93 mg, 0.15 mmol), and palladium acetate (34 mg, 0.15 mmol). Under argon protection, the mixture was allowed to react overnight at 95 °C. The reaction solution was filtered. The filtrate was washed with 20 mL of water, extracted with 20 mL of ethyl acetate, washed with saturated brine, dried over anhydrous sodium sulfate, concentrated, and purified by column chromatography, to provide 160 mg of compound N-(4-(1H-1,3,4-triazol-1-yl)phenyl)-5-(3,5-dimethylisoxazol-4-yl)-2-methylaniline, with a yield of 30%. LC/MS (ESI⁺) calcd for C₂₀H₁₉N₅O ([M+H]⁺) *m*/*z* 346.2.

60% sodium hydride (37 mg, 0.93 mmol) was dissolved in 3 mL of dimethylsulfoxide, to which was added N-(4-(1H-1,3,4-triazol-1-yl)phenyl)-5-(3,5-dimethylisoxazol-4-yl)-2-methylaniline (160 mg, 0.46 mmol). The mixture was stirred for 10 min at room temperature, and then 2-t-butyl (4-((methanesulfonyl)oxy)pentyloxy)acetate (650 mg, 2.32 mmol) was added. The mixture was allowed to react overnight at 60 °C, and then washed with 10 ml of water, followed by extraction with 10 mL of ethyl acetate. The organic layer was concentrated under reduced pressure, and the residue was separated and purified by TLC, to provide 35 mg of compound 2-t-butyl ((5 -((4-(1H-1,3,4-triazol-1-yl)phenyl)(5-(3,5-dimethylisoxazol-4-yl)-2-methylanilino) amino)pentyl)oxy)acetate, with a yield of 13%. LC/MS (ESI⁺) calcd for C₃₁H₃₉N₅O₄ ([M+H]⁺) *m*/*z* 546.3.

Compound t-butyl 2-((5-((4-(1H-1,3,4-triazol-1-yl)phenyl)(5-(3,5-dimethylisoxazol-4-yl)-2-methylanilino)amino)pentyl)oxy)acetate (35 mg, 0.064 mmol) was dissolved in 3 mL of dichloromethane, to which was added 3 mL of trifluoroacetic acid, and the mixture was allowed to react at room temperature for 1 h. The reaction solution was concentrated to dryness under reduced pressure, to provide 2-((5-((4-(1H-1,3,4-triazol-1-yl)phenyl)(5-(3,5-dimethylisoxazol-4-yl)-2- methylanilino)amino)pentyl)oxy)acetic acid (28 mg), with a yield of 89.2%.

2-((5-((4-(1H-1,2,4-triazol-1-yl)phenyl)(5-(3,5-dimethylisoxazol-4-yl)-2-methylanilino)amino) pentyl)oxy)acetic acid (7 mg, 0.014 mmol) was dissolved in 1 mL of DMF, to which were successively added diisopropylethylamine (9 mg, 0.07 mmol), HATU (6 mg, 0.015 mmol), and (2*S*,4*R*)-1-((*S*)-2-amino-3,3-dimethylbutyryl)-4-hydroxy-*N*-((*S*)-1-(4-(4-methylthiazol-5-yl)phe nyl)ethyl)-pyrrolidinyl-2-formamide (6 mg, 0.014 mmol). The mixture was allowed to react 1 h at room temperature, and then washed with 10 ml of water, followed by extraction with 10 mL of ethyl acetate. The organic layer was concentrated under reduced pressure, and the residue was separated and purified by TLC, to provide 8 mg of compound (2*S*,4*R*)-1-((*S*)-2-(2-((5-((4-(1H-1,3,4-triazol-1-yl)phenyl)(5-(3,5-dimethylisoxazol-4-yl)-2-met hylphenyl)amino)pentyl)oxy)acetylamino)-3,3-dimethylbutyryl)-4-hydroxy-*N*-((*S*)-1-(4-(4-met hylthiazol-5-yl)phenyl)ethyl)-pyrrolidinyl-2-formamide. LC/MS (ESI⁺) calcd for C₅₀H₆₁N₉O₆S ([M+H]⁺) *m*/*z* 916.3. ¹H NMR (400 MHz, CDCl₃) *δ* 8.70 (s, 1H), 8.42 (s, 1H), 7.39 (ddd, *J* = 11.1, 9.0, 5.8 Hz, 7H), 7.17 (dt, *J* = 6.7, 3.8 Hz, 4H), 7.04 (d, *J* = 1.5 Hz, 1H), 6.57 (d, *J* = 8.1 Hz, 2H), 5.35 (t, *J* = 4.9 Hz, 1H), 5.11 - 5.06 (m, 1H), 4.75 - 4.70 (m, 1H), 4.58 - 4.50 (m, 2H), 4.13 (d, *J* = 11.3 Hz, 2H), 3.93 (s, 2H), 3.65 - 3.60 (m, 2H), 3.55 - 3.50 (m, 2H), 2.53 (t, *J* = 2.9 Hz, 4H), 2.42 (s, 3H), 2.28 (s, 3H), 2.17 (s, 3H), 2.09 (s, 1H), 1.79 - 1.74 (m, 4H), 1.49 - 1.45 (m, 5H), 1.06 (s, 9H).

### 94: (3R,5S)-1-((S)-2-(2-((5-((4-(1H-1,3,4-triazol-1-yl)phenyl)(5-(3,5-dimethylisoxazol-4-yl)-2-methylphenyl)amino)pentyl)oxy)acetylamino)-3,3-dimethylbutyryl)-5-(((S)-1-(4-(4-meth ylthiazol-5-yl)phenyl)ethyl)aminoformyl)pyrrolidinyl-3-yl acetate (94)

2-((5-((4-(1H-1,3,4-triazol-1-yl)phenyl)(5-(3,5-dimethylisoxazol-4-yl)-2-methylanilino) amino) pentyl)oxy)acetic acid (21 mg, 0.04 mmol) was dissolved in 4 mL of DMF, to which were successively added diisopropylethylamine (28 mg, 0.22 mmol), HATU (17 mg, 0.045 mmol), and (3*R*,5*S*)-1-((*S*)-2-amino-3,3-dimethylbutyryl)-5-(((*S*)-1-(4-(4-methylthiazol-5-yl)phenyl) ethyl)aminomethyl)pyrrolidinyl-3-acetate (21 mg, 0.04 mmol). The mixture was allowed to react 1 h at room temperature, and then washed with 10 ml of water, followed by extraction with 10 mL of ethyl acetate. The organic layer was concentrated under reduced pressure, and the residue was separated and purified by TLC, to provide 17 mg of compound (3*R*,5*S*)-1-((*S*)-2-(2-((5-((4-(1H-1,3,4-triazol-1-yl)phenyl)(5-(3,5-dimethylisoxazol-4-yl)-2-met hylphenyl)amino)pentyl)oxy)acetylamino)-3,3-dimethylbutyryl)-5-(((*S*)-1-(4-(4-methylthiazol-5-yl)phenyl)ethyl)aminoformyl)pyrrolidinyl-3-yl acetate. LC/MS (ESI⁺) calcd for C₅₂H₆₃N₉O₇S ([M+H]⁺) *m*/*z* 959.3. ¹H NMR (400 MHz, CDCl₃) *δ* 8.68 (d, *J* = 2.4 Hz, 1H), 8.36 (d, *J* = 9.1 Hz, 2H), 7.45 - 7.29 (m, 6H), 7.20 - 7.09 (m, 4H), 7.04 (d, *J* = 1.7 Hz, 1H), 6.56 (dd, *J* = 9.4, 2.7 Hz, 2H), 5.36 (s, 1H), 5.12 - 5.05 (m, 1H), 4.72 - 4.67 (m, 1H), 4.58 (d, *J* = 9.3 Hz, 1H), 4.05 (s, 1H), 3.94 (s, 2H), 3.85 (dd, *J* = 11.5, 4.8 Hz, 1H), 3.68 - 3.59 (m, 2H), 3.52 (d, *J* = 6.5 Hz, 2H), 2.72 - 2.63 (m, 1H), 2.53 (d, *J* = 3.5 Hz, 3H), 2.42 (s, 3H), 2.28 (d, *J* = 2.2 Hz, 3H), 2.16 (d, *J* = 3.6 Hz, 3H), 2.12 (dd, *J* = 8.3, 4.2 Hz, 1H), 2.04 (s, 3H), 1.68 (d, *J* = 8.1 Hz, 4H), 1.46 (t, *J* = 7.6 Hz, 5H), 1.04 (s, 9H).

### 95: (2S,4R)-1-((S)-2-(2-((5-((5-(3,5-dimethylisoxazol-4-yl)-2-methylphenyl)(4-(4-methyl-1H-imidazol-1-yl)phenyl)amino)pentyl)oxy)acetylamino)-3,3-dimethylbutyryl)-4-hydroxy-N-((S)-1-(4-(4-methylthiazol-5-yl)phenyl)ethyl)-pyrrolidinyl-2-formamide (95)

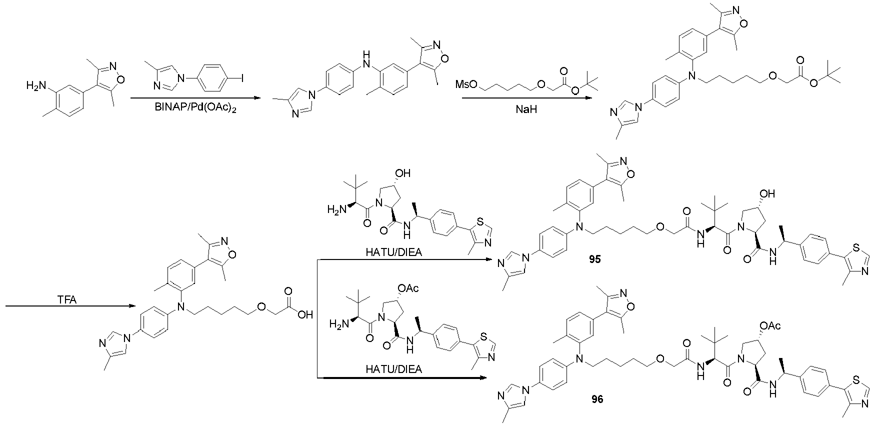

5-(3,5-Dimethylisoxazol-4-yl)-2-methylaniline (320 mg, 1.58 mmol) was dissolved in 6 mL of 1,4-dioxane, to which were successively added 1-(4-iodobenzene)-4-methyl-1H-imidazole (450 mg, 1.58 mmol), cesium carbonate (1.28 g, 3.95 mmol), BINAP (50 mg, 0.08 mmol), and palladium acetate (18 mg, 0.08 mmol). Under argon protection, the mixture was allowed to react overnight at 95 °C. The reaction solution was filtered. The filtrate was washed with 20 mL of water, extracted with 20 mL of ethyl acetate, washed with saturated brine, dried over anhydrous sodium sulfate, concentrated, and purified by column chromatography, to provide 280 mg of compound 5-(3,5-dimethylisoxazol-4-yl)-2-methyl-*N*-(4-(4-methyl-1H-imidazol-1-yl)phenyl)aniline, with a yield of 49%. LC/MS (ESI⁺) calcd for C₂₂H₂₂N₄O ([M+H]⁺) *m*/*z* 359.2.

60% sodium hydride (54 mg, 1.34 mmol) was dissolved in 3 mL of dimethylsulfoxide, to which was added 5-(3,5-dimethylisoxazol-4-yl)-2-methyl-*N*-(4-(4-methyl-1H-imidazol-1-yl) phenyl)aniline (240 mg, 0.67 mmol). The mixture was stirred for 10 min at room temperature, and then 2-t-butyl (4-((methanesulfonyl)oxy)pentyloxy)acetate (937 mg, 3.34 mmol) was added. The mixture was allowed to react overnight at 60 °C, and then washed with 10 ml of water, followed by extraction with 10 mL of ethyl acetate. The organic layer was concentrated under reduced pressure, and the residue was separated and purified by TLC, to provide 35 mg of compound t-butyl 2-((5-((5-(3,5-dimethylisoxazol-4-yl)-2-methylphenyl)(4-(4-methyl-1H-imidazol-1-yl)phenyl)amino)pentyl)oxy)acetate, with a yield of 9.3%. LC/MS (ESI⁺) calcd for C₃₃H₄₂N₄O₄ ([M+H]⁺) *m*/*z* 559.3.

Compound t-butyl 2-((5-((5-(3,5-dimethylisoxazol-4-yl)-2-methylphenyl)(4-(4-methyl-1H-imidazol-1-yl)phenyl)amino)pentyl)oxy)acetate (35 mg, 0.06 mmol) was dissolved in 3 mL of dichloromethane, to which was added 3 mL of trifluoroacetic acid, and the mixture was allowed to react at room temperature for 1 h. The reaction solution was concentrated to dryness under reduced pressure, to provide 2-((5-((5-(3,5-dimethylisoxazol-4-yl)-2-methylphenyl)(4-(4-methyl-1H-imidazol-1-yl)phenyl)amino)pentyl)oxy)acetic acid (25 mg), with a yield of 79.0%.

2-((5-((5-(3,5-Dimethylisoxazol-4-yl)-2-methylphenyl)(4-(4-methyl-1H-imidazol-1-yl)phenyl)a mino)pentyl)oxy)acetic acid (5 mg, 0.01 mmol) was dissolved in 1 mL of DMF, to which were successively added diisopropylethylamine (7 mg, 0.05 mmol), HATU(4 mg, 0.01 mmol), and (2*S*,4*R*)-1-((*S*)-2-amino-3,3-dimethylbutyryl)-4-hydroxy-*N*-((*S*)-1-(4-(4-methylthiazol-5-yl)phe nyl)ethyl)-pyrrolidinyl-2-formamide (5 mg, 0.01 mmol). The mixture was allowed to react 1 h at room temperature, and then washed with 10 ml of water, followed by extraction with 10 mL of ethyl acetate. The organic layer was concentrated under reduced pressure, and the residue was separated and purified by TLC, to provide 4 mg of compound (2*S*,4*R*)-1-((*S*)-2-(2-((5-((5-(3,5-dimethylisoxazol-4-yl)-2-methylphenyl)(4-(4-methyl-1H-imidazol-1-yl)phenyl)amino)pent yl)oxy)acetylamino)-3,3-dimethylbutyryl)-4-hydroxy-*N*-((*S*)-1-(4-(4-methylthiazol-5-yl)phenyl )ethyl)-pyrrolidinyl-2-formamide. LC/MS (ESI⁺) calcd for C₅₂H₆₄N₈O₆S ([M+H]⁺) *m*/*z* 930.4.

### 96: (3R,5S)-1-((S)-2-(2-((5-((5-(3,5-dimethylisoxazol-4-yl)-2-methylphenyl)(4-(4-methyl-1H-imidazol-1-yl)phenyl)amino)pentyl)oxy)acetylamino)-3,3-dimethylbutyryl)-5-(((S)-1-(4 -(4-methylthiazol-5-yl)phenyl)ethyl)aminoformyl)pyrrolidinyl-3-yl acetate (96)

2-((5-((5-(3,5-Dimethylisoxazol-4-yl)-2-methylphenyl)(4-(4-methyl-IH-imidazol-1-yl)phenyl)a mino)pentyl)oxy)acetic acid (20 mg, 0.04 mmol) was dissolved in 4 mL of DMF, to which were successively added diisopropylethylamine (26 mg, 0.22 mmol), HATU (17 mg, 0.045 mmol), and (3*R*,5*S*)-1-((*S*)-2-amino-3,3-dimethylbutyryl)-5-(((*S*)-1-(4-(4-methylthiazol-5-yl)phenyl) ethyl)aminomethyl)pyrrolidinyl-3-acetate (21 mg, 0.04 mmol). The mixture was allowed to react 1 h at room temperature, and then washed with 10 ml of water, followed by extraction with 10 mL of ethyl acetate. The organic layer was concentrated under reduced pressure, and the residue was separated and purified by TLC, to provide 8 mg of compound (3*R*,5*S*)-1-((*S*)-2-(2-((5-((5-(3,5-dimethylisoxazol-4-yl)-2-methylphenyl)(4-(4-methyl-1H-imida zol-1-yl)phenyl)amino)pentyl)oxy)acetylamino)-3,3-dimethylbutyryl)-5-(((*S*)-1-(4-(4-methylthi azol-5-yl)phenyl)ethyl)aminoformyl)pyrrolidinyl-3-yl acetate. LC/MS (ESI⁺) calcd for C₅₄H₆₆N₈O₇S ([M+H]⁺) *m*/*z* 971.3. ¹H NMR (400 MHz, CDCl₃) *δ* 8.68 (d, *J* = 2.8 Hz, 1H), 7.70 (s, 1H), 7.41 - 7.34 (m, 5H), 7.30 (d, *J* = 7.8 Hz, 1H), 7.13 (t, *J* = 8.0 Hz, 4H), 7.04 (d, *J* = 1.6 Hz, 1H), 6.90 - 6.85 (m, 1H), 6.54 (d, *J* = 8.9 Hz, 2H), 5.35 (s, 1H), 5.11 - 5.05 (m, 1H), 4.74 - 4.69 (m, 1H), 4.58 (d, *J* = 9.3 Hz, 1H), 4.17 (d, *J* = 6.5 Hz, 1H), 4.06 (d, *J* = 1.6 Hz, 1H), 3.94 (d, *J* = 6.2 Hz, 2H), 3.84 (dd, *J* = 11.7, 4.8 Hz, 1H), 3.65 - 3.62 (m, 1H), 3.52 (d, *J* = 6.4 Hz, 2H), 2.53 (d, *J=* 3.9 Hz, 4H), 2.42 (s, 3H), 2.28 (s, 6H), 2.16 (s, 3H), 2.14 - 2.09 (m, 1H), 2.04 (s, 3H), 1.71 - 1.67 (m, 4H), 1.50 - 1.46 (m, 5H), 1.04 (s, 9H).

### 97: (2S,4R)-1-((S)-2-(2-((5-((5-(3,5-dimethylisoxazol-4-yl)-2-methylphenyl)(4-(2-methyl-1H-imidazol-1-yl)phenyl)amino)pentyl)oxy)acetylamino)-3,3-dimethylbutyryl)-4-hydroxy-N-((S)-1-(4-(14-methylthiazol-5-yl)phenyl)ethyl)-pyrrolidinyl-2-formamide (97)

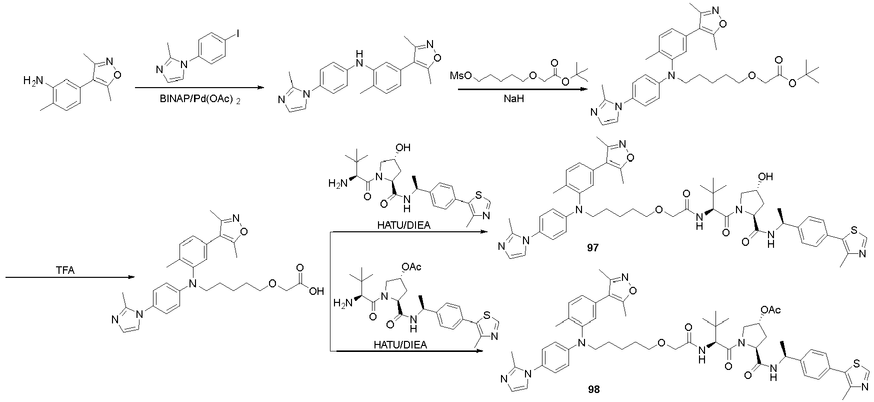

5-(3,5-Dimethylisoxazol-4-yl)-2-methylaniline (607 mg, 3 mmol) was dissolved in 10 mL of 1,4-dioxane, to which were successively added 1-(4-iodobenzene)-4-methyl-1H-imidazole (855 mg, 3.0 mmol), cesium carbonate (2.4 g, 7.5 mmol), BINAP (93 mg, 0.15 mmol), and palladium acetate (34 mg, 0.15 mmol). Under argon protection, the mixture was allowed to react overnight at 95 °C. The reaction solution was filtered. The filtrate was washed with 20 mL of water, extracted with 20 mL of ethyl acetate, washed with saturated brine, dried over anhydrous sodium sulfate, concentrated, and purified by column chromatography, to provide 620 mg of compound 5-(3,5-dimethylisoxazol-4-yl)-2-methyl-*N*-(4-(2-methyl-1H-imidazol-1-yl) phenyl)aniline, with a yield of 56%. LC/MS (ESI⁺) calcd for C₂₂H₂₂N₄O ([M+H]⁺) *m*/*z* 359.2. 60% sodium hydride (80 mg, 2.0 mmol) was dissolved in 3 mL of dimethylsulfoxide, to which was added 5-(3,5-dimethylisoxazol-4-yl)-2-methyl-N-(4-(2-methyl-1H-imidazol-1-yl) phenyl)aniline (360 mg, 1.0 mmol). The mixture was stirred for 10 min at room temperature, and then 2-t-butyl (4-((methanesulfonyl)oxy)pentyloxy)acetate (1.4 g, 5.0 mmol) was added. The mixture was allowed to react overnight at 60 °C, and then washed with 15 ml of water, followed by extraction with 15 mL of ethyl acetate. The organic layer was concentrated under reduced pressure, and the residue was separated and purified by TLC, to provide 65 mg of compound t-butyl 2-((5-((5-(3,5-dimethylisoxazol-4-yl)-2-methylphenyl)(4-(2-methyl-1H-imidazol-1-yl)phenyl)amino)pentyl)oxy)acetate, with a yield of 11.6%. LC/MS (ESI⁺) calcd for C₃₃H₄₂N₄O₄ ([M+H]⁺) *m*/*z* 559.3.

Compound t-butyl 2-((5-((5-(3,5-dimethylisoxazol-4-yl)-2-methylphenyl)(4-(2-methyl-1H-imidazol-1-yl)phenyl)amino)pentyl)oxy)acetate (65 mg, 0.12 mmol) was dissolved in 5 mL of dichloromethane, to which was added 5 mL of trifluoroacetic acid, and the mixture was allowed to react at room temperature for 1 h. The reaction solution was concentrated to dryness under reduced pressure, to provide 2-((5-((5-(3,5-dimethylisoxazol-4-yl)-2-methylphenyl)(4-(2-methyl-1H-imidazol-1-yl)phenyl)amino)pentyl)oxy)acetic acid (41 mg), with a yield of 81.6%.

2-((5-((5-(3,5-Dimethylisoxazol-4-yl)-2-methylphenyl)(4-(2-methyl-1H-imidazol-1-yl)phenyl)a mino)pentyl)oxy)acetic acid (10 mg, 0.02 mmol) was dissolved in 1 mL of DMF, to which were successively added diisopropylethylamine (14 mg, 0.11 mmol), HATU (10 mg, 0.025 mmol), and (2*S*,4*R*)-1-((*S*)-2-amino-3,3-dimethylbutyryl)-4-hydroxy-*N*-((*S*)-1-(4-(4-methylthiazol-5-yl)phenyl)ethyl)-pyrrolidinyl-2-formamide (10 mg, 0.023 mmol). The mixture was allowed to react 1 h at room temperature, and then washed with 10 ml of water, followed by extraction with 10 mL of ethyl acetate. The organic layer was concentrated under reduced pressure, and the residue was separated and purified by TLC, to provide 12 mg of compound (2*S*,4*R*)-1-((*S*)-2-(2-((5-((5-(3,5-dimethylisoxazol-4-yl)-2-methylphenyl)(4-(2-methyl-1H-imida zol-1-yl)phenyl)amino)pentyl)oxy)acetylamino)-3,3-dimethylbutyryl)-4-hydroxy-*N*-((*S*)-1-(4-(4 -methylthiazol-5-yl)phenyl)ethyl)-pyrrolidinyl-2-formamide. LC/MS (ESI⁺) calcd for C₅₂H₆₄N₈O₆S ([M+H]⁺) *m*/*z* 930.4.

¹H NMR (400 MHz, CDCl₃) *δ* 8.67 (s, 1H), 7.42 - 7.32 (m, 6H), 7.22 - 7.13 (m, 2H), 7.05 (d, *J* = 8.8 Hz, 3H), 6.98 (s, 1H), 6.93 (s, 1H), 6.59 - 6.48 (m, 2H), 5.39 - 5.31 (m, 1H), 5.10 - 5.05 (m, 1H), 4.73 (d, *J* = 7.7 Hz, 1H), 4.56 (d, *J* = 8.8 Hz, 1H), 4.16 - 4.10 (m, 2H), 3.93 (d, *J* = 2.9 Hz, 2H), 3.64 - 3.59 (m, 2H), 3.51 (d, *J* = 6.5 Hz, 2H), 2.52 (d, *J* = 2.7 Hz, 4H), 2.42 (s, 3H), 2.32 (s, 3H), 2.28 (s, 3H), 2.18 (s, 3H), 2.10 (d, *J* = 9.1 Hz, 1H), 1.67 (d, *J* = 7.4 Hz, 4H), 1.48 - 1.44 (m, 5H), 1.05 (s, 9H).

### 98: (3R,5S)-1-((S)-2-(2-((5-((5-(3,5-dimethylisoxazol-4-yl)-2-methylphenyl)(4-(2-methyl-1H-imidazol-1-yl)phenyl)amino)pentyl)oxy)acetylamino)-3,3-dimethylbutyryl)-5-(((S)-1-(4 -(4-methylthiazol-5-yl)phenyl)ethyl)aminoformyl)pyrrolidinyl-3-yl acetate (98)

2-((5-((5-(3,5-Dimethylisoxazol-4-yl)-2-methylphenyl)(4-(2-methyl-1H-imidazol-1-yl)phenyl)a mino)pentyl)oxy)acetic acid (16 mg, 0.03 mmol) was dissolved in 4 mL of DMF, to which were successively added diisopropylethylamine (20 mg, 0.15 mmol), HATU (15 mg, 0.04 mmol), and (3*R*,5*S*)-1-((*S*)-2-amino-3,3-dimethylbutyryl)-5-(((*S*)-1-(4-(4-methylthiazol-5-yl)phenyl) ethyl)aminomethyl)pyrrolidinyl-3-acetate (16 mg, 0.03 mmol). The mixture was allowed to react 1 h at room temperature, and then washed with 10 ml of water, followed by extraction with 10 mL of ethyl acetate. The organic layer was concentrated under reduced pressure, and the residue was separated and purified by TLC, to provide 12 mg of compound (3*R*,5*S*)-1-((*S*)-2-(2-((5-((5-(3,5-dimethylisoxazol-4-yl)-2-methylphenyl)(4-(2-methyl-1H-imida zol-1-yl)phenyl)amino)pentyl)oxy)acetylamino)-3,3-dimethylbutyryl)-5-(((*S*)-1-(4-(4-methylthi azol-5-yl)phenyl)ethyl)aminoformyl)pyrrolidinyl-3-yl acetate. LC/MS (ESI⁺) calcd for C₅₄H₆₆N₈O₇S ([M+H]⁺) *m*/*z* 971.3. ¹H NMR (400 MHz, CDCl₃) *δ* 8.68 (s, 1H), 7.37 (dd, *J =* 21.9, 8.1 Hz, 5H), 7.21 (d, *J* = 7.6 Hz, 1H), 7.14 (dd, *J* = 13.0, 8.7 Hz, 2H), 7.05 (d, *J* = 8.0 Hz, 3H), 6.99 (s, 1H), 6.93 (s, 1H), 6.53 (d, *J* = 8.6 Hz, 2H), 5.36 (s, 1H), 5.12 - 5.05 (m, 1H), 4.72 - 4.66 (m, 1H), 4.58 (d, *J* = 9.1 Hz, 1H), 4.06 (d, *J* = 11.3 Hz, 1H), 3.93 (s, 2H), 3.83 (d, *J* = 6.9 Hz, 1H), 3.67 - 3.59 (m, 2H), 3.52 (t, *J* = 6.2 Hz, 2H), 2.73 - 2.65 (m, 1H), 2.53 (s, 3H), 2.42 (s, 3H), 2.33 (s, 3H), 2.28 (s, 3H), 2.18 (s, 3H), 2.12 (dd, *J* = 9.6, 5.2 Hz, 1H), 2.04 (s, 3H), 1.69 - 1.63 (m, 4H), 1.47 (d, *J* = 6.6 Hz, 5H), 1.04 (s, 9H).

### 99: (3R,5S)-1-((S)-2-(3-(4-(((3-Bromo-4-(1H-imidazol-1-yl)phenyl)(5-(3,5-dimethylisoxazol-4-yl)-2-methylphenyl)amino)methyl)piperidin-1-yl)propionamide)-3,3-di methylbutyryl)-5-(((S)-1-(4-(4-methylthiazol-5-yl)phenyl)ethyl)carbamoyl)pyrrolidine-3-yl acetate (99)

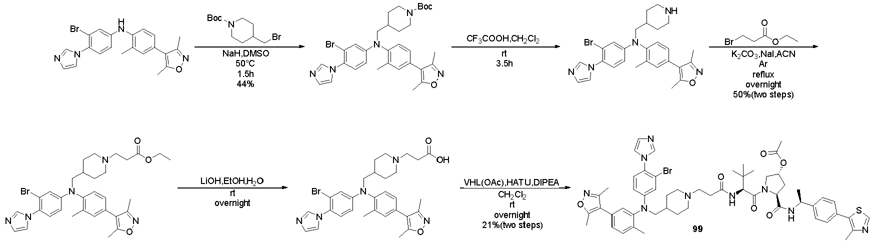

*N*-(3-bromo-4-(1H-imidazol-1-yl)phenyl)-4-(3,5-dimethylisoxazol-4-yl)-2-methylaniline (250 mg, 0.59 mmol) was weighed and placed in a 25 mL single-neck round bottom flask, to which was added DMSO (10 mL), and the mixture was thoroughly stirred at room temperature. The system was then transferred to an oil bath at 50 °C for heating under stirring. After 10 min, NaH (492 mg, 2.95 mmol) was slowly added to the system. After additional 15 min, t-butyl 14-(bromomethyl)piperidine-1-carboxylate (250 mg, 1.77 mmol) was added to the system. After addition, the system was stirred and reacted in an oil bath. After 1.5 h, TLC detection showed the disappearance of raw material. Heating was stopped, and the system was allowed to warm to room temperature, to which were added ethyl acetate (30 mL) and water (15 mL). The mixture was stirred vigorously. After 5 min, the system was allowed to stand still for separation of layers. The aqueous layer was further extracted with ethyl acetate (10 mL^{∗}3). The organic phases were combined, successively washed with water (10 mL^{∗}3) and saturated brine (15 mL), dried over anhydrous sodium sulfate, concentrated *in vacuo*, to obtain a crude product, which was separated and purified by Pre-TLC, to provide t-butyl 4-(((3-bromo-4-(1H-imidazol-1-yl)phenyl)(4-(3,5-dimethylisoxazol-4-yl)-2-methylphenyl)amin o)methyl)piperidine-1-carboxylate (160 mg), with a yield of 44%. LC/MS (ESI⁺) Calcd for C₃₂H₃₈BrN₅O₃ [M+H]⁺ *m*/*z*, 620.6; Found: 620.2.

t-Butyl 4-(((3-bromo-4-(1H-imidazol-1-yl)phenyl)(4-(3,5-dimethylisoxazol-4-yl)-2-methylphenyl)amino)methyl)piperidine-1-carboxylate (150 mg, 0.24 mmol) was weighed and placed in a 25 mL single-neck round bottom flask, to which was added dichloromethane (5 mL), and the mixture was stirred at room temperature. Subsequently, trifluoroacetic acid (2 mL) was added to the system. After addition, the system was stirred and reacted at room temperature. After 3.5 h, the sample was taken out and subjected to TLC, and the result showed the disappearance of the raw materials. The solvent and excess trifluoroacetic acid were removed by rotatory evaporation, and the residual trifluoroacetic acid was removed by repeated evaporation with dichloromethane, to provide an off-white solid *N*-(3-bromo-4-(1H-imidazol-1-yl)phenyl)-4-(3,5-dimethylisoxazol-4-yl)-2-methyl-*N*-(piperidin-4-ylmethyl)aniline trifluoroacetate. Without further purification, it was directly used in the next reaction.

To a 25 mL single-neck round bottom flask containing *N*-(3-bromo-4-(1H-imidazol-1-yl) phenyl)-4-(3,5-dimethylisoxazol-4-yl)-2-methyl-*N*-(piperidin-4-ylmethyl)aniline trifluoroacetate, was added acetonitrile (10 mL), and the mixture was thoroughly stirred at room temperature. Subsequently, ethyl 3-bromopropionate (86 mg, 0.26 mmol), potassium carbonate (133 mg, 0.96 mmol), and sodium iodide (39 mg, 0.26 mmol) were sequentially added to the system. After that, the system was evacuated, and then argon was purged, that was repeated 5 times to ensure an inert gas atmosphere in the system. The system was placed in an oil bath, and then heated and reacted overnight under reflux. The next day, the sample was taken out and subjected to TLC, and the result indicated that the reaction was completed. The solvent was removed by rotary evaporation, and then ethyl acetate (20 mL) and water (10 mL) were added to the system. The resultant solution was stirred vigorously, and then left to stand for separation of layers. The aqueous layer was extracted with ethyl acetate (10 mL^{∗}3), and the organic phase was successively washed with water (10 mL^{∗}2) and saturated brine (10 mL), dried over anhydrous sodium sulfate, and concentrated *in vacuo* to obtain a crude product, which was separated and purified by column chromatography, to provide ethyl 3 -(4-(((3 -bromo-4-(1H-imidazol-1-yl)phenyl)(4-(3, 5-dimethylisoxazol-4-yl)-2-methylphenyl)a mino)methyl)piperidin-1-yl)propionate (75 mg), with a two-step yield of 50%. LC/MS (ESI⁺) Calcd for C₃₂H₃₈BrN₅O₃ [M+H] ⁺ *m*/*z*, 620.6; Found: 620.2.

Ethyl 3 -(4-(((3 -bromo-4-(1H-imidazol-1-yl)phenyl)(4-(3,5-dimethylisoxazol-4-yl)-2-methylphenyl)amino)methyl)piperidin-1-yl)propionate (65 mg, 0.11 mmol) was placed in a 25 mL single-neck round bottom flask, to which was added ethanol (5 mL). The mixture was stirred to dissolve and make the solution become clear at room temperature. Subsequently, 2 mL aqueous solution of lithium hydroxide (14 mg, 0.33 mmol) was added to the system. After addition, the system was stirred and reacted overnight at room temperature. The next day, TLC indicated the completion of the reaction. The solvent was removed by rotary evaporation, and then water (10 mL) was added to the system. The system was stirred at room temperature to dissolve and make the solution become clear. The system was placed in an ice-water bath to cool down under stirring. After 15 min, HCl (1 N) solution was slowly added dropwise into the system to adjust the pH of the system to about 4-5. Water was removed by rotatory evaporation, and the residual water was removed by coevaporation with toluene several times, to provide 3 -(4-(((3 -bromo-4-(1H-imidazol-1-yl)phenyl)(4-(3, 5-dimethylisoxazol-4-yl)-2-methylphenyl)a mino)methyl)piperidin-1-yl)propionic acid. Without further purification, it was directly used in the next reaction.

To a 25 mL single-neck round bottom flask containing 3-(4-(((3-bromo-4-(1H-imidazol-1-yl)phenyl)(4-(3,5-dimethylisoxazol-4-yl)-2-methylphenyl)amino)methyl)piperidin-1-yl)propi onic acid, was added dichloromethane (10 mL), and the mixture was thoroughly stirred at room temperature. Then, DIPEA (43 mg, 0.33 mmol) and HATU (65 mg, 0.17 mmol) were successively added. After 15 min, VHL(OAc) (57 mg, 0.11 mmol) was added to the system. After addition, the system was stirred and reacted overnight at room temperature. The next day, TLC indicated the disappearance of the starting materials. To the system, were added dichloromethane (20 mL) and water (15 mL). The resultant solution was stirred vigorously, and then allowed to stand still for separation of layers. The water layer was further extracted with dichloromethane. The organic phase was combined, successively washed with water (10 mL^{∗}2) and saturated brine (10 mL), dried over anhydrous sodium sulfate, and concentrated *in vacuo* to obtain a crude product, which was separated and purified by Pre-TLC, to provide (3R,5S)-1-((S)-2-(3-(4-(((3-bromo-4-(1H-imidazol-1-yl)phenyl)(5-(3,5-dimethylisoxazol-4-yl)-2-methylphenyl)amino)methyl)piperidin-l-yl)propionamide)-3,3-dimethylbutyryl)-5-(((S)-1-(4-(4-methylthiazol-5-yl)phenyl)ethyl)carbamoyl)pyrrolidine-3-yl acetate (23 mg), with a two-step yield of 21%. LC/MS (ESI⁺) Calcd for C₅₅H₆₆BrN₉O₆S [M+H]⁺ *m*/*z*, 1061.1; Found: 1062.5.

### 100: (2S,4R)-1-((S)-2-(3-(4-(((3-Bromo-4-(1H-imidazol-1-yl)phenyl)(5-(3,5-dimethylisoxazol-4-yl)-2-methylphenyl)amino)methyl)piperidin-1-yl)propionamide)-3,3-di methylbutyryl)-4-hydroxy-N-((S)-1-(4-(4-methylthiazol-5-yl)phenyl)ethyl)pyrrolidine-2-fo rmamide (100)

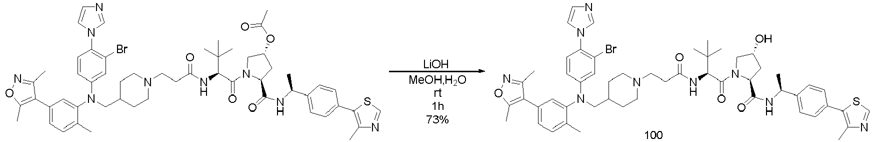

(3R,5S)-1-((S)-2-(3-(4-(((3-bromo-4-(1H-imidazol-1-yl)phenyl)(5-(3,5-dimethylisoxazol-4-yl)-2-methylphenyl)amino)methyl)piperidin-1-yl)propionamide)-3,3-dimethylbutyryl)-5-(((S)-1-(4-(4-methylthiazol-5-yl)phenyl)ethyl)carbamoyl)pyrrolidine-3-yl acetate (10 mg, 0.01 mmol) was weighed and placed in a 25 mL single-neck round bottom flask, to which was added methanol (2 mL). The mixture was stirred to dissolve and make the solution become clear at room temperature. Subsequently, 0.5 mL aqueous solution of lithium hydroxide (2 mg, 0.05 mmol) was added to the system. After addition, the system was stirred and reacted overnight at room temperature. After 1 h, TLC indicated the completion of the reaction. The solvent was removed by rotary evaporation, and then dichloromethane (10 mL) and water (5 mL) were added to the system. The resultant solution was stirred vigorously, and then allowed to stand still for separation of layers. The water layer was further extracted with dichloromethane (5 mL^{∗}3). The organic phase was combined, successively washed with water (5 mL^{∗}2) and saturated brine (10 mL), dried over anhydrous sodium sulfate, and concentrated *in vacuo* to obtain a crude product, which was separated and purified by Pre-TLC, to provide (2S,4R)-1-((S)-2-(3-(4-(((3-bromo-4-(1H-imidazol-1-yl)phenyl)(5-(3,5-dimethylisoxazol-4-yl)-2-methylphenyl)amino)methyl)piperidin-1-yl)propionamide)-3,3-dimethylbutyryl)-4-hydroxy-N-((S)-1-(4-(4-methylthiazol-5-yl)phenyl)ethyl)pyrrolidine-2-formamide (7 mg), with a yield of 73%. LC/MS (ESI⁺) Calcd for C₅₃H₆₄BrN₉O₅S [M+H]⁺ *m*/*z*, 1019.1; Found:1020.5.

### 101: (3R,5S)-1-((S)-2-(2-((1-(2-((3-Bromo-4-(1H-imidazol-1-yl)phenyl)(5-(3,5-dimethyl isoxazol-4-yl)-2-methylphenyl)amino)ethyl)azetidin-3-yl)oxy)acetylamino)-3,3-dimethylbu tyryl)-5-(((S)-1-(4-(4-methylthiazol-5-yl)phenyl)ethyl)carbamoyl)pyrrolidine-3-yl acetate (101)

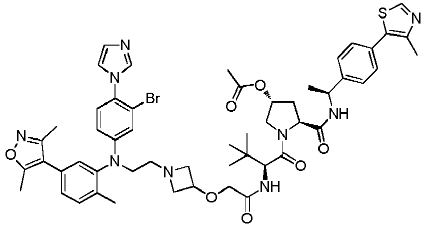

The title compound was synthesized by referring to the method of the previous example. LC/MS (ESI⁺) Calcd for C₅₃H₆₂BrN₉O₇S [M+H]⁺ *m*/*z*, 1049.1; Found: 1050.4.

### 102: 1-(4-((5-(3,5-Dimethylisoxazol-4-yl)-2-methylphenyl)(2-(((3R)-1-(2-(2,6-dioxopiperidin-3-yl)-1,3-dioxoisoindolin-5-yl)pyrrolidin-3-yl)oxy)propyl)amino)phenyl)cyc lopropanecarbonitrile (102)

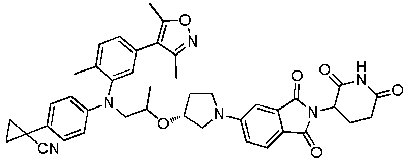

The title compound was synthesized by referring to the method of the previous example. LC/MS (ESI⁺) Calcd for C₄₂H₄₂N₆O₆ [M+H]⁺ *m*/*z*, 726.8; Found: 727.3. ¹H NMR (400 MHz, DMSO) *δ* 11.06 (s, 1H), 7.56 (t, *J* = 7.5 Hz, 1H), 7.33 (t, *J* = 7.8 Hz, 1H), 7.26 (d, *J* = 23.3 Hz, 1H), 7.21 - 7.07 (m, 2H), 7.02 (d, *J* = 8.3 Hz, 1H), 6.70 (d, *J* = 33.1 Hz, 2H), 6.62 - 6.43 (m, 2H), 5.13 - 5.02 (m, 1H), 4.31 (br, 1H), 3.99 - 3.77 (m, 2H), 3.72 (ddd, *J* = 12.0, 8.6, 6.2 Hz, 1H), 3.66 - 3.47 (m, 2H), 3.21 - 3.10 (m, 1H), 3.10 - 3.00 (m, 1H), 2.98 - 2.82 (m, 1H), 2.60 (ddd, *J* = 10.1, 7.9, 5.5 Hz, 1H), 2.33 (d, *J* = 15.2 Hz, 3H), 2.16 (d, *J* = 16.9 Hz, 2H), 2.06 - 1.89 (m, 4H), 1.59 (s, 2H), 1.30 (dd, *J* = 12.3, 6.7 Hz, 2H), 1.24 (s, 3H), 1.21 - 1.11 (m, 3H).

### 103: 1-(4-((5-(3,5-Dimethylisoxazol-4-yl)-2-methylphenyl)(2-(((3S)-1-(2-(2,6-dioxopiperidin-3-yl)-1,3-dioxoisoindolin-5-yl)pyrrolidin-3-yl)oxy)propyl)amino)phenyl)cyc lopropanecarbonitrile (103)

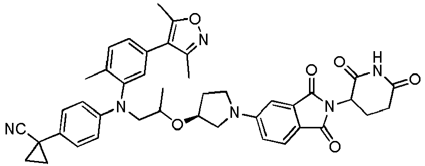

The title compound was synthesized by referring to the method of the previous example. LC/MS (ESI⁺) Calcd for C₄₂H₄₂N₆O₆ [M+H]⁺ *m*/*z*, 726.8; Found: 727.3. ¹H NMR (400 MHz, DMSO) *δ* 11.05 (s, 1H), 7.56 (t, *J* = 7.4 Hz, 1H), 7.33 (t, *J* = 7.7 Hz, 1H), 7.26 (d, *J* = 23.8 Hz, 1H), 7.16 (t, *J* = 7.5 Hz, 1H), 7.10 (d, *J* = 8.2 Hz, 1H), 7.02 (d, *J* = 8.4 Hz, 1H), 6.78 - 6.40 (m, 4H), 5.12 - 5.00 (m, 1H), 4.31 (br, 1H), 3.99 - 3.86 (m, 1H), 3.77 (dd, *J* = 42.8, 14.3 Hz, 1H), 3.56 (ddd, *J* = 21.1, 14.7, 9.1 Hz, 2H), 3.45 - 3.37 (m, 1H), 3.27 (dd, *J* = 14.3, 5.3 Hz, 1H), 3.15 (dd, *J* = 15.1, 7.7 Hz, 1H), 3.05 (d, *J* = 11.1 Hz, 1H), 2.97 - 2.80 (m, 1H), 2.66 - 2.54 (m, 2H), 2.33 (d, *J* = 15.1 Hz, 3H), 2.16 (d, *J* = 16.9 Hz, 3H), 1.95 (d, *J* = 16.6 Hz, 3H), 1.60 (d, *J* = 7.6 Hz, 2H), 1.36 - 1.26 (m, 2H), 1.24 (br, 1H), 1.22 - 1.13 (m, 4H).

### 104: 2-Chloro-4-((5-(3,5-dimethylisoxazol-4-yl)-2-methylphenyl)((1r,4r)-4-((2-(2,6-dioxopiperidin-3-yl)-1,3-dioxoisoindolin-5-yl)oxy)methyl)cyclohexyl)methyl)amino)benzon itrile (104)

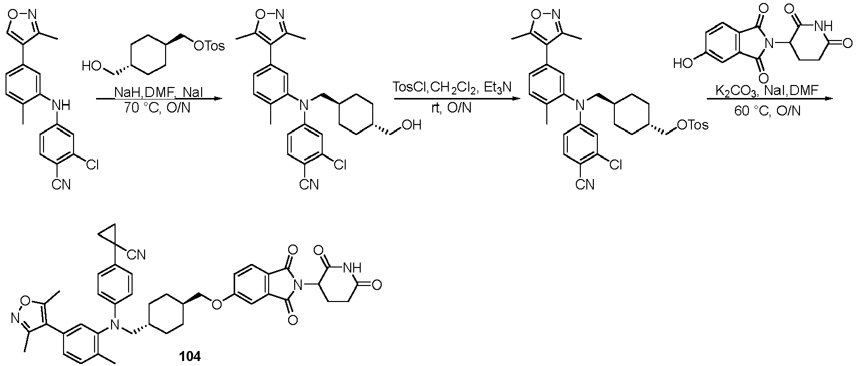

By referring to the method in the above example, the target compound was synthesized according to the above route. LC/MS (ESI⁺) calcd for C₄₀H₃₉ClN₅O₆⁺ ( [M+H]⁺) *m*/*z*: 720.3; found 720.4. ¹H NMR (400 MHz, DMSO-d₆) *δ* 11.12 (s, 1H), 7.82 (d, *J* = 8.3 Hz, 1H), 7.59 (d, *J* = 8.9 Hz, 1H), 7.53 (d, *J* = 8.0 Hz, 1H), 7.44 - 7.36 (m, 2H), 7.33 (dd, *J* = 8.3, 2.1 Hz, 1H), 7.30 (s, 1H), 6.68 (s, 1H), 6.49 (d, *J* = 7.3 Hz, 1H), 5.12 (dd, *J* = 12.9, 5.3 Hz, 1H), 4.01 (dd, *J* = 17.9, 6.6 Hz, 2H), 3.84 (s, 1H), 3.32 - 3.15 (m, 1H), 2.95 - 2.87 (m, 1H), 2.57 (dd, *J* = 20.9, 10.9 Hz, 2H), 2.44 (s, 3H), 2.26 (s, 3H), 2.15 - 1.98 (m, 4H), 1.86 (d, *J* = 10.9 Hz, 4H), 1.75 (s, 1H), 1.66 (s, 1H), 1.20 - 0.95 (m, 4H).

### 105: 2-Chloro-4-((5-(3,5-dimethylisoxazol-4-yl)-2-methylphenyl)((1r,4r)-4-((2-(2,6-dioxopiperidin-3-yt)-1-oxoisoindolin-5-yt)oxy)methyl)cyclohexyl)methyl)amino)benzonitril e

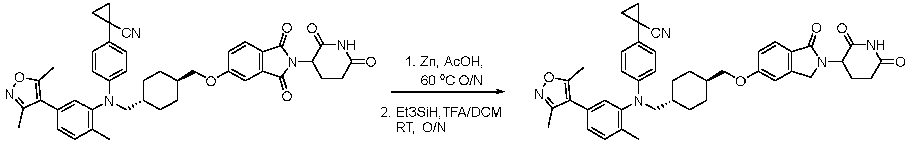

By referring to the method in the above example, the target compound was synthesized according to the above route. LC/MS (ESI⁺) calcd for C₄₀H₄₁ClN₅O₅⁺ ([M+H]⁺) *m*/*z*: 706.3; found 706.3. ¹H NMR (400 MHz, CDCl₃) *δ* 8.06 (s, 1H), 7.77 (d, *J* = 8.3 Hz, 1H), 7.44 (d, *J* = 7.9 Hz, 1H), 7.37 (d, *J* = 8.9 Hz, 1H), 7.31 (d, *J* = 2.1 Hz, 1H), 7.22 (dd, *J* = 7.8, 1.7 Hz, 1H), 7.17 (dd, *J* = 8.3, 2.2 Hz, 1H), 7.05 (d, *J* = 1.6 Hz, 1H), 6.52 (s, 1H), 6.37 (d, *J* = 7.2 Hz, 1H), 4.95 (dd, *J* = 12.3, 5.2 Hz, 1H), 3.89 (d, *J* = 6.2 Hz, 2H), 3.70 (s, 1H), 3.44 - 3.15 (m, 1H), 2.95 - 2.81 (m, 2H), 2.77 - 2.68 (m, 1H), 2.46 - 2.41 (m, 3H), 2.32 - 2.27 (m, 3H), 2.18 - 2.09 (m, 4H), 1.96 (s, 4H), 1.81 (s, 2H), 1.66 (s, 2H), 1.10 (dd, *J* = 20.5, 11.0 Hz, 4H).

### 106: 1-(4-((5-(3,5-Dimethylisoxazol-4-yl)-2-methylphenyl)(2-((1-(2,6-dioxopiperidin-3-yl)-1,3-dioxoisoindolin-5-yl)piperidin-4-yl)oxy)propyl)amino)phenyl)cyclopropanecarbonitril e (106)

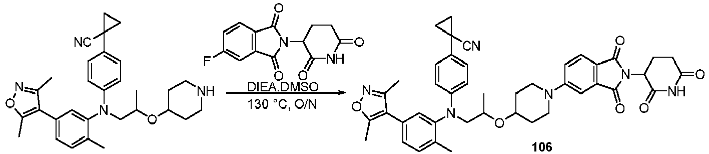

By referring to the method in the above example, the target compound was synthesized according to the above route. LC/MS (ESI⁺) calcd for C₄₃H₄₅ClN₆O₆⁺ ( [M+H]⁺ ) *m*/*z*: 741.3; found 741.4.

### 107: 1-(4-((5-(3,5-Dimethylisoxazol-4-yl)-2-methylphenyl)((1-(2-(2,6-dioxopiperidin-3-yl)-6-fluoro-1,3-dioxoisoindolin-5-yl)pyrrolidin-3-yl)methyl)amino)phenyl)cyclopropanecarbo nitrile (107)

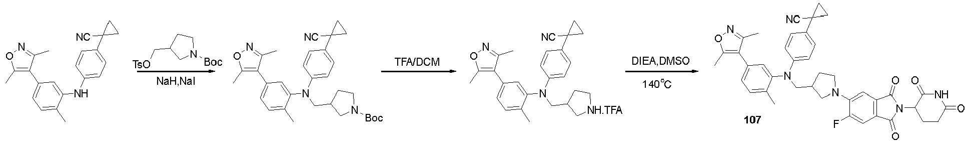

1-(4-((5-(3,5-Dimethylisoxazol-4-yl)-2-methylphenyl)amino)phenyl)cyclopropanecarbonitrile (200 mg, 0.58 mmol) was dissolved in 5mL of DMF, to which was added NaH (42 mg, 1.75 mmol), and the mixture was stirred for 30 min. Then, t-butyl 3-((tosyloxy)methyl)pyrrolidine-1-carboxylate (414 mg, 1.16 mmol) and NaI (9 mg, 0.06 mmol) were added. The reaction solution was heated to 80 °C and reacted overnight, and then cooled to room temperature, to which were added water and EA for extraction. The organic phase was successively washed with water and saturated brine, and then dried over anhydrous sodium sulfate and concentrated, followed by purification by column chromatography, to provide t-butyl 3-((((4-(1-cyanocyclopropyl)phenyl)(5-(3,5-dimethylisoxazol-4-yl)-2-methylphenyl) amino)methyl)pyrrolidine-1-carboxylate (120 mg), with a yield of 39.7%. MS (ESI) *m*/*z* 526.3 [M+H]⁺.

t-Butyl 3-((((4-(1-cyanocyclopropyl)phenyl)(5-(3,5-dimethylisoxazol-4-yl)-2-methylphenyl) amino)methyl)pyrrolidine-1-carboxylate (110 mg, 0.26 mmol) was dissolved in 2 mL of DCM, to which was added 2 mL of TFA, and the mixture was allowed to react 1 h at room temperature. The reaction was completed by TLC detection. The reaction solution was directly concentrated to dry, and then dichloromethane was added to dissolve the residue, followed by concentration, that was repeated several times, to remove most of TFA and provide compound 1-(4-((5-(3,5-dimethylisoxazol-4-yl)-2-methylphenyl)(pyrrolidin-3-ylmethyl)amino)phenyl)acr ylonitrile trifluoroacetate (121 mg), with a yield of 107.1%. MS (ESI) *m*/*z* 426.2 [M+H]⁺. 1-(4-((5-(3,5-Dimethylisoxazol-4-yl)-2-methylphenyl)(pyrrolidin-3-ylmethyl)amino)phenyl)acr ylonitrile trifluoroacetate (110 mg, 0.20 mmol) and 2-(2,6-dioxopiperidin-3-yl)-5,6-difluoroisoindoline-1,3-dione (66 mg, 0.22 mmol) were dissolved in 3 mL of DMSO, to which was added DIEA (131 mg, 1.02 mmol), and then the solution was heated to 120 °C and reacted 2 h under stirring. The reaction was completed by detection. The system was slowly added into water under stirring, and then extracted with EA. EA phase was washed with the saturated aqueous solution of citric acid, dried, concentrated *in vacuo*, and purified to provide 1-(4-((5-(3,5-dimethylisoxazol-4-yl)-2-methylphenyl) ((1-(2-(2,6-dioxopiperidin-3-yl)-6-fluoro-1,3-dioxoisoindol-5-yl)pyrrolidin-3-yl)methyl)amino) phenyl)cyclopropanecarbonitrile (103 mg), with a yield of 72.6%. LC/MS (ESI⁺) Calcd for C₄₀H₃₇FN₆O₅ (M+H⁺) *m*/*z*, 700.3; found 700.3.

### 108: 1-(4-((5-(3,5-Dimethylisoxazol-4-yl)-2-methylphenyl)(4-((2-(2,6-dioxopiperidin-3-yl)-1,3-dioxoisoindolin-5-yl)oxy)butyl)amino)phenyl)cyclopropanecarbonitrile (108)

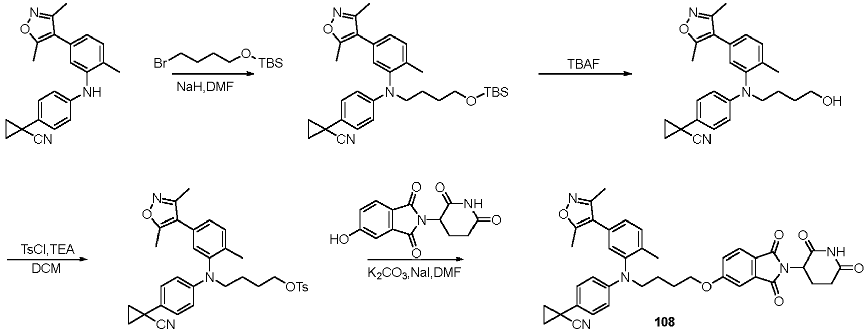

1-(4-((5-(3,5-Dimethylisoxazol-4-yl)-2-methylphenyl)amino)phenyl)cyclopropanecarbonitrile (200 mg, 0.58 mmol) was dissolved in 6 mL of DMF, to which was added NaH (70 mg, 1.75 mmol), and the reaction solution was stirred for 30 min. Then, (4-bromobutoxy)(t-butyl)dimethylsilane (311.3 mg, 1.16 mmol) was added. Once the reaction was completed, the reaction was quenched with water. The resultant solution was extracted with ethyl acetate, and the organic phase was washed several times with water, followed by drying, concentrating, and purifying, to provide 1-(4-((4-((t-butyldimethylsilyl)oxy)butyl) (5-(3,5-dimethylisoxazol-4-yl)-2-methylphenyl)amino)phenyl)propane (213.5 mg), with a yield of 69.2%. LC/MS (ESI⁺) Calcd for C₃₂H₄₃N₃O₂Si (M+H⁺) *m*/*z*, 530.2; found 530.2.

1-(4-((4-((t-Butyldimethylsilyl)oxy)butyl)(5-(3,5-dimethylisoxazol-4-yl)-2-methylphenyl)amin o)phenyl)propane (200 mg, 0.38 mmol) was dissolved in 5 mL of DCM, to which was added TBAF (148 mg, 0.57 mmol), and the mixture was allowed to react at room temperature for 2h. The solution was washed with water, dired, and purified, to provide 1-(4-((5-(3,5-dimethylisoxazol-4-yl)-2-methylphenyl)(4-hydroxybutyl)amino)phenyl)cycloprop anecarbonitrile (164 mg). LC/MS (ESI⁺) Calcd for C₂₆H₂₉N₃O₂ (M+H⁺) *m*/*z*, 415.2; found 415.2.

1-(4-((5-(3,5-Dimethylisoxazol-4-yl)-2-methylphenyl)(4-hydroxybutyl)amino)phenyl)cyclopro panecarbonitrile (150 mg, 0.36 mmol) was dissolved in 3 mL of dichloromethane, to which was added 0.25 mL of triethylamine, and the mixture was cooled to about 0 °C. Then, a mixture of p-toluenesulfonyl chloride (103 mg, 0.54 mmol) and 1 mL DCM was added dropwise. The resultant mixture was allowed to react overnight at room temperature. The next day, the solution was washed with water, dried, and purified by column chromatography, to provide compound t-buytl 4-((4-(1-cyanochloropropyl)phenyl)(5 -(3,5-dimethylisoxazol-4-yl)-2-methylphenyl)amino)4-toluenesulfonate (108 mg). LC/MS (ESI⁺) Calcd for C₃₃H₃₅N₃O₄S (M+H⁺) *m*/*z*, 570.2; found 570.2.

t-Butyl 4-((4-(1-cyanochloropropyl)phenyl)(5-(3,5-dimethylisoxazol-4-yl)-2-methylphenyl) amino)4-toluenesulfonate (50 mg, 0.09 mmol), 2-(2,6-dioxopiperidin-3-yl)-5-hydroxyisoindole-1,3-dione (29 mg, 0.11 mmol), and potassium carbonate (18 mg, 0.13 mmol) were mixed in 2 mL of DMF, and the mixture was heated to 60 °C and reacted 2 h. The reaction was quenched with water, and then the reaction solution was extracted with EA, followed by drying and purification, to provide 1-(4-((5-(3,5-dimethylisoxazol-4-yl)-2-methylphenyl) (4-((2-(2,6-dioxopiperidin-3-yl)-1,3-dioxoisoindol-5-yl)oxy)butyl)amino)phenyl)cyclopropanec arbonitrile (35 mg). LC/MS (ESI⁺) Calcd for C₃₉H₃₇N₅0₆ (M+H⁺) *m*/*z*, 672.2; found 672.2. ¹H NMR (400 MHz, DMSO-d₆) δ 11.12 (s, 1H), 7.82 (d, *J* = 8.2 Hz, 1H), 7.45 (d, *J* = 7.8 Hz, 1H), 7.40 (s,1H), 7.29 (dd, *J* = 17.3, 7.7 Hz, 2H), 7.20-7.07 (m, 3H), 6.49 (d, *J* = 8.6 Hz, 2H), 5.12 (dd, *J* = 12.8, 5.5Hz, 1H), 4.21 (s, 2H), 3.68 (s, 2H), 2.88 (d, *J* = 13.0 Hz, 2H), 2.39 (s, 3H), 2.21 (s, 3H), 2.08 (s, 3H), 1.82(s, 4H), 1.60 (s, 2H), 1.32 (s, 4H).

### 109: 1-(4-((5-(3,5-Dimethylisoxazol-4-yl)-2-methylphenyl)((1-(1-(2-(2,6-dioxopiperidin-3-yl)-1,3-dioxoisoindolin-5-yl)azetidin-3-yl)piperidin-4-yl)methyl)amino)phenyl)cyclopropan ecarbonitrile (109)

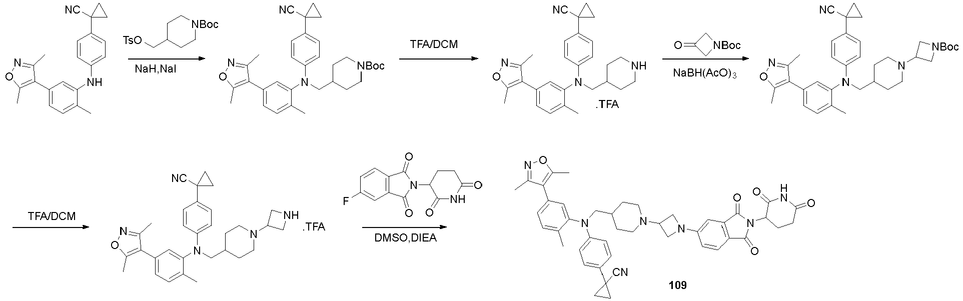

By referring to the method in the above example, the target compound was synthesized according to the above route. LC/MS (ESI⁺) Calcd for C₄₄H₄₅N₇O₅ (M+H⁺) *m*/*z*, 751.3; found 751.3. ¹H NMR (400 MHz, CDCl₃) *δ* 8.17 (s, 1H), 7.64 (d, *J* = 8.3 Hz, 1H), 7.36 (d, *J* = 7.9 Hz, 1H), 7.11 (dd, *J* = 11.5, 5.3 Hz, 3H), 7.04 (d, *J* = 1.7 Hz, 1H), 6.78 (d, *J* = 2.0 Hz, 1H), 6.52 (dd, *J* = 8.3, 2.1 Hz, 1H), 6.47 (d, *J* = 8.9 Hz, 2H), 4.92 (dd, *J* = 12.3, 5.3 Hz, 2H), 4.10 (t, *J* = 7.5 Hz, 2H), 3.89 (s, 2H), 3.52 (d, *J* = 6.4 Hz, 2H), 3.34 (s, 1H), 2.91 (s, 2H), 2.88 - 2.66 (m, 4H), 2.41 (s, 3H), 2.27 (s, 3H), 2.15 - 2.12 (m, 1H), 2.08 (s, 3H), 1.61 (dd, *J* = 7.3, 4.7 Hz, 3H), 1.37 (d, *J* = 10.5 Hz, 2H), 1.28 (d, *J* = 2.5 Hz, 2H), 1.25 (s, 2H).

### 110: 1-(4-((5-(3,5-Dimethylisoxazol-4-yl)-2-methylphenyl)(2-((1-(2-(2,6-dioxopiperidin-3-yl)-1,3-dioxoisoindolin-5-yl)-3-methylazetidin-3-yl)oxy)ethyl)amino)phenyl)cyclopropan ecarbonitrile (110)

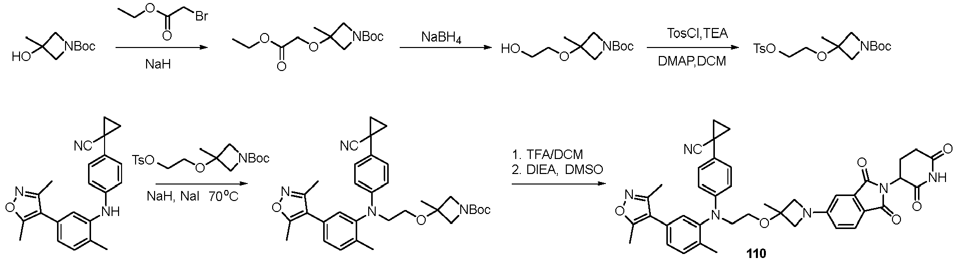

By referring to the method in the above example, the target compound was synthesized according to the above route. LC/MS (ESI⁺) Calcd for C₄₁H₄₀N₆O₆ (M+H⁺) *m*/*z*, 713.3; found 713.3. ¹H NMR (400 MHz, DMSO-d₆) *δ* 10.98 (s, 1H), 7.37 - 7.27 (m, 2H), 7.15 (s, 1H), 7.10 (d, *J* = 8.7 Hz, 2H),7.08 (s, 1H), 6.97 (s, 1H), 6.76 (d, *J* = 8.5 Hz, 1H), 6.63 (d, *J* = 21.0 Hz, 1H), 6.47 (d, *J* = 8.9 Hz, 2H), 5.30 (d, *J* = 19.3 Hz, 1H), 5.08 (dd, *J* = 13.3, 5.2 Hz, 1H), 4.26 (dd, *J* = 48.5, 16.8 Hz, 3H), 3.78 (d, *J* = 6.3 Hz, 4H),3.58 (s, 3H), 2.89 (d, *J* = 12.4 Hz, 1H), 2.31 (d, *J* = 11.9 Hz, 1H), 2.13 (s, 1H), 2.01 (d, *J* = 7.3 Hz, 3H), 1.85 (s, 3H), 1.81 (s, 1H), 1.59 (d, *J* = 2.5 Hz, 1H), 1.45 (s, 3H), 1.37 - 1.30 (m, 2H), 1.23 (s, 3H).

### 111: 1-(4-((5-(3,5-Dimethylisoxazol-4-yl)-2-methylphenyl)(2-((1-(2-(2,6-dioxopiperidin-3-yl)-6-fluoro-1,3-dioxoisoindolin-5-yl)-3-methylazetidin-3-yl)oxy)ethyl)amino)phenyl)cyclo propanecarbonitrile (111)

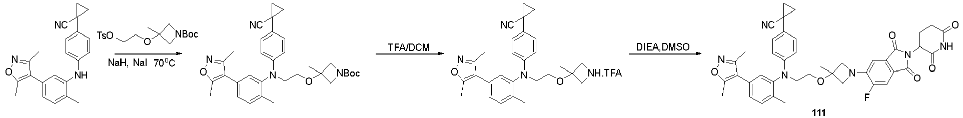

By referring to the method in the above example, the target compound was synthesized according to the above route. LC/MS (ESI⁺) Calcd for C₄₁H₃₉FN₆O₆ (M+H⁺) *m*/*z*, 731.3; found 731.3. ¹H NMR (400 MHz, DMSO-d₆) *δ* 10.93 (s, 1H), 7.37 - 7.27 (m, 2H), 7.15 (s, 1H), 7.10 (d, *J* = 8.7 Hz, 2H), 6.97 (s, 1H), 6.76 (d, *J* = 8.5 Hz, 1H), 6.63 (d, *J* = 21.0 Hz, 1H), 6.47 (d, *J* = 8.9 Hz, 2H), 5.30 (d, *J* = 19.3 Hz, 1H), 5.08 (dd, *J* = 13.3, 5.2 Hz, 1H), 4.26 (dd, *J* = 48.5, 16.8 Hz, 3H), 3.78 (d, *J* = 6.3 Hz, 4H),3.58 (s, 3H), 2.89 (d, *J* = 12.4 Hz, 1H), 2.31 (d, *J* = 11.9 Hz, 1H), 2.13 (s, 1H), 2.01 (d, *J* = 7.3 Hz, 3H), 1.85 (s, 3H), 1.81 (s, 1H), 1.59 (d, *J* = 2.5 Hz, 1H), 1.45 (s, 3H), 1.37 - 1.30 (m, 2H), 1.21 (s, 3H).

### 112: 1-(4-((5-(3,5-Dimethylisoxazol-4-yl)-2-methylphenyl)(2-((1-(2-(2,6-dioxopiperidin-3-yl)-6-fluoro-3-oxoisoindolin-5-yl)-3-methylazetidin-3-yl)oxy)ethyl)amino)phenyl)cycloprop anecarbonitrile (112)

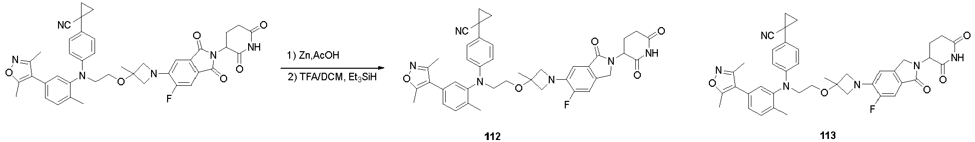

By referring to the method in the above example, the target compound was synthesized according to the above route. LC/MS (ESI⁺) Calcd for C₄₁H₄₁FN₆O₅ (M+H⁺) *m*/*z,* 717.3; found 717.3. ¹H NMR (400 MHz, DMSO-d₆) *δ* 10.98 (s, 1H), 7.37 - 7.27 (m, 2H), 7.15 (s, 1H), 7.10 (d, *J* = 8.7 Hz, 2H), 6.97 (s, 1H), 6.76 (d, *J* = 8.5 Hz, 1H), 6.63 (d, *J* = 21.0 Hz, 1H), 6.47 (d, *J* = 8.9 Hz, 2H), 5.30 (d, *J* = 19.3 Hz, 1H), 5.08 (dd, *J* = 13.3, 5.2 Hz, 1H), 4.26 (dd, *J* = 48.5, 16.8 Hz, 3H), 3.78 (d, *J* = 6.3 Hz, 4H), 3.71 (d, *J* = 7.2 Hz, 2H), 3.58 (s, 3H), 2.89 (d, *J* = 12.4 Hz, 1H), 2.31 (d, *J* = 11.9 Hz, 1H), 2.13 (s, 1H), 2.01 (d, *J* = 7.3 Hz, 3H), 1.85 (s, 3H), 1.81 (s, 1H), 1.59 (d, *J* = 2.5 Hz, 1H), 1.45 (s, 3H), 1.37 - 1.30 (m, 2H), 1.21 (s, 3H).

### 113: 1-(4-((5-(3,5-Dimethylisoxazol-4-yl)-2-methylphenyl)(2-((1-(2-(2,6-dioxopiperidin-3-yl)-6-fluoro-1-oxoisoindolin-5-yl)-3-methylazetidin-3-yl)oxy)ethyl)amino)phenyl)cycloprop anecarbonitrile (113)

By referring to the method in the above example, the target compound was synthesized according to the above route. LC/MS (ESI⁺) Calcd for C₄₁H₄₁FN₆O₅ (M+H⁺) *m*/*z*, 717.3; found 717.3. ¹H NMR (400 MHz, DMSO-d₆) *δ* 10.98 (s, 1H), 7.37 - 7.27 (m, 2H), 7.15 (s, 1H), 7.10 (d, *J* = 8.7 Hz, 2H), 6.97 (s, 1H), 6.76 (d, *J* = 8.5 Hz, 1H), 6.63 (d, *J* = 21.0 Hz, 1H), 6.47 (d, *J* = 8.9 Hz, 2H), 5.30 (d, *J* = 19.3 Hz, 1H), 5.08 (dd, *J* = 13.3, 5.2 Hz, 1H), 4.26 (dd, *J* = 48.5, 16.8 Hz, 3H), 3.78 (d, *J* = 6.3 Hz, 4H), 3.71 (d, *J* = 7.2 Hz, 2H), 3.58 (s, 3H), 2.89 (d, *J* = 12.4 Hz, 1H), 2.35 (d, *J* = 11.9 Hz, 1H), 2.13 (s, 1H), 2.01 (d, *J* = 7.3 Hz, 3H), 1.85 (s, 3H), 1.81 (s, 1H), 1.59 (d, *J* = 2.5 Hz, 1H), 1.45 (s, 3H), 1.37 - 1.30 (m, 2H), 1.24 (s, 3H).

### 114: 1-(2-Chloro-4-((5-(3,5-dimethylisoxazol-4-yl)-2-methylphenyl)((((1r,4r)-4-((2-(2,6-dioxopiperidin-3-yl)-6-fluoro-3-oxoisoindolin-5-yl)amino)cyclohexyl)methyl)amino)phenyl )cyclopropanecarbonitrile (114)

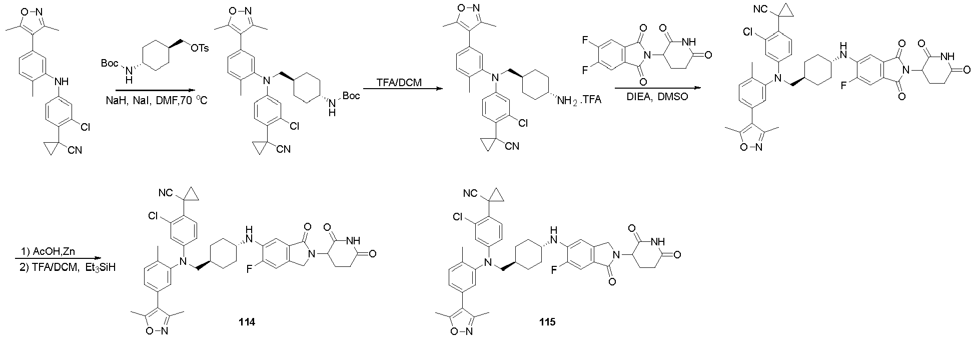

By referring to the method in the above example, the target compound was synthesized according to the above route. LC/MS (ESI⁺) Calcd for C₄₂H₄₂FN₆O₄ (M+H⁺) *m*/*z,* 749.3; found 749.3. ¹H NMR (400 MHz, CDCl₃) *δ* 8.01 (s, 1H), 7.42 (t, *J* = 9.0 Hz, 2H), 7.14 (dd, *J* = 11.2, 5.2 Hz, 3H), 7.08 (d, *J=* 7.5 Hz, 2H), 6.51 (d, *J=* 8.8 Hz, 2H), 5.30 (s, 1H), 4.94 (dd, *J=* 12.2, 5.2 Hz, 1H), 3.39 (d, *J=* 6.7 Hz, 2H), 3.37 (s, 1H), 2.84 (ddd, *J=* 30.1, 28.5, 14.9 Hz, 4H), 2.45 (s, 3H), 2.31 (s, 3H), 2.21 (d, *J* = 10.1 Hz, 2H), 2.12 (s, 3H), 2.04 (d, *J* = 10.5 Hz, 2H), 1.85 (s, 2H), 1.64 (q, *J=* 4.8 Hz, 2H), 1.32 (dd, *J=* 7.5, 5.1 Hz, 3H), 1.27 - 1.23 (m, 4H).

### 115: 1-(4-((5-(3,5-Dimethylisoxazol-4-yl)-2-methylphenyl)((((1r,4r)-4-((2-(2,6-dioxopiperidin-3-yl)-6-fluoro-1-oxoisoindolin-5-yl)amino)cyclohexyl)methyl)amino)phenyl )cyclopropanecarbonitrile (115)

LC/MS (ESI⁺) Calcd for C₄₂H₄₂FN₆O₄ (M+H⁺) *m*/*z,* 749.3; found 749.3. ¹H NMR (400 MHz, CDCl₃) *δ* 8.01 (s, 1H), 7.41 (t, *J* = 9.0 Hz, 2H), 7.15 (dd, *J* = 11.2, 5.2 Hz, 3H), 7.08 (d, *J* = 7.5 Hz, 2H), 6.50 (d, *J* = 8.8 Hz, 2H), 5.32 (s, 1H), 4.94 (dd, *J* = 12.2, 5.2 Hz, 1H), 3.54 (d, *J* = 6.7 Hz, 2H), 3.38 (s, 1H), 2.84 (ddd, *J=* 30.1, 28.5, 14.9 Hz, 4H), 2.45 (s, 3H), 2.31 (s, 3H), 2.21 (d, *J* = 10.1 Hz, 2H), 2.12 (s, 3H), 2.04 (d, *J* = 10.5 Hz, 2H), 1.84 (s, 2H), 1.64 (q, *J* = 4.8 Hz, 2H), 1.31 (dd, *J=* 7.5, 5.1 Hz, 3H), 1.29 - 1.21 (m, 4H).

### 116: 1-(4-((5-(3,5-Dimethylisoxazol-4-yl)-2-methylphenyl)((((1r,4r)-4-((2-(2,6-dioxopiperidin-3-yl)-1,3-dioxoisoindolin-5-yl)amino)cyclohexyl)methyl)amino)phenyl)cycl opropanecarbonitrile (116)

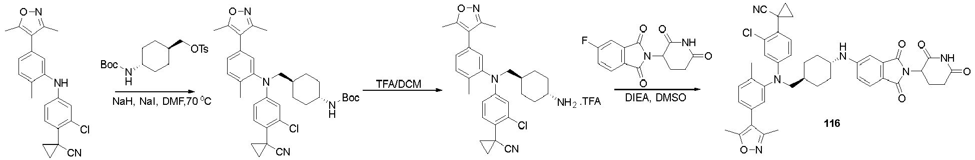

By referring to the method in the above example, the target compound was synthesized according to the above route. LC/MS (ESI⁺) Calcd for C₄₂H₄₁N₆O₅ (M+H⁺) *m*/*z,* 745.3; found 745.3. ¹H NMR (400 MHz, CDCl₃) *δ* 8.01 (s, 1H), 7.41 (t, *J* = 9.0 Hz, 2H), 7.33 (s, 1H),7.15 (dd, *J* = 11.2, 5.2 Hz, 3H), 7.08 (d, *J* = 7.5 Hz, 2H), 6.50 (d, *J* = 8.8 Hz, 2H), 5.32 (s, 1H), 4.94 (dd, *J* = 12.2, 5.2 Hz, 1H), 3.54 (d, *J* = 6.7 Hz, 2H), 3.38 (s, 1H), 2.84 (ddd, *J* = 30.1, 28.5, 14.9 Hz, 4H), 2.45 (s, 3H), 2.31 (s, 3H), 2.21 (d, *J* = 10.1 Hz, 2H), 2.12 (s, 3H), 2.04 (d, *J* = 10.5 Hz, 2H), 1.84 (s, 2H), 1.64 (q, *J* = 4.8 Hz, 2H), 1.31 (dd, *J* = 7.5, 5.1 Hz, 3H), 1.30- 1.22 (m, 4H).

### 117: 1-(4-((5-(3,5-Dimethylisoxazol-4-yl)-2-methylphenyl)((1-(2-((2-(2,6-dioxopiperidin-3-yl)-1,3-dioxoisoindolin-5-yl)amino)ethylpiperidin-4-yl)methyl)amino)phenyl)cyclopropa necarbonitrile (117)

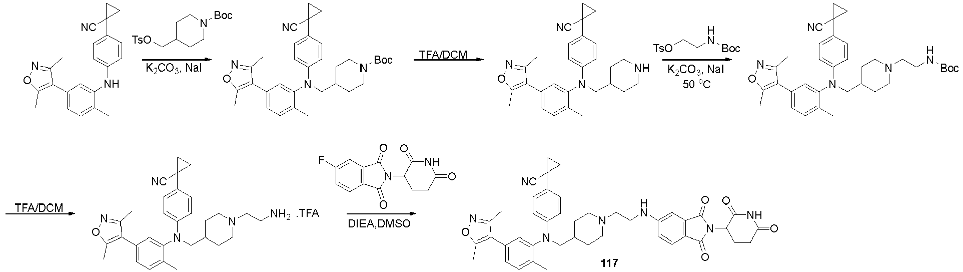

By referring to the method in the above example, the target compound was synthesized according to the above route. LC/MS (ESI⁺) Calcd for C₄₃H₄₅N₇O₅ (M+H⁺) *m*/*z,* 740.3; found 740.3. ¹H NMR (400 MHz, DMSO-*d₆*) *δ* 10.98 (s, 1H), 7.39 - 7.29 (m, 2H), 7.11 (s, 1H), 7.10 (d, *J* = 8.7 Hz, 2H),7.08 (s, 1H), 6.97 (s, 1H), 6.76 (d, *J* = 8.5 Hz, 1H), 6.63 (d, *J* = 21.0 Hz, 1H), 6.47 (d, *J* = 8.9 Hz, 2H), 5.30 (d, *J* = 19.3 Hz, 1H), 5.08 (dd, *J* = 13.3, 5.2 Hz, 1H), 4.26 (dd, *J* = 48.5, 16.8 Hz, 3H), 3.78 (d, *J* = 6.3 Hz, 4H),3.58 (s, 3H), 2.89 (d, *J* = 12.4 Hz, 1H), 2.31 (d, *J* = 11.9 Hz, 1H), 2.13 (s, 1H), 2.01 (d, *J* = 7.3 Hz, 3H), 1.85 (s, 3H), 1.81 (s, 1H), 1.59 (d, *J* = 2.5 Hz, 1H), 1.45 (s, 3H), 1.37 - 1.30 (m, 2H), 1.21(s, 3H).

### 118: 3-(4-((6-((3-Bromo-4-(1H-imidazol-1-yl)phenyl)(5-(3,5-dimethylisoxazol-4-yl)-2-methylphenyl)amino)hexyl)amino)-1-oxoisoindolin-2-yl)piperidine-2,6-dione (118)

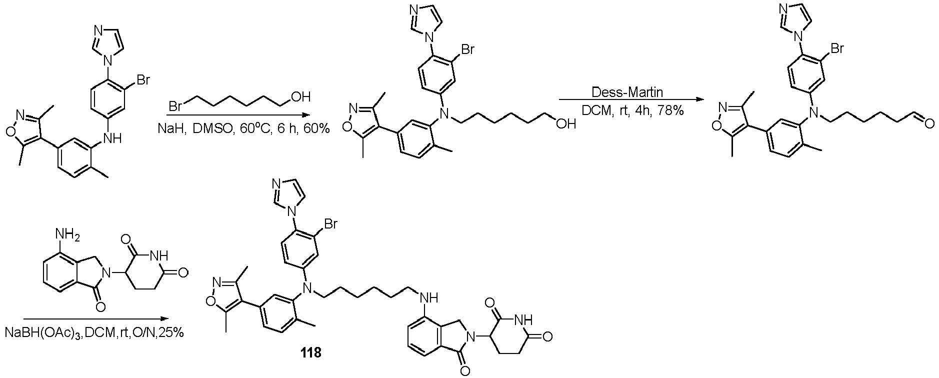

Compound *N*-(3-bromo-4-(1H-imidazol-1-yl)phenyl)-5-(3,5-dimethylisoxazol-4-yl)-2-methylaniline (423 mg, 1.00 mmol) was added into 5 mL of DMSO, and the mixture was moved in an ice bath and stirred. Then, NaH (120 mg, 3.00 mmol) was added, and the mixture was allowed to react for 30 min. 6-Bromohexanol (217 mg, 1.20 mmol) was added dropwise to the reaction solution, and after addition, the ice bath was removed. The reaction solution was heated to 60 °C and stirred for 6 h. 1 mL of H₂O was added to quench the reaction, and then the reaction solution was diluted with a large amount of EA. The organic phase was respectively washed with saturated NH₄Cl solution, saturated NaHCO₃ solution, water and saturated NaCl solution, and then dried over anhydrous sodium sulfate, rotatory evaporated to dry, and purified by silica gel column chromatography, to provide 6-((3-bromo-4-(1H-imidazol-1-yl)phenyl) (5-(3,5-dimethylisoxazol-4-yl)-2-methylphenyl)amino)hexane-1-ol (313 mg, 0.60 mmol), with a yield of 60%. LC/MS (ESI⁺) calcd for: C₂₇H₃₁BrN₄O₂ (M + H⁺) *m*/*z,* 524.2; found, 524.2.

Compound 6-((3 -bromo-4-(1*H*-imidazol-1-yl)phenyl)(5 -(3,5 -dimethylisoxazol-4-yl)-2-methylphenyl)amino)hexane-1-ol (115 mg, 0.22 mmol) was added into 5 mL of DCM, and the mixture was moved in an ice bath and stirred, to which was added Dess-Martin periodinane (117 mg, 0.27 mmol). After addition, the ice bath was removed, and then the mixture was allowed to react 4 h at room temperature. The reaction solution was diluted with DCM. The organic phase was washed with saturated NaHSO₃ solution, saturated NaHCO₃ solution, and saturated NaCl solution, respectively, and then dried over anhydrous sodium sulfate, rotatory evaporated to dry, and purified by silica gel column chromatography, to provide 6-((3 -bromo-4-(1*H*-imidazol-1-yl)phenyl)(5-(3,5-dimethylisoxazol-4-yl)-2-methylphenyl)amino)hexanal (78 mg, 0.17 mmol), with a yield of 78%. LC/MS (ESI⁺) calcd for: C₂₇H₂₉BrN₄O₂ (M + H⁺) *m*/*z,* 521.2; found, 521.2.

Compound 6-((3 -bromo-4-(1*H*-imidazol-1-yl)phenyl)(5 -(3,5 -dimethylisoxazol-4-yl)-2-methylphenyl)amino)hexanal (50 mg, 0.10 mmol) and 3-(4-amino-1-oxoisoindol-2-yl) piperidine-2,6-dione (26 mg, 0.10 mmol) were added into 3 mL of DCM, followed by addition of one drop of CH₃COOH. After the mixture was stirred for 30 min, NaBH(OAc)₃ (63 mg, 0.30 mmol) was added, and then the mixture was allowed to react overnight at room temperature. The reaction solution was diluted with DCM. The organic phase was washed with water and saturated NaCl solution respectively, and then dried over anhydrous sodium sulfate, rotatory evaporated to dry, and purified by silica gel column chromatography, to provide 3 -(4-((6-((3 -bromo-4-(1*H*-imidazol-1-yl)phenyl)(5 -(3, 5-dimethylisoxazol-4-yl)-2-methylphenyl )amino)hexyl)amino)-1-oxoisoindol-2-yl)piperidine-2,6-dione (18 mg, 0.02 mmol), with a yield of 25%. LC/MS (ESI⁺) calcd for: C₄₀H₄₂BrN₇O₂ (M + H⁺) *m*/*z,* 764.2; found, 764.2. ¹H NMR (400 MHz, CDCl₃) *δ* 8.27 (s, 1H), 7.54 (s, 1H), 7.45 (d, *J* = 7.8 Hz, 2H), 7.37 (t, *J* = 7.7 Hz, 2H), 7.21 (d, *J* = 8.1 Hz, 1H), 7.10 (d, *J* = 8.6 Hz, 1H), 7.03 (s, 1H), 6.80 (d, *J* = 8.6 Hz, 2H), 6.45 (d, *J=* 8.7 Hz, 1H), 5.39 - 5.21 (m, 3H), 3.62 (s, 3H), 3.24 (t, *J=* 6.9 Hz, 2H), 2.96 - 2.79 (m, 2H), 2.44 (s, 3H), 2.30 (s, 3H), 2.21 (s, 3H), 1.46 (m, 6H), 0.89 (m, 3H).

### 119: 1-(4-((5-(3,5-dimethylisoxazol-4-yl)-2-methylphenyl)(3-((1-(2-(2,6-dioxopiperidin-3-yl)-1,3-dioxoisoindolin-5-yl)azetidin-3-yl)oxy)propyl)amino)phenyl)cyclopropane-1-nitrile (119)

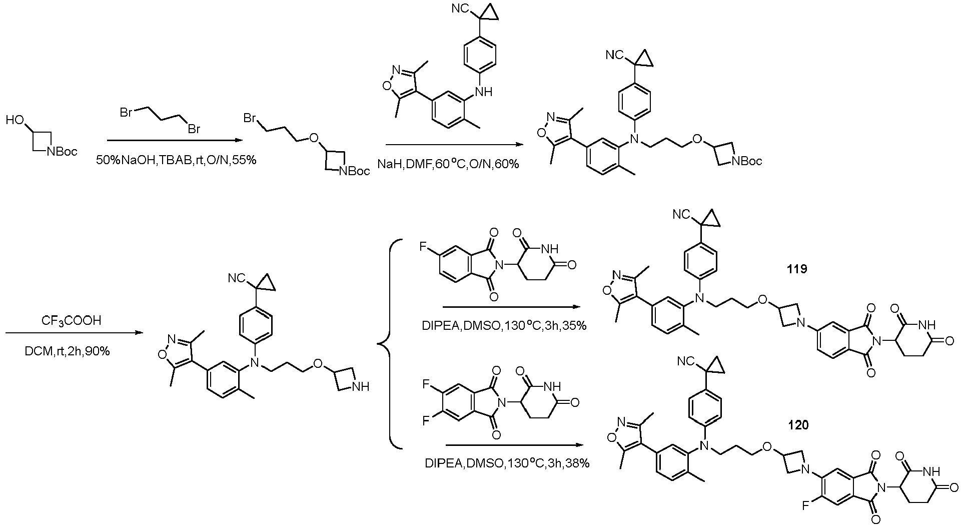

Compound t-butyl 3-hydroxyazetidine-1-carboxylate (294 mg, 1.00 mmol) and 1,3-dibromopropane (1.00 g, 5.00 mmol) were added to 5 mL of 50% NaOH solution, and then TBAB (322 mg, 1.00 mmol) was added. The mixture was stirred and reacted overnight at room temperature. The reaction solution was diluted with DCM. The organic phase was respectively washed with 2 N HCl solution, saturated NaHCO₃ solution, and saturated NaCl solution, and then dried over anhydrous sodium sulfate, rotatory evaporated to dry, and purified by silica gel column chromatography, to provide t-butyl 3-(3-bromopropoxy)azetidine-1-carboxylate (161 mg, 0.55 mmol), with a yield of 55%. LC/MS (ESI⁺) calcd for: C₁₁H₂₀BrNO₃ (M + H⁺) *m*/*z*, 238.1; found, 238.1.

Compound 1-(4-((5 -(3,5-dimethylisoxazol-4-yl)-2-methylphenyl)amino)phenyl) cyclopropane-1-nitrile (170 mg, 0.50 mmol) was added into 5 mL of DMF, and the mixture was moved in an ice bath and stirred. Then, NaH (60 mg, 1.50 mmol) was added, and the mixture was allowed to react for 30 min. t-Butyl 3-(3-bromopropoxy)azetidine-1-carboxylate (150 mg, 0.50 mmol) was added dropwise to the reaction solution, and after addition, the ice bath was removed. The reaction solution was heated to 60 °C and stirred overnight. 1 mL of H₂O was added to quench the reaction, and then the reaction solution was diluted with a large amount of EA. The organic phase was respectively washed with saturated NH₄Cl solution, saturated NaHCO₃ solution, water and saturated NaCl solution, and then dried over anhydrous sodium sulfate, rotatory evaporated to dry, and purified by silica gel column chromatography, to provide t-butyl 3-(3-((4-(1-cyanocyclopropyl)phenyl)(5-(3,5-dimethylisoxazol-4-yl)-2-methylphenyl) amino)propoxy)azetidine-1-carboxylate (160 mg, 0.30 mmol), with a yield of 60%. LC/MS (ESI⁺) calcd for: C₃₃H₄₀N₄O₄ (M + H⁺) *m*/*z,* 557.3; found, 557.3.

Compound t-butyl 3-(3-((4-(1-cyanocyclopropyl)phenyl)(5-(3,5-dimethylisoxazol-4-yl)-2-methylphenyl)amino)propoxy)azetidine-1-carboxylate (111 mg, 0.20 mmol) was added into 2 mL of DCM, and the mixture was moved in an ice bath and stirred, to which was slowly added 4 mL of TFA dropwise. After addition, the ice bath was removed, and then the mixture was allowed to react 2 h at room temperature. The reaction solution was diluted with DCM. The organic phase was washed with saturated NaHCO₃ solution, water, and saturated NaCl solution, respectively, and then dried over anhydrous sodium sulfate, rotatory evaporated to dry, and purified by silica gel column chromatography, to provide compound 1-(4-((3-(azetidin-3-yloxy)propyl)(5-(3,5-dimethylisoxazol-4-yl)-2-methylphenyl)amino)pheny 1)cyclopropane-1-nitrile (82 mg, 0.18 mmol), with a yield of 90%. LC/MS (ESI⁺) calcd for: C₂₈H₃₂N₄O₂ (M + H⁺) *m*/*z,* 457.3; found, 457.3.

Compound 1-(4-((3-(azetidin-3-yloxy)propyl)(5-(3,5-dimethylisoxazol-4-yl)-2-methylphenyl) amino)phenyl)cyclopropane-1-nitrile (68 mg, 0.15 mmol), 2-(2,6-dioxopiperidin-3-yl)-5-fluoroisoindole-1,3-dione (41 mg, 0.15 mmol) and DIEA (58 mg, 0.45 mmol) were added into DMSO, and the mixture was heated to 130 °C and stirred for 3 h. The reaction solution was cooled to room temperature, to which were added water and ethyl acetate for extraction. The organic layer was washed with brine, dried over anhydrous sodium sulfate, rotatory evaporated to dry, and purified by silica gel column chromatography, to provide compound 1-(4-((5-(3,5-dimethylisoxazol-4-yl)-2-methylphenyl)(3-((1-(2-(2,6-dioxopiperidin-3-yl)-1,3 -dioxoisoindolin-5-yl)azetidin-3-yl)oxy)propyl)amino)phenyl)cyclopropane-1-nitrile (35 mg, 0.05 mmol), with a yield of 35%. LC/MS (ESI⁺) calcd for: C₄₃H₄₀ClN₆O₆ (M + H⁺) *m*/*z,* 713.2; found, 713.2. ¹H NMR (400 MHz, Chloroform-*d*) *δ* 8.02 (s, 1H), 7.68 (d, *J* = 8.2 Hz, 1H), 7.40 (d, *J* = 7.8 Hz, 1H), 7.16 (dd, *J* = 7.7, 1.8 Hz, 1H), 7.13 - 7.09 (m, 2H), 7.03 (d, *J* = 1.9 Hz, 1H), 6.79 (d, *J* = 2.1 Hz, 1H), 6.57 - 6.49 (m, 3H), 4.96 (dd, *J* = 12.1, 5.3 Hz, 1H), 4.54 - 4.38 (m, 1H), 4.29 - 4.08 (m, 2H), 3.91 - 3.73 (m, 4H), 3.53 (t, *J=* 5.8 Hz, 2H), 2.99 - 2.67 (m, 4H), 2.41 (s, 3H), 2.27 (s, 3H), 2.16 (s, 3H), 2.07 (s, 1H), 1.37 - 1.18 (m, 5H).

### 120: 1-(4-((5-(3,5-dimethylisoxazol-4-yl)-2-methylphenyl)(3-((1-(2-(2,6-dioxopiperidin-3-yl)-6-fluoro-1,3-dioxoisoindol-5-yl)azetidin-3-yl)oxy)propyl)amino)phenyl)cyclopropane-1-carbonitrile (120)

LC/MS (ESI⁺) calcd for: C₄₁H₃₉FN₆O₆ (M + H⁺) *m*/*z,* 731.3; found, 731.3.

### 121: 1-(4-((5-(3,5-dimethylisoxazol-4-yl)-2-methylphenyl)(3-((1-(2-(2,6-dioxopiperidin-3-yl)-3-oxoisoindolin-5-yl)azetidin-3-yl)oxy)propyl)amino)phenyl)cyclopropane-1-nitrile (121)

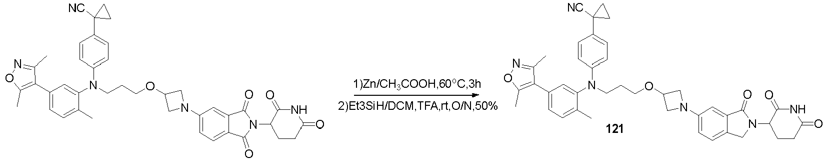

By referring to the method in the above example, the target compound was synthesized according to the above route. LC/MS (ESI⁺) calcd for: C₄₁H₄₂N₆O₅ (M + H⁺) *m*/*z,* 699.3; found, 699.3. ¹H NMR (400 MHz, CDCl₃) *δ* 7.98 (s, 1H), 7.54 (s, 1H), 7.47 (s, 1H), 7.40 (d, *J* = 7.8 Hz, 2H), 7.18 - 7.09 (m, 3H), 7.03 (d, *J* = 11.0 Hz, 1H), 6.52 (d, *J* = 8.7 Hz, 2H), 4.52 (d, *J* = 31.4 Hz, 4H), 4.37 (d, *J* = 16.0 Hz, 1H), 3.92 (s, 2H), 3.79 (s, 2H), 3.55 (s, 2H), 2.39 (s, 3H), 2.25 (s, 3H), 2.16 (s, 3H), 2.04 (d, *J* = 24.7 Hz, 4H), 1.45 (s, 1H), 1.36 (s, 1H), 0.89 (d, *J* = 10.1 Hz, 4H), 0.09 (s, 1H).

### 122: 1-(4-((5-(3,5-dimethylisoxazol-4-yl)-2-methylphenyl)(3-((1-(2-(2,6-dioxopiperidin-3-yl)-1-oxoisoindolin-5-yl)azetidin-3-yl)oxy)propyl)amino)phenyl)cyclopropane-1-nitrile (122)

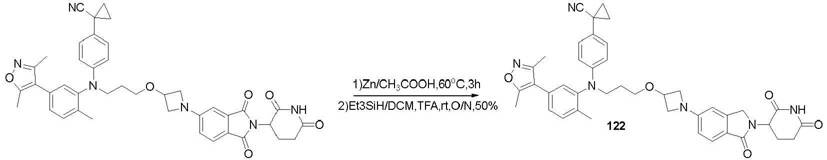

By referring to the method in the above example, the target compound was synthesized according to the above route. LC/MS (ESI⁺) calcd for: C₄₁H₄₂N₆O₅ (M + H⁺) *m*/*z,* 699.3; found, 699.3. ¹H NMR (400 MHz, DMSO-*d₆*) *δ* 10.95 (s, 1H), 7.48 (dd, *J* = 15.3, 8.1 Hz, 2H), 7.27 (dd, *J* = 7.9, 1.8 Hz, 1H), 7.18 (d, *J* = 1.9 Hz, 1H), 7.11 (dd, *J* = 9.3, 2.6 Hz, 2H), 6.48 (m, 4H), 4.41 (s, 1H), 4.30 (d, *J* = 16.9 Hz, 1H), 4.18 (d, *J* = 16.9 Hz, 1H), 4.10 (t, *J* = 7.4 Hz, 2H), 3.72 (t, *J* = 7.4 Hz, 2H), 3.68 - 3.59 (m, 2H), 3.49 (t, *J* = 6.0 Hz, 2H), 2.39 (s, 3H), 2.22 (s, 3H), 2.07 (s, 3H), 1.87 (d, *J* = 8.8 Hz, 4H), 1.59 (q, *J* = 4.6 Hz, 2H), 1.37 - 1.16 (m, 5H).

### 123: 1-(4-((5-(3,5-dimethylisoxazol-4-yl)-2-methylphenyl)(3-((1-(2-(2,6-dioxopiperidin-3-yl)-6-fluoro-3-oxoisoindolin-5-yl)azetidin-3-yl)oxy)propyl)amino)phenyl)cyclopropane-1 -nitrile (123)

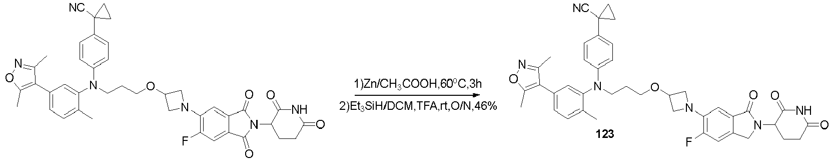

By referring to the method in the above example, the target compound was synthesized according to the above route. LC/MS (ESI⁺) calcd for: C₄₁H₄₁FN₆O₅ (M + H⁺) *m*/*z,* 717.3; found 717.3.

### 124: 1-(4-((5-(3,5-dimethylisoxazol-4-yl)-2-methylphenyl)(3-((1-(2-(2,6-dioxopiperidin-3-yl)-6-fluoro-1-oxoisoindolin-5-yl)azetidin-3-yl)oxy)propyl)amino)phenyl)cyclopropane-1 -nitrile (124)

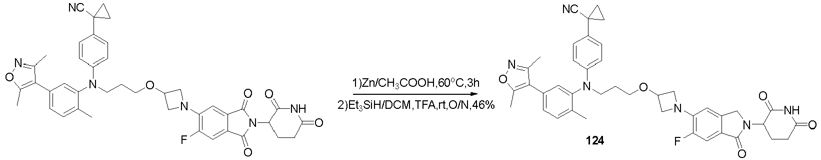

By referring to the method in the above example, the target compound was synthesized according to the above route. LC/MS (ESI⁺) calcd for: C₄₁H₄₁FN₆O₅ (M + H⁺) *m*/*z,* 717.3; found 717.3.

### 125: 1-(4-((5-(3,5-dimethylisoxazol-4-yl)-2-methylphenyl)((2-(2-(2-,2,6-dioxopiperidin-3-yl)-6-fluoro-1-oxoisoindolin-5-yl)-2-azaspiro[3.3]heptan-6-yl)methyl)amino)phenyl)cyclo propane-1-carbonitrile (125)

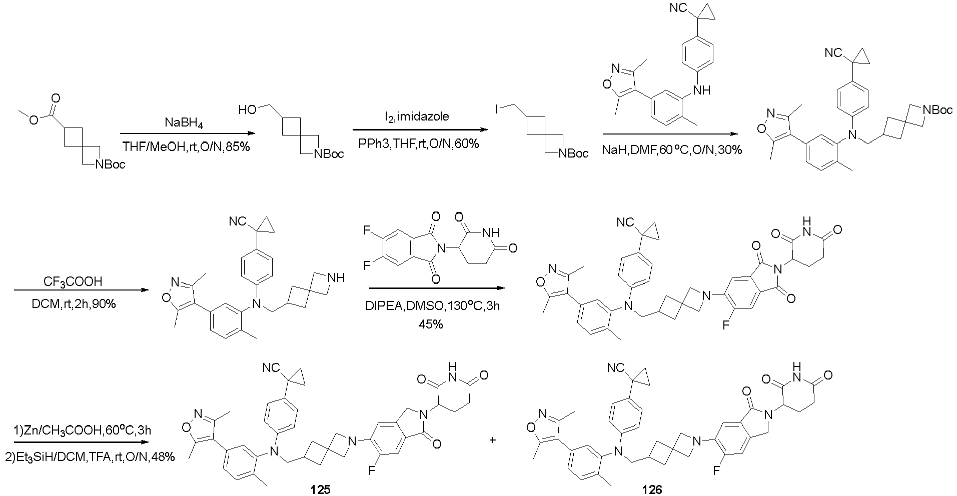

Compound 2-(t-butyl) 6-methyl 2-azaspiro[3.3]heptane-2,6-dicarboxylate (765 mg, 3.00 mmol) was added to 5 mL of THF, and then 1 mL of MeOH was added to aid the solubility of the compound, followed by addition of NaBH₄ in an ice bath. After addition, the ice bath was removed, and the mixture was stirred overnight at room temperature. The reaction solution was diluted with DCM. The organic phase was washed with saturated NH₄Cl solution, saturated NaHCO₃ solution, and saturated NaCl solution, and then dried over anhydrous sodium sulfate, and rotatory evaporated to dry, to provide t-butyl 6-(hydroxymethyl)-2-azaspiro[3.3] heptane-2-carboxylate (579 mg, 2.55 mmol), with a yield of 85%. LC/MS (ESI⁺) calcd for: C₁₂H₂₁NO₃ (M + H⁺) *m*/*z,* 228.2; found, 228.2.

Compound t-butyl 6-(hydroxymethyl)-2-azaspiro[3.3]heptane-2-carboxylate (579 mg, 2.50 mmol), PPh3 (786 mg, 3.00 mmol) and imidazole (204 mg, 3.00 mmol) were added to 5 mL of THF, and then the solution of I₂ (761 mg, 3.00 mmol) in THF was added in an ice bath. After addition, the ice bath was removed, and the mixture was allowed to react overnight at room temperature. The reaction was quenched with saturated NaHSO₃ solution, and the reaction solution was diluted with a large amount of EA. The organic phase was washed with water and saturated NaCl solution respectively, and then dried over anhydrous sodium sulfate, rotatory evaporated to dry, and purified by column chromatography, to provide t-butyl 6-(iodomethyl)-2--azaspiro[3.3]heptane-2-carboxylate (505 mg, 1.50 mmol), with a yield of 60%. LC/MS (ESI⁺) calcd for: C₁₂H₂₀INO₂ (M + H⁺) *m*/*z,* 338.1; found, 338.1.

t-Butyl 6-((((4-(1-cyanocyclopropyl)phenyl)(5-(3,5-dimethylisoxazol-4-yl)-2-methylphenyl) amino)methyl)-2-azaspiro[3.3]heptane-2-carboxylate, synthesized according to the examples mentioned above, with a yield of 30%. LC/MS (ESI⁺) calcd for: C₃₄H₄₀N₄O₃ (M + H⁺) *m*/*z,* 553.3; found, 497.2.

1-(4-((2-Azaspiro[3.3]heptan-6-yl)methyl)(5-(3,5-dimethylisoxazol-4-yl)-2-methylphenyl)amin o)phenyl)cyclopropane-1-carbonitrile, synthesized according to the examples mentioned above, with a yield of 90%. LC/MS (ESI⁺) calcd for: C₂₉H₃₁N₄O (M + H⁺) *m*/*z,* 453.2; found, 453.2. 1-(4-((5-(3,5-Dimethylisoxazol-4-yl)-2-methylphenyl)((2-(2-(2,6-dioxopiperidin-3-yl)-6-fluoro-1,3-dioxoisoindol-5-yl)-2-azaspiro[3.3]heptan-6-yl)methyl)amino)phenyl)cyclopropane-1-carbo nitrile, synthesized according to the examples mentioned above, with a yield of 45%. LC/MS (ESI⁺) calcd for: C₄₂H₃₉FN₆O₅ (M + H⁺) *m*/*z,* 727.3; found, 727.3.

1-(4-((5-(3,5-dimethylisoxazol-4-yl)-2-methylphenyl)((2-(2-(2,6-dioxopiperidin-3-yl)-6-fl uoro-1-oxoisoindolin-5-yl)-2-azaspiro[3.3]heptan-6-yl)methyl)amino)phenyl)cyclopropane-1-c arbonitrile, synthesized according to the examples mentioned above, with a total yield of 48%. LC/MS (ESI⁺) calcd for: C₄₂H₄₁FN₆O₄ (M + H⁺) *m*/*z,* 713.3; found, 713.3. ¹H NMR (400 MHz, CDCl₃) *δ* 7.96 (s, 1H), 7.79 - 7.66 (m, 1H), 7.53 (d, *J* = 9.1 Hz, 1H), 7.39 (d, *J=* 7.5 Hz, 1H), 7.26 - 7.19 (m, 1H), 7.17 - 7.05 (m, 2H), 7.04 - 6.93 (m, 1H), 6.47 (d, *J=* 8.7 Hz, 1H), 5.30 - 5.03 (m, 2H), 4.50 (d, *J* = 16.2 Hz, 1H), 4.36 (s, 1H), 4.32 (s, 1H), 4.07 (s, 1H), 3.94 (s, 1H), 3.75 - 3.57 (m, 2H), 3.40 (s, 1H), 3.03 - 2.77 (m, 3H), 2.55 (d, *J=* 3.0 Hz, 2H), 2.47 - 2.33 (s, 3H), 2.32 - 2.21 (s, 3H), 2.10 (s, 3H), 1.64 (m, 5H), 1.44 (d, *J=* 5.0 Hz, 1H), 0.96 (m, 2H).

### 126: 1-(4-((5-(3,5-dimethylisoxazol-4-yl)-2-methylphenyl)((2-(2-(2-,2,6-dioxopiperidin-3-yl)-6-fluoro-3-oxoisoindolin-5-yl)-2-azaspiro[3.3]heptan-6-yl)methyl)amino)phenyl)cyclop ropane-1-carbonitrile (126)

Synthesized according to the examples mentioned above, with a total yield of 48%. LC/MS (ESI⁺) calcd for: C₄₂H₄₁FN₆O₄ (M + H⁺) *m*/*z,* 713.3; found, 713.3. ¹H NMR (400 MHz, CDCl₃) *δ* 7.99 (s, 1H), 7.48 - 7.42 (m, 1H), 7.39 (d, *J=* 8.2 Hz, 1H), 7.14 (t, *J=* 8.5 Hz, 2H), 7.05 - 6.99 (m, 1H), 6.50 - 6.43 (m, 2H), 5.39 - 5.10 (m, 2H), 4.37 (dd, *J* = 15.6, 7.3 Hz, 1H), 4.26 - 4.05 (m, 4H), 3.97 (s, 1H), 3.70 (dd, *J* = 18.1, 7.2 Hz, 2H), 3.50 (d, *J* = 58.6 Hz, 2H), 2.91 (s, 3H), 2.42 (d, *J* = 6.7 Hz, 3H), 2.29 (d, *J* = 6.1 Hz, 3H), 2.12 (d, *J* = 2.2 Hz, 3H), 2.07 (s, 1H), 1.94 (t, *J=* 10.6 Hz, 2H), 1.63 (q, *J=* 4.8 Hz, 3H), 1.44 (d, *J=* 5.0 Hz, 1H), 0.90 (t, *J=* 6.7 Hz, 2H).

### 127: 1-(4-((5-(3,5-dimethylisoxazol-4-yl)-2-methylphenyl)((1-(2-(2,6-dioxopiperidin-3-yl)-6-fluoro-3-oxoisoindolin-5-yl)piperidin-4-yl)methyl)amino)phenyl)cyclopropane-1-ni trile (127)

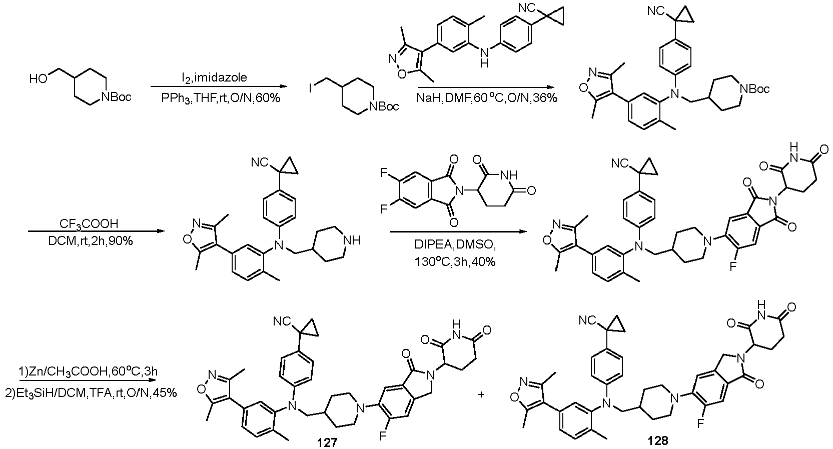

t-Butyl 4-(iodomethyl)piperidine-1-carboxylate, synthesized according to the examples mentioned above, with a yield of 45%. LC/MS (ESI⁺) calcd for: C₁₁H₁₉INO₂ (M + H⁺) *m*/*z,* 326.1; found, 326.1. t-Butyl 4-(((4-(1-cyanocyclopropyl)phenyl)(5-(3,5-dimethylisoxazol-4-yl)-2-methylphenyl) amino)methyl)piperidine-1-carboxylate, synthesized according to the examples mentioned above, with a yield of 36%. LC/MS (ESI⁺) calcd for: C₃₃H₄₀N₄O₃ (M+ H⁺) *m*/*z,* 541.3; found, 541.3.

1-(4-((5-(3,5-dimethylisoxazol-4-yl)-2-methylphenyl)(piperidin-4-ylmethyl)amino)phenyl)cycl opropane-1-nitrile, synthesized according to the examples mentioned above, with a yield of 90%. LC/MS (ESI⁺) calcd for: C₂₈H₃₂N₄O (M + H⁺) *m*/*z,* 441.2; found, 441.2. 1-(4-((5-(3,5-Dimethylisoxazol-4-yl)-2-methylphenyl)((1-(2-(2,6-dioxopiperidin-3-yl)-6-fluoro-1,3-dioxoisoindol-5-yl)piperidin-4-yl)methyl)amino)phenyl)cyclopropane-1-nitrile, synthesized according to the examples mentioned above, with a yield of 40%. LC/MS (ESI⁺) calcd for: C₄₁H₃₉FN₆O₅ (M + H⁺) *m*/*z,* 715.3; found, 715.3.

1-(4-((5-(3,5-Dimethylisoxazol-4-yl)-2-methylphenyl)((1-(2-(2,6-dioxopiperidin-3-yl)-6-fluoro-3-oxoisoindol-5-yl)piperidin-4-yl)methyl)amino)phenyl)cyclopropane-1-nitrile, synthesized according to the examples mentioned above, with a total yield of 45%. LC/MS (ESI⁺) calcd for: C₄₁H₄₁FN₆O₄ (M + H⁺) *m*/*z,* 701.3; found, 701.3. ¹H NMR (400 MHz, CDCl₃) *δ* 7.97 (s, 1H), 7.54 (s, 1H), 7.40 (d, *J=* 7.8 Hz, 2H), 7.15 (t, *J=* 6.9 Hz, 3H), 7.09 (s, 1H), 6.53 (d, *J=* 8.6 Hz, 2H), 5.21 (d, *J* = 12.1 Hz,2H), 4.38 (d, *J* = 47.2 Hz, 2H), 4.33 (s, 2H), 3.63 (d, *J* = 6.3 Hz, 5H), 2.45 (s, 3H), 2.31 (s, 3H), 2.13 (s, 3H), 2.00 (d, *J=* 12.1 Hz, 4H), 1.44 (d, *J=* 5.1 Hz, 2H), 0.95 - 0.80 (m, 5H).

### 128: 1-(4-((5-(3,5-dimethylisoxazol-4-yl)-2-methylphenyl)((1-(2-(2,6-dioxopiperidin-3-yl)-6-fluoro-1-oxoisoindolin-5-yl)piperidin-4-yl)methyl)amino)phenyl)cyclopropane-1-nitrile (128)

Synthesized according to the examples mentioned above, with a total yield of 45%. LC/MS (ESI⁺) calcd for: C₄₁H₄₁FN₆O₄ (M + H⁺) *m*/*z,* 701.3; found, 701.3. ¹H NMR (400 MHz, CDCl₃) *δ* 7.98 (s, 1H), 7.60 - 7.49 (m, 2H), 7.40 (d, *J* = 7.8 Hz, 2H), 7.21 - 7.12 (m, 2H), 7.09 (d, *J* = 1.8 Hz, 1H), 6.52 (d, *J* = 8.6 Hz, 2H), 5.26 - 5.08 (m, 2H), 4.43 (d, *J* = 15.8 Hz, 2H), 4.29 (d, *J* = 15.9 Hz, 2H), 3.61 (d, *J* = 6.9 Hz, 5H), 2.45 (s, 3H), 2.31 (s, 3H), 2.12 (s, 3H), 1.99 (d, *J* = 12.4 Hz, 4H), 1.45 (s, 2H), 0.90 (m, 5H).

### 129: 1-(4-((5-(3,5-Dimethylisoxazol-4-yl)-2-methylphenyl)(2-(1-(2-(2,6-dioxopiperidin-3-yl)-6-fluoro-1,3-dioxoisoindolin-5-yl)azetidin-3-yl)ethyl)amino)phenyl)cyclopropane-1-c arbonitrile (129)

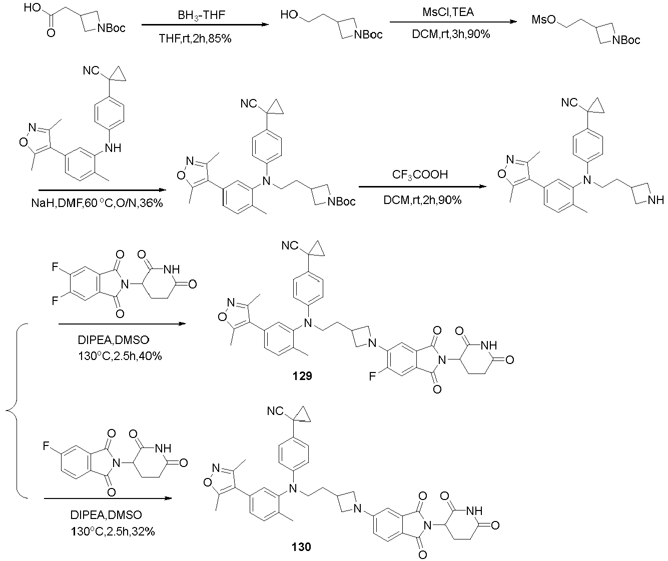

Compound 2-(1-(t-butoxycarbonyl)azetidin-3-yl)acetic acid (636 mg, 3.00 mmol) was added to 4 mL of THF, to which was slowly added 1 mol/L solution of borane in tetrahydrofuran (9 mL, 9.00 mmol) dropwise in an ice bath. After addition, the ice bath was removed, and the solution was stirred at room temperature for 2 h. Water was slowly added dropwise to the reaction solution to quench the reaction, and then the reaction solution was diluted with EA. The organic phase was washed with water and saturated NaCl solution, and then dried over anhydrous sodium sulfate, rotatory evaporated to dry, to provide t-butyl 3-(2-hydroxyethyl)azetidine-1-carboxylic acid (513 mg, 2.55 mmol), with a yield of 85%. LC/MS (ESI⁺) calcd for: C₁₀H₁₉NO₃ (M + H⁺) *m*/*z,* 202.1; found, 202.1.

Compound t-butyl 3-(2-hydroxyethyl)azetidine-1-carboxylate (402 mg, 2.00 mmol) was added to 10 mL of DCM, to which was slowly added MsCl (343 mg, 3.00 mmol) dropwise in an ice bath. After addition, the ice bath was removed, and the solution was stirred at room temperature for 3 h. The reaction solution was diluted with DCM. The organic phase was washed with saturated citric acid solution, saturated NaHCO₃ solution, and saturated NaCl solution respectively, and then dried over anhydrous sodium sulfate, rotatory evaporated to dry, to provide t-butyl 3-(2-(((methylsulfonyl)oxy)ethyl)azetidine-1-carboxylate (502 mg, 1.80 mmol), with a yield of 90%. LC/MS (ESI⁺) calcd for : C₁₁H₂₁NO₅S (M + H⁺) *m*/*z,* 280.2; found, 280.2. t-Butyl 3-(2-(((4-(1-cyanocyclopropyl)phenyl)(5-(3,5-dimethylisoxazol-4-yl)-2-methylphenyl) amino)ethyl)azetidine-1-carboxylate, synthesized according to the examples mentioned above, with a yield of 36%. LC/MS (ESI⁺) calcd for : C₃₂H₃₈N₄O₃ (M + H⁺) *m*/*z,* 527.2; found, 427.2. 1-(4-((2-(Azetidin-3-yl)ethyl)(5-(3,5-dimethylisoxazol-4-yl)-2-methylphenyl)amino)phenyl)cyc lopropane-1-nitrile, synthesized according to the examples mentioned above, with a yield of 90%. LC/MS (ESI⁺) calcd for: C₂₇H₃₀N₄O (M + H⁺) *m*/*z,* 427.2; found, 427.2. 1-(4-((5-(3,5-Dimethylisoxazol-4-yl)-2-methylphenyl)(2-(1-(2-(2,6-dioxopiperidin-3-yl)-6-fluor o-1,3-dioxoisoindol-5-yl)azetidin-3-yl)ethyl)amino)phenyl)cyclopropane-1-carbonitrile, synthesized according to the examples mentioned above, with a yield of 40%. LC/MS (ESI⁺) calcd for: C₄₀H₃₇FN₆O₅ (M + H⁺) *m*/*z,* 701.2; found, 701.2. ¹H NMR (400 MHz, CDCl₃) *δ* 7.97 (s, 1H), 7.45 - 7.35 (m, 2H), 7.20 - 7.11 (m, 3H), 7.04 (d, *J=* 1.8 Hz, 1H), 6.81 (d, *J=* 7.5 Hz, 1H), 6.49 (d, *J* = 8.7 Hz, 2H), 4.93 (dd, *J* = 12.2, 5.3 Hz, 1H), 4.32 (t, *J* = 8.0 Hz, 2H), 4.14 (q, *J* = 7.1 Hz, 1H), 3.85 (t, *J* = 5.4 Hz, 2H), 3.62 (t, *J* = 7.8 Hz, 2H), 2.97 - 2.68 (m, 5H), 2.43 (s, 3H), 2.29 (s, 3H), 2.16 (s, 3H), 2.11 (d, *J* = 8.1 Hz, 2H), 2.07 (s, 1H), 0.90 (t, *J* = 8.9 Hz, 2H).

### 130: 1-(4-((5-(3,5-dimethylisoxazol-4-yl)-2-methylphenyl)(2-(1-(2-(2,6-dioxopiperidin-3-yl)-6-fluoro-1,3-dioxoisoindolin-5-yl)azetidin-3-yl)ethyl)amino)phenyl)cyclopropane-1-car bonitrile (130)

Synthesized according to the examples mentioned above, with a yield of 32%. LC/MS (ESI⁺) calcd for: C₄₀H₃₈N₆O₅ (M + H⁺) *m*/*z,* 683.2; found, 683.2. ¹H NMR (400 MHz, DMSO-*d₆*) *δ* 11.08 (s, 1H), 7.63 (d, *J* = 8.2 Hz, 1H), 7.46 (d, *J* = 7.9 Hz, 1H), 7.28 (s, 1H), 7.18 (s, 1H), 7.12 (d, *J* = 8.4 Hz, 2H), 6.75 (d, *J* = 2.1 Hz, 1H), 6.61 (dd, *J* = 8.1, 2.1 Hz, 1H), 6.49 (d, *J* = 8.4 Hz, 2H), 5.05 (dd, *J* = 12.9, 5.4 Hz, 1H), 4.12 (t, *J* = 8.2 Hz, 2H), 3.66 (dt, *J* = 44.0, 6.9 Hz, 4H), 2.85 (dd, *J* = 13.5, 5.8 Hz, 2H), 2.39 (s, 3H), 2.22 (s, 3H), 2.08 (s, 3H), 1.98 (d, *J* = 8.9 Hz, 3H), 1.60 (q, *J* = 4.8 Hz, 2H), 1.44 - 1.04 (m, 4H).

### 131: 2-Chloro-4-((5-(3,5-dimethylisoxazol-4-yl)-2-methylphenyl)(((1r,4r)-4-(((2-(2,6-dioxopiperidin-3-yl)-6-fluoro-1,3-dioxoisoindolin-5-yl)amino)methyl)cyclohexyl)methyl)a mino)benzonitrile (131)

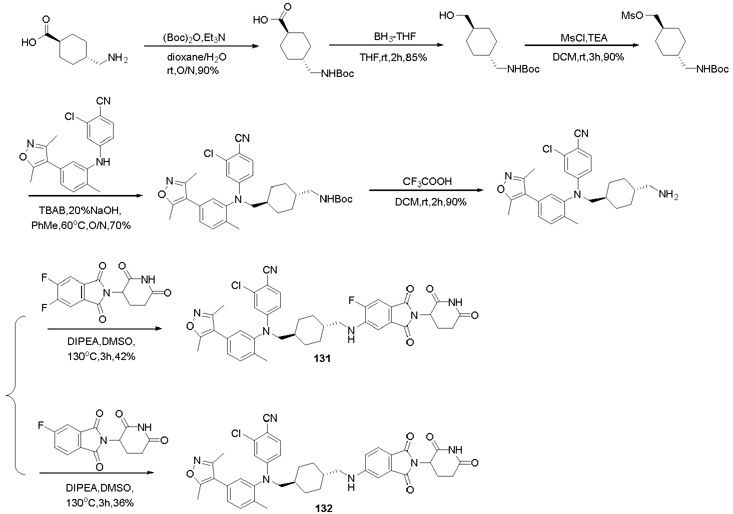

Compound (1r,4r)-4-(aminomethyl)cyclohexane-1-carboxylic acid (549 mg, 3.50 mmol) was added to 10 mL mixed solution of dioxane and water (*v*/*v*, 1:1), to which was added Et₃N (531 mg, 5.20 mmol) in an ice bath, and finally (Boc)₂O (840 mg, 3.80 mmol) was slowly added. After addition, the ice bath was removed, and the reaction solution was stirred overnight at room temperature. The reaction solution was diluted with EA. The organic phase was washed twice with 2 N HCl solution, washed with saturated NaCl solution, dried over anhydrous sodium sulfate, and rotatory evaporated to dry, to provide (1*r*,4*r*)-4-(((t-butoxycarbonyl)amino)methyl)cyclohexane-1-carboxylic acid (810 mg, 3.15 mmol), with a yield of 90%. LC/MS (ESI⁺) calcd for: C₁₃H₂₃NO₄ (M + H⁺) *m*/*z,* 258.3; found, 258.3.

t-Butyl (((1*r*,4*r*)-4-(hydroxymethyl)cyclohexyl)methyl)aminoformate, synthesized according to the examples mentioned above, with a yield of 85%. LC/MS (ESI⁺) calcd for : C₁₃H₂₅NO₃ (M + H⁺) *m*/*z,* 244.3; found, 188.3.

Methyl ((1*r*,4*r*)-4-(((t-Butoxycarbonyl)amino)methyl)cyclohexyl)methanesulfonate, synthesized according to the examples mentioned above, with a yield of 90%. LC/MS (ESI⁺) calcd for: C₁₄H₂₇NO₅S (M + H⁺) *m*/*z,* 322.2; found, 322.2.

Compound 2-chloro-4-((5-(3,5-dimethylisoxazol-4-yl)-2-methylphenyl)amino)benzonitrile (337 mg, 1.00 mmol) and TBAB (322 mg, 1.00 mmol) were added to 5 mL of toluene, and the mixture was stirred to mix the materials thoroughly, to which was added 5 mL of 20% NaOH solution, and then methyl ((1r,4r)-4-(((t-butoxycarbonyl)amino)methyl)cyclohexyl) methanesulfonate (321 mg, 1.00 mmol) was added. The reaction solution was heated to 60 °C and stirred overnight. The solution was cooled to room temperature, and the layers were separated. The organic phase was diluted with EA, washed with saturated NaCl solution, dried over anhydrous sodium sulfate, rotatory evaporated to dry, and purified by column chromatography, to provide compound t-butyl (((1r,4r)-4-(((3-chloro-4-cyanophenyl)(5-(3,5-dimethylisoxazol-4-yl)-2-methylphenyl)amino)m ethyl)cyclohexyl)methyl)aminoformate (394 mg, 0.70 mmol), with a yield of 70%. LC/MS (ESI⁺) calcd for: C₃₂H₃₉CIN₄O₃ (M + H⁺) *m*/*z,* 563.2; found, 507.2.

4-((((1r,4r)-4-(Aminomethyl)cyclohexyl)methyl)(5-(3,5-dimethylisoxazol-4-yl)-2-methylphenyl )amino)-2-chlorobenzonitrile, synthesized according to the examples mentioned above, with a yield of 90%. LC/MS (ESI⁺) calcd for: C₂₇H₃₁ClN₄O (M + H⁺) *m*/*z,* 462.2; found, 462.2. 2-Chloro-4-((5-(3,5-dimethylisoxazol-4-yl)-2-methylphenyl)(((1r,4r)-4-(((2-(2,6-dioxopiperidi n-3-yl)-6-fluoro-1,3-dioxoisoindol-5-yl)amino)methyl)cyclohexyl)methyl)amino)benzonitrile, synthesized according to the examples mentioned above, with a yield of 42%. LC/MS (ESI⁺) calcd for: C₄oH₃₈ClFN₆O₅ (M + H⁺) *m*/*z,* 737.2; found, 737.2. ¹H NMR (400 MHz, CDCl₃) *δ* 8.01 (s, 1H), 7.48 -7.36 (m, 3H), 7.26 - 7.20 (m, 1H), 7.10 - 7.03 (m, 2H), 6.53 (s, 1H), 6.38 (d, *J* = 8.9 Hz, 1H), 4.14 (q, *J* = 7.1 Hz, 1H), 3.17 (d, *J* = 6.8 Hz, 2H), 2.98 - 2.64 (m, 4H), 2.45 (s, 3H), 2.31 (s, 3H), 2.13 (s, 3H), 1.94 (s, 4H),1.35 - 1.16 (m, 4H), 1.07 (q, *J=* 10.7 Hz, 4H), 0.89 (d, *J* = 11.2 Hz, 1H).

### 132: 2-Chloro-4-((5-(3,5-dimethylisoxazol-4-yl)-2-methylphenyl)(((1r,4r)-4-(((2-(2,6-dioxopiperidin-3-yl)-1,3-dioxoisoindolin-5-yl)amino)methyl)cyclohexyl)methyl)amino)ben zonitrile (132)

The title compound was synthesized according to the example mentioned above, with a yield of 42%. LC/MS (ESI⁺) calcd for: C₄₀H₃₉ClN₆O₅ (M + H⁺) *m*/*z,* 719.2; found, 719.2. ¹H NMR (400 MHz, CDCl₃) *δ* 8.02 (s, 1H), 7.63 (d, *J=* 8.2 Hz, 1H), 7.45 (d, *J=* 7.9 Hz, 1H), 7.39 (d, *J=* 8.8 Hz, 1H), 7.23 (dd, *J* = 7.8, 1.8 Hz, 1H), 7.07 - 6.98 (m, 2H), 6.78 (d, *J* = 8.3 Hz, 1H), 6.53 (s, 1H), 6.38 (d, *J=* 8.9 Hz, 1H),4.95 (dd, *J=* 12.1, 5.2 Hz, 1H), 4.14 (q, *J=* 7.1 Hz, 1H), 3.12 (d, *J* = 6.6 Hz, 2H), 2.45 (s, 3H), 2.31 (s, 3H), 2.12 (s, 3H), 2.07 (s, 1H), 1.64 (s, 2H), 1.34 - 1.24 (m, 5H), 1.06 (q, *J=* 11.2 Hz, 5H), 0.89 (d, *J=* 10.6 Hz, 2H).

### 133: 2-Chloro-4-((5-(3,5-dimethylisoxazol-4-yl)-2-methylphenyl)((1r,4r)-4-((2-(2,6-dioxopiperidin-3-yl)-1-oxoisoindolin-5-yl)amino)methyl)cyclohexyl)methyl)amino)benzoni trile (133)

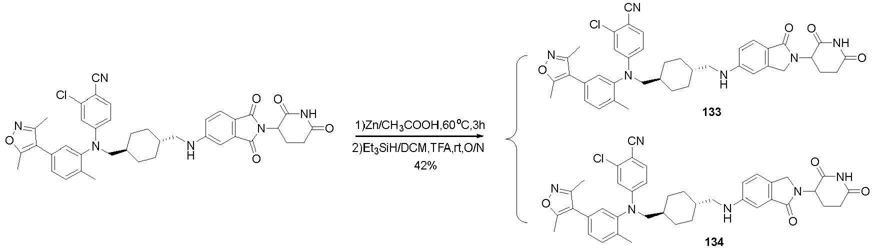

By referring to the method in the above example, the target compound was synthesized according to the above route. LC/MS (ESI⁺) calcd for: C₄₀H₄₁ClN₆O₄ (M + H⁺) *m*/*z,* 705.2; found, 705.2. ¹H NMR (400 MHz, DMSO-*d₆*) *δ* 10.97 (s, 1H), 7.62 - 7.47 (m, 3H), 7.37 (d, *J* = 8.5 Hz, 2H), 7.30 - 7.20 (m, 2H), 6.85 (d, *J* = 8.5 Hz, 1H), 6.76 (s, 1H), 6.67 (s, 1H), 5.08 (ddd, *J* = 19.4, 12.6, 4.7 Hz, 2H), 4.47 (d, *J* = 17.6 Hz, 1H), 4.39 - 4.21 (m, 2H), 4.12 (d, *J* = 16.4 Hz, 1H), 2.89 (q, *J* = 6.1 Hz, 3H), 2.42 (s, 3H), 2.25 (s, 3H), 2.06 (s, 3H), 1.83 (s, 4H), 1.57 (d, *J* = 33.7 Hz, 2H), 1.07 (d, *J* = 11.7 Hz, 3H), 0.97 - 0.80 (m, 3H).

### 134: 2-Chloro-4-((5-(3,5-dimethylisoxazol-4-yl)-2-methylphenyl)((1r,4r)-4-((2-(2,6-dioxopiperidin-3-yl)-3-oxoisoindolin-5-yl)amino)methyl)cyclohexyl)methyl)amino)benzoni trile (134)

Synthesized according to the examples mentioned above, with a total yield of 42%. LC/MS (ESI⁺) calcd for: C₄₀H₄₁ClN₆O₄ (M + H⁺) *m*/*z,* 705.2; found, 705.2. ¹H NMR (400 MHz, DMSO-*d₆*) *δ* 10.91 (s, 1H), 7.57 (d, *J* = 8.9 Hz, 1H), 7.52 (d, *J* = 8.0 Hz, 1H), 7.39 - 7.33 (m, 2H), 7.28 (d, *J* = 1.8 Hz, 1H), 6.71 - 6.57 (m, 3H), 6.52 - 6.36 (m, 2H), 5.01 (dd, *J* = 13.3, 5.1 Hz, 1H), 4.25 (d, *J* = 16.8 Hz, 1H), 4.12 (d, *J* = 16.7 Hz, 1H), 3.82 (s, 1H), 2.95 - 2.81 (m, 3H), 2.63 - 2.53 (m, 1H), 2.42 (s, 3H), 2.32 (dd, *J* = 13.2, 4.6 Hz, 1H), 2.25 (s, 3H), 2.06 (s, 3H), 1.93 (ddd, *J* = 12.3, 6.5, 4.2 Hz, 1H), 1.82 (d, *J* = 10.9 Hz, 4H), 1.57 (d, *J* = 35.9 Hz, 2H), 1.22 (s, 1H), 1.07 (q, *J* = 13.2, 12.1 Hz, 2H), 0.89 (dt, *J* = 20.5, 9.3 Hz, 2H).

### 135: 2-Chloro-4-((5-(3,5-dimethylisoxazol-4-yl)-2-methylphenyl)(((1r,4r)-4-(((2-(2,6-dioxopiperidin-3-yl)-6-fluoro-1-oxoisoindolin-5-yl)amino)methyl)cyclohexyl)methyl)amino )benzonitrile (135)

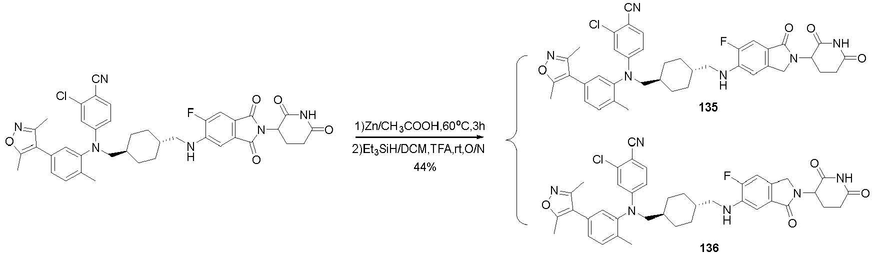

By referring to the method in the above example, the target compound was synthesized according to the above route. LC/MS (ESI⁺) calcd for : C₄₀H₄₀ClFN₆O₄ (M + H⁺) *m*/*z,* 723.2; found, 723.2. ¹H NMR (400 MHz, CDCl₃) *δ* 8.03 (s, 1H), 7.44 (d, *J* = 7.9 Hz, 1H), 7.39 (d, *J* = 8.9 Hz, 1H), 7.23 (dd, *J=* 7.8, 1.8 Hz, 1H), 7.18 (s, 1H), 7.11 - 7.03 (m, 2H), 6.52 (s, 1H), 6.38 (d, *J* = 8.9 Hz, 1H), 5.20 (dd, *J* = 13.2, 4.9 Hz, 1H), 4.37 (d, *J* = 15.4 Hz, 1H), 4.25 (d, *J* = 15.5 Hz, 1H), 3.11 (d, *J=* 6.0 Hz, 2H), 3.00 - 2.81 (m, 3H), 2.45 (s, 3H), 2.31 (s, 3H), 2.25 (d, *J* = 7.1 Hz, 1H), 2.12 (s, 3H), 1.66 (s, 2H), 1.34 - 1.24 (m, 5H), 1.05 (q, *J* = 11.3 Hz, 4H), 0.90 (m, 1H).

### 136: 2-Chloro-4-((5-(3,5-dimethylisoxazol-4-yl)-2-methylphenyl)(((1r,4r)-4-(((2-(2,6-dioxopiperidin-3-yl)-6-fluoro-3-oxoisoindolin-5-yl)amino)methyl)cyclohexyl)methyl)amino )benzonitrile (136)

Synthesized according to the examples mentioned above, with a total yield of 44%. LC/MS (ESI⁺) calcd for: C₄₀H₄₀ClFN₆O₄ (M + H⁺) *m*/*z,* 723.2; found, 723.2. ¹H NMR (400 MHz, CDCl₃) *δ* 8.54 (d, *J=* 5.4 Hz, 1H), 7.40 - 7.25 (m, 3H), 7.14 (dd, *J=* 7.9, 1.8 Hz, 1H), 6.96 (d, *J* = 1.9 Hz, 1H), 6.53 (d, *J* = 7.0 Hz, 1H), 6.44 (d, *J* = 2.4 Hz, 1H), 6.33 - 6.24 (m, 1H), 5.08 (dd, *J* = 13.2, 4.9 Hz, 1H), 4.41 (s, 1H), 4.27 (d, *J* = 15.5 Hz, 1H), 4.14 (d, *J* = 15.5 Hz, 1H), 3.05 - 2.92 (m, 2H), 2.84 - 2.67 (m, 2H), 2.35 (s, 3H), 2.21 (s, 3H), 2.14 - 2.06 (m, 1H), 2.03 (s, 3H), 1.85 (d, *J* = 10.6 Hz, 4H), 1.62 (d, *J* = 53.2 Hz, 2H), 1.19 (d, *J* = 12.3 Hz, 2H), 0.97 (q, *J* = 12.3 Hz, 4H).

### 137: 1-(4-((5-(3,5-Dimethylisoxazol-4-yl)-2-methylphenyl)((1-(((1-(2-(2,6-dioxopiperidin-3-yl)-1,3-dioxoisoindolin-5-yl)azetidin-3-yl)oxy)methyl)cyclopropyl)methyl)amino)phenyl)cy clopropane-1-nitrile

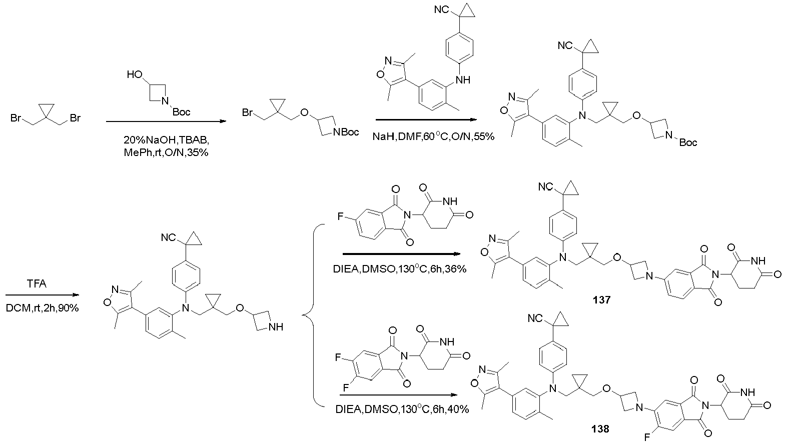

t-Butyl 3-((1-(bromomethyl)cyclopropyl)methoxy)azetidine-1-carboxylate, synthesized according to the examples mentioned above, with a yield of 35%.

t-Butyl 3-((1-(((4-(1-cyanocyclopropyl)phenyl)(5 -(3,5-dimethylisoxazol-4-yl)-2-methyl phenyl)amino)methyl)cyclopropyl)methoxy)azetidine-1-carboxylate, synthesized according to the examples mentioned above, with a yield of 55%. LC/MS (ESI⁺) calcd for: C₃₅H₄₂N₄O₄ (M + H⁺) *m*/*z,* 583.3; found, 583.3.

1-(4-(((1-((Azetidin-3-yloxy)methyl)cyclopropyl)methyl)(5-(3,5-dimethylisoxazol-4-yl)-2-meth ylphenyl)amino)phenyl)cyclopropane-nitrile, synthesized according to the examples mentioned above, with a yield of 90%. LC/MS (ESI⁺) calcd for: C₃₀H₃₄N₄O₂ (M + H⁺) *m*/*z,* 483.3; found, 483.3.

1-(4-((5-(3,5-Dimethylisoxazol-4-yl)-2-methylphenyl)((1-(((1-(2-(2,6-dioxopiperidin-3-yl)-1,3-dioxoisoindolin-5-yl)azetidin-3-yl)oxy)methyl)cyclopropyl)methyl)amino)phenyl)cyclopropane -1-nitrile, synthesized according to the examples mentioned above, with a yield of 36%. LC/MS (ESI⁺) calcd for: C₄₃H₄₂N₆O₆ (M + H⁺) *m*/*z,* 739.3; found, 739.3. ¹H NMR (400 MHz, DMSO-*d₆*) *δ* 11.09 (s, 1H), 7.65 (dd, *J* = 8.2, 3.5 Hz, 1H), 7.44 (d, *J* = 7.9 Hz, 1H), 7.29 - 7.20 (m, 2H), 7.00 (d, *J* = 8.8 Hz, 2H), 6.77 (d, *J* = 2.1 Hz, 1H), 6.64 (dd, *J* = 8.4, 2.1 Hz, 1H), 6.59 - 6.53 (m, 2H), 5.07 (dd, *J* = 12.9, 5.3 Hz, 1H), 4.46 - 4.36 (m, 1H), 4.22 (dd, *J* = 9.4, 6.3 Hz, 2H), 3.84 - 3.70 (m, 4H), 3.00 (s, 1H), 2.91 - 2.82 (m, 1H), 2.56 (d, *J* = 6.0 Hz, 2H), 2.37 (s, 3H), 2.20 (s, 3H), 2.06 (s, 3H), 1.53 (t, *J* = 3.7 Hz, 2H), 1.25 - 1.18 (m, 4H), 0.38 (d, *J* = 4.7 Hz, 2H), 0.24 (s, 2H).

### 138: 1-(4-((5-(3,5-dimethylisoxazol-4-yl)-2-methylphenyl)((1-(((1-(2-(2,6-dioxopiperidin-3-yl)-6-fluoro-1,3-dioxoisoindolin-5-yl)azetidin-3-yl)oxy)methyl)cyclopropyl)methyl)amino )phenyl)cyclopropane-1-nitrile

Synthesized according to the examples mentioned above, with a yield of 40%. LC/MS (ESI⁺) calcd for: C₄₃H₄₁FN₆O₆ (M + H⁺) *m*/*z,* 757.3; found, 757.3. ¹H NMR (400 MHz, DMSO-*d₆*) *δ* 11.11 (s, 1H), 7.61 (d, *J* = 11.1 Hz, 1H), 7.43 (d, *J* = 7.8 Hz, 1H), 7.28 - 7.17 (m, 2H), 7.06 - 6.97 (m, 2H), 6.90 (d, *J* = 7.6 Hz, 1H), 6.61 - 6.49 (m, 2H), 5.08 (dd, *J* = 12.8, 5.4 Hz, 1H), 4.44 - 4.18 (m, 4H), 3.91 (d, *J=* 8.6 Hz, 2H), 3.75 (s, 2H), 2.89 (s, 2H), 2.73 (s, 2H), 2.64 - 2.55 (m, 1H), 2.37 (s, 3H), 2.20 (s, 3H), 2.06 (s, 3H), 1.23 (t, *J* = 3.8 Hz, 4H), 0.38 (d, *J* = 4.6 Hz, 2H), 0.24 (d, *J* = 5.4 Hz, 2H).

### 139: 1-(4-((5-(3,5-dimethylisoxazol-4-yl)-2-methylphenyl)((1-(1-(2-(2,6-dioxopiperidin-3-yl)-6-fluoro-1,3-dioxoisoindolin-5-yl)piperidin-4-yl)azetidin-3-yl)methyl)amino)phenyl)cyc lopropane-1-carbonitrile

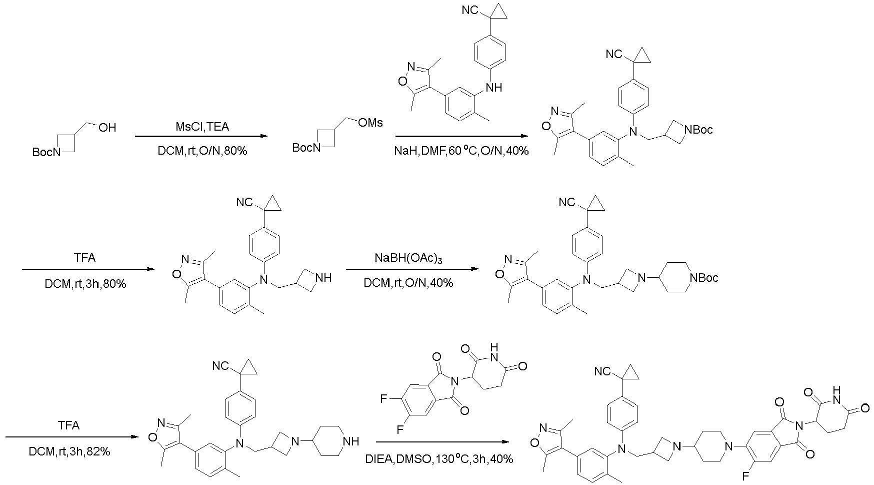

By referring to the method in the above example, the target compound was synthesized according to the above route, with a yield of 40%. LC/MS (ESI⁺) calcd for: C₄₄H₄₄FN₇O₅ (M + H⁺) *m*/*z,* 770.3; found, 770.3.

### 140: 1-(4-((5-(3,5-Dimethylisoxazol-4-yl)-2-methylphenyl)(3-((1-(2-(2,6-dioxopiperidin-3-yl)-1,3-dioxoisoindolin-5-yl)-3-methylazetidin-3-yl)oxy)propyl)amino)phenyl)cyclopropa ne-1-carbonate (140)

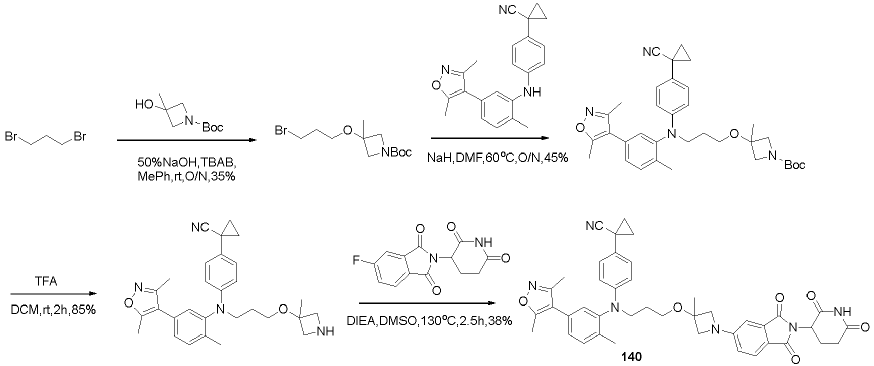

By referring to the method in the above example, the target compound was synthesized according to the above route, with a yield of 38%. LC/MS (ESI⁺) calcd for: C₄₂H₄₂N₆O₆ (M + H⁺) *m*/*z,* 727.3; found, 727.3. ¹H NMR (400 MHz, DMSO-*d₆*) *δ* 11.09 (s, 1H), 7.65 (d, *J* = 8.3 Hz, 1H), 7.44 (d, *J* = 7.9 Hz, 1H), 7.26 (d, *J* = 7.9 Hz, 1H), 7.17 (s, 1H), 7.07 (d, *J* = 8.3 Hz, 2H), 6.76 (s, 1H), 6.64 (d, *J* = 8.3 Hz, 1H), 6.48 (d, *J* = 8.3 Hz, 2H), 5.07 (dd, *J* = 13.1, 5.4 Hz, 1H), 3.83 (m, 4H), 3.73 (t, *J=* 7.2 Hz, 2H), 3.45 (t, *J=* 5.8 Hz, 2H), 2.36 (s, 3H), 2.19 (s, 3H), 2.06 (s, 3H), 1.85 (t, *J* = 6.6 Hz, 2H), 1.57 (q, *J=* 4.8 Hz, 2H), 1.46 (s, 3H), 1.31 - 1.18 (m, 6H).

### 141: (2S,4R)-1-((S)-2-(2-(4-(3-((3-bromo-4-(1H-imidazol-1-yl)phenyl)(5-(3,5-dimethylisoxazol-4-yl)-2-methylphenyl)amino)propyl)piperidin-1-yl)acetamido)-3,3-dimet hylbutyryl)-4-hydroxy-N-((S)-1-(4-(4-methylthiazol-5-yl)phenyl)ethyl)tetrahydropyrrole-2 -amide (141)

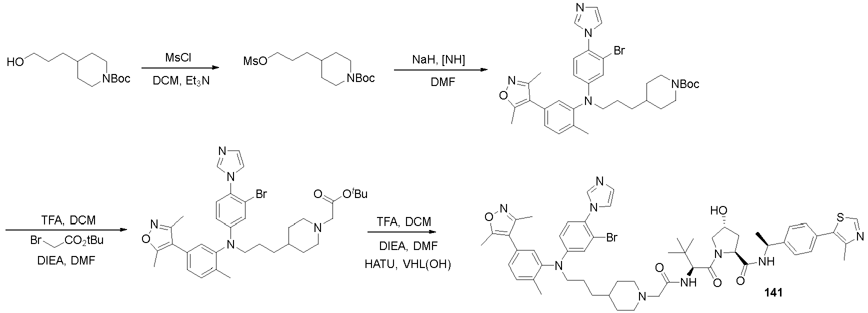

N-Boc-4-(3-hydroxypropyl)-piperidine was dissolved in dichloromethane, to which was added triethylamine, and then the system was cooled to 0 °C. Methylsulfonyl chloride was slowly added, and after addition, the reaction solution was warmed to room temperature and stirred for 1 h. After the reaction was completed, the reaction was quenched with 0.5 N dilute hydrochloric acid. The resultant solution was extracted with dichloromethane, successively washed with saturated NaHCO₃ solution, water, and saturated NaCl solution, and then dried, concentrated, and purified by column chromatography, to provide the product (3.4 g, yield 87%). LC/MS (ESI⁺) calcd for C₁₄H₂₈NO₅S ([M + H]⁺) *m*/*z* 322.2; found 322.2.

*N*-(3-Bromo-4-(1H-imidazol-1-yl)phenyl)-5-(3,5-dimethylisoxazol-4-yl)-2-methylaniline was dissolved in DMF, and the solution was cooled to 0 °C in an ice bath, to which was then added NaH. After addition, the reaction solution was heated to room temperature and stirred for 0.5 h. Liquid seal was used to observe whether the system was still bubbling. If no bubbles were generated, the intermediate t-butyl 4-(3-((methylsulfonyl)oxy)propyl)piperidin-1-ylcarboxylate, obtained in the previous step, was added, and the reaction solution was stirred at room temperature overnight. After completion of the reaction, the reaction was quenched with 0.5 N dilute hydrochloric acid. The resultant solution was extracted with ethyl acetate, successively washed with saturated NaHCO₃ solution, water, and saturated NaCl solution, and then dried, concentrated, and purified by column chromatography, to provide the product (477 mg, yield 56%). LC/MS (ESI⁺) calcd for C₃₄H₄₃BrN₅O₅ ([M + H]⁺) *m*/*z* 648.3; found 648.3.

t-Butyl 4-(3 -((3 -bromo-4-(1*H*-imidazol-1-yl)phenyl)(5-(3,5-dimethylisoxazol-4-yl)-2-methylphenyl)amino)propyl)piperidin-1-ylcarboxylate was dissolved in DCM, to which was added trifluoroacetic acid, and the solution was stirred for 6 h. After completion of the reaction, the reaction solution was concentrated several times. The obtained concentrate was dissolved in DMF, and then DIEA and t-butyl bromoacetate were added. The mixture was stirred at room temperature overnight. After completion of the reaction, the reaction was quenched with 0.5 N dilute hydrochloric acid. The resultant solution was extracted with ethyl acetate, successively washed with saturated NaHCO₃ solution, water, and saturated NaCl solution, and then dried, concentrated, and purified by column chromatography, to provide the product (180 mg, yield 77%). LC/MS (ESI⁺) calcd for C₃₅H₄₅BrN₅O₃ ([M + H]⁺) *m*/*z* 622.3; found 622.3.

t-Butyl 2-(4-(3 -((3 -Bromo-4-(1*H*-imidazol-1-yl)phenyl)(5-(3,5-dimethylisoxazol-4-yl)-2-methylphenyl)amino)propyl)piperidin-1-yl)acetate was dissolved in dichloromethane, to which was added trifluoroacetic acid, and the solution was stirred overnight at room temperature. TLC indicated the disappearance of the starting material, and then the reaction solution was concentrated. The concentrated product was dissolved in DMF, to which was added excess DIEA, followed by addition of VHL(OH) and HATU, and the resultant solution was stirred at room temperature for 4 h. After completion of the reaction, the reaction was quenched with 0.5 N dilute hydrochloric acid. The resultant solution was extracted with ethyl acetate, successively washed with saturated NaHCO₃ solution, water, and saturated NaCl solution, and then dried, concentrated, and purified by column chromatography, to provide the product (8 mg, yield 16%).

LC/MS (ESI⁺) calcd for C₅₄H₆₇BrN₉O₅S ([M + H]⁺) *m*/*z* 1034.1; found 1034.1.

¹H NMR (400 MHz, DMSO-*d₆*) *δ* 8.97 (s, 1H), 8.44 (d, *J* = 7.7 Hz, 1H), 7.84 (d, *J* = 8.7 Hz, 1H), 7.44 (d, *J* = 7.9 Hz, 1H), 7.41 (m, 3H), 7.39 (d, *J* = 9.3, 5.8 Hz, 2H), 7.19 (s, 1H), 7.08 (dd, *J* = 8.8, 2.4 Hz, 1H), 6.62 (m, 2H), 6.38 (t, *J* = 5.2 Hz, 1H), 5.19 (s, 1H), 4.88 (m, 1H), 3.88 (m, 4H), 3.78 (dd, *J* = 11.8, 3.9 Hz, 1H), 3.67 (s, 3H), 3.58 (s, 1H), 3.52 (dd, *J* = 15.0, 8.6 Hz, 2H), 2.56 (m, 4H), 2.40 (s, 3H), 2.26 (m, 4H), 2.14 (s, 3H), 2.00 (s, 3H), 1.95 (m, 1H), 1.58 (dd, *J* = 13.8, 7.0 Hz, 4H), 1.44 (m, 5H), 1.33 (m, 3H), 0.94 (s, 9H).

### 142: (3R,5S)-1-((S)-2-(2-(4-(3-((3-Bromo-4-(1H imidazol-1-yl)phenyl)(5-(3,5--4-yl)-2-methylphenyl)amino)propyl)piperidin-1-yl)acetamido)-3,3-dimethylbutyryl)-5-((( S)-1-(4-(4-methylthiazol-5-yl)phenyl)ethyl)carbamoyl)pyrrolidinyl-3-acetate (142)

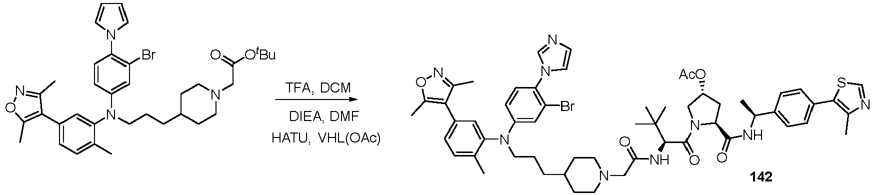

t-Butyl 2-(4-(3 -((3 -bromo-4-(1H-imidazol-1-yl)phenyl)(5-(3,5-dimethyli soxazol-4-yl)-2-methylphenyl)amino)propyl)piperidin-1-yl)acetate obtained above was dissolved in dichloromethane, to which was added trifluoroacetic acid, and the solution was stirred overnight at room temperature. TLC indicated the disappearance of the starting material, and then the reaction solution was concentrated. The concentrated product was dissolved in DMF, to which was added excess DIEA, followed by addition of VHL(OH) and HATU, and the resultant solution was stirred at room temperature for 4 h. After completion of the reaction, the reaction was quenched with 0.5 N dilute hydrochloric acid. The resultant solution was extracted with ethyl acetate, successively washed with saturated NaHCO₃ solution, water, and saturated NaCl solution, and then dried, concentrated, and purified by column chromatography, to provide the product (20 mg, yield 10%). LC/MS (ESI⁺) calcd for C₅₆H₆₉BrN₉O₆S ([M + H]⁺) *m*/*z* 1076.2; found 1076.2. ¹H NMR (400 MHz, DMSO-*d₆*) *δ* 8.97 (s, 1H), 8.41 (d, *J* = 7.7 Hz, 1H), 7.854 (d, *J* = 8.7 Hz, 1H), 7.42 (d, *J* = 7.9 Hz, 1H), 7.40 (m, 3H), 7.39 (d, *J* = 9.3, 5.9 Hz, 2H), 7.22 (s, 1H), 7.08 (dd, *J* = 8.4, 2.4 Hz, 1H), 6.65 (m, 2H), 6.37 (t, *J* = 5.2 Hz, 1H), 4.88 (m, 1H), 3.87 (m, 4H), 3.78 (dd, *J* = 11.8, 3.9 Hz, 1H), 3.67 (s, 3H), 3.58 (s, 1H), 3.52 (dd, *J* = 15.0, 8.6 Hz, 2H), 2.56 (m, 4H), 2.40 (s, 3H), 2.26 (m, 7H), 2.14 (s, 3H), 2.00 (s, 3H), 1.95 (m, 1H), 1.58 (dd, *J* = 13.8, 7.0 Hz, 4H), 1.44 (m, 5H), 1.33 (m, 3H), 0.92 (s, 9H).

### 143: (2S,4R)-1-((S)-2-(2-((5-((4-(1-Cyanocyclopropyl)phenyl)(5-(3,5-dimethylisoxazol-4-yl)-2-methylphenyl)amino)pentyloxy)acetamido)-3,3-dimethylbutyryl)-4-hydroxy-N-((S)-1 -(4-(4-methylthiazol-5-yl)phenyl)ethyl)tetrahydropyrrole-2-amide (143)

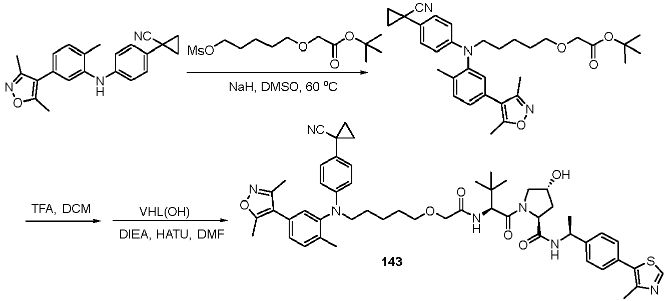

1-(4-(5-(3,5-Dimethylisoxazol-4-yl)-2-methylphenyl)amino)phenylcyclopropanecarbonitrile was dissolved in DMSO, to which was then added NaH. After addition, the reaction solution was stirred at room temperature for 0.5 h. Liquid seal was used to observe whether the system was still bubbling. If no bubbles were generated, t-butyl 2-((5-((methylsulfonyl)pentyloxy)acetate was added, and the reaction solution was stirred overnight at 60 °C. After completion of the reaction, the reaction solution was cooled to room temperature, and then quenched with the saturated solution of ammonium chloride. The resultant solution was extracted with ethyl acetate, successively washed with water and saturated NaCl solution, and then dried, concentrated, and purified by column chromatography, to provide the product (247 mg, yield 29%). LC/MS (ESI⁺) calcd for C₃₃H₄₂N₃O₄ ([M + H]⁺) *m*/*z* 544.3; found 544.3.

t-Butyl 2-((5-((4-(1-1-cyanocyclopropyl)phenyl)(5-(3,5-dimethylisoxazol-4-yl)-2-methylphenyl)amino)pentoxyacetate was dissolved in dichloromethane, to which was added trifluoroacetic acid, and the solution was stirred overnight at room temperature. TLC indicated the disappearance of the starting material, and then the reaction solution was concentrated. The concentrated product was dissolved in DMF, to which was added excess DIEA, followed by addition of VHL(OH) and HATU, and the resultant solution was stirred at room temperature for 4 h. After completion of the reaction, the reaction was quenched with 0.5 N dilute hydrochloric acid. The resultant solution was extracted with ethyl acetate, successively washed with saturated NaHCO₃ solution, water, and saturated NaCl solution, and then dried, concentrated, and purified by column chromatography, to provide the product (24 mg, yield 60%).

LC/MS (ESI⁺) calcd for C₅₂H₆₄N₇O₆S ([M + H]⁺) *m*/*z* 914.5; found 914.5. ¹H NMR (400 MHz, DMSO-*d₆*) *δ* 8.99 (s, 1H), 8.14 (d, *J* = 7.7 Hz, 1H), 7. 48 (d, *J* = 8.7 Hz, 1H), 7.44 (m, 4H), 7.20 (m, 2H), 7.08 (dd, J= 8.8, 2.4 Hz, 1H), 6.64 (m, 2H), 6.31 (d, *J=* 7.9 Hz, 2H), 5.19 (s, 1H), 4.88 (m, 1H), 4.52 (s, 1H), 4.25 (s, 1H), 3.78 (dd, *J* = 11.8, 3.9 Hz, 1H), 3.58 (s, 1H), 3.52 (dd, *J* = 15.0, 8.6 Hz, 2H), 3.07 (m, 2H), 2.40 (s, 3H), 2.26 (m, 6H), 2.14 (s, 3H), 1.95 (m, 1H), 1.58 (dd, *J=* 13.8, 7.0 Hz, 4H), 1.44 (m, 1H), 1.35 (s, 6H), 1.14 (s, 6H), 0.96 (s, 9H).

### 144: 2-((5-((4-(1-1-Cyanocyclopropyl)phenyl)(5-(3,5-dimethylisoxazol-4-yl)-2-methylphenyl)amino)pentyloxy)-N-(2-(2,6-dioxopiperidin-3)-1-isoindolinone-4-yl)-acetami de (144)

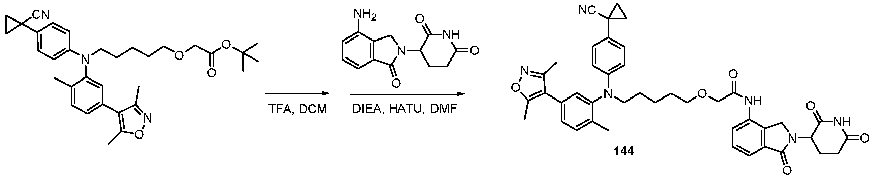

t-Butyl 2-((5-((4-(1-1-cyanocyclopropyl)phenyl)(5-(3,5-dimethyli soxazol-4-yl)-2-methylphenyl) amino)pentoxyacetate was dissolved in dichloromethane, to which was added trifluoroacetic acid, and the solution was stirred overnight at room temperature. TLC indicated the disappearance of the starting material, and then the reaction solution was concentrated. The concentrated product was dissolved in DMF, to which was added excess DIEA, followed by addition of 3-(4-amino-1-isoindolinone-2-yl)hexahydropyridine-2,6-dione and HATU, and the resultant solution was stirred at room temperature for 4 h. After completion of the reaction, the reaction was quenched with 0.5 N dilute hydrochloric acid. The resultant solution was extracted with ethyl acetate, successively washed with saturated NaHCO₃ solution, water, and saturated NaCl solution, and then dried, concentrated, and purified by column chromatography, to provide the product (6 mg, yield 8%). LC/MS (ESI⁺) calcd for C₄₂H₄₅N₆O₆ ([M + H]⁺) *m*/*z* 729.3; found 729.3.

### 145: 1-(4-((5-(3,5-dimethylisoxazol-4-yl)-2-methylphenyl)(5-((2-(2,6-dioxopiperidin-3)-1-isoindolinone-5-yl))amino)pentyl)amino)phenylcyclopropanecarbonitrile (145)

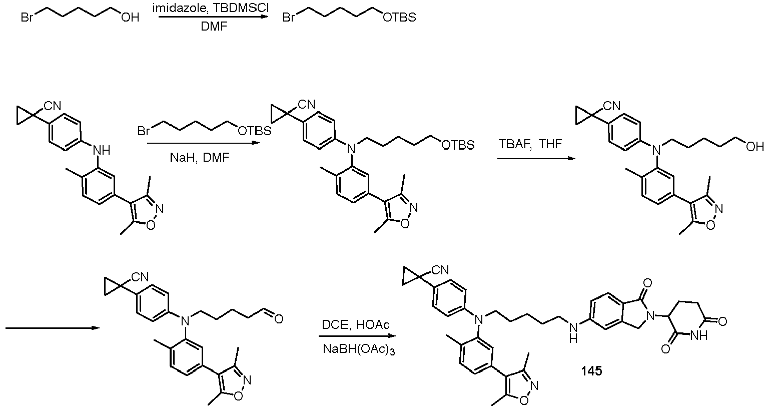

Bromopentanol was dissolved in DMF, to which was added imidazole, and the mixture was cooled to 0 °C in an ice bath, followed by addition of TBDMSCl. The reaction solution was warmed to room temperature, and stirred at room temperature for 6 h. After the reaction was completed, the reaction was quenched with the saturated solution of ammonium chloride, extracted with ethyl acetate, successively washed with water and saturated NaCl solution, dried, concentrated, and purified by column chromatography, to provide the product (1.48 g, yield 89%).

1-(4-(5-(3,5-Dimethylisoxazol-4-yl)-2-methylphenyl)amino)phenylcyclopropanecarbonitrile was dissolved in DMF, and the solution was cooled to 0 °C in an ice bath, to which was then added NaH. After addition, the reaction solution was warmed to room temperature and stirred for 0.5 h. Liquid seal was used to observe whether the system was still bubbling. If no bubbles were generated, (5-bromopentyloxy)(t-butyl)dimethylsilane, obtained in the previous step, was added, and the reaction solution was stirred at room temperature overnight. After completion of the reaction, the reaction was quenched with the saturated solution of ammonium chloride. The resultant solution was extracted with ethyl acetate, successively washed with water and saturated NaCl solution, and then dried, concentrated, and purified by column chromatography, to provide the product (286 mg, yield 75%). LC/MS (ESI⁺) calcd for C₃₃H₄₆N₃O₂Si ([M + H]⁺) *m*/*z* 544.3; found 544.3.

The product 1-(4-(5-t-butyldimethylpentoxysilane)(5-(3,5-dimethylisoxazol-4-yl)-2-methylphenyl)amino)phenylcyclopropanecarbonitrile, obtained in the previous step, was dissolved in tetrahydrofuran, to which was added tetrabutylammonium fluoride trihydrate, and the mixture was stirred at room temperature for 3 h. After the reaction was completed, the reaction solution was concentrated and purified by column chromatography, to provide the product (122 mg, yield 88%). LC/MS (ESI⁺) calcd for C₂₇H₃₂N₃O₂ ([M + H]⁺) *m*/*z* 430.2; found 430.2.

The product 1-(4-((5-(3,5-dimethylisoxazol-4-yl)-2-methylphenyl)(5-hydroxypentyl)amino) phenylcyclopropanecarbonitrile, obtained in the previous step, was dissolved in dichloromethane, to which was added Dess-Martin periodinane, and the mixture was stirred for 6 h at room temperature. After the reaction was completed, the reaction solution was filtered through the pad of celite, and then rinsed with dichloromethane several times. The filtrate was concentrated, and the residue was purified by column chromatography, to provide the product (122 mg, yield 88%). LC/MS (ESI⁺) calcd for C₂₇H₃₀N₃O₂ ([M + H]⁺) *m*/*z* 428.2; found 428.2. 1-(4-((5-(3,5-Dimethylisoxazol-4-yl)-2-methylphenyl)(5-formylpentyl)amino)phenylcycloprop anecarbonitrile was dissolved in dichloroethane, to which was added 3-(5-amino-1-isoindolinon-2-yl)hexahydropyridine-2,6-dione, sodium triacetylborohydride and glacial acetic acid, and the mixture was stirred at room temperature for 6 h. After completion of the reaction, the reaction was quenched with the saturated solution of ammonium chloride. The resultant solution was extracted with ethyl acetate, successively washed with water and saturated NaCl solution, and then dried, concentrated, and purified by column chromatography, to provide the product (36 mg, yield 66%). LC/MS (ESI⁺) calcd for C₄₀H₄₃N₆O₄ ([M + H]⁺) *m*/*z* 671.3; found 671.3. ¹H NMR (400 MHz, CDCl₃) *δ* 8.08 (s, 1H), 7.61 (d, *J* = 8.3 Hz, 1H), 7.37 (d, *J* = 7.9 Hz, 1H), 7.12 (dd, *J* = 11.9, 5.2 Hz, 3H), 7.00 (d, *J* = 1.6 Hz, 1H), 6.96 (s, 1H), 6.75 (d, *J* = 7.6 Hz, 1H), 6.45 (d, *J* = 8.8 Hz, 2H), 4.92 (m, 1H), 4.28 (dd, *J* = 58.1, 15.5 Hz, 2H), 3.60 (m, 2H), 3.23 (d, *J* = 7.3 Hz, 2H), 3.20 (s, 3H), 2.97 (dd, *J* = 13.3, 2.2 Hz, 1H), 2.76 (dd, *J* = 19.7, 8.3 Hz, 2H), 2.39 (s, 3H), 2.26 (s, 3H), 2.11 (m, 4H), 1.71 (dt, *J* = 22.6, 7.6 Hz, 4H), 1.46 (m, 2H), 1.27 (m, 4H).

### 146: 1-(4-((5-(3,5-dimethylisoxazol-4-yl)-2-methylphenyl)(5-(4-(2-(2,6-dioxopiperidin-3)-6-fluoro-1,3-dicarbonylisoindolin-5-yl)piperidin-1-yl)pentyl)amino)phenylcyclopropaneca rbonitrile (146)

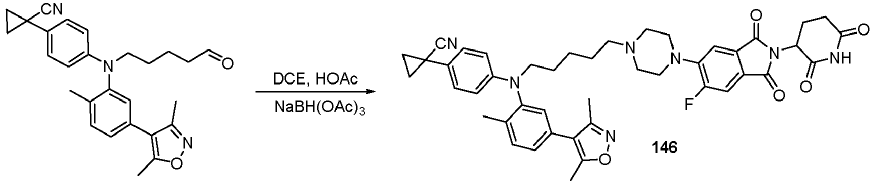

t-Butyl 4-(2-(dioxopiperidin-3-yl)-6-fluoro-1,3-dicarbonylisoindolin-5-yl)piperazine-1-carboxylate was dissolved in DCM, to which was added trifluoroacetic acid, and the solution was stirred for 6 h. After completion of the reaction, the reaction solution was concentrated several times. The obtained concentrate was dissolved in DCM, and then sodium triacetylborohydride, 1-(4-((5-(3,5-dimethylisoxazol-4-yl)-2-methylphenyl)(5-formylpentyl) amino)phenylcyclopropanecarbonitrile and glacial acetic acid were added. The mixture was stirred for 6 h at room temperature. After completion of the reaction, the reaction was quenched with the saturated aqueous solution of ammonium chloride. The resultant solution was extracted with ethyl acetate, successively washed with water and saturated NaCl solution, and then dried, concentrated, and purified by column chromatography, to provide the product (11 mg, yield 54%). LC/MS (ESI⁺) calcd for C₄₄H₄₇FN₇O_{S} ([M + H]⁺) *m*/*z* 772.4; found 772.4. ¹H NMR (400 MHz, CDCl₃) *δ 8.05* (s, 1H), 7.50 (m, 1H), 7.37 (m, 2H), 7.10 (m, 3H), 7.00 (s, 1H), 6.45 (d, *J* = 8.8 Hz, 2H), 4.93 (m, 1H), 3.51 (m, 8H), 2.81 (m, 6H), 2.41 (s, 3H), 2.16 (s, 3H), 2.11 (m, 4H), 1.41 (m, 4H), 0.86 (m, 4H).

### 147: 1-(4-((5-(3,5-Dimethylisoxazol-4-yl)-2-methylphenyl)(5-((2-(2,6-dioxopiperidin-3)-1,3-dicarbonyl isoindolin-5))amino)pentyl)amino)phenylcyclopropanecarbonitrile (147)

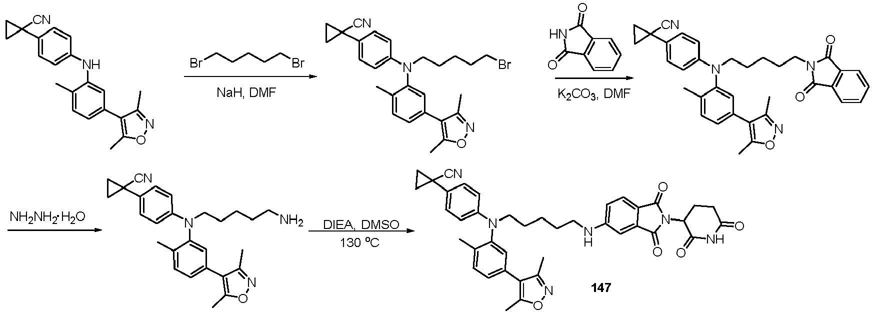

1-(4-(5-(3,5-Dimethylisoxazol-4-yl)-2-methylphenyl)amino)phenylcyclopropanecarbonitrile was dissolved in DMF, and the solution was cooled to 0 °C in an ice bath, to which was then added NaH. After addition, the reaction solution was warmed to room temperature and stirred for 0.5 h. Liquid seal was used to observe whether the system was still bubbling. If no bubbles were generated, 1,5-dibromopentane was added, and the reaction solution was stirred at room temperature overnight. After completion of the reaction, the reaction was quenched with the saturated solution of ammonium chloride. The resultant solution was extracted with ethyl acetate, successively washed with water and saturated NaCl solution, and then dried, concentrated, and purified by column chromatography, to provide the product (370 mg, yield 88%). LC/MS (ESI⁺) calcd for C₂₇H₃₁BrN₃O ([M + H]⁺) *m*/*z* 492.2; found 492.2. 1-(4-((5-Bromopentyl)(5-(3,5-dimethylisoxazol-4-yl)-2-methylphenyl)amino)phenylcyclopropa necarbonitrile was dissoved in DMF, to which were added potassium carbonate and phthalimide, and the mixture was stirred for 3 h at room temperature. After completion of the reaction, the reaction was quenched with ethyl acetate. The reaction solution was washed with water and saturated NaCl solution, and then dried, concentrated, and purified by column chromatography, to provide the product (372 mg, yield 86%). LC/MS (ESI⁺) calcd for C₃₅H₃₅N₄O₃ ([M + H]⁺) *m*/*z* 559.3; found 559.3.

1-(4-((5-(3,5-Dimethylisoxazol-4-yl)-2-methylphenyl)(5-(1,3-dicarbonylisoindolin-2)pentyl)am ino)phenylcyclopropanecarbonitrile was dissolved in THF, to which was added hydrazine hydrate, and the mixture was stirred for 3 h at room temperature. After completion of the reaction, the reaction was quenched with ethyl acetate. The reaction solution was successively washed several times with water and washed with saturated NaCl solution, and then concentrated, and purified by column chromatography, to provide the product (300 mg, yield 90%). LC/MS (ESI⁺) calcd for C₂₇H₃₃N₄O ([M + H]⁺) *m*/*z* 429.3; found 429.3.

1-(4-((5-Aminopentyl)(5-(3,5-dimethylisoxazol-4-yl)-2-methylphenyl)amino)phenylcyclopropa necarbonitrile and 5-amino-2-(2,6-dioxopiperidin-3-yl)isoindoline-1,3-dione were dissolved in DMSO, to which was added DIEA, and the mixture was allowed to react at 130 °C for 3 h. After the reaction was completed, the system was cooled to room temperature, and then the reaction was quenched with the saturated solution of ammonium chloride. The resultant solution was extracted with ethyl acetate, successively washed with water and saturated NaCl solution, and then dried, concentrated, and purified by column chromatography, to provide the product (17 mg, yield 74%). LC/MS (ESI⁺) calcd for C₄₀H₄₁N₆O₅ ([M + H]⁺) *m*/*z* 685.3; found 685.3. ¹H NMR (400 MHz, CDCl₃) *δ* 8.11 (s, 1H), 7.37 (d, *J=* 7.9 Hz, 1H), 7.12 (dd, *J=* 11.9, 5.2 Hz, 3H), 7.00 (d, *J=* 1.6 Hz, 1H), 6.96 (s, 1H), 6.75 (d, *J=* 7.6 Hz, 1H), 6.45 (d, *J=* 8.8 Hz, 2H), 4.92 (m, 1H), 3.60 (m, 2H), 3.23 (d, *J=* 7.3 Hz, 2H), 2.97 (dd, *J=* 13.3, 2.2 Hz, 1H), 2.76 (dd, *J* = 19.7, 8.3 Hz, 2H), 2.39 (s, 3H), 2.26 (s, 3H), 2.11 (m, 4H), 1.71 (dt, *J* = 22.6, 7.6 Hz, 4H), 1.46 (m, 2H), 1.27 (m, 4H).

### 148: 1-(4-((5-(3,5-Dimethylisoxazol-4-yl)-2-methylphenyl)(5-((2-(2,6-dioxopiperidin-3)-6-fluoro-1,3-dicarbonylisoindolin-5))amino)pentyl)amino)phenylcyclopropanecarbonitrile (148)

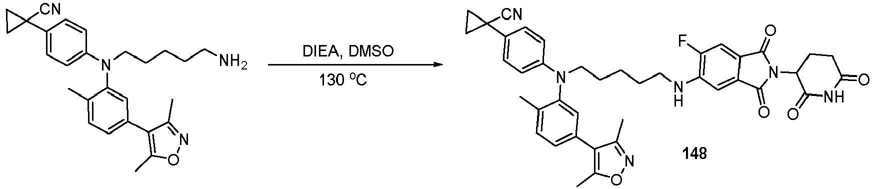

1-(4-((5-Aminopentyl)(5-(3,5-dimethylisoxazol-4-yl)-2-methylphenyl)amino)phenylcyclopropa necarbonitrile and 5-amino-2-(2,6-dioxopiperidin-3-)-6-fluoroisoindoline-1,3-dione were dissolved in DMSO, to which was added DIEA, and the mixture was allowed to react at 130 °C for 3 h. After the reaction was completed, the system was cooled to room temperature, and then the reaction was quenched with the saturated solution of ammonium chloride. The resultant solution was extracted with ethyl acetate, successively washed with water and saturated NaCl solution, and then dried, concentrated, and purified by column chromatography, to provide the product (22 mg, yield 80%). LC/MS (ESI⁺) calcd for C₄₀H₄₀FN₆O₅ ([M + H]⁺) *m*/*z* 703.3; found 703.3. ¹H NMR (400 MHz, CDCl₃) *δ* 8.29 (s, 1H), 7.37 (m, 2H), 7.11 (m, 3H), 7.04 (d, *J* = 7.6 Hz, 1H), 7.00 (d, *J* = 1.6 Hz, 1H), 6.45 (d, *J* = 8.8 Hz, 2H), 4.92 (m, 1H), 3.60 (t, *J* = 7.6 Hz, 2H), 3.26 (t, *J* = 6.8 Hz, 2H), 2.76 (m, 3H), 2.39 (s, 3H), 2.25 (s, 3H), 2.12 (m, 4H), 1.72 (m, 4H), 1.59 (m, 2H), 1.44 (m, 2H), 1.27 (m, 3H).

### 149: 1-(4-((5-(Dimethylisoxazol-4-yl)-2-methylphenyl)(((1r,4r)-4-(((2-(2,6-dioxopiperidin-3)-1,3-dicarbonylisoindolin-5-yl)amino)methyl)cyclohexyl)methyl)amino)phenylcycloprop anecarbonitrile (149)

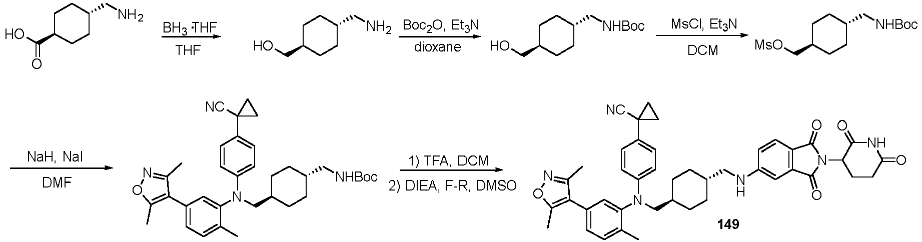

Tranexamic acid was dissolved in tetrahydrofuran, and the system was purged with nitrogen, to which was added the tetrahydrofuran complex of borane. The mixture was stirred overnight at room temperature. After the reaction was completed, water was carefully and slowly added to quench the reaction. The resultant solution was extracted with ethyl acetate, dried, concentrated, and directly used in the next step.

The compound obtained in the previous step was dissolved in dioxane, and then triethylamine was added, followed by addition of Boc anhydride. The mixture was stirred at room temperature for 6 h. The reaction was quenched by adding ethyl acetate. The resultant solution was successively washed with 0.5 N hydrochloric acid, water, and saturated NaCl solution, and then dried, concentrated, and purified by column chromatography, to provide the product (5.7 mg, two-step yield 77%). LC/MS (ESI⁺) calcd for C₁₃H₂₆NO₃ ([M + H]⁺) *m*/*z* 244.2; found 188.2.

t-Butyl (((1r,4r)-4-(hydroxymethyl)cyclohexyl)methyl)aminoformate was dissolved in dichloromethane, to which was added triethylamine, and then the system was cooled to 0 °C. Then, methanesulfonyl chloride was slowly added. After addition, the reaction solution was warmed to room temperature and stirred for 1 h. After the reaction was completed, the reaction was quenched with 0.5 N dilute hydrochloric acid. The resultant solution was extracted with dichloromethane, successively washed with the saturated solution of sodium bicarbonate, water, and saturated NaCl solution, dried, concentrated, and purified by column chromatography, to provide the product (6.1 g, yield 96%). LC/MS (ESI⁺) calcd for C₁₄H₂₈NO₅S ([M + H]⁺) *m*/*z* 322.2; found 322.2.

1-(4-(5-(3,5-Dimethylisoxazol-4-yl)-2-methylphenyl)amino)phenylcyclopropanecarbonitrile was dissolved in DMF, and the solution was cooled to 0 °C in an ice bath, to which was then added NaH. After addition, the reaction solution was warmed to room temperature and stirred for 0.5 h. Liquid seal was used to observe whether the system was still bubbling. If no bubbles were generated, t-butyl (((1*r*,4*r*)-4-(((4-(1-cyanocyclopropyl)phenyl) (5-(3,5-dimethylisoxazol-4-yl)-2-methylphenyl)amino)methyl)cyclohexyl)formate and sodium iodide were added, and the reaction solution was stirred for 48 h at 60 °C. After completion of the reaction, the reaction was quenched with the saturated solution of ammonium chloride. The resultant solution was extracted with ethyl acetate, successively washed with water and saturated NaCl solution, and then dried, concentrated, and purified by column chromatography, to provide the product (550 mg, yield 28%). LC/MS (ESI⁺) calcd for C₃₅H₄₅N₄O₃ ([M + H]⁺) *m*/*z* 569.4; found 569.4.

t-Butyl (((1*r*,4*r*)-4-(((4-(1 -cyanocyclopropyl)phenyl)(5 -(3,5-dimethylisoxazol-4-yl)-2-methylphenyl)amino)methyl)cyclohexyl)fomate was dissolved in DMC, to which was added trifluoroacetic acid, and the mixture was stirred overnight at room temperature. After the reaction was completed, the reaction solution was concentrated, and the residue was dissolved in dichloromethane, and then the resultant solution was concentrated, that was repeated several times. The resultant system was dissolved in DMSO, to which was added DIEA, and the mixture was stirred for 10 min at room temperature, followed by addition of 5-amino-2-(2,6-dioxopiperidin-3-yl)isoindoline-1,3-dione. The mixture was allowed to react at 130 °C for 3 h. After completion of the reaction, the system was cooled to room temperature, and then the reaction was quenched with the saturated solution of ammonium chloride. The resultant solution was extracted with ethyl acetate, successively washed with water and saturated NaCl solution, and then dried, concentrated, and purified by column chromatography, to provide the product (17 mg, yield 80%). LC/MS (ESI⁺) calcd for C₄₃H₄₅N₆O₅ ([M + H]⁺) *m*/*z* 725.4; found 725.4. ¹H NMR (400 MHz, CDCl₃) *δ* 8.07 (s, 1H), 7.60 (d*, J* = 8.0 Hz, 1H), 7.35 (d, *J* = 8.0 Hz, 1H), 7.08 (m, 4H), 6.93 (d, *J* = 2.0 Hz, 1H), 6.71 (dd, *J* = 8.4, 1.6 Hz, 1H), 6.46 (d, *J* = 8.8 Hz, 1H), 4.92 (m, 1H), 3.46 (d, *J* = 6.8 Hz, 2H), 3.07 (d, *J* = 6.8 Hz, 2H), 2.83 (m, 4H), 2.41 (s, 3H), 2.28 (s, 3H), 2.09 (m, 4H), 1.91 (m, 5H), 1.26 (m, 4H), 0.96 (m, 4H).

### 150: 1-(4-((5-(3,5-Dimethylisoxazol-4-yl)-2-methylphenyl)(((1r,4r)-4-(((2-(2,6-dioxopiperidin-3)-6-fluoro-1,3-dicarbonylisoindolin-5-yl)amino)methyl)cyclohexyl)methyl )amino)phenylcyclopropanecarbonitrile (150)

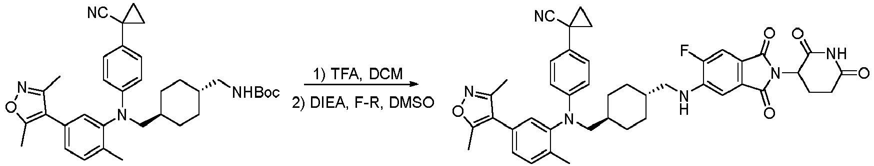

t-Butyl (((1*r*,4*r*)-4-(((4-(1-cyanocyclopropyl)phenyl)(5 -(3,5-dimethylisoxazol-4-yl)-2-methylphenyl)amino)methyl)cyclohexyl)formate was dissolved in DCM, to which was added trifluoroacetic acid, and the mixture was stirred overnight at room temperature. After the reaction was completed, the reaction solution was concentrated, and the residue was dissolved in dichloromethane, and then the resultant solution was concentrated, that was repeated several times. The resultant system was dissolved in DMSO, to which was added DIEA, and the mixture was stirred for 10 min at room temperature, followed by addition of 5-amino-2-(2,6-dioxopiperidin-3-yl)-6-fluoroisoindoline-1,3-dione. The mixture was allowed to react at 130 °C for 3 h. After completion of the reaction, the system was cooled to room temperature, and then the reaction was quenched with the saturated solution of ammonium chloride. The resultant solution was extracted with ethyl acetate, successively washed with water and saturated NaCl solution, and then dried, concentrated, and purified by column chromatography, to provide the product (22 mg, yield 86%). LC/MS (ESI⁺) calcd for C₄₃H₄₄FN₆O₅ ([M + H]⁺) *m*/*z* 743.3; found 743.3. ¹H NMR (400 MHz, CDCl₃) *δ* 8.08 (s, 1H), 7.59 (d*, J* = 8.0 Hz, 1H), 7.36 (d, *J* = 8.0 Hz, 1H), 7.06 (m, 5H), 6.93 (d, *J* = 2.0 Hz, 1H), 6.71 (dd, *J* = 8.4, 1.6 Hz, 1H), 6.47 (d, *J* = 8.8 Hz, 1H), 4.92 (m, 1H), 3.46 (d, *J* = 6.8 Hz, 2H), 3.07 (d, *J* = 6.8 Hz, 2H), 2.83 (m, 4H), 2.42 (s, 3H), 2.28 (s, 3H), 2.09 (m, 4H), 1.89 (m, 5H), 1.26 (m, 4H), 0.95 (m, 4H).

### 151: 1-(4-((5-(3,5-Dimethylisoxazol-4-yl)-2-methylphenyl)(5-((2-(1-methyl-2,6-dioxopiperidin-3)-1,3-dicarbonylisoindolin-5-yl))amino)pentyl)amino)phenylcyclopropane carbonitrile (151)

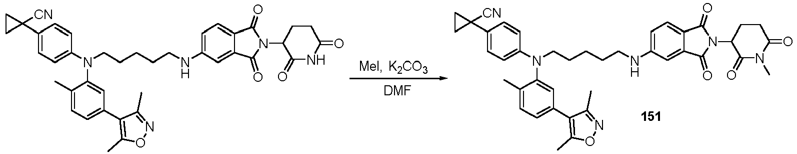

1-(4-((5-(dimethylisoxazol-4-yl)-2-methylphenyl)(((1r,4r)-4-(((2-(2,6-dioxopiperidin-3)-1,3-dic arbonylisoindolin-5-yl)amino)methyl)cyclohexyl)methyl)amino)phenylcyclopropanecarbonitril e was dissolved in DMF, to which were added potassium carbonate and iodomethane, and the mixture was stirred 30 min at room temperature. After completion of the reaction, the reaction was quenched with ethyl acetate. The resultant solution was washed with water and saturated NaCl solution, and then concentrated, dried, and purified by column chromatography, to provide the product (22 mg, yiled 86%). LC/MS (ESI⁺) calcd for C₄₄H₄₇N₆O₅ ([M + H]⁺) *m*/*z* 739.4; found 739.4. ¹H NMR (400 MHz, CDCl₃) *δ* 7.61 (d, *J* = 8.3 Hz, 1H), 7.37 (d, *J* = 7.9 Hz, 1H), 7.12 (dd, *J* = 11.9, 5.2 Hz, 3H), 7.00 (d, *J* = 1.6 Hz, 1H), 6.96 (s, 1H), 6.75 (d, *J* = 7.6 Hz, 1H), 6.45 (d, *J* = 8.8 Hz, 2H), 4.92 (m, 1H), 3.60 (m, 2H), 3.23 (d, *J* = 7.3 Hz, 2H), 3.20 (s, 3H), 2.97 (dd, *J* = 13.3, 2.2 Hz, 1H), 2.76 (dd, *J* = 19.7, 8.3 Hz, 2H), 2.39 (s, 3H), 2.26 (s, 3H), 2.11 (m, 4H), 1.71 (dt, *J* = 22.6, 7.6 Hz, 4H), 1.46 (m, 2H), 1.27 (m, 4H).

### 152: 1-(4-((5-(3,5-Dimethylisoxazol-4-yl)-2-methylphenyl)((1-(1-(2-(2,6-dioxopiperidin-3)-1,3-dicarbonylisoindolin-5-yl)piperidin-4-yl)azetidin-3-yl)methyl)amino)phenylcyclopropa necarbonitrile (152)

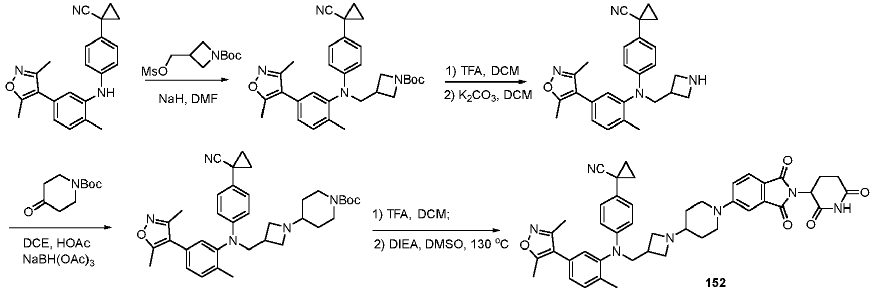

1-(4-(5-(3,5-Dimethylisoxazol-4-yl)-2-methylphenyl)amino)phenylcyclopropanecarbonitrile was dissolved in DMF, and the solution was cooled to 0 °C in an ice bath, to which was then added NaH. After addition, the reaction solution was warmed to room temperature and stirred for 0.5 h. Liquid seal was used to observe whether the system was still bubbling. If no bubbles were generated, t-butyl 3-(methanesulfonyloxymethylene)azetidine-1-carboxylate was added, and the reaction solution was stirred overnight at 60 °C. After completion of the reaction, the reaction was quenched with the saturated solution of ammonium chloride. The resultant solution was extracted with ethyl acetate, successively washed with water and saturated NaCl solution, and then dried, concentrated, and purified by column chromatography, to provide the product (550 mg, yield 28%). LC/MS (ESI⁺) calcd for C₃₁H₃₇N₄O₃ ([M + H]⁺) *m*/*z* 513.3; found 513.3.

t-Butyl 3 -(((4-(1-cyanocyclopropyl)phenyl)(5 -(3,5-dimethylisoxazol-4-yl)-2-methylphenyl) amino)methyl)azetidine-1-carboxylic acid, obtained in the previous step, was dissolved in DCM, to which was added trifluoroacetic acid, and the mixture was stirred overnight at room temperature. After the reaction was completed, the reaction solution was concentrated, and the residue was dissolved in dichloromethane, and then the resultant solution was concentrated, that was repeated several times. The residue was further dissolved in DCM, to which was added solid potassium carbonate, and the mixture was stirred at room temperature for half an hour. The reaction solution was filtered, and then the filtrate was concentrated to provide the target compound, which was directly used in the next step.

1-(4-((azetidine-3-methylene)(5-(3,5-dimethylisoxazol-4-yl)-2-methylphenyl)amino) phenylcyclopropanecarbonitrile was dissolved in dichloroethane, to which were added N-t-butoxycarbonyl-4-piperidone, sodium triacetylborohydride and glacial acetic acid, and then the solution was stirred for 6 h at room temperature. After completion of the reaction, the reaction was quenched with the saturated solution of ammonium chloride. The resultant solution was extracted with ethyl acetate, successively washed with water and saturated NaCl solution, and then dried, concentrated, and purified by column chromatography, to provide the product (82 mg, yield 73%). LC/MS (ESI⁺) calcd for C₃₆H₄₆N₅O₃ ([M + H]⁺) *m*/*z* 596.4; found 596.4.

t-Butyl 4-(3 -(((4-(1-cyanocyclopropyl)phenyl)(5 -(3,5-dimethylisoxazol-4-yl)-2-methylphenyl) amino)methyl))azetidin-1-yl)piperidin-1-ylcarboxylate was dissolved in DCM, to which was added trifluoroacetic acid, and the mixture was stirred overnight at room temperature. After the reaction was completed, the reaction solution was concentrated, and the residue was dissolved in dichloromethane, and then the resultant solution was concentrated, that was repeated several times. The resultant system was dissolved in DMSO, to which was added DIEA, and the mixture was stirred for 10 min at room temperature, followed by addition of 5-amino-2-(2,6-dioxopiperidin-3-yl)isoindoline-1,3-dione. The mixture was allowed to react at 130 °C for 3 h. After completion of the reaction, the system was cooled to room temperature, and then the reaction was quenched with the saturated solution of ammonium chloride. The resultant solution was extracted with ethyl acetate, successively washed with water and saturated NaCl solution, and then concentrated, dried, and purified by column chromatography, to provide the product (84 mg, yield 62%).

LC/MS (ESI⁺) calcd for C₄₄H₄₆N₇O₅ ([M + H]⁺) *m*/*z* 752.4; found 752.4.

¹H NMR (400 MHz, CDCl₃) *δ* 8.26 (s, 1H), 7.59 (d*, J* = 8.4 Hz, 1H), 7.36 (d, *J* = 8.0 Hz, 1H), 7.12 (m, 3H), 6.97 (d, *J* = 1.2 Hz, 1H), 6.75 (d, *J* = 2.0 Hz, 1H), 6.48 (m, 3H), 4.91 (m, 1H), 4.00 (t, *J* = 7.6 Hz, 2H), 3.89 (d, *J* = 6.8 Hz, 1H), 3.80 (m, 2H), 3.60 (m, 1H), 3.41 (m, 2H), 2.78 (m, 4H), 2.39 (s, 3H), 2.25 (s, 3H), 2.09 (m, 4H), 1.62 (m, 6H), 0.95 (m, 4H).

### 153: 1-(4-((5-(3,5-Dimethylisoxazol-4-yl)-2-methylphenyl)((1'-(2-(2,6-dioxopiperidin-3)-6-fluoro-1,3-dicarbonylisoindolin-5-yl)-[1,3'-diazetidine]-3-yl)methyl)amino)phenylcyclop ropanecarbonitrile (153)

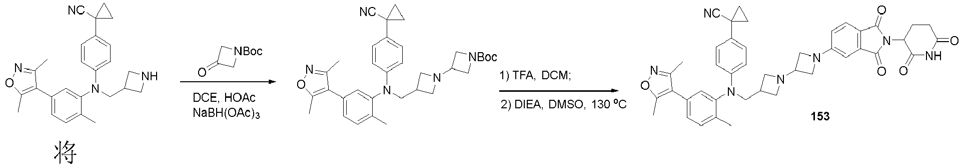

1-(4-((Azetidine-3-methylene)(5-(3,5-dimethylisoxazol-4-yl)-2-methylphenyl)amino)phenylcyc lopropanecarbonitrile was dissolved in DCM, to which were added 1-Boc-3-azetidinone, sodium triacetylborohydride, and glacial acetic acid, and then the mixture was stirred for 6 h at room temperature. After the reaction was completed, the reaction was quenched with the saturated solution of ammonium chloride. The resultant solution was extracted with ethyl acetate, successively washed with water and saturated NaCl solution, and then dried, concentrated, and purified by column chromatography, to provide the product (35 mg, yield 66%). LC/MS (ESI⁺) calcd for C₃₄H₄₂N₅O₃ ([M + H]⁺) *m*/*z* 568.3; found 568.3.

t-Butyl 3-(4-(((4-(1-cyanocyclopropyl)phenyl)(5 -(3,5-dimethylisoxazol-4-yl)-2-methylphenyl) amino)methyl)-[1,3'-diazetidine]-1'-carboxylate was dissolved in DCM, to which was added trifluoroacetic acid, and the mixture was stirred overnight at room temperature. After the reaction was completed, the reaction solution was concentrated, and the residue was dissolved in dichloromethane, and then the resultant solution was concentrated, that was repeated several times. The resultant system was dissolved in DMSO, to which was added DIEA, and the mixture was stirred for 10 min at room temperature, followed by addition of 5-amino-2-(2,6-dioxopiperidin-3-yl)isoindoline-1,3-dione. The mixture was allowed to react at 130 °C for 3 h. After completion of the reaction, the system was cooled to room temperature, and then the reaction was quenched with the saturated solution of ammonium chloride. The resultant solution was extracted with ethyl acetate, successively washed with water and saturated NaCl solution, and then concentrated, dried, and purified by column chromatography, to provide the product (14 mg, yield 60%).

LC/MS (ESI⁺) calcd for C₄₂H₄₂N₇O₅ ([M + H]⁺) *m*/*z* 724.3; found 724.3.

¹H NMR (400 MHz, CDCl₃) *δ* 8.28 (s, 1H), 7.63 (d*, J* = 8.4 Hz, 1H), 7.36 (d, *J* = 8.0 Hz, 1H), 7.12 (m, 3H), 6.97 (d, *J* = 1.2 Hz, 1H), 6.75 (d, *J* = 2.0 Hz, 1H), 6.48 (m, 3H), 4.91 (m, 1H), 4.00 (t, *J* = 7.6 Hz, 2H), 3.89 (d, *J* = 6.8 Hz, 1H), 3.80 (m, 2H), 3.60 (m, 1H), 3.41 (m, 2H), 2.78 (m, 4H), 2.39 (s, 3H), 2.25 (s, 3H), 2.09 (m, 4H), 1.60 (m, 3H), 1.27 (m, 3H).

### 154: 1-(4-((5-(3,5-dimethylisoxazol-4-yl)-2-methylphenyl)((7-(2-(2,6-dioxopiperidin-3-yl)-6-fluoro-1,3-dicarbonylisoindolin-5-yl-)-7-azaspiro[3.5]nonan-2-yl)methyl)amino)phenylcy clopropanecarbonitrile (154)

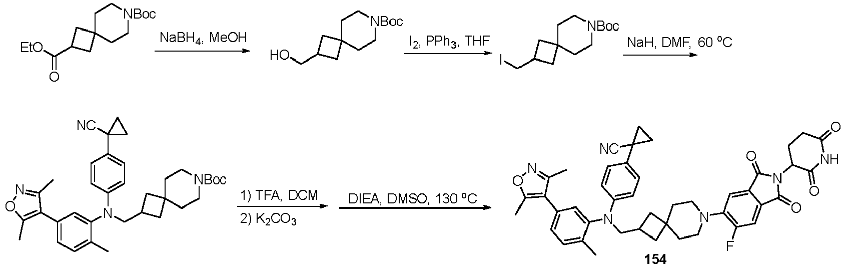

7-Boc-7-azaspiro[3.5]nonan-2-ylformic acid was dissolved in methanol, to which was added sodium borohydride in an ice bath, and after addition, the reaction solution was warmed to room temperature and stirred for 6 h. After completion of the reaction, the reaction was quenched by slowly adding the saturated solution of ammonium chloride. The resultant solution was extracted with ethyl acetate, successively washed with water and saturated NaCl solution, and then dried, concentrated, and purified by column chromatography, to provide the product (280 mg, yield 66%). LC/MS (ESI⁺) calcd for C₁₄H₂₆NO₃ ([M + H]⁺) *m*/*z* 256.2; found 256.2.

7-Boc-7-azaspiro[3.5]nonan-2-ylmethanol was dissolved in tetrahydrofuran, to which were added triphenylphosphine and iodine in an ice bath, and then the mixture was stirred at room temperature for 4 h. After completion of the reaction, the reaction was quenched with the solution of sodium bisulfite. The resultant solution was extracted with ethyl acetate, successively washed with water and saturated NaCl solution, and then dried, concentrated, and purified by column chromatography, to provide the product (360 mg, yield 96%). LC/MS (ESI⁺) calcd for C₁₄H₂₅INO₂ ([M + H]⁺) *m*/*z* 366.1; found 366.1.

1-(4-(5-(3,5-Dimethylisoxazol-4-yl)-2-methylphenyl)amino)phenylcyclopropanecarbonitrile was dissolved in DMF, and the solution was cooled to 0 °C in an ice bath, to which was then added NaH. After addition, the reaction solution was warmed to room temperature and stirred for 0.5 h. Liquid seal was used to observe whether the system was still bubbling. If no bubbles were generated, t-butyl 2-(iodomethyl)-7-azaspiro[3.5]nonane-7-carboxylate was added, and the reaction solution was stirred overnight at 60 °C. The reaction was quenched with the saturated solution of ammonium chloride. The resultant solution was extracted with ethyl acetate, successively washed with water and saturated NaCl solution, and then dried, concentrated, and purified by column chromatography, to provide the product (80 mg, yield 30%). LC/MS (ESI⁺) calcd for C₃₆H₃₅N₄O₃ ([M + H]⁺) *m*/*z* 581.4; found 581.4.

t-Butyl 2-(((4-(1-cyanocyclopropyl)phenyl)(5 -(3,5-dimethylisoxazol-4-yl)-2-methylphenyl) amino)methyl)-7-azaspiro[3.5]nonane-7-carboxylate was dissolved in DCM, to which was added trifluoroacetic acid, and the mixture was stirred overnight at room temperature. After the reaction was completed, the reaction solution was concentrated, and the residue was dissolved in dichloromethane, and then the resultant solution was concentrated, that was repeated several times. The resultant system was dissolved in DMSO, to which was added DIEA, and the mixture was stirred for 10 min at room temperature, followed by addition of 5-amino-2-(2,6-dioxopiperidin-3-)-6-fluoroisoindoline-1,3-dione. The mixture was allowed to react at 130 °C for 3 h. After completion of the reaction, the system was cooled to room temperature, and then the reaction was quenched with the saturated solution of ammonium chloride. The resultant solution was extracted with ethyl acetate, successively washed with water and saturated NaCl solution, and then concentrated, dried, and purified by column chromatography, to provide the product (8 mg, yield 36%).

LC/MS (ESI⁺) calcd for C₄₄H₄₄FN₆O₅ ([M + H]⁺) *m*/*z* 755.3; found 755.3.

### 155: 1-(4-((5-(3,5-Dimethylisoxazol-4-yl)-2-methylphenyl)((1'-(2-(2,6-dioxopiperidin-3)-6-fluoro-3-isoindolinone-5-yl)azetidin-3-yl)methyl)amino)phenylcyclopropanecarbonitrile (155)

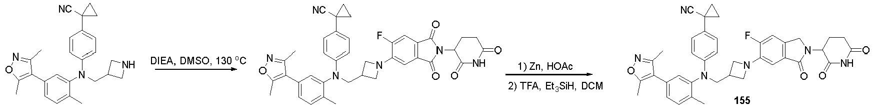

1-(4-((Azetidine-3-methylene)(5-(3,5-dimethylisoxazol-4-yl)-2-methylphenyl)amino)phenylcyc lopropanecarbonitrile was dissolved in DMSO, to which were added DIEA and 5-amino-2-(2,6-dioxopiperidin-3-)-6-fluoroisoindoline-1,3-dione. The mixture was allowed to react at 130 °C for 3 h. After completion of the reaction, the system was cooled to room temperature, and then the reaction was quenched with the saturated solution of ammonium chloride. The resultant solution was extracted with ethyl acetate, successively washed with water and saturated NaCl solution, and then concentrated, dried, and purified by column chromatography, to provide the product (46 mg, yield 80%). LC/MS (ESI⁺) calcd for C₃₉H₃₅FN₆O₅ ([M + H]⁺) *m*/*z* 686.3; found 686.3.

1-(4-((5-(3,5-Dimethylisoxazol-4-yl)-2-methylphenyl)((1-(2-(2,6-dioxopiperidin-3-)-6-fluoro-1, 3-dicarbonylisoindolin-5-)azetidin-3-yl-)methyl)amino)phenylcyclopropanecarbonitrile was dissolved in glacial acetic acid, to which was added zinc powder, and the mixture was allowed to react at 60 °C for 6 h. After the reaction was completed, the reaction solution was cooled, filtered through celite, and then the filter cake was rinsed with ethyl acetate several times. The filtrate was concentrated. The obtained product was dissolved in dichloromethane, to which were successively added trifluoroacetic acid and triethylsilane, and then the reaction solution was stirred overnight at room temperature. After the reaction was completed, the system was concentrated and purified by column chromatography, to provide the product (4 mg, yield 12%). LC/MS (ESI⁺) calcd for C₃₉H₃₇FN₆O₄ ([M + H]⁺) *m*/*z* 672.3; found 672.3. ¹H NMR (400 MHz, CDCl₃) *δ* 7.92 (s, 1H), 7.52 (m, 3H), 7.14 (m, 2H), 7.00 (m, 4H), 5.18 (m, 1H), 4.21 (m, 4H), 3.75 (m, 1H), 2.94 (m, 4H), 2.40 (s, 3H), 2.26 (s, 3H), 2.13 (s, 3H), 1.65 (m, 4H), 0.88 (m, 4H).

### 156: 1-(4-((5-(3,5-Dimethylisoxazol-4-yl)-2-methylphenyl)((1'-(2-(2,6-dioxopiperidin-3-yl)-6-fluoro-1-isoindolinone-5-yl)azetidin-3-yl)methyl)amino) phenylcyclopropanecarbonitrile (156)

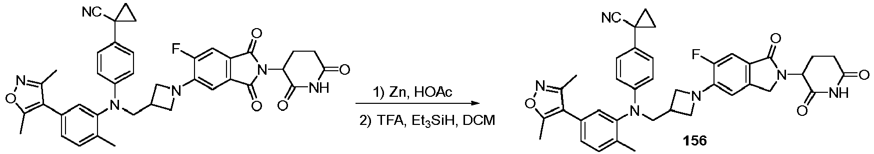

1-(4-((5-(3,5-Dimethylisoxazol-4-yl)-2-methylphenyl)((1 -(2-(2,6-dioxopiperidin-3-)-6-fluoro-1, 3-dicarbonylisoindolin-5-)azetidin-3-yl-)methyl)amino)phenylcyclopropanecarbonitrile was dissolved in glacial acetic acid, to which was added zinc powder, and the mixture was allowed to react at 60 °C for 6 h. After the reaction was completed, the reaction solution was cooled, filtered through celite, and then the filter cake was rinsed with ethyl acetate several times. The filtrate was concentrated. The obtained product was dissolved in dichloromethane, to which were successively added trifluoroacetic acid and triethylsilane, and then the reaction solution was stirred overnight at room temperature. After the reaction was completed, the system was concentrated and purified by column chromatography, to provide the product (12 mg, yield 36%). LC/MS (ESI⁺) calcd for C₃₉H₃₇FN₆O₄ ([M + H]⁺) *m*/*z* 672.3; found 672.3. ¹H NMR (400 MHz, CDCl₃) *δ* 8.02 (s, 1H), 7.45 (m, 3H), 7.12 (m, 2H), 6.94 (m, 2H), 6.57 (m, 2H), 5.15 (m, 1H), 4.21 (m, 4H), 3.75 (m, 1H), 2.94 (m, 4H), 2.40 (s, 3H), 2.27 (s, 3H), 2.12 (s, 3H), 1.65 (m, 4H), 0.87 (m, 4H).

### 157: 1-(4-((5-(3,5-Dimethylisoxazol-4-yl)-2-methylphenyl)((1'-(2-(2,6-dioxopiperidin-3)-6-fluoro-3-isoindolinone-5-yl)-[1,3'-diazetidine]-3)methyl)amino)phenylcyclopropanecarb onitrile (157)

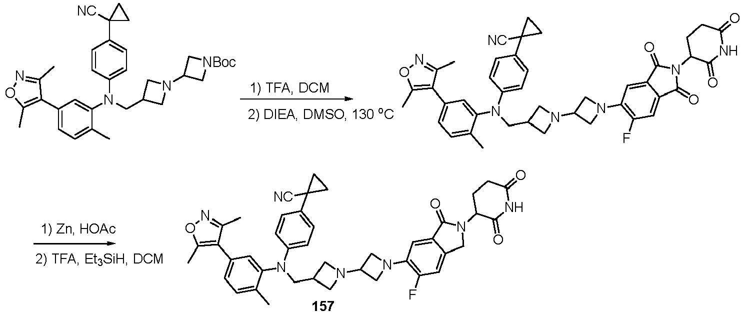

t-Butyl 3-(4-(((4-(1-cyanocyclopropyl)phenyl)(5 -(3,5-dimethylisoxazol-4-yl)-2-methylphenyl) amino)methyl)-[1,3'-diazetidine]-1'-carboxylate was dissolved in DCM, to which was added trifluoroacetic acid, and the mixture was stirred overnight at room temperature. After the reaction was completed, the reaction solution was concentrated, and the residue was dissolved in dichloromethane, and then the resultant solution was concentrated, that was repeated several times. The resultant system was dissolved in DMSO, to which was added DIEA, and the mixture was stirred for 10 min at room temperature, followed by addition of 5-amino-2-(2,6-dioxopiperidin-3-yl)-6-fluoroisoindoline-1,3-dione. The mixture was allowed to react at 130 °C for 3 h. After completion of the reaction, the system was cooled to room temperature, and then the reaction was quenched with the saturated solution of ammonium chloride. The resultant solution was extracted with ethyl acetate, successively washed with water and saturated NaCl solution, and then concentrated, dried, and purified by column chromatography, to provide the product (72 mg, yield 76%). LC/MS (ESI⁺) calcd for C₄₂H₄₁FN₇O₅ ([M + H]⁺) *m*/*z* 742.3; found 742.3.

1-(4-((5-(3,5-Dimethylisoxazol-4-yl)-2-methylphenyl)((1'-(2-(2,6-dioxopiperidin-3)-6-fluoro-1, 3-dicarbonylisoindolin-5-yl)-[1,3'-diazetidine]-3)methyl)amino)phenylcyclopropanecarbonitrile was dissolved in glacial acetic acid, to which was added zinc powder, and the mixture was allowed to react at 60 °C for 6 h. After the reaction was completed, the reaction solution was cooled, filtered through celite, and then the filter cake was rinsed with ethyl acetate several times. The filtrate was concentrated. The obtained product was dissolved in dichloromethane, to which were successively added trifluoroacetic acid and triethylsilane, and then the reaction solution was stirred overnight at room temperature. After the reaction was completed, the system was concentrated and purified by column chromatography, to provide the product (3 mg, yield 7%). LC/MS (ESI⁺) calcd for C₄₂H₄₃FN₇O₄ ([M + H]⁺) *m*/*z* 728.3; found 728.3. ¹H NMR (400 MHz, CDCl₃) *δ* 8.36 (s, 1H), 7.35 (dd, *J* = 9.4, 6.5 Hz, 2H), 7.09 (m, 3H), 7.03 (m, 1H), 6.44 (dd, *J* = 16.0, 8.0 Hz, 3H), 5.12 (m, 1H), 4.32 (m, 2H), 3.59 (m, 10H), 3.10 (m, 2H), 2.40 (s, 3H), 2.27 (s, 3H), 2.06 (s, 3H), 1.94 (m, 2H), 1.41 (m, 2H), 0.85 (m, 4H).

### 158: 1-(4-((5-(3,5-dimethylisoxazol-4-yl)-2-methylphenyl)((1'-(2-(2,6-dioxopiperidin-3)-6-fluoro-1-isoindolinone-5-yl)-[1,3'-diazetidine]-3)methyl)amino)phenylcyclopropanecarb onitrile (158)

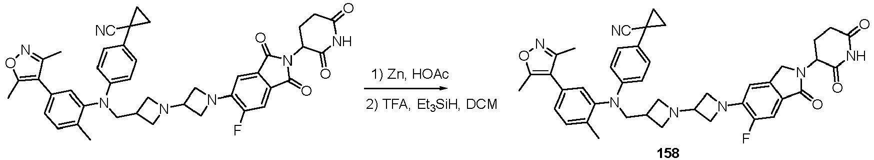

1-(4-((5-(3,5-Dimethylisoxazol-4-yl)-2-methylphenyl)((1'-(2-(2,6-dioxopiperidin-3)-6-fluoro-1, 3-dicarbonylisoindolin-5-yl)-[1,3'-diazetidine]-3)methyl)amino)phenylcyclopropanecarbonitrile was dissolved in glacial acetic acid, to which was added zinc powder, and the mixture was allowed to react at 60 °C for 6 h. After the reaction was completed, the reaction solution was cooled, filtered through celite, and then the filter cake was rinsed with ethyl acetate several times. The filtrate was concentrated. The obtained product was dissolved in dichloromethane, to which were successively added trifluoroacetic acid and triethylsilane, and then the reaction solution was stirred overnight at room temperature. After the reaction was completed, the system was concentrated and purified by column chromatography, to provide the product (6 mg, yield 14%). LC/MS (ESI⁺) calcd for C₄₂H₄₃FN₇O₄ ([M + H]⁺) *m*/*z* 728.3; found 728.3. ¹H NMR (400 MHz, CDCl₃) *δ* 8.35 (s, 1H), 7.35 (dd, *J* = 9.4, 6.5 Hz, 2H), 7.09 (t, *J* = 7.46.8 Hz, 3H), 7.03 (m, 1H), 6.44 (dd, *J* = 16.0, 8.0 Hz, 3H), 5.12 (m, 1H), 4.32 (m, 2H), 3.59 (m, 10H), 3.10 (m, 2H), 2.40 (s, 3H), 2.27 (s, 3H), 2.06 (s, 3H), 1.94 (m, 2H), 1.40 (m, 2H), 0.86 (m, 4H).

### 159: 1-(4-((5-(3,5-Dimethylisoxazol-4-yl)-2-methylphenyl)((1-(1-(2-(2,6-dioxopiperidin-3)-6-fluoro-3-isoindolinone-5-yl)azetidin-3-yl)piperidin-4-yl)methyl)amino)phenylcyclopropa necarbonitrile (159)

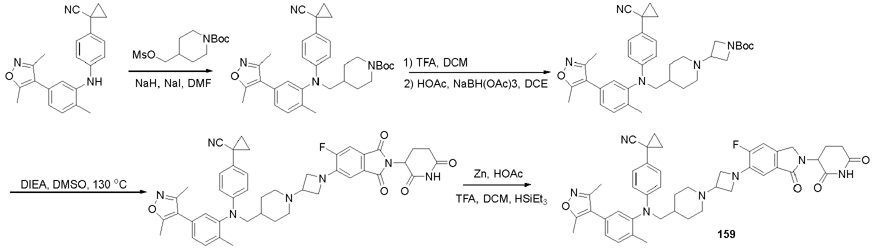

1-(4-(5-(3,5-Dimethylisoxazol-4-yl)-2-methylphenyl)amino)phenylcyclopropanecarbonitrile was dissolved in DMF, and the solution was cooled to 0 °C in an ice bath, to which was then added NaH. After addition, the reaction solution was warmed to room temperature and stirred for 0.5 h. Liquid seal was used to observe whether the system was still bubbling. If no bubbles were generated, t-butyl 4-(methanesulfonyloxymethylene)piperidine-1-carboxylate and sodium iodide were added, and the reaction solution was stirred overnight at 60 °C. After completion of the reaction, the reaction was quenched with the saturated solution of ammonium chloride. The resultant solution was extracted with ethyl acetate, successively washed with water and saturated NaCl solution, and then dried, concentrated, and purified by column chromatography, to provide the product (84 mg, yield 65%). LC/MS (ESI⁺) calcd for C₃₃H₄₁N₄O₃ ([M + H]⁺) *m*/*z* 541.3; found 541.3.

t-Butyl 3 -(((4-(1-cyanocyclopropyl)phenyl)(5 -(3,5-dimethylisoxazol-4-yl)-2-methylphenyl) amino)methyl)azetidine-1-carboxylate, obtained in the previous step, was dissolved in DCM, to which was added trifluoroacetic acid, and the mixture was stirred for 6 h at room temperature. After the reaction was completed, the reaction solution was concentrated, and the residue was dissolved in dichloromethane, and then the resultant solution was concentrated, that was repeated several times. The residue was further dissolved in DCM, to which was added solid potassium carbonate, and the mixture was stirred at room temperature for half an hour. The reaction solution was filtered, and then the filtrate was concentrated to provide the target compound, which was dissolved in dichloromethane. Then, sodium triacetylborohydride was added, and the mixture was stirred at room temperature for 10 min, to which were added N-t-butoxycarbonyl-4-piperidone and glacial acetic acid. The resultant solution was stirred at room temperature for 6 h. After completion of the reaction, the reaction was quenched with the saturated solution of ammonium chloride. The resultant solution was extracted with ethyl acetate, successively washed with water and saturated NaCl solution, and then dried, concentrated, and purified by column chromatography, to provide the product (38 mg, yield 41%). LC/MS (ESI⁺) calcd for C₃₆H₄₆N₅O₃ ([M + H]⁺) *m*/*z* 596.4; found 596.4.

t-Butyl 3-(4-(((4-(1-cyanocyclopropyl)phenyl)(5 -(3,5-dimethylisoxazol-4-yl)-2-methylphenyl) amino)methyl)piperidin-1-yl)azetidine-1-carboxylate was dissolved in DCM, to which was added trifluoroacetic acid, and the mixture was stirred overnight at room temperature. After the reaction was completed, the reaction solution was concentrated, and the residue was dissolved in dichloromethane, and then the resultant solution was concentrated, that was repeated several times. The resultant system was dissolved in DMSO, to which was added DIEA, and the mixture was stirred for 10 min at room temperature, followed by addition of 5-amino-2-(2,6-dioxopiperidin-3-)-6-fluoroisoindoline-1,3-dione. The mixture was allowed to react at 130 °C for 3 h. After completion of the reaction, the system was cooled to room temperature, and then the reaction was quenched with the saturated solution of ammonium chloride. The resultant solution was extracted with ethyl acetate, successively washed with water and saturated NaCl solution, and then concentrated, dried, and purified by column chromatography, to provide the product (26 mg, yield 64%). LC/MS (ESI⁺) calcd for C₄₄H₄₅FN₇O₅ ([M + H]⁺) *m*/*z* 770.4; found 770.4.

1-(4-((5-(3,5-Dimethylisoxazol-4-yl)-2-methylphenyl)((1-(1-(2-(2,6-dioxopiperidin-3)-6-fluoro -1,3-dicarbonylisoindolin-5-yl)azetidin-3-yl)piperidin-4-yl)methyl)amino)phenylcyclopropanec arbonitrile was dissolved in glacial acetic acid, to which was added zinc powder, and the mixture was allowed to react at 60 °C for 6 h. After the reaction was completed, the reaction solution was cooled, filtered through celite, and then the filter cake was rinsed with ethyl acetate several times. The filtrate was concentrated. The obtained product was dissolved in dichloromethane, to which were successively added trifluoroacetic acid and triethylsilane, and then the reaction solution was stirred overnight at room temperature. After the reaction was completed, the system was concentrated and purified by column chromatography, to provide the product (3 mg, yield 28%).

LC/MS (ESI⁺) calcd for C₄₄H₄₇FN₇O₄ ([M + H]⁺) *m*/*z* 756.4; found 756.4.

### 160: 1-(4-((5-(3,5-dimethylisoxazol-4-yl)-2-methylphenyl)((1-(1-(2-(2,6-dioxopiperidin-3-yl)-6-fluoro-1-isoindolinone-5-yl)azetidin-3-yl)piperidin-4-yl)methyl)amino)phenylcyclo propanecarbonitrile (160)

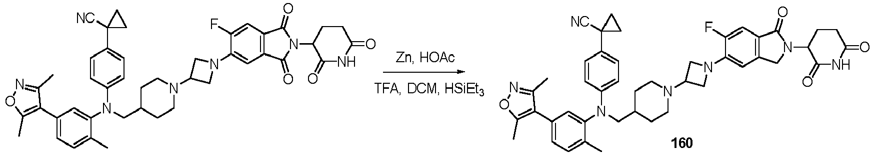

1-(4-((5-(3,5-Dimethylisoxazol-4-yl)-2-methylphenyl)((1-(1-(2-(2,6-dioxopiperidin-3)-6-fluoro -1,3-dicarbonylisoindolin-5-yl)azetidin-3-yl)piperidin-4-yl)methyl)amino)phenylcyclopropanec arbonitrile was dissolved in glacial acetic acid, to which was added zinc powder, and the mixture was allowed to react at 60 °C for 6 h. After the reaction was completed, the reaction solution was cooled, filtered through celite, and then the filter cake was rinsed with ethyl acetate several times. The filtrate was concentrated. The obtained product was dissolved in dichloromethane, to which were successively added trifluoroacetic acid and triethylsilane, and then the reaction solution was stirred overnight at room temperature. After the reaction was completed, the system was concentrated and purified by column chromatography, to provide the product (7 mg, yield 60%).

LC/MS (ESI⁺) calcd for C₄₄H₄₇FN₇O₄ ([M + H]⁺) *m*/*z* 756.4; found 756.4.

### 161: 1-(4-((5-(3,5-dimethylisoxazol-4-yl)-2-methylphenyl)(((1s,4s)-4-((2-(2,6-dioxopiperidin-3-yl)-6-fluoro-1,3-dicarbonylisoindolin-5-yl)amino)cyclohexyl)methyl)ami no)phenyl)phenylcyclopropanecarbonitrile (161)

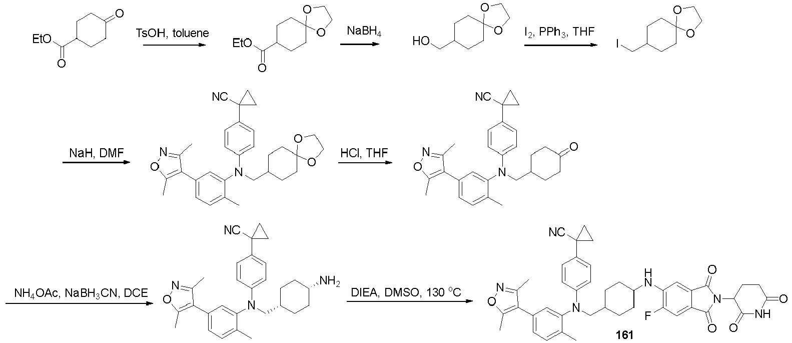

Ethyl 4-oxocyclohexanecarboxylate was dissolved in toluene, to which was added ethylene glycol and p-toluenesulfonic acid, and the mixture was allowed to react overnight at 80 °C. After the reaction was completed, the reaction system was cooled, and then the saturated solution of sodium bicarbonate was added to quench the reaction. The resultant solution was extracted with ethyl acetate, successively washed with water and saturated NaCl solution, and then concentrated, dried, and purified by column chromatography, to provide the product (5.2 g, yield 98%).

Methyl 1,4-dioxospiro[4.5]decane-8-carboxylate was dissolved in methanol, to which was added sodium borohydride in an ice bath, and after addition, the reaction solution was warmed to room temperature and stirred for 6 h. After completion of the reaction, the reaction was quenched by slowly adding the saturated solution of ammonium chloride. The resultant solution was extracted with ethyl acetate, successively washed with water and saturated NaCl solution, and then dried, concentrated, and purified by column chromatography, to provide the product (4.4 g, yield 85%).

8-(Hydroxymethyl)-1,4-dioxospiro[4.5]decane was dissolved in tetrahydrofuran, to which were added triphenylphosphine and iodine in an ice bath, and then the mixture was stirred at room temperature for 4 h. After completion of the reaction, the reaction was quenched with the solution of sodium bisulfite. The resultant solution was extracted with ethyl acetate, successively washed with water and saturated NaCl solution, and then dried, concentrated, and purified by column chromatography, to provide the product (4.6 g, yield 90%). LC/MS (ESI⁺) calcd for C₉H₆IO₂ ([M + H]⁺) *m*/*z* 283.0; found 283.0.

1-(4-(5-(3,5-Dimethylisoxazol-4-yl)-2-methylphenyl)amino)phenylcyclopropanecarbonitrile was dissolved in DMF, and the solution was cooled to 0 °C in an ice bath, to which was then added NaH. After addition, the reaction solution was warmed to room temperature and stirred for 0.5 h. Liquid seal was used to observe whether the system was still bubbling. If no bubbles were generated, 8-(iodomethyl)-1,4-dioxospiro[4.5]decane was added, and the reaction solution was stirred overnight at 60 °C. The reaction was quenched with the saturated solution of ammonium chloride. The resultant solution was extracted with ethyl acetate, successively washed with water and saturated NaCl solution, and then dried, concentrated, and purified by column chromatography, to provide the product (168 mg, yield 59%). LC/MS (ESI⁺) calcd for C₃₁H₃₆N₃O₃ ([M + H]⁺) *m*/*z* 498.3; found 498.3.

1-(4-((1,4-Dioxospiro[4.5]decane-8-methyl)(5-(3,5-dimethylisoxazol-4-yl)-2-methylphenyl)ami no))phenylcyclopropanecarbonitrile was dissolved in tetrahydrofuran, to which was added 2N hydrochloric acid, and the mixture was stirred at room temperature for 3 h. After the reaction was completed, ethyl acetate was added to quench the reaction. The resultant solution was successively washed with water, the saturated solution of sodium bicarbonate, water, and the saturated solution of NaCl, followed by drying, concentrating, and purifying by column chromatography, to provide the product (110 mg, yield 88%). LC/MS (ESI⁺) calcd for C₂₉H₃₂N₃O₂ ([M + H]⁺) *m*/*z* 454.3; found 454.3.

1-(4-((5-(3,5-Dimethylisoxazol-4-yl)-2-methylphenyl)((4-carbonylcyclohexyl)methyl)amino)ph enyl)phenylcyclopropanecarbonitrile was dissolved in dichloroethane, to which were added ammonium chloride, sodium triacetylborohydride, and glacial acetic acid, and the mixture was stirred at room temperature for 6 h. After the reaction was completed, the reaction was quenched with the saturated solution of ammonium chloride. The resultant solution was extracted with ethyl acetate, successively washed with water and saturated NaCl solution, and then dried, concentrated, and purified by column chromatography, to provide the product (52 mg, yield 68%). LC/MS (ESI⁺) calcd for C₂₉H₃₅N₄O ([M + H]⁺) *m*/*z* 455.3; found 455.3. 1-(4-((((1*s*,4*s*)-4-aminocyclohexyl)methyl)(5-(3,5-dimethylisoxazol-4-yl)-2-methylphenyl)amin o)phenyl)phenylcyclopropanecarbonitrile was dissolved in DMSO, to which were added DIEA and 5-amino-2-(2,6-dioxopiperidin-3-)-6-fluoroisoindoline-1,3-dione, and the mixture was stirred for 10 min at room temperature, followed by addition of 5-amino-2-(2,6-dioxopiperidin-3-)-6-fluoroisoindoline-1,3-dione. The mixture was allowed to react at 130 °C for 3 h. After completion of the reaction, the system was cooled to room temperature, and then the reaction was quenched with the saturated solution of ammonium chloride. The resultant solution was extracted with ethyl acetate, successively washed with water and saturated NaCl solution, and then concentrated, dried, and purified by column chromatography, to provide the product (44 mg, yield 46%).

LC/MS (ESI+) calcd for C₄₂H₄₂FN₆O₅ ([M + H]⁺) *m*/*z* 729.3; found 729.3.

### 162: 1-(4-((5-(3,5-Dimethylisoxazol-4-yl)-2-methylphenyl)(((1s,4s)-4-((2-(2,6-dioxopiperidin-3)-6-fluoro-3-isoindolinone-5-yl)amino)cyclohexyl)methyl)amino)phenyl)p henylcyclopropanecarbonitrile (162)

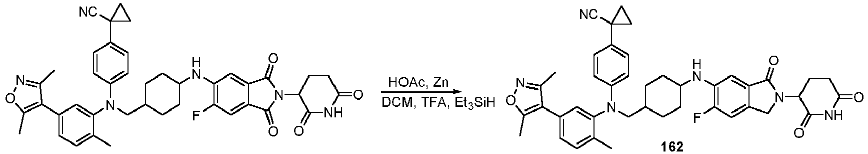

1-(4-((5-(3,5-Dimethylisoxazol-4-yl)-2-methylphenyl)(((1*s*,4*s*)-4-((2-(2,6-dioxopiperidin-3)-6-f luoro-1,3-dicarbonylisoindolin-5-yl)amino)cyclohexyl)methyl)amino)phenyl)phenylcyclopropa necarbonitrile was dissolved in glacial acetic acid, to which was added zinc powder, and the mixture was allowed to react at 60 °C for 6 h. After the reaction was completed, the reaction solution was cooled, filtered through celite, and then the filter cake was rinsed with ethyl acetate several times. The filtrate was concentrated. The obtained product was dissolved in dichloromethane, to which were successively added trifluoroacetic acid and triethylsilane, and then the reaction solution was stirred overnight at room temperature. After the reaction was completed, the system was concentrated and purified by column chromatography, to provide the product (5 mg, yield 15%).

LC/MS (ESI⁺) calcd for C₄₂H₄₄FN₆O₄ ([M + H]⁺) *m*/*z* 715.3; found 715.3.

### 163: 1-(4-((5-(3,5-Dimethylisoxazol-4-yl)-2-methylphenyl)(((1s,4s)-4-((2-(2,6-dioxopiperidin-3)-6-fluoro-1-isoindolinone-5-yl)amino)cyclohexyl)methyl)amino)phenyl)p henylcyclopropanecarbonitrile (163)

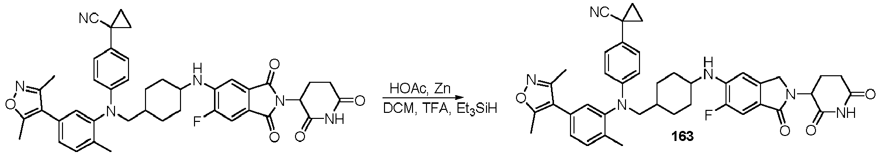

1-(4-((5-(3,5-Dimethylisoxazol-4-yl)-2-methylphenyl)(((1*s*,4*s*)-4-((2-(2,6-dioxopiperidin-3)-6-f luoro-1,3-dicarbonylisoindolin-5-yl)amino)cyclohexyl)methyl)amino)phenyl)phenylcyclopropa necarbonitrile was dissolved in glacial acetic acid, to which was added zinc powder, and the mixture was allowed to react at 60 °C for 6 h. After the reaction was completed, the reaction solution was cooled, filtered through celite, and then the filter cake was rinsed with ethyl acetate several times. The filtrate was concentrated. The obtained product was dissolved in dichloromethane, to which were successively added trifluoroacetic acid and triethylsilane, and then the reaction solution was stirred overnight at room temperature. After the reaction was completed, the system was concentrated and purified by column chromatography, to provide the product (5 mg, yield 15%).

LC/MS (ESI⁺) calcd for C₄₂H₄₄FN₆O₄ ([M + H]⁺) *m*/*z* 715.3; found 715.3.

### 164: 2-Chloro-4-((5-(3,5-dimethylisoxazol-4-yl)-2-methylphenyl)(4-((2-(2,6-dioxopiperidin-3)-6-fluoro-1,3-dioxyisoindolin-5-yl)amino)butyl)amino)benzonitrile (164)

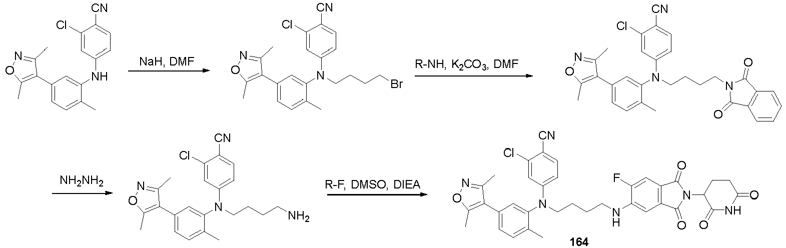

2-Chloro-4-((5-(3,5-dimethylisoxazol-4-yl)-2-methylphenyl)amino)benzonitrile was dissolved in DMF, and the solution was cooled to 0 °C in an ice bath, to which was then added NaH. After addition, the reaction solution was warmed to room temperature and stirred for 0.5 h. Liquid seal was used to observe whether the system was still bubbling. If no bubbles were generated, 1,5-dibromopentane was added, and the reaction solution was stirred overnight at room temperature. After completion of the reaction, the reaction was quenched with the saturated solution of ammonium chloride. The resultant solution was extracted with ethyl acetate, successively washed with water and saturated NaCl solution, and then dried, concentrated, and purified by column chromatography, to provide the product (840 mg, yield 82%). LC/MS (ESI⁺) calcd for C₂₃H₂₄BrClN₃O ([M + H]⁺) *m*/*z* 472.1; found 472.1.

4-((4-Bromobutyl)(5-(3,5-dimethylisoxazol-4-yl)-2-methylphenyl)amino)-2-chlorobenzonitrile was dissoved in DMF, to which were added potassium carbonate and phthalimide, and the mixture was stirred for 3 h at room temperature. After completion of the reaction, the reaction was quenched with ethyl acetate. The reaction solution was washed with water and saturated NaCl solution, and then dried, concentrated, and purified by column chromatography, to provide the product (950 mg, yield 90%). LC/MS (ESI⁺) calcd for C₃₁H₂₈ClN₄O₃ ([M + H]⁺) *m*/*z* 539.2; found 539.2.

2-Chloro-4-((5 -(3,5-dimethylisoxazol-4-yl)-2-methylphenyl)(4-(1,3-dicarbonylisoindolin-2-yl)b utyl)amino)benzonitrile was dissolved in THF, to which was added hydrazine hydrate, and the mixture was stirred for 3 h at room temperature. After completion of the reaction, the reaction was quenched with ethyl acetate. The reaction solution was successively washed several times with water, and then washed with saturated NaCl solution, followed by concentrating and purifying by column chromatography, to provide the product (700 mg, yield 80%). LC/MS (ESI⁺) calcd for C₂₃H₂₆ClN₄O ([M + H]⁺) *m*/*z* 409.2; found 409.2.

4-((4-Aminobutyl)(5-(3,5-dimethylisoxazol-4-yl)-2-methylphenyl)amino)-2-chlorobenzonitrile and 5-amino-2-(2,6-dioxopiperidin-3-)-6-fluoroisoindoline-1,3-dione were dissolved in DMSO, to which was added DIEA, and the mixture was allowed to react at 130 °C for 3 h. After the reaction was completed, the system was cooled to room temperature, and then the reaction was quenched with the saturated solution of ammonium chloride. The resultant solution was extracted with ethyl acetate, successively washed with water and saturated NaCl solution, and then concentrated, dried, and purified by column chromatography, to provide the product (320 mg, yield 86%). LC/MS (ESI⁺) calcd for C₃₆H₃₃FClN₆O₅ ([M + H]⁺) *m*/*z* 683.2; found 683.2. ¹H NMR (400 MHz, CDCl₃) *δ* 8.11 (s, 1H), 7.45 (d, *J* = 7.6 Hz, 1H), 7.39 (dd, *J* = 10.0, 6.4 Hz, 2H), 7.22 (dd, *J* = 10.0, 3.6 Hz, 1H), 7.05 (d, *J* = 7.2 Hz, 1H), 6.97 (d, *J* = 1.6 Hz, 1H), 6.50 (d, *J* = 2.0 Hz, 1H), 6.36 (dd, *J* = 8.8, 1.6 Hz, 1H), 4.98 (m, 1H), 3.32 (t, *J* = 6.8 Hz, 2H), 2.93 (m, 4H), 2.40 (s, 3H), 2.26 (s, 3H), 2.14 (m, 4H), 1.79 (m, 6H).

### 165: 2-Chloro-4-((5-(3,5-dimethylisoxazol-4-yl)-2-methylphenyl)(4-((2-(2,6-dioxopiperidin-3)-1,3-dioxyisoindolin-5-yl)amino)butyl)amino)benzonitrile (165)

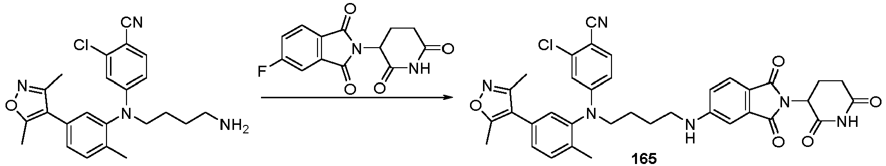

4-((4-Aminobutyl)(5-(3,5-dimethylisoxazol-4-yl)-2-methylphenyl)amino)-2-chlorobenzonitrile and 5-amino-2-(2,6-dioxopiperidin-3-)isoindoline-1,3-dione were dissolved in DMSO, to which was added DIEA, and the mixture was allowed to react at 130 °C for 3 h. After the reaction was completed, the system was cooled to room temperature, and then the reaction was quenched with the saturated solution of ammonium chloride. The resultant solution was extracted with ethyl acetate, successively washed with water and saturated NaCl solution, and then concentrated, dried, and purified by column chromatography, to provide the product (114 mg, yield 86%). LC/MS (ESI⁺) calcd for C₃₆H₃₄ClN₆O₅ ([M + H]⁺) *m*/*z* 665.2; found 665.2. ¹H NMR (400 MHz, CDCl₃) *δ* 8.11 (s, 1H), 7.45 (d, *J* = 7.6 Hz, 1H), 7.39 (dd, *J* = 10.0, 6.4 Hz, 2H), 7.22 (dd, *J* = 10.0, 3.6 Hz, 1H), 7.05 (d, *J* = 7.2 Hz, 1H), 6.97 (m, 2H), 6.50 (d, *J* = 2.0 Hz, 1H), 6.36 (dd, *J* = 8.8, 1.6 Hz, 1H), 4.98 (m, 1H), 3.32 (t, *J* = 6.8 Hz, 2H), 2.93 (m, 4H), 2.40 (s, 3H), 2.26 (s, 3H), 2.14 (m, 4H), 1.79 (m, 6H).

### 166: 2-Chloro-4-((5-(3,5-dimethylisoxazol-4-yl)-2-methylphenyl)(4-((2-(2,6-dioxopiperidin-3)-6-fluoro-3-isoindolinone-5-yl)amino)butyl)amino)benzonitrile (166)

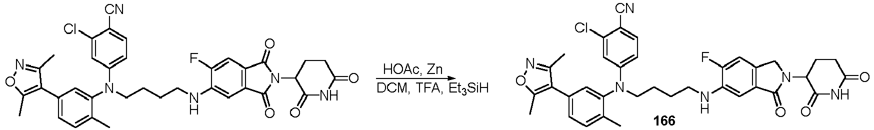

2-Chloro-4-((5-(3,5-dimethylisoxazol-4-yl)-2-methylphenyl)(4-((2-(2,6-dioxopiperidin-3)-6-flu oro-1,3-dicarbonylisoindolin-5-yl)amino)butyl)amino)benzonitrile was dissolved in glacial acetic acid, to which was added zinc powder, and the mixture was allowed to react at 60 °C for 6 h. After the reaction was completed, the reaction solution was cooled, filtered through celite, and then the filter cake was rinsed with ethyl acetate several times. The filtrate was concentrated. The obtained product was dissolved in dichloromethane, to which were successively added trifluoroacetic acid and triethylsilane, and then the reaction solution was stirred overnight at room temperature. After the reaction was completed, the system was concentrated and purified by column chromatography, to provide the product (50 mg, yield 20%). LC/MS (ESI⁺) calcd for C₃₆H₃₅ClFN₆O₄ ([M + H]⁺) *m*/*z* 669.2; found 669.2. ¹H NMR (400 MHz, CDCl₃) *δ* 8.33 (s, 1H), 7.37 (m, 3H), 7.20 (m, 1H), 6.97 (m, 1H), 6.66 (d, *J* = 7.2 Hz, 1H), 6.50 (s, 1H), 6.34 (m, 1H), 5.18 (dd, *J* = 13.2, 5.0 Hz, 1H), 4.30 (dd, *J* = 58.1, 15.5 Hz, 2H), 3.67 (m, 2H), 3.21 (m, 2H), 2.80 (m, 3H), 2.39 (s, 3H), 2.26 (s, 3H), 2.19 (m, 4H), 1.72 (m, 5H).

### 167: 2-Chloro-4-((5-(3,5-dimethylisoxazol-4-yl)-2-methylphenyl)(4-((2-(2,6-dioxopiperidin-3)-6-fluoro-1-isoindolinone-5-yl)amino)butyl)amino)benzonitrile (167)

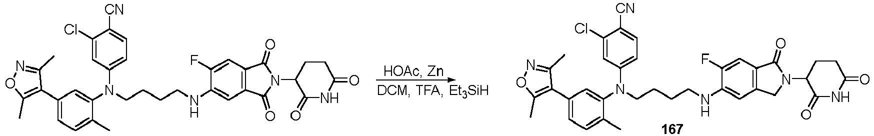

2-Chloro-4-((5-(3,5-dimethylisoxazol-4-yl)-2-methylphenyl)(4-((2-(2,6-dioxopiperidin-3)-6-flu oro-1,3-dicarbonylisoindolin-5-yl)amino)butyl)amino)benzonitrile was dissolved in glacial acetic acid, to which was added zinc powder, and the mixture was allowed to react at 60 °C for 6 h. After the reaction was completed, the reaction solution was cooled, filtered through celite, and then the filter cake was rinsed with ethyl acetate several times. The filtrate was concentrated. The obtained product was dissolved in dichloromethane, to which were successively added trifluoroacetic acid and triethylsilane, and then the reaction solution was stirred overnight at room temperature. After the reaction was completed, the system was concentrated and purified by column chromatography, to provide the product (150 mg, yield 60%). LC/MS (ESI⁺) calcd for C₃₆H₃₅ClFN₆O₄ ([M + H]⁺) *m*/*z* 669.2; found 669.2. ¹H NMR (400 MHz, CDCl₃) *δ* 8.32 (s, 1H), 7.39 (m, 3H), 7.20 (m, 1H), 6.97 (m, 1H), 6.66 (d, *J* = 7.2 Hz, 1H), 6.50 (s, 1H), 6.34 (m, 1H), 5.18 (dd, *J* = 13.2, 5.0 Hz, 1H), 4.30 (dd, *J* = 58.1, 15.5 Hz, 2H), 3.67 (m, 2H), 3.21 (m, 2H), 2.80 (m, 3H), 2.39 (s, 3H), 2.19 (m, 7H), 1.75 (m, 5H).

### 168: 1-(4-((5-(3,5-Dimethylisoxazol-4-yl)-2-methylphenyl)(((1r,4r)-4-(((2-(2,6-dioxopiperidin-3)-6-fluoro-3-isoindolinone-5-yl)amino)methyl)cyclohexyl)methyl)amino)p henylcyclopropanecarbonitrile (168)

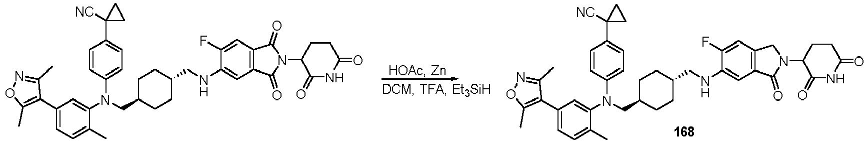

1-(4-((5-(3,5-Dimethylisoxazol-4-yl)-2-methylphenyl)(((1*r*,4*r*)-4-(((2-(2,6-dioxopiperidin-3)-6-fluoro-1,3-dicarbonylisoindolin-5-yl)amino)methyl)cyclohexyl)methyl)amino)phenylcycloprop anecarbonitrile was dissolved in glacial acetic acid, to which was added zinc powder, and the mixture was allowed to react at 60 °C for 6 h. After the reaction was completed, the reaction solution was cooled, filtered through celite, and then the filter cake was rinsed with ethyl acetate several times. The filtrate was concentrated. The obtained product was dissolved in dichloromethane, to which were successively added trifluoroacetic acid and triethylsilane, and then the reaction solution was stirred overnight at room temperature. After the reaction was completed, the system was concentrated and purified by column chromatography, to provide the product (8 mg, yield 22%). LC/MS (ESI⁺) calcd for C₄₃H₄₆FN₆O₄ ([M + H]⁺) *m*/*z* 729.4; found 729.4. ¹H NMR (400 MHz, CDCl₃) *δ* 8.02 (s, 1H), 7.34 (t, *J* = 7.1 Hz, 2H), 7. 08 (m, 5H), 6.44 (m, 2H), 5.18 (dd, *J* = 13.3, 5.2 Hz, 1H), 4.28 (dd, *J* = 51.8, 15.6 Hz, 2H), 3.44 (d, *J* = 6.7 Hz, 2H), 3.04 (d, *J* = 3.6 Hz, 2H), 2.85 (m, 2H), 2.41 (s, 3H), 2.28 (s, 3H), 2.07 (s, 3H), 1.92 (m, 3H), 1.58 (m, 2H), 1.28 (m, 8H), 0.85 (m, 4H).

### 169: 1-(4-((5-(3,5-Dimethylisoxazol-4-yl)-2-methylphenyl)(((1r,4r)-4-(((2-(2,6-dioxopiperidin-3)-6-fluoro-1-isoindolinone-5-yl)amino)methyl)cyclohexyl)methyl)amino)p henylcyclopropanecarbonitrile (169)

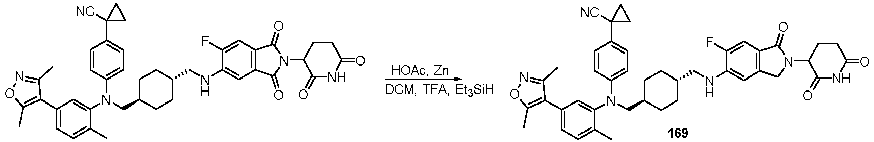

1-(4-((5-(3,5-Dimethylisoxazol-4-yl)-2-methylphenyl)(((1r,4*r*)-4-(((2-(2,6-dioxopiperidin-3)-6-fluoro-1,3-dicarbonylisoindolin-5-yl)amino)methyl)cyclohexyl)methyl)amino)phenylcycloprop anecarbonitrile was dissolved in glacial acetic acid, to which was added zinc powder, and the mixture was allowed to react at 60 °C for 6 h. After the reaction was completed, the reaction solution was cooled, filtered through celite, and then the filter cake was rinsed with ethyl acetate several times. The filtrate was concentrated. The obtained product was dissolved in dichloromethane, to which were successively added trifluoroacetic acid and triethylsilane, and then the reaction solution was stirred overnight at room temperature. After the reaction was completed, the system was concentrated and purified by column chromatography, to provide the product (16 mg, yield 44%). LC/MS (ESI⁺) calcd for C₄₃H₄₆FN₆O₄ ([M + H]⁺) *m*/*z* 729.4; found 729.4. ¹H NMR (400 MHz, CDCl₃) *δ* 8.01 (s, 1H), 7.41 (d, *J* = 10.4 Hz, 1H), 7. 35 (d, *J* = 7.8 Hz, 1H), 7.06 (m, 4H), 7.63 (m, 1H), 6.46 (m, 2H), 5.16 (dd, *J* = 13.2, 5.0 Hz, 1H), 4.28 (dd, *J* = 58.1, 15.5 Hz, 2H), 3.46 (d, *J* = 6.7 Hz, 2H), 3.04 (d*, J* = 3.6 Hz, 2H), 2.85 (m, 2H), 2.41 (s, 3H), 2.28 (s, 3H), 2.08 (s, 3H), 1.94 (m, 3H), 1.59 (m, 2H), 1.27 (m, 8H), 0.85 (m, 4H).

### 170: 1-(4-((5-(3,5-Dimethylisoxazol-4-yl)-2-methylphenyl)(2-(2,6-dioxopiperidin-3-yl)-6-fluoro-1,3-dioxoidindolin-5-yl)-2-azaspiro[3.3]heptan-6-yl)amino)phenyl)cyclopropane-1-c arbonitrile

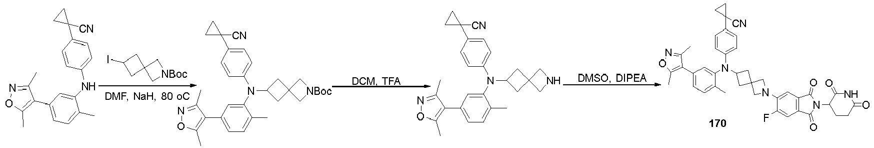

The starting compound (686.86 mg, 2 mmol) was added into 5 mL DMF, and then NaH (160 mg, 4 mmol) was added in an ice bath. After the mixture was stirred for 0.5 h, t-butyl 6-iodo-2-azaspiro[3.3]heptane-2-carboxylate (1.29 g, 4 mmol) and catalytic equivalent NaI (37.18 mg, 0.2 mmol) were added. After addition, the reaction solution was heated to 80 °C and allowed to react for 15 h. After the reaction was completed, the reaction solution was cooled to room temperature, to which were added 20 ml of water and 30 ml of ethyl acetate for extraction. The obtained organic phase was evaporated under reduced pressure to remove the solvent. The crude product was purified by column chromatography (PE:EA = 3:1), to obtain 250 mg of intermediate t-butyl 6-((4-(1-cyanocyclopropyl)phenyl)(5-(3,5-dimethylisoxazol-4-yl)-2-methylphenyl)amino)-2-azaspiro[3.3]heptane-2-carboxylate, with a yield of 23.1%. MS (ES): *m*/*z* 539 [M+H]⁺.

Compound t-buytl 6-((4-(1-cyanocyclopropyl)phenyl)(5-(3,5-dimethylisoxazol-4-yl)-2-methylphenyl)amino)-2-azaspiro[3.3]heptane-2-carboxylate (215 mg, 0.4 mmol) was dissolved in 6 mL of dichlormethane, to which was added 3 mL of trifluoroacetic acid, and then the reaction solution was stirred for 2 h at room temperature. The reaction was completed, and then the solvent was removed by evaporation under reduced pressure. The product was directly used in the next step without further purification.

Compound 6-((4-(1-cyanocyclopropyl)phenyl)(5 -(3,5-dimethylisoxazol-4-yl)-2-methylphenyl) amino)-2-azaspiro[3.3]heptane-2-carboxylic acid (intermediate **28-3**) (121 mg, 0.25 mmol) was dissolved in 3 mL of DMSO, to which was added 2-(2, 6-dioxopiperidin-3-yl)-5,6-difluoroisonicotinamide-1,3-dione (74 mg, 0.25 mmol). Then, the reaction solution was heated to 130 °C, and stirred for 2 h. The reaction was completed. The reaction solution was cooled to room temperature, to which were added 10 ml of water and 20 ml of ethyl acetate for extraction. The obtained organic phase was evaporated under reduced pressure to remove the solvent. The crude product was purified by TLC (DCM: MeOH = 20:1), to provide 105 mg of the final target product, with a yield of 59%. MS (ES): *m*/*z* 713 [M+H]⁺. ¹H NMR (400 MHz, DMSO-d₆) *δ* 11.09 (s, 1H), 8.17 (t, *J* = 7.7 Hz, 1H), 7.59 (d, *J* = 11.1 Hz, 1H), 7.49 (d, *J* = 8.0 Hz, 1H), 7.33 (s, 1H), 7.19-7.02 (m, 2H), 6.85 (d, *J* = 7.7 Hz, 1H), 6.42 (d, *J* = 8.7 Hz, 2H), 5.22-5.15 (m, 1H), 5.10-5.02 (m, 1H), 4.31 (s, 2H), 4.01 (s, 2H), 2.97-2.81 (m, 2H), 2.65 (d, *J* = 10.5 Hz, 2H), 2.41 (s, 3H), 2.24 (s, 3H), 2.10 (s, 3H), 2.01 (d, *J* = 11.7 Hz, 4H), 1.66-1.55 (m, 2H), 1.34-1.29 (m, 2H).

### 171: 1-(4-((5-(3,5-Dimethylisoxazol-4-yl)-2-methylphenyl)(3-((2-(2,6-dioxopiperidin-3-yl)-6-fluoro-1,3-dioxoidindolin-5-yl)amino)propyl)amino)phenyl)cyclopropane-1-nitrile

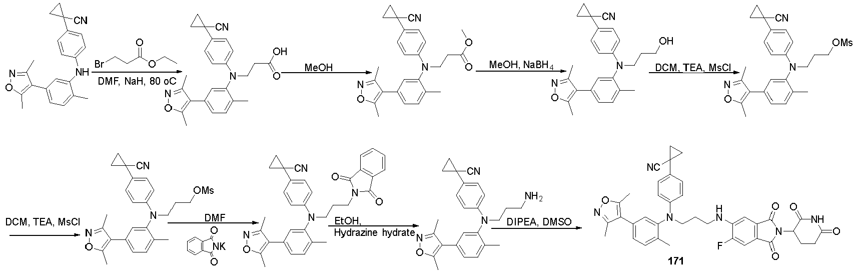

The starting compound (686.86 mg, 2 mmol) was added into 5 mL DMF, and then NaH (160 mg, 4 mmol) was added in an ice bath. After the mixture was stirred for 0.5 h, ethyl bromopropanoate (0.724 g, 4 mmol) and catalytic equivalent NaI (37.18 mg, 0.2 mmol) were added. After addition, the reaction solution was heated to 80 °C and allowed to react for 15 h. After the reaction was completed, the reaction solution was cooled to room temperature, to which were added 20 ml of water and 30 ml of ethyl acetate for extraction. The obtained organic phase was evaporated under reduced pressure to remove the solvent. The crude product was purified by column chromatography (PE:EA = 1:1), to obtain 450 mg of intermediate 3-((4-(1-cyanocyclopropyl)phenyl)(5-(3,5-dimethylisoxazol-4-yl)-2-methylphenyl)amino)propi onic acid, with a yield of 54.2%. MS (ES): *m*/*z* 416 [M+H]⁺.

The product obtained in the previous step (415 mg, 1 mmol) was dissolved in 10 ml of dichloromethane, to which was added oxalyl chloride (140 mg, 1.1 mmol) in an ice bath, and then the mixture was stirred for 1 h. After completion of the reaction, the reaction solution was drop added into anhydrous methanol, and the resultant solution was further stirred for 0.5 h. After the reaction was completed, 5 ml of water and 20 ml of ethyl acetate were added for extraction. The obtained organic phase was evaporated under reduced pressure to remove the solvent. The crude product was purified by TLC (PE:EA = 3:1), to provide 408 mg of intermediate methyl 3-((4-(1-cyanocyclopropyl)phenyl)(5-(3,5-dimethylisoxazol-4-yl)-2-methylphenyl)amino)propionate, with a yield of 94.9%. MS(ES): *m*/*z* 430 [M+H]⁺.

The intermediate (430 mg, 1 mmol), obtained in the previous step, was dissolved in 10 ml of anhydrous methanol, to which was added sodium borohydride (76 mg, 2.0 mmol), and the mixture was stirred for 6 h. After the reaction was completed, 5 ml of water and 20 ml of ethyl acetate were added for extraction. The obtained organic phase was evaporated under reduced pressure to remove the solvent. The crude product 1-(4-((5-(3, 5-dimethylisoxazol-4-yl)-2-methylphenyl)(3-hydroxypropyl)amino)phenyl)cyclopropane-1-nitr ile was directly used in the next step without further purification.

The intermediate (402 mg, 1 mmol), obtained in the previous step, was dissolved in 10 ml of DCM, to which was added TEA (152 mg, 1.5 mmol). Then, methylsulfonyl chloride (137 mg, 1.2 mmol) was added dropwise in an ice bath, and the mixture was stirred for 3 h. After the reaction was completed, 5 ml of water and 20 ml of dichloromethane were added for extraction. The obtained organic phase was evaporated under reduced pressure to remove the solvent. The crude product propanyl 3-((4-(1-cyanocyclopropyl)phenyl)(5-(3,5-dimethylisoxazol-4-yl)-2-methylphenyl)amino)methylsulfonate was directly used in the next step without further purification.

The intermediate (480 mg, 1 mmol), obtained in the previous step, was dissolved in 5 ml of DMF, to which was added potassium phthalimide (278 mg, 1.5 mmol), and then the reaction solution was heated to 80 °C. The mixture was allowed to react 3 h under stirring. After the reaction was completed, 10 ml of water and 30 ml of dichloromethane were added for extraction. The obtained organic phase was evaporated under reduced pressure to remove the solvent. The crude product was purified by TLC (PE:EA = 3:1), to provide 478 mg of intermediate 1-(4-((5-(3,5-dimethylisoxazol-4-yl)-2-methylphenyl)(3-(1,3-dioxoisobutanol-2-yl)propyl)amino)phenyl)cyclopropane-1-nitrile, with a yield of 90%. MS(ES): *m*/*z* 531 [M+H]⁺.

The intermediate (531 mg, 1 mmol), obtained in the previous step, was dissolved in 10 ml of absolute ethanol, to which was added hydrazine hydrate (75 mg, 1.5 mmol). The reaction solution was heatd to 80 °C, and allowed to react for 3 h under stirring. After the reaction was completed, the reaction solution was cooled, and then filtered to remove the insoluble matter. 10 ml of water and 30 ml of ethyl acetate were added to the filtrate for extraction. The obtained organic phase was evaporated under reduced pressure to remove the solvent. The crude product 1-(4-((3-aminopropyl)(5-(3,5-dimethylisoxazol-4-yl)-2-methylphenyl)amino)phenyl)cycloprop ane-1-nitrile was directly used in the next step without further purification.

Compound 1-(4-(((1s,2s)-2-(aminomethyl)cyclopropyl)methyl)(5-(3,5-dimethylisoxazol-4-yl)-2-methylphenyl)amino)phenyl)cyclopropane-1-nitrile (100 mg, 0.25 mmol) was dissolved in 3 mL of DMSO, to which was added 2-(2,6-dioxopiperidin-3-yl)-5,6-difluoroisonicotinamide-1,3-dione (74 mg, 0.25 mmol). The reaction solution was heatd to 130 °C, and stirred for 2 h. The reaction was completed. The reaction solution was cooled to room temperature, to which were added 10 ml of water and 20 ml of ethyl acetate for extraction. The obtained organic phase was evaporated under reduced pressure to remove the solvent. The crude product was purified by TLC (DCM: MeOH = 20:1), to provide 115 mg of target product, with a yield of 68%. MS(ES): *m*/*z* 675 [M+H]⁺. ¹H NMR (400 MHz, DMSO-d₆) *δ* 11.09 (s, 1H), 7.55 (d, *J* = 10.3 Hz, 1H), 7.44 (d, *J* = 7.9 Hz, 1H), 7.25 (dd, *J* = 7.8, 1.6 Hz, 1H), 7.16 (d, *J* = 7.2 Hz, 1H), 7.10 (d, *J* = 8.9 Hz, 2H), 6.95 (s, 1H), 6.50 (d, *J* = 8.8 Hz, 2H), 5.06 (dd, *J* = 12.8, 5.3 Hz, 1H), 4.03 (q, *J* = 7.1 Hz, 1H), 3.78 - 3.66 (m, 2H), 2.96-2.82 (m, 1H), 2.68-2.55 (m, 1H), 2.35 (s, 3H), 2.18 (s, 3H), 2.07 (s, 3H), 2.01 (d, *J* = 12.5 Hz, 2H), 1.97-1.89 (m, 2H), 1.59 (q, *J* = 4.6 Hz, 2H), 1.31 (q, *J* = 4.9 Hz, 2H), 1.18 (t, *J* = 7.1 Hz, 2H).

### 172: 1-(4-((5-(3,5-Dimethylisoxazol-4-yl)-2-methylphenyl)(3-((2-(2,6-dioxopiperidin-3-yl)-1,3-dioxoisoindolin-5-yl)amino)propyl)amino)phenyl)cyclopropane-1-nitrile

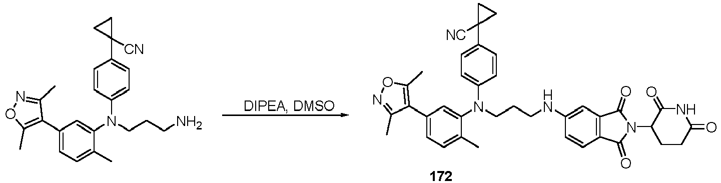

Compound 1-(4-(((1s,2s)-2-(aminomethyl)cyclopropyl)methyl)(5-(3,5-dimethylisoxazol-4-yl)-2-methylphenyl)amino)phenyl)cyclopropane-1-nitrile (intermediate **29-6**) (100 mg, 0.25 mmol) was dissolved in 3 mL of DMSO, to which was added 2-(2, 6-dioxopiperidin-3-yl)-5-fluoroisoindolin-1,3-dione (69 mg, 0.25 mmol). The reaction solution was heatd to 130 °C, and stirred for 2 h. The reaction was completed. The reaction solution was cooled to room temperature, to which were added 10 ml of water and 20 ml of ethyl acetate for extraction. The obtained organic phase was evaporated under reduced pressure to remove the solvent. The crude product was purified by TLC (DCM: MeOH = 20:1), to provide 120 mg of final product HC-4065-01, with a yield of 73.1%. MS(ES): *m*/*z* 657 [M+H]⁺. ¹H NMR (400 MHz, DMSO-d6) *δ* 11.06 (s, 1H), 7.54 (d, *J* = 8.3 Hz, 1H), 7.45 (d, *J* = 7.8 Hz, 1H), 7.25 (d, *J* = 7.7 Hz, 1H), 7.11 (d, *J* = 10.5 Hz, 4H), 6.94 (s, 1H), 6.82 (d, *J* = 8.5 Hz, 1H), 6.51 (d, *J* = 8.7 Hz, 2H), 5.03 (s, 2H), 3.79-3.70 (m, 3H), 2.36 (s, 3H), 2.18 (s, 3H), 2.07 (s, 3H), 1.95 (d, *J* = 33.4 Hz, 4H), 1.59 (dd, *J* = 7.2, 4.7 Hz, 2H), 1.31 (dd, *J* = 7.5, 4.9 Hz, 2H), 1.24 (s, 2H).

### 173: 1-(4-((5-(3,5-dimethylisoxazol-4-yl)-2-methylphenyl)(2-((1-(2-(2,6-dioxopiperidin-3-yl)-6-fl uoro-1,3-dioxoisoindolin-5-yl)azacyclobutane-3-yl)oxy)propyl)amino)phenyl)cyclopropane -1-nitrile

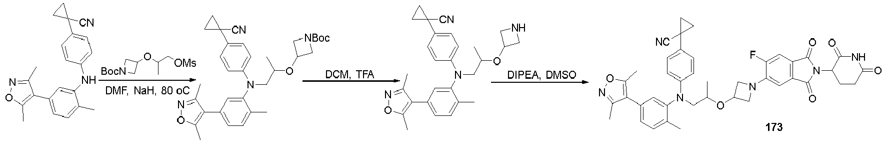

The starting compound (686.86 mg, 2 mmol) was added into 5 mL DMF, and then NaH (160 mg, 4 mmol) was added in an ice bath. After the mixture was stirred for 0.5 h, t-butyl 3-((1-((methanesulfonyl)oxy)propane-2-oxy)azetidine-1-carboxylate (1.24 g, 4 mmol) and catalytic equivalent NaI (37.18 mg, 0.2 mmol) were added. After addition, the reaction solution was heated to 80 °C and allowed to react for 15 h. After the reaction was completed, the reaction solution was cooled to room temperature, to which were added 20 ml of water and 30 ml of ethyl acetate for extraction. The obtained organic phase was evaporated under reduced pressure to remove the solvent. The crude product was purified by column chromatography (PE:EA = 3:1), to obtain 250 mg of intermediate t-butyl 3-((1-((4-(1-cyanocyclopropyl) phenyl)(5-(3,5-dimethylisoxazol-4-yl)-2-methylphenyl)amino)propan-2-yl)oxy)azetidine-1-car boxylate, with a yield of 22.5%. MS(ES): *m*/*z* 557 [M+H]⁺.

Compound t-butyl 3-((1-((4-(1-cyanocyclopropyl)phenyl)(5 -(3,5-dimethylisoxazol-4-yl) -2-methylphenyl)amino)propan-2-yl)oxy)azetidine-1-carboxylate (intermediate **31-1**) (223 mg, 0.4 mmol) was dissolved in 6 ml of DCM, to which was added 3 mL of trifluoroacetic acid. The mixture was stirred for 2 h at room temperature. The reaction was completed. The solvent was removed by evaporation under reduced pressure. The crude product 1-(4-((2-(azacyclobutane-3-oxy)propyl)(5-(3,5-dimethylisoxazol-4-yl)-2-methylphenyl)amino) phenyl)cyclopropane-1-nitrile was directly used in the next step without further purification. Compound 1-(4-(((1s,2s)-2-(aminomethyl)cyclopropyl)methyl)(5-(3,5-dimethylisoxazol-4-yl)-2-methylphenyl)amino)phenyl)cyclopropane-1-nitrile (intermediate **31-2**) (114 mg, 0.25 mmol) was dissolved in 3 mL of DMSO, to which was added 2-(2,6-dioxopiperidin-3-yl)-5,6-difluoroisonicotinamide-1,3-dione (74 mg, 0.25 mmol). The reaction solution was heatd to 130 °C, and stirred for 2 h. The reaction was completed. The reaction solution was cooled to room temperature, to which were added 10 ml of water and 20 ml of ethyl acetate for extraction. The obtained organic phase was evaporated under reduced pressure to remove the solvent. The crude product was purified by TLC (DCM: MeOH = 20:1), to provide 95 mg of final product, with a yield of 52%. MS(ES): *m*/*z* 731 [M+H]⁺. ¹H NMR (400 MHz, DMSO-d₆) *δ* 11.09 (s, 1H), 7.56 (d, *J* = 11.1 Hz, 1H), 7.39 (d, *J* = 7.9 Hz, 1H), 7.30-7.15 (m, 2H), 7.11 (d, *J* = 8.7 Hz, 2H), 6.82 (d, *J* = 7.5 Hz, 1H), 6.52 (d, *J* = 8.8 Hz, 2H), 5.07 (dd, *J* = 12.8, 5.3 Hz, 1H), 4.36 (dd, *J* = 55.1, 47.9 Hz, 3H), 3.91-3.54 (m, 5H), 2.96-2.79 (m, 2H), 2.59 (d, *J* = 18.0 Hz, 2H), 2.36 (s, 3H), 2.18 (s, 3H), 2.02 (s, 3H), 1.60 (dd, *J* = 7.1, 4.5 Hz, 2H), 1.32 (dd, *J* = 7.2, 4.7 Hz, 2H), 1.16 (d, *J* = 6.0 Hz, 3H).

### 174: 1-(4-((5-(3,5-Dimethylisoxazol-4-yl)-2-methylphenyl)(2-((1-(2-(2,6-dioxopiperidin-3-yl)-1,3-dioxoisoindolin-5-yl)azacyclobutane-3-yl)oxy)propyl)amino)phenyl)cyclopropane -1-nitrile

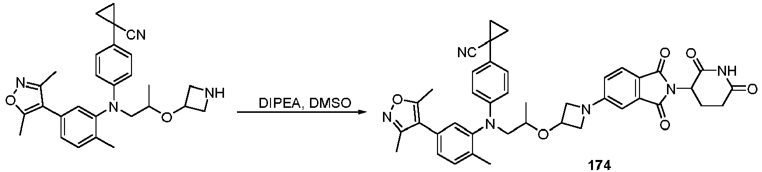

Compound 1-(4-(((1s,2s)-2-(aminomethyl)cyclopropyl)methyl)(5-(3,5-dimethylisoxazol-4-yl)-2-methylphenyl)amino)phenyl)cyclopropane-1-nitrile (intermediate **31-2**) (114 mg, 0.25 mmol) was dissolved in 3 mL of DMSO, to which was added 2-(2,6-dioxopiperidin-3-yl)-5-difluoroisindoline-1,3-dione (69 mg, 0.25 mmol). The reaction solution was heatd to 130 °C, and stirred for 2 h. The reaction was completed. The reaction solution was cooled to room temperature, to which were added 10 ml of water and 20 ml of ethyl acetate for extraction. The obtained organic phase was evaporated under reduced pressure to remove the solvent. The crude product was purified by TLC (DCM: MeOH = 20:1), to provide 92 mg of final product, with a yield of 51.7%. MS(ES): *m*/*z* 713 [M+H]⁺. ¹H NMR (400 MHz, DMSO-d₆) *δ* 11.09 (s, 1H), 7.61 (d, *J* = 8.3 Hz, 1H), 7.39 (d*, J* = 7.9 Hz, 1H), 7.24 (d, *J* = 8.0 Hz, 2H), 7.19 (m, 1H), 7.11 (d*, J* = 8.7 Hz, 2H), 6.70 (s, 1H), 6.54 (dd, *J* = 17.8, 8.6 Hz, 2H), 5.36-5.28 (m, 1H), 5.09-5.03 (m, 1H), 4.57-4.50 (m, 1H), 4.29-4.22 (m, 1H), 4.16-4.09 (m, 1H), 3.68 (s, 5H), 2.36 (s, 3H), 2.18 (s, 3H), 2.01 (s, 3H), 1.63-1.56 (m, 2H), 1.34-1.28 (m, 2H), 1.24 (s, 3H), 1.17 (d, *J* = 6.1 Hz, 3H).

### 175: 1-(4-((5-(3,5-dimethylisoxazol-4-yl)-2-methylphenyl)(3-((2-(2,6-dioxopiperidin-3-yl)-6-fluoro-3-oxoisoindolin-5-yl)amino)propyl)amino)phenyl)cyclopropane-1-nitrile

### 176: 1-(4-((5-(3,5-dimethylisoxazol-4-yl)-2-methylphenyl)(3-((2-(2,6-dioxopiperidin-3-yl)-6-fluoro-1-oxoisoindolin-5-yl)amino)propyl)amino)phenyl)cyclopropane-1-nitrile

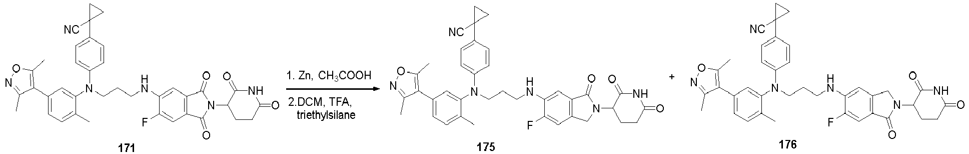

Compound 1-(4-((5-(3,5-dimethylisoxazol-4-yl)-2-methylphenyl)(3-((2-(2,6-dioxopiperidin-3-yl)-6-fluoro-1,3-dioxopiperidin-5-yl)amino)propyl)amino)phenyl)cyclopropane-1-nitrile **(HC-4064-01)** (67 mg, 0.1 mmol) was dissolved in 3 mL of acetic acid, to which was added zinc powder (26 mg, 0.4 mmol), and the mixture was heated 80 °C and stirred overnight. After the reaction was completed, the reaction solution was cooled to room temperature, and then the solvent was removed by evaporation under reduced pressure, to obtain the crude product. The obtained product was dissolved in 2 mL of dichloromethane, to which were successively added 2 mL of trifluoroacetic acid and 1 mL of triethylsilane, and then the reaction solution was stirred for 3 h at room temperature. Then, the solvent was removed by evaporation under reduced pressure, and the crude product was purified by TLC (DCM:MeOH = 20:1), to provide 20 mg of **175** (with a yield of 30.3%) and 35 mg of **176** (with a yield of 53%).

**175:** MS(ES): *m*/*z* 661 [M+H]⁺. ¹H NMR (400 MHz, DMSO-d₆) *δ* 11.09 (s, 1H), 7.44 (d, *J* = 7.9 Hz, 1H), 7.26 (dd, *J* = 13.5, 6.3 Hz, 2H), 7.12-7.07 (m, 2H), 6.95 (d, *J* = 8.0 Hz, 1H), 6.49 (d, *J* = 8.8 Hz, 2H), 5.87-5.74 (m, 1H), 5.13-5.03 (m, 1H), 4.26 (s, 1H), 4.18 (s, 1H), 3.79-3.66 (m, 2H), 3.23 (d, *J* = 6.3 Hz, 2H), 2.95-2.81 (m, 1H), 2.70-2.57 (m, 1H), 2.35 (s, 3H), 2.18 (s, 3H), 2.07 (s, 3H), 1.96 (dd, *J* = 15.7, 8.6 Hz, 3H), 1.59 (q, *J* = 4.6 Hz, 2H), 1.31 (dd, *J* = 7.6, 4.9 Hz, 2H), 1.18-1.09 (m, 1H), 0.86 (s, 1H).

**176:** MS(ES): *m*/*z* 661 [M+H]⁺. ¹H NMR (400 MHz, DMSO-d₆) *δ* 11.05 (s, 1H), 7.45 (d, *J* = 7.9 Hz, 1H), 7.27 (t, *J* = 7.7 Hz, 2H), 7.17-7.06 (m, 3H), 6.80 (d, *J* = 7.5 Hz, 1H), 6.50 (d, *J* = 8.7 Hz, 2H), 6.28 (s, 1H), 5.04 (dd, *J* = 13.2, 5.0 Hz, 1H), 4.18 (dd, *J* = 43.0, 16.8 Hz, 2H), 3.81-3.61 (m, 2H), 3.25 (d, *J* = 5.9 Hz, 2H), 2.89 (d, *J* = 12.2 Hz, 1H), 2.59 (d, *J* = 15.2 Hz, 1H), 2.36 (s, 3H), 2.19 (s, 3H), 2.07 (s, 3H), 1.94 (d, *J* = 5.3 Hz, 2H), 1.60 (q, *J* = 4.7 Hz, 2H), 1.31 (q, *J* = 4.8 Hz, 2H), 1.24 (s, 1H).

### 177: 1-(4-((5-(3,5-Dimethylisoxazol-4-yl)-2-methylphenyl)(2-((1-((2-(2,6-dioxopiperidin-3-yl)-6-fluoro-3-oxoisoindolin-5-yl)azacyclobutane-3-yl)oxy)propyl)amino)phenyl)cyclopro pane-1-nitrile

### 178: 1-(4-((5-(3,5-dimethylisoxazol-4-yl)-2-methylphenyl)(2-((1-(2-(2,6-dioxopiperidin-3-yl)-6-fluoro-1-oxoisoindolin-5-yl)azetidin-3-yl)oxy)propyl)amino)phenyl)cyclopropane-1 -nitrile

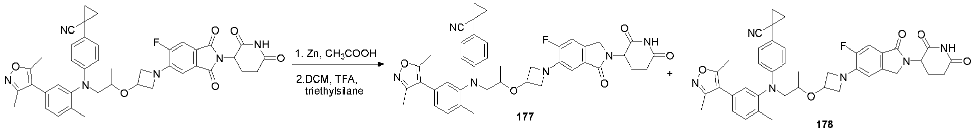

Compound 1-(4-((5-(3,5-dimethylisoxazol-4-yl)-2-methylphenyl)(2-((1-(2-(2,6-dioxopiperidin-3-yl)-6-fluoro-1,3-dioxoisobutanol-5-yl)azacyclobutane-3-yl)oxy)propyl)amino)phenyl)cyclopr opane-1-nitrile (73 mg, 0.1 mmol) was dissolved in 3 mL of acetic acid, to which was added zinc powder (26 mg, 0.4 mmol), and the reaction solution was heated 80 °C and stirred overnight. After the reaction was completed, the reaction solution was cooled to room temperature, and then the solvent was removed by evaporation under reduced pressure, to obtain the crude product. The obtained product was dissolved in 2 mL of dichloromethane, to which were successively added 2 mL of trifluoroacetic acid and 1 mL of triethylsilane, and then the reaction solution was stirred for 3 h at room temperature. Then, the solvent was removed by evaporation under reduced pressure, and the crude product was purified by TLC (DCM:MeOH = 20:1), to provide 26 mg of final product **177** (with a yield of 36.1%) and 42 mg of final product **178** (with a yield of 58.3%). **177:** MS(ES): *m*/*z* 717 [M+H]⁺. ¹H NMR (400 MHz, DMSO-d₆) *δ* 11.03 (s, 1H), 7.42-7.36 (m, 1H), 7.27 (s, 2H), 7.24-7.20 (m, 1H), 7.12 (d*, J* = 8.6 Hz, 2H), 6.82-6.65 (m, 1H), 6.51 (d, *J* = 8.8 Hz, 2H), 5.13-5.02 (m, 1H), 4.51-4.39 (m, 1H), 4.34-4.26 (m, 1H), 4.25-4.13 (m, 2H), 4.09-4.00 (m, 1H), 3.87-3.76 (m, 1H), 3.75-3.53 (m, 3H), 3.45-3.39 (m, 1H), 2.95-2.84 (m, 1H), 2.69-2.56 (m, 1H), 2.38 (d, *J* = 10.6 Hz, 3H), 2.20 (d, *J* = 8.9 Hz, 3H), 2.01 (d, *J* = 11.5 Hz, 3H), 1.60 (d, *J* = 2.5 Hz, 2H), 1.31 (d*, J* = 5.9 Hz, 2H), 1.16 (d, *J* = 6.1 Hz, 2H).

**178:** MS(ES): *m*/*z* 717 [M+H]⁺. ¹H NMR (400 MHz, DMSO-d₆) *δ* 11.09 (s, 1H), 7.38 (s, 1H), 7.30 (d, *J* = 11.3 Hz, 2H), 7.23 (s, 1H), 7.12 (d, *J* = 8.6 Hz, 2H), 6.51 (d, *J* = 8.5 Hz, 2H), 5.11-4.99 (m, 1H), 4.56-4.41 (m, 1H), 4.20 (s, 2H), 3.89-3.47 (m, 4H), 2.96-2.80 (m, 1H), 2.37 (d, *J* = 12.8 Hz, 3H), 2.20 (d, *J* = 9.9 Hz, 3H), 2.00 (d, *J* = 17.7 Hz, 4H), 1.60 (s, 2H), 1.31 (d, *J* = 4.4 Hz, 2H), 1.25 (d, *J* = 9.5 Hz, 3H), 1.16 (d, *J* = 5.9 Hz, 2H).

### 179: 1-(4-((5-(3,5-Dimethylisoxazol-4-yl)-2-methylphenyl)(2-((1-(2-(2,6-dioxopiperidin-3-yl)-3-oxoisoindolin-5-yl)azacyclobutane-3-yl)oxy)propyl)amino)phenyl)cyclopropane-1-nitrile

### 180: 1-(4-((5-(3,5-dimethylisoxazol-4-yl)-2-methylphenyl)(2-((1-(2-(2,6-dioxopiperidin-3-yl)-1-oxoisoindolin-5-yl)azacyclobutane-3-yl)oxy)propyl)amino)phenyl)cyclopropane-1-nit rile

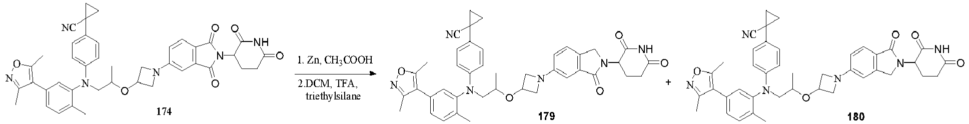

Compound 1-(4-((5-(3,5-dimethylisoxazol-4-yl)-2-methylphenyl)(2-((1-(2-(2,6-dioxopiperidin-3-yl)-1,3-dioxoisobutanol-5-yl)azacyclobutane-3-yl)oxy)propyl)amino)phenyl)cyclopropane-1-nitrile (**HC-4067-01**) (71 mg, 0.1 mmol) was dissolved in 3 mL of acetic acid, to which was added zinc powder (26 mg, 0.4 mmol), and the reaction solution was heated to 80 °C and stirred overnight. After the reaction was completed, the reaction solution was cooled to room temperature, and then the solvent was removed by evaporation under reduced pressure, to obtain the crude product. The obtained product was dissolved in 2 mL of dichloromethane, to which were successively added 2 mL of trifluoroacetic acid and 1 mL of triethylsilane, and then the reaction solution was stirred for 3 h at room temperature. Then, the solvent was removed by evaporation under reduced pressure, and the crude product was purified by TLC (DCM:MeOH = 20:1), to provide 23 mg of final product **179** (with a yield of 32.9%) and 44 mg of final product **180** (with a yield of 62.9%). **179:** MS(ES): *m*/*z* 699 [M+H]⁺. ¹H NMR (400 MHz, DMSO) *δ* 11.09 (s, 1H), 7.45 (d, *J* = 8.1 Hz, 1H), 7.37 (d, *J* = 7.7 Hz, 1H), 7.29 (d, *J* = 15.3 Hz, 2H), 7.15 (d, *J* = 8.3 Hz, 2H), 6.56 (d, *J* = 8.4 Hz, 1H), 6.44 (s, 1H), 5.15- 4.96 (m, 1H), 4.61-4.44 (m, 1H), 4.25 (s, 1H), 4.14 (d, *J* = 17.1 Hz, 2H), 4.02-3.88 (m, 1H), 3.81 - 3.48 (m, 4H), 3.36 (m, 2H), 2.95-2.80 (m, 1H), 2.68-2.54 (m, 2H), 2.34 (s, 3H), 2.19 (d, J = 2.2 Hz, 3H), 1.99 (d, *J* = 15.9 Hz, 3H), 1.57 (s, 1H), 1.32 (d, *J* = 5.6 Hz, 2H), 1.31-1.21 (m, 4H), 1.15 (d, *J* = 5.9 Hz, 2H)

**180:** MS(ES): *m*/*z* 699[M+H]⁺. ¹H NMR (400 MHz, DMSO) *δ* 11.14 (s, 1H), 7.47 (d, *J* = 8.1 Hz, 1H), 7.39 (d, *J* = 7.7 Hz, 1H), 7.26 (d, *J* = 15.3 Hz, 2H), 7.12 (d, *J* = 8.3 Hz, 2H), 6.52 (d, *J* = 8.4 Hz, 1H), 6.42 (s, 1H), 5.13-4.98 (m, 1H), 4.59-4.46 (m, 1H), 4.28 (s, 1H), 4.18 (d, *J* = 17.1 Hz, 2H), 4.08-3.94 (m, 1H), 3.88-3.48 (m, 4H), 3.38 (m, 2H), 2.97-2.82 (m, 1H), 2.70-2.56 (m, 2H), 2.36 (s, 3H), 2.20 (d, *J* = 2.2 Hz, 3H), 2.00 (d, *J* = 15.9 Hz, 3H), 1.60 (s, 1H), 1.31 (d, *J* = 5.6 Hz, 2H), 1.32-1.22 (m, 4H), 1.17 (d*, J* = 5.9 Hz, 2H).

### 181: 1-(4-((5-(3,5-dimethylisoxazol-4-yl)-2-methylphenyl)((1s,2s)-2-((2-(2,6-dioxopiperidin-3-yl)-6-fluoro-1,3-dioxoidindolin-5-yl)amino)methyl)cyclopropyl)amino)phenyl)cycloprop ane-1-nitrile

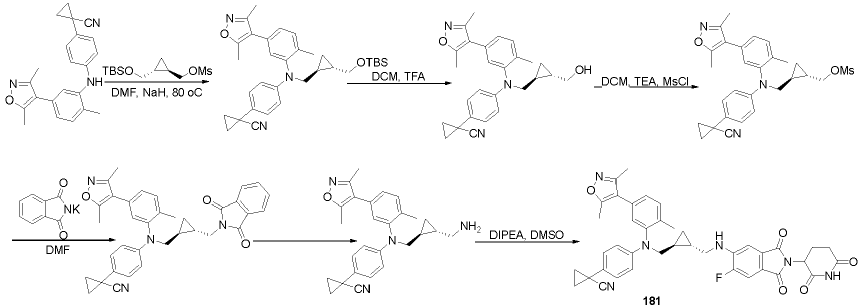

The starting compound (343 mg, 1 mmol) was dissolved in 10 mL of DMF, and then NaH (80 mg, 2 mmol) was added in an ice bath. After the mixture was stirred for 0.5 h, ((1R, 2R)-2-((t-butyldimethylsilyloxy)methyl)cyclopropyl)methyl methanesulfonate (589 mg, 2 mmol) was added. Then, the reaction solution was heated to 80 °C and allowed to react for 15 h. After the reaction was completed, the reaction solution was cooled to room temperature, to which were added 40 ml of water and 120 ml of ethyl acetate for extraction. The obtained organic phase was evaporated under reduced pressure to remove the solvent. The crude product was purified by column chromatography (PE:EA = 3:1), to obtain 704 mg of intermediate 1-(4-(((1s,2s)-2-((t-butyldimethylsilyloxy)methyl)cyclopropyl)methyl)(5-(3,5-dimethylisoxazol -4-yl)-2-methylphenyl)amino)phenyl)cyclopropane-1-nitrile, with a yield of 65%. MS(ES): *m*/*z* 542 [M+H]⁺.

The intermediate (541 mg, 1 mmol), obtained in the previous step, was dissolve in 10 ml of THF, to which was added TBAF (392 mg, 1.5 mmol), and the mixture was stirred for 3 h. The reaction was completed. 20 mL of water and 20 mL of ethyl acetate were added to the reaction solution for extraction. The obtained organic phase was evaporated under reduced pressure to remove the solvent. The crude product was purified by column chromatography (PE:EA = 1:1), to provide 381 mg of intermediate 1-(4-((5-(3,5-dimethylisoxazol-4-yl)-2-methylphenyl) ((1s,2s)-2-(hydroxymethyl)cyclopropyl)methyl)amino)phenyl)cyclopropane-1-nitrile, with a yield of 89%. MS(ES): *m*/*z* 428 [M+H]⁺.

The intermediate (428 mg, 1 mmol), obtained in the previous step, was dissolved in 10 ml of DCM, to which was added TEA (152 mg, 1.5 mmol). Then, methylsulfonyl chloride (137 mg, 1.2 mmol) was added dropwise in an ice bath, and the mixture was stirred for 3 h. After the reaction was completed, 5 ml of water and 20 ml of dichloromethane were added for extraction. The obtained organic phase was evaporated under reduced pressure to remove the solvent. The crude product ((1s,2s)-2-((4-(1-cyanocyclopropyl)phenyl)(5-(3,5-dimethylisoxazol-4-yl)-2-methylphenyl)amino)methyl)cyclopropyl)methyl methanesulfonate was directly used in the next step without further purification.

The intermediate (505 mg, 1 mmol), obtained in the previous step, was dissolved in 5 ml of DMF, to which was added potassium phthalimide (278 mg, 1.5 mmol), and then the reaction solution was heated to 80 °C. The mixture was allowed to react 3 h under stirring. After the reaction was completed, 10 ml of water and 30 ml of ethyl acetate were added for extraction. The obtained organic phase was evaporated under reduced pressure to remove the solvent. The crude product was purified by column chromatography (PE:EA = 3:1), to provide 512 mg of intermediate 1-(4-((5-(3,5-dimethylisoxazol-4-yl)-2-methylphenyl)((1s,2s)-2-((1,3-dioxoisobutanol-2-yl)methyl)cyclopropyl)methyl)amino)phenyl)cyclopropane-1-nitrile, with a yield of 92%. MS(ES): *m*/*z* 557 [M+H]⁺.

The intermediate (557 mg, 1 mmol), obtained in the previous step, was dissolved in 10 ml of absolute ethanol, to which was added hydrazine hydrate (75 mg, 1.5 mmol). The reaction solution was heatd to 80 °C, and allowed to react for 3 h under stirring. After the reaction was completed, the reaction solution was cooled, and then filtered to remove the insoluble matter. 10 ml of water and 30 ml of ethyl acetate were added to the filtrate for extraction. The obtained organic phase was evaporated under reduced pressure to remove the solvent. The crude product 1-(4-(((1s,2s)-2-(aminomethyl)cyclopropyl)methyl)(5-(3,5-dimethylisoxazol-4-yl)-2-methylphe nyl)amino)phenyl)cyclopropane-1-nitrile was directly used in the next step without further purification.

Compound 1-(4-(((1s,2s)-2-(aminomethyl)cyclopropyl)methyl)(5-(3,5-dimethylisoxazol-4-yl)-2-methylphenyl)amino)phenyl)cyclopropane-1-nitrile (intermediate **39-5**) (106 mg, 0.25 mmol) was dissolved in 3 mL of DMSO, to which was added 2-(2,6-dioxopiperidin-3-yl)-5,6-difluoroisonicotinamide-1,3-dione (74 mg, 0.25 mmol). The reaction solution was heatd to 130 °C, and stirred for 2 h. The reaction was completed. The reaction solution was cooled to room temperature, to which were added 10 ml of water and 20 ml of ethyl acetate for extraction. The obtained organic phase was evaporated under reduced pressure to remove the solvent. The crude product was purified by TLC (DCM: MeOH = 20:1), to provide 85 mg of final product **181,** with a yield of 48.6%. MS(ES): *m*/*z* 701 [M+H]⁺. ¹H NMR (400 MHz, DMSO) *δ* 11.09 (s, 1H), 8.17 (t, *J* = 7.7 Hz, 1H), 7.52 (d, *J* = 10.3 Hz, 1H), 7.43 (dd, *J* = 7.8, 3.0 Hz, 1H), 7.23 (d, *J* = 7.8 Hz, 1H), 7.17 (s, 1H), 7.08 (d, *J* = 8.3 Hz, 2H), 7.04-6.91 (m, 2H), 6.51-6.44 (m, 2H), 5.06 (dd, *J* = 12.8, 5.3 Hz, 2H), 3.16-2.80 (m, 4H), 2.58 (t, *J* = 12.8 Hz, 2H), 2.36 (s, 3H), 2.19 (s, 3H), 2.06 (s, 3H), 1.59 (s, 2H), 1.29 (d, *J* = 6.6 Hz, 2H), 1.10 (s, 1H), 0.94 (s, 1H), 0.50-0.32 (m, 2H).

### 182: 1-(4-((5-(3,5-dimethylisoxazol-4-yl)-2-methylphenyl)((1s,2s)-2-((2-(2,6-dioxopiperidin-3-yl)-1,3-dioxoisoindolin-5-yl)amino)methyl)cyclopropyl)amino)phenyl)cyclopropane-1-ca rbonitrile

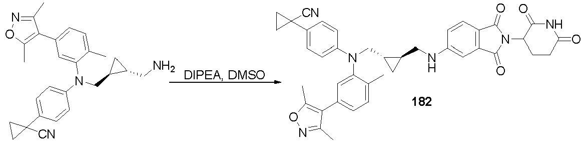

Compound 1-(4-(((1s,2s)-2-(aminomethyl)cyclopropyl)methyl)(5-(3,5-dimethylisoxazol-4-yl)-2-methylphenyl)amino)phenyl)cyclopropane-1-nitrile (intermediate **39-5**) (106 mg, 0.25 mmol) was dissolved in 3 mL of DMSO, to which was added 2-(2, 6-dioxopiperidin-3-yl)-5-fluoroisoindolin-1,3-dione (69 mg, 0.25 mmol). The reaction solution was heatd to 130 °C, and stirred for 2 h. The reaction was completed. The reaction solution was cooled to room temperature, to which were added 10 ml of water and 20 ml of ethyl acetate for extraction. The obtained organic phase was evaporated under reduced pressure to remove the solvent. The crude product was purified by TLC (DCM: MeOH = 20:1), to provide 89 mg of final product, with a yield of 52.1%. MS(ES): *m*/*z* 683 [M+H]⁺. ¹H NMR (400 MHz, DMSO-d₆) *δ* 11.07 (s, 1H), 7.48 (dd, *J* = 16.7, 8.1 Hz, 2H), 7.26 (d, *J* = 7.5 Hz, 1H), 7.18 (s, 1H), 7.11 (d, *J* = 8.6 Hz, 3H), 6.87 (s, 1H), 6.75 (d, *J* = 8.2 Hz, 1H), 6.49 (d, *J* = 8.5 Hz, 2H), 5.04 (dd, *J* = 12.9, 5.3 Hz, 1H), 3.55 (d, *J* = 5.9 Hz, 2H), 3.07-2.77 (m, 4H), 2.66-2.55 (m, 2H), 2.37 (s, 3H), 2.19 (s, 3H), 2.09 (s, 3H), 1.60 (d, *J* = 2.2 Hz, 2H), 0.96 (d, *J* = 82.3 Hz, 4H), 0.48-0.35 (m, 2H).

### 184: 1-(4-((5-(3,5-Dimethylisoxazol-4-yl)-2-methylphenyl)((1s,2s)-2-((2-(2,6-dioxopiperidin-3-yl)-6-fluoro-3-oxoisoindolin-5-yl)amino)methyl)cyclopropyl)methyl)amin o)phenyl)cyclopropane-1-nitrile

### 185: 1-(4-((5-(3,5-Dimethylisoxazol-4-yl)-2-methylphenyl)((1s,2s)-2-((2-(2,6-dioxopiperidin-3-yl)-6-fluoro-1-oxoisoindolin-5-yl)amino)methyl)cyclopropyl)methyl)amin o)phenyl)cyclopropane-1-nitrile

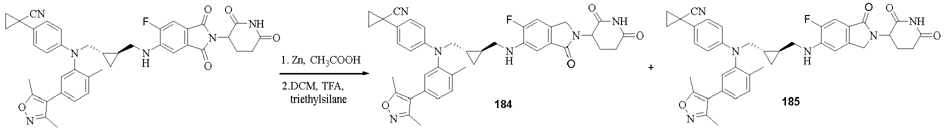

Compound 1-(4-((5-(3,5-dimethylisoxazol-4-yl)-2-methylphenyl)((1s,2s)-2-((2-(2,6-dioxopiperidin-3-yl)-6-fluoro-1,3-dioxopiperidin-5-yl)amino)methyl)cyclopropyl)amino)phenyl )cyclopropane-1-nitrile (**HC-4106-01**) (70 mg, 0.1 mmol) was dissolved in 3 mL of acetic acid, to which was added zinc powder (26 mg, 0.4 mmol), and the reaction solution was heated to 80 °C and stirred overnight. After the reaction was completed, the reaction solution was cooled to room temperature, and then the solvent was removed by evaporation under reduced pressure, to obtain the crude product. The obtained product was dissolved in 2 mL of dichloromethane, to which were successively added 2 mL of trifluoroacetic acid and 1 mL of triethylsilane, and then the reaction solution was stirred for 3 h at room temperature. Then, the solvent was removed by evaporation under reduced pressure, and the crude product was purified by TLC (DCM:MeOH = 20:1), to provide 28 mg of final product **184** (with a yield of 40.6%) and 46 mg of final product **185** (with a yield of 66.6%). **184:** MS(ES): *m*/*z* 687 [M+H]⁺. ¹H NMR (400 MHz, DMSO-d₆) *δ* 11.09 (s, 1H), 7.81 (s, 2H), 7.49-7.44 (m, 1H), 7.26 (d, *J* = 10.8 Hz, 2H), 7.12-7.07 (m, 2H), 6.87 (s, 1H), 6.48 (d, *J* = 8.8 Hz, 2H), 5.10 (d, *J* = 4.9 Hz, 1H), 4.48 -4.13 (m, 4H), 3.53 (d, *J* = 6.2 Hz, 2H), 3.03-2.85 (m, 4H), 2.36 (s, 3H), 2.19 (s, 3H), 2.06 (s, 3H), 1.59 (s, 2H), 1.29 (d, *J* = 6.6 Hz, 2H), 1.10 (s, 1H), 0.94 (s, 1H), 0.50-0.32 (m, 2H).

**185:** MS(ES): *m*/*z* 687 [M+H]⁺. ¹H NMR (400 MHz, DMSO-d₆) *δ* 10.95 (s, 1H), 7.44 (d, *J* = 7.8 Hz, 1H), 7.25 (dd, *J* = 11.9, 4.4 Hz, 2H), 7.16 (s, 1H), 7.10 (d, *J* = 8.5 Hz, 2H), 6.70-6.61 (m, 1H), 6.49 (d, *J* = 8.8 Hz, 2H), 6.21 (s, 1H), 5.03 (dd, *J* = 13.3, 4.0 Hz, 1H), 4.14 (dd, *J* = 19.9, 17.2 Hz, 2H), 3.53 (dd, *J* = 16.6, 5.3 Hz, 2H), 3.04-2.84 (m, 4H), 2.36 (s, 2H), 2.19 (d, *J* = 1.9 Hz, 3H), 2.08 (s, 3H), 1.84 (s, 1H), 1.60 (dd, *J* = 6.9, 4.3 Hz, 2H), 1.31 (s, 2H), 1.25 (d, *J* = 9.4 Hz, 2H), 0.97 (d, *J* = 86.4 Hz, 2H), 0.52-0.25 (m, 2H).

### 186: 1-(4-((5-(3,5-Dimethylisoxazol-4-yl)-2-methylphenyl)((1s,2s)-2-((2-(2,6-dioxopiperidin-3-yl)-3-oxoisobutanol-5-yl)amino)methyl)cyclopropyl)amino)phenyl)cyclop ropane-1-nitrile

### 187: 1-(4-((5-(3,5-Dimethylisoxazol-4-yl)-2-methylphenyl)((1s,2s)-2-((2-(2, 6-dioxopiperidin-3-yl)-1-oxoisoindolin-5-yl)amino)methyl)cyclopropyl)amino)phenyl)cyclo propane-1-nitrile

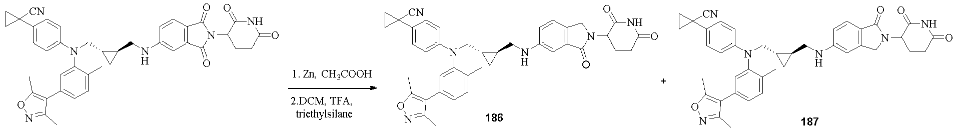

Compound 1-(4-((5-(3,5-dimethylisoxazol-4-yl)-2-methylphenyl)((1s,2s)-2-((2-(2,6-dioxopiperidin-3-yl)-6-fluoro-1,3-dioxopiperidin-5-yl)amino)methyl)cyclopropyl)amino)phenyl )cyclopropane-1-nitrile (**HC-4107-01**) (68 mg, 0.1 mmol) was dissolved in 3 mL of acetic acid, to which was added zinc powder (26 mg, 0.4 mmol), and the reaction solution was heated to 80 °C and stirred overnight. After the reaction was completed, the reaction solution was cooled to room temperature, and then the solvent was removed by evaporation under reduced pressure, to obtain the crude product. The obtained product was dissolved in 2 mL of dichloromethane, to which were successively added 2 mL of trifluoroacetic acid and 1 mL of triethylsilane, and then the reaction solution was stirred for 3 h at room temperature. Then, the solvent was removed by evaporation under reduced pressure, and the crude product was purified by TLC (DCM:MeOH = 20:1), to provide 20 mg of final product **186** (with a yield of 29.9%) and 45 mg of final product **187** (with a yield of 67.2%). **186:** MS(ES): *m*/*z* 687 [M+H]⁺. ¹H NMR (400 MHz, DMSO-d₆) *δ* 11.09 (s, 1H), 7.48 (dd, *J* = 16.7, 8.1 Hz, 2H), 7.26 (d, *J* = 7.5 Hz, 1H), 7.18 (s, 1H), 7.15 (d, *J* = 8.6 Hz, 3H), 6.89 (s, 1H), 6.75 (d, *J* = 8.2 Hz, 2H), 6.49 (d, *J* = 8.5 Hz, 2H), 5.04 (dd, *J* = 12.9, 5.3 Hz, 1H), 3.55 (d, *J* = 5.9 Hz, 2H), 3.07-2.77 (m, 4H), 2.66-2.55 (m, 2H), 2.37 (s, 3H), 2.19 (s, 3H), 2.09 (s, 3H), 1.60 (d, *J* = 2.2 Hz, 2H), 0.96 (d, *J* = 82.3 Hz, 4H), 0.48-0.35 (m, 2H).

**187:** MS(ES): *m*/*z* 669 [M+H]⁺. ¹H NMR (400 MHz, DMSO-d₆) *δ* 10.98(s, 1H), 7.47 (d, *J* = 7.9 Hz, 1H), 7.33 (d, *J* = 8.4 Hz, 1H), 7.28 (d, *J* = 7.7 Hz, 1H), 7.18 (s, 1H), 7.11 (d*, J* = 8.7 Hz, 2H), 6.57 (d*, J* = 8.5 Hz, 1H), 6.50 (d, *J* = 8.4 Hz, 2H), 6.35 (s, 1H), 5.05-4.97 (m, 1H), 4.19 (d, *J* = 8.9 Hz, 1H), 4.12 (d, *J* = 5.3 Hz, 1H), 3.55 (s, 2H), 2.92 (d, *J* = 12.8 Hz, 2H), 2.80-2.69 (m, 1H), 2.55 (s, 2H), 2.37 (s, 3H), 2.20 (d, *J* = 1.5 Hz, 3H), 2.09 (s, 3H), 2.03-1.89 (m, 2H), 1.60 (dd, *J* = 7.1, 4.6 Hz, 2H), 1.10-0.97 (m, 2H), 0.86 (s, 2H), 0.46-0.40 (m, 1H), 0.39-0.31 (m, 1H).

### 188: 1-(4-((5-(3,5-Dimethylisoxazol-4-yl)-2-methylphenyl)(2-((1-(2,6-dioxopiperidin-3-yl)-6-fluoro-1,3-dioxoisoindolin-5-yl)piperidin-4-yl)oxy)propyl)amino)phenyl)cyclopropane-1-nitrile

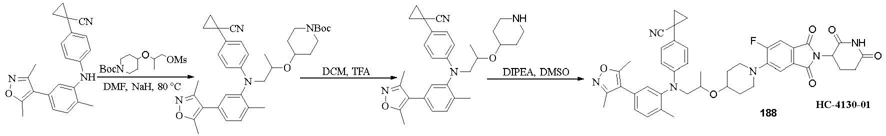

The starting compound (686.86 mg, 2 mmol) was dissolved in 5 mL of DMF, and then NaH (160 mg, 4 mmol) was added in an ice bath. After the mixture was stirred for 0.5 h, t-butyl 4-((1-((methylsulfonyl)oxy)propan-2-yl)oxy)piperidine-1-carboxylate (1.35 g, 4 mmol) and sodium iodide (37.18 mg, 0.2 mmol) were added. After addition, the reaction solution was heated to 80 °C and allowed to react for 15 h. After the reaction was completed, the reaction solution was cooled to room temperature, to which were added 20 ml of water and 30 ml of ethyl acetate for extraction. The obtained organic phase was evaporated under reduced pressure to remove the solvent. The crude product was purified by column chromatography (PE:EA = 3:1), to obtain 100 mg of intermediate t-buytl 4-((1-((4-(1-cyanocyclopropyl)phenyl)(5-(3, 5-dimethylisoxazol-4-yl)-2-methylphenyl)amino)propan-2-yl)oxy)piperidine-1-carboxylate, with a yield of 8.5%. MS(ES): *m*/*z* 586 [M+H]⁺.

Compound t-buytl 4-((1-((4-(1-cyanocyclopropyl)phenyl)(5-(3,5-dimethylisoxazol-4-yl) -2-methylphenyl)amino)propan-2-yl)oxy)piperidine-1-carboxylate (intermediate **45-1**) (95 mg, 0.16 mmol) was dissolved in 2 mL of DCM, to which was added 1 mL of trifluoroacetic acid. The mixture was stirred for 2 h at room temperature. After the reaction was completed, the solvent was removed by evaporation under reduced pressure. The crude product 4-((1-((4-(1-cyanocyclopropyl)phenyl)(5-(3,5-dimethylisoxazol-4-yl)-2-methylphenyl)amino)pr opan-2-yl)oxy)piperidine-1-carboxylic acid was directly used in the next step without further purification.

Compound 4-((1-((4-(1-cyanocyclopropyl)phenyl)(5-(3,5-dimethylisoxazol-4-yl)-2-methylphenyl)amino)propan-2-yl)oxy)piperidine-1-carboxylic acid (intermediate **45-2**) (65 mg, 0.13 mmol) was dissolved in 2 mL of DMSO, to which was added 2-(2,6-dioxopiperidin-3-yl)-5,6-difluoroisonicotinamide-1,3-dione (38 mg, 0.13 mmol). The reaction solution was heatd to 130 °C, and stirred for 2 h. The reaction was completed. The reaction solution was cooled to room temperature, to which were added 10 ml of water and 15 ml of ethyl acetate for extraction. The obtained organic phase was evaporated under reduced pressure to remove the solvent. The crude product was purified by TLC (DCM: MeOH = 20:1), to provide 45 mg of final product, with a yield of 50.7%. MS(ES): *m*/*z* 759 [M+H]⁺. ¹H NMR (400 MHz, DMSO-d₆) *δ* 11.09 (s, 1H), 7.72-7.66 (m, 1H), 7.43-7.38 (m, 1H), 7.31 (s, 2H), 7.25 -7.20 (m, 1H), 7.10 (d, *J* = 8.6 Hz, 2H), 6.56 (d, *J* = 8.7 Hz, 2H), 5.14-5.07 (m, 1H), 3.95-3.85 (m, 1H), 3.72-3.55 (m, 3H), 3.06 -2.81 (m, 6H), 2.71-2.56 (m, 2H), 2.39 (s, 3H), 2.22 (s, 3H), 2.03 (s, 3H), 1.60 (dd, *J* = 7.1, 4.5 Hz, 2H), 1.51 (m, 4H), 1.32 (dd, *J* = 7.2, 4.7 Hz, 2H), 1.16 (d, *J* = 6.0 Hz, 3H).

### 189: 1-(4-((5-(3,5-dimethylisoxazol-4-yl)-2-methylphenyl)(5-((2-(2,6-dioxopiperidin-3-yl)-6-fluoro-1,3-dioxoisoindolin-5-yl)amino)-3-methylpentanyl)amino)phenyl)cyclopropa ne-1-carbonitrile

### 190: 1-(4-((5-(3,5-dimethylisoxazol-4-yl)-2-methylphenyl)(5-((2-(2,6-dioxopiperidin-3-yl)-6-fluoro-3-oxoisoindolin-5-yl)amino)-3-methylpentanyl)amino)phenyl)cyclopropane-1-carbonitrile

### 191: 1-(4-((5-(3,5-dimethylisoxazol-4-yl)-2-methylphenyl)(5-((2-(2,6-dioxopiperidin-3-yl)-6-fluoro-1-oxoisoindolin-5-yl)amino)-3-methylpentanyl)amino)phenyl)cyclopropane-1-carbonitrile

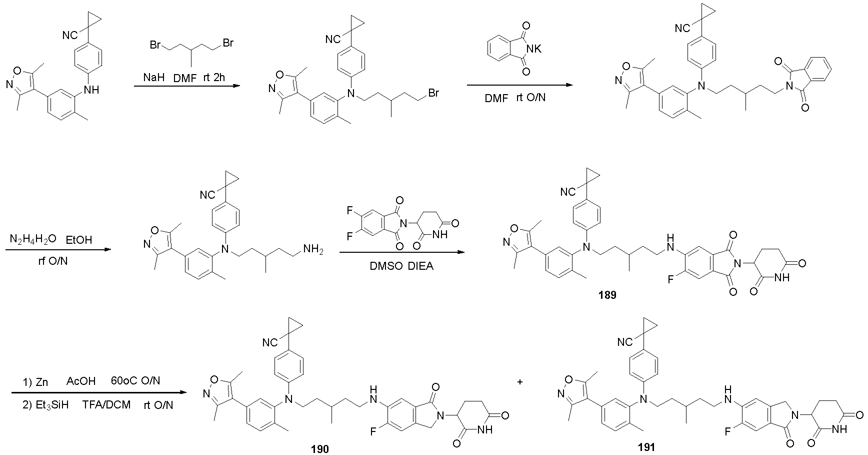

Compounds 1-(4-((5-(3,5-dimethylisoxazol-4-yl)-2-methylphenyl)amino)phenyl)cyclopropane-1-carbonitrile (800 mg, 2.33 mmol) and *N*-(3-bromo-4-(1H-imidazol-1-yl)phenyl)-5-(3,5-dimethylisoxazol-4-yl)-2-methylaniline (85 mg, 0.2 mmol) were dissolved in 10 mL of DMF, to which was added NaH (186 mg, 4.66 mmol) under nitrogen protection at 0 °C. Then, the temperature was maintained, and the mixture was stirred and reacted for 1 h, to which was added 1,5-dibromo-3-methylpentane (3.41 g, 13.98 mmol). The reaction solution was gradually warmed to room temperature, and then the resultant solution was allowed to react 1 h under stirring. The reaction was completed by TLC detection, and then water was added to the reaction solution. The reaction solution was extracted three times with ethyl acetate. The organic layer was combined and extracted three times with saturated brine, dried over anhydrous sodium sulfate, and then rotatory evaporated to dry, to obtain the crude product, which was purified by silica gel column chromatography, to provide compound 1-(4-((5-bromo-3-methylpentanyl)(5-(3,5-dimethylisoxazol-4-yl)-2-methylphenyl) amino)phenyl)cyclopropane-1-carbonitrile as off-whithe solid (1.10 g, 2.17 mmol), with a yield of 93%. LC/MS (ESI⁺) calcd for C₂₈H₃₂BrN₃O (M + H⁺) *m*/*z,* 506.2; found, 506.2.

Compound 1-(4-((5-bromo-3-methylpentanyl)(5-(3,5-dimethylisoxazol-4-yl)-2-methylphenyl) amino)phenyl)cyclopropane-1-carbonitrile (500 mg, 0.99 mmol) was dissolved in 5 mL of DMF, to which was added potassium phthalimide (219 mg, 1.18 mmol) under nitrogen protection. The reaction solution was stirred and reacted overnight at room temperature. The reaction was completed by TLC detection, and then water was added to the reaction solution. The reaction solution was extracted three times with ethyl acetate. The organic layer was combined and extracted three times with saturated brine, dried over anhydrous sodium sulfate, and then rotatory evaporated to dry, to obtain the crude product, which was purified by silica gel column chromatography, to provide compound 1-(4-((5-(3,5-dimethylisoxazol-4-yl)-2-methylphenyl) (5-(1,3-dioxisoindole-2-yl)-3-methylpentanyl)amino)phenyl)cyclopropane-1-carbonitrile as off-white solid (504 mg, 0.88 mmol), with a yield of 89%. LC/MS (ESI⁺) calcd for C₃₆H₃₆N₄O₃ (M + H⁺) *m*/*z,* 573.3; found, 573.3.

Compound 1-(4-((5-bromo-3-methylpentanyl)(5-(3,5-dimethylisoxazol-4-yl)-2-methylphenyl) amino)phenyl)cyclopropane-1-carbonitrile (500 mg, 0.99 mmol) was added in an reactor, to which were added 4 mL of absolute ethanol and hydrazine hydrate (1.89 mg, 3.49 mmol), and the mixture was allowed to react overnight under reflux. TLC indicated the completion of the reaction. Then, the solvent was removed by evaporation under reduced pressure, followed by co-evaporation with absolute ethanol for three times, to provide crude compound 1-(4-((5-amino-3-methylpentanyl)(5-(3,5-dimethylisoxazol-4-yl)-2-methylphenyl)amino)pheny l)cyclopropane-1-carbonitrile as white foam solid (302 mg, 0.68 mmol), with a yield of 98%. LC/MS (ESI⁺) calcd for C₂₈H₃₄N₄O (M + H⁺) m/z, 443.3; found, 443.3. 1-(4-((5-Amino-3-methylpentanyl)(5-(3,5-dimethylisoxazol-4-yl)-2-methylphenyl)amino)phen yl)cyclopropane-1-carbonitrile (100 mg, 0.22 mmol), 2-(2,6-dioxopiperidin-3-yl)-4,5-difluoroisindole-1,3-dione (66 mg, 0.22 mmol) and DIEA(88 mg, 0.68 mmol) were added into 2 mL of DMSO. The reaction solution was heated to 130 °C, stirred and reacted for 2 h. The reaction was completed by TLC detection, and then water was added to the reaction solution. The reaction solution was extracted three times with ethyl acetate. The organic layer was combined and extracted three times with saturated brine, dried over anhydrous sodium sulfate, and then rotatory evaporated to dry. The residue was purified by pre-TLC, to provide compound 1-(4-((5-(3,5-dimethylisoxazol-4-yl)-2-methylphenyl)(5-((2-(2,6-dioxopiperidin-3-yl)-6-fluoro-1,3-dioxoisoindolin-5-yl)amino)-3-methylpentanyl)amino)phenyl)cyclopropane-1-carbonitrile as a yellow solid (102 mg, 0.14 mmol), with a yield of 63%. LC/MS (ESI⁺) calcd for C₄₁H₄₁FN₆O₅ (M + H⁺) *m*/*z,* 717.3; found, 716.5. ¹H NMR (400 MHz, CDCl₃) *δ* 8.31 (d*, J* = 29.6 Hz, 1H), 7.71 (t, *J* = 7.2 Hz, 1H), 7.39 (dd, *J* = 8.7, 7.0 Hz, 2H), 7.12 (dd, *J* = 12.3, 5.3 Hz, 3H), 7.04 (d, *J* = 7.1 Hz, 1H), 7.00 (d, *J* = 1.7 Hz, 1H), 6.45 (d, *J* = 8.9 Hz, 2H), 4.95 (ddd, *J* = 21.1, 12.3, 5.3 Hz, 2H), 4.60 (d, *J* = 4.1 Hz, 1H), 3.74-3.54 (m, 2H), 3.36-3.18 (m, 2H), 2.96 - 2.70 (m, 5H), 2.40 (d, *J* = 9.1 Hz, 3H), 2.24 (d, *J* = 10.7 Hz, 3H), 2.11 (d, *J* = 16.9 Hz, 3H), 1.59 (dt, *J* = 9.8, 5.0 Hz, 3H), 1.30 - 1.26 (m, 3H), 1.03 (d, *J* = 6.2 Hz, 3H).

Compound 1-(4-((5 -(3,5-dimethylisoxazol-4-yl)-2-methylphenyl)(5-((2-(2, 6-dioxopiperidin-3-yl)-6-fluoro-1,3-dioxoisoindol-5-yl)amino)-3-methylpentanyl)amino)phenyl)cyclopropane-1-carbonitrile (150 mg, 0.21 mmol) was dissolved in 1 mL of AcOH, to which was added Zn (274 mg, 4.19 mmol), and the mixture was allowed to react overnight at 60 °C under stirring. TLC indicated the reaction was completed. Then, the reaction solution was filtered, and the filter cake was rinsed with DCM. The filtrate was rotatory evaporated to dry to obtain the crude product. To the obtained product, were added 2 ml of DCM and triethylsilane (49 mg, 0.42mmol), followed by addition of 1 ml of TFA in an ice bath. The reaction solution was stirred and reacted overnight at room temperature. The reaction was completed by TLC detection. The solvent was removed by evaporation under reduced pressure, and the saturated aqueous solution of sodium bicarbonate was added to the residue. The resultant solution was extracted three times with dichloromethane. The organic phase was combined, dried, and rotatory evaporated to dry. The residue was separated by pre-TLC (PE:EA 2:3), to provide an off-white solid compound 1-(4-((5-(3,5-dimethylisoxazol-4-yl)-2-methylphenyl) (5-((2-(2,6-dioxopiperidin-3-yl)-6-fluoro-3-oxoisoindolin-5-yl)amino)-3-methylpentanyl)amino )phenyl)cyclopropane-1-carbonitrile (37 mg, 0.053 mmol) as the upper spot, with a yield of 25%. LC/MS (ESI⁺) calcd for C₄₁H₄₃FN₆O₄ (M + H⁺) *m*/*z,* 703.3; found, 703.3.

The off-white solid compound 1-(4-((5-(3,5-dimethylisoxazol-4-yl)-2-methylphenyl) (5-((2-(2,6-dioxopiperidin-3-yl)-6-fluoro-1-oxoisoindolin-5-yl)amino)-3-methylpentanyl)amino )phenyl)cyclopropane-1-carbonitrile (64 mg, 0.091 mmol) as the lower spot, with a yield of 44%. LC/MS (ESI⁺) calcd for C₄₁H₄₃FN₆O₄ (M + H⁺) *m*/*z,* 703.3; found, 703.3. ¹H NMR (400 MHz, CDCl₃) δ 8.14 (s, 1H), 7.43 (d, *J* = 10.3 Hz, 1H), 7.38 (d, *J* = 7.9 Hz, 1H), 7.15 - 7.05 (m, 3H), 7.00 (s, 1H), 6.65 (d, *J* = 6.9 Hz, 1H), 6.45 (d, *J* = 8.5 Hz, 2H), 5.17 (dd, *J* = 13.2, 3.5 Hz, 1H), 4.34 (t, *J* = 14.5 Hz, 1H), 4.22 (d, *J* = 15.7 Hz, 1H), 3.73 - 3.53 (m, 2H), 3.30 - 3.11 (m, 2H), 2.95 - 2.80 (m, 2H), 2.39 (s, 3H), 2.33 (dd, *J* = 13.2, 5.0 Hz, 1H), 2.26 (s, 3H), 2.20 (d, *J* = 13.5 Hz, 1H), 2.13 (s, 3H), 1.82- 1.65 (m, 3H), 1.57 (d, *J* = 11.4 Hz, 2H), 1.30- 1.22 (m, 4H), 1.01 (t, *J* = 10.1 Hz, 3H).

### 192: Synthesis of 1-(4-((5-(3,5-Dimethylisoxazol-4-yl)-2-methylphenyl) (5-(1,3-dioxisoindolin-2-yl)-3-methylpentanyl)amino)phenyl)cyclopropane-1-carbonitrile

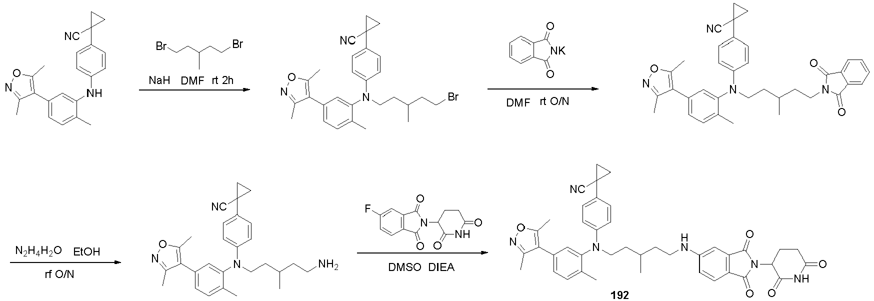

Compound 1-(4-((5-(3,5-dimethylisoxazol-4-yl)-2-methylphenyl)amino)phenyl)cyclopropane-1-carbonitrile (800 mg, 2.33 mmol) were dissolved in 10 mL of DMF, to which was added NaH (186 mg, 4.66 mmol) under nitrogen protection at 0 °C. Then, the temperature was maintained, and the mixture was stirred and reacted for 1 h, to which was added 1,5-dibromo-3-methylpentane (3.41 g, 13.98 mmol). The reaction solution was gradually warmed to room temperature, and then the resultant solution was allowed to react 1 h under stirring. The reaction was completed by TLC detection, and then water was added to the reaction solution. The reaction solution was extracted three times with ethyl acetate. The organic layer was combined and extracted three times with saturated brine, dried over anhydrous sodium sulfate, and then rotatory evaporated to dry, to obtain the crude product, which was purified by silica gel column chromatography, to provide compound 1-(4-((5-bromo-3-methylpentanyl)(5-(3,5-dimethylisoxazol-4-yl)-2-methylphenyl)amino)pheny l)cyclopropane-1-carbonitrile as an off-white solid (1.10 g, 2.17 mmol), with a yield of 93%. LC/MS (ESI⁺) calcd for C₂₈H₃₂BrN₃O (M + H⁺) *m*/*z,* 506.2; found, 506.2.

Compound 1-(4-((5-bromo-3-methylpentanyl)(5-(3,5-dimethylisoxazol-4-yl)-2-methylphenyl) amino)phenyl)cyclopropane-1-carbonitrile (500 mg, 0.99 mmol) was dissolved in 5 mL of DMF, to which was added potassium phthalimide (219 mg, 1.18 mmol) under nitrogen protection. The reaction solution was stirred and reacted overnight at room temperature. The reaction was completed by TLC detection, and then water was added to the reaction solution. The reaction solution was extracted three times with ethyl acetate. The organic layer was combined and extracted three times with saturated brine, dried over anhydrous sodium sulfate, and then rotatory evaporated to dry, to obtain the crude product, which was purified by silica gel column chromatography, to provide compound 1-(4-((5-(3,5-dimethylisoxazol-4-yl)-2-methylphenyl) (5-(1,3-dioxisoindole-2-yl)-3-methylpentanyl)amino)phenyl)cyclopropane-1-carbonitrile (504 mg, 0.88 mmol) as off-white solid (504 mg, 0.88 mmol), with a yield of 89%.

LC/MS (ESI⁺) calcd for C₃₆H₃₆N₄O₃ (M + H⁺) *m*/*z,* 573.3; found, 573.3.

Compound 1-(4-((5-bromo-3-methylpentanyl)(5-(3,5-dimethylisoxazol-4-yl)-2-methylphenyl) amino)phenyl)cyclopropane-1-carbonitrile (500 mg, 0.99 mmol) was added in an reactor, to which were added 4 mL of absolute ethanol and hydrazine hydrate (1.89 mg, 3.49 mmol), and the mixture was allowed to react overnight under reflux. TLC indicated the completion of the reaction. Then, the solvent was removed by evaporation under reduced pressure, followed by co-evaporation with absolute ethanol for three times, to provide crude compound 1-(4-((5-amino-3-methylpentanyl)(5-(3,5-dimethylisoxazol-4-yl)-2-methylphenyl)amino)pheny 1)cyclopropane-1-carbonitrile as off-white foam solid (302 mg, 0.68 mmol), with a yield of 98%. LC/MS (ESI⁺) calcd for C₂₈H₃₄N₄O (M + H⁺) *m*/*z,* 443.3; found, 443.3.

1-(4-((5-Amino-3-methylpentanyl)(5-(3,5-dimethylisoxazol-4-yl)-2-methylphenyl)amino)phen yl)cyclopropane-1-carbonitrile (100 mg, 0.22 mmol), 2-(2,6-dioxopiperidin-3-yl)-5-fluoroisoindole-1,3-dione (62 mg, 0.22 mmol) and DIEA (88 mg, 0.68 mmol) were added into 2 mL of DMSO. The reaction solution was heated to 130 °C, stirred and reacted for 2 h. The reaction was completed by TLC detection, and then water was added to the reaction solution. The reaction solution was extracted three times with ethyl acetate. The organic layer was combined and extracted three times with saturated brine, dried over anhydrous sodium sulfate, and then rotatory evaporated to dry. The residue was purified by pre-TLC, to provide compound 1-(4-((5-(3,5-dimethylisoxazol-4-yl)-2-methylphenyl)(5-((2-(2,6-dioxopiperidin-3-yl) -1,3-dioxoisoindolin-5-yl)amino)-3-methylpentanyl)amino)phenyl)cyclopropane-1-carbonitrile as a yellow solid (43 mg, 0.061 mmol), with a yield of 27%. LC/MS (ESI⁺) calcd for C₄₁H₄₁N₆O₅ (M + H⁺) *m*/*z,* 699.3; found, 699.2. ¹H NMR (400 MHz, CDCl₃) *δ* 8.07 (s, 1H), 7.60 (d, *J* = 8.3 Hz, 1H), 7.38 (d, *J* = 7.9 Hz, 1H), 7.16 - 7.07 (m, 3H), 7.00 (d, *J* = 1.7 Hz, 1H), 6.93 (d, *J* = 1.8 Hz, 1H), 6.71 (dd, *J* = 8.3, 1.9 Hz, 1H), 6.45 (d, *J* = 8.8 Hz, 2H), 4.93 (dd, *J* = 12.2, 5.2 Hz, 1H), 3.73 - 3.54 (m, 2H), 3.21 (ddd, *J* = 19.0, 12.5, 6.9 Hz, 2H), 2.93 - 2.71 (m, 3H), 2.39 (s, 3H), 2.24 (d, *J* = 11.3 Hz, 3H), 2.15 - 2.09 (m, 4H), 1.60 (dd, *J* = 7.3, 4.8 Hz, 3H), 1.27 (dd, *J* = 7.2, 4.6 Hz, 6H), 1.03 (d, *J* = 6.1 Hz, 3H).

### 193: Synthesis of 1-(4-((5-(3,5-dimethylisoxazol-4-yl)-2-methylphenyl)((1r,4r)-4-((2-(2,6-dioxopiperidin-3-yl)-3-oxoisoindolin-5-yl)amino)methyl)cyclohexyl)methyl)phenyl)cyclopr opane-1-carbonitrile

### 194: Synthesis of 1-(4-((5-(3,5-dimethylisoxazol-4-yl)-2-methylphenyl)((1r,4r)-4-((2-(2,6-dioxopiperidin-3-yl)-1-oxoisoindolin-5-yl)amino)methyl)cyclohexyl)methyl)phenyl)cyclopr opane-1-carbonitrile

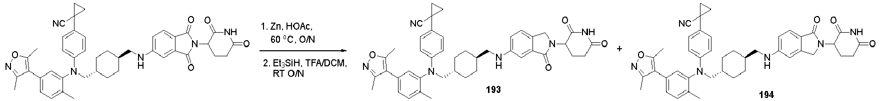

Compound 1-(4-((5-(3,5-dimethylisoxazol-4-yl)-2-methylphenyl)((1*r*,4*r*)-4-((2-(2,6-dioxopiperidin-3-yl)-1,3-dioxoisoindolin-5-yl)amino)methyl)cyclohexyl)methyl)phenyl)cyclopr opane-1-carbonitrile (430 mg, 0.59 mmol) was dissolved in 2 mL of AcOH, to which was added Zn (776 mg, 11.86 mmol), and the mixture was allowed to react overnight at 60 °C under stirring. TLC indicated the reaction was completed. Then, the reaction solution was filtered, and the filter cake was rinsed with DCM. The filtrate was rotatory evaporated to dry to obtain the crude product. To the obtained product, were added 2 ml of DCM and triethylsilane (138 mg, 1.19 mmol), followed by addition of 1 ml of TFA. The reaction solution was stirred and reacted overnight at room temperature. The reaction was completed by TLC detection. The solvent was removed by evaporation under reduced pressure, and the saturated aqueous solution of sodium bicarbonate was added to the residue. The resultant solution was extracted three times with dichloromethane. The organic phase was combined, dried, and rotatory evaporated to dry. The residue was separated by pre-TLC (EA:MeOH 10:1), to provide an off-white solid compound 1-(4-((5-(3,5-dimethylisoxazol-4-yl)-2-methylphenyl)((1*r*,4*r*)-4-((2-(2,6-dioxopiperidin-3-yl)-3 -oxoisoindolin-5-yl)amino)methyl)cyclohexyl)methyl)phenyl)cyclopropane-1-carbonitrile (45 mg, 0.063 mmol) as the upper spot, with a yield of 11%. LC/MS (ESI⁺) calcd for C₄₃H₄₆N₆O₄ (M + H⁺) *m*/*z,* 711.4; found, 711.3. ¹H NMR (400 MHz, CDCl₃) *δ* 8.01 (s, 1H), 7.86 (d, *J* = 8.5 Hz, 1H), 7.56 (d, *J* = 8.7 Hz, 1H), 7.48 (d, *J* = 7.7 Hz, 1H), 7.12 (t, *J* = 9.9 Hz, 1H), 7.00 (t, *J* = 4.4 Hz, 1H), 6.86 (dd, *J* = 8.7, 2.3 Hz, 1H), 6.67 (d, *J* = 8.5 Hz, 1H), 5.19 (dd, *J* = 13.2, 5.1 Hz, 1H), 4.34 (dd, *J* = 54.6, 15.8 Hz, 4H), 4.21 (s, 2H), 3.73 (s, 3H), 3.49 (s, 2H), 2.99 - 2.83 (m, 2H), 2.80 - 2.70 (m, 2H), 2.59 (s, 3H), 2.35 (dt, *J* = 13.0, 8.2 Hz, 2H), 2.30 - 2.19 (m, 3H), 2.02 (s, 4H), 1.77 - 1.57 (m, 10H).

The off-white solid compound 1-(4-((5-(3,5-dimethylisoxazol-4-yl)-2-methylphenyl) ((1*r*,4*r*)-4-((2-(2,6-dioxopiperidin-3-yl)-1-oxoisoindolin-5-yl)amino)methyl)cyclohexyl)methyl) phenyl)cyclopropane-1-carbonitrile (154 mg, 0.22 mmol) as the lower spot, with a yield of 36%. LC/MS (ESI⁺) calcd for C₄₃H₄₆N₆O₄ (M + H⁺) *m*/*z,* 711.4; found, 711.3. ¹H NMR (400 MHz, CDCl₃) *δ* 8.08 (s, 1H), 7.63 (d, *J* = 8.3 Hz, 1H), 7.35 (d, *J* = 7.8 Hz, 1H), 7.09 (dd, *J* = 15.1, 6.4 Hz, 4H), 6.63 (d, *J* = 7.9 Hz, 1H), 6.55 (s, 1H), 6.46 (d, *J* = 8.7 Hz, 2H), 5.18 (dd, *J* = 13.1, 4.7 Hz, 1H), 4.37 (d, *J* = 15.4 Hz, 1H), 4.22 (d, *J* = 15.6 Hz, 1H), 3.46 (d, *J* = 6.6 Hz, 2H), 3.02 (d, *J* = 6.4 Hz, 2H), 2.90 - 2.79 (m, 2H), 2.42 (s, 3H), 2.28 (s, 3H), 2.08 (s, 3H), 1.97 - 1.90 (m, 4H), 1.60 (dd, *J* = 7.4, 4.8 Hz, 4H), 1.31 - 1.26 (m, 3H), 1.03 (dd, *J* = 20.1, 10.8 Hz, 5H).

In the following, the beneficial effect of the present invention was demonstrated by experimental examples.

### Experimental example 1 The inhibitory activity of the compound according to the present invention on the proliferation of prostate cancer cells

### 1. Biological assay of the inhibition on the proliferation of LNCap/AR cells

(1) Experimental materials and instruments:
   LNCaP/AR cell line and 22RV1 cell line (provided by Sichuan Kangcheng Biotechnology Co., Ltd.)
   Fetal bovine serum FBS (Gibco, Cat. No. 10099-141)
   0.01M PBS (Biosharp, Cat. No. 162262)
   RIPM1640 medium (Hyclone, Cat. No. 308090.01)
   Penicillin-Streptomycin (Hyclone, Cat. No. SV30010)
   Cell counting kit-8 (Signalway Antibody, Cat. No. CP002)
   DMSO (Sigma, Cat. No. D5879)
   Centrifuge Tube, 15 ml (Excell Bio, Cat. No. CS015-0001)
   Cell Culture Dish, (Excell Bio, Cat. No. CS016-0128)
   96-well cell culture plate (Corning, Cat. No. 3599)
   Microplate reader (Thermo Multiskan MK3 type)
(2) Experimental methods:
   a. Preparation of buffer
      Cell culture medium: RIPM1640 medium, 10% FBS, 1% Pen Strep;
      PBS buffer: PBS powder was dissolved in 2 L of ultrapure water and sterilized.
   b. Experimental procedures
      1) LNCaP/AR cells were subcultured in the cell culture medium, and then the cells in good growth condition were seeded in a 96-well plate at 80 µL/well (1000 cells/well), and cultured overnight in a 37 °C, 5% CO₂ cell incubator.
      2) The drug was prepared into a 10 mM stock solution with dimethyl sulfoxide (DMSO). Prior to use, the stock solution was diluted in a ratio of 1:3 with DMSO, and then diluted at a 3-fold gradient to obtain 9 serial concentrations. Then, each concentration of compound was further diluted in a ratio of 1:200 with the culture medium (to ensure that the DMSO concentration in the culture system was 0.1%), and two wells were set for each concentration. 20 µL of the diluted compound solution was added to the well of the cell culture (with a final concentration of 10 µM, 3.3 µM, 1.1 µM...), and then the plate was gently tapped to throroughly mix. In addition, the experiment included 3 negative control wells only containing cells and 3 blank control wells only containing culture medium (6 wells were each added with DMSO diluted in a ratio of 1:200 with 20 µL of culture medium).
   c. Result detection:
      1) After culturing for 6 days, 10 µL of CCK-8 was added to each well, and the plate was further incubated for 1 h in a 37°C, 5% CO₂ cell incubator.
      2) The absorbance (OD value) was measured at 450 nm with a multifunctional microplate reader.
      3) The data was analyzed by the Dose-response-inhibition equation in the software GraphPad Prism5, and the IC₅₀ value was obtained.

### 2. Biological assay of the inhibition on the proliferation of other drug-resistant prostate cancer cells

The same test method was used to determine the inhibitory activity of the compound according to the present invention against drug-resistant prostate cancer cell 22RV1.

**Table 1. The inhibitory activities of compounds 1-87 according to the present invention against prostate cancer cells LNCap/AR and 22RV1.**

| **Co mpo und** | **LNCap /AR IC₅₀** | **22RV1 IC₅₀** | | **Co mp ou nd** | **LNCap/AR IC₅₀** | **22RV1 IC₅₀** | | **Co mp oun d** | **LNCap /AR IC₅₀** | **22RV1 IC₅₀** |
|---|---|---|---|---|---|---|---|---|---|---|
| **1** | D | --- | | **30** | B | --- | | **59** | C | --- |
| **2** | D | --- | | **31** | B | A | | **60** | C | --- |
| **3** | B | --- | | **32** | B | A | | **61** | A | A |
| **4** | D | --- | | **33** | B | --- | | **62** | A | A |
| **5** | D | --- | | **34** | D | D | | **63** | A | A |
| **6** | C | --- | | **35** | D | D | | **64** | C | A |
| **7** | D | --- | | **36** | --- | --- | | **65** | C | B |
| **8** | C | --- | | **37** | C | B | | **66** | B | A |
| **9** | C | --- | | **38** | D | C | | 67 | B | A |
| **10** | A | --- | | **39** | C | A | | **68** | A | A |
| **11** | C | --- | | **40** | C | B | | **69** | D | D |
| **12** | B | --- | | **41** | B | A | | **70** | C | A |
| **13** | D | --- | | **42** | B | C | | **71** | D | D |
| **14** | D | --- | | **43** | C | A | | **72** | D | D |
| **15** | D | --- | | **44** | B | D | | **73** | D | D |
| **16** | D | --- | | **45** | C | D | | **74** | D | D |
| **17** | D | --- | | **46** | D | --- | | **75** | D | D |
| **18** | D | --- | | **47** | C | --- | | **76** | D | D |
| **19** | A | --- | | **48** | C | --- | | **77** | D | D |
| **20** | A | --- | | **49** | C | --- | | **78** | D | D |
| **21** | C | --- | | **50** | C | --- | | **79** | B | D |
| **22** | C | --- | | **51** | D | --- | | **80** | C | D |
| **23** | D | --- | | **52** | B | --- | | **81** | C | --- |
| **24** | D | --- | | **53** | A | --- | | **82** | B | --- |
| **25** | D | --- | | **54** | D | --- | | **83** | A | --- |
| **26** | B | --- | | **55** | D | --- | | **84** | C | --- |
| **27** | C | --- | | **56** | B | --- | | **85** | B | --- |
| **28** | B | --- | | **57** | B | --- | | **86** | B | --- |

| | | | | | | | | | | |
|---|---|---|---|---|---|---|---|---|---|---|
| **29** | D | --- | | **58** | B | --- | | **87** | C | --- |

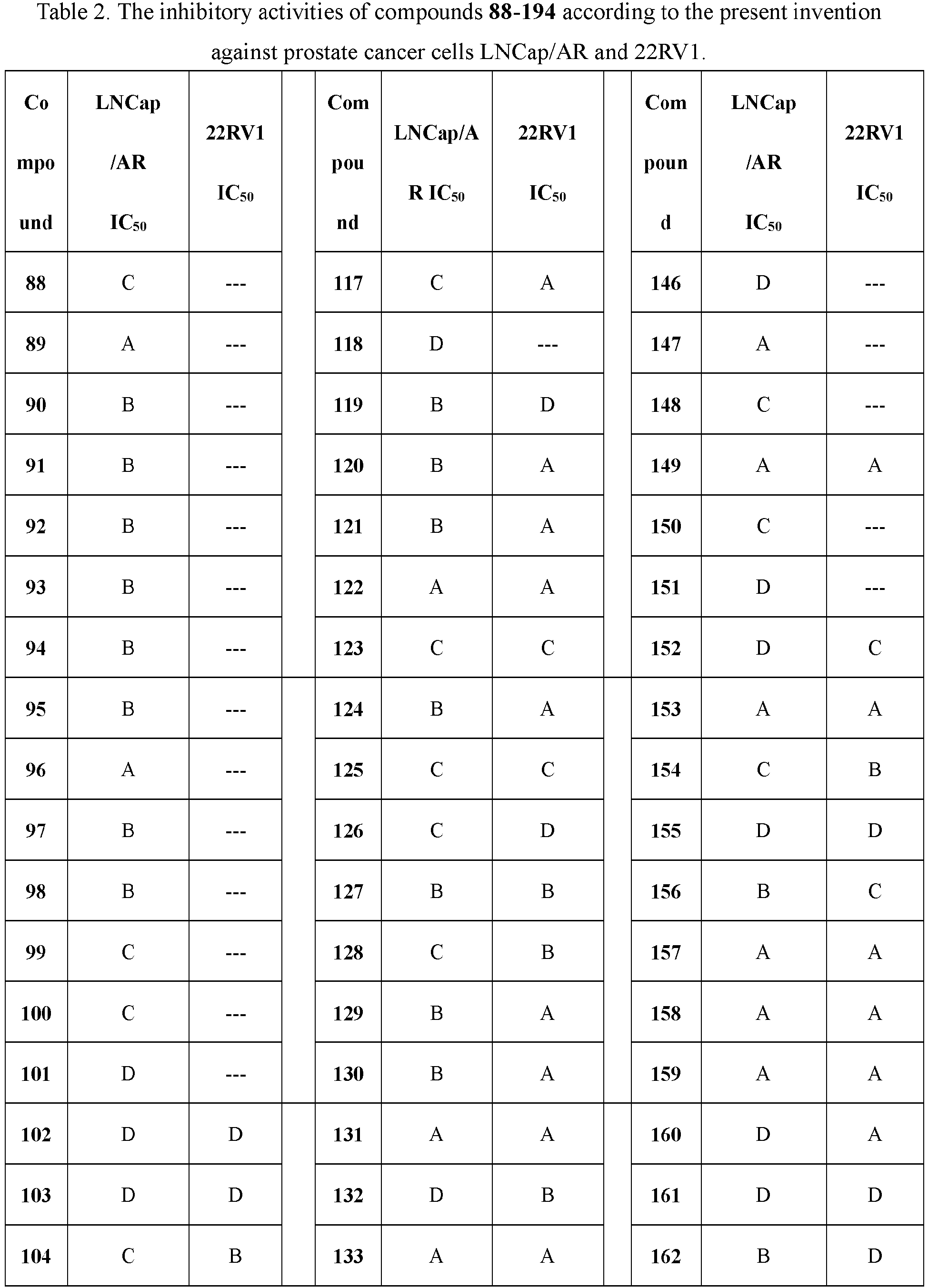

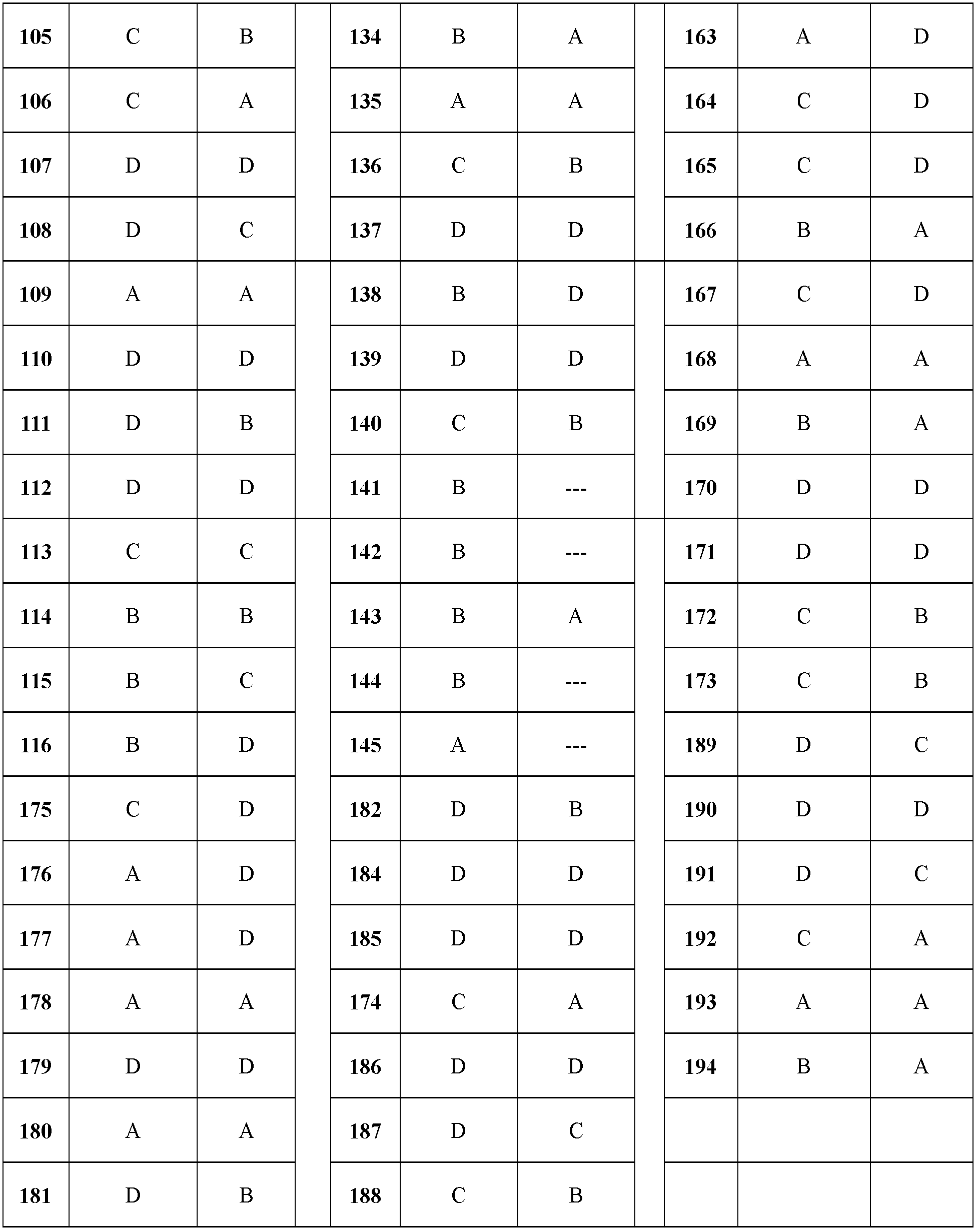

In Tables 1 and 2, A: IC₅₀ < 100 nM; B: IC₅₀: 101 nM - 500 nM; C: IC₅₀ 501 nM - 2000 nM; D: IC₅₀ >2001 nM; ---: not tested

As shown in Tables 1 and 2, the compounds of the present invention could not only effectively inhibit the proliferation of LNCap/AR cells with overexpression of androgen receptor AR, but also had good inhibitory effect on the prostate cancer cell line 22RV1, which was resistant to the marketed prostate cancer drug (enzalutamide).

### Experimental example 2. The down-regulation on the protein expression of androgen receptors AR and Brd4 determined by Western blot.

### 1. Experimental materials:

CWR22RV1 cells (National Collection of Authenticated Cell Cultures, TCHu100) FBS (Gibco, Cat. No. 10099-141)
0.01M PBS (Biosharp, Cat. No. 162262)
RIPM1640 (Hyclone, Cat. No. 308090.01)
Penicillin-Streptomycin (Hyclone, Cat. No. SV30010)
DMSO (Sigma, Cat. No. D5879)
Centrifuge tube, 15 ml (Excell Bio, Cat. No. CS015-0001)
Cell culture plate (Excell Bio, Cat. No. CS016-0128)
6-well cell culture cluster (Corning, Cat. No. 3516)
RIPA lysate buffer (Beyotime, Cat. No. P0013B)
Protein loading buffer (Beyotime, Cat. No. P0015L)
CA protein assay kit (Beyotime, Cat. No. P0012)
SDS-PAGE gel preparation kit (Chengdu Baihe Technology Co., Ltd, Cat. No. PG112)
Anti-β-tubulin mouse mAb (Zen Bioscience, Cat. No. 200608)
Androgen Receptor (D6F11) XP Rabbit mAb (CST, Cat. No. 5153)
Anti-cMyc(D3N8F) Rabbit mAb (CST, Cat. No. 13987)
Anti-BRD4(E2A7X) rabbit mAb (CST, Cat. No. 13440)
Peroxidase Affinipure(HRP) Goat Anti-Mouse IgG (Zen Bioscience, Cat. No. 511103)
Peroxidase Affinipure(HRP) Goat Anti-Rabbit IgG (Zen Bioscience, Cat. No. 511203)
TBST (Biosharp, Cat. No. BL601A)
ECL chemiluminescence kit (Beyotime, Cat. No. P0018)

### 2. Experimental methods:

(1) Preparation of buffer

| Cell culture medium: | PBS buffer: | Cell lysate: |
|---|---|---|
| RIPM1640 medium, 10% FBS, 1% Pen Strep. | PBS powder was dissolved in 2 L of ultrapure water and sterilized. | Protease inhibitor was added in RIPA lysate buffer in a ratio of 1:1000 prior to use. |

(2) Experimental procedures:
1) CWR22RV1 cells were subcultured in the cell culture medium, and then the cells in good growth condition were seeded in a 6-well plate at 2 ml/well (1×10⁶ cells/well), and cultured overnight in a 37 °C, 5% CO₂ cell incubator.
2) The drug was prepared as a 10 mM stock solution with dimethyl sulfoxide (DMSO). Prior to use, the stock solution was diluted in a ratio of 1:3 with DMSO. 2 µL of the diluted compound solution was added to the cell culture well (ensuring that the DMSO concentration in the culture system was 0.1%), and two wells were set for each concentration. The plate was gently tapped to thoroughly mix. In addition, the experiment included negative control wells (containing equal amount of DMSO) and positive control wells.
3) After culturing 24 h, the cells were lysed with RIPA cell lysate, and the protein was extracted. The protein concentration was detected with BCA kit. 5-fold concentrated protein loading buffer was added, and after heated at 100 °C for 5 min, the sample was stored at -20 °C.
4) For each well, 30 µg of protein was loaded on polyacrylamide gel for electrophoresis.
5) The protein was transferred from polyacrylamide gel to PVDF membrane, and then 5% skim milk was added to block for 1 h at room temperature. The first antibody (Androgen Receptor (D6F11) XP Rabbit mAb, Anti-cMyc(D3N8F) Rabbit mAb, Anti-BRD4(E2A7X) rabbit mAb and Anti-β-Tubulin Mouse mAb) was added and incubated overnight at 4 °C. The membrane was rinsed with TBST solution three times, 10 min for each time. The secondary antibody (horseradish peroxidase labeled goat anti mouse IgG) was added and incubated at room temperature for 2 h, and then the membrane was washed with TBST solution three times, 10 min for each time.

Finally, the ECL chemiluminescence solution was added, and then photos were taken with automatic chemiluminescence instrument to collect pictures for analysis.

### 3. Experimental results:

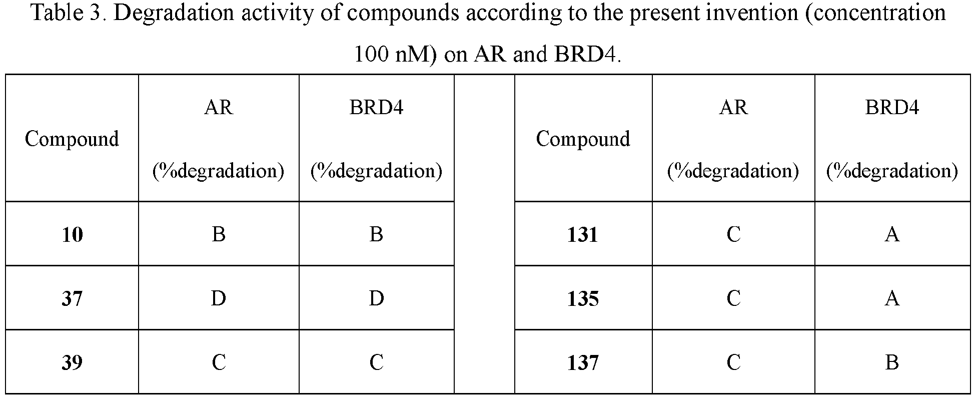

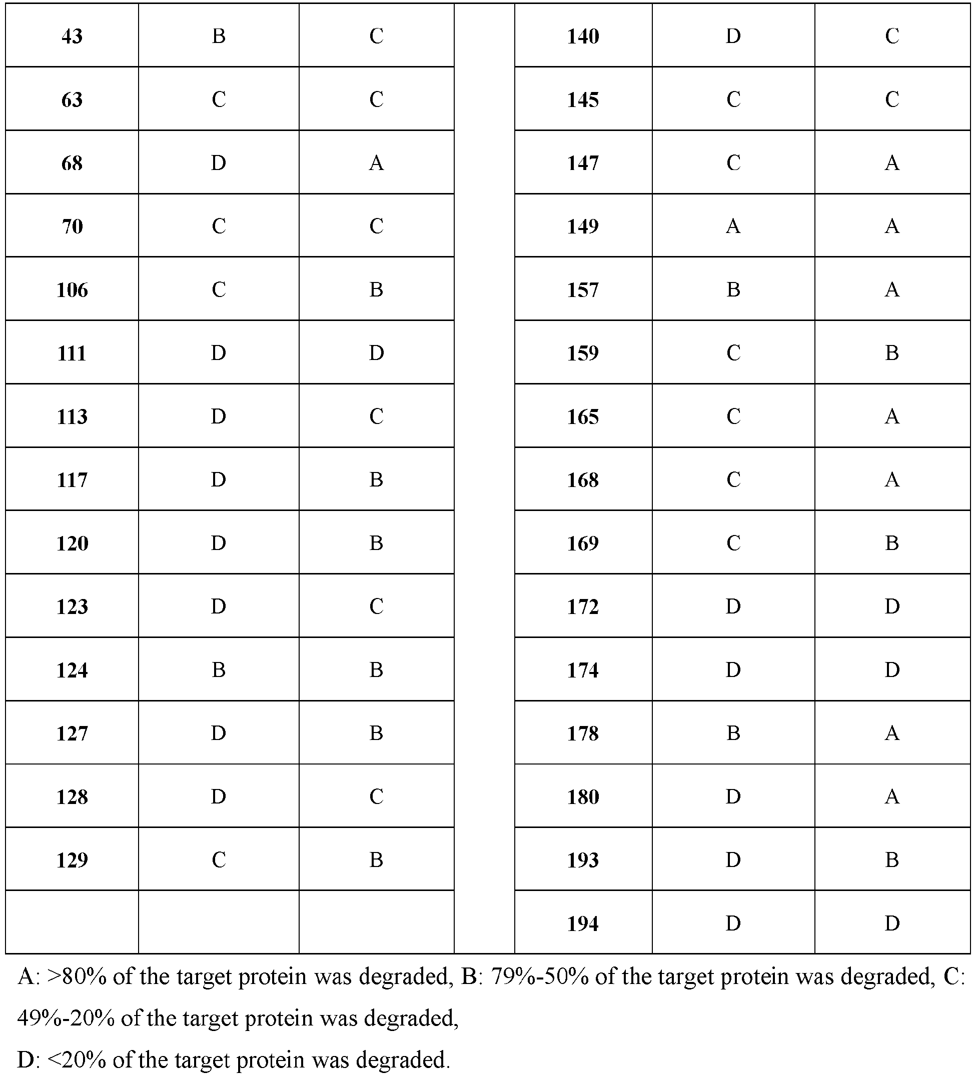

The degradation activity of the compound according to the present invention on AR and BRD4 at the concentration of 100 nM was shown in Table 3. As shown, the compound of the present invention showed a targetting degradation on both AR and BRD4, together with the down-regulation on the protein expression of AR and BRD4.

### Experiment example 3: Experiment on metabolic stability of the compound of the present invention

### 1. Materials and instruments

HPLC (Shimadzu), MS (API 4000 instrument from AB Inc (Canada) with an ESI interface), chromatographic column (ACE Excel 3 AQ 30×2.1mm Column), human hepatic drug enzyme (Corning, Cat. #452117), phosphate buffer, ultrapure water, MgCl₂ solution, NADPH.

### 2. Methods

10 µl of 20 mg/ml liver microsomes and 40 µl of 10 mM NADPH were added to the incubation tube. The final concentrations of liver microsomes and NADPH were 0.5 mg/mL and 1 mM, respectively. At the same time, a group without NADPH and only containing the same amount of ultrapure water was used as the control group. Then, 4 µl solution of control compound (verapamil) or test compound at the concentration of 200 µM was added. The final concentration of the compound was 2 µM. 50 µl of the reaction solution was collected when incubating for 0 min, 15 min, 30 min, 45 min and 60 min, respectively, and 4-fold volume of ice acetonitrile was added to the solution to stop the reaction. After the sample was centrifuged for 40 min (3220 g), 100 µl of the supernatant was taken out, to which was added 100 µl of ultrapure water, followed by mixing well for LC-MS/MS detection. Finally, the pharmacokinetic parameters were calculated.

The experimental results showed that the compound of the present invention had good metabolic stability.

In summary, the present invention provided proteolytic targeting chimera compounds, which showed targeting degradation on both AR and BRD4, as well as down-regulation on the protein expression of AR and BRD4. The compound of the present invention could inhibit the proliferation of a variety of prostate cancer cells; the compound could inhibit the proliferation of a prostate cancer cell line LNCaP/AR with overexpression of AR, and could have a good inhibition effect on a prostate cancer cell line 22RV1, which was resistant to a marketed prostate cancer drug (enzalutamide). The compound of the present invention also showed a good metabolic stability, and had a good application prospect in the preparation of an AR and/or BET proteolytic targeting chimera, and a drug for the treatment of related diseases regulated by the AR and BET.

## Claims

1. Compound of formula I, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a combination thereof, or a metabolite thereof, or a metabolic precursor thereof, or an isotopic compound thereof: 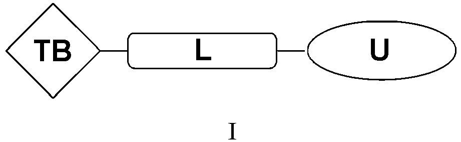 wherein, TB is an androgen receptor (AR) and/or a BET target recognition/binding part, L is the linker part, and U is a ubiquitin protease recognition/binding part; and the three parts are connected by chemical bonds;
the structure of said TB is represented by formula (I-A): 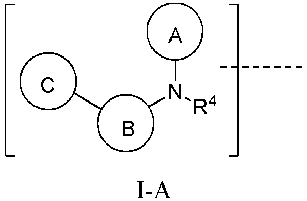 wherein, each of rings A, B and C is independently selected from the group consisting of none, substituted or unsubstituted unsaturated heterocycles, substituted or unsubstituted unsaturated carbocycles, and substituted or unsubstituted fused rings, and rings A, B and C are not none at the same time, preferably, each of rings A, B and C is independently selected from the group consisting of none, a substituted or unsubstituted monocyclic aromatic ring, a substituted or unsubstituted monocyclic heteroaromatic ring, and a substituted or unsubstituted fused ring; more preferably, each of rings A, B and C is independently selected from the group consisting of none, a substituted or unsubstituted 3-8 membered monocyclic aromatic ring, a substituted or unsubstituted 3-8 membered monocyclic heteroaromatic ring, a substituted or unsubstituted heteroaromatic-ring-fused heteroaromatic ring, a substituted or unsubstituted benzene-fused aromatic ring, a substituted or unsubstituted benzene-fused heteroaromatic ring, a substituted or unsubstituted benzene-fused saturated carbocycle, and a substituted or unsubstituted benzene-fused saturated heterocyclic ring;
each of the substituents in above rings A, B and C is independently selected from the group consisting of deuterium, halogen, -CN, hydroxyl, nitro, amino, 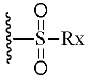 , -Qo-OH, -Q₃-C(O)R⁷, -Q₄-CO(O)R⁸, -Q₅-(O)COR⁹, -Q₆-NHC(O)R¹⁰, -Q₇-C(O)NHR¹¹, -Q₈-CN, alkenyl substituted with one or more R¹², alkynyl substituted with one or more R¹³, alkyl substituted with one or more R¹, alkoxy substituted with one or more R², aryl or heteroaryl substituted with one or more R³, cycloalkyl substituted with one or more R⁵, and heterocyclic group substituted with one or more R⁶; each of R_{X}, R¹, R², R³, R⁵, R⁶, R⁷, R⁸, R⁹, R¹⁰, R¹¹, R¹², and R¹³ is independently selected from the group consisting of H, deuterium, halogen, -CN, hydroxyl, nitro, amino, alkyl or deuterated compounds thereof or halogenated compounds thereof, and -Qo-OH, wherein each of Q₀, Q₃, Q₄, Q₅, Q₆, Q₇, and Q₈ is independently selected from the group consisting of none, C₁-C₈ alkyl, and C₃-C₆ cycloalkyl;
R⁴ is selected from the group consisting of none, hydrogen, deuterium, halogen, -CN, hydroxyl, nitro, amino, 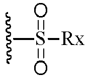 , -Q₀₋OH, -Q₃-C(O)R⁷, -Q₄-CO(O)R⁸, -Q₅-(O)COR⁹, -Q₆-NHC(O)R¹⁰, -Q₇-C(O)NHR¹¹, alkenyl substituted with one or more R¹², alkynyl substituted with one or more R¹³, alkyl substituted with one or more R¹, alkoxy substituted with one or more R², aryl or heteroaryl substituted with one or more R³, cycloalkyl substituted with one or more R⁵, and heterocyclic group substituted with one or more R⁶; each of R_{X}, R¹, R², R³, R⁵, R⁶, R⁷, R⁸, R⁹, R¹⁰, R¹¹, R¹², and R¹³ is independently selected from the group consisting of H, deuterium, halogen, -CN, hydroxyl, nitro, amino, alkyl or deuterated compounds thereof or halogenated compounds thereof, and -Q₀₋OH, wherein each of Q₀, Q₃, Q₄, Q₅, Q₆, and Q₇ is independently selected from 0-8 methylene groups;
or, any two groups of substituents in rings A, B, C and R⁴, together with the substituted atoms to which they are linked, are connected to form a ring; 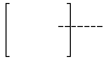 represents that formula I-A is the remaining group of the molecule 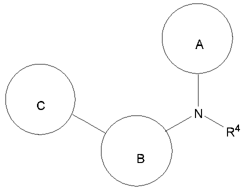 after removing any hydrogen.

2. The compound according to claim 1, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a combination thereof, or a metabolite thereof, or a metabolic precursor thereof, or an isotopic compound thereof, **characterized in that** said TB has the structure of formula III: 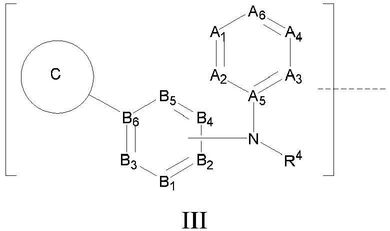 B₁ is CR^{b1} or N; B₂ is CR^{b2} or N; B₃ is CR^{b3} or N; B₄ is CR^{b4} or N; B₅ is CR^{b5} or N; B₆ is C; A₁ is CR^{a1} or N; A₂ is CR^{a2} or N; A₃ is CR^{a3} or N; A₄ is CR^{a4} or N; A₅i s C; A₆ is CR^{a6} or N; each of R^{b1}, R^{b2}, R^{b3}, R^{b4}, R^{b5}, R^{a1}, R^{a2}, R^{a3}, R^{a4}, and R^{a6} is independently selected from the group consisting of hydrogen, deuterium, -CN, amino, nitro, halogen, -Q₀₋OH, 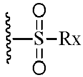 -Q₇-C(O)NHR¹¹, C₁-C₅ alkyl or deuterated compounds thereof or halogenated compounds thereof or cyanated compounds thereof, C₁-C₅ alkoxy or deuterated compounds thereof or halogenated compounds thereof or cyanated compounds thereof, a substituted or unsubstituted 3-6 membered cycloalkyl, a substituted or unsubstituted 4-6 membered unsaturated heterocyclic group, and a substituted or unsubstituted 5-6 membered heteroaryl group, or two adjacent substituents in the ring, together with the substituted atoms to which they are linked, form substituted or unsubstituted 3-6 membered heterocycles; wherein, each of the substituents in said 3-6 membered cycloalkyl, said 4-6 membered unsaturated heterocyclic group, and said 5-6 membered heteroaryl group is independently selected from the group consisting of -CN, amino, nitro, halogen, C₁-C₃ alkyl or deuterated compounds thereof or halogenated compounds thereof, and -Q₁-OH; each of Q₀ and Q₁ is independently selected from 0-5 methylene groups; each of Rₓ and R¹¹ is independently selected from the group consisting of H, deuterium, and C₁-C₃ alkyl;
ring C and R⁴ are as described in claim 1;
said isotope-substituted form is a deuterated compound.

3. The compound according to claim 2, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a combination thereof, or a metabolite thereof, or a metabolic precursor thereof, or an isotopic compound thereof, **characterized in that** said TB has the structure of formula VI-A: 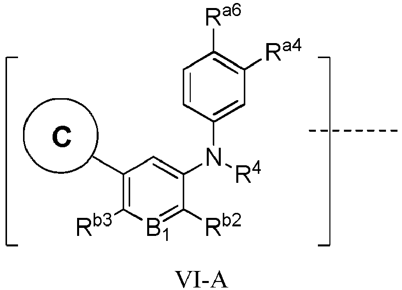 wherein, each of R^{a4} and R^{a6} is independently selected from the group consisting of H, deuterium, halogen, CN, C₁-C₅ alkyl or deuterated compounds thereof or halogenated compounds thereof or cyanated compounds thereof, C₁-C₅ alkoxy or deuterated compounds thereof or halogenated compounds thereof or cyanated compounds thereof, amino, amido, a substituted and unsubstituted 3-6 membered saturated cycloalkyl, and a substituted or unsubstituted 4-6 membered unsaturated heterocyclic group; wherein each of the substituents in said saturated cycloalkyl and said unsaturated heterocyclic group is independently selected from halogen, CN, a deuterated or non-deuterated C₁-C₂ alkyl, and -Q₁-OH; Q₁ is selected from 0-2 methylene groups;
R⁴ is selected from the group consisting of none, H, and a deuterated or non-deuterated C₁-C₂ alkyl;
B₁ is selected from the group consisting of CR^{b1} and N; R^{b1} is selected from the group consisting of H, deuterium, and halogen; preferably, B₁ is CH;
R^{b3} is selected from the group consisting of H, deuterium, and halogen;
R^{b2} is selected from the group consisting of deuterated or non-deuterated methyl and ethyl;
ring C is as described in claim 2.

4. The compound according to any one of claims 1 to 3, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a combination thereof, or a metabolite thereof, or a metabolic precursor thereof, or an isotopic compound thereof, **characterized in that**:
ring C is selected from a 5-membered monocyclic heteroaromatic ring substituted with 0-4 substituents; the heteroatom in said 5-membered monocyclic heteroaromatic ring is selected from one or more of O, S and N; each of the substituents is independently selected from the group consisting of deuterium, halogen, C₁-C₆ alkyl and C₃-C₆ cycloalkyl.

5. The compound according to claim 4, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a combination thereof, or a metabolite thereof, or a metabolic precursor thereof, or an isotopic compound thereof, **characterized in that** ring C is one of the following structures:

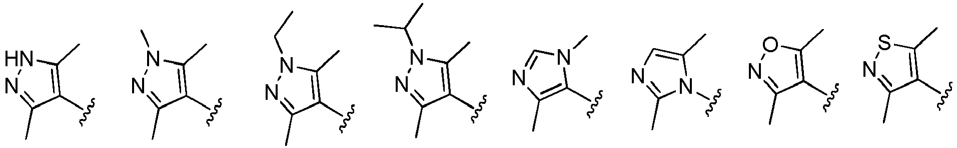

6. The compound according to claim 2, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a combination thereof, or a metabolite thereof, or a metabolic precursor thereof, or an isotopic compound thereof, **characterized in that** ring C is none, and said TB has the structure of formula VI-B: 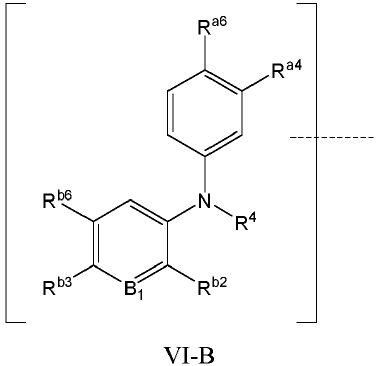 wherein, each of R^{a4} and R^{a6} is independently selected from the group consisting of H, deuterium, halogen, CN, C₁-C₅ alkyl or deuterated compounds thereof or halogenated compounds thereof or cyanated compounds thereof, C₁-C₅ alkoxy or deuterated compounds thereof or halogenated compounds thereof or cyanated compounds thereof, amino, amido, a substituted and unsubstituted 3-6 membered saturated cycloalkyl, and a substituted or unsubstituted 4-6 membered unsaturated heterocyclic group; wherein each of the substituents in said saturated cycloalkyl and said unsaturated heterocyclic group is independently selected from the group consisting of halogen, CN, a deuterated or non-deuterated C₁~C₂ alkyl, and -Q₁-OH; Q₁ is selected from 0-2 methylene groups;
R⁴ is selected from the group consisting of none, H, and a deuterated or non-deuterated C₁-C₂ alkyl;
B₁ is selected from the group consisting of CR^{b1} and N; R^{b1} is selected from the group consisting of H, deuterium, and halogen; preferably, B₁ is CH;
R^{b2} is selected from the group consisting of deuterated or non-deuterated methyl and ethyl;
R^{b3} and R^{b6}, together with the substituted atoms to which they are linked, form a substituted or unsubstituted 5-membered unsaturated heterocycle; wherein each of the substituents in the 5-membered unsaturated heterocycle is independently selected from the group consisting of -CN, amino, nitro, halogen, C₁-C₂ alkyl or deuterated compounds thereof or halogenated compounds thereof, and -Q₁-OH; Q1 is selected from 0-2 methylene groups.

7. The compound according to any one of claims 1 to 6, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a combination thereof, or a metabolite thereof, or a metabolic precursor thereof, or an isotopic compound thereof, **characterized in that** the structure of TB is one of the following structures:

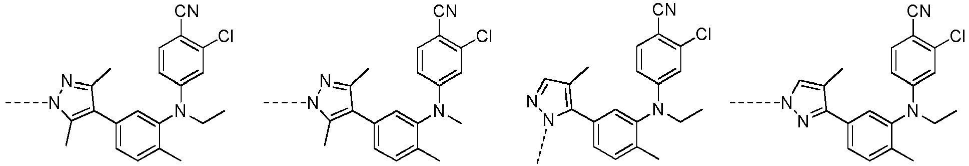

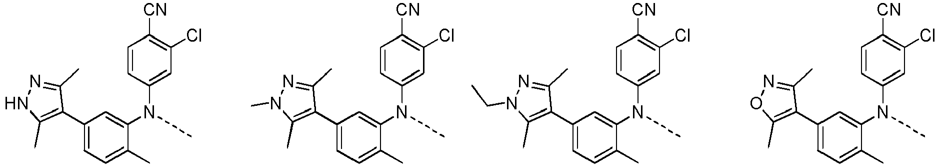

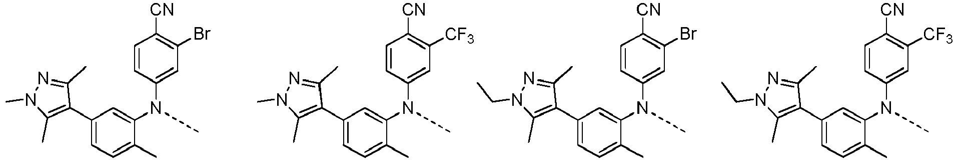

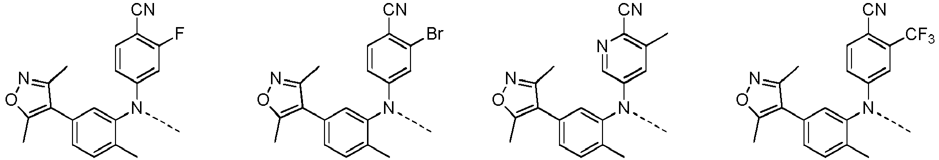

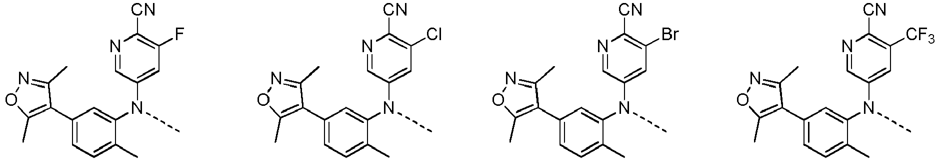

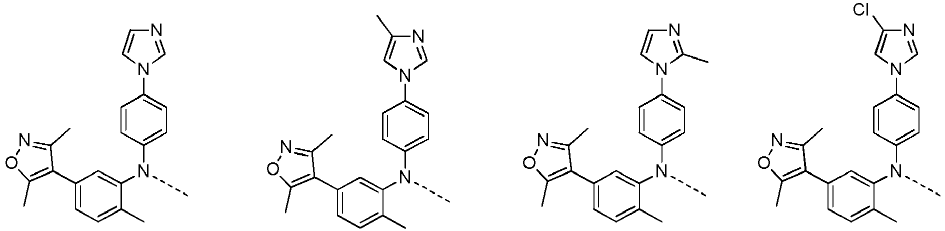

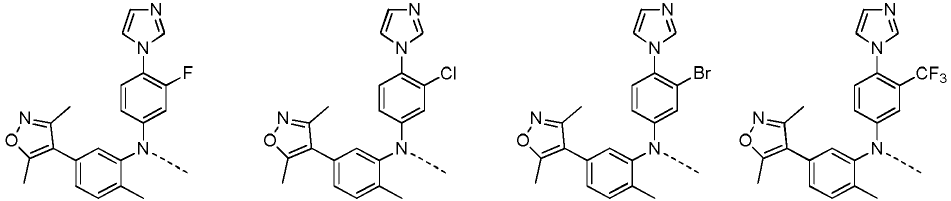

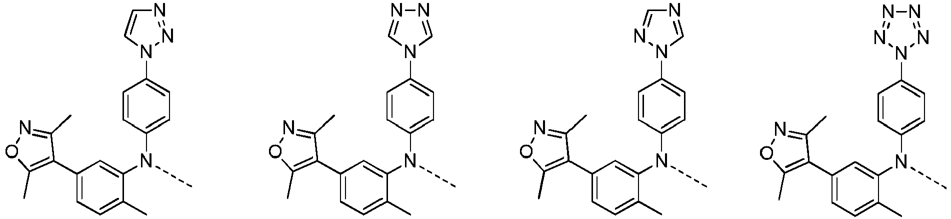

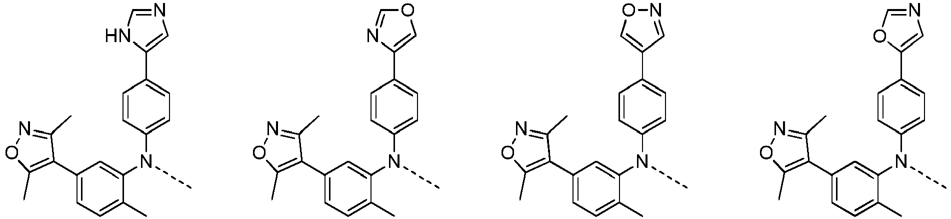

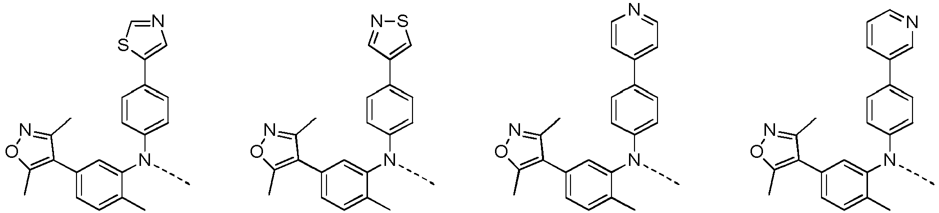

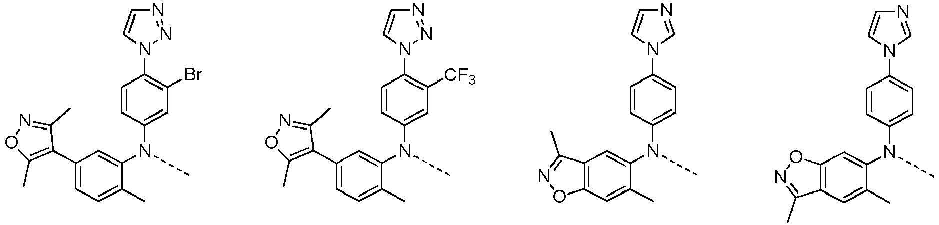

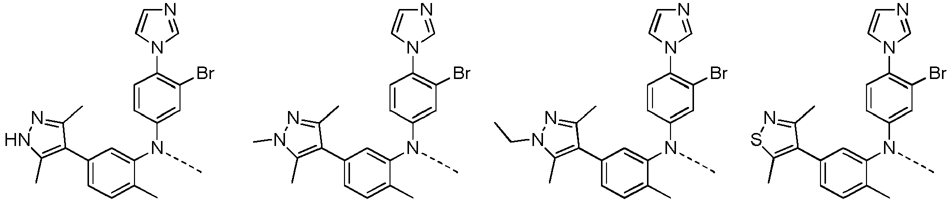

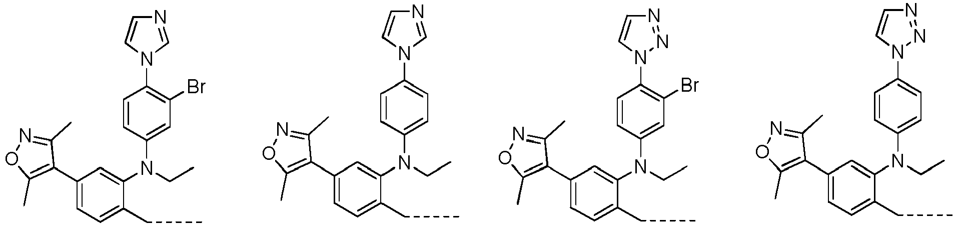

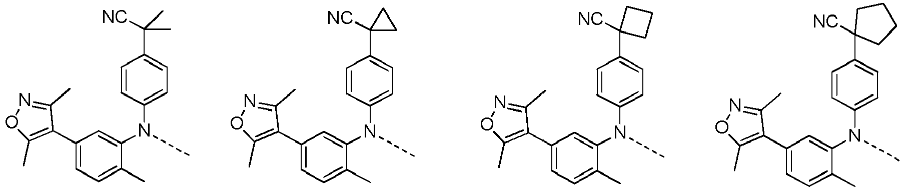

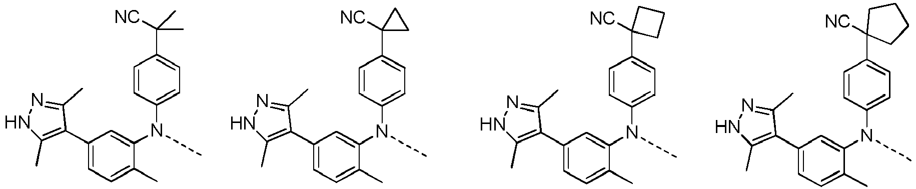

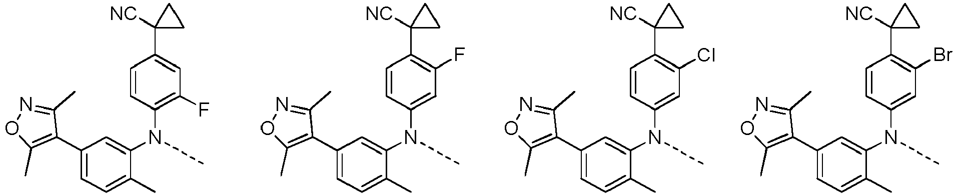

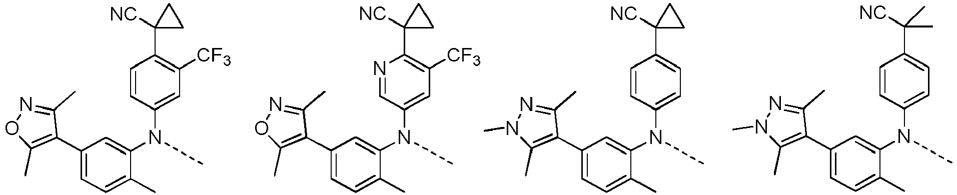

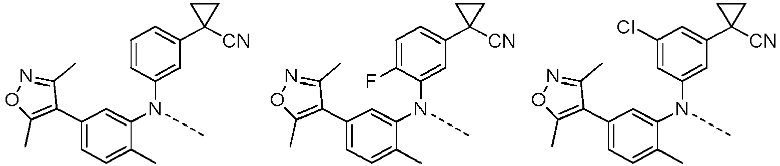

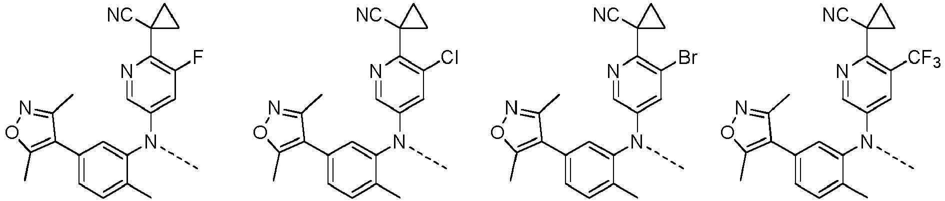

8. The compound according to any one of claims 1 to 7, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a combination thereof, or a metabolite thereof, or a metabolic precursor thereof, or an isotopic compound thereof, **characterized in that**:
said U has the structure of formula II-A: 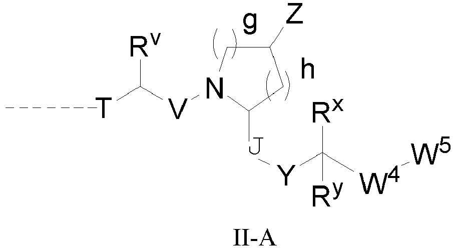 wherein, each of T and Y is respectively selected from the group consisting of none, O, S, NR^{T1}, and CR^{T2}R^{T3};
each of V and J is respectively selected from the group consisting of none, C=O, -SO-, -SO₂-, and CR^{s1}R^{s2};
each of R^{s1}, R^{s2}, R^{T1}, R^{T2}, and R^{T3} is respectively selected from the group consisting of H, deuterium, C₁₋₆ alkyl or a halogenated compound thereof or a deuterated compound thereof, and 3-8 membered cycloalkyl containing 0-2 heteroatoms, or R^{T2} and R^{T3} are linked to form a 3-8 membered ring containing 0-2 heteroatoms;
R^{v} is selected from the group consisting of H, deuterium, C₁₋₆ alkyl or a halogenated compound thereof or a deuterated compound thereof, and a cycloalkyl containing 0-3 heteroatoms or a halogenated compound thereof;
each of g and h is independently an integer of from 0 to 3, and g and h are not 0 at the same time;
Z is selected from the group consisting of H, deuterium, hydroxy, amino, C₁₋₆ alkyl, C₃₋₆ cycloalkyl, halogenated C₁₋₆ alkyl, -OR^{Z1}, -NR^{Z1}R^{Z2}, -COR^{Z3}, -CO₂R^{Z3}, -OCOR^{Z3}, -NHCOR^{Z3}, -CONHR^{Z3}, and -SO₂R^{Z3}; each of R^{Z1} and R^{Z2} is selected from the group consisting of H, deuterium, C₁₋₆ alkyl or a halogenated compound thereof or a deuterated compound thereof, and a 3-8 membered cycloalkyl with 0-2 heteroatoms; R^{Z3} is selected from the group consisting of substituted or unsubstituted C₁₋₆ alkyl, substituted or unsubstituted C₃₋₆ cycloalkyl, substituted or unsubstituted C₃₋₆ heterocyclic group, substituted or unsubstituted aryl, and substituted or unsubstituted heteroaryl; the substituent of R^{Z3} is selected from the group consisting of halogen and C₁₋₃ alkyl;
each of R^{x} and R^{y} is respectively selected from the group consisting of H, deuterium, C₁₋₆ alkyl, halogenated C₁₋₆ alkyl, C₁₋₆ alkyl substituted with the substituent containing a heteroatom, -L_{y}-OH, and a cycloalkyl with 0-3 heteroatoms or a halogenated compound thereof, or R^{x} and R^{y} are linked to form a 3-8 membered ring containing 0-2 heteroatoms; wherein, L_{y} is selected from the group consisting of 0-5 methylene groups;
each of W⁴ and W⁵ is respectively selected from the group consisting of ary and heteroaryl substituted with 0-3 substituents; each of said substituents is independently selected from the group consisting of H, deuterium, halogen, hydroxy, amino, thiol, sulfonyl, sulfoxide, nitro, cyano, CF₃, heterocyclic group, C₁₋₆ alkyl, C₃₋₆ cycloalkyl, C₁₋₆ alkoxy, C₁₋₆ alkylamino, C₂₋₆ alkenyl, and C₂₋₆ alkynyl;
or,
said U has the structure of formula II-B: 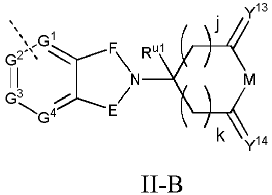 wherein, M is selected from the group consisting of O, S, and NR^{m}; wherein R^{m} is selected from the group consisting of H, deuterium, C₁₋₆ alkyl, C₃₋₆ cycloalkyl, C₃₋₆ heterocyclic group, and 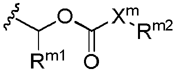 ;
said R^{m1} is selected from the group consisting of H, deuterium, C₁₋₆ alkyl, and C₃₋₆ cycloalkyl; X^{m} is selected from the group consisting of none, O, S, and NR^{m3};
each of R^{m2} and R^{m3} is respectively selected from the group consisting of H, deuterium, C₁₋₆ alkyl, C₃₋₆ cycloalkyl, C₃₋₆ heterocyclic group, 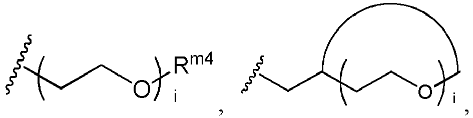 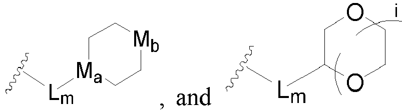 ; said i is an integer of from 0 to 12; R^{m4} is selected from the group consisting of H, deuterium, and C₁₋₆ alkyl; Lₘ is selected from the group consisting of 0-5 methylene groups; Mₐ is selected from the group consisting of N and CH; M_{b} is selected from the group consisting of O, S, CH₂, and NH;
each of E and F is respectively selected from the group consisting of CO, CS, NR^{e1}, O, S, SO₂, CH₂, CD₂, CR^{e2}R^{e3}, 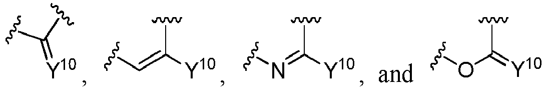 ; each of R^{e1}, R^{e2}, and R^{e3} is respectively selected from the group consisting of C₁₋₆ alkyl, C₁₋₆ alkoxy, H, deuterium, halogen, hydroxy, and amino;
each of Y¹⁰, Y¹³, and Y¹⁴ is respectively selected from the group consisting of O, S, and C₁₋₃ alkeylene;
each of j and k is respectively an integer of from 0 to 3, and j and k are not 0 at the same time; each of G¹, G², G³, and G⁴ is respectively selected from the group consisting of O, S, N, CR^{g1}, CR^{g2}, CR^{g3}, and CR^{g4}; wherein each of R^{g1}, R^{g2}, R^{g3}, and R^{g4} is respectively selected from the group consisting of H, deuterium, halogen, hydroxy, amino, thiol, sulfonyl, sulfoxide, nitro, cyano, CF₃, heterocyclic group, C₁₋₆ alkyl, C₃₋₆ cycloalkyl, C₁₋₆ alkoxy, C₁₋₆ alkylamino, C₂₋₆ alkenyl, and C₂₋₆ alkynyl;
R^{u1} is selected from the group consisting of H, deuterium, and C₁₋₆ alkyl;
or,
said U has the structure of II-C: 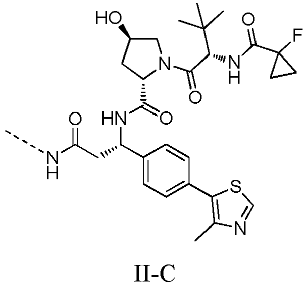 the isotope-substituted form is a deuterated compound.

9. The compound according to claim 8, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a combination thereof, or a metabolite thereof, or a metabolic precursor thereof, or a deuterated compound thereof, **characterized in that**:
said formula II-A has the structure of VIII-A: 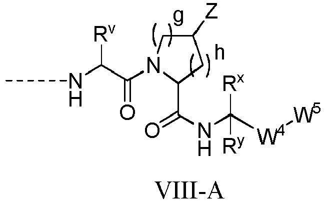 wherein, R^{v}, Z, g, h, R^{x}, R^{y}, W⁴, and W⁵ are as described in claim 8; or, in said formula II-B, 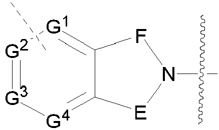 is selected from the group consisting of the structures of formulas (XI-B), (XI-C), (XI-D), (XI-E) and (XI-F): 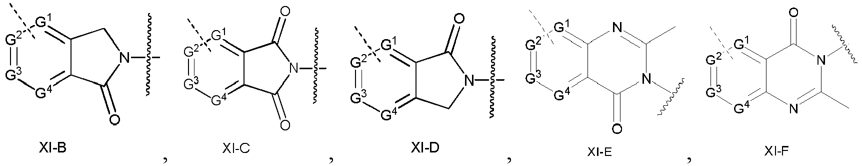 wherein, G¹, G², G³, and G⁴ are as described in claim 8.

10. The compound according to claim 9, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a combination thereof, or a metabolite thereof, or a metabolic precursor thereof, or an isotopic compound thereof, **characterized in that**:
said formula VIII-A has the structure of IX-A: 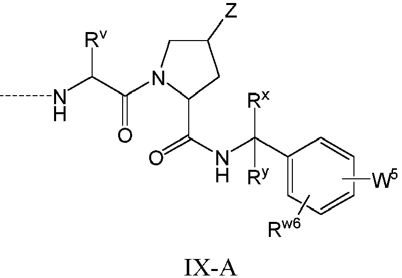 wherein, R^{w6} is selected from the group consisting of H, deuterium, halogen, hydroxy, amino, thiol, sulfonyl, sulfoxide, nitro, cyano, CF₃, heterocyclic group, C₁₋₆ alkyl, C₃₋₆ cycloalkyl, C₁₋₆alkoxy, C₁₋₆ alkylamino, C₂₋₆ alkenyl, and C₂₋₆ alkynyl;
W⁵ is selected from the group consisting of 5-6 membered aryl substituted with 0-3 substituents, and 5-6 membered heteroaryl; the heteroatom in said 5-6 membered heteroaryl is selected from one or more of O, S, and N; each of said substituents is respectively selected from the group consisting of halogen, hydroxy, amino, thiol, sulfonyl, sulfoxide, nitro, cyano, CF₃, heterocyclic group, C₁₋₆ alkyl, C₃₋₆ cycloalkyl, C₁₋₆alkoxy, C₁₋₆ alkylamino, C₂₋₆ alkenyl, and C₂₋₆ alkynyl; R^{v}, Z, R^{x}, and R^{y} are as described in claim 9.

11. The compound according to claim 10, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a combination thereof, or a metabolite thereof, or a metabolic precursor thereof, or an isotopic compound thereof, **characterized in that** said W⁵ is selected from the following structures:

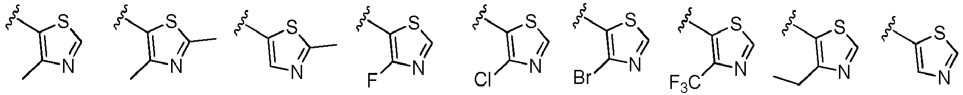

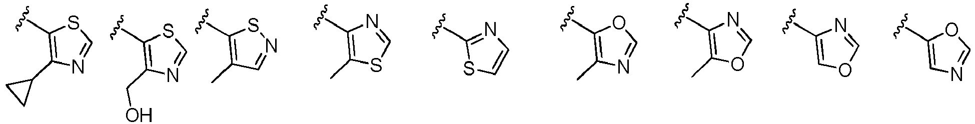

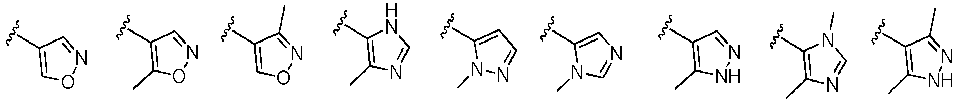

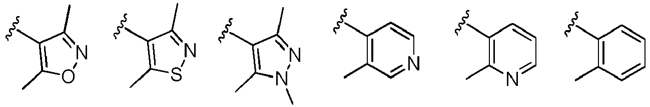

12. The compound according to any one of claims 8 to 11, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a combination thereof, or a metabolite thereof, or a metabolic precursor thereof, or an isotopic compound thereof, **characterized in that** said U is selected from the following structures:

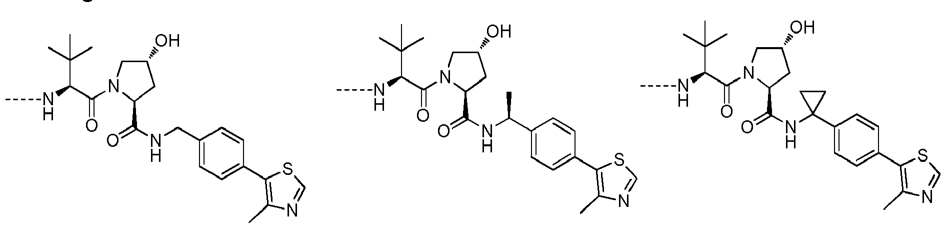

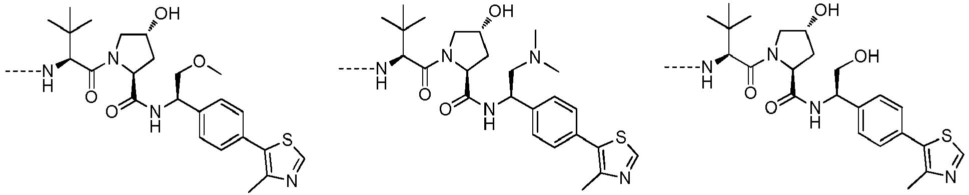

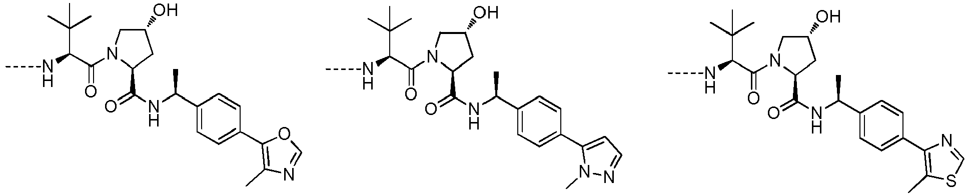

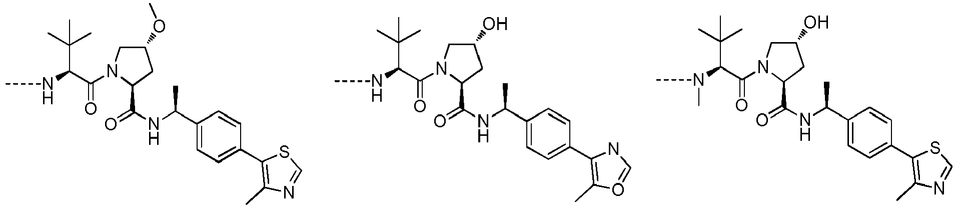

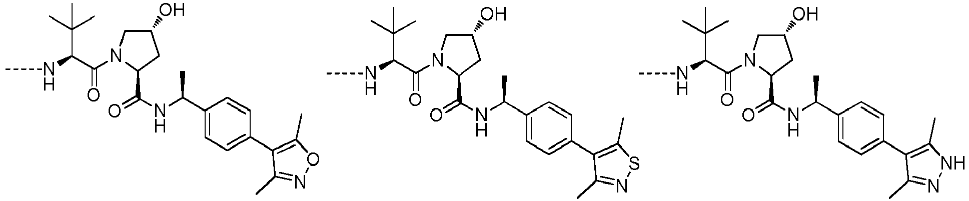

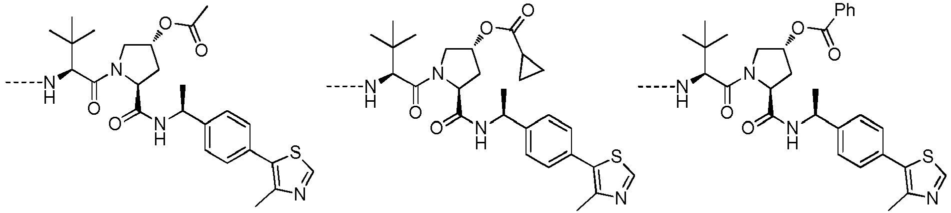

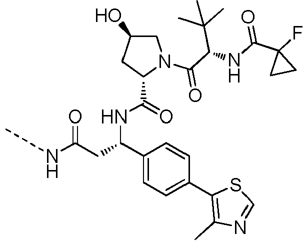

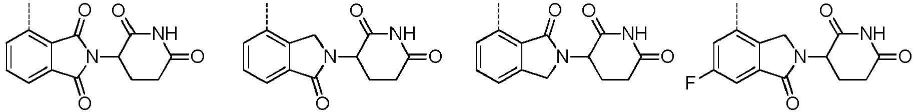

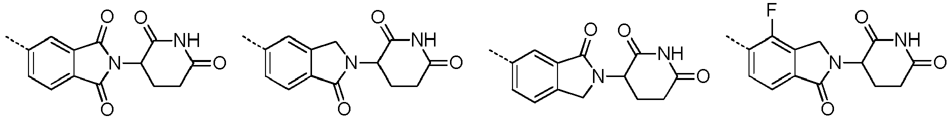

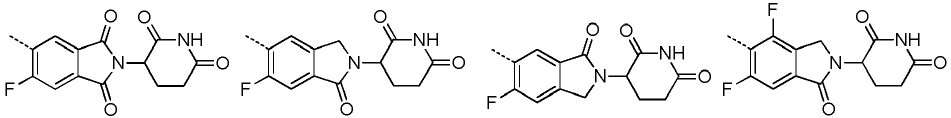

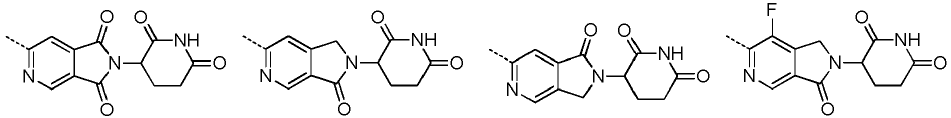

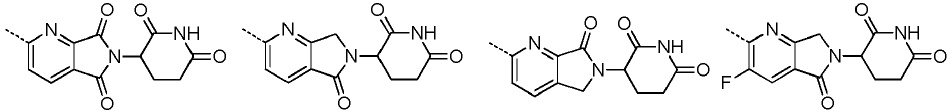

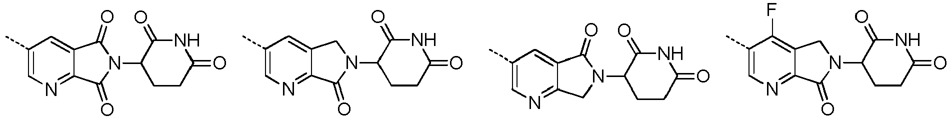

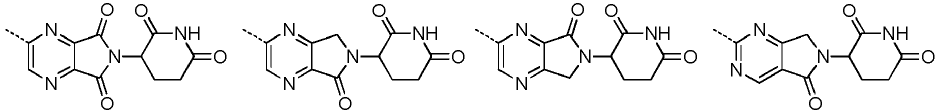

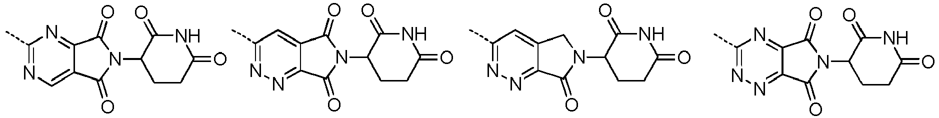

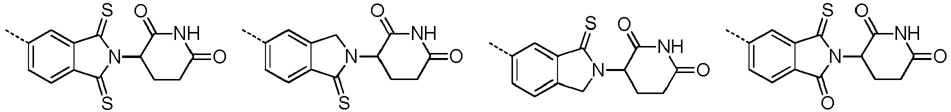

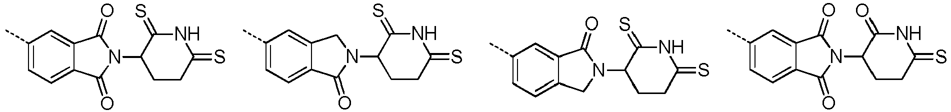

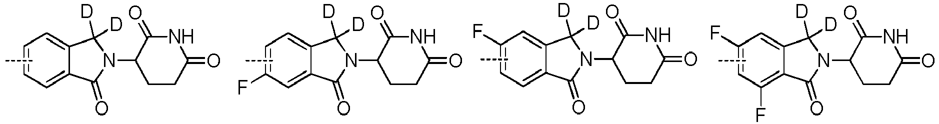

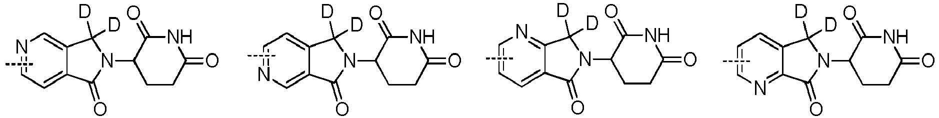

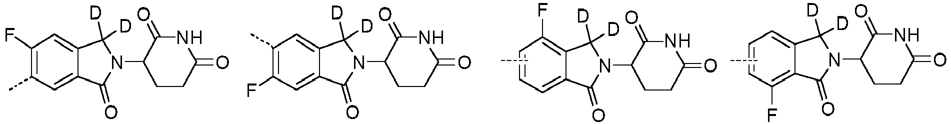

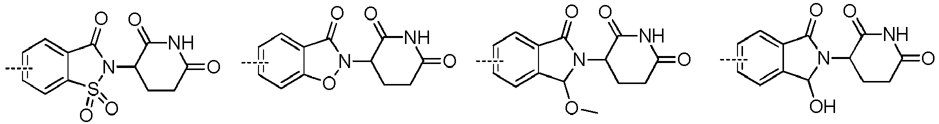

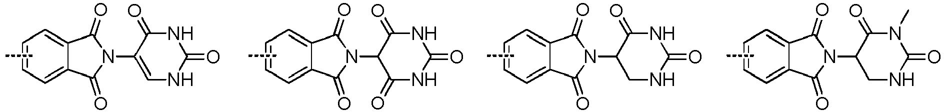

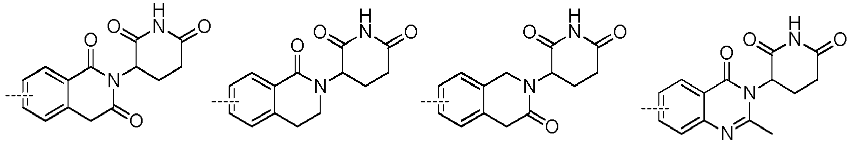

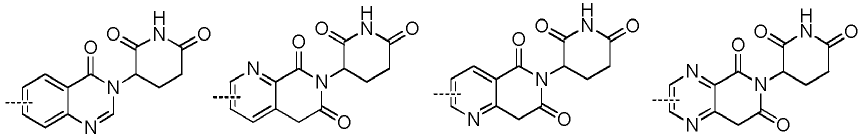

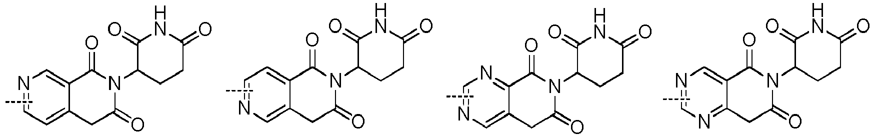

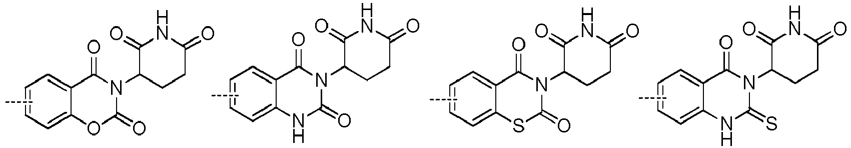

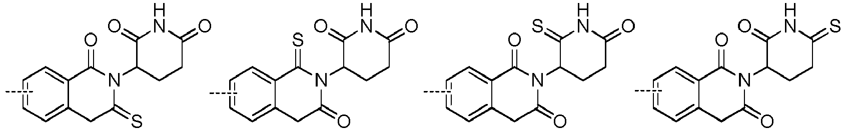

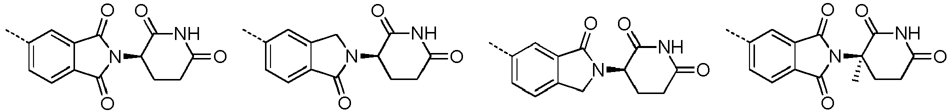

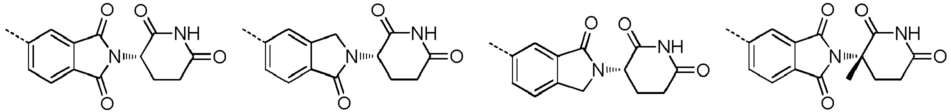

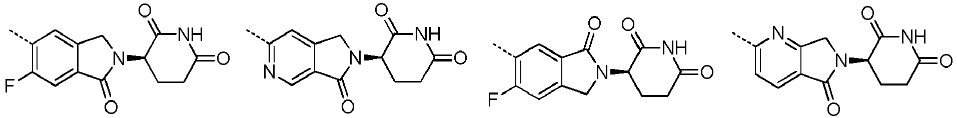

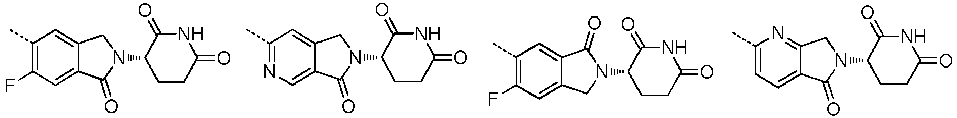

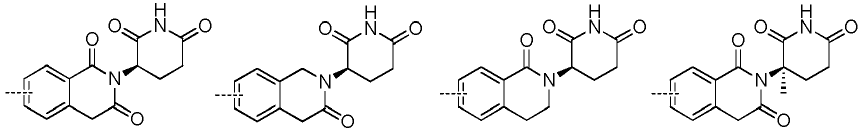

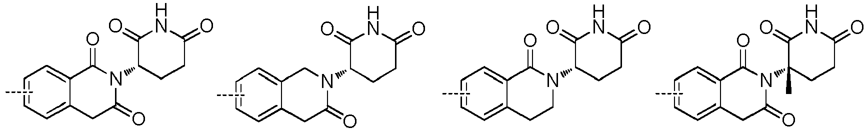

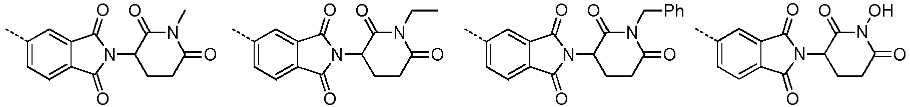

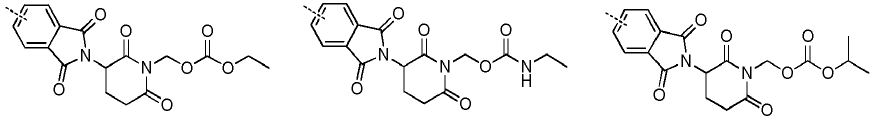

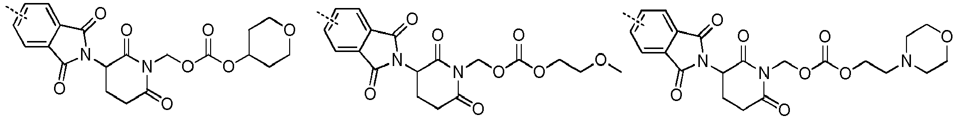

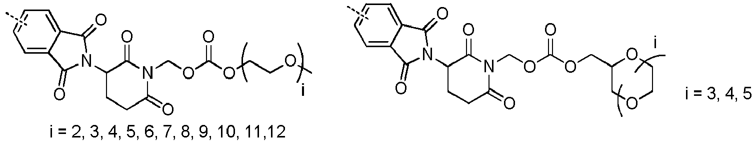

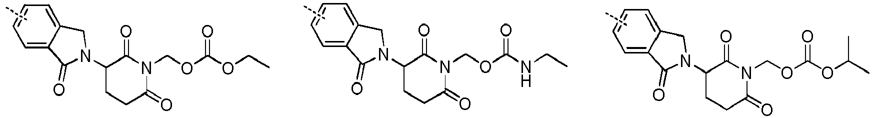

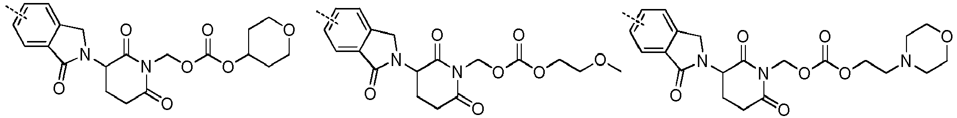

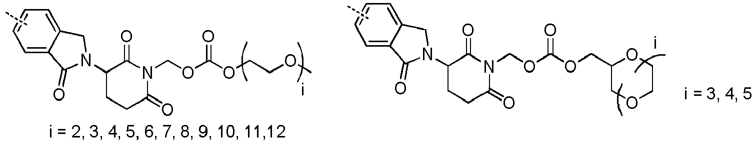

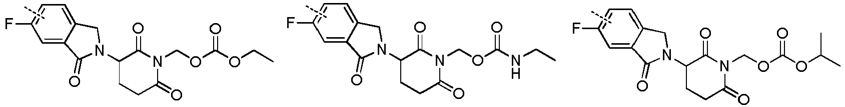

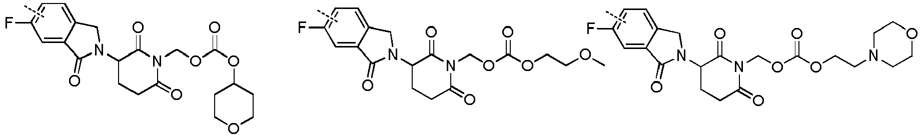

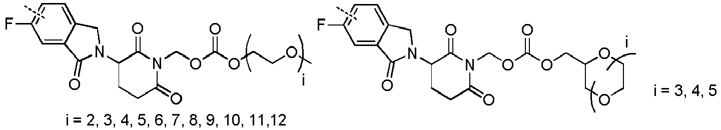

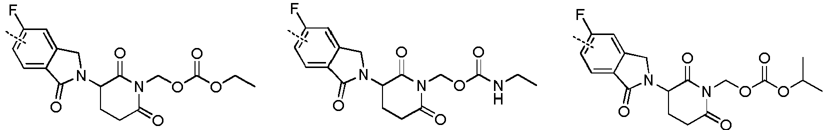

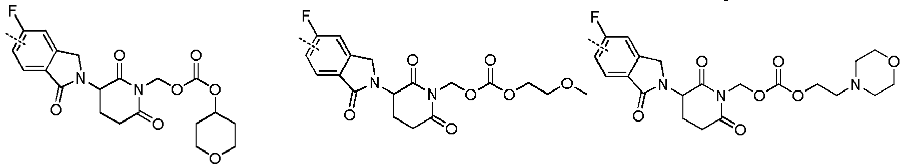

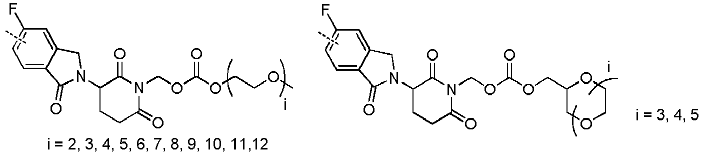

13. The compound according to claim 1, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a combination thereof, or a metabolite thereof, or a metabolic precursor thereof, or an isotopic compound thereof, **characterized in that** said L has the structure of formula XII: 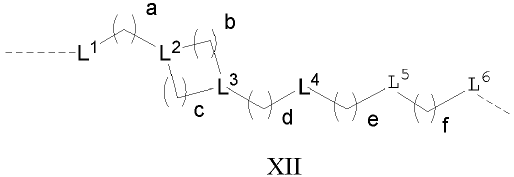 wherein, each of L¹, L², L³, L⁴, L⁵, and L⁶ is respectively selected from the group consisting of none, a bone, O, S, NR^{L1}, CR^{L2}R^{L3}, C=O, C=S, SO, SO₂, a substituted or unsubstituted alkenyl, a substituted or unsubstituted alkynyl, a substituted or unsubstituted monocycloalkyl, a substituted or unsubstituted monoheterocyclic group, a substituted or unsubstituted aryl, a substituted or unsubstituted heteroaryl, a substituted or unsubstituted bridged cycloalkyl, a substituted or unsubstituted bridged-heterocyclic group, a substituted or unsubstituted spirocycloalkyl, a substituted or unsubstituted spiroheterocyclic group, a substituted or unsubstituted fused cycloalkyl, and a substituted or unsubstituted fused heterocyclic group;
the above substituent is selected from the group consisting of C₁₋₆ alkyl, -L-OH, and halogen; L is selected from 0-6 methylene groups;
each of R^{L1}, R^{L2}, and R^{L3} is respectively selected from the group consisting of H, deuterium, C₁₋₆ alkyl or a halogenated compound thereof or a deuterated compound thereof, and a 3-8 membered cycloalkyl with 0-2 heteroatoms, or R^{L2} and R^{L3} are linked to form a 3-8 membered ring containing 0-2 heteroatoms;
each of a, b, c, d, e, and f is respectively selected from an integer of 0 to 5;
the isotope-substituted form is a deuterated compound.

14. The compound according to claim 13, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a combination thereof, or a metabolite thereof, or a metabolic precursor thereof, or an isotopic compound thereof, **characterized in that**:
said L has the structure of formula XII-A: 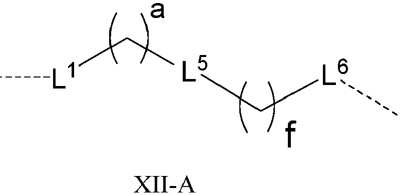 wherein, L₁, L₅, L₆, a, and f are as described in claim 13;
or, said L has the structure of formula XII-B: 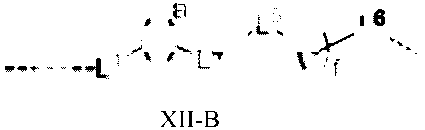 wherein, L₁, L₄, L₅, L₆, a, and f are as described in claim 13;
or, said L has the structure of formula XII-C: 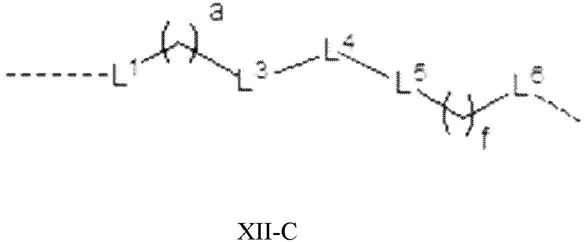 wherein, L₁, L₃, L₄, L₅, L₆, a, and f are as described in claim 13;
or, said L has the structure of formula XII-D: 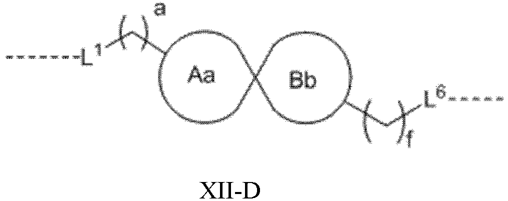 wherein, L₁, L₆, a, and f are as described in claim 13; rings Aa and Bb share one carbon atom, and each of rings Aa and Bb is independently selected from the group consisting of 3-6 membered saturated monocycloalkyl and 3-6 membered saturated monocyclic heterocyclyl;
or, said L has the structure of formula XII-E: 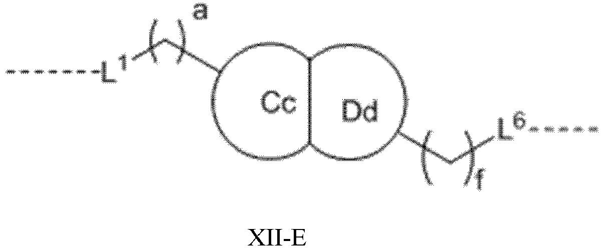 wherein, L₁, L₆, a, and f are as described in claim 13; rings Cc and Dd share two carbon atoms, and each of rings Cc and Dd is independently selected from the group consisting of 3-6 membered saturated monocycloalkyl and 3-6 membered saturated monocyclic heterocyclyl.

15. The compound according to claim 13 or 14, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a combination thereof, or a metabolite thereof, or a metabolic precursor thereof, or an isotopic compound thereof, **characterized in that** said L is selected from the group consisting of the following structures: 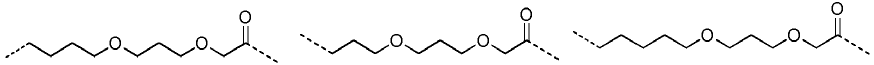 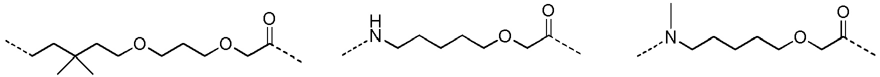 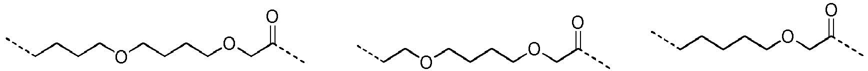 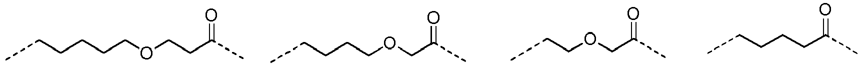 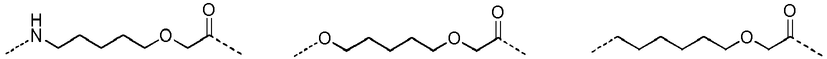 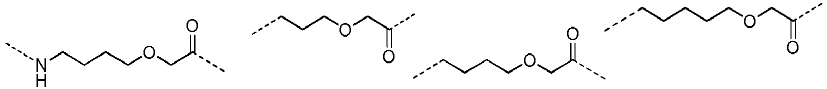 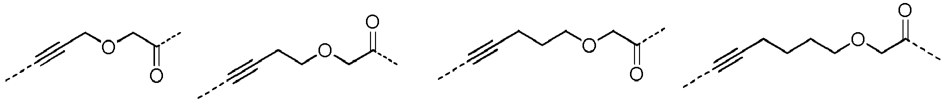 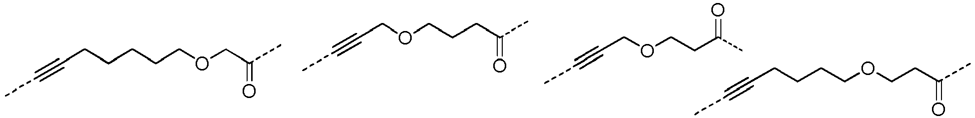 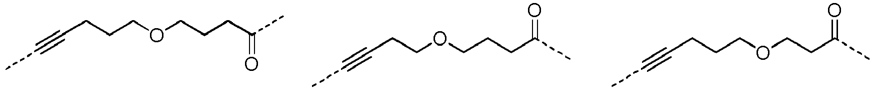 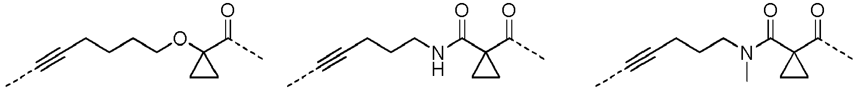 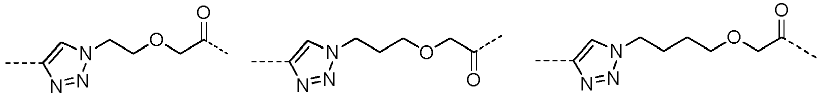 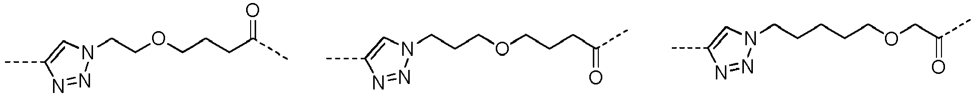 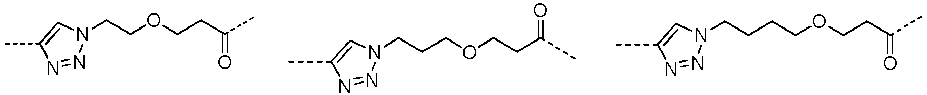 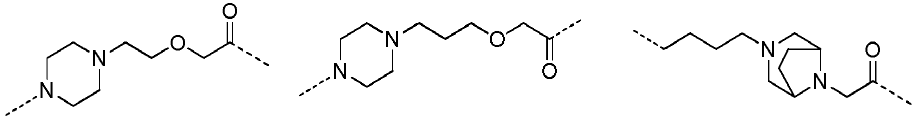 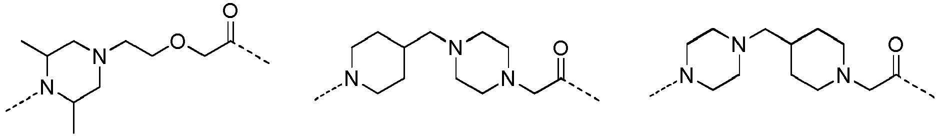 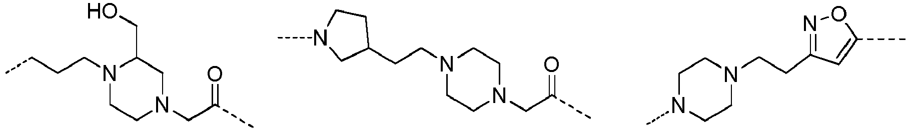 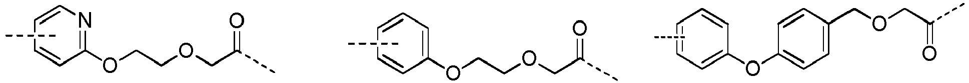 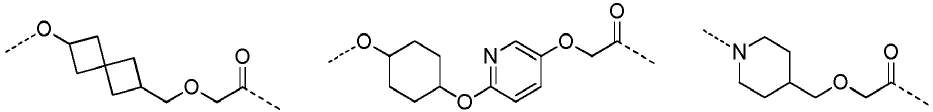 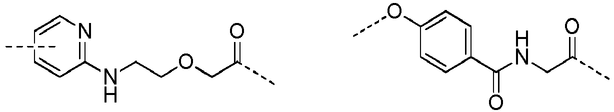 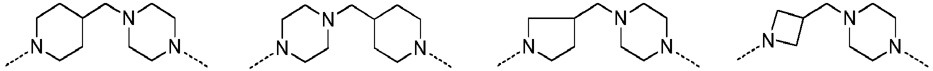 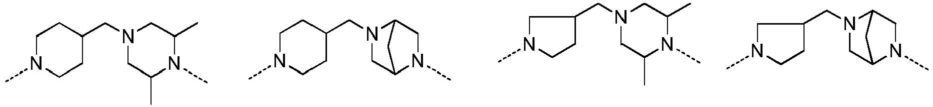 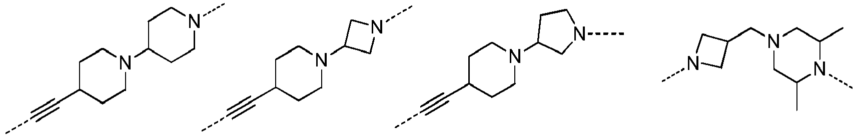 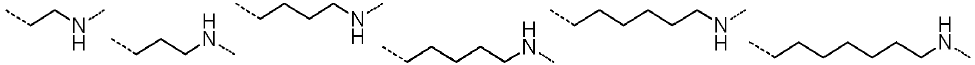 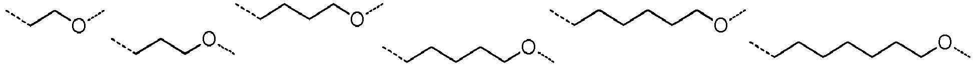 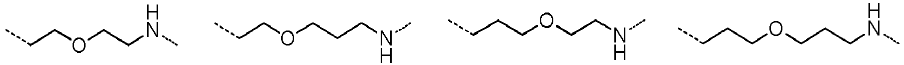 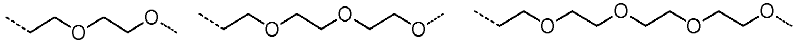 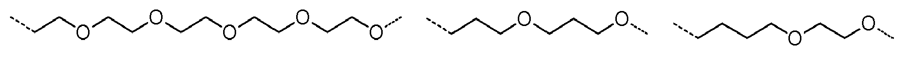 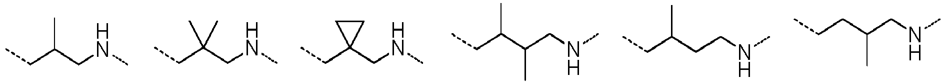 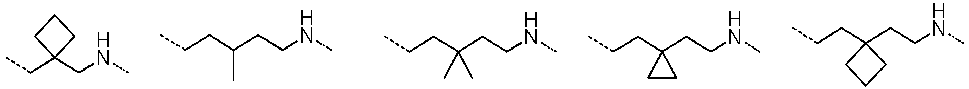 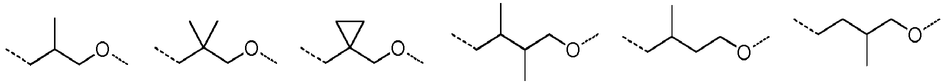 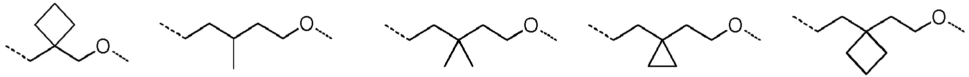 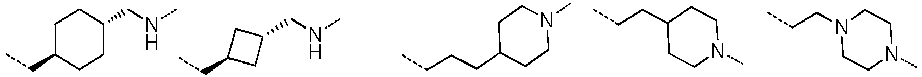 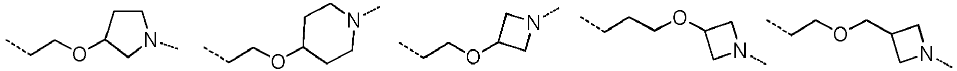 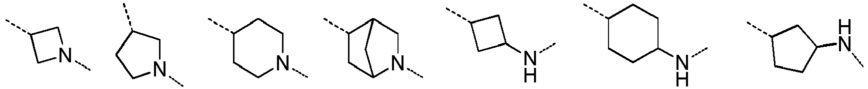 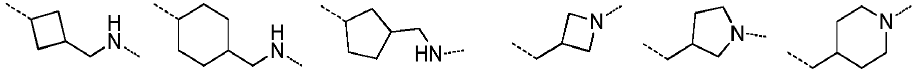 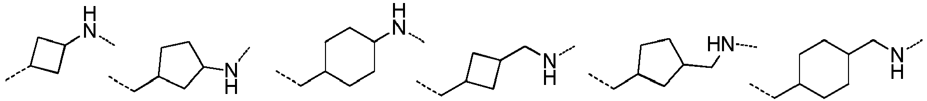 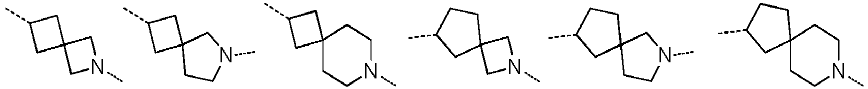 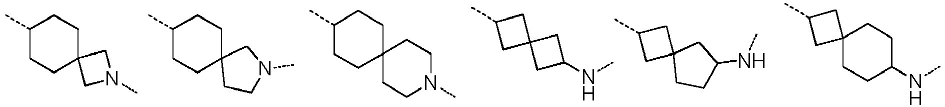 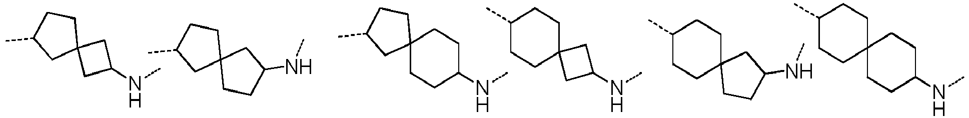 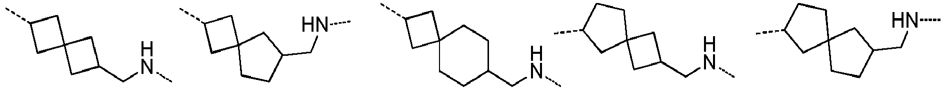 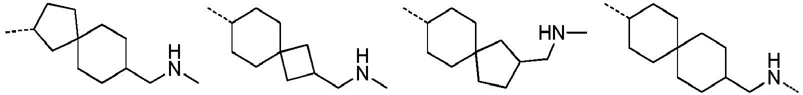 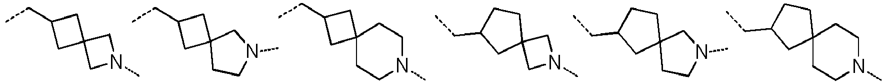 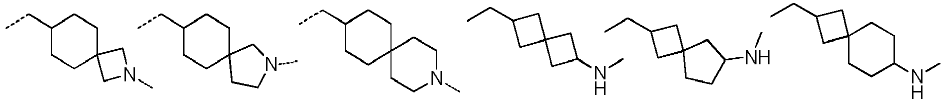 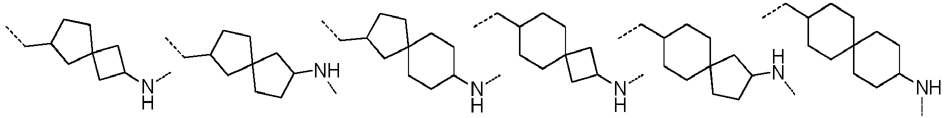 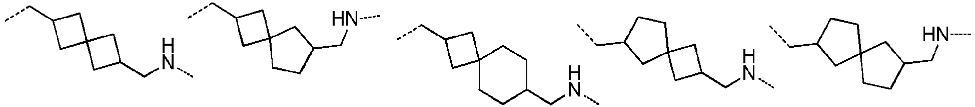 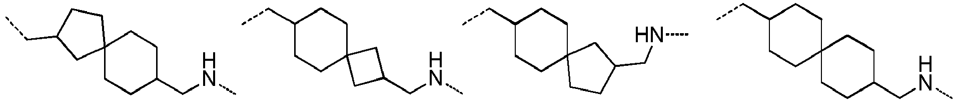 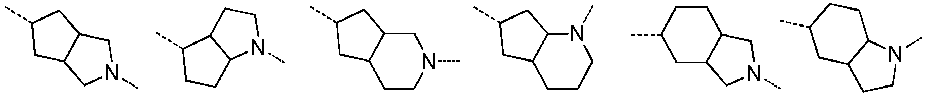 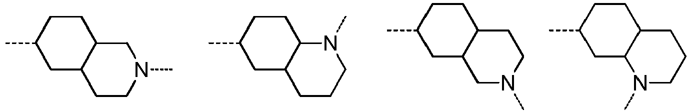 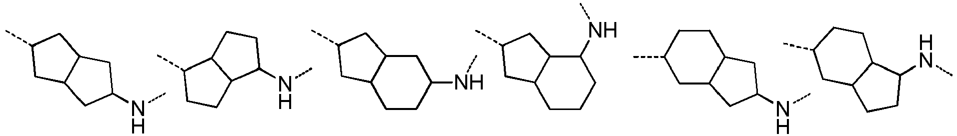 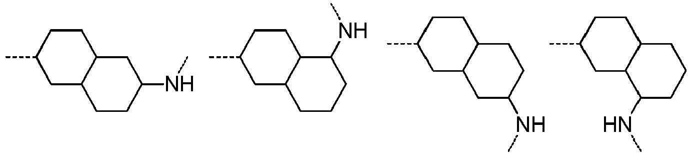 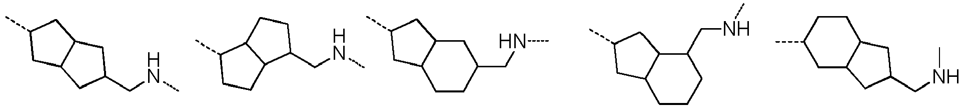 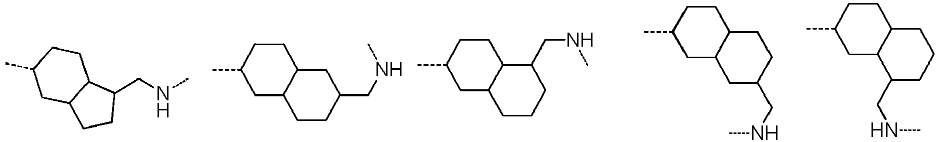 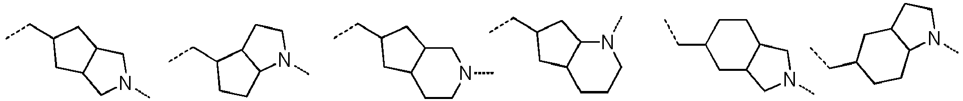 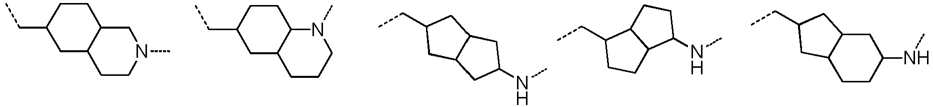 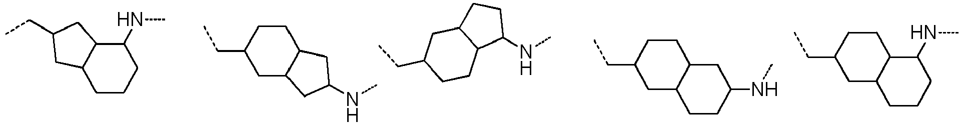 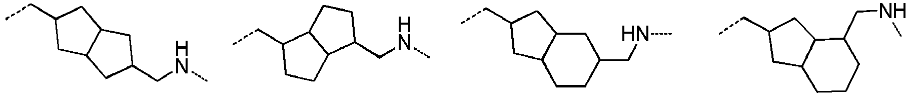 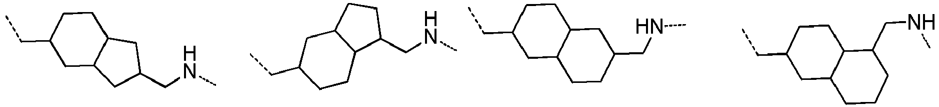 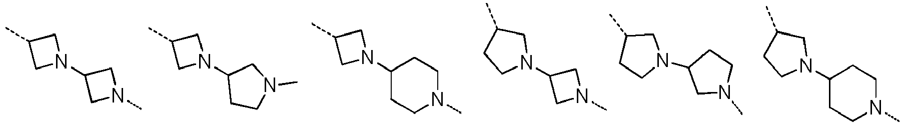 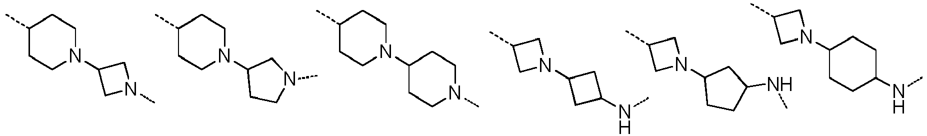 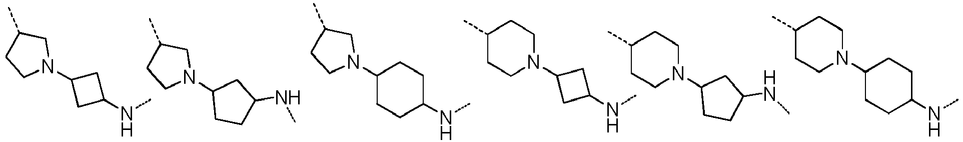 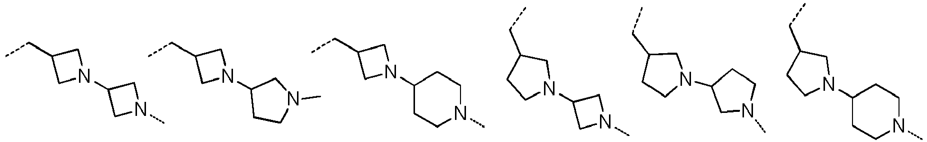 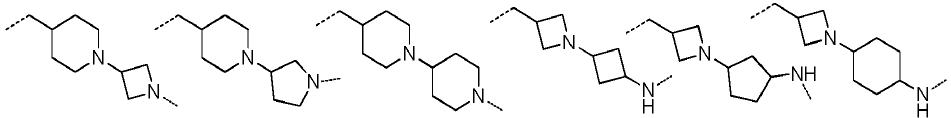 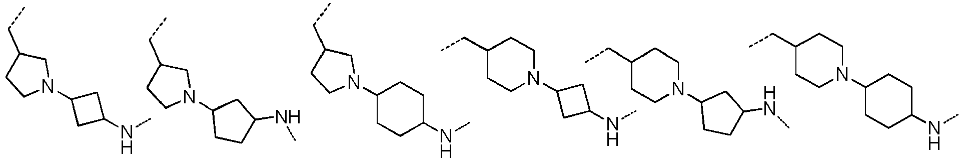 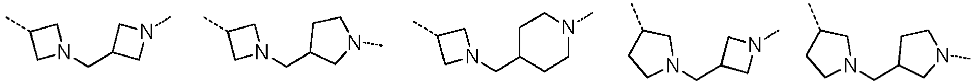 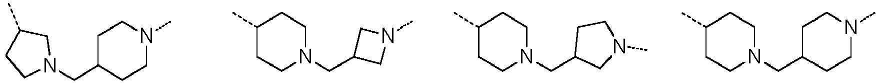 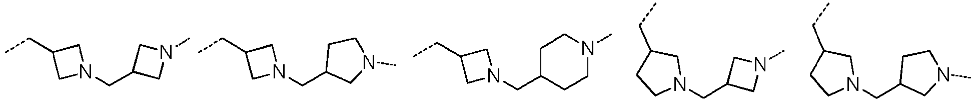 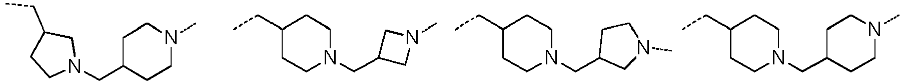 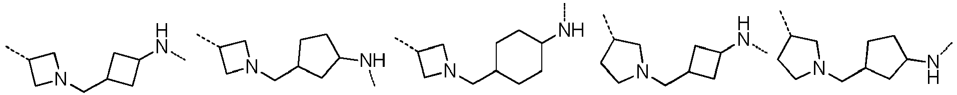 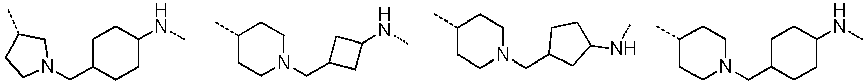 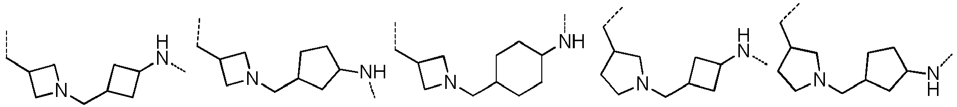 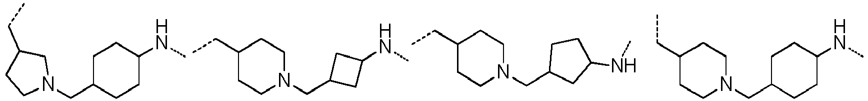 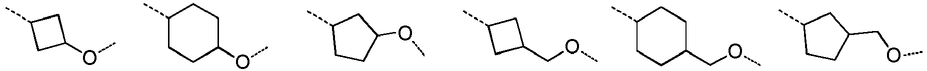 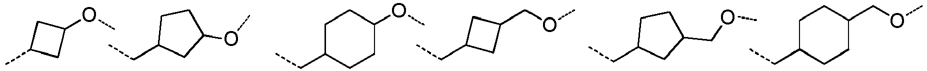 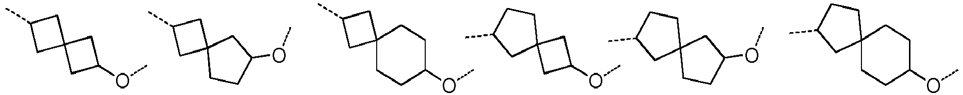 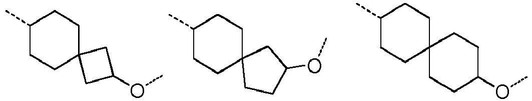 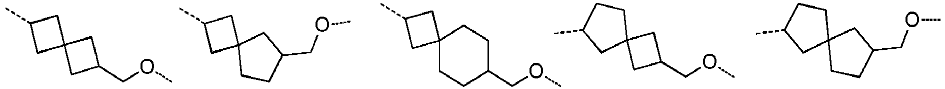 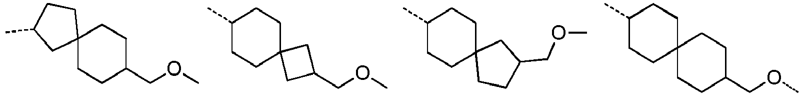 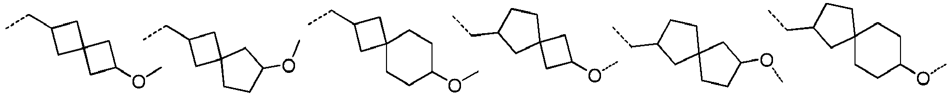 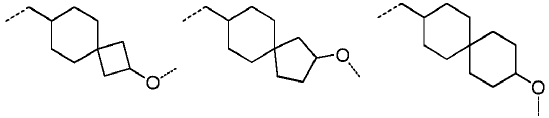 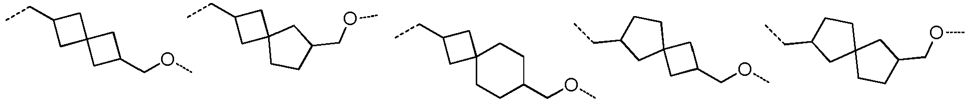 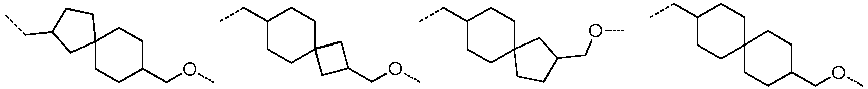 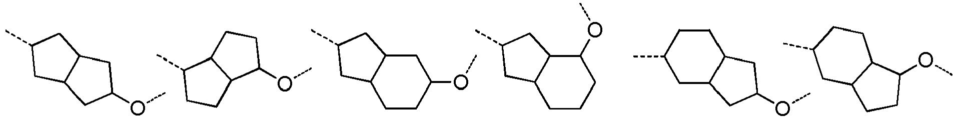 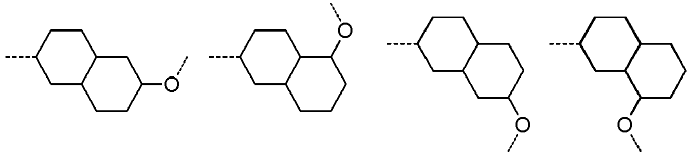 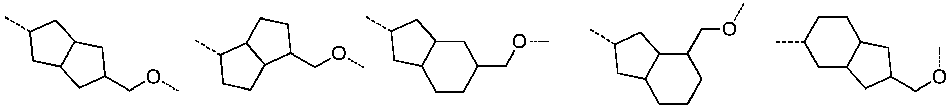 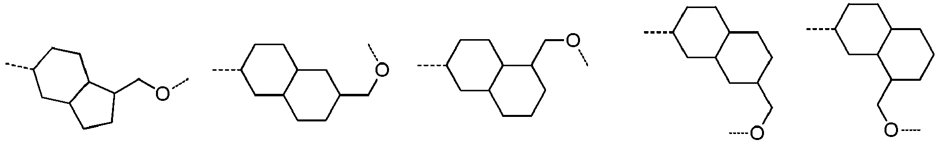 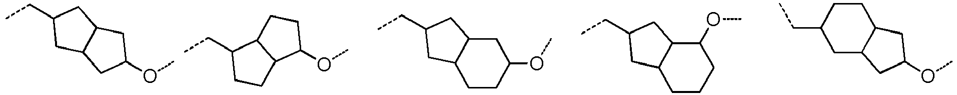 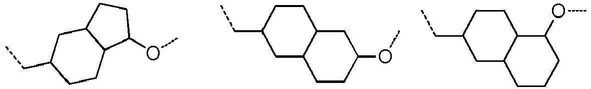 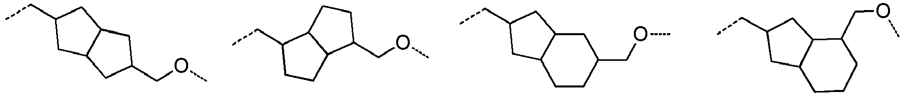 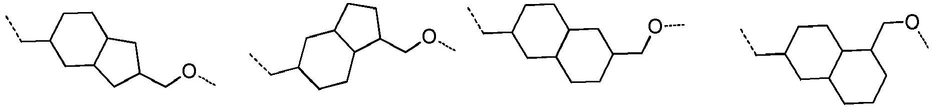 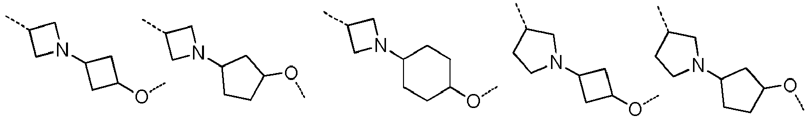 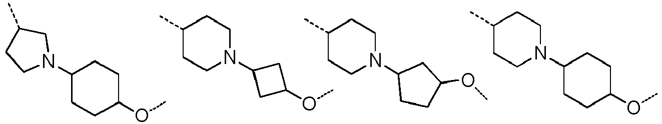 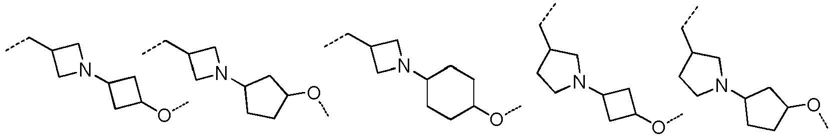 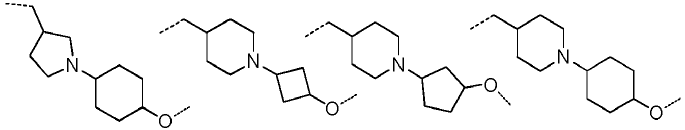 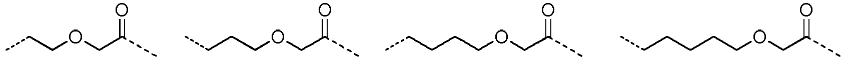 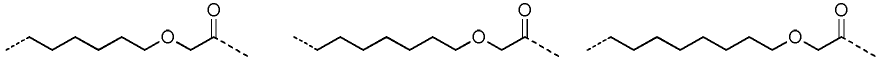 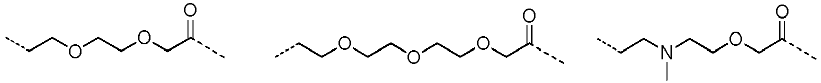 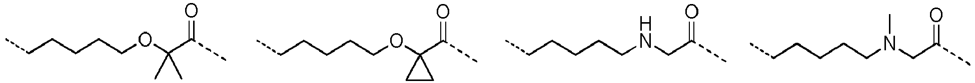 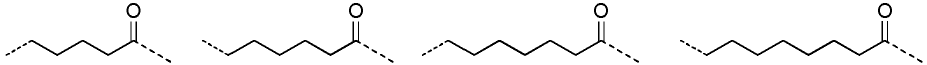 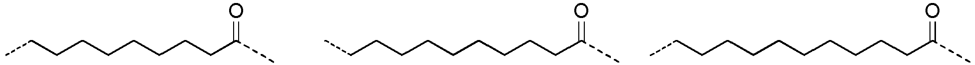 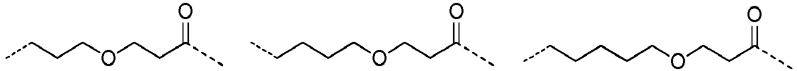 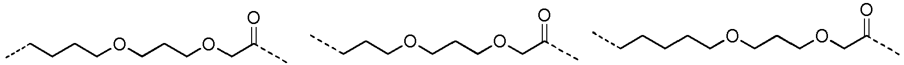 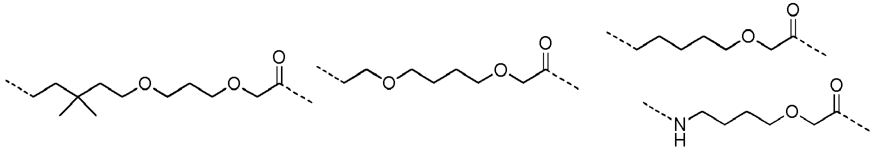 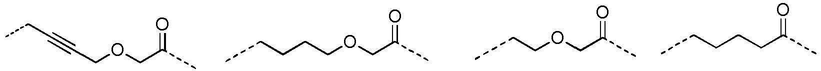 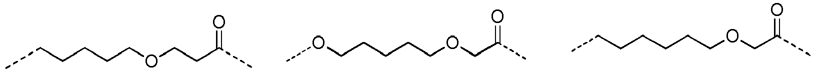 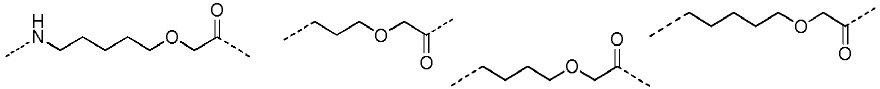 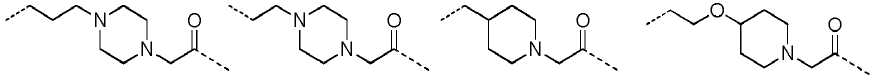 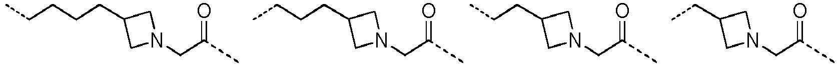 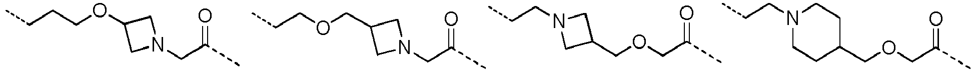 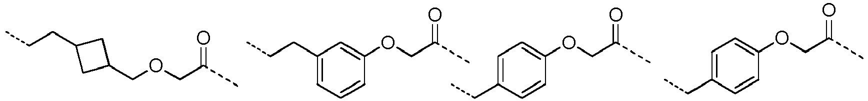 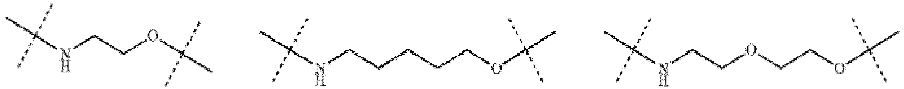 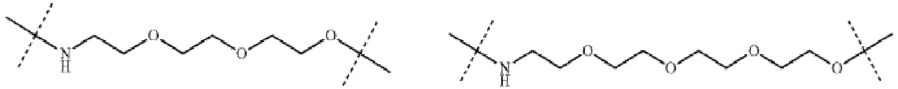 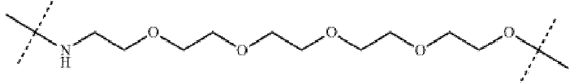 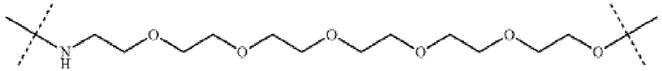 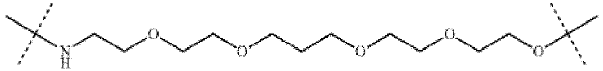 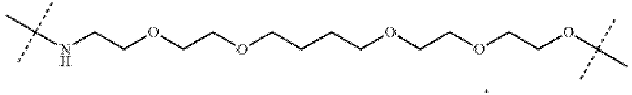 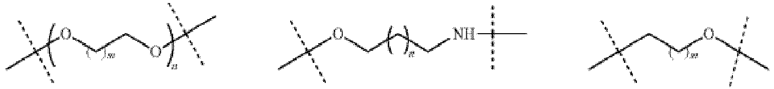 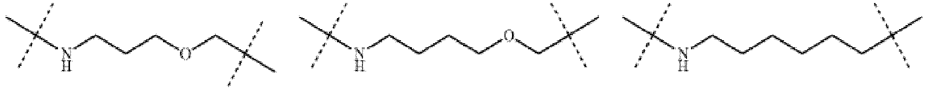 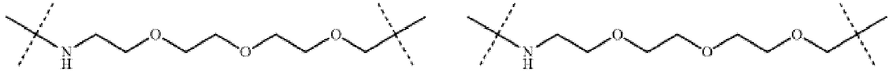 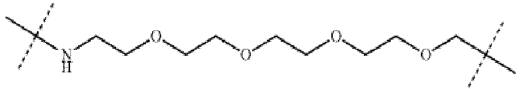 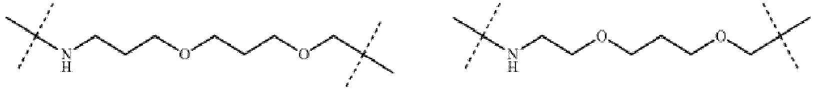 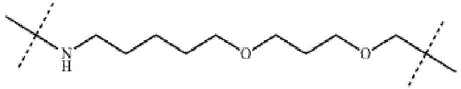 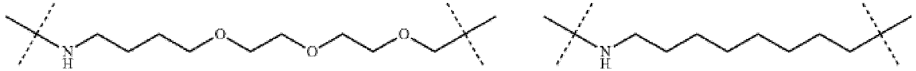 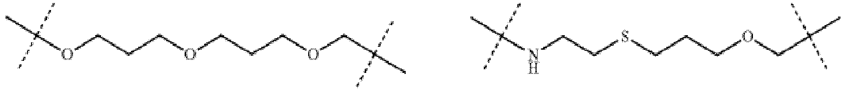 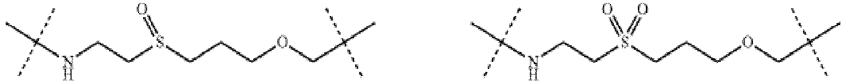 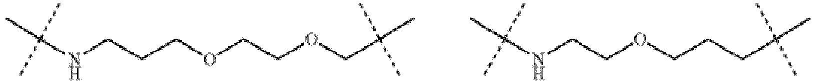 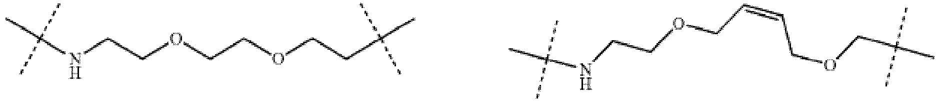 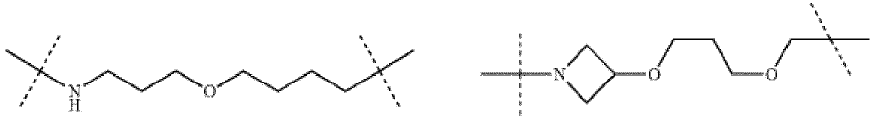 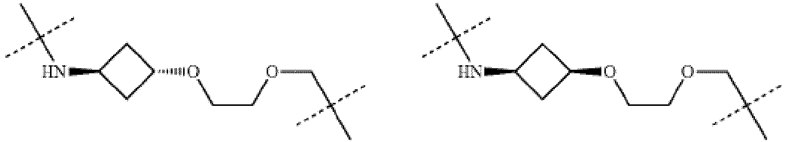 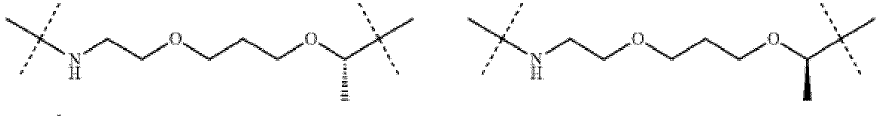 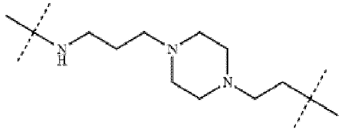 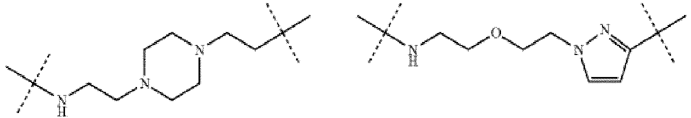 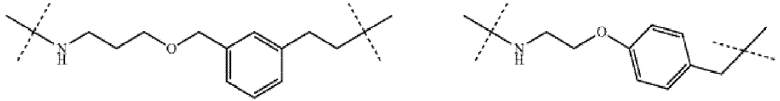 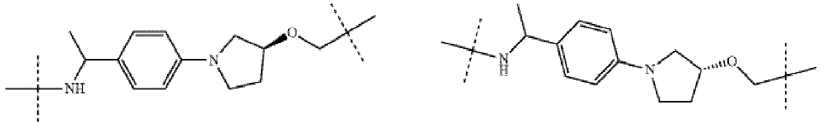 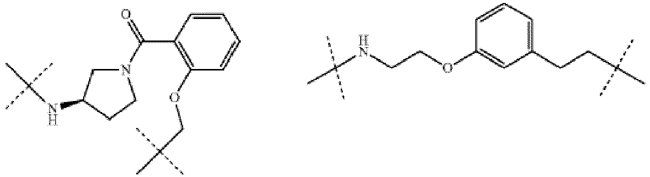 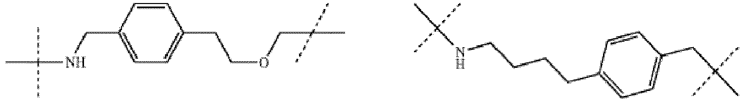 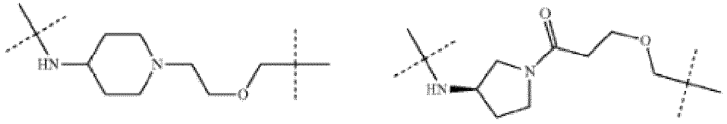 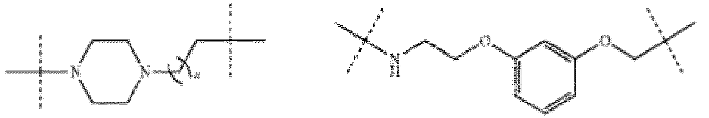 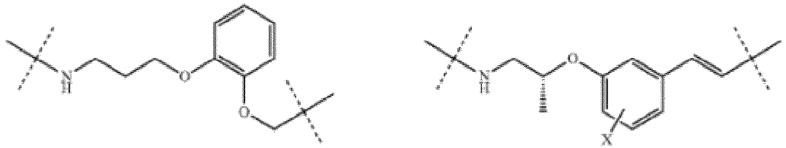 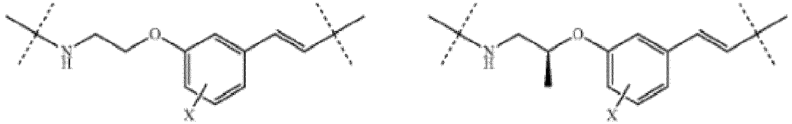 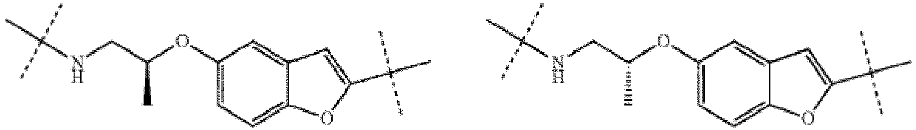 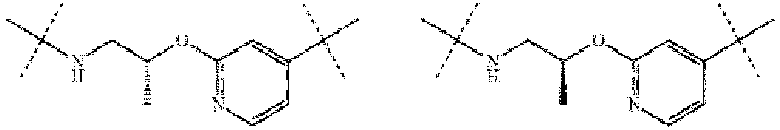 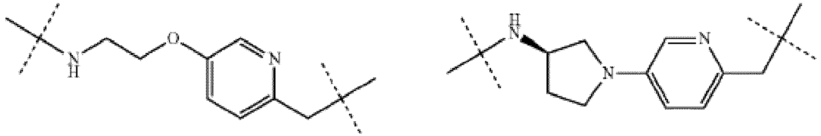 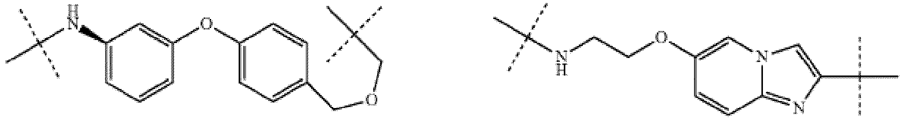 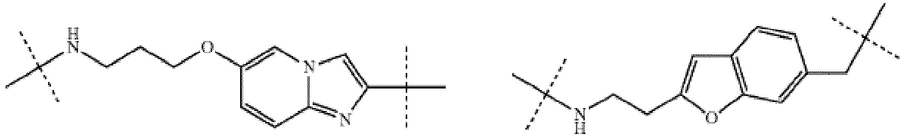 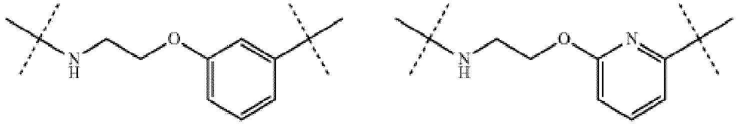 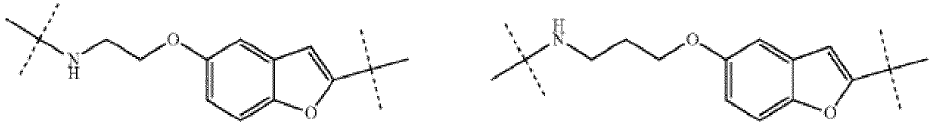 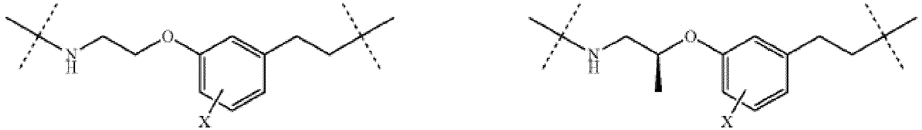 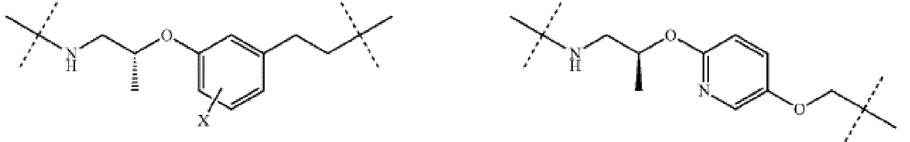 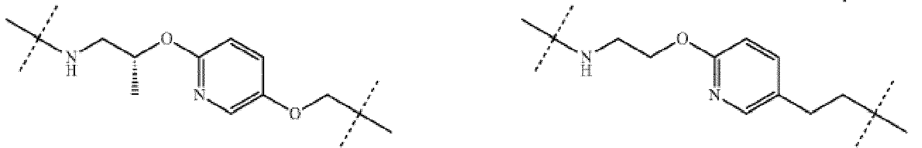 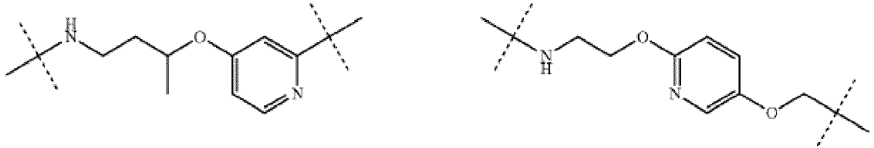 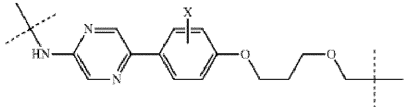 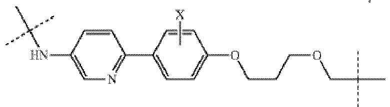 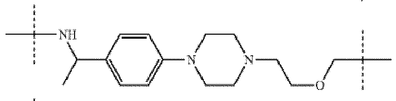 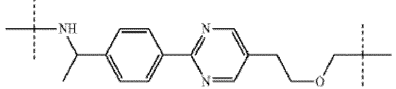 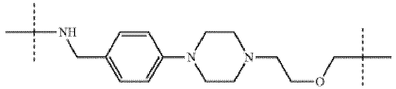 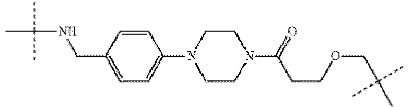 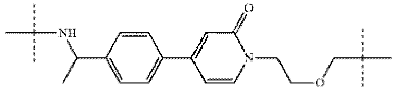 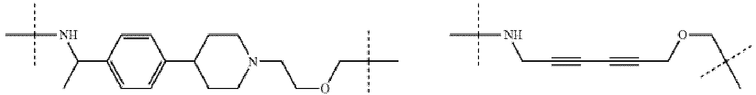 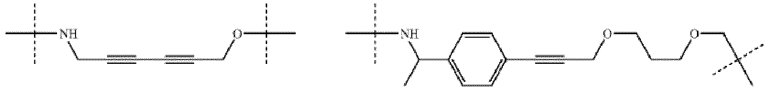 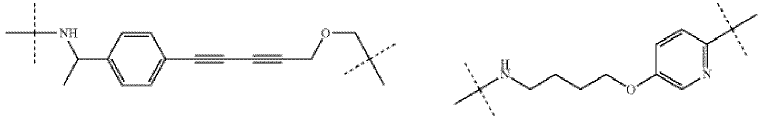 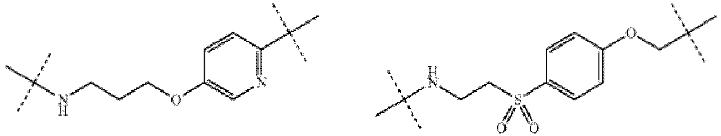 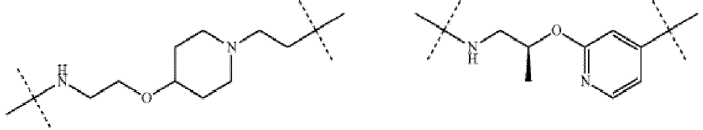 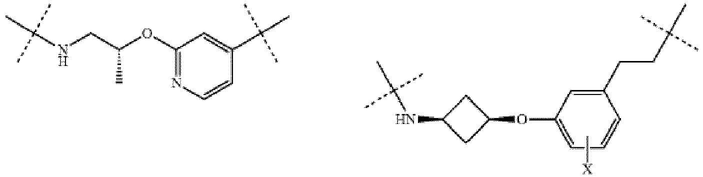 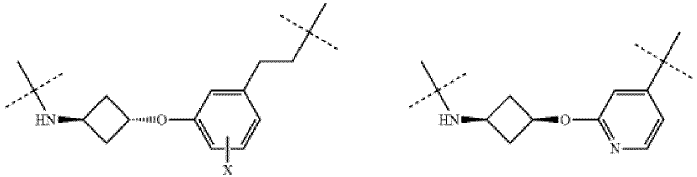 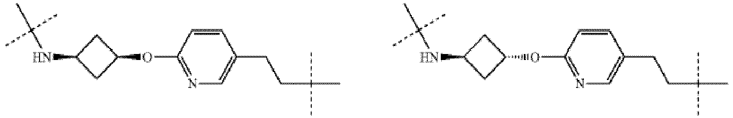 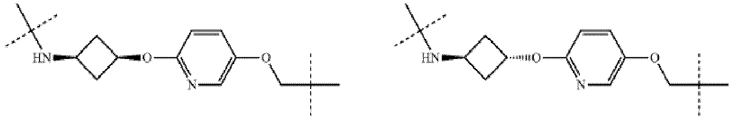 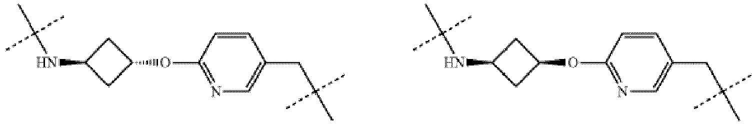 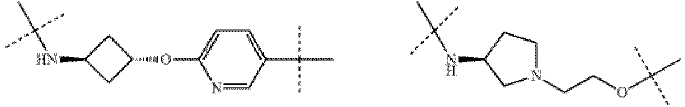 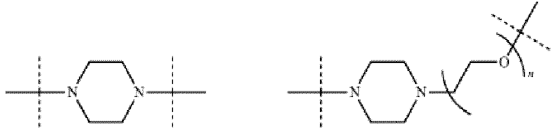 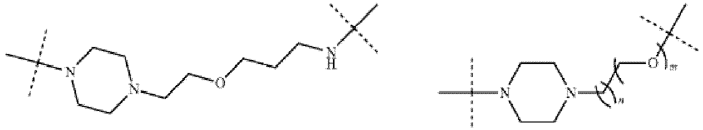 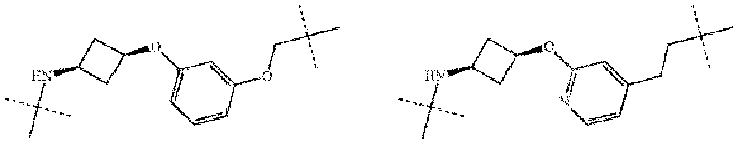 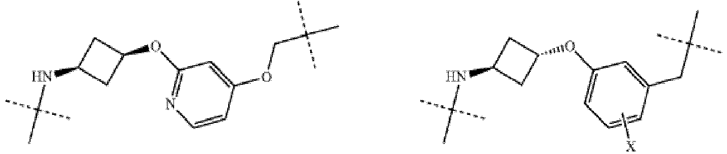 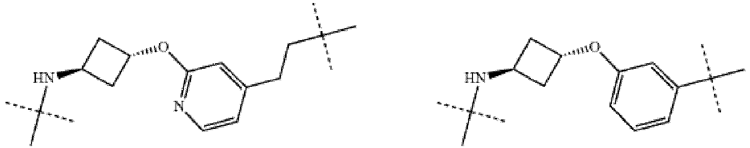 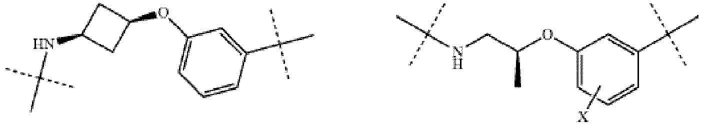 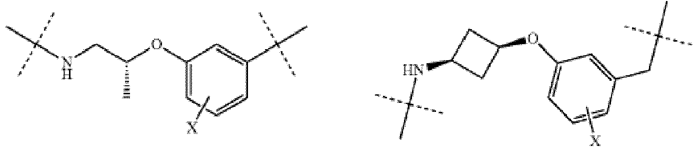 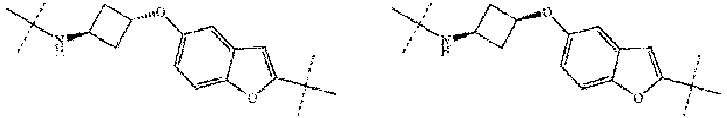 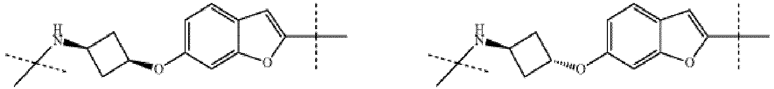 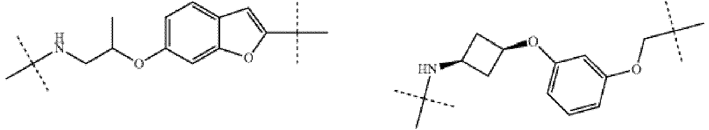 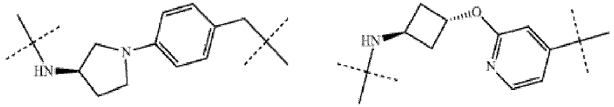 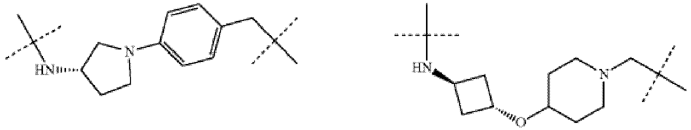 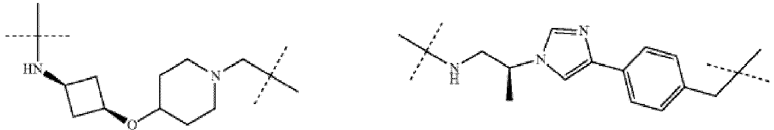 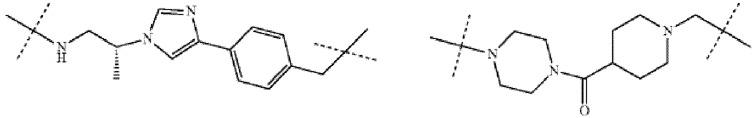 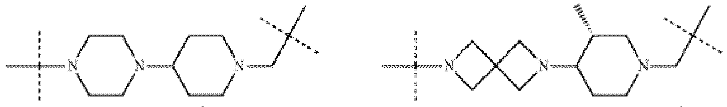 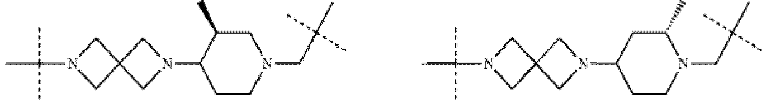 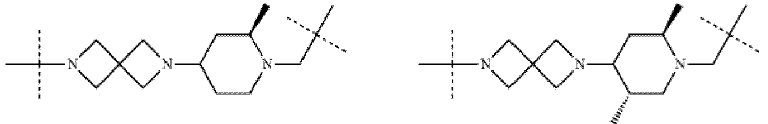 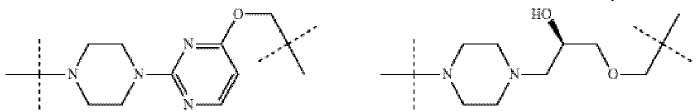 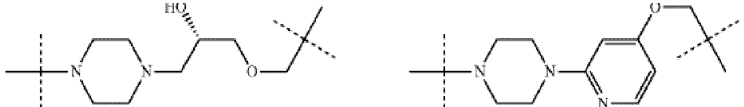 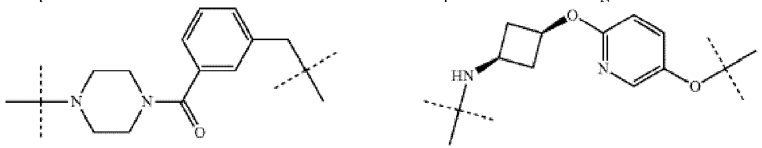 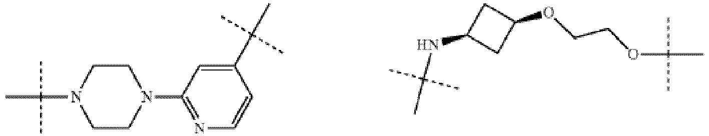 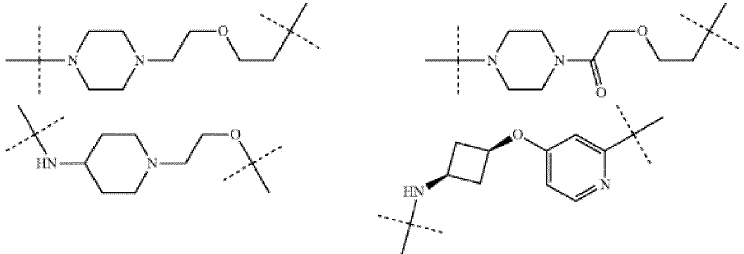 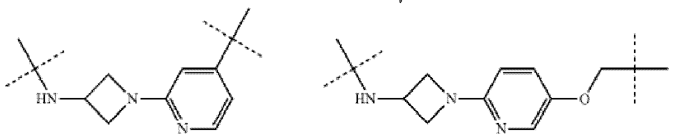 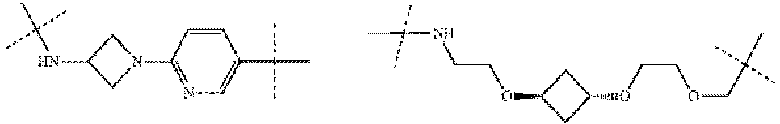 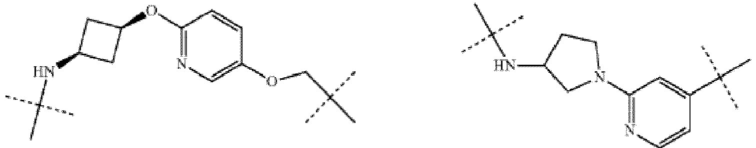 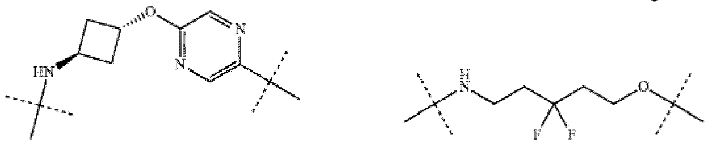 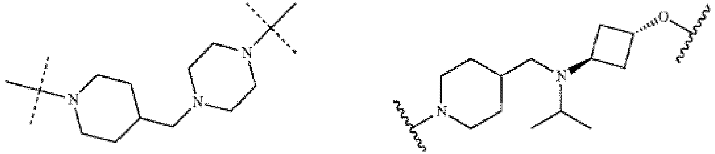 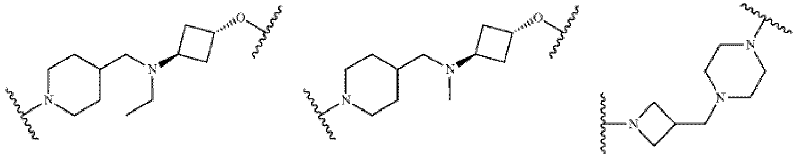 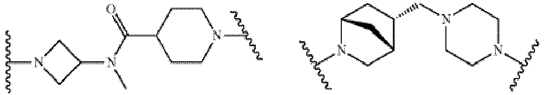 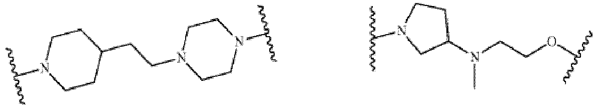 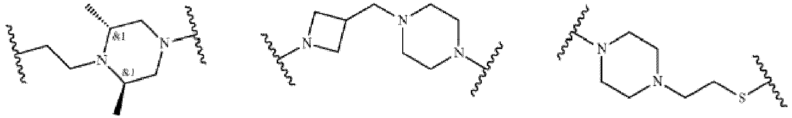 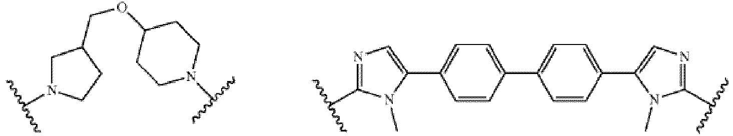 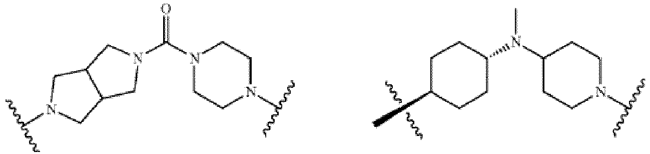 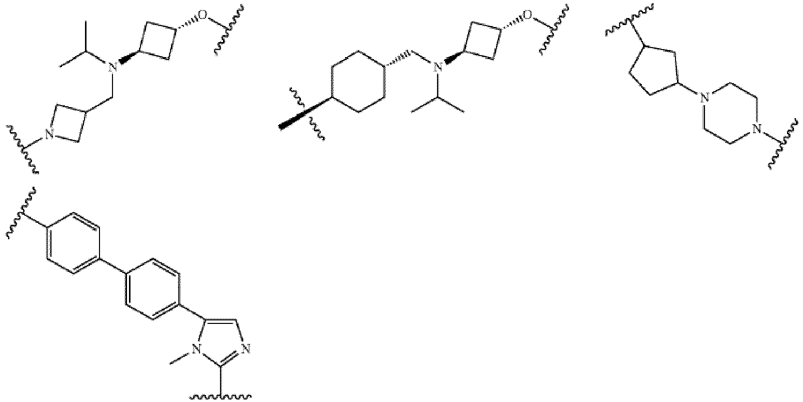 wherein, X is selected from the group consisting of H, deuterium and halogen; each of m and n is an integer of from 0 to 5.

16. The compound according to any one of claims 1 to 15, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a combination thereof, or a metabolite thereof, or a metabolic precursor thereof, or an isotopic compound thereof, **characterized in that** the structure of said compound is selected from the group consisting of:

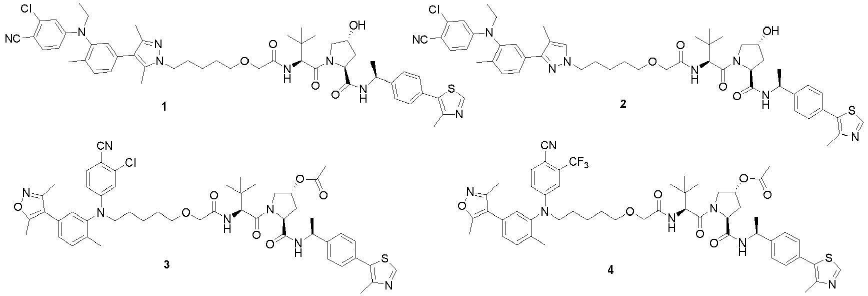

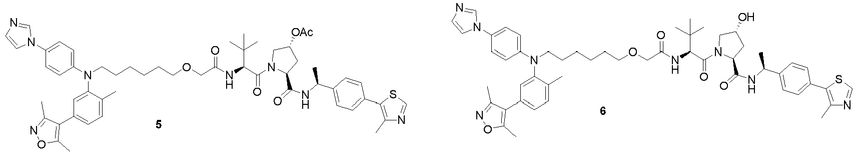

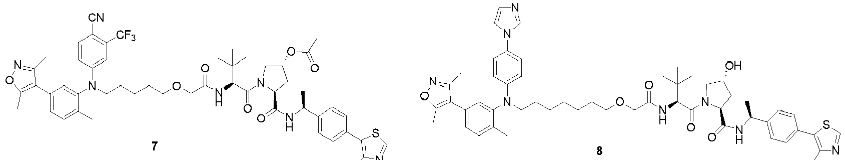

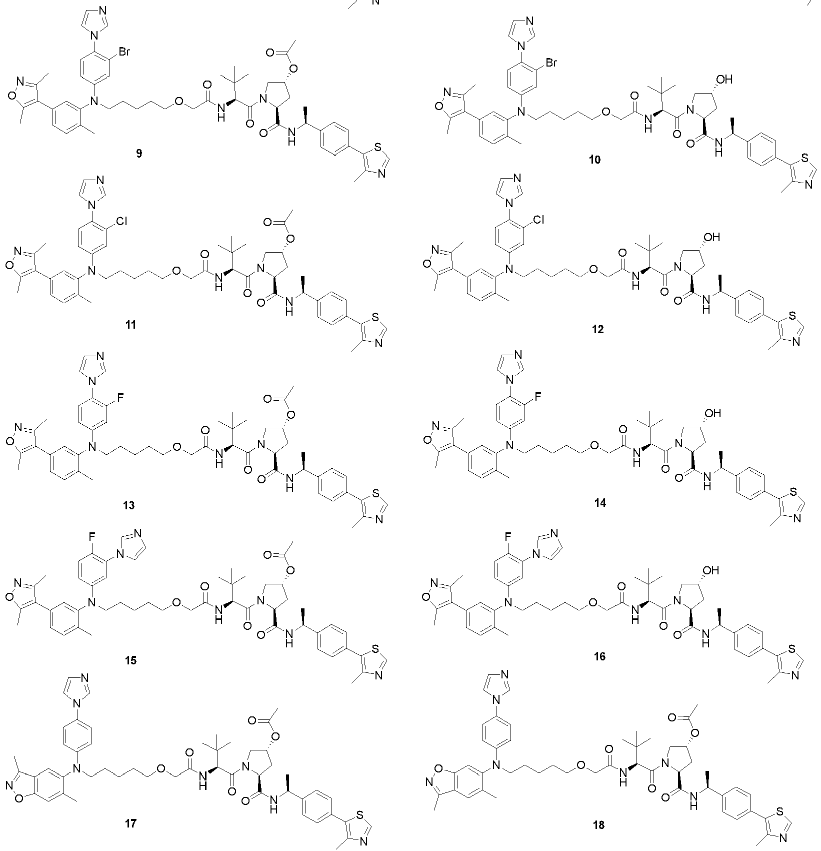

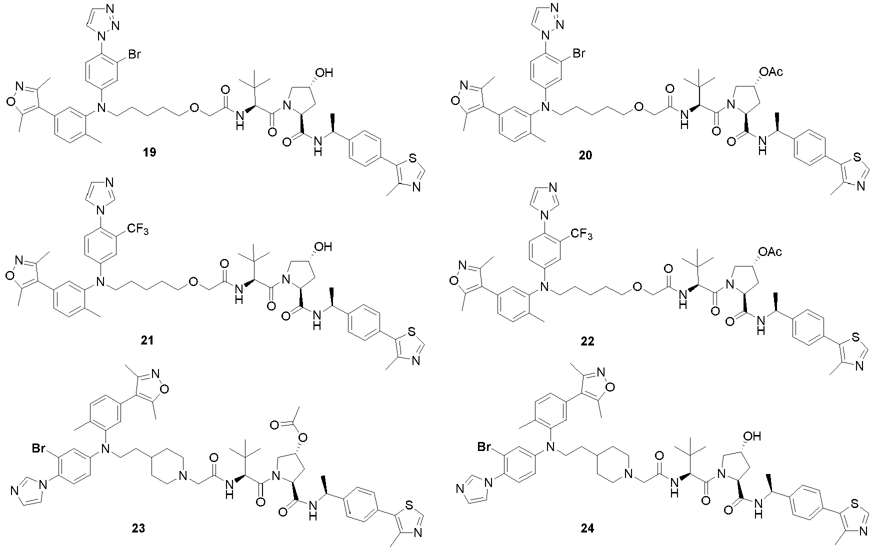

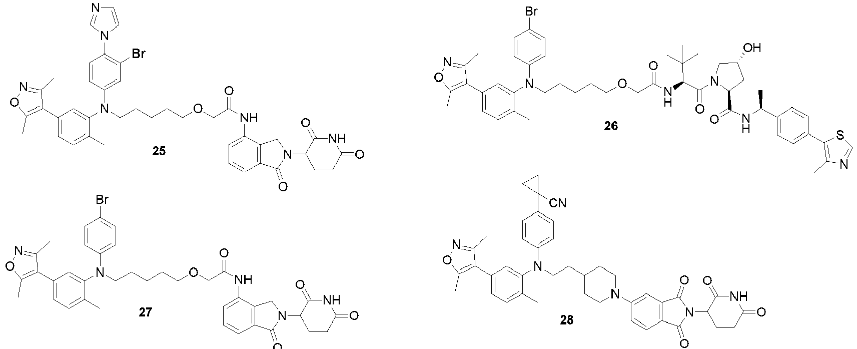

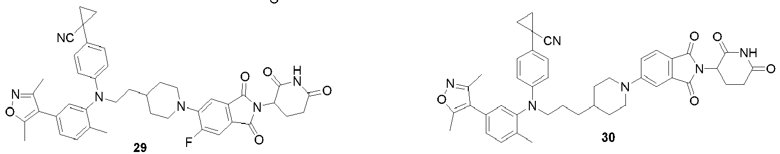

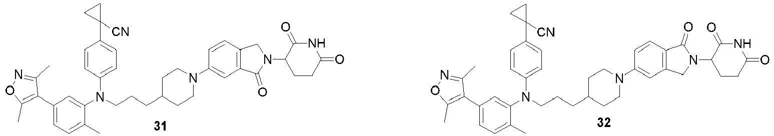

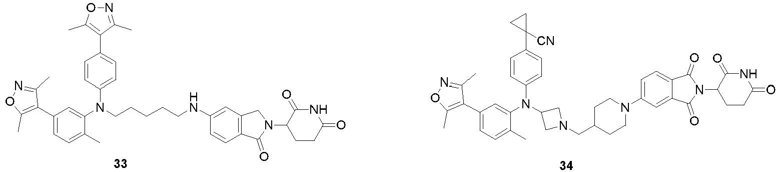

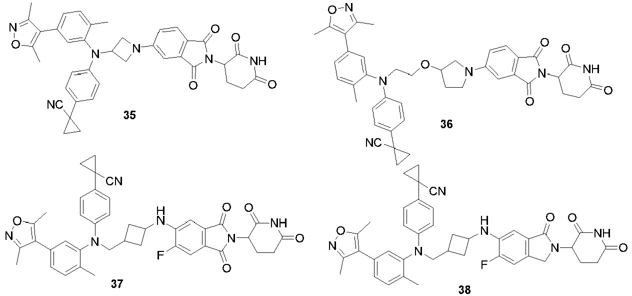

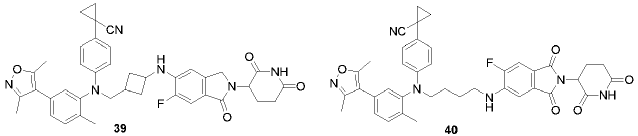

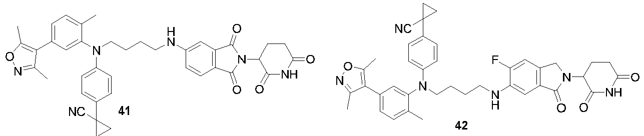

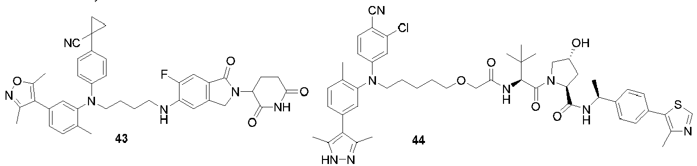

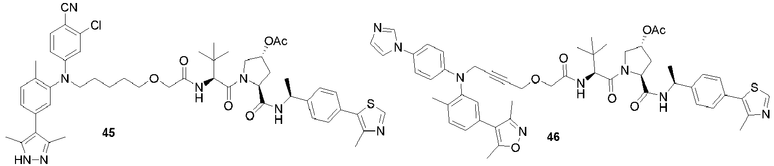

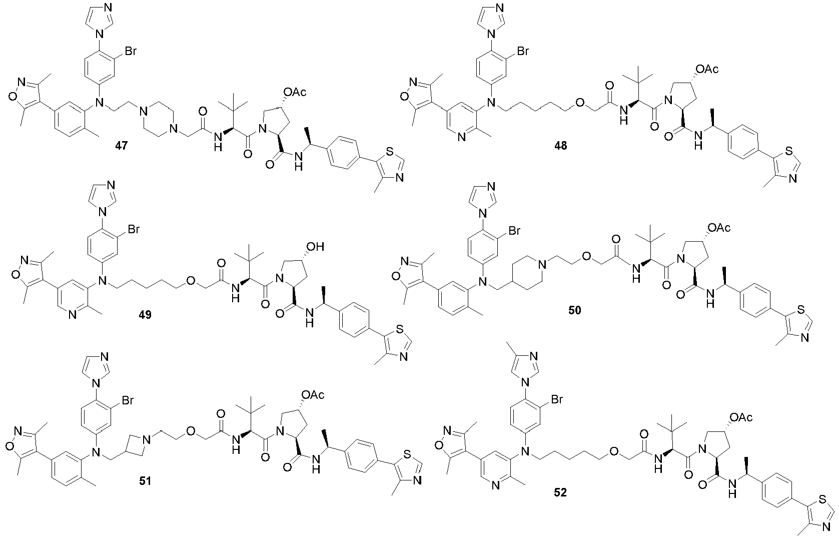

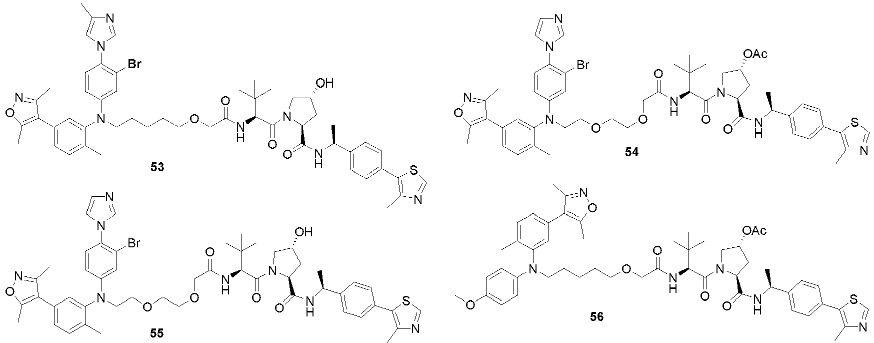

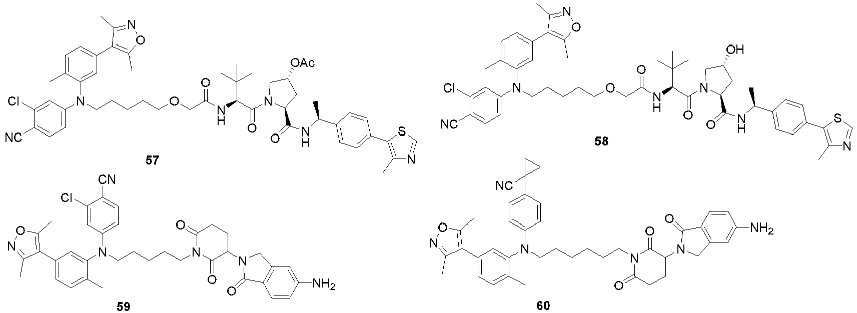

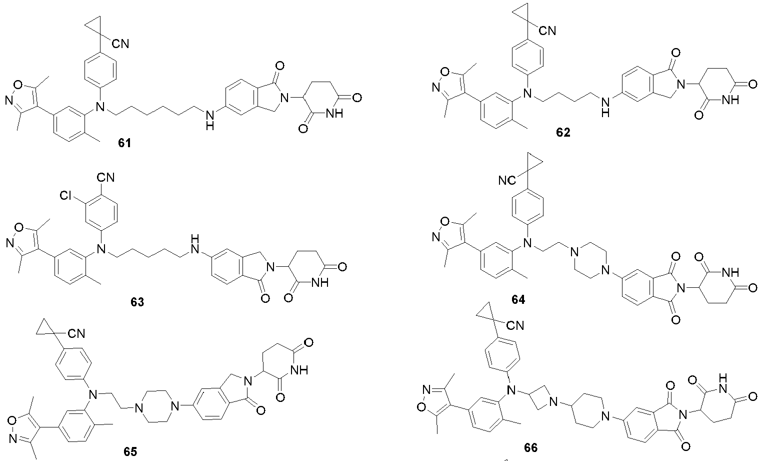

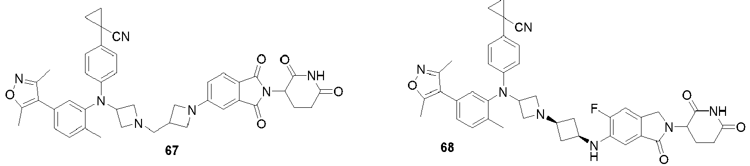

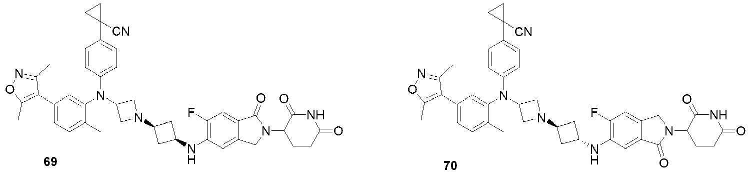

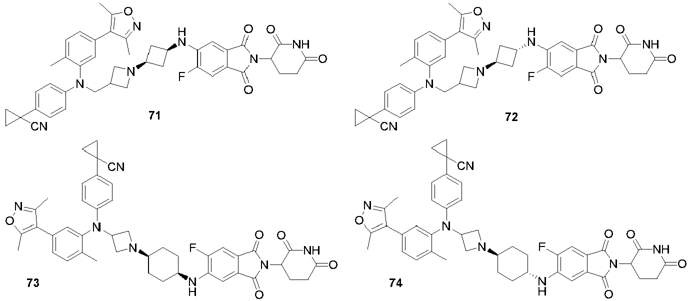

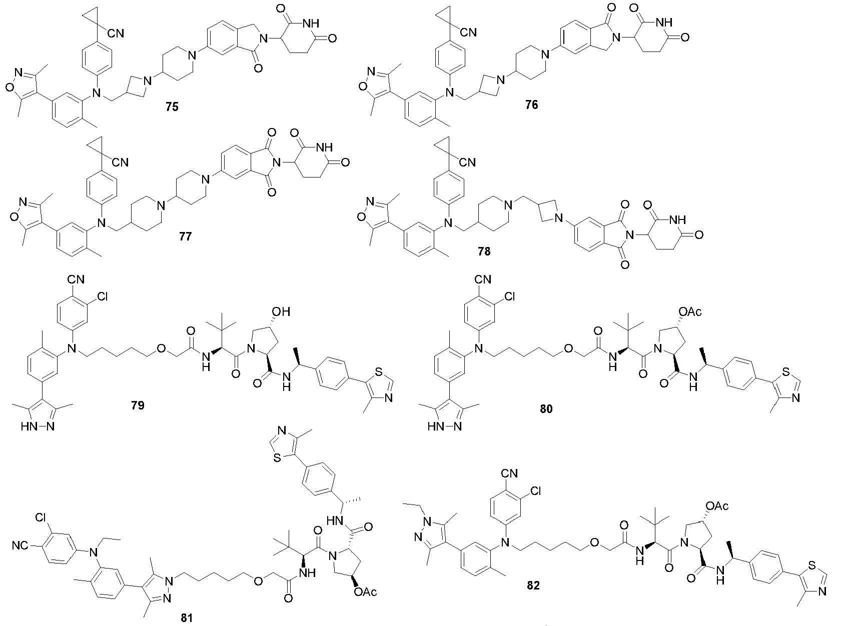

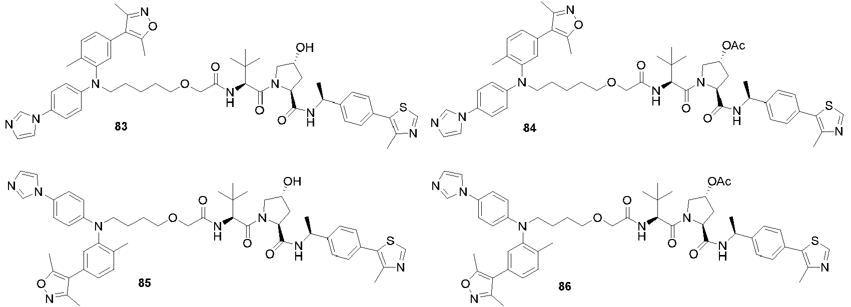

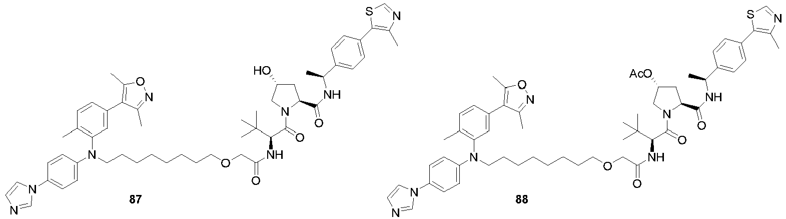

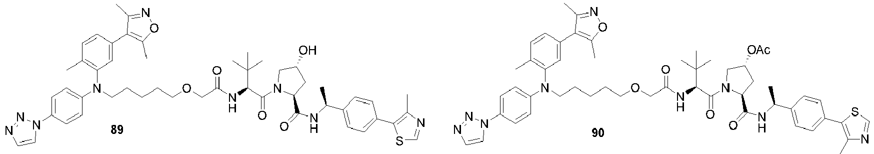

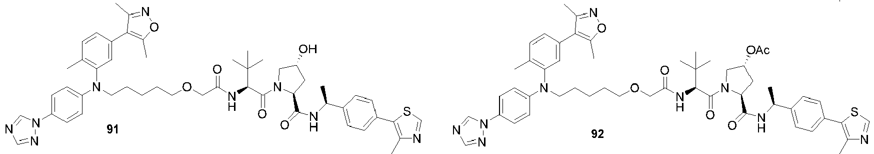

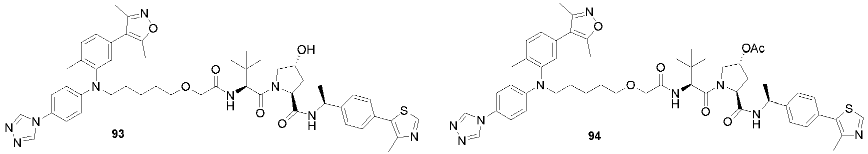

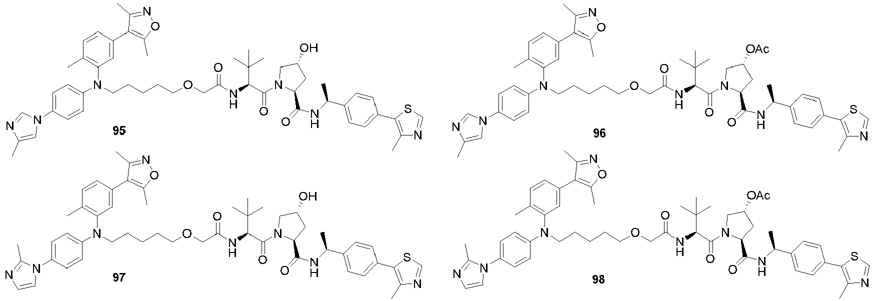

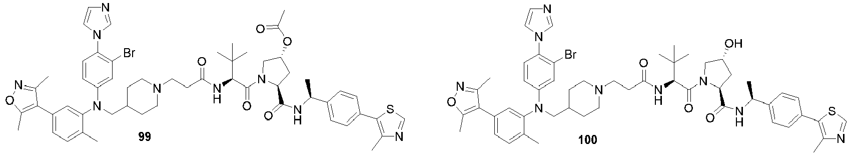

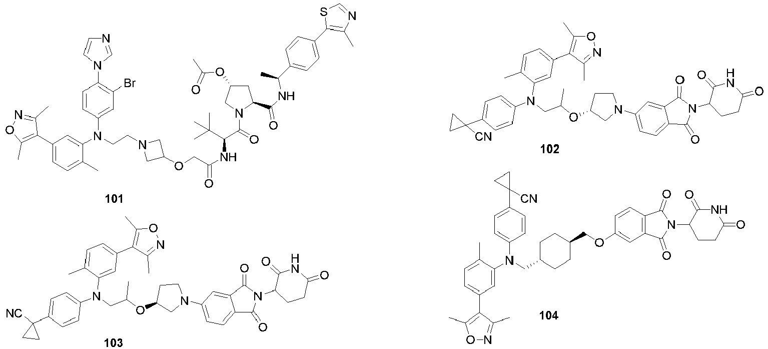

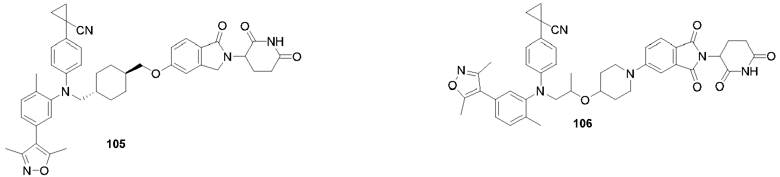

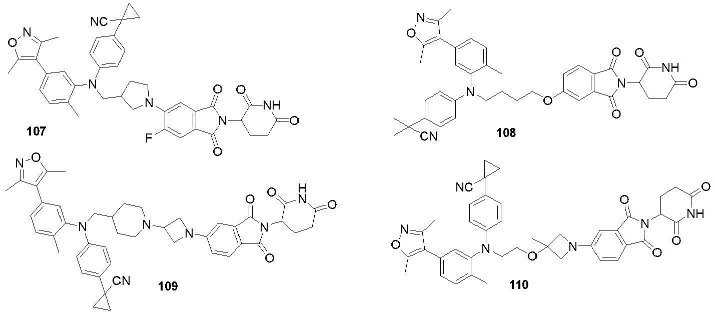

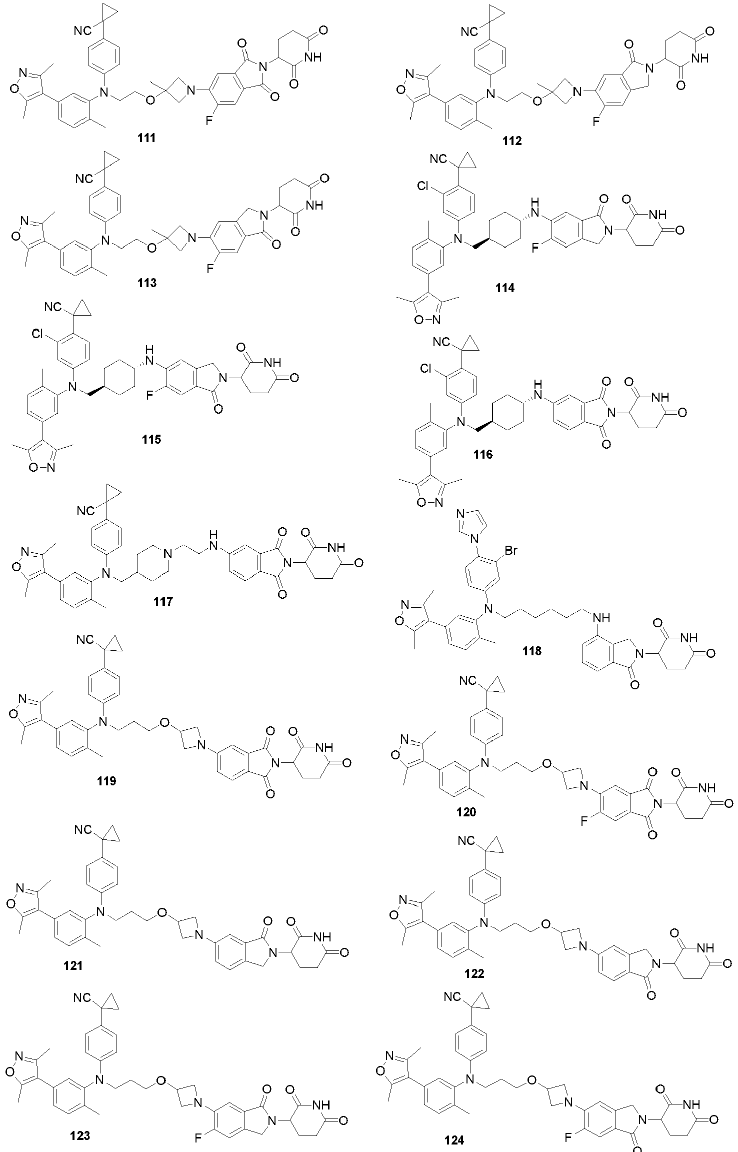

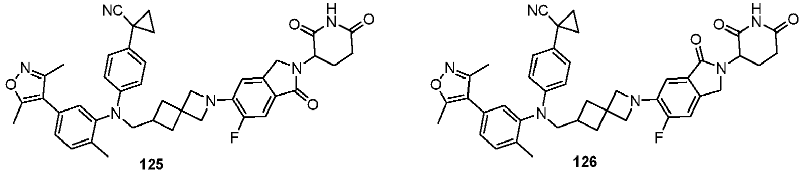

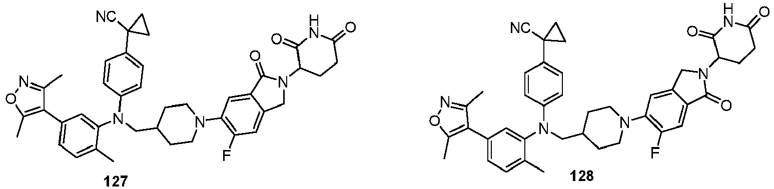

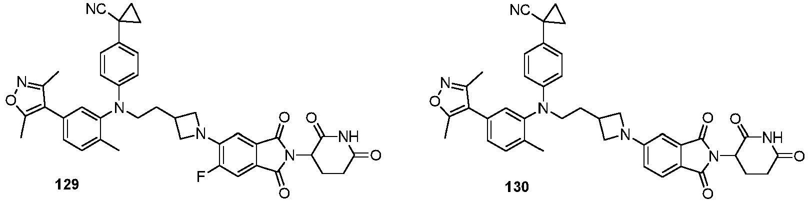

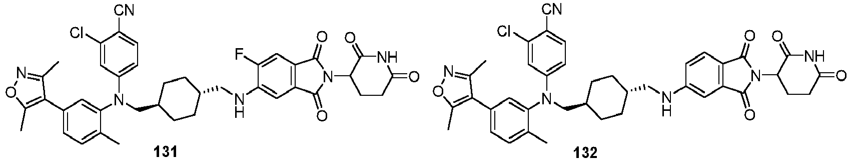

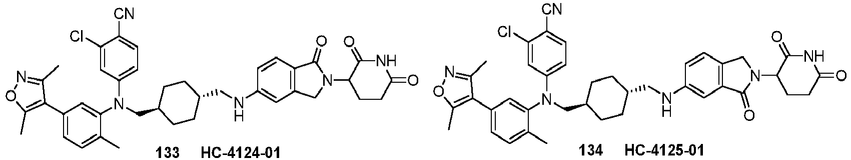

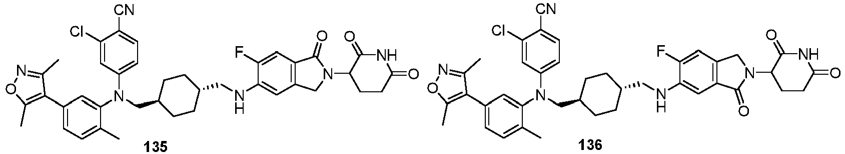

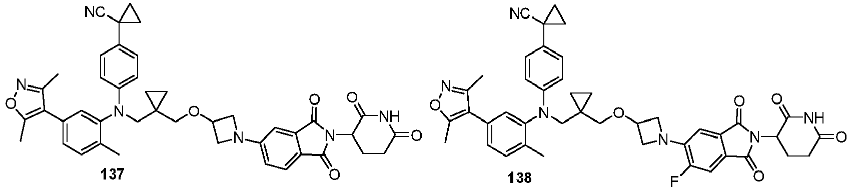

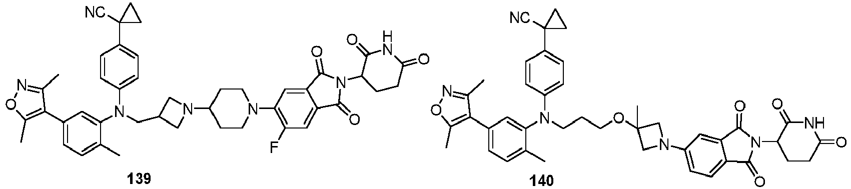

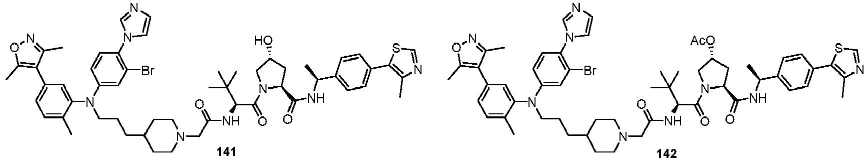

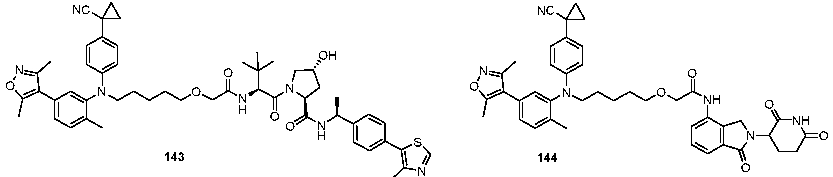

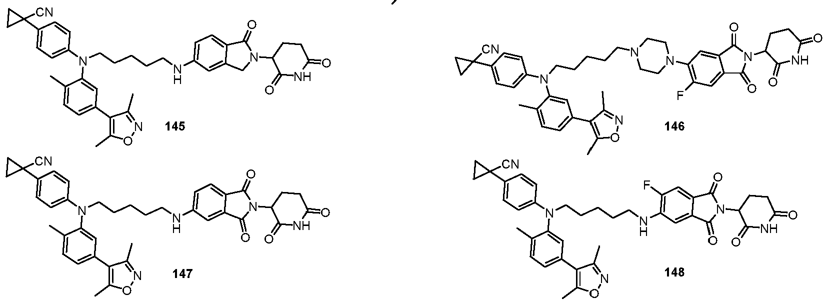

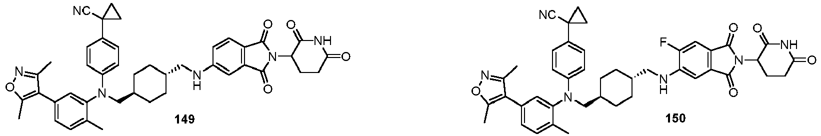

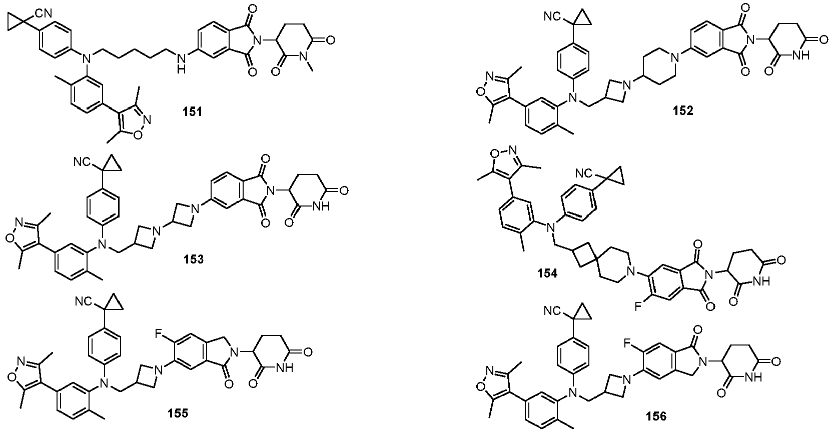

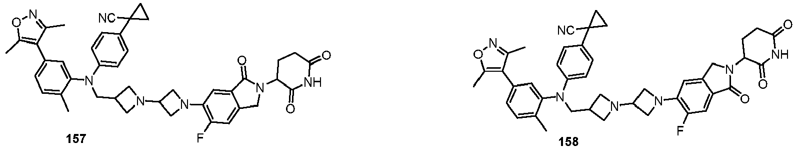

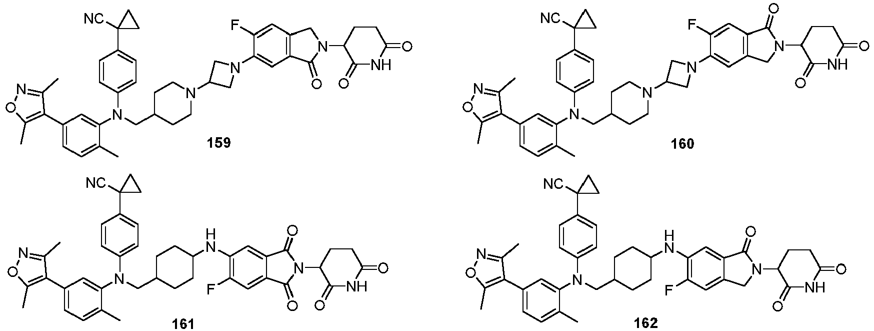

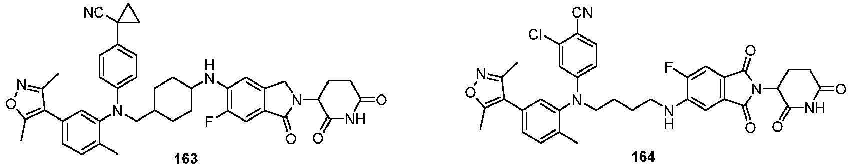

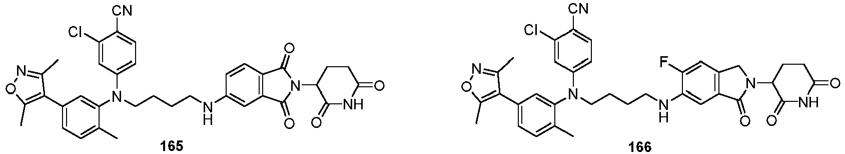

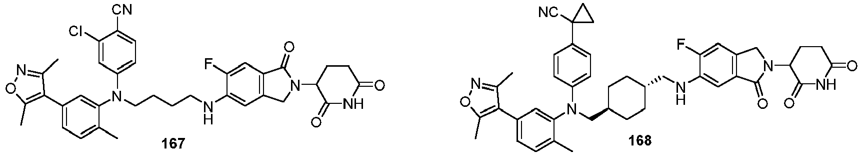

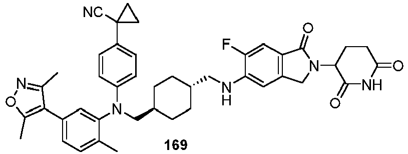

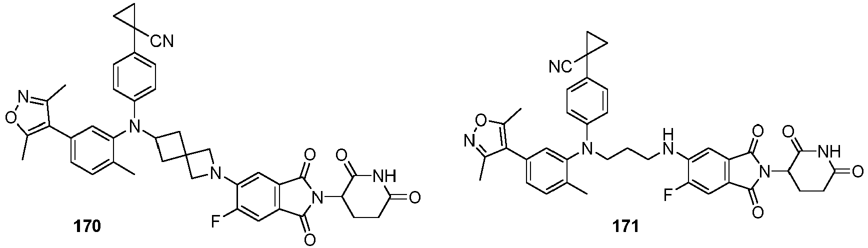

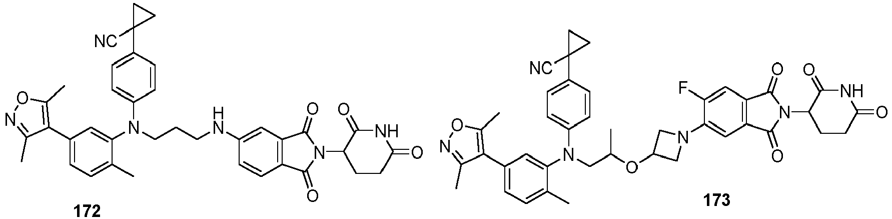

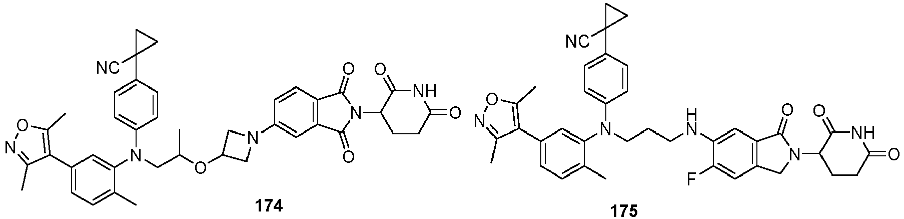

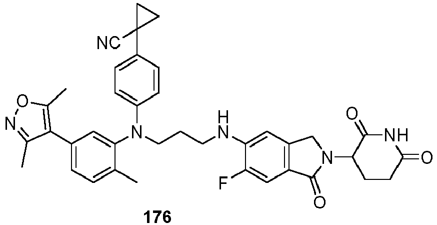

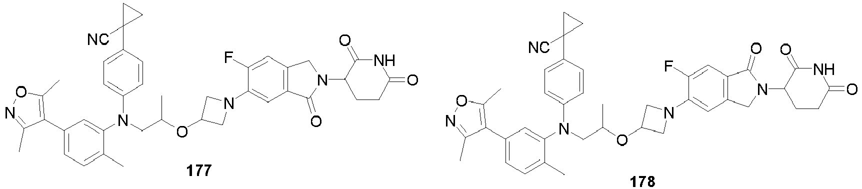

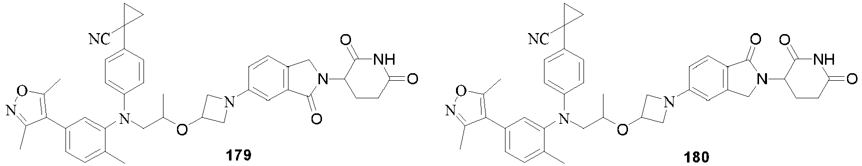

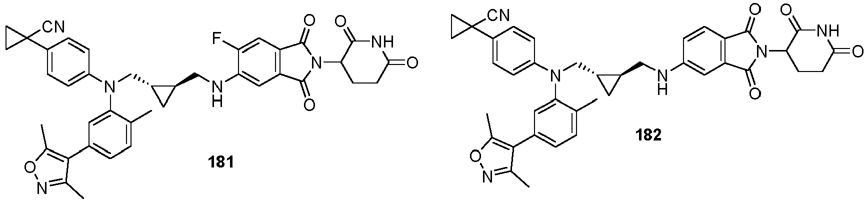

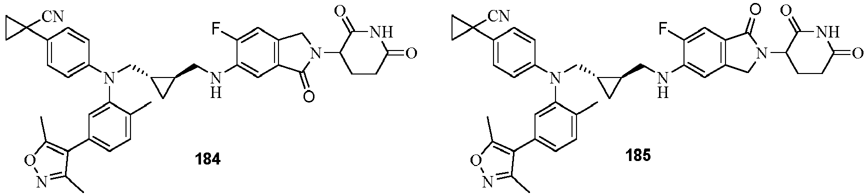

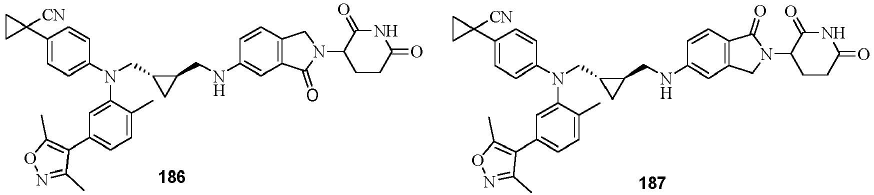

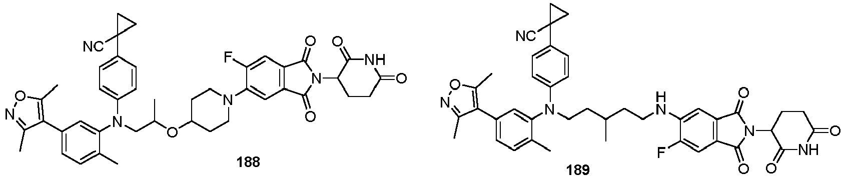

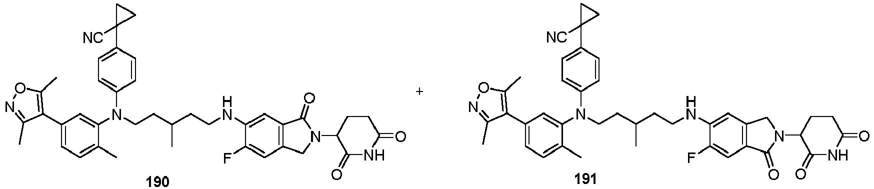

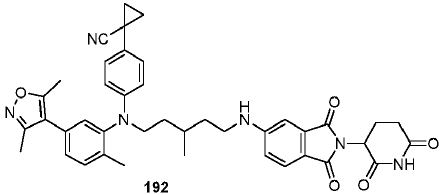

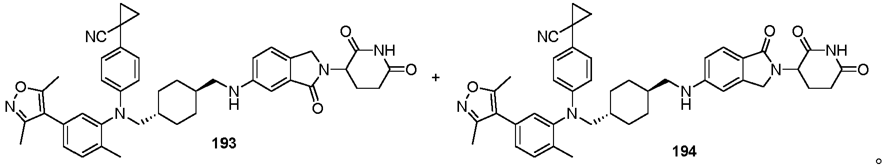

17. Use of a compound according to any one of claims 1 to 16, or an optical isomer thereof, or a solvate thereof, or a pharmaceutically acceptable salt thereof, or a prodrug thereof, or a tautomer thereof, or a mesomer thereof, or a racemate thereof, or an enantiomer thereof, or a diastereomer thereof, or a combination thereof, or a metabolite thereof, or a metabolic precursor thereof, or an isotopic compound thereof in the preparation of chimeras targeting the protein degradation of androgen receptors and/or BET.

18. The use according to claim 17, **characterized in that** the proteolytic targeting chimera can specifically recognize/bind AR and/or BET.

19. The use according to claim 17, **characterized in that** the proteolytic targeting chimera can degrade AR and/or BET.

20. The use according to any one of claims 17 to 19, **characterized in that** the proteolytic targeting chimera is a drug for the treatment of the diseases related to AR and/or BET.

21. The use according to claim 20, **characterized in that** said disease is selected from the group consisting of prostate cancer, breast cancer and Kennedy's disease.
